# EUROPEAN PATENT APPLICATION

(11) **EP 2 540 817 A1**
(43) Date of publication of application: **02.01.2013**
(21) Application number: 12173233.3
(22) Date of filing: 29.04.2008
(51) Int. Cl.: C12N 1/20, C12N 15/52, C12P 7/16

(54) **Methods and recombinant microbial hosts for the production of isobutanol**

(30) Priority: 02.05.2007 US 915467 P
(62) Divisional of application: 08780577.6
(71) Applicant: Butamax (TM) Advanced Biofuels LLC, Wilmington, DE 19803 (US)
(72) Inventor: Bramucci, Michael G., Boothwyn, PA 19061 (US); Miller, Edward, S. Jr., Knoxville, TN 37394 (US); Flint, Dennis, Newark, DE 19711 (US); Nagarajan, Vasantha, Wilmington, DE 19807 (US); Sedkova, Natalia, Cherry Hill, NJ 08003 (US); Singh, Manjari, West Chester, PA 19382 (US); Van Dyk, Tina, K., Wilmington, DE 19803 (US)
(74) Representative: Rigby, Barbara Nicole

(57) **Abstract**

A method for the production of isobutanol by fermentation using a microbial production host is disclosed. The method employs a reduction in temperature during the fermentation process that results in a more robust tolerance of the production host to the butanol product.

## Description

### FIELD OF THE INVENTION

The invention relates to a method for the production of isobutanol by fermentation using a recombinant microbial host. Specifically, the method employs a decrease in temperature during fermentation that results in more robust tolerance of the production host to the isobutanol product.

### BACKGROUND OF THE INVENTION

Butanol is an important industrial chemical, useful as a fuel additive, as a feedstock chemical in the plastics industry, and as a foodgrade extractant in the food and flavor industry. Each year 10 to12 billion pounds of butanol are produced by petrochemical means and the need for this commodity chemical will likely increase.

Methods for the chemical synthesis of isobutanol are known, such as oxo synthesis, catalytic hydrogenation of carbon monoxide (Ullmann's Encyclopedia of Industrial Chemistry, 6th edition, 2003, Wiley-VCHVerlag GmbH and Co., Weinheim, Germany, Vol, 5, pp. 716-719) and Guerbet condensation of methanol with n-propanol (Carlini et al., J. Mol. Catal. A:Chem. 220:215-220 (2004)). These processes use starting materials derived from petrochemicals and are generally expensive and are not environmentally friendly. The production of isobutanol from plant-derived raw materials would minimize greenhouse gas emissions and would represent an advance in the art.

Isobutanol is produced biologically as a by-product of yeast fermentation. It is a component of "fusel oil" that forms as a result of incomplete metabolism of amino acids by this group of fungi. Isobutanol is specifically produced from catabolism of L-valine. After the amine group of L-valine is harvested as a nitrogen source, the resulting α-keto acid is decarboxylated and reduced to isobutanol by enzymes of the so-called Ehrlich pathway (Dickinson et al., J. Biol. Chem. 273(40):25752-25756 (1998)). Yields of fusel oil and/or its components achieved during beverage fermentation are typically low. For example, the concentration of isobutanol produced in beer fermentation is reported to be less than 16 parts per million (Garcia et al., Process Biochemistry 29:303-309 (1994)). Addition of exogenous L-valine to the fermentation increases the yield of isobutanol, as described by Dickinson et al., *supra,* wherein it is reported that a yield of isobutanol of 3 g/L is obtained by providing L-valine at a concentration of 20 g/L in the fermentation. However, the use of valine as a feed-stock would be cost prohibitive for industrial scale isobutanol production. The biosynthesis of isobutanol directly from sugars would be economically viable and would represent an advance in the art,

Recombinant microbial production hosts expressing an isobutanol biosynthetic pathway have been described (Donaldson et al., copending and commonly owned U.S. Patent Application Publication No. US20070092957 A1). However, biological production of isobutanol is believed to be limited by butanol toxicity to the host microorganism used in the fermentation.

Some microbial strains that are tolerant to isobutanol are known in the art (Bramucci et al., copending and commonly owned U.S. Patent Application Nos. 11/743220and 11/761497). However, biological methods of producing isobutanol to higher levels are required for cost effective commercial production.

There have been reports describing the effect of temperature on the tolerance of some microbial strains to ethanol. For example, Amartey et al. (Biotechnol. Lett. 13(9):627-632 (1991)) disclose that *Bacillus stearothermophillus* is less tolerant to ethanol at 70 °C than at 60 °C. Herrero et al. (Appl. Environ. Microbiol. 40(3):571-577 (1980)) report that the optimum growth temperature of a wild-type strain of *Clostridium thermocellum* decreases as the concentration of ethanol challenge increases, whereas the optimum growth temperature of an ethanol-tolerant mutant remains constant. Brown et al. (Biotechnol. Lett. 4(4):269-274 (1982)) disclose that the yeast *Saccharomyces uvarum* is more resistant to growth inhibition by ethanol at temperatures 5 °C and 10 °C below its growth optimum of 35 °C. However, fermentation became more resistant to ethanol inhibition with increasing temperature. Additionally, Van Uden (CRC Crit. Rev. Biotechnol. 1(3):263-273 (1984)) report that ethanol and other alkanols depress the maximum and the optimum growth temperature for growth of *Saccharomyces cerevisiae* while thermal death is enhanced. Moreover, Lewis et al. (U.S. Patent Application Publication No. 2004/0234649) describe methods for producing high levels of ethanol during fermentation of plant material comprising decreasing the temperature during saccharifying, fermenting, or simultaneously saccharifying and fermenting.

Much less is known about the effect of temperature on the tolerance of microbial strains to butanol. Harada (Hakko Kyakaishi 20:155-156 (1962)) discloses that the yield of 1-butanol in acetone-butanol-ethanol (ABE) fermentation is increased from 18.4%-18.7% to 19.1 %-21.2% by lowering the temperature from 30 °C to 28 °C when the growth of the bacteria reaches a maximum. Jones et al. (Microbiol. Rev. 50(4):484-524 (1986)) review the role of temperature in ABE fermentation. They report that the solvent yields of three different solvent producing strains remains fairly constant at 31 % at 30 °C and 33 °C, but decreases to 23 to 25% at 37 °C. Similar results were reported for *Clostridium acetobutylicum* for which solvent yields decreased from 29% at 25 °C to 24% at 40 °C. In the latter case, the decrease in solvent yield was attributed to a decrease in acetone production while the yield of 1-butanol was unaffected. However, Carnarius (U.S. Patent No. 2,198,104) reports that an increase in the butanol ratio is obtained in the ABE process by decreasing the temperature of the fermentation from 30 °C to 24 °C after 16 hours. However, the effect of temperature on the production of isobutanol by recombinant microbial hosts is not known in the art.

There is a need, therefore, for a cost-effective process for the production of isobutanol by fermentation that provides higher yields than processes known in the art. The present invention addresses this need through the discovery of a method for producing isobutanol by fermentation using a recombinant microbial host, which employs a decrease in temperature during fermentation, resulting in more robust tolerance of the production host to the isobutanol product.

### SUMMARY OF THE INVENTION

The invention provides a method for the production of isobutanol by fermentation using a recombinant microbial host, which employs a decrease in temperature during fermentation that results in more robust tolerance of the production host to the isobutanol product.

Accordingly, the invention provides a method for the production of isobutanol comprising:
a) providing a recombinant microbial production host which produces isobutanol;
b) seeding the production host of (a) into a fermentation medium comprising a fermentable carbon substrate to create a fermentation culture;
c) growing the production host in the fermentation culture at a first temperature for a first period of time;
d) lowering the temperature of the fermentation culture to a second temperature; and
e) incubating the production host at the second temperature of step (d) for a second period of time;
whereby isobutanol is produced.

### BRIEF DESCRIPTION OF THE FIGURES AND SEQUENCE DESCRIPTIONS

The invention can be more fully understood from the following detailed description, figure, and the accompanying sequence descriptions, which form a part of this application.

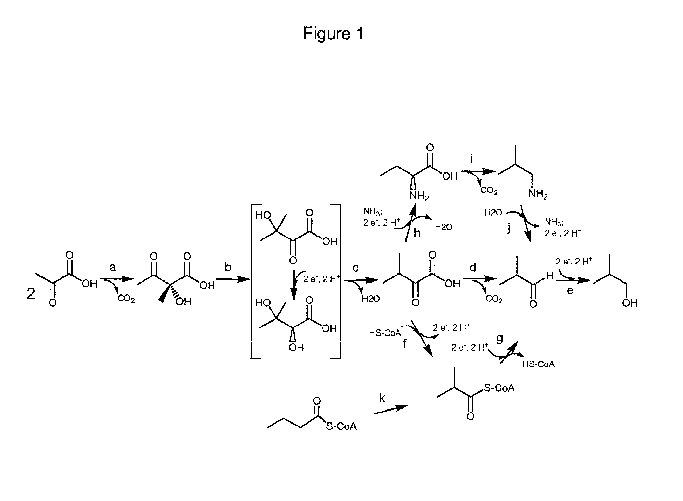

Figure 1 shows four different isobutanol biosynthetic pathways. The steps labeled "a", "b", "c", "d", "e", "f', "g", "h", "i", "j" and "k" represent the substrate to product conversions described below.

The following sequences conform with 37 C.F.R. 1.821-1.825 ("Requirements for Patent Applications Containing Nucleotide Sequences and/or Amino Acid Sequence Disclosures - the Sequence Rules") and are consistent with World Intellectual Property Organization (WIPO) Standard ST.25 (1998) and the sequence listing requirements of the EPO and PCT (Rules 5.2 and 49.5(a-bis), and Section 208 and Annex C of the Administrative Instructions). The symbols and format used for nucleotide and amino acid sequence data comply with the rules set forth in 37 C.F.R. §1.822.

**Table 1**

| Summary of Gene and Protein SEQ ID Numbers | | |
|---|---|---|
| Description | SEQ ID NO: Nucleic acid | SEQ ID NO: Peptide |
| *Klebsiella pneumoniae budB* (acetolactate synthase) | 1 | 2 |
| *Bacillus subtilis a*/*sS* (acetolactate synthase) | 78 | 178 |
| *Lactococcus lactis als* (acetolactate synthase) | 179 | 180 |
| *E. coli ilvC* (acetohydroxy acid reductoisomerase) | 3 | 4 |
| *S. cerevisiae ILV5* {acetohydroxy acid reductoisomerase) | 80 | 181 |
| *M. maripaludis* ilvC (Ketol-acid reductoisomerase) | 182 | 183 |
| *B. subtilis ilvC* (acetohydroxy acid reductoisomerase) | 184 | 185 |
| *E. coli ilvD* (acetohydroxy acid dehydratase) | 5 | 6 |
| *S. cerevisiae ILV3* (Dihydroxyacid dehydratase) | 83 | 186 |
| *M. maripaludis ilvD* (Dihydroxy-acid dehydratase) | 187 | 188 |
| *B. subtilis ilvD* (dihydroxy-acid dehydratase) | 189 | 190 |
| *Lactococcus lactis kivD* (branched-chain α-keto acid decarboxylase), | 7 | 8 |
| codon optimized | | |
| *Lactococcus lactis kivD* (branched-chain α-keto acid decarboxylase), | 191 | 8 |
| *Lactococcus lactis kdcA* (branched-chain alpha-ketoacid decarboxylase) | 192 | 193 |
| *Salmonella typhimurium* (indolepyruvate decarboxylase) | 194 | 195 |
| *Clostridium acetobutylicum pdc* (Pyruvate decarboxylase) | 196 | 197 |
| *E. coli yqhD* (branched-chain alcohol dehydrogenase) | 9 | 10 |
| *S. cerevisiae YPR1* (2-methylbutyraldehyde reductase) | 198 | 199 |
| *S. cerevisiae ADH6* (NADPH-dependent cinnamyl alcohol dehydrogenase) | 200 | 201 |
| *Clostridium acetobutylicum bdhA* (NADH-dependent butanol dehydrogenase A) | 202 | 203 |
| *Clostridium acetobutylicum bdhB* Butanol dehydrogenase | 158 | 204 |
| *B. subtilis bkdAA* (branched-chain keto acid dehydrogenase E1 subunit) | 205 | 206 |
| *B. subtilis* bkdAB (branched-chain alpha-keto acid dehydrogenase E1 subunit) | 207 | 208 |
| *B*. *subtilis bkdB* (branched-chain alpha-keto acid dehydrogenase E2 subunit) | 209 | 210 |
| *B*. *subtilis lpdV* (branched-chain alpha-keto acid dehydrogenase E3 subunit) | 211 | 212 |
| *P. putida bkdA1* (keto acid dehydrogenase E1-alpha subunit) | 213 | 214 |
| *P. putida bkdA2* (keto acid dehydrogenase E1-beta | 215 | 216 |
| subunit) | | |
| *P. putida bkdB* (transacylase E2) | 217 | 218 |
| *P. putida 1pdv* (lipoamide dehydrogenase) | 219 | 220 |
| *C. beijerinckii ald* (coenzyme A acylating aldehyde dehydrogenase) | 221 | 222 |
| *C*. *acetobutylicum adhe 1* (aldehyde dehydrogenase) | 223 | 224 |
| *C*. *acetobutylicum* adhe (alcohol-aldehyde dehydrogenase) | 225 | 226 |
| *P. putida nahO* (acetaldehyde dehydrogenase) | 227 | 228 |
| *T. thermophilus* (acetaldehyde dehydrogenase) | 229 | 230 |
| *E. coli avtA* (valine-pyruvate transaminase) | 231 | 232 |
| *B. licheniformis avtA* (valine-pyruvate transaminase) | 233 | 234 |
| *E. coli ilvE* (branched chain amino acid aminotransferase) | 235 | 236 |
| *S*. *cerevisiae BAT2* (branched chain amino acid aminotransferase) | 237 | 238 |
| *M. thermoautotrophicum* (branched chain amino acid aminotransferase) | 239 | 240 |
| *S. coelicolor* (valine dehydrogenase) | 241 | 242 |
| *B. subtilis bcd* (leucine dehydrogenase) | 243 | 244 |
| *S*. *viridifaciens* (valine decarboxyase) | 245 | 246 |
| *A. denitrificans aptA* (omega-amino acid:pyruvate | 247 | 248 |
| transaminase) | | |
| *R. eutropha* (alanine-pyruvate transaminase) | 249 | 250 |
| *S. oneidensis* (beta alanine-pyruvate transaminase) | 251 | 252 |
| *P. putida* (beta alanine-pyruvate transaminase) | 253 | 254 |
| *S. cinnamonensis icm* (isobutyrl-CoA mutase) | 255 | 256 |
| *S. cinnamonensis icmB* (isobutyrl-CoA mutase) | 257 | 258 |
| *S*. *coelicolor SCO5415* (isobutyrl-CoA mutase) | 259 | 260 |
| *S*. *coelicolor SCO4800* (isobutyrl-CoA mutase) | 261 | 262 |
| *S. avermitilis icmA* (isobutyrl-CoA mutase) | 263 | 264 |
| *S. avermitilis icmB* (isobutyrl-CoA mutase) | 265 | 266 |

SEQ ID NOs:11-38, 40-69, 72-75, 85-138, 144, 145, 147-157,159-176 are the nucleotide sequences of oligonucleotide cloning, screening or sequencing primers used in the Examples described herein.

SEQ ID NO:39 is the nucleotide sequence of the *cscBKA* gene cluster described in Example 20.

SEQ ID NO:70 is the nucleotide sequence of the glucose isomerase promoter 1.6GI described in Example 17.

SEQ ID NO:71 is the nucleotide sequence of the 1.5GI promoter described in Example 17.

SEQ ID NO:76 is the nucleotide sequence of the GPD promoter described in Example 21.

SEQ ID NO:77 is the nucleotide sequence of the CYC1 terminator described in Example 21.

SEQ ID NO:79 is the nucleotide sequence of the FBA promoter described in Example 21.

SEQ ID NO:81 is the nucleotide sequence of ADH1 promoter described in Example 21.

SEQW ID NO:82 is the nucleotide sequence of ADH1 terminator described in Example 21.

SEQ ID NO:84 is the nucleotide sequence of GPM promoter described in Example 21.

SEQ ID NO:139 is the amino acid sequence of sucrose hydrolase (CscA).

SEQ ID NO:140 is the amino acid sequence of D-fructokinase (CscK).

SEQ ID NO:141 is the amino acid sequence of sucrose permease (CscB).

SEQ ID NO:142 is the nucleotide sequence of plasmid pFP988DssPspac described in Example 24.

SEQ ID NO:143 is the nucleotide sequence of plasmid pFP988DssPgroE described in Example 24.

SEQ ID NO:146 is the nucleotide sequence of the pFP988Dss vector fragment described in Example 24.

SEQ ID NO:177 is the nucleotide sequence of the pFP988 integration vector described in Example 25.

SEQ ID NO:267 is the nucleotide sequence of plasmid pC194 described in Example 25.

### DETAILED DESCRIPTION OF THE INVENTION

The present invention relates to a method for the production of isobutanol using recombinant microorganisms that employs a decrease in temperature during fermentation, resulting in more robust tolerance of the production host to the isobutanol product and therefore a higher titer of isobutanol. The present invention meets a number of commercial and industrial needs. Butanol is an important industrial commodity chemical with a variety of applications, where its potential as a fuel or fuel additive is particularly significant. Although only a four-carbon alcohol, butanol has an energy content similar to that of gasoline and can be blended with any fossil fuel. Butanol is favored as a fuel or fuel additive as it yields only CO₂ and little or no SOₓ or NOₓ when burned in the standard internal combustion engine. Additionally butanol is less corrosive than ethanol, the most preferred fuel additive to date.

In addition to its utility as a biofuel or fuel additive, butanol has the potential of impacting hydrogen distribution problems in the emerging fuel cell industry. Fuel cells today are plagued by safety concerns associated with hydrogen transport and distribution. Butanol can be easily reformed for its hydrogen content and can be distributed through existing gas stations in the purity required for either fuel cells or vehicles.

Finally the present invention produces isobutanol from plant derived carbon sources, avoiding the negative environmental impact associated with standard petrochemical processes for butanol production.

The following definitions and abbreviations are to be used for the interpretation of the claims and the specification.

As used herein, the terms "comprises," "comprising," "includes," "including," "has," "having," "contains" or "containing," or any other variation thereof, are intended to cover a non-exclusive inclusion. For example, a composition, a mixture, process, method, article, or apparatus that comprises a list of elements is not necessarily limited to only those elements but may include other elements not expressly listed or inherent to such composition, mixture, process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

Also, the indefinite articles "a" and "an" preceding an element or component of the invention are intended to be nonrestrictive regarding the number of instances (i.e. occurrences) of the element or component. Therefore "a" or "an" should be read to include one or at least one, and the singular word form of the element or component also includes the plural unless the number is obviously meant to be singular.

The term "invention" or "present invention" as used herein is a non-limiting term and is not intended to refer to any single embodiment of the particular invention but encompasses all possible embodiments as described in the specification and the claims.

As used herein, the term "about" modifying the quantity of an ingredient or reactant of the invention employed refers to variation in the numerical quantity that can occur, for example, through typical measuring and liquid handling procedures used for making concentrates or use solutions in the real world; through inadvertent error in these procedures; through differences in the manufacture, source, or purity of the ingredients employed to make the compositions or carry out the methods; and the like. The term "about" also encompasses amounts that differ due to different equilibrium conditions for a composition resulting from a particular initial mixture. Whether or not modified by the term "about", the claims include equivalents to the quantities. In one embodiment, the term "about" means within 10% of the reported numerical value, preferably within 5% of the reported numerical value.

The term "isobutanol biosynthetic pathway" refers to an enzyme pathway to produce isobutanol.

The terms "acetolactate synthase" and "acetolactate synthetase" are used intechangeably herein to refer to an enzyme that catalyzes the conversion of pyruvate to acetolactate and CO₂. Preferred acetolactate synthases are known by the EC number 2.2.1.6 9 (*Enzyme Nomenclature* 1992, Academic Press, San Diego). These enzymes are available from a number of sources, including, but not limited to, *Bacillus subtilis* (GenBank Nos: CAB15618 (SEQ ID NO:178), Z99122 (SEQ ID NO:78), NCBI (National Center for Biotechnology Information) amino acid sequence, NCBI nucleotide sequence, respectively), *Klebsiella pneumoniae* (GenBank Nos: AAA25079 (SEQ ID NO:2), M73842 (SEQ ID NO:1)), and *Lactococcus lactis* (GenBank Nos: AAA25161 (SEQ ID NO:180), L16975 (SEQ ID NO:179)).

The terms "acetohydroxy acid isomeroreductase" and "acetohydroxy acid reductoisomerase" are used interchangeably herein to refer to an enzyme that catalyzes the conversion of acetolactate to 2,3-dihydroxyisovalerate using NADPH (reduced nicotinamide adenine dinucleotide phosphate) as an electron donor. Preferred acetohydroxy acid isomeroreductases are known by the EC number 1.1.1.86 and sequences are available from a vast array of microorganisms, including, but not limited to, *Escherichia coli* (GenBank Nos: NP_418222 (SEQ ID NO:4), NC_000913 (SEQ ID NO:3)), *Saccharomyces cerevisiae* (GenBank Nos: NP_013459 (SEQ ID NO:181), NC_001144 (SEQ ID NO:80)), *Methanococcus maripaludis* (GenBank Nos: CAF30210 (SEQ ID NO:183), BX957220 (SEQ ID NO:182)), and *Bacillus. subtilis* (GenBank Nos: CAB14789 (SEQ ID NO:185), Z99118 (SEQ ID NO:184)).

The term "acetohydroxy acid dehydratase" refers to an enzyme that catalyzes the conversion of 2,3-dihydroxyisovalerate to α-ketoisovalerate. Preferred acetohydroxy acid dehydratases are known by the EC number 4.2.1.9. These enzymes are available from a vast array of microorganisms, including, but not limited to, *E*. *coli* (GenBank Nos: YP_026248 (SEQ ID NO:6), NC_000913 (SEQ ID NO:5)), *S. cerevisiae* (GenBank Nos: NP_012550 (SEQ ID NO:186), NC_001142 (SEQ ID NO:83)), *M. maripaludis* (GenBank Nos: CAF29874 (SEQ ID NO:188), BX957219 (SEQ ID NO:187)), and *B. subtilis* (GenBank Nos: CAB14105 (SEQ ID NO:190), Z99115 (SEQ ID NO:189)).

The term "branched-chain α-keto acid decarboxylase" refers to an enzyme that catalyzes the conversion of α-ketoisovalerate to isobutyraldehyde and CO₂. Preferred branched-chain α-keto acid decarboxylases are known by the EC number 4.1.1.72 and are available from a number of sources, including, but not limited to, *Lactococcus lactis* (GenBank Nos: AAS49156 (SEQ ID NO:193), AY548760 (SEQ ID NO:192); CAG34226 (SEQ ID NO:8), AJ746364 (SEQ ID NO:191), *Salmonella typhimurium* (GenBank Nos: NP_461346 (SEQ ID NO:195), NC_003197 (SEQ ID NO:194)), and *Clostridium acetobutylicum* (GenBank Nos: NP_149189 (SEQ ID NO:197), NC_001988 (SEQ ID NO:196)).

The term "branched-chain alcohol dehydrogenase" refers to an enzyme that catalyzes the conversion of isobutyraldehyde to isobutanol. Preferred branched-chain alcohol dehydrogenases are known by the EC number 1.1.1.265, but may also be classified under other alcohol dehydrogenases (specifically, EC 1.1.1.1 or 1.1.1.2). These enzymes utilize NADH (reduced nicotinamide adenine dinucleotide) and/or NADPH as electron donor and are available from a number of sources, including, but not limited to, *S. cerevisiae* (GenBank Nos: NP_010656 (SEQ ID NO:199), NC_001136 (SEQ ID NO:198); NP_014051 (SEQ ID NO:201) NC_001145 (SEQ ID NO:200)), *E*. *coli* (GenBank Nos: NP_417484 (SEQ ID NO:10), NC_000913 (SEQ ID NO:9)), and C. *acetobutylicum* (GenBank Nos: NP_349892 (SEQ ID NO:203), NC_003030 (SEQ ID NO:202); NP_349891 (SEQ ID NO:204), NC_003030 (SEQ ID NO:158)).

The term "branched-chain keto acid dehydrogenase" refers to an enzyme that catalyzes the conversion of α-ketoisovalerate to isobutyryl-CoA (isobutyryl-coenzyme A), using NAD⁺ (nicotinamide adenine dinucleotide) as electron acceptor. Preferred branched-chain keto acid dehydrogenases are known by the EC number 1.2.4.4. These branched-chain keto acid dehydrogenases are comprised of four subunits and sequences from all subunits are available from a vast array of microorganisms, including, but not limited to, *B. subtilis* (GenBank Nos: CAB14336 (SEQ ID NO:206), Z99116 (SEQ ID NO:205); CAB14335 (SEQ ID NO:208), Z99116 (SEQ ID NO:207); CAB14334 (SEQ ID NO:210), Z99116 (SEQ ID NO:209); and CAB14337 (SEQ ID NO:212), Z99116 (SEQ ID NO:211)) and *Pseudomonas putida* (GenBank Nos: AAA65614 (SEQ ID NO:214), M57613 (SEQ ID NO:213); AAA65615 (SEQ ID NO:216), M57613 (SEQ ID NO:215); AAA65617 (SEQ ID NO:218), M57013 (SEQ ID NO:217); and AAA65618 (SEQ ID NO:220), M57613 (SEQ ID NO:219)).

The term "acylating aldehyde dehydrogenase" refers to an enzyme that catalyzes the conversion of isobutyryl-CoA to isobutyraldehyde, using either NADH or NADPH as electron donor. Preferred acylating aldehyde dehydrogenases are known by the EC numbers 1.2.1.10 and 1,2.1.57. These enzymes are available from multiple sources, including, but not limited to, *Clostridium beijerinckii* (GenBank Nos: AAD31841 (SEQ ID NO:222), AF157306 (SEQ ID NO:221)), C. *acetobutylicum* (GenBank Nos: NP_149325 (SEQ ID NO:224), NC_001988 (SEQ ID NO:223); NP_149199 (SEQ ID NO:226), NC_001988 (SEQ ID NO:225)), *P. putida* (GenBank Nos: AAA89106 (SEQ ID NO:228), U13232 (SEQ ID NO:227)), and *Thermus thermophilus* (GenBank Nos: YP_145486 (SEQ ID NO:230), NC_006461 (SEQ ID NO:229)).

The term "transaminase" refers to an enzyme that catalyzes the conversion of α-ketoisovalerate to L-valine, using either alanine or glutamate as amine donor. Preferred transaminases are known by the EC numbers 2.6.1.42 and 2.6.1.66. These enzymes are available from a number of sources. Examples of sources for alanine-dependent enzymes include, but are not limited to, *E*. *coli* (GenBank Nos: YP_026231 (SEQ ID NO:232), NC_000913 (SEQ ID NO:231)) and *Bacillus licheniformis* (GenBank Nos: YP_093743 (SEQ ID NO:234), NC_006322 (SEQ ID NO:233)). Examples of sources for glutamate-dependent enzymes include, but are not limited to, *E. coli* (GenBank Nos: YP_026247 (SEQ ID NO:236), NC_000913 (SEQ ID NO:235)), *S. cerevisiae* (GenBank Nos: NP_012682 (SEQ ID NO:238), NC_001142 (SEQ ID NO:237)) and *Methanobacterium thermoautotrophicum* (GenBank Nos: NP_276546 (SEQ ID NO:240), NC_000916 (SEQ ID NO:239)).

The term "valine dehydrogenase" refers to an enzyme that catalyzes the conversion of α-ketoisovalerate to L-valine, using NAD(P)H as electron donor and ammonia as amine donor. Preferred valine dehydrogenases are known by the EC numbers 1.4.1.8 and 1.4.1.9 and are available from a number of sources, including, but not limited to, *Streptomyces coelicolor* (GenBank Nos: NP_628270 (SEQ ID NO:242), NC_003888 (SEQ ID NO:241)) and *B. subtilis* (GenBank Nos: CAB14339 (SEQ ID NO:244), Z99116 (SEQ ID NO:243)).

The term "valine decarboxylase" refers to an enzyme that catalyzes the conversion of L-valine to isobutylamine and CO₂. Preferred valine decarboxylases are known by the EC number 4.1.1.14. These enzymes are found in Streptomycetes, such as for example, *Streptomyces viridifaciens* (GenBank Nos: AAN10242 (SEQ ID NO:246), AY116644 (SEQ ID NO:245)).

The term "omega transaminase" refers to an enzyme that catalyzes the conversion of isobutylamine to isobutyraldehyde using a suitable amino acid as amine donor. Preferred omega transaminases are known by the EC number 2.6.1.18 and are available from a number of sources, including, but not limited to, *Alcaligenes denitrificans* (AAP92672 (SEQ ID NO:248), AY330220 (SEQ ID NO:247)), *Ralstonia eutropha* (GenBank Nos: YP_294474 (SEQ ID NO:250), NC_007347 (SEQ ID NO:249)), *Shewanella oneidensis* (GenBank Nos: NP_719046 (SEQ ID NO:252), NC_004347 (SEQ ID NO:251)), and *P. putida* (GenBank Nos: AAN66223 (SEQ ID NO:254), AE016776 (SEQ ID NO:253)).

The term "isobutyryl-CoA mutase" refers to an enzyme that catalyzes the conversion of butyryl-CoA to isobutyryl-CoA. This enzyme uses coenzyme B₁₂ as cofactor. Preferred isobutyryl-CoA mutases are known by the EC number 5.4.99.13. These enzymes are found in a number of Streptomycetes, including, but not limited to, *Streptomyces cinnamonensis* (GenBank Nos: AAC08713 (SEQ ID NO:256), U67612 (SEQ ID NO:255); CAB59633 (SEQ ID NO:258), AJ246005 (SEQ ID NO:257)), *S*. *coelicolor* (GenBank Nos: CAB70645 (SEQ ID NO:260), AL939123 (SEQ ID NO:259); CAB92663 (SEQ ID NO:262), AL939121 (SEQ ID NO:261)), and *Streptomyces avermitilis* (GenBank Nos: NP_824008 (SEQ ID NO:264), NC_003155 (SEQ ID NO:263); NP_824637 (SEQ ID NO:266), NC_003155 (SEQ ID NO:265)).

The term "a facultative anaerobe" refers to a microorganism that can grow in both aerobic and anaerobic environments.

The term "carbon substrate" or "fermentable carbon substrate" refers to a carbon source capable of being metabolized by host organisms disclosed herein and particularly carbon sources selected from the group consisting of monosaccharides, oligosaccharides, polysaccharides, and one-carbon substrates or mixtures thereof.

The term "gene" refers to a nucleic acid fragment that is capable of being expressed as a specific protein, optionally including regulatory sequences preceding (5' non-coding sequences) and following (3' non-coding sequences) the coding sequence. "Native gene" refers to a gene as found in nature with its own regulatory sequences. "Chimeric gene" refers to any gene that is not a native gene, comprising regulatory and coding sequences that are not found together in nature. Accordingly, a chimeric gene may comprise regulatory sequences and coding sequences that are derived from different sources, or regulatory sequences and coding sequences derived from the same source, but arranged in a manner different than that found in nature. "Endogenous gene" refers to a native gene in its natural location in the genome of an organism. A "foreign gene" or "heterologous gene" refers to a gene not normally found in the host organism, but that is introduced into the host organism by gene transfer. Foreign genes can comprise native genes inserted into a non-native organism, or chimeric genes. A "transgene" is a gene that has been introduced into the genome by a transformation procedure.

As used herein, an "isolated nucleic acid fragment" or "isolated nucleic acid molecule" or "genetic construct" will be used interchangeably and will mean a polymer of RNA or DNA that is single- or double-stranded, optionally containing synthetic, non-natural or altered nucleotide bases. An isolated nucleic acid fragment in the form of a polymer of DNA may be comprised of one or more segments of cDNA, genomic DNA or synthetic DNA.

A nucleic acid fragment is "hybridizable" to another nucleic acid fragment, such as a cDNA, genomic DNA, or RNA molecule, when a single-stranded form of the nucleic acid fragment can anneal to the other nucleic acid fragment under the appropriate conditions of temperature and solution ionic strength. Hybridization and washing conditions are well known and exemplified in Sambrook, J., Fritsch, E. F. and Maniatis, T. Molecular Cloning. A Laboratory Manual, 2^{nd} ed., Cold Spring Harbor Laboratory: Cold Spring Harbor, NY (1989), particularly Chapter 11 and Table 11.1 therein (entirely incorporated herein by reference). The conditions of temperature and ionic strength determine the "stringency" of the hybridization. Stringency conditions can be adjusted to screen for moderately similar fragments (such as homologous sequences from distantly related organisms), to highly similar fragments (such as genes that duplicate functional enzymes from closely related organisms). Post-hybridization washes determine stringency conditions. One set of preferred conditions uses a series of washes starting with 6X SSC, 0.5% SDS at room temperature for 15 min, then repeated with 2X SSC, 0.5% SDS at 45 °C for 30 min, and then repeated twice with 0.2X SSC, 0.5% SDS at 50 °C for 30 min. A more preferred set of stringent conditions uses higher temperatures in which the washes are identical to those above except for the temperature of the final two 30 min washes in 0.2X SSC, 0.5% SDS was increased to 60 °C. Another preferred set of highly stringent conditions uses two final washes in 0.1X SSC, 0.1% SDS at 65 °C. An additional set of stringent conditions include hybridization at 0.1X SSC, 0.1% SDS, 65 °C and washes with 2X SSC, 0.1% SDS followed by 0.1X SSC, 0.1% SDS, for example.

Hybridization requires that the two nucleic acids contain complementary sequences, although depending on the stringency of the hybridization, mismatches between bases are possible. The appropriate stringency for hybridizing nucleic acids depends on the length of the nucleic acids and the degree of complementation, variables well known in the art. The greater the degree of similarity or homology between two nucleotide sequences, the greater the value of Tm for hybrids of nucleic acids having those sequences. The relative stability (corresponding to higher Tm) of nucleic acid hybridizations decreases in the following order: RNA:RNA, DNA:RNA, DNA:DNA. For hybrids of greater than 100 nucleotides in length, equations for calculating Tm have been derived (see Sambrook et al., *supra,* 9.50-9.51). For hybridizations with shorter nucleic acids, i.e., oligonucleotides, the position of mismatches becomes more important, and the length of the oligonucleotide determines its specificity (see Sambrook et al., *supra,* 11.7-11.8). In one embodiment the length for a hybridizable nucleic acid is at least about 10 nucleotides. Preferably a minimum length for a hybridizable nucleic acid is at least about 15 nucleotides; more preferably at least about 20 nucleotides; and most preferably the length is at least about 30 nucleotides. Furthermore, the skilled artisan will recognize that the temperature and wash solution salt concentration may be adjusted as necessary according to factors such as length of the probe.

A "substantial portion" of an amino acid or nucleotide sequence is that portion comprising enough of the amino acid sequence of a polypeptide or the nucleotide sequence of a gene to putatively identify that polypeptide or gene, either by manual evaluation of the sequence by one skilled in the art, or by computer-automated sequence comparison and identification using algorithms such as BLAST (Altschul, S. F., et al., J. Mol. Biol., 215:403-410 (1993)). In general, a sequence of ten or more contiguous amino acids or thirty or more nucleotides is necessary in order to putatively identify a polypeptide or nucleic acid sequence as homologous to a known protein or gene. Moreover, with respect to nucleotide sequences, gene specific oligonucleotide probes comprising 20-30 contiguous nucleotides may be used in sequence-dependent methods of gene identification (e.g., Southern hybridization) and isolation (e.g., *in situ* hybridization of bacterial colonies or bacteriophage plaques). In addition, short oligonucleotides of 12-15 bases may be used as amplification primers in PCR in order to obtain a particular nucleic acid fragment comprising the primers. Accordingly, a "substantial portion" of a nucleotide sequence comprises enough of the sequence to specifically identify and/or isolate a nucleic acid fragment comprising the sequence. The instant specification teaches the complete amino acid and nucleotide sequence encoding particular fungal proteins. The skilled artisan, having the benefit of the sequences as reported herein, may now use all or a substantial portion of the disclosed sequences for purposes known to those skilled in this art. Accordingly, the instant invention comprises the complete sequences as reported in the accompanying Sequence Listing, as well as substantial portions of those sequences as defined above.

The term "complementary" is used to describe the relationship between nucleotide bases that are capable of hybridizing to one another. For example, with respect to DNA, adenosine is complementary to thymine and cytosine is complementary to guanine.

The terms "homology" and "homologous" are used interchangeably herein. They refer to nucleic acid fragments wherein changes in one or more nucleotide bases do not affect the ability of the nucleic acid fragment to mediate gene expression or produce a certain phenotype. These terms also refer to modifications of the nucleic acid fragments of the instant invention such as deletion or insertion of one or more nucleotides that do not substantially alter the functional properties of the resulting nucleic acid fragment relative to the initial, unmodified fragment. It is therefore understood, as those skilled in the art will appreciate, that the invention encompasses more than the specific exemplary sequences.

Moreover, the skilled artisan recognizes that homologous nucleic acid sequences encompassed by this invention are also defined by their ability to hybridize, under moderately stringent conditions (e.g., 0.5 X SSC, 0.1% SDS, 60 °C) with the sequences exemplified herein, or to any portion of the nucleotide sequences disclosed herein and which are functionally equivalent to any of the nucleic acid sequences disclosed herein.

"Codon degeneracy" refers to the nature in the genetic code permitting variation of the nucleotide sequence without effecting the amino acid sequence of an encoded polypeptide. The skilled artisan is well aware of the "codon-bias" exhibited by a specific host cell in usage of nucleotide codons to specify a given amino acid. Therefore, when synthesizing a gene for improved expression in a host cell, it is desirable to design the gene such that its frequency of codon usage approaches the frequency of preferred codon usage of the host cell.

The term "percent identity", as known in the art, is a relationship between two or more polypeptide sequences or two or more polynucleotide sequences, as determined by comparing the sequences. In the art, "identity" also means the degree of sequence relatedness between polypeptide or polynucleotide sequences, as the case may be, as determined by the match between strings of such sequences. "Identity" and "similarity" can be readily calculated by known methods, including but not limited to those described in: 1.) Computational Molecular Biology (Lesk, A. M., Ed.) Oxford University: NY (1988); 2.) Biocomputing: Informatics and Genome Projects (Smith, D. W., Ed.) Academic: NY (1993); 3.) Computer Analysis of Sequence Data, Part I (Griffin, A. M., and Griffin, H. G., Eds.) Humania: NJ (1994); 4.) Sequence Analysis in Molecular Biology (von Heinje, G., Ed.) Academic (1987); and 5.) Sequence Analysis Primer (Gribskov, M. and Devereux, J., Eds.) Stockton: NY (1991).

Preferred methods to determine identity are designed to give the best match between the sequences tested. Methods to determine identity and similarity are codified in publicly available computer programs. Sequence alignments and percent identity calculations may be performed using the MegAlign™ program of the LASERGENE bioinformatics computing suite (DNASTAR Inc., Madison, WI). Multiple alignment of the sequences is performed using the "Clustal method of alignment" which encompasses several varieties of the algorithm including the "Clustal V method of alignment" corresponding to the alignment method labeled Clustal V (described by Higgins and Sharp, CABIOS. 5:151-153 (1989); Higgins, D.G. et al., Comput. Appl. Biosci., 8:189-191 (1992)) and found in the MegAlign™ program of the LASERGENE bioinformatics computing suite (DNASTAR Inc.). For multiple alignments, the default values correspond to GAP PENALTY=10 and GAP LENGTH PENALTY=10. Default parameters for pairwise alignments and calculation of percent identity of protein sequences using the Clustal method are KTUPLE=1, GAP PENALTY=3, WINDOW=5 and DIAGONALS SAVED=5. For nucleic acids these parameters are KTUPLE=2, GAP PENALTY=5, WINDOW=4 and DIAGONALS SAVED=4. After alignment of the sequences using the Clustal V program, it is possible to obtain a "percent identity" by viewing the "sequence distances" table in the same program. Additionally the "Clustal W method of alignment" is available and corresponds to the alignment method labeled Clustal W (described by Higgins and Sharp, CABIOS. 5:151-153 (1989); Higgins, D.G. et al., Comput. Appl. Biosci. 8:189-191(1992)) and found in the MegAlign™ v6.1 program of the LASERGENE bioinformatics computing suite (DNASTAR Inc.). Default parameters for multiple alignment (GAP PENALTY=10, GAP LENGTH PENALTY=0.2, Delay Divergen Seqs(%)=30, DNA Transition Weight=0.5, Protein Weight Matrix=Gonnet Series, DNA Weight Matrix=IUB). After alignment of the sequences using the Clustal W program, it is possible to obtain a "percent identity" by viewing the "sequence distances" table in the same program.

It is well understood by one skilled in the art that many levels of sequence identity are useful in identifying polypeptides, from other species, wherein such polypeptides have the same or similar function or activity. Useful examples of percent identities include, but are not limited to: 24%, 30%, 35%, 40%, 45%, 50%, 55%, 60%, 65%, 70%, 75%, 80%, 85%, 90%, or 95%, or any integer percentage from 24% to 100% may be useful in describing the present invention, such as 25%, 26%, 27%, 28%, 29%, 30%, 31%, 32%, 33%, 34%, 35%, 36%, 37%, 38%, 39%, 40%, 41% 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99%. Suitable nucleic acid fragments not only have the above homologies but typically encode a polypeptide having at least 50 amino acids, preferably at least 100 amino acids, more preferably at least 150 amino acids, still more preferably at least 200 amino acids, and most preferably at least 250 amino acids. The term "sequence analysis software" refers to any computer algorithm or software program that is useful for the analysis of nucleotide or amino acid sequences. "Sequence analysis software" may be commercially available or independently developed. Typical sequence analysis software will include, but is not limited to: 1.) the GCG suite of programs (Wisconsin Package Version 9.0, Genetics Computer Group (GCG), Madison, WI); 2.) BLASTP, BLASTN, BLASTX (Altschul et al., J. Mol. Biol., 215:403-410 (1990)); 3.) DNASTAR (DNASTAR, Inc. Madison, WI); 4.) Sequencher (Gene Codes Corporation, Ann Arbor, MI); and 5.) the FASTA program incorporating the Smith-Waterman algorithm (W. R. Pearson, Comput. Methods Genome Res., [Proc. Int. Symp.] (1994), Meeting Date 1992, 111-20. Editor(s): Suhai, Sandor. Plenum: New York, NY). Within the context of this application it will be understood that where sequence analysis software is used for analysis, that the results of the analysis will be based on the "default values" of the program referenced, unless otherwise specified. As used herein "default values" will mean any set of values or parameters that originally load with the software when first initialized.

As used herein the term "coding sequence" refers to a DNA sequence that codes for a specific amino acid sequence. "Suitable regulatory sequences" refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences may include promoters, translation leader sequences, introns, polyadenylation recognition sequences, RNA processing site, effector binding site and stem-loop structure.

The term "promoter" refers to a DNA sequence capable of controlling the expression of a coding sequence or functional RNA. In general, a coding sequence is located 3' to a promoter sequence. Promoters may be derived in their entirety from a native gene, or be composed of different elements derived from different promoters found in nature, or even comprise synthetic DNA segments. It is understood by those skilled in the art that different promoters may direct the expression of a gene in different tissues or cell types, or at different stages of development, or in response to different environmental or physiological conditions. Promoters which cause a gene to be expressed in most cell types at most times are commonly referred to as "constitutive promoters". It is further recognized that since in most cases the exact boundaries of regulatory sequences have not been completely defined, DNA fragments of different lengths may have identical promoter activity.

The term "operably linked" refers to the association of nucleic acid sequences on a single nucleic acid fragment so that the function of one is affected by the other. For example, a promoter is operably linked with a coding sequence when it is capable of effecting the expression of that coding sequence (i.e., that the coding sequence is under the transcriptional control of the promoter). Coding sequences can be operably linked to regulatory sequences in sense or antisense orientation.

The term "expression", as used herein, refers to the transcription and stable accumulation of sense (mRNA) or antisense RNA derived from the nucleic acid fragment disclosed herein. Expression may also refer to translation of mRNA into a polypeptide.

As used herein the term "transformation" refers to the transfer of a nucleic acid fragment into a host organism, resulting in genetically stable inheritance. Host organisms containing the transformed nucleic acid fragments are referred to as "transgenic" or "recombinant" or "transformed" organisms.

The terms "plasmid" and "vector" refer to an extra chromosomal element often carrying genes which are not part of the central metabolism of the cell, and usually in the form of circular double-stranded DNA moledules. Such elements may be autonomously replicating sequences, genome integrating sequences, phage or nucleotide sequences, linear or circular, of a single- or double-stranded DNA or RNA, derived from any source, in which a number of nucleotide sequences have been joined or recombined into a unique construction which is capable of introducing a promoter fragment and DNA sequence for a selected gene product along with appropriate 3' untranslated sequence into a cell. "Transformation vector" refers to a specific vector containing a foreign gene and having elements in addition to the foreign gene that facilitates transformation of a particular host cell.

As used herein the term "codon degeneracy" refers to the nature in the genetic code permitting variation of the nucleotide sequence without effecting the amino acid sequence of an encoded polypeptide. The skilled artisan is well aware of the "codon-bias" exhibited by a specific host cell in usage of nucleotide codons to specify a given amino acid. Therefore, when synthesizing a gene for improved expression in a host cell, it is desirable to design the gene such that its frequency of codon usage approaches the frequency of preferred codon usage of the host cell.

The term "codon-optimized" as it refers to genes or coding regions of nucleic acid molecules for transformation of various hosts, refers to the alteration of codons in the gene or coding regions of the nucleic acid molecules to reflect the typical codon usage of the host organism without altering the polypeptide encoded by the DNA.

Standard recombinant DNA and molecular cloning techniques used herein are well known in the art and are described by Sambrook, J., Fritsch, E. F. and Maniatis, T., Molecular Cloning: A Laboratory Manual, Second Edition, Cold Spring Harbor Laboratory Press, Cold Spring Harbor, NY (1989) (hereinafter "Maniatis"); and by Silhavy, T. J., Bennan, M. L. and Enquist, L. W., Experiments with Gene Fusions, Cold Spring Harbor Laboratory Press, Cold Spring Harbor, NY (1984); and by Ausubel, F. M. et al., Current Protocols in Molecular Biology, published by Greene Publishing Assoc. and Wiley-Interscience (1987).

### Isobutanol Biosynthetic Pathways

Carbohydrate utilizing microorganisms employ the Embden-Meyerhof-Parnas (EMP) pathway, the Entner-Doudoroff pathway and the pentose phosphate cycle as the central, metabolic routes to provide energy and cellular precursors for growth and maintenance. These pathways have in common the intermediate glyceraldehyde-3-phosphate and, ultimately, pyruvate is formed directly or in combination with the EMP pathway. Subsequently, pyruvate is transformed to acetyl-coenzyme A (acetyl-CoA) via a variety of means. Acetyl-CoA serves as a key intermediate, for example, in generating fatty acids, amino acids and secondary metabolites. The combined reactions of sugar conversion to pyruvate produce energy (e.g. adenosine-5'-triphosphate, ATP) and reducing equivalents (e.g. reduced nicotinamide adenine dinucleotide, NADH, and reduced nicotinamide adenine dinucleotide phosphate, NADPH). NADH and NADPH must be recycled to their oxidized forms (NAD⁺ and NADP⁺, respectively). In the presence of inorganic electron acceptors (e.g. O₂, NO₃⁻ and SO4²⁻), the reducing equivalents may be used to augment the energy pool; alternatively, a reduced carbon by-product may be formed. As described by Donaldson et al., *supra,* isobutanol can be produced from carbohydrate sources by recombinant microorganisms comprising one of four complete isobutanol biosynthetic pathways, as shown in Figure 1.

Three of the pathways comprise conversion of pyruvate to isobutanol via a series of enzymatic steps. The preferred isobutanol pathway (Figure 1, steps a to e), comprises the following substrate to product conversions:
a) pyruvate to acetolactate, as catalyzed for example by acetolactate synthase,
b) acetolactate to 2,3-dihydroxyisovalerate, as catalyzed for example by acetohydroxy acid isomeroreductase,
c) 2,3-dihydroxyisovalerate to α-ketoisovalerate, as catalyzed for example by acetohydroxy acid dehydratase,
d) α-ketoisovalerate to isobutyraldehyde, as catalyzed for example by a branched-chain keto acid decarboxylase, and
e) isobutyraldehyde to isobutanol, as catalyzed for example by, a branched-chain alcohol dehydrogenase.

This pathway combines enzymes known to be involved in well-characterized pathways for valine biosynthesis (pyruvate to α-ketoisovalerate) and valine catabolism (α-ketoisovalerate to isobutanol). Since many valine biosynthetic enzymes also catalyze analogous reactions in the isoleucine biosynthetic pathway, substrate specificity is a major consideration in selecting the gene sources. For this reason, the primary genes of interest for the acetolactate synthase enzyme are those from *Bacillus* (*alsS*) and *Klebsiella (budB).* These particular acetolactate synthases are known to participate in butanediol fermentation in these organisms and show increased affinity for pyruvate over ketobutyrate (Gollop et al., J. Bacteriol. 172(6):3444-3449 (1990); Holtzclaw et al., J. Bacteriol. 121 (3):917-922 (1975)). The second and third pathway steps are catalyzed by acetohydroxy acid reductoisomerase and dehydratase, respectively. These enzymes have been characterized from a number of sources, such as for example, *E. coli* (Chunduru et al., Biochemistry 28(2):486-493 (1989); Flint et al., J. Biol. Chem. 268(29):14732-14742 (1993)). The final two steps of the preferred isobutanol pathway are known to occur in yeast, which can use valine as a nitrogen source and, in the process, secrete isobutanol. α-Ketoisovalerate can be converted to isobutyraldehyde by a number of keto acid decarboxylase enzymes, such as for example pyruvate decarboxylase. To prevent misdirection of pyruvate away from isobutanol production, a decarboxylase with decreased affinity for pyruvate is desired. So far, there are two such enzymes known in the art (Smit et al., Appl. Environ. Microbiol. 71(1):303-311 (2005); de la Plaza et al., FEMS Microbiol. Lett. 238(2):367-374 (2004)), Both enzymes are from strains of *Lactococcus lactis* and have a 50-200-fold preference for ketoisovalerate over pyruvate. Finally, a number of aldehyde reductases have been identified in yeast, many with overlapping substrate specificity. Those known to prefer branched-chain substrates over acetaldehyde include, but are not limited to, alcohol dehydrogenase VI (ADH6) and Ypr1p (Larroy et al., Biochem. J. 361(Pt 1):163-172 (2002); Ford et al., Yeast 19(12):1087-1096 (2002)), both of which use NADPH as electron donor. An NADPH-dependent reductase, YqhD, active with branched-chain substrates has also been recently identified in *E. coli* (Sulzenbacher et al., J. Mol. Biol. 342(2):489-502 (2004)).

### Pathway steps.

a) Pyruvate to acetolactate, is catalyzed by acetolactate synthase. The skilled person will appreciate that polypeptides having acetolactate synthase activity isolated from a variety of sources will be useful in the present invention independent of sequence homology. Example of suitable acetolactate synthase enzymes are available from a number of sources, for example, *Bacillus subtilis* (GenBank Nos: CAB15618 (SEQ ID NO:178), Z99122 (SEQ ID NO:78), NCBI (National Center for Biotechnology Information) amino acid sequence, NCBI nucleotide sequence, respectively), *Klebsiella pneumoniae* (GenBank Nos: AAA25079 (SEQ ID NO:2), M73842 (SEQ ID NO:1)), and *Lactococcus lactis* ((GenBank Nos: AAA25161 (SEQ ID NO:180), L16975 (SEQ ID NO:179)). Preferred acetolactate synthase enzymes are those that have at least 80% ― 85% identity to SEQ ID NO:2, SEQ ID NO:178, and SEQ ID: 180 where at least 85% - 90% identity is more preferred and where at least 95% identity based on the Clustal W method of alignment using the default parameters of GAP PENALTY=10, GAP LENGTH PENALTY=0.1, and Gonnet 250 series of protein weight matrix, is most preferred.
b) Acetolactate to 2,3-dihydroxyisovalerate, is catalyzed by acetohydroxy acid isomeroreductase. The skilled person will appreciate that polypeptides having acetohydroxy acid isomeroreductase activity isolated from a variety of sources will be useful in the present invention independent of sequence homology. Example of suitable acetohydroxy acid isomeroreductase enzymes are available from a number of sources, for example, *Escherichia coli* (GenBank Nos: NP_418222 (SEQ ID NO:4), NC_000913 (SEQ ID NO:3)), *Saccharomyces cerevisiae* (GenBank Nos: NP_013459 (SEQ ID NO:181), NC_001144 (SEQ ID NO:80)), *Methanococcus maripaludis* (GenBank Nos: CAF30210 (SEQ ID NO:183), BX957220 (SEQ ID NO:182)), and *Bacillus. subtilis* (GenBank Nos: CAB14789 (SEQ ID NO:185), Z99118 (SEQ ID NO:184)). Preferred acetohydroxy acid isomeroreductase enzymes are those that have at least 80% ― 85% identity to SEQ ID NO:43, SEQ ID NO:181, SEQ ID NO:183, and SEQ ID NO:185, where at least 85% - 90% identity is more preferred and where at least 95% identity based on the Clustal W method of alignment using the default parameters of GAP PENALTY=10, GAP LENGTH PENALTY=0.1, and Gonnet 250 series of protein weight matrix, is most preferred.
c) 2,3-dihydroxyisovalerate to α-ketoisovalerate, as catalyzed for example by acetohydroxy acid dehydratase. The skilled person will appreciate that polypeptides having acetohydroxy acid dehydratase activity isolated from a variety of sources will be useful in the present invention independent of sequence homology. Example of suitable acetohydroxy acid dehydratase enzymes are available from a number of sources, for example, *E. coli* (GenBank Nos: YP_026248 (SEQ ID NO:6), NC_000913 (SEQ ID NO:5)), *S. cerevisiae* (GenBank Nos: NP_012550 (SEQ ID NO:186), NC_001142 (SEQ ID NO:83)), *M. maripaludis* (GenBank Nos: CAF29874 (SEQ ID NO:188), BX957219 (SEQ ID NO:187)), and *B. subtilis* (GenBank Nos: CAB14105 (SEQ ID NO:190), Z99115 (SEQ ID NO:189)). Preferred acetohydroxy acid dehydratase enzymes are those that have at least 80% ― 85% identity to SEQ ID NO:6, SEQ ID NO:186, SEQ ID NO:188, and SEQ ID NO:190, where at least 85% - 90% identity is more preferred and where at least 95% identity based on the Clustal W method of alignment using the default parameters of GAP PENALTY=10, GAP LENGTH PENALTY=0.1, and Gonnet 250 series of protein weight matrix, is most preferred.
d) α-ketoisovalerate to isobutyraldehyde, as catalyzed for example by a branched-chain keto acid decarboxylase. The skilled person will appreciate that polypeptides having branched-chain keto acid decarboxylase activity isolated from a variety of sources will be useful in the present invention independent of sequence homology. Example of suitable branched-chain keto acid decarboxylase enzymes are available from a number of sources, for example, *Lactococcus lactis* (GenBank Nos: AAS49166 (SEQ ID NO:193), AY548760 (SEQ ID NO:192); CAG34226 (SEQ ID NO:8), AJ746364 (SEQ ID NO:191), *Salmonella typhimurium* (GenBank Nos: NP_461346 (SEQ ID NO:195), NC_003197 (SEQ ID NO:194)), and *Clostridium acetobutylicum* (GenBank Nos: NP_149189 (SEQ ID NO:197), NC_001988 (SEQ ID NO:196)). Preferred branched-chain keto acid decarboxylase enzymes are those that have at least 80% ― 85% identity to SEQ ID NO:8, SEQ ID NO:193, SEQ ID NO:195, and SEQ ID NO:197, where at least 85% - 90% identity is more preferred and where at least 95% identity based on the Clustal W method of alignment using the default parameters of GAP PENALTY=10, GAP LENGTH PENALTY=0.1, and Gonnet 250 series of protein weight matrix, is most preferred.
e) Isobutyraldehyde to isobutanol, as catalyzed for example by, a branched-chain alcohol dehydrogenase. The skilled person will appreciate that polypeptides having branched-chain alcohol dehydrogenase activity isolated from a variety of sources will be useful in the present invention independent of sequence homology. Example of suitable branched-chain alcohol dehydrogenase enzymes are available from a number of sources, for example, *S. cerevisiae* (GenBank Nos: NP_010656 (SEQ ID NO:199), NC_001136 (SEQ ID NO:198); NP_014051 (SEQ ID NO:201) NC_001145 (SEQ ID NO:200)), *E. coli* (GenBank Nos: NP_417484 (SEQ ID NO:10), NC_000913 (SEQ ID NO:9)), and C. *acetobutylicum* (GenBank Nos: NP_349892 (SEQ ID NO:203), NC_003030 (SEQ ID NO:202); NP_349891 (SEQ ID NO:204), NC_003030 (SEQ ID NO:158)). Preferred branched-chain alcohol dehydrogenase enzymes are those that have at least 80% ― 85% identity to SEQ ID NO:10, SEQ ID NO:199, SEQ ID NO:201, SEQ ID NO:203, and SEQ ID NO:204.where at least 85% - 90% identity is more preferred and where at least 95% identity based on the Clustal W method of alignment using the default parameters of GAP PENALTY=10, GAP LENGTH PENALTY=0.1, and Gonnet 250 series of protein weight matrix, is most preferred.

Additional pathways are discussed below and in commonly owned and copending US 11/586315, incorporated herein by reference.

Another pathway for converting pyruvate to isobutanol comprises the following substrate to product conversions (Figure 1, steps a,b,c,f,g,e):
a) pyruvate to acetolactate, as catalyzed for example by acetolactate synthase,
b) acetolactate to 2,3-dihydroxyisovalerate, as catalyzed for example by acetohydroxy acid isomeroreductase,
c) 2,3-dihydroxyisovalerate to α-ketoisovalerate, as catalyzed for example by acetohydroxy acid dehydratase,
f) α-ketoisovalerate to isobutyryl-CoA, as catalyzed for example by a branched-chain keto acid dehydrogenase,
g) isobutyryl-CoA to isobutyraldehyde, as catalyzed for example by an acylating aldehyde dehydrogenase, and
e) isobutyraldehyde to isobutanol, as catalyzed for example by, a branched-chain alcohol dehydrogenase.

The first three steps in this pathway (a,b,c) are the same as those described above. The α-ketoisovalerate is converted to isobutyryl-CoA by the action of a branched-chain keto acid dehydrogenase. While yeast can only use valine as a nitrogen source, many other organisms (both eukaryotes and prokaryotes) can use valine as the carbon source as well. These organisms have branched-chain keto acid dehydrogenase (Sokatch et al. *J. Bacteriol.* 148(2):647-652 (1981)), which generates isobutyryl-CoA. Isobutyryl-CoA may be converted to isobutyraldehyde by an acylating aldehyde dehydrogenase. Dehydrogenases active with the branched-chain substrate have been described, but not cloned, in *Leuconostoc* and *Propionibacterium* (Kazahaya et al., J. Gen. Appl. Microbiol. 18:43-55 (1972); Hosoi et al., J. Ferment. Technol. 57:418-427 (1979)). However, it is also possible that acylating aldehyde dehydrogenases known to function with straight-chain acyl-CoAs (i.e. butyryl-CoA), may also work with isobutyryl-CoA. The isobutyraldehyde is then converted to isobutanol by a branched-chain alcohol dehydrogenase, as described above for the first pathway.

Another pathway for converting pyruvate to isobutanol comprises the following substrate to product conversions (Figure 1, steps a,b,c,h,i,j,e):
a) pyruvate to acetolactate, as catalyzed for example by acetolactate synthase,
b) acetolactate to 2,3-dihydroxyisovalerate, as catalyzed for example by acetohydroxy acid isomeroreductase,
c) 2,3-dihydroxyisovalerate to α-ketoisovalerate, as catalyzed for example by acetohydroxy acid dehydratase,
h) α-ketoisovalerate to valine, as catalyzed for example by valine dehydrogenase or transaminase,
i) valine to isobutylamine, as catalyzed for example by valine decarboxylase,
j) isobutylamine to isobutyraldehyde, as catalyzed for example by omega transaminase, and
e) isobutyraldehyde to isobutanol, as catalyzed for example by, a branched-chain alcohol dehydrogenase.

The first three steps in this pathway (a,b,c) are the same as those described above. This pathway requires the addition of a valine dehydrogenase or a suitable transaminase. Valine (and or leucine) dehydrogenase catalyzes reductive amination and uses ammonia; Kₘ values for ammonia are in the millimolar range (Priestly et al., Biochem J. 261(3):853-861 (1989); Vancura et al., J. Gen. Microbiol. 134(12):3213-3219 (1988) Zink et al., Arch. Biochem. Biophys. 99:72-77 (1962); Sekimoto et al. J. Biochem (Japan) 116(1):176-182 (1994)). Transaminases typically use either glutamate or alanine as amino donors and have been characterized from a number of organisms (Lee-Peng et al,. J. Bacteriol. 139(2):339-345 (1979); Berg et al., J. Bacteriol. 155(3):1009-1014 (1983)). An alanine-specific enzyme may be desirable, since the generation of pyruvate from this step could be coupled to the consumption of pyruvate later in the pathway when the amine group is removed (see below). The next step is decarboxylation of valine, a reaction that occurs in valanimycin biosynthesis in *Streptomyces* (Garg et al., Mol. Microbiol. 46(2):505-517 (2002)). The resulting isobutylamine may be converted to isobutyraldehyde in a pyridoxal 5'-phosphate-dependent reaction by, for example, an enzyme of the omega-aminotransferase family. Such an enzyme from *Vibrio fluvialis* has demonstrated activity with isobutylamine (Shin et al., Biotechnol. Bioeng. 65(2):206-211 (1999)). Another omega-aminotransferase from *Alcaligenes denitrificans* has been cloned and has some activity with butylamine (Yun et al., Appl. Environ. Microbiol. 70(4):2529-2534 (2004)). In this direction, these enzymes use pyruvate as the amino acceptor, yielding alanine. As mentioned above, adverse affects on the pyruvate pool may be offset by using a pyruvate-producing transaminase earlier in the pathway. The isobutyraldehyde is then converted to isobutanol by a branched-chain alcohol dehydrogenase, as described above for the first pathway.

The fourth isobutanol biosynthetic pathway comprises the substrate to product conversions shown as steps k,g,e in Figure 1. A number of organisms are known to produce butyrate and/or butanol via a butyryl-CoA intermediate (Dürre et al., FEMS Microbiol. Rev. 17(3):251-262 (1995); Abbad-Andaloussi et al., Microbiology 142(5):1149-1158 (1996)). Isobutanol production may be engineered in these organisms by addition of a mutase able to convert butyryl-CoA to isobutyryl-CoA (Figure 1, step k). Genes for both subunits of isobutyryl-CoA mutase, a coenzyme B₁₂-dependent enzyme, have been cloned from a Streptomycete (Ratnatilleke et al., J. Biol. Chem. 274(44):31679-31685 (1999)). The isobutyryl-CoA is converted to isobutyraldehyde (step g in Figure 1), which is converted to isobutanol (step e in Figure 1).

Thus, in providing multiple recombinant pathways from pyruvate to isobutanol, there exist a number of choices to fulfill the individual conversion steps, and the person of skill in the art will be able to utilize publicly available sequences to construct the relevant pathways. A listing of a representative number of genes known in the art and useful in the construction of isobutanol biosynthetic pathways are listed below in Table 2.

**Table 2**

| Sources of Isobuatnol Biosynthetic Pathway Genes | |
|---|---|
| Gene | GenBank Citation |
| acetolactate synthase | Z99122, *Bacillus subtilis* complete genome (section 19 of 21): from 3608981 to 3809670 gi\|32468830\|emb\|Z99122.2\|BSUB0019[32468830] |
| | M73842, *Klebsiella pneumoniae* acetolactate synthase *(iluk)* gene, complete cds gi\|149210\|gb\|M73842.\|KPNILUK[149210] |
| | L16975, *Lactococcus lactis* alpha-acetolactate synthase (als) gene, complete cds gi\|473900\|gb\|L16975.1\|LACALS[473900] |
| acetohydroxy acid isomeroreductase | NC_000913, *Escherichia coli* K12, complete genome gi\|49175990\|ref\|NC_000913.2\|[49175990] |
| | NC__001144, *Saccharomyces cerevisiae* chromosome XII, complete chromosome sequence gi\|42742286\|ref\|NC_001144.3\|[42742286] |
| | BX957220, *Methanococcus maripaludis* S2 complete genome; segment 2/5 gi\|44920669\|emb\|BX957220.1\|[44920669] |
| | Z99118, *Bacillus subtilis* complete genome (section 15 of 21): from 2812801 to 3013507 gi\|32468802\|emb\|Z99118.2\|BSUB0015[32468802] |
| acetohydroxy acid dehydratase | NC_000913, *Escherichia coli* K12, complete genome gi\|49175990\|ref\|NC_000913.2\|[49175990] |
| | NC_001142, *Saccharomyces cerevisiae* chromosome X, complete chromosome sequence gi\|42742252\|ref\|NC_001142.5\|[42742252] |
| | BX957219, *Methanococcus maripaludis* S2 complete genome; segment 1/5 |
| | gi\|45047123\|emb\|BX957219.1\|[45047123] |
| | Z99115, *Bacillus subtilis* complete genome (section 12 of 21): from 2207806 to 2409180 gi\|32468778\|emb\|Z99115.2\|ZSUB0012[32468778] |
| branched-chain α-keto acid decarboxylase | AY548760, *Lactococcus lactis* branched-chain alpha-ketoacid decarboxylase (*kdcA*) gene, complete cds gi\|44921616\|gb\|AY548760.1\|[44921616] |
| | AJ746364, Lactococcus *lactis* subsp. *lactis kivd* gene for alpha-ketoisovalerate decarboxylase, strain IFPL730 gi\|51870501\|emb\|AJ746364.1\|[51870501] |
| | NC_003197, *Salmonella typhimurium* LT2, complete genome gi\|16763390\|ref\|NC_003197.1\|[16763390] |
| | NC_001988, *Clostridium* acetobutylicum ATCC 824 plasmid pSOL1, complete sequence gi\|15004705\|ref\|NC_001988.2\|[15004705] |
| branched-chain alcohol dehydrogenase | NC_001136, *Saccharomyces cerevisiae* chromosome IV, complete chromosome sequence gi\|50593138\|ref\|NC_001136.6\|[50593138] |
| | NC_001145, *Saccharomyces cerevisiae* chromosome XIII, complete chromosome sequence gi\|44829554\|ref\|NC_001145.2\|[44829554] |
| | NC_000913, *Escherichia coli K12,* complete genome gi\|49175990\|ref\|NC_000913.2\|[49175990] |
| | NC_{_}003030, *Clostridium acetobutylicum* ATCC 824, complete genome gi\|15893298\|ref\|NC_003030.1\|[15893298] |
| branched-chain keto acid dehydrogenase | Z99116, *Bacillus subtilis* complete genome (section 13 of 21): from 2409151 to 2613687 gi\|32468787\|emb\|Z99116.2\|BSUB0013[32468787] |
| | M57613, *Pseudomonas putida* branched-chain keto acid dehydrogenase operon (*bkdA1, bkdA1* and *bkdA2*), transacylase E2 (*bkdB*)*, bkdR* and lipoamide dehydrogenase (*IpdV*) genes, complete cds gi\|790512\|gb\|M57613.1\|PSEBKDPPG2[790512] |
| acylating aldehyde | AF157306, *Clostridium beijerinckii* strain NRRL B593 |
| dehydrogenase | hypothetical protein, coenzyme A acylating aldehyde dehydrogenase *(ald)*, acetoacetate:butyrate/acetate coenzyme A transferase (*ctfA*)*,* acetoacetate:butyrate/acetate coenzyme A transferase (*ctfB*)*,* and acetoacetate decarboxylase *(adc)* genes, complete cds gi\|47422980\|gb\|AF157306.2\|[47422980] |
| | NC_001988, *Clostridium acetobutylicum* ATCC 824 plasmid pSOL1, complete sequence gi\|15004705\|ref\|NC_001988.2\|[15004705] |
| | U13232, *Pseudomonas putida* NC\|B9816 acetaldehyde dehydrogenase (*nahO*) and 4-hydroxy-2-oxovalerate aldolase (*nahM*) genes, complete cds, and 4-oxalocrotonate decarboxylase (*nahK*) and 2-oxopent-4-enoate hydratase (*nahL*) genes, partial cds gi\|595671\|gb\|U13232.1\|PPU13232[595671] |
| transaminase | NC_000913, *Escherichia coli* K12, complete genome gi\|49175990\|ref\|NC_000913.2\|[49175990] |
| | NC_006322, *Bacillus licheniformis* ATCC 14580, complete genome g\|52783855\|ref\|NC_006322.1\|[52783855] |
| | NC_001142, *Saccharomyces cerevisiae* chromosome X, complete chromosome sequence gi\|42742252\|ref\|NC_001142.5\|[42742252] |
| | NC_000916, *Methanothermobacter thermautotrophicus* str. Delta H, complete genome gi\|15678031\|ref\|NC_000916.1\|[15678031] |
| valine dehydrogenase | NC_003888, *Streptomyces coeicolor* A3(2), complete genome gi\|32141095\|ref\|NC_003888.3\|[32141095] |
| | Z99116, *Bacillus subtilis* complete genome (section 13 of 21): from 2409151 to 2613687 gi\|32468787\|emb\|Z99116.2\|BSUB0013[32468787] |
| valine decarboxylase | AY116644, *Streptomyces viridifaciens* amino acid aminotransferase gene, partial cds; ketol-acid reductoisomerase, acetolactate synthetase small |
| | subunit, acetolactate synthetase large subunit, complete cds; azoxy antibiotic valanimycin gene cluster, complete sequence; and putative transferase, and putative secreted protein genes, complete cds gi\|27777548\|gb\|AY116644.1\|[27777548] |
| omega transaminase | AY330220, *Achromobacter denitrificans* omega-amino acid:pyruvate transaminase (*aptA*) gene, complete cds gi\|33086797\|gb\|AY330220.1\|[33086797] |
| | NC_007347, *Ralstonia eutropha* JMP134 chromosome 1, complete sequence gi\|73539706\|ref\|NC_007347.1\|[73539706] |
| | NC_004347, *Shewanella oneidensis* MR-1, complete genome gi\|24371600\|ref\|NC_004347.1\|[24371600] |
| | NZ_AAAG02000002, *Rhodospirillum rubrum* Rrub02_2, whole genome shotgun sequence gi\|48764549\|ref\|NZ_AAAG02000002.1\|[48764549] |
| | AE016776, *Pseudomonas putida* KT2440 section 3 of 21 of the complete genome gi\|26557019\|gb\|AE016776.1\|[26557019] |
| isobutyryl-CoA mutase | U67612, *Streptomyces cinnamonensis* coenzyme B12-dependent isobutyrylCoA mutase *(icm)* gene, complete cds gi\|3002491\|gb\|U67612.1\|SCU67612[3002491] |
| | AJ246005, *Streptomyces cinnamonensis icmB* gene for isobutyryl-CoA mutase, small subunit gi\|6137076\|emb\|AJ246005.1\|SCl246005[6137076] |
| | AL939123, *Streptomyces coelicolor* A3(2) complete genome; segment 20/29 gi\|24430032\|emb\|AL939123.1\|SC0939123[24430032] |
| | AL9939121, *Streptomyces coelicolor* A3(2) complete genome; segment 18/29 gi\|24429533\|emb\|AL939121.1\|SCO939121[24429533] |
| | NC_003155, *Streptomyces avermitilis* MA-4680, complete genome gi\|57833846\|ref\|NC_003155.3\|[57833846] |

### Microbial Hosts for Isobutanol Production

Microbial hosts for isobutanol production may be selected from bacteria, cyanobacteria, filamentous fungi and yeasts. The microbial host used for isobutanol production is preferably tolerant to isobutanol so that the yield is not limited by isobutanol toxicity. Microbes that are metabolically active at high titer levels of isobutanol are not well known in the art. Although 1-butanol-tolerant mutants have been isolated from solventogenic *Clostridia,* little information is available concerning the butanol tolerance of other potentially useful bacterial strains. Most of the studies on the comparison of alcohol tolerance in bacteria suggest that 1-butanol is more toxic than ethanol (de Cavalho et al., Microsc. Res. Tech. 64:215-22 (2004) and Kabelitz et al., FEMS Microbiol. Lett. 220:223-227 (2003)). Tomas et al. (J. Bacteriol. 186:2006-2018 (2004)) report that the yield of 1-butanol during fermentation in *Clostridium acetobutylicum* may be limited by 1-butanol toxicity. The primary effect of 1-butanol on *Clostridium acetobutylicum* is disruption of membrane functions (Hermann et al., Appl. Environ. Microbiol. 50:1238-1243 (1985)).

The microbial hosts selected for the production of isobutanol are preferably tolerant to isobutanol and should be able to convert carbohydrates to isobutanol. The criteria for selection of suitable microbial hosts include the following: intrinsic tolerance to isobutanol, high rate of glucose utilization, availability of genetic tools for gene manipulation, and the ability to generate stable chromosomal alterations.

Suitable host strains with a tolerance for isobutanol may be identified by screening based on the intrinsic tolerance of the strain. The intrinsic tolerance of microbes to isobutanol may be measured by determining the concentration of isobutanol that is responsible for 50% inhibition of the growth rate (IC50) when grown in a minimal medium. The IC50 values may be determined using methods known in the art. For example, the microbes of interest may be grown in the presence of various amounts of isobutanol and the growth rate monitored by measuring the optical density at 600 nanometers. The doubling time may be calculated from the logarithmic part of the growth curve and used as a measure of the growth rate. The concentration of isobutanol that produces 50% inhibition of growth may be determined from a graph of the percent inhibition of growth versus the isobutanol concentration. Preferably, the host strain should have an IC50 for isobutanol of greater than about 0.5%.

The microbial host for isobutanol production should also utilize glucose at a high rate. Most microbes are capable of utilizing carbohydrates. However, certain environmental microbes cannot utilize carbohydrates to high efficiency, and therefore would not be suitable hosts.

The ability to genetically modify the host is essential for the production of any recombinant microorganism. The mode of gene transfer technology may be by electroporation, conjugation, transduction or natural transformation. A broad range of host conjugative plasmids and drug resistance markers are available. The cloning vectors are tailored to the host organisms based on the nature of antibiotic resistance markers that can function in that host.

The microbial host also has to be manipulated in order to inactivate competing pathways for carbon flow by deleting various genes. This requires the availability of either transposons to direct inactivation or chromosomal integration vectors. Additionally, the production host should be amenable to chemical mutagenesis so that mutations to improve intrinsic isobutanol tolerance may be obtained.

Based on the criteria described above, suitable microbial hosts for the production of isobutanol include, but are not limited to, members of the genera *Clostridium, Zymomonas, Escherichia, Salmonella, Rhodococcus, Pseudomonas, Bacillus, Lactobacillus, Enterococcus, Alcaligenes, Klebsiella, Paenibacillus, Arthrobacter, Corynebacterium, Brevibacterium, Pichia, Candida, Hansenula* and *Saccharomyces.* Preferred hosts include: *Escherichia coli, Alcaligenes eutrophus, Bacillus licheniformis, Paenibacillus macerans, Rhodococcus erythropolis, Pseudomonas putida, Lactobacillus plantarum, Enterococcus faecium, Enterococcus gallinarium, Enterococcus faecalis, Bacillus subtilis* and *Saccharomyces cerevisiae.*

### Construction of Production Host

Recombinant organisms containing the necessary genes that will encode the enzymatic pathway for the conversion of a fermentable carbon substrate to isobutanol may be constructed using techniques well known in the art. Genes encoding the enzymes of one of the isobutanol biosynthetic pathways of the invention, for example, acetolactate synthase, acetohydroxy acid isomeroreductase, acetohydroxy acid dehydratase, branched-chain α-keto acid decarboxylase, and branched-chain alcohol dehydrogenase, may be isolated from various sources, as described above.

Methods of obtaining desired genes from a bacterial genome are common and well known in the art of molecular biology. For example, if the sequence of the gene is known, suitable genomic libraries may be created by restriction endonuclease digestion and may be screened with probes complementary to the desired gene sequence. Once the sequence is isolated, the DNA may be amplified using standard primer-directed amplification methods such as polymerase chain reaction (U.S. 4,683,202) to obtain amounts of DNA suitable for transformation using appropriate vectors. Tools for codon optimization for expression in a heterologous host are readily available. Some tools for codon optimization are available based on the GC content of the host organism. The GC content of some exemplary microbial hosts is given Table 3.

**Table 3**

| GC Content of Microbial Hosts | |
|---|---|
| Strain | % GC |
| *B. licheniformis* | 46 |
| *B. subtilis* | 42 |
| C. acetobutylicum | 37 |
| *E. coli* | 50 |
| *P. putida* | 61 |
| *A. eutrophus* | 61 |
| *Paenibacillus macerans* | 51 |
| *Rhodococcus erythropolis* | 62 |
| *Brevibacillus* | 50 |
| *Paenibacillus polymyxa* | 50 |

Once the relevant pathway genes are identified and isolated they may be transformed into suitable expression hosts by means well known in the art. Vectors or cassettes useful for the transformation of a variety of host cells are common and commercially available from companies such as EPICENTRE^{®} (Madison, WI), lnvitrogen Corp. (Carlsbad, CA), Stratagene (La Jolla, CA), and New England Biolabs, Inc. (Beverly, MA). Typically the vector or cassette contains sequences directing transcription and translation of the relevant gene, a selectable marker, and sequences allowing autonomous replication or chromosomal integration. Suitable vectors comprise a region 5' of the gene which harbors transcriptional initiation controls and a region 3' of the DNA fragment which controls transcriptional termination. Both control regions may be derived from genes homologous to the transformed host cell, although it is to be understood that such control regions may also be derived from genes that are not native to the specific species chosen as a production host.

Initiation control regions or promoters, which are useful to drive expression of the relevant pathway coding regions in the desired host cell are numerous and familiar to those skilled in the art. Virtually any promoter capable of driving these genetic elements is suitable for the present invention including, but not limited to, *CYC1, HIS3, GAL1, GAL 10, ADH1, PGK, PHO5, GAPDH, ADC1, TRP1, URA3, LEU2, ENO, TPI, CUP1, FBA, GPD, and GPM* (useful for expression in *Saccharomyces); AOX1* (useful for expression in *Pichia);* and *lac, ara, tet, trp, IP_{L}, IP_{R}, T7, tac, and trc* (useful for expression in *Escherichia coli, Alcaligenes,* and *Pseudomonas);* the *amy, apr, npr* promoters and various phage promoters useful for expression in *Bacillus subtilis, Bacillus licheniformis, and Paenibacillus macerans; nisA* (useful for expression Gram-positive bacteria, Eichenbaum et al. Appl. Environ. Microbiol. 64(8):2763-2769 (1998)); and the synthetic P11 promoter (useful for expression in *Lactobacillus plantarum,* Rud et al., Microbiology 152:1011-1019 (2006)).

Termination control regions may also be derived from various genes native to the preferred hosts. Optionally, a termination site may be unnecessary, however, it is most preferred if included.

Certain vectors are capable of replicating in a broad range of host bacteria and can be transferred by conjugation. The complete and annotated sequence of pRK404 and three related vectors-pRK437, pRK442, and pRK442(H) are available. These derivatives have proven to be valuable tools for genetic manipulation in Gram-negative bacteria (Scott et al., Plasmid 50(1):74-79 (2003)). Several plasmid derivatives of broad-host-range Inc P4 plasmid RSF1010 are also available with promoters that can function in a range of Gram-negative bacteria. Plasmid pAYC36 and pAYC37, have active promoters along with multiple cloning sites to allow for the heterologous gene expression in Gram-negative bacteria.

Chromosomal gene replacement tools are also widely available. For example, a thermosensitive variant of the broad-host-range replicon pWV101 has been modified to construct a plasmid pVE6002 which can be used to effect gene replacement in a range of Gram-positive bacteria (Maguin et al., J. Bacteriol. 174(17):5633-5638 (1992)). Additionally, in vitro transposomes are available to create random mutations in a variety of genomes from commercial sources such as EPICENTRE^{®}.

The expression of an isobutanol biosynthetic pathway in various preferred microbial hosts is described in more detail below.

### Expression of an isobutanol biosynthetic pathway in E. coli

Vectors or cassettes useful for the transformation of *E*. *coli* are common and commercially available from the companies listed above. For example, the genes of an isobutanol biosynthetic pathway may be isolated from various sources, cloned into a modified pUC19 vector and transformed into *E. coli* NM522, as described in Examples 10 and 11.

### Expression of an isobutanol biosynthetic pathway in Rhodococcus erythropalis

A series of *E. coli-Rhodococcus* shuttle vectors are available for expression in *R. erythropolis,* including, but not limited to, pRhBR17 and pDA71 (Kostichka et al., Appl. Microbiol. Biotechnol. 62:61-68(2003)). Additionally, a series of promoters are available for heterologous gene expression in *R. erythropolis* (see for example Nakashima et al., Appl. Environ. Microbiol. 70:5557-5568 (2004), and Tao et al., Appl. Microbiol. Biotechnol. 2005*,* DOI 10.1007/s00253-005-0064). Targeted gene disruption of chromosomal genes in *R. erythropolis* may be created using the method described by Tao et al., *supra,* and Brans et al. (Appl. Environ. Microbiol. 66: 2029-2036 (2000)).

The heterologous genes required for the production of isobutanol, as described above, may be cloned initially in pDA71 or pRhBR71 and transformed into *E*. *coli.* The vectors may then be transformed into R. *erythropolis* by electroporation, as described by Kostichka et al., *supra.* The recombinants may be grown in synthetic medium containing glucose and the production of isobutanol can be followed using methods known in the art.

### Expression of an isobutanol biosynthetic pathway in B. Subtilis

Methods for gene expression and creation of mutations in *B. subtilis* are also well known in the art. For example, the genes of an isobutanol biosynthetic pathway may be isolated from various sources, cloned into a modified pUC19 vector and transformed into *Bacillus subtilis* BE1010, as described in Example 12. Additionally, the five genes of an isobutanol biosynthetic pathway can be split into two operons for expression, as described in Example 24. The three genes of the pathway (*bubB, ilvD,* and *kivD*) were integrated into the chromosome of *Bacillus subtilis* BE1010 (Payne and Jackson, J. Bacteriol. 173:2278-2282 (1991)). The remaining two genes (*ilvC* and *bdhB*) were cloned into an expression vector and transformed into the *Bacillus* strain carrying the integrated isobutanol genes

### Expression of an isobutanol biosynthetic pathway in B. licheniformis

Most of the plasmids and shuttle vectors that replicate in *B. subtilis* may be used to transform *B. licheniformis* by either protoplast transformation or electroporation. The genes required for the production of isobutanol may be cloned in plasmids pBE20 or pBE60 derivatives (Nagarajan et al., Gene 114:121-126 (1992)). Methods to transform *B. licheniformis* are known in the art (for example see Fleming et al. Appl. Environ. Microbiol., 61(11):3775-3780 (1995)). The plasmids constructed for expression in *B. subtilis* may be transformed into *B. licheniformis* to produce a recombinant microbial host that produces isobutanol.

### Expression of an isobutanol biosynthetic pathway in Paenibacillus macerans

Plasmids may be constructed as described above for expression in *B. subtilis* and used to transform *Paenibacillus macerans* by protoplast transformation to produce a recombinant microbial host that produces isobutanol.

### Expression of the isobutanol biosynthetic pathways in Alcaligenes (Ralstonia) eutrophus

Methods for gene expression and creation of mutations in *Alcaligenes eutrophus* are known in the art (see for example Taghavi et al., Appl. Environ. Microbiol., 60(10):3585-3591 (1994)). The genes for an isobutanol biosynthetic pathway may be cloned in any of the broad host range vectors described above, and electroporated to generate recombinants that produce isobutanol. The poly(hydroxybutyrate) pathway in *Alcaligenes* has been described in detail, a variety of genetic techniques to modify the *Alcaligenes eutrophus* genome is known, and those tools can be applied for engineering an isobutanol biosynthetic pathway.

### Expression of an isobutanol biosynthetic pathway in Pseudomonas putida

Methods for gene expression in *Pseudomonas putida* are known in the art (see for example Ben-Bassat et al., U.S. Patent No. 6,586,229, which is incorporated herein by reference). The isobutanol pathway genes may be inserted into pPCU18 and this ligated DNA may be electroporated into electrocompetent *Pseudomonas putida* DOT-T1 C5aAR1 cells to generate recombinants that produce isobutanol.

### Expression of an isobutanol biosynthetic pathway in Saccharomyces cerevisiae

Methods for gene expression in *Saccharomyces cerevisiae* are known in the art (see for example Methods in Enzymology, Volume 194, Guide to Yeast Genetics and Molecular and Cell Biology (Part A, 2004, Christine Guthrie and Gerald R. Fink (Eds.), Elsevier Academic Press, San Diego, CA). Expression of genes in yeast typically requires a promoter, followed by the gene of interest, and a transcriptional terminator. A number of yeast promoters can be used in constructing expression cassettes for genes encoding an isobutanol biosynthetic pathway, including, but not limited to constitutive promoters FBA, GPD, ADH1, and GPM, and the inducible promoters GAL1, GAL10, and CUP1. Suitable transcriptional terminators include, but are not limited to FBAt, GPDt, GPMt, ERG10t, GAL1t, CYC1, and ADH1. For example, suitable promoters, transcriptional terminators, and the genes of an isobutanol biosynthetic pathway may be cloned into *E.* coli-yeast shuttle vectors as described in Example 21.

### Expression of an isobutanol biosynthetic pathway in Lactobacillus plantarum

The *Lactobacillus* genus belongs to the *Lactobacillales* family and many plasmids and vectors used in the transformation of *Bacillus subtilis* and *Streptococcus* may be used for *lactobacillus.* Non-limiting examples of suitable vectors include pAMβ1 and derivatives thereof (Renault et al., Gene 183:175-182 (1996); and O'Sullivan et al., Gene 137:227-231 (1993)); pMBB1 and pHW800, a derivative of pMBB1 (Wyckoff et al. Appl. Environ. Microbiol. 62:1481-1486 (1996)); pMG1, a conjugative plasmid (Tanimoto et al., J. Bacteriol. 184:5800-5804 (2002)); pNZ9520 (Kleerebezem et al., Appl. Environ. Microbiol. 63:4581-4584 (1997)); pAM401 (Fujimoto et al., Appl. Environ. Microbiol. 67:1262-1267 (2001)); and pAT392 (Arthur et al., Antimicrob. Agents Chemother. 38:1899-1903 (1994)). Several plasmids from *Lactobacillus plantarum* have also been reported (e.g., van Kranenburg R, Golic N, Bongers R, Leer RJ, de Vos WM, Siezen RJ, Kleerebezem M. Appl. Environ. Microbiol. 2005 Mar; 71 (3): 1223-1230). For example, expression of an isobutanol biosynthetic pathway in *Lactobacillus plantarum* is described in Example 25.

### Expression of an isobutanol biosynthetic pathway in Enterococcus faecium Enterococcus gallinarium, and Enterococcus faecalis

The *Enterococcus* genus belongs to the *Lactobacillales* family and many plasmids and vectors used in the transformation of *Lactobacillus, Bacillus subtilis,* and *Streptococcus* may be used for *Enterococcus.* Non-limiting examples of suitable vectors include pAMβ1 and derivatives thereof (Renault et al., Gene 183:175-182 (1996); and O'Sullivan et al., Gene 137:227-231 (1993)); pMBB1 and pHW800, a derivative of pMBB1 (Wyckoff et al. Appl. Environ. Microbiol. 62:1481-1486 (1996)); pMG1, a conjugative plasmid (Tanimoto et al., J. Bacteriol. 184:5800-5804 (2002)); pNZ9520 (Kleerebezem et al., Appl. Environ. Microbiol. 63:4581-4584 (1997)); pAM401 (Fujimoto et al., Appl. Environ. Microbiol. 67:1262-1267 (2001)); and pAT392 (Arthur et al., Antimicrob. Agents Chemother. 38:1899-1903 (1994)). Expression vectors for *E. faecalis* using the *nisA* gene from *Lactococcus* may also be used (Eichenbaum et al., Appl. Environ. Microbiol. 64:2763-2769 (1998). Additionally, vectors for gene replacement in the *E*. *faecium* chromosome may be used (Nallaapareddy et al., Appl. Environ. Microbiol. 72:334-345 (2006)). For example, expression of an isobutanol biosynthetic pathway in *Enterococcus faecalis* is described in Example 26.

### Fermentation Media

Fermentation media in the present invention must contain suitable carbon substrates. Suitable substrates may include but are not limited to monosaccharides such as glucose and fructose, oligosaccharides such as lactose or sucrose, polysaccharides such as starch or cellulose or mixtures thereof and unpurified mixtures from renewable feedstocks such as cheese whey permeate, cornsteep liquor, sugar beet molasses, and barley malt. Additionally the carbon substrate may also be one-carbon substrates such as carbon dioxide, or methanol for which metabolic conversion into key biochemical intermediates has been demonstrated. In addition to one and two carbon substrates methylotrophic organisms are also known to utilize a number of other carbon containing compounds such as methylamine, glucosamine and a variety of amino acids for metabolic activity. For example, methylotrophic yeast are known to utilize the carbon from methylamine to form trehalose or glycerol (Bellion et al., Microb. Growth C1 Compd., [Int. Symp.], 7th (1993), 415-32. Editor(s): Murrell, J. Collin; Kelly, Don P. Publisher: Intercept, Andover, UK). Similarly, various species of *Candida* will metabolize alanine or oleic acid (Sulter et al., Arch. Microbiol. 153:485-489 (1990)). Hence it is contemplated that the source of carbon utilized in the present invention may encompass a wide variety of carbon containing substrates and will only be limited by the choice of organism.

Although it is contemplated that all of the above mentioned carbon substrates and mixtures thereof are suitable in the present invention, preferred carbon substrates are glucose, fructose, and sucrose. Sucrose may be derived from renewable sugar sources such as sugar cane, sugar beets, cassava, sweet sorghum, and mixtures thereof. Glucose and dextrose may be derived from renewable grain sources through saccharification of starch based feedstocks including grains such as corn, wheat, rye, barley, oats, and mixtures thereof. In addition, fermentable sugars may be derived from renewable cellulosic or lignocellulosic biomass through processes of pretreatment and saccharification, as described, for example, in co-owned and co-pending U.S. Patent Application Publication No. 2007/0031918A1, which is herein incorporated by reference. Biomass refers to any cellulosic or lignocellulosic material and includes materials comprising cellulose, and optionally further comprising hemicellulose, lignin, starch, oligosaccharides and/or monosaccharides. Biomass may also comprise additional components, such as protein and/or lipid. Biomass may be derived from a single source, or biomass can comprise a mixture derived from more than one source; for example, biomass may comprise a mixture of corn cobs and corn stover, or a mixture of grass and leaves. Biomass includes, but is not limited to, bioenergy crops, agricultural residues, municipal solid waste, industrial solid waste, sludge from paper manufacture, yard waste, wood and forestry waste. Examples of biomass include, but are not limited to, corn grain, corn cobs, crop residues such as corn husks, corn stover, grasses, wheat, wheat straw, barley, barley straw, hay, rice straw, switchgrass, waste paper, sugar cane bagasse, sorghum, soy, components obtained from milling of grains, trees, branches, roots, leaves, wood chips, sawdust, shrubs and bushes, vegetables, fruits, flowers, animal manure, and mixtures thereof.

In addition to an appropriate carbon source, fermentation media must contain suitable minerals, salts, cofactors, buffers and other components, known to those skilled in the art, suitable for the growth of the cultures and promotion of the enzymatic pathway necessary for isobutanol production.

### Culture Conditions with Temperature Lowering

In the present method, the recombinant microbial production host which produces isobutanol is seeded into a fermentation medium comprising a fermentable carbon substrate to create a fermentation culture. The production host is grown in the fermentation culture at a first temperature for a first period of time. The first temperature is typically from about 25 °C to about 40 °C.

Suitable fermentation media in the present invention include common commercially prepared media such as Luria Bertani (LB) broth, Sabouraud Dextrose (SD) broth or Yeast Medium (YM) broth. Other defined or synthetic growth media may also be used, and the appropriate medium for growth of the particular microorganism will be known by one skilled in the art of microbiology or fermentation science. The use of agents known to modulate catabolite repression directly or indirectly, e.g., cyclic adenosine 2':3'-monophosphate, may also be incorporated into the fermentation medium.

Suitable pH ranges for the fermentation are between pH 5.0 to pH 9.0, where pH 6.0 to pH 8.0 is preferred as the initial condition.

Fermentations may be performed under aerobic or anaerobic conditions, where anaerobic or microaerobic conditions are preferred.

The first period of time to grow the production host at the first temperature may be determined in a variety of ways. For example, during this period of growth a metabolic parameter of the fermentation culture may be monitored. The metabolic parameter that is monitored may be any parameter known in the art, including, but not limited to the optical density, pH, respiratory quotient, fermentable carbon substrate utilization, CO₂ production, and isobutanol production. During this period of growth, additional fermentable carbon substrate may be added, the pH may be adjusted, oxygen may be added for aerobic cells, or other culture parameters may be adjusted to support the metabolic activity of the culture. Though nutrients and culture conditions are supportive of growth, after a period of time the metabolic activity of the fermentation culture decreases as determined by the monitored parameter described above. For example, a decrease in metabolic activity may be indicated by a decrease in one or more of the following parameters: rate of optical density change, rate of pH change, rate of change in respiratory quotient (if the host cells are aerobic), rate of fermentable carbon substrate utilization, rate of isobutanol production, rate of change in CO₂ production, or rate of another metabolic parameter. The decrease in metabolic activity is related to the sensitivity of the host cells to the production of isobutanol and/or the presence of isobutanol in the culture. When decreased metabolic activity is detected, the temperature of the fermentation culture is lowered to reduce the sensitivity of the host cells to isobutanol and thereby allow further production of isobutanol. In one embodiment, the lowering of the temperature coincides with a change in the metabolic parameter that is monitored.

In one embodiment, the change in metabolic activity is a decrease in the rate of isobutanol production. Isobutanol production may be monitored by analyzing the amount of isobutanol present in the fermentation culture medium as a function of time using methods well known in the art, such as using high performance liquid chromatography (HPLC) or gas chromatography (GC), which are described in the Examples herein. GC is preferred due to the short assay time.

Alternatively, the lowering of the temperature of the fermentation culture may occur at a predetermined time. The first period of time may be predetermined by establishing a correlation between a metabolic parameter of the fermentation culture and time in a series of test fermentations runs. A correlation between a metabolic parameter, as described above, and time of culture growth may be established for any isobutanol producing host by one skilled in the art. The specific correlation may vary depending on conditions used including, but not limited to, carbon substrate, fermentation conditions, and the specific recombinant isobutanol producing microbial production host. The correlation is most suitably made between isobutanol production or specific glucose consumption rate and time of culture growth. Once the predetermined time has been established from the correlation, the temperature of the fermentation culture in subsequent fermentation runs is lowered at the predetermined time. For example, if it is determined by monitoring a metabolic parameter in the test fermentation runs that the rate of production of isobutanol decreases after 12 hours, the temperature in subsequent fermentations runs is lowered after 12 hours without the need to monitor isobutanol production in the subsequent runs.

After the first period of time, the temperature of the fermentation culture is lowered to a second temperature. Typically, the second temperature is about 3 °C to about 25 °C lower than the first temperature. Reduction in temperature to enhance tolerance of the host cells to isobutanol is balanced with maintaining the temperature at a level where the cells continue to be metabolically active for isobutanol production. For example, a fermentation culture that has been grown at about 35 °C may be reduced in temperature to about 28 °C; or a culture grown at about 30 °C may be reduced in temperature to about 25 °C. The change in temperature may be done gradually over time or may be made as a step change. The production host is incubated at the second temperature for a second period of time, so that isobutanol production continues. The second period of time may be determined in the same manner as the first period of time described above, e.g., by monitoring a metabolic parameter or by using a predetermined time.

Additionally, the temperature lowering and incubation steps may be repeated one or more times to more finely balance metabolic activity for isobutanol production and isobutanol sensitivity. For example, a culture that has been grown at about 35 °C may be reduced in temperature to about 32 °C, followed by an incubation period. During this period a metabolic parameter of the fermentation culture may be monitored as described above, or a predetermined time may be used. It is particularly suitable to monitor the production of isobutanol during this incubation period. When monitoring indicates a decrease in metabolic activity or at a predetermined time, the temperature may be reduced a second time. For example, the temperature may be reduced from about 32 °C to about 28 °C. The temperature lowering and incubation steps may be repeated a third time where the temperature is reduced, for example, to about 20 °C. The production host is incubated at the lowered temperature so that isobutanol production continues. The steps may be repeated further as necessary to obtain the desired isobutanol titer.

### Industrial Batch and Continuous Fermentations

The present process employs a batch method of fermentation. A classical batch fermentation is a closed system where the composition of the medium is set at the beginning of the fermentation and not subject to artificial alterations during the fermentation. Thus, at the beginning of the fermentation the medium is inoculated with the desired organism or organisms, and fermentation is permitted to occur without adding anything to the system. Typically, however, a "batch" fermentation is batch with respect to the addition of carbon source and attempts are often made at controlling factors such as pH and oxygen concentration. In batch systems the metabolite and biomass compositions of the system change constantly up to the time the fermentation is stopped. Within batch cultures cells moderate through a static lag phase to a high growth log phase and finally to a stationary phase where growth rate is diminished or halted. If untreated, cells in the stationary phase will eventually die. Cells in log phase generally are responsible for the bulk of production of end product or intermediate.

A variation on the standard batch system is the Fed-Batch system. Fed-Batch fermentation processes are also suitable in the present invention and comprise a typical batch system with the exception that the substrate is added in increments as the fermentation progresses. Fed-Batch systems are useful when catabolite repression is apt to inhibit the metabolism of the cells and where it is desirable to have limited amounts of substrate in the media. Measurement of the actual substrate concentration in Fed-Batch systems is difficult and is therefore estimated on the basis of the changes of measurable factors such as pH, dissolved oxygen and the partial pressure of waste gases such as CO₂. Batch and Fed-Batch fermentations are common and well known in the art and examples may be found in Thomas D. Brock in Biotechnology: A Textbook of Industrial Microbiology, Second Edition (1989) Sinauer Associates, Inc., Sunderland, MA., or Deshpande, Mukund V., Appl. Biochem. Biotechnol., 36:227, (1992), herein incorporated by reference.

Although the present invention is performed in batch mode it is contemplated that the method would be adaptable to continuous fermentation methods. Continuous fermentation is an open system where a defined fermentation medium is added continuously to a bioreactor and an equal amount of conditioned media is removed simultaneously for processing. Continuous fermentation generally maintains the cultures at a constant high density where cells are primarily in log phase growth.

Continuous fermentation allows for the modulation of one factor or any number of factors that affect cell growth or end product concentration. For example, one method will maintain a limiting nutrient such as the carbon source or nitrogen level at a fixed rate and allow all other parameters to moderate. In other systems a number of factors affecting growth can be altered continuously while the cell concentration, measured by media turbidity, is kept constant. Continuous systems strive to maintain steady state growth conditions and thus the cell loss due to the medium being drawn off must be balanced against the cell growth rate in the fermentation. Methods of modulating nutrients and growth factors for continuous fermentation processes as well as techniques for maximizing the rate of product formation are well known in the art of industrial microbiology and a variety of methods are detailed by Brock, *supra.*

It is contemplated that the present invention may be practiced using either batch, fed-batch or continuous processes and that any known mode of fermentation would be suitable. Additionally, it is contemplated that cells may be immobilized on a substrate as whole cell catalysts and subjected to fermentation conditions for isobutanol production.

### Methods for Isobutanol Isolation from the Fermentation Medium

The bioproduced isobutanol may be isolated from the fermentation medium using methods known in the art. For example, solids may be removed from the fermentation medium by centrifugation, filtration, decantation, or the like. Then, the isobutanol may be isolated from the fermentation medium, which has been treated to remove solids as described above, using methods such as distillation, liquid-liquid extraction, or membrane-based separation. Because isobutanol forms a low boiling point, azeotropic mixture with water, distillation can only be used to separate the mixture up to its azeotropic composition. Distillation may be used in combination with another separation method to obtain separation around the azeotrope. Methods that may be used in combination with distillation to isolate and purify isobutanol include, but are not limited to, decantation, liquid-liquid extraction, adsorption, and membrane-based techniques. Additionally, isobutanol may be isolated using azeotropic distillation using an entrainer (see for example Doherty and Malone, Conceptual Design of Distillation Systems, McGraw Hill, New York, 2001).

The isobutanol-water mixture forms a heterogeneous azeotrope so that distillation may be used in combination with decantation to isolate and purify the isobutanol. In this method, the isobutanol containing fermentation broth is distilled to near the azeotropic composition. Then, the azeotropic mixture is condensed, and the isobutanol is separated from the fermentation medium by decantation. The decanted aqueous phase may be returned to the first distillation column as reflux. The isobutanolrich decanted organic phase may be further purified by distillation in a second distillation column.

The isobutanol may also be isolated from the fermentation medium using liquid-liquid extraction in combination with distillation. In this method, the isobutanol is extracted from the fermentation broth using liquid-liquid extraction with a suitable solvent. The isobutanol-containing organic phase is then distilled to separate the isobutanol from the solvent.

Distillation in combination with adsorption may also be used to isolate isobutanol from the fermentation medium. In this method, the fermentation broth containing the isobutanol is distilled to near the azeotropic composition and then the remaining water is removed by use of an adsorbent, such as molecular sieves (Aden et al. Lignocellulosic Biomass to Ethanol Process Design and Economics Utilizing Co-Current Dilute Acid Prehydrolysis and Enzymatic Hydrolysis for Corn Stover, Report NREL/TP-510-32438, National Renewable Energy Laboratory, June 2002).

Additionally, distillation in combination with pervaporation may be used to isolate and purify the isobutanol from the fermentation medium. In this method, the fermentation broth containing the isobutanol is distilled to near the azeotropic composition, and then the remaining water is removed by pervaporation through a hydrophilic membrane (Guo et al., J. Membr. Sci. 245, 199-210 (2004)).

### EXAMPLES

The present invention is further defined in the following Examples. It should be understood that these Examples, while indicating preferred embodiments of the invention, are given by way of illustration only. From the above discussion and these Examples, one skilled in the art can ascertain the essential characteristics of this invention, and without departing from the spirit and scope thereof, can make various changes and modifications of the invention to adapt it to various uses and conditions.

### General Methods

Standard recombinant DNA and molecular cloning techniques used in the Examples are well known in the art and are described by Sambrook, J., Fritsch, E. F. and Maniatis, T. Molecular Cloning: A Laboratory Manual; Cold Spring Harbor Laboratory Press: Cold Spring Harbor, NY (1989) (Maniatis) and by T. J. Silhavy, M. L. Bennan, and L. W. Enquist, Experiments with Gene Fusions, Cold Spring Harbor Laboratory Press, Cold Spring Harbor, N.Y. (1984) and by Ausubel, F. M. et al., Current Protocols in Molecular Biology, pub. by Greene Publishing Assoc. and Wiley-Interscience (1987).

Materials and methods suitable for the maintenance and growth of bacterial cultures are well known in the art. Techniques suitable for use in the following Examples may be found as set out in Manual of Methods for General Bacteriology (Phillipp Gerhardt, R. G. E. Murray, Ralph N. Costilow, Eugene W. Nester, Willis A. Wood, Noel R. Krieg and G. Briggs Phillips, eds), American Society for Microbiology, Washington, DC. (1994)) or by Thomas D. Brock in Biotechnology: A Textbook of Industrial Microbiology, Second Edition, Sinauer Associates, Inc., Sunderland, MA (1989). All reagents, restriction enzymes and materials used for the growth and maintenance of bacterial cells were obtained from Aldrich Chemicals (Milwaukee, WI), BD Diagnostic Systems (Sparks, MD), Life Technologies (Rockville, MD), or Sigma Chemical Company (St. Louis, MO) unless otherwise specified.

Microbial strains were obtained from The American Type Culture Collection (ATCC), Manassas, VA, unless otherwise noted.

The oligonucleotide primers to use in the following Examples are given in Table 4. All the oligonucleotide primers are synthesized by Sigma-Genosys (Woodlands, TX).

**Table 4**

| Oligonucleotide Cloning, Screening, and Sequencing Primers | | | |
|---|---|---|---|
| Name | Sequence | Description | SEQ ID NO: |
| N80 | CACCATGGACAAACAGTATCCGG TACGCC | budB forward | 11 |
| N81 | CGAAGGGCGATAGCTTTACCAAT CC | budB reverse | 12 |
| N100 | CACCATGGCTAACTACTTCAATA CACTGA | ilvC forward | 13 |
| N101 | CCAGGAGAAGGCCTTGAGTGTTT TCTCC | ilvC reverse | 14 |
| N102 | CACCATGCCTAAGTACCGTTCCG CCACCA | ilvD forward | 15 |
| N103 | CGCAGCACTGCTCTTAAATATTC GGC | ilvD reverse | 16 |
| N104 | CACCATGAACAACTTTAATCTGC ACACCC | yqhD forward | 17 |
| N105 | GCTTAGCGGGCGGCTTCGTATAT ACGGC | yqhD reverse | 18 |
| N110 | GCATGCCTTAAGAAAGGAGGGG GGTCACATGGACAAACAGTATCC | budB forward | 19 |
| N111 | ATGCATTTAATTAATTACAGAATC TGACTCAGATGCAGC | budB reverse | 20 |
| N112 | GTCGACGCTAGCAAAGGAGGGA ATCACCATGGCTAACTACTTCAA | ilvC forward | 21 |
| N113 | TCTAGATTAACCCGCAACAGCAA TACGTTTC | ilvC reverse | 22 |
| N114 | TCTAGAAAAGGAGGAATAAAGTA TGCCTAAGTACCGTTC | ilvD forward | 23 |
| N115 | GGATCCTTATTAACCCCCCAGTT TCGATTTA | ilvD reverse | 24 |
| N116 | GGATCCAAAGGAGGCTAGACATA TGTATACTGTGGGGGA | kivD forward | 25 |
| N117 | GAGCTCTTAGCTTTTATTTTGCTC CGCAAAC | kivD reverse | 26 |
| N118 | GAGCTCAAAGGAGGAGCAAGTA ATGAACAACTTTAATCT | yqhD forward | 27 |
| N119 | GAATTCACTAGTCCTAGGTTAGC GGGCGGCTTCGTATATACGG | yqhD reverse | 28 |
| BenNF | CAACATTAGCGATTTTCTTTTCTC T | Npr forward | 29 |
| BenASR | CATGAAGCTTACTAGTGGGCTTA AGTTTTGAAAATAATGAAAACT | Npr reverse | 30 |
| N110.2 | GAGCTCACTAGTCAATTGTAAGT AAGTAAAAGGAGGTGGGTCACAT | budB forward | 31 |
| | GGACAAACAGTATCC | | |
| N111.2 | GGATCCGATCGACTTAAGCCTCA GCTTACAGAATCTGACTCAGATG CAGC | budB reverse | 32 |
| N112.2 | GAGCTCCTTAAGAAGGAGGTAAT CACCATGGCTAACTACTTCAA | ilvC forward | 33 |
| N113.2 | GGATCCGATCGAGCTAGCGCGG CCGCTTAACCCGCAACAGCAATA CGTTTC | ilvC reverse | 34 |
| N114.2 | GAGCTCGCTAGCAAGGAGGTAT AAAGTATGCCTAAGTACCGTTC | ilvD forward | 35 |
| N115.2 | GGATCCGATCGATTAATTAACCT AAGGTTATTAACCCCCCAGTTTC GATTTA | ilvD reverse | 36 |
| N116.2 | GAGCTCTTAATTAAAAGGAGGTT AGACATATGTATACTGTGGGGGA | kivD forward | 37 |
| N117.2 | GGATCCAGATCTCCTAGGACATG TTTAGCTTTTATTTTGCTCCGCAA AC | kivD reverse | 38 |
| N130SeqF1 | TGTTCCAACCTGATCACCG | sequencing primer | 40 |
| N130SeqF2 | GGAAAACAGCAAGGCGCT | sequencing primer | 41 |
| N130SeqF3 | CAGCTGAACCAGTTTGCC | sequencing primer | 42 |
| N130SeqF4 | AAAATACCAGCGCCTGTCC | sequencing primer | 43 |
| N130SeqR1 | TGAATGGCCACCATGTTG | sequencing primer | 44 |
| N130SeqR2 | GAGGATCTCCGCCGCCTG | sequencing primer | 45 |
| N130SeqR3 | AGGCCGAGCAGGAAGATC | sequencing primer | 46 |
| N130SeqR4 | TGATCAGGTTGGAACAGCC | sequencing primer | 47 |
| N131SeqF1 | AAGAACTGATCCCACAGGC | sequencing primer | 48 |
| N131SeqF2 | ATCCTGTGCGGTATGTTGC | sequencing primer | 49 |
| N131SeqF3 | ATTGCGATGGTGAAAGCG | sequencing primer | 50 |
| N131SeqR1 | ATGGTGTTGGCAATCAGCG | sequencing primer | 51 |
| N131SeqR2 | GTGCTTCGGTGATGGTTT | sequencing primer | 52 |
| N131SeqR3 | TTGAAACCGTGCGAGTAGC | sequencing primer | 53 |
| N132SeqF1 | TATTCACTGCCATCTCGCG | sequencing primer | 54 |
| N132SeqF2 | CCGTAAGCAGCTGTTCCT | sequencing primer | 55 |
| N132SeqF3 | GCTGGAACAATACGACGTTA | sequencing primer | 56 |
| N132SeqF4 | TGCTCTACCCAACCAGCTTC | sequencing primer | 57 |
| N132SeqR1 | ATGGAAAGACCAGAGGTGCC | sequencing primer | 58 |
| N132SeqR2 | TGCCTGTGTGGTACGAAT | sequencing primer | 59 |
| N132SeqR3 | TATTACGCGGCAGTGCACT | sequencing primer | 60 |
| N132SeqR4 | GGTGATTTTGTCGCAGTTAGAG | sequencing primer | 61 |
| N133SeqF1 | TCGAAATTGTTGGGTCGC | sequencing primer | 62 |
| N133SeqF2 | GGTCACGCAGTTCATTTCTAAG | sequencing primer | 63 |
| N133SeqF3 | TGTGGCAAGCCGTAGAAA | sequencing primer | 64 |
| N133SeqF4 | AGGATCGCGTGGTGAGTAA | sequencing primer | 65 |
| N133SeqR1 | GTAGCCGTCGTTATTGATGA | sequencing primer | 66 |
| N133SeqR2 | GCAGCGAACTAATCAGAGATTC | sequencing primer | 67 |
| N133SeqR3 | TGGTCCGATGTATTGGAGG | sequencing primer | 68 |
| N133SeqR4 | TCTGCCATATAGCTCGCGT | sequencing primer | 69 |
| Scr1 | CCTTTCTTTGTGAATCGG | sequencing primer | 72 |
| Scr2 | AGAAACAGGGTGTGATCC | sequencing primer | 73 |
| Scr3 | AGTGATCATCACCTGTTGCC | sequencing primer | 74 |
| Scr4 | AGCACGGCGAGAGTCGACGG | sequencing primer | 75 |
| T-budB (BamHI) | AGATAGATGGATCCGGAGGTGG GTCACATGGACAAACAGT | budB forward | 144 |
| B-kivD (BamHI) | CTCTAGAGGATCCAGACTCCTAG GACATG | kivD reverse | 145 |
| T-groE(XhoI) | AGATAGATCTCGAGAGCTATTGT AACATAATCGGTACGGGGGTG | PgroE forward | 147 |
| B-groEL(SpeI, BamH1) | ATTATGTCAGGATCCACTAGTTT CCTCCTTTAATTGGGAATTGTTAT CCGC | PgroE reverse | 148 |
| T-groEL | AGCTATTGTAACATAATCGGTAC GGGGGTG | PgroE forward | 149 |
| T-ilvCB.s. (BamHI) | ACATTGATGGATCCCATAACAAG GGAGAGATTGAAATGGTAAAAG | ilvC forward | 150 |
| B-ilvCB.s. (SpeIBamHI) | TAGACAACGGATCCACTAGTTTA ATTTTGCGCAACGGAGACCACCG C | ilvC reverse | 151 |
| T-BD64 (DraIII) | TTACCGTGGACTCACCGAGTGG GTAACTAGCCTCGCCGGAAAGA GCG | pBD64 forward | 152 |
| B-BD64 (DraIII) | TCACAGTTAAGACACCTGGTGCC GTTAATGCGCCATGACAGCCATG | p8D64 reverse | 153 |
| | AT | | |
| T-lacIq (DraIII) | ACAGATAGATCACCAGGTGCAAG CTAATTCCGGTGGAAACGAGGTC ATC | lacIq forward | 154 |
| B-lacIq (DraIII) | ACAGTACGATACACGGGGTGTCA CTGCCCGCTTTCCAGTCGGGAAA CC | laclq reverse | 155 |
| T-groE (DraIII) | TCGGATTACGCACCCCGTGAGCT ATTGTAACATAATCGGTACGGGG GTG | PgroE forward | 156 |
| B-B.s.ilvC (DraIII) | CTGCTGATCTCACACCGTGTGTT AATTTTGCGCAACGGAGACCACC GC | ilvC reverse | 157 |
| T-bdhB (DraIII) | TCGATAGCATACACACGGTGGTT AACAAAGGAGGGGTTAAAATGGT TGATTTCG | bdhB forward | 159 |
| B-bdhB (rrnBT1DraIII) | ATCTACGCACTCGGTGATAAAAC GAAAGGCCCAGTCTTTCGACTGA GCCTTTCGTTTTATCTTACACAGA TTTTTTGAATATTTGTAGGAC | bdhB reverse | 160 |
| LDH EcoRV F | GACGTCATGACCACCCGCCGATCCC TTTT | IdhL forward | 161 |
| LDH AatIIR | GATATCCAACACCAGCGACCGACGT ATTAC | ldhL reverse | 162 |
| Cm F | ATTTAAATCTCGAGTAGAGGATCCCA ACAAACGAAAATTGGATAAAG | Cm forward | 163 |
| Cm R | ACGCGTTATTATAAAAGCCAGTCATT AGG | Cm reverse | 164 |
| P11 F-StuI | CCTAGCGCTATAGTTGTTGACAG AATGGACATACTATGATATATTGT TGCTATAGCGA | P11 promoter forward | 165 |
| P11 R-SpeI | CTAGTCGCTATAGCAACAATATA TCATAGTATGTCCATTCTGTCAAC AACTATAGCGCTAGG | P11 promoter reverse | 166 |
| PldhL F-HindIII | AAGCTTGTCGACAAACCAACATT ATGACGTGTCTGGGC | ldhL forward | 167 |
| PldhL R- | GGATCCTCATCCTCTCGTAGTGA | ldhL reverse | 168 |
| BamHI | AAATT | | |
| F-bdhB-AvrII | TTCCTAGGAAGGAGGTGGTTAAA ATGGTTGATTTCG | bdhB forward | 169 |
| R-bdhB-BamHl | TTGGATCCTTACACAGATTTTTTG AATAT | bdhB reverse | 170 |
| F-ilvC(B.s.)-AflII | AACTTAAGAAGGAGGTGATTGAA ATGGTAAAAGTATATT | ilvC forward | 171 |
| R-ilvC(B.s.)-Notl | AAGCGGCCGCTTAATTTTGCGCA ACGGAGACC | ivlC reverse | 172 |
| F-PnisA(HindIII) | TTAAGCTTGACATACTTGAATGACCT AGTC | nisA promoter forward | 173 |
| R-PnisA(SpeI BamHI) | TTGGATCCAAACTAGTATAATTTATT TTGTAGTTCCTTC | nisA promoter reverse | 174 |

### Methods for Determining Isobutanol Concentration in Culture Media

The concentration of isobutanol in the culture media can be determined by a number of methods known in the art. For example, a specific high performance liquid chromatography (HPLC) method utilized a Shodex SH-1011 column with a Shodex SH-G guard column, both purchased from Waters Corporation (Milford, MA), with refractive index (RI) detection. Chromatographic separation was achieved using 0.01 M H₂SO₄ as the mobile phase with a flow rate of 0.5 mL/min and a column temperature of 50 °C. Isobutanol had a retention time of 46.6 min under the conditions used. Alternatively, gas chromatography (GC) methods are available. For example, a specific GC method utilized an HP-INNOWax column (30 m x 0.53 mm id,1 µm film thickness, Agilent Technologies, Wilmington, DE), with a flame ionization detector (FID). The carrier gas was helium at a flow rate of 4.5 mL/min, measured at 150 °C with constant head pressure; injector split was 1:25 at 200 °C; oven temperature was 45 °C for 1 min, 45 to 220 °C at 10 °C/min, and 220 °C for 5 min; and FID detection was employed at 240 °C with 26 mL/min helium makeup gas. The retention time of isobutanol was 4.5 min.

The meaning of abbreviations is as follows: "s" means second(s), "min" means minute(s), "h" means hour(s), "psi" means pounds per square inch, "nm" means nanometers, "d" means day(s), "µL" means microliter(s), "mL" means milliliter(s), "L" means liter(s), "mm" means millimeter(s), "nm" means nanometers, "mM" means millimolar, "µM" means micromolar, "M" means molar, "mmol" means millimole(s), "µmol" means micromole(s)", "g" means gram(s), "µg" means microgram(s) and "ng" means nanogram(s), "PCR" means polymerase chain reaction, "OD" means optical density, "OD₆₀₀" means the optical density measured at a wavelength of 600 nm, "kDa" means kilodaltons, "g" means the gravitation constant, "bp" means base pair(s), "kbp" means kilobase pair(s), "% w/v" means weight/volume percent, % v/v" means volume/volume percent, "IPTG" means isopropyl-β-D-thiogalactopyranoiside, "RBS" means ribosome binding site, "nt" means not tested, "HPLC" means high performance liquid chromatography, and "GC" means gas chromatography. The term "molar selectivity" is the number of moles of product produced per mole of sugar substrate consumed and is reported as a percent.

### EXAMPLE 1

### Increased tolerance of Lactobacillus plantarum PN0512 to 1-butanol, isobutanol and 2-butanol at decreased growth temperatures

Tolerance levels of bacterial strain *Lactobacillus plantarum* PN0512 (ATCC # PTA-7727) were determined at 25 °C, 30 °C and 37 °C as follows. The strain was cultured in S30L medium (i.e., 10 mM ammonium sulfate, 5 mM potassium phosphate buffer, pH 7.0, 50 mM MOPS, pH 7.0, 2 mM MgCl₂, 0.7 mM CaCl₂, 50 µM MnCl₂, 1 µM FeCl₃, 1 µM ZnCl₂, 1.72 µM CuCl₂, 2.53 µM CoCl₂, 2.42 µM Na₂MoO₄, 2 µM thiamine hydrochloride, 10 mM glucose, and 0.2% yeast extract). An overnight culture in the absence of any test compound was started in 15 mL of the S30L medium in a 150 mL flask, with incubation at 37 °C in a shaking water bath. The next morning, the overnight culture was diluted into three 500 mL flasks containing 150 mL of fresh medium to an initial OD₆₀₀ of about 0.08. Each flask was incubated in a shaking water bath, one each at 25 °C, 30 °C and 37 °C. Each large culture was allowed to acclimate at the test temperature for at least 0.5 h. After the acclimation period, each large culture was split into flasks in the absence (control) and in the presence of various amounts of 1-butanol, isobutanol or 2-butanol, as listed in Tables 5, 6, and 7, respectively. Growth was followed by measuring OD₆₀₀ for six hours after addition of the compounds. The results are summarized in Tables 5, 6, and 7 below.

**Table 5. Growth of L. plantarum PN0512 in the presence of 1-butanol at different temperatures**

| Concentration 1-butanol (% w/v) | 37 °C | 30 °C | 25 °C |
|---|---|---|---|
| 0.0 | + | + | + |
| 1.0 | + | nt | nt |
| 1.2 | + | nt | nt |
| 1.4 | + | nt | nt |
| 1.5 | + | + | + |
| 1.6 | + | nt | nt |
| 1.8 | + | nt | nt |
| 2.0 | + | + | + |
| 2.1 | + | nt | nt |
| 2.2 | + | nt | nt |
| 2.3 | + | nt | nt |
| 2.4 | - | + | + |
| 2.5 | - | nt | nt |
| 2.7 | - | + | nt |
| 2.9 | - | - | + |
| 3.1 | - | - | + |
| 3.2 | nt | - | - |
| 3.3 | nt | nt | - |
| 3.4 | nt | - | - |

**Table 6. Growth of L. plantarum PN0512 in the presence of isobutanol at different temperatures**

| Concentration isobutanol (% w/v) | 37 °C | 30 °C | 25 °C |
|---|---|---|---|
| 0.0 | + | + | + |
| 0.5 | + | nt | nt |
| 1.0 | + | nt | nt |
| 1.5 | + | + | + |
| 1.6 | + | nt | nt |
| 1.8 | + | nt | nt |
| 2.0 | + | + | + |
| 2.1 | + | nt | nt |
| 2.3 | + | nt | nt |
| 2.4 | + | + | + |
| 2.5 | + | nt | nt |
| 2.7 | + | + | + |
| 2.9 | + | + | + |
| 3.1 | + | + | + |
| 3.3 | nt | - | + |
| 3.4 | - | nt | nt |
| 3.5 | nt | nt | + |
| 3.6 | nt | nt | - |
| 3.8 | - | nt | nt |
| 4.3 | - | nt | nt |

**Table 7. Growth of L. plantarum PN0512 in the presence of 2-butanol at different temperatures**

| Concentration 2-butanol (% w/v) | 37 °C | 30 °C | 25 °C |
|---|---|---|---|
| 0.0 | + | + | + |
| 1.8 | + | nt | nt |
| 2.1 | + | nt | nt |
| 2.5 | + | nt | nt |
| 2.9 | + | + | + |
| 3.1 | + | nt | nt |
| 3.5 | + | nt | nt |
| 3.6 | + | nt | nt |
| 3.8 | + | + | + |
| 4.0 | nt | + | nt |
| 4.3 | + | + | + |
| 4.5 | - | + | nt |
| 4.7 | - | + | + |
| 4.9 | nt | - | + |
| 5.2 | - | nt | + |
| 5.6 | - | nt | - |
| 6.0 | - | nt | nt |
| 6.4 | - | nt | nt |
| 7.3 | - | nt | nt |

| | | | |
|---|---|---|---|
| "+" = growth observed as an increase in OD₆₀₀. "-" = no growth observed, i.e. no change in OD₆₀₀. | | | |

All three butanols showed a similar effect of temperature on growth inhibition of *L. plantarum* PN0512. The concentration that resulted in full growth inhibition was greater at 25 °C than at 37 °C. In the case of 1-butanol, growth was observed at 37 °C in 2.3% 1-butanol, but not 2.4%. However, at 30 °C growth was observed in 2.7%, but not 2.9%, and at 25 °C growth was observed even in 3.1% 1-butanol. Thus, the concentration of 1-butanol that completely inhibited growth increased as growth temperature decreased. Likewise, in the case of isobutanol, growth was observed in 3.5% at 25 °C while growth was observed in 3.1 % at 30 °C and 37 °C, but not in 3.3% or 3.4%. Similarly, in the case of 2-butanol growth was observed at 37 °C in 4.3%, but not in 4.5%; at 30 °C in 4.7%, but not in 4.9%; and at 25 °C in 5.2 %. Thus the tolerance of *L*. *plantarum* PN0512 to butanols increased with decreased growth temperature.

### EXAMPLE 2

### Increased tolerance of Escherichia coli to 1-butanol at decreased exposure temperature

The effect of growth and exposure temperature on survival of *Escherichia coli* in the presence of 1-butanol was tested using stationary phase cultures in a rich medium and log phase cultures in a defined medium. For the stationary phase studies, *E. coli* strain MG1655 (ATCC # 700926) was grown overnight in LB medium (Teknova, Half Moon Bay, CA) with shaking at 250 rpm at 42 °C, 29 °C or 28 °C. Survival of 1-butanol shock was tested at exposure temperatures of 0 °C, 28 °C or 42 °C. The 1-butanol exposure at 28 °C or 42 °C was started immediately after removing the overnight cultures from the growth incubators. The 1-butanol exposure at 0 °C was done after allowing the overnight cultures to cool on ice for about 15 min. A series of solutions of 1-butanol at different concentrations in LB medium was made and 90 µL aliquots were put in microfuge tubes. To these were added 10 µL of the overnight cultures and the tubes were immediately placed in shaking incubators at 42 °C or 28 °C or left on ice for 30 min. To stop the effect of 1-butanol on the cultures, a 10⁻² dilution was done by placing 2 µL of the treated culture into 198 µL of LB medium in wells of a microplate. Then, 5 µL of the undiluted treated cultures were spotted on LB agar plates. Subsequent 10-fold serial dilutions of 10⁻³, 10⁻⁴, 10⁻⁵ and 10⁻⁶ of the exposed cultures were done by serial pipetting of 20 µL, starting with the 10⁻² dilution cultures, into 180 µL of LB medium in the microplate, using a multi-channel pipette. Prior to each transfer, the cultures were mixed by pipetting up and down six times. Each dilution (5 µL) was spotted onto an LB plate using a multi-channel pipette and allowed to soak into the plate. The plates were inverted and incubated overnight at 37 °C. The number of colonies for each dilution was counted and the % growth inhibition was calculated by comparison with a control culture that had not been exposed to 1-butanol. Survival of 0% was recorded when no colonies in the spots of the undiluted or any of the serial dilutions were observed. The results are shown in Table 8.

**Table 8. Survival of stationary phase E. coli in 1-butanol at 42 °C, 28 °C, or 0 °C**

| | Grown at 42 °C | Grown at 29 °C | Grown at 42 °C | Grown at 28°C | Grown at 42 °C | Grown at 29 °C |
|---|---|---|---|---|---|---|
| 1-Butanol % (w/v) | % survival after 30 min exposure at 42 °C | | % survival after 30 min exposure at 28 °C | | % survival after 30 min exposure at 0 °C | |
| 1.0 | 100 | 100 | 100 | 100 | 100 | 100 |
| 1.5 | 0.1 | 0.1 | 100 | 100 | 100 | 100 |
| 2.0 | 0 | 0.1 | 100 | 100 | 100 | 100 |
| 2.5 | 0 | 0 | 100 | 100 | 100 | 100 |
| 3.0 | 0 | 0 | 100 | 100 | 100 | 100 |
| 3.5 | 0 | 0 | 3 | 10 | 100 | 100 |
| 4.0 | 0 | 0 | 0.0004 | 0.0003 | 100 | 100 |
| 5.0 | nt | nt | nt | nt | 1 | 1 |
| 6.0 | nt | nt | nt | nt | 0 | 0.001 |
| 7.0 | nt | nt | nt | nt | 0 | 0 |

A similar study was done with log-phase cultures of *E. coli* grown in a defined medium. *E. coli* strain MG1655 was allowed to grow overnight in MOPS 0.2% glucose medium (Teknova, Half Moon Bay, CA) at 42 °C or 28 °C. The following day, the cultures were diluted into fresh medium and allowed to grow at the same temperature until in the log phase of growth. The OD₆₀₀ was 0.74 for the 28 °C culture and was 0.72 for the 42 °C culture. Both of these log phase cultures were exposed to 1-butanol at 42 °C, 28 °C and 0 °C as follows. A series of solutions of 1-butanol at different concentrations in MOPS 0.2% glucose medium was made and 90 µL aliquots were put in microfuge tubes. To these were added 10 µL of the log phase cultures and the tubes were immediately placed in shaking incubators at 42 °C or 28 °C or left on ice for 30 min. To stop the effect of 1-butanol on the cultures, a 10⁻² dilution was done by placing 2 µL of the treated culture into 198 µL of LB medium in wells of a microplate. Then 5 µL of the undiluted treated cultures were spotted on LB agar plates. Subsequent 10-fold serial dilutions of 10⁻³, 10⁻⁴, 10⁻⁵ and 10⁻⁶ of the exposed cultures were done by serial pipetting of 20 µL, starting with the 10⁻² dilution cultures, into 180 µL of LB medium in the microplate, using a multi-channel pipette. Prior to each transfer, the cultures were mixed by pipetting up and down six times. Each dilution (5 µL) was spotted onto an LB plate using a multi-channel pipette and allowed to soak into the plate. The plates were inverted and incubated overnight at 37 °C. The number of colonies for each dilution was counted and the % growth inhibition was calculated by comparison with a control culture that had not been exposed to 1-butanol. Survival of 0% was recorded when no colonies in the spots of the undiluted or any of the serial dilutions were observed. The results are shown in Table 9.

**Table 9. Survival of log-phase E. coli in 1-butanol at 42 °C, 28 °C, or 0 °C**

| | Grown at 42 °C | Grown at 28 °C | Grown at 42 °C | Grown at 28 °C | Grown at 42 °C | Grown at 29 °C |
|---|---|---|---|---|---|---|
| 1-Butanol % (w/v) | % survival after 30 min exposure at 42 °C | | % survival after 30 min exposure at 28 °C | | % survival after 30 min exposure at 0 °C | |
| 1.0 | 100 | 100 | nt | nt | nt | nt |
| 1.5 | 0 | 0 | 100 | 100 | nt | nt |
| 2.0 | 0 | 0 | 100 | 100 | nt | nt |
| 2.5 | 0 | 0 | 0.1 | 50 | 100 | 100 |
| 3.0 | 0 | 0 | 0 | 0 | 100 | 100 |
| 3.5 | 0 | 0 | 0.01 | 0 | 100 | 100 |
| 4.0 | 0 | 0 | 0.001 | 0 | 100 | 100 |
| 4.5 | nt | nt | 0 | 0 | 100 | 100 |
| 5.0 | nt | nt | nt | nt | 10 | 50 |
| 6.0 | nt | nt | nt | nt | 1 | 1 |

For both the stationary phase and log-phase cultures of *E. coli* MG1655, the growth temperature had very little, if any, effect on the survival of a 1-butanol shock. However, the exposure temperature had a major effect on the survival of *E. coli* to 1-butanol shock. As can be seen from the data in Tables 8 and 9, the tolerance of *E*. *coli* MG1655 to 1-butanol increased with decreasing exposure temperature.

### EXAMPLE 3

### Increased tolerance of Escherichia coli to 2-butanone at decreased exposure temperature

The effect of exposure temperature on survival of *Escherichia coli* in the presence of 2-butanone (also referred to herein as methyl ethyl ketone or MEK) was tested as follows. *E. coli* strain BW25113 (The Coli Genetic Stock Center (CGSC), Yale University; # 7636) was grown overnight in LB medium (Teknova, Half Moon Bay, CA) with shaking at 250 rpm at 37 °C. Survival of MEK shock was tested at exposure temperatures of 28 °C or 37 °C. A series of solutions of MEK at different concentrations in LB medium was made and 90 µL aliquots were put in microfuge tubes. To these were added 10 µL of the overnight culture and the tubes were immediately placed in shaking incubators at 37 °C or 28 °C for 30 min. To stop the effect of MEK on the cultures, a 10⁻² dilution was done by placing 2 µL of the MEK treated culture into 198 µL of LB medium in wells of a microplate. Then 5 µL of the undiluted treated cultures were spotted on LB agar plates. Subsequent 10-fold serial dilutions of 10⁻³, 10⁻⁴, 10⁻⁵ and 10⁻⁶ of the exposed cultures were done by serial pipetting of 20 µL, starting with the 10⁻² dilution cultures, into 180 µL of LB medium in the microplate, using a multi-channel pipette. Prior to each transfer, the cultures were mixed by pipetting up and down six times. Each dilution (5 µL) was spotted onto LB plates using a multi-channel pipette and allowed to soak into the plate. The plates were inverted and incubated overnight at 37 °C. The number of colonies for each dilution was counted and the % growth inhibition was calculated by comparison with a control culture that had not been exposed to MEK. Survival of 0% was recorded when no colonies in the spots of the undiluted or any of the serial dilutions were observed. The results, given as the average of duplicate experiments, are shown in Table 10.

**Table 10. Survival of E. coli in MEK at 37 °C and 28 °C**

| MEK % w/v | % Survival at 37 °C | % Survival at 28 °C |
|---|---|---|
| 0 | 100 | 100 |
| 4 | 100 | 100 |
| 6 | 0 | 100 |
| 8 | 0 | 0.002 |

Reducing the exposure temperature from 37 °C to 28 °C dramatically improved survival of *E*. *coli* to MEK treatment. At 37 °C there was full survival at 4% w/v and no survival at 6% w/v, while at 28 °C there was full survival at 6% w/v. Thus, the tolerance of *E*. *coli* to MEK increased with decreasing exposure temperature.

### EXAMPLE 4

### Increased tolerance of E. coli and L. plantarum PN0512 to 1-Butanol at decreased exposure temperature

This Example demonstrates that the toxic effects of 1-butanol and 2-butanol on various microbial cells was reduced at lower temperatures. This was demonstrated by incubating *E*. *coli* (strain MG1655; ATCC # 700926), and *L. plantarum* (strain PN0512; ATCC # PTA-7727) with either 1-butanol or 2-butanol at different temperatures and then determining the fraction of the cells that survived the treatment at the different temperatures.

Using overnight cultures or cells from plates, 30 mL cultures of the microorganisms to be tested were started in the following culture media:
*E. coli -* Miller's LB medium (Teknova, Half Moon Bay, CA):
*L. plantarum PN0512 - Lactobacilli* MRS Broth (BD Diagnostic Systems, Sparks, MD).
The *E*. *coli* and *L. plantarum* cultures were grown at 37 °C aerobically with shaking until the cultures were in log phase and the OD₆₀₀ was between 0.6 and 0.8. A 50 µL aliquot of each culture was removed for a time zero sample. The remainder of the cultures was divided into six 5 mL portions and placed in six small incubation flasks or tubes. Different amounts of 1-butanol or 2-butanol were added to the six flasks to bring the concentration to predetermined values, as listed in the tables below. The flasks or tubes were incubated at a desired temperature, aerobically without shaking for 1 h. After the incubation with one of the butanols, 2 µL from each of the flasks (and in addition 2 µL of the time zero sample of the culture before exposure to one of the butanols) were pipetted into the "head" wells of a 96 well (8 X 12) microtiter plate, each containing 198 µL of LB medium to give a 10⁻² dilution of the culture. Subsequently, 10⁻³, 10⁻⁴, 10⁻⁵, and 10⁻⁶ serial dilutions of the cultures were prepared as follows. The 10⁻³ dilution was prepared by pipetting 20 µL of the sample from the head well into the 180 µL LB medium in the next well using a multi-channel pipette. This procedure was repeated 3 more times on successive wells to prepare the 10⁻⁴, 10⁻⁵, and 10⁻⁶ dilutions. After each liquid transfer, the solution in the well was mixed by pipetting it up and down 10 times with the multi-channel pipetor. A 5 µL aliquot of each dilution was spotted onto an LB plate using a multi-channel pipette starting with the 10⁻⁶ dilution, then the 10⁻⁵, and so on working from more to less dilute without a change of tips. The spots were allowed to soak into the agar by leaving the lid of the plate slightly open for 15 to 30 min in a sterile transfer hood. The plates were covered, inverted, and incubated overnight at 37 °C. The following day, the number of colonies in the spots were counted from the different dilutions. The number of living cells/mL in each of the original culture solutions from which the 2 µL was withdrawn was calculated and compared to the number of cells in the control untreated culture to determine the % of the cells surviving.

The results of experiments in which E. *coli* cells were treated with 1-butanol at temperatures of 0, 30, and 37 °C are shown Table 11.

**Table 11. Percentage of E. coli cells surviving in 1-butanol at 0, 30 and 37 °C**

| 1-butanol % v/v | % Survival at 0 °C | % Survival at 30 °C | % Survival at 37 °C |
|---|---|---|---|
| 0 | 100 | 100 | 100 |
| 1 | nt | 100 | 72 |
| 1.5 | nt | 100 | 20 |
| 2 | nt | 100 | 0 |
| 2.5 | 100 | 23 | 0 |
| 3 | 100 | 0 | 0 |
| 3.5 | 100 | 0 | nt |
| 4 | 100 | nt | nt |
| 4.5 | 100 | nt | nt |

The concentration at which 1-butanol kills *E. coli* cells was affected by the treatment temperature. At 0 °C, concentrations of 1-butanol as high as 4.5% v/v had no toxic effect on *E. coli* cells during a one hour treatment. At 30 °C, *E. coli cells* were killed when treated with 3% v/v 1-butanol for one hour. At 37 °C, *E. coli* cells were killed when treated with 2% v/v 1-butanol for one hour.

The results of experiments in which *L. plantarum* PN0512 cells were treated with 1-butanol at temperatures of 0, 23, and 37 °C for one hour are shown Table 12.

**Table 12. Percentage of L. plantarum PN0512 cells surviving in 1-butanol at 0, 23 and 37 °C**

| 1-butanol % v/v | % Survival at 0 °C | % Survival at 23 °C | % Survival at 37 °C |
|---|---|---|---|
| 0 | 100 | 100 | 100 |
| 1 | nt | nt | 80 |
| 1.5 | nt | nt | 58 |
| 2 | nt | 100 | 29 |
| 2.5 | nt | 100 | 8 |
| 3 | 100 | 82 | 0 |
| 3.5 | 100 | 0 | 0 |
| 4 | 100 | 0 | nt |
| 4.5 | 100 | 0 | nt |
| 5 | 0 | nt | nt |
| 5.5 | 0 | nt | nt |

The concentration at which 1-butanol kills *L. plantarum* PN0512 cells was affected by the treatment temperature. At 0 °C, concentrations of 1-butanol as high as 4.5% v/v had no toxic effect on *L. plantarum* PN0512 cells during a one hour treatment. At 23 °C, *L. plantarum* PN0512 cells were killed when treated with 3.5% v/v 1-butanol for one hour. At 37 °C, *L. plantarum* PN0512 cells were killed when treated with 2.5 % v/v 1-butanol for one hour.

### EXAMPLE 5

### Cloning and Expression of Acetolactate Synthase

The purpose of this Example was to clone the *budB* gene from *Klebsiella pneumonia* and express it in *E. coli* BL21-AI. The *budB* gene was amplified from *Klebsiella pneumaniae* strain ATCC 25955 genomic DNA using PCR, resulting in a 1.8 kbp product.

Genomic DNA was prepared using the Gentra Puregene kit (Gentra Systems, Inc., Minneapolis, MN; catalog number D-5000A). The *budB* gene was amplified from *Klebsiella pneumoniae* genomic DNA by PCR using primers N80 and N81 (see Table 2), given as SEQ ID NOs:11 and 12, respectively. Other PCR amplification reagents were supplied in manufacturers' kits, for example, Finnzymes Phusion™ High-Fidelity PCR Master Mix (New England Biolabs Inc., Beverly, MA; catalog no. F-531) and used according to the manufacturer's protocol. Amplification was carried out in a DNA Thermocycler GeneAmp 9700 (PE Applied Biosystems, Foster city, CA).

For expression studies the Gateway cloning technology (Invitrogen Corp., Carlsbad, CA) was used. The entry vector pENTRSDD-TOPO allowed directional cloning and provided a Shine-Dalgarno sequence for the gene of interest. The destination vector pDEST14 used a T7 promoter for expression of the gene with no tag. The forward primer incorporated four bases (CACC) immediately adjacent to the translational start codon to allow directional cloning into pENTRSDD-TOPO (Invitrogen) to generate the plasmid pENTRSDD-TOPObudB. The pENTR construct was transformed into *E. coli* Top10 (Invitrogen) cells and plated according to manufacturer's recommendations. Transformants were grown overnight and plasmid DNA was prepared using the QIAprep Spin Miniprep kit (Qiagen, Valencia, CA; catalog no. 27106) according to manufacturer's recommendations. Clones were sequenced to confirm that the genes inserted in the correct orientation and to confirm the sequence. The nucleotide sequence of the open reading frame (ORF) for this gene and the predicted amino acid sequence of the enzyme are given as SEQ ID NO:1 and SEQ ID NO:2, respectively.

To create an expression clone, the *budB* gene was transferred to the pDEST 14 vector by recombination to generate pDEST14budB. The pDEST14budB vector was transformed into *E. coli* BL21-AI cells (Invitrogen). Transformants were inoculated into Luria Bertani (LB) medium supplemented with 50 µg/mL of ampicillin and grown overnight. An aliquot of the overnight culture was used to inoculate 50 mL of LB supplemented with 50 µg/mL of ampicillin. The culture was incubated at 37 °C with shaking until the OD₆₀₀ reached 0.6-0.8. The culture was split into two 25-mL cultures and arabinose was added to one of the flasks to a final concentration of 0.2% w/v. The negative control flask was not induced with arabinose. The flasks were incubated for 4 h at 37 °C with shaking. Cells were harvested by centrifugation and the cell pellets were resuspended in 50 mM MOPS, pH 7.0 buffer. The cells were disrupted either by sonication or by passage through a French Pressure Cell. The whole cell lysate was centrifuged yielding the supernatant or cell free extract and the pellet or the insoluble fraction. An aliquot of each fraction (whole cell lysate, cell free extract and insoluble fraction) was resuspended in SDS (MES) loading buffer (Invitrogen), heated to 85 °C for 10 min and subjected to SDS-PAGE analysis (NuPAGE 4-12% Bis-Tris Gel, catalog no. NP0322Box, Invitrogen). A protein of the expected molecular weight of about 60 kDa, as deduced from the nucleic acid sequence, was present in the induced culture but not in the uninduced control.

Acetolactate synthase activity in the cell free extracts is measured using the method described by Bauerle et al. (Biochim. Biophys. Acta 92(1):142-149 (1964)).

### Example 6 (Prophetic)

### Cloning and Expression of Acetohydroxy Acid Reductoisomerase

The purpose of this prophetic Example is to describe how to clone the *ilvC* gene from *E. coli* K12 and express it in *E. coli* BL21-AI. The *ilvC* gene is amplified from *E. coli* genomic DNA using PCR.

The *ilvC* gene is cloned and expressed in the same manner as the *budB* gene described in Example 5. Genomic DNA from *E. coli* is prepared using the Gentra Puregene kit (Gentra Systems, Inc., Minneapolis, MN; catalog number D-5000A). The *ilvC* gene is amplified by PCR using primers N100 and N101 (see Table 2), given as SEQ ID NOs:13 and 14, respectively, creating a 1.5 kbp product. The forward primer incorporates four bases (CCAC) immediately adjacent to the translational start codon to allow directional cloning into pENTR/SD/D-TOPO (Invitrogen) to generate the plasmid pENTRSDD-TOPOilvC. Clones are sequenced to confirm that the genes are inserted in the correct orientation and to confirm the sequence. The nucleotide sequence of the open reading frame (ORF) for this gene and the predicted amino acid sequence of the enzyme are given as SEQ ID NO:3 and SEQ ID NO:4, respectively.

To create an expression clone, the *ilvC* gene is transferred to the pDEST 14 (Invitrogen) vector by recombination to generate pDEST14ilvC. The pDEST14ilvC vector is transformed into *E. coli* BL21-AI cells and expression from the T7 promoter is induced by addition of arabinose. A protein of the expected molecular weight of about 54 kDa, as deduced from the nucleic acid sequence, is present in the induced culture, but not in the un induced control.

Acetohydroxy acid reductoisomerase activity in the cell free extracts is measured using the method described by Arfin and Umbarger (J. Biol. Chem. 244(5):1118-1127 (1969)).

### Example 7 (Prophetic)

### Cloning and Expression of Acetohydroxy Acid Dehydratase

The purpose of this prophetic Example is to describe how to clone the *ilvD* gene from *E. coli* K12 and express it in *E. coli* BL21-AI. The *ilvD* gene is amplified from *E. coli* genomic DNA using PCR.

The *ilvD* gene is cloned and expressed in the same manner as the *budB* gene described in Example 5. Genomic DNA from *E. coli* is prepared using the Gentra Puregene kit (Gentra Systems, Inc., Minneapolis, MN; catalog number D-5000A). The *ilvD* gene is amplified by PCR using primers N102 and N103 (see Table 2), given as SEQ ID NOs:15 and 16, respectively, creating a 1.9 kbp product. The forward primer incorporates four bases (CCAC) immediately adjacent to the translational start codon to allow directional cloning into pENTR/SD/D-TOPO (Invitrogen) to generate the plasmid pENTRSDD-TOPOilvD. Clones are submitted for sequencing to confirm that the genes are inserted in the correct orientation and to confirm the sequence. The nucleotide sequence of the open reading frame (ORF) for this gene and the predicted amino acid sequence of the enzyme are given as SEQ ID NO:5 and SEQ ID NO:6, respectively.

To create an expression clone, the *ilvD* gene is transferred to the pDEST 14 (Invitrogen) vector by recombination to generate pDEST14ilvD. The pDEST14ilvD vector is transformed into *E. coli* BL21-AI cells and expression from the T7 promoter is induced by addition of arabinose. A protein of the expected molecular weight of about 66 kDa, as deduced from the nucleic acid sequence, is present in the induced culture, but not in the uninduced control.

Acetohydroxy acid dehydratase activity in the cell free extracts is measured using the method described by Flint et al. (J. Biol. Chem. 268(20):14732-14742 (1993)).

### Example 8 (Prophetic)

### Cloning and Expression of Branched-Chain Keto Acid Decarboxylase

The purpose of this prophetic example is to describe how to clone the *kivD* gene from *Lactococcus lactis* and express it in *E. coli* BL21-AI.

A DNA sequence encoding the branched-chain keto acid decarboxylase *(kivD)* from *L. lactis* is obtained from GenScript (Piscataway, NJ). The sequence obtained is codon-optimized for expression in both *E. coli* and *B. subtilis* and is cloned into pUC57, to form pUC57-kivD. The codon-optimized nucleotide sequence of the open reading frame (ORF) for this gene and the predicted amino acid sequence of the enzyme are given as SEQ ID NO:7 and SEQ ID NO:8, respectively.

To create an expression clone *Ndel* and *BamHI* restriction sites are utilized to clone the 1.7 kbp kivD fragment from pUC57-kivD into vector pET-3a (Novagen, Madison, WI). This creates the expression clone pET-3a-kivD. The pET-3a-kivD vector is transformed into *E. coli* BL21-AI cells and expression from the T7 promoter is induced by addition of arabinose. A protein of the expected molecular weight of about 61 kDa, as deduced from the nucleic acid sequence, is present in the induced culture, but not in the uninduced control.

Branched-chain keto acid decarboxylase activity in the cell free extracts is measured using the method described by Smit et al. (Appl. Microbiol. Biotechnol. 64:396-402 (2003)).

### Example 9 (Prophetic)

### Cloning and Expression of Branched-Chain Alcohol Dehydrogenase

The purpose of this prophetic Example is to describe how to clone the *yqhD* gene from *E. coli* K12 and express it in *E. coli* BL21-AI. The *yqhD* gene is amplified from *E. coli* genomic DNA using PCR.

The *yqhD* gene is cloned and expressed in the same manner as the *budB* gene described in Example 5. Genomic DNA from *E. coli* is prepared using the Gentra Puregene kit (Gentra Systems, Inc., Minneapolis, MN; catalog number D-5000A). The *yqhD* gene is amplified by PCR using primers N104 and N105 (see Table 2), given as SEQ ID NOs:17 and 18, respectively, creating a 1.2 kbp product. The forward primer incorporates four bases (CCAC) immediately adjacent to the translational start codon to allow directional cloning into pENTR/SD/D-TOPO (Invitrogen) to generate the plasmid pENTRSDD-TOPOyqhD. Clones are submitted for sequencing to confirm that the genes are inserted in the correct orientation and to confirm the sequence. The nucleotide sequence of the open reading frame (ORF) for this gene and the predicted amino acid sequence of the enzyme are given as SEQ ID NO 9 and SEQ ID NO:10, respectively.

To create an expression clone, the *yqhD* gene is transferred to the pDEST 94 (Invitrogen) vector by recombination to generate pDEST14yqhD. The pDEST14ilvD vector is transformed into *E. coli* BL21-AI cells and expression from the T7 promoter is induced by addition of arabinose. A protein of the expected molecular weight of about 42 kDa, as deduced from the nucleic acid sequence, is present in the induced culture, but not in the uninduced control.

Branched-chain alcohol dehydrogenase activity in the cell free extracts is measured using the method described by Sulzenbacher et al. (J. Mol. Biol. 342(2):489-502 (2004)).

### Example 10 (Prophetic)

### Construction of a Transformation Vector for the Genes in an Isobutanol Biosynthetic Pathway

The purpose of this prophetic Example is to describe how to construct a transformation vector comprising the genes encoding the five steps in an isobutanol biosynthetic pathway. All genes are placed in a single operon under the control of a single promoter. The individual genes are amplified by PCR with primers that incorporate restriction sites for later cloning and the forward primers contain an optimized *E. coli* ribosome binding site (AAAGGAGG). PCR products are TOPO cloned into the pCR 4Blunt-TOPO vector and transformed into *E. coli* Top10 cells (Invitrogen). Plasmid DNA is prepared from the TOPO clones and the sequence of the genes is verified. Restriction enzymes and T4 DNA ligase (New England Biolabs, Beverly, MA) are used according to manufacturer's recommendations. For cloning experiments, restriction fragments are gel-purified using QIAquick Gel Extraction kit (Qiagen). After confirmation of the sequence, the genes are subcloned into a modified pUC19 vector as a cloning platform. The pUC19 vector is modified by HindIII/SapI digestion, creating pUC19dHS. The digest removes the lac promoter adjacent to the MCS (multiple cloning site), preventing transcription of the operons in the vector.

The *budB* gene is amplified from *K. pneumoniae* ATCC 25955 genomic DNA by PCR using primer pair N110 and N111 (see Table 2), given as SEQ ID NOs:19 and 20, respectively, creating a 1.8 kbp product. The forward primer incorporates SphI and AflII restriction sites and a ribosome binding site (RBS). The reverse primer incorporates Pacl and Nsil restriction sites. The PCR product is cloned into pCR4 Blunt-TOPO creating pCR4 Blunt-TOPO-budB. Plasmid DNA is prepared from the TOPO clones and the sequence of the gene is verified.

The *ilvc* gene is amplified from *E. coli* K12 genomic DNA by PCR using primer pair N112 and N113 (see Table 2) given as SEQ ID NOs:21 and 22, respectively, creating a 1.5 kbp product. The forward primer incorporates SalI and Nhel restriction sites and a RBS. The reverse primer incorporates a Xbal restriction site. The PCR product is cloned into pCR4 Blunt-TOPO creating pCR4 Blunt-TOPO-ilvC. Plasmid DNA is prepared from the TOPO clones and the sequence of the gene is verified.

The *ilvd* gene is amplified from *E. coli* K12 genomic DNA by PCR using primer pair N114 and N115 (see Table 2) given as SEQ ID NOs:23 and 24, respectively, creating a 1.9 kbp product. The forward primer incorporates a XbaI restriction site and a RBS. The reverse primer incorporates a BamHI restriction site. The PCR product is cloned into pCR4 Blunt-TOPO creating pCR4 Blunt-TOPO-ilvD. Plasmid DNA is prepared from the TOPO clones and the sequence of the gene is verified.

The *kivD* gene is amplified from pUC57-kivD (described in Example 8) by PCR using primer pair N116 and N117 (see Table 2), given as SEQ ID NOs:25 and 26, respectively, creating a 1.7 bp product. The forward primer incorporates a BamHI restriction site and a RBS. The reverse primer incorporates a SacI restriction site. The PCR product is cloned into pCR4 Blunt-TOPO creating pCR4 Blunt-TOPO-kivD. Plasmid DNA is prepared from the TOPO clones and the sequence of the gene is verified.

The *yqhD* gene is amplified from *E. coli* K12 genomic DNA by PCR using primer pair N118 and N119 (see Table 2) given as SEQ ID NOs:27 and 28, respectively, creating a 1.2 kbp product. The forward primer incorporates a Sacl restriction site. The reverse primer incorporates Spel and EcoRI restriction sites. The PCR product is cloned into pCR4 Blunt-TOPO creating pCR4 Blunt-TOPO-yqhD. Plasmid DNA is prepared from the TOPO clones and the sequence of the gene is verified.

To construct the isobutanol pathway operon, the yqhD gene is excised from pCR4 Blunt-TOPO-*yqhD* with SacI and EcoRI, releasing a 1.2 kbp fragment. This is ligated with pUC19dHS, which has previously been digested with SacI and EcoRI. The resulting clone, pUC19dHS-yqhD, is confirmed by restriction digest. Next, the *ilvC* gene is excised from pCR4 Blunt-TOPO-ilvC with SalI and Xbal, releasing a 1.5 kbp fragment. This is ligated with pUC19dHS-yqhD, which has previously been digested with SalI and XbaI. The resulting clone, pUC19dHS-ilvC-yqhD, is confirmed by restriction digest. The *budB* gene is then excised from pCR4 Blunt-TOPO-*budB* with SphI and Nsil, releasing a 1.8 kbp fragment. pUC19dHS-ilvC-yqhD is digested with SphI and Pstl and ligated with the SphI/NsiI *budB* fragment (Nsil and PstI generate compatible ends), forming pUC19dHS-budB-ilvC-yqhD. A 1.9 kbp fragment containing the *ilvD* gene is excised from pCR4 Blunt-TOPO-ilvD with Xbal and BamHI and ligated with pUC19dHS-budB-ilvC-yqhD, which is digested with these same enzymes, forming pUCI9dHS-budB-ilvC-ilvD-yqhD. Finally, *kivD* is excised from pCR4 Blunt-TOPO-kivD with BamHI and SacI, releasing a 1.7 kbp fragment. This fragment is ligated with pUC19dHS-budB-ilvC-ilvD-yqhD, which has previously been digested with BamHI and SacI, forming pUC19dHS-budB-ilvC-ilvD-kivD-yqhD.

The pUC19dHS-budB-ilvC-ilvD-kivD-yqhD vector is digested with AflII and Spel to release a 8.2 kbp operon fragment that is cloned into pBenAS, an *E.coli-B, subtilis* shuttle vector. Plasmid pBenAS is created by modification of the pBE93 vector, which is described by Nagarajan, (WO 93/24631, Example 4). To make pBenAS the *Bacillus amyloliquefaciens* neutral protease promoter (NPR), signal sequence, and the *phoA* gene are removed with a NcoI/HindIII digest of pBE93. The NPR promoter is PCR amplified from pBE93 by primers BenNF and BenASR, given as SEQ ID NOS:29 and 30, respectively. Primer BenASR incorporates AflII, Spel, and HindIII sites downstream of the promoter. The PCR product is digested with Ncol and HindIII and the fragment is cloned into the corresponding sites in the vector creating pBenAS. The operon fragment is subcloned into the Aflll and Spel sites in pBenAS creating pBen-budB-ilvC-ilvD-kivD-yqhD.

### EXAMPLE 11 (Prophetic)

### Expression of the Isobutanol Biosynthetic Pathway in E. coli

The purpose of this prophetic Example is to describe how to express an isobutanol biosynthetic pathway in *E. coli.*

The plasmid pBen-budB-ilvC-ilvD-kivD-yqhD, constructed as described in Example 10, is transformed into *E. coli* NM522 (ATCC No. 47000) to give *E. coli* strain NM522/pBen-budB-ilvC-ilvD-kivD-yqhD and expression of the genes in the operon is monitored by SDS-PAGE analysis, enzyme assay and Western blot analysis. For Western blots, antibodies are raised to synthetic peptides by Sigma-Genosys (The Woodlands, TX).

*E. coli* strain NM522/pBen-budB-ilvC-ilvD-kivD-yqhD is inoculated into a 250 mL shake flask containing 50 mL of medium and shaken at 250 rpm and 35 °C. The medium is composed of: glucose (5 g/L), MOPS (0.05 M), ammonium sulfate (0.01 M), potassium phosphate, monobasic (0.005 M), S10 metal mix (1 % (v/v)) yeast extract (0.1 % (w/v)), casamino acids (0.1 % (w/v)), thiamine (0.1 mg/L), proline (0.05 mg/L), and biotin (0.002 mg/L), and is titrated to pH 7.0 with KOH. S10 metal mix contains: MgCl₂ (200 mM), CaCl₂ (70 mM), MnCl₂ (5 mM), FeCl₃ (0.1 mM), ZnCl₂ (0.1 mM), thiamine hydrochloride (0.2 mM), CuSO₄ (172 µM), CoCl₂ (253 µM), and Na₂MoO₄ (242 µM). After 18 h, isobutanol is detected by HPLC or GC analysis, using methods that are well known in the art, for example, as described in the General Methods section above.

### EXAMPLE 12 (Prophetic)

### Expression of the Isobutanol Biosynthetic Pathway in Bacillus subtilis

The purpose of this prophetic Example is to describe how to express an isobutanol biosynthetic pathway in *Bacillus subtilis.* The same approach as described in Example 11 is used.

The plasmid pBen-budB-ilvC-ilvD-kivD-yqhD, constructed as described in Example 10, is used. This plasmid is transformed into *Bacillus subtilis* BE1010 *(*J. Bacteriol. 173:2278-2282 (1991)) to give B. *subtilis* strain BE1010/pBen-budB-ilvC-ilvD-kivD-yqhD and expression of the genes in each operon is monitored as described in Example 11.

*B. subtilis* strain BE1010/pBen-budB-ilvc-ilvD-kivD-yqhD is inoculated into a 250 mL shake flask containing 50 mL of medium and shaken at 250 rpm and 35 °C for 18 h. The medium is composed of: dextrose (5 g/L), MOPS (0.05 M), glutamic acid (0.02 M), ammonium sulfate (0.01 M), potassium phosphate, monobasic buffer (0.005 M), S10 metal mix (as described in Example 11, 1 % (v/v)), yeast extract (0.1 % (w/v)), casamino acids (0.1% (w/v)), tryptophan (50 mg/L), methionine (50 mg/L), and lysine (50 mg/L), and is titrated to pH 7.0 with KOH. After 18 h, isobutanol is detected by HPLC or GC analysis using methods that are well known in the art, for example, as described in the General Methods section above.

### EXAMPLE 13

### Cloning and Expression of Acetolactate Synthase

To create another acetolactate synthase expression clone, the *budB* gene was cloned into the vector pTrc99A. The *budB* gene was first amplified from pENTRSDD-TOPObudB (described in Example 5) using primers (N110.2 and N111.2, given as SEQ ID NOs:31 and 32, respectively) that introduced *Sacl, Spel* and *Mfe*I sites at the 5' end and *BbvC*I*, Afl*II*,* and *BamHI* sites at the 3' end. The resulting 1.75 kbp PCR product was cloned into pCR4-Blunt TOPO (Invitrogen) and the DNA sequence was confirmed (using N130Seq sequencing primers F1-F4 and R1-R4, given as SEQ ID NOs:40-47, respectively). The *budB* gene was then excised from this vector using *Sac*I and *BamH*I and cloned into pTrc99A (Amann et al. Gene 69(2):301-315 (1988)), generating pTrc99A::budB. The pTrc99A::budB vector was transformed into E. *coli* TOP10 cells and the transformants were inoculated into LB medium supplemented with 50 µg/mL of ampicillin and grown overnight at 37 °C. An aliquot of the overnight culture was used to inoculate 50 mL of LB medium supplemented with 50 µg/mL of ampicillin. The culture was incubated at 37 °C with shaking until the OD₆₀₀ reached 0.6 to 0.8. Expression of *budB* from the Trc promoter was then induced by the addition of 0.4 mM IPTG. Negative control flasks were also prepared that were not induced with IPTG. The flasks were incubated for 4 h at 37 °C with shaking. Cell-free extracts were prepared as described in Example 5.

Acetolactate synthase activity in the cell free extracts was measured as described in Example 5. Three hours after induction with IPTG, an acetolactate synthase activity of 8 units/mg was detected. The control strain carrying only the pTrc99A plasmid exhibited 0.03 units/mg of acetolactate synthase activity.

### EXAMPLE 14

### Cloning and Expression of Acetohydroxy Acid Reductoisomerase

The purpose of this Example was to clone the *ilvC* gene from E. *coli* K12 and express it in *E. coli* TOP10. The *ilvc* gene was amplified from *E. coli* K12 strain FM5 (ATCC 53911) genomic DNA using PCR.

The *ilvC* gene was cloned and expressed in a similar manner as described for the cloning and expression of *ilvc* in Example 6 above. PCR was used to amplify *ilvc* from the *E. coli* FM5 genome using primers N112.2 and N113.2 (SEQ ID NOs:33 and 34, respectively). The primers created *Sac*I and *Afl*III sites and an optimal RBS at the 5' end and *Not*I*, Nhel* and *BamHl* sites at the 3' end of *ilvC.* The 1.5 kbp PCR product was cloned into pCR4Blunt TOPO according to the manufacturer's protocol (Invitrogen) generating pCR4Blunt TOPO::ilvC. The sequence of the PCR product was confirmed using sequencing primers (N131SeqF1-F3, and N131SeqR1-R3, given as SEQ ID NOs:48-53, respectively). To create an expression clone, the *ilvC* gene was excised from pCR4Blunt TOPO::ilvC using SacI and *BamHl* and cloned into pTrc99A. The pTrc99A::ilvC vector was transformed into *E. coli* TOP 10 cells and expression from the Trc promoter was induced by addition of IPTG, as described in Example 13. Cell-free extracts were prepared as described in Example 5.

Acetohydroxy acid reductoisomerase activity in the cell free extracts was measured as described in Example 6. Three hours after induction with IPTG, an acetohydroxy acid reductoisomerase activity of 0.026 units/mg was detected. The control strain carrying only the pTrc99A plasmid exhibited less than 0.001 units/mg of acetohydroxy acid reductoisomerase activity.

### EXAMPLE 15

### Cloning and Expression of Acetohydroxy Acid Dehydratase

The purpose of this Example was to clone the *ilvD* gene from *E. coli* K12 and express it in *E. coli* Top10. The *ilvD* gene was amplified from *E. coli* K12 strain FM5 (ATCC 53911) genomic DNA using PCR.

The *ilvD* gene was cloned and expressed in a similar manner as the *ilvC* gene described in Example 14. PCR was used to amplify *ilvD* from the *E. coli* FM5 genome using primers N114.2 and N115.2 (SEQ ID NOs:35 and 36, respectively). The primers created SacI and NheI sites and an optimal RBS at the 5' end and BsU36I, PacI and *BamH*I sites at the 3' end of *ilvD.* The 1.9 kbp PCR product was cloned into pCR4Blunt TOPO according to the manufacturer's protocol (Invitrogen) generating pCR4Blunt TOPO::ilvD. The sequence of the PCR product was confirmed (sequencing primers N132SeqF1-F4 and N132SeqR1-R4, given as SEQ ID NOs:54-61, respectively). To create an expression clone, the *ilvD* gene was excised from plasmid pCR4Blunt TOPO::ilvD using SacI and *BamHI,* and cloned into pTrc99A. The pTrc99A::iivD vector was transformed into *E. coli TOP10* cells and expression from the Trc promoter was induced by addition of IPTG, as described in Example 13. Cell-free extracts were prepared as described in Example 5.

Acetohydroxy acid dehydratase activity in the cell free extracts was measured as described in Example 7. Three hours after induction with IPTG, an acetohydroxy acid dehydratase activity of 46 units/mg was measured. The control strain carrying only the pTrc99A plasmid exhibited no detectable acetohydroxy acid dehydratase activity.

### EXAMPLE 16

### Cloning and Expression of Branched-Chain Keto Acid Decarboxylase

The purpose of this Example was to clone the *kivD* gene from *Lactococcus lactis* and express it in *E. coli* TOP10.

The *kivD* gene was cloned and expressed in a similar manner as that described for *ilvc* in Example 14 above. PCR was used to amplify *kivD* from the plasmid pUC57-kivD (see Example 8, above) using primers N116.2 and N117.2 (SEQ ID NOs:37 and 38, respectively). The primers created *Sac*I and *Pac*I sites and an optimal RBS at the 5' end and *Pcil, Avr*II*, Bgl*II and *BamH*I sites at the 3' end of *kind.* The 1.7 kbp PCR product was cloned into pCR4Blunt TOPO according to the manufacturer's protocol (Invitrogen) generating pCR4Blunt TOPO::kivD. The sequence of the PCR product was confirmed using primers N133SeqF1-F4 and N133SeqR1-R4 (given as SEQ ID NOs:62-69, respectively). To create an expression clone, the *kivD* gene was excised from plasmid pCR4Blunt TOPO::kivD using Sacl and *BamH*I, and cloned into pTrc99A. The pTrc99A::kivD vector was transformed into *E. coli* TOP10 cells and expression from the Trc promoter was induced by addition of IPTG, as described in Example 13. Cell-free extracts were prepared as described in Example 5.

Branched-chain keto acid decarboxylase activity in the cell free extracts was measured as described in Example 8, except that Purpald® reagent (Aldrich, Catalog No. 162892) was used to detect and quantify the aldehyde reaction products. Three hours after induction with IPTG, a branched-chain keto acid decarboxylase activity of greater than 3.7 units/mg was detected. The control strain carrying only the pTrc99A plasmid exhibited no detectable branched-chain keto acid decarboxylase activity.

### EXAMPLE 17

### Expression of Branched-Chain Alcohol Dehydrogenase

*E. coli* contains a native gene *(yqhD)* that was identified as a 1,3-propanediol dehydrogenase (U.S. Patent No. 6,514,733). The YqhD protein has 40% identity to AdhB (encoded by *adhB)* from *Clostridium,* a putative NADH-dependent butanol dehydrogenase. The *yqhD* gene was placed under the constitutive expression of a variant of the glucose isomerase promoter 1.6G1 (SEQ ID NO. 70) in *E. coli strain* MG1655 1.6yqhD::Cm (WO 2004/033646) using X Red technology (Datsenko and Wanner, *Proc. Natl. Acad. Sci. U.S.A.* 97:6640 (2000)). MG1655 1.6yqhD::Cm contains a FRT-CmR-FRT cassette so that the antibiotic marker can be removed. Similarly, the native promoter was replaced by the 1.5GI promoter (WO 2003/089621) (SEQ ID NO. 71), creating strain MG1655 1.5G1-yqhD::Cm, thus, replacing the 1.6GI promoter of MG1655 1.6yqhD::Cm with the 1.5GI promoter.

Strain MG1655 1.5GI-yqhD::Cm was grown in LB medium to mid-log phase and cell free extracts were prepared as described in Example 5. This strain was found to have NADPH-dependent isobutyraldehyde reductase activity when the cell extracts were assayed by following the decrease in absorbance at 340 nm at pH 7.5 and 35 °C.

To generate a second expression strain containing 1.5GI yqhD::Cm, a P1 lysate was prepared from MG1655 1.5GI yqhD::Cm and the cassette was transferred to BL21 (DE3) (Invitrogen) by transduction, creating BL21 (DE3) 1.5G1-yqhD::Cm.

### EXAMPLE 18

### Construction of a Transformation Vector for the First Four Genes in an Isobutanol Biosynthetic Pathway

The purpose of this Example was to construct a transformation vector comprising the first four genes (i.e., *budB, ilvc, ilvD* and *kivD)* in an isobutanol biosynthetic pathway.

To construct the transformation vector, first, the *ilvC* gene was obtained from pTrc99A::iIvC (described in Example 14) by digestion with *Afl*II and *BamH*I and cloned into pTrc99A::budB (described in Example 13), which was digested with *Afl*II and *BamHI* to produce plasmid pTrc99A::budB-ilvC. Next, the *ilvD* and *kivD* genes were obtained from pTrc99A::ilvD (described in Example 15) and pTrc99A::kivD (described in Example 16), respectively, by digestion with *Nhe*I and Pacl *(ilvD)* and Pacl and *BamHI (kivD).* These genes were introduced into pTrc99A::budB-ilvC, which was first digested with NheI and *BamH*I*,* by three-way ligation. The presence of all four genes in the final plasmid, pTrc99A::budB-ilvC-ilvD-kivD, was confirmed by PCR screening and restriction digestion.

### EXAM PLE 1 9

### Expression of an Isobutanol Biosynthetic Pathway in E. coli Grown on Glucose

To create *E. coli* isobutanol production strains, pTrc99A::budB-ilvC-ilvD-kivD (described in Example 18) was transformed into *E. coli* MG1655 1.5GI yqhD::Cm and *E. coli* BL21 (DE3) 1.5GI yqhD::Cm (described in Example 17). Transformants were initially grown in LB medium containing 50 µg/mL kanamycin and 100 µg/mL carbenicillin. The cells from these cultures were used to inoculate shake flasks (approximately 175 mL total volume) containing 50 or 170 mL of TM3a/glucose medium (with appropriate antibiotics) to represent high and low oxygen conditions, respectively. TM3a/glucose medium contained (per liter): glucose (10 g), KH₂PO₄ (13.6 g), citric acid monohydrate (2.0 g), (NH₄)₂SO₄ (3-0 g), MgSO₄·7H₂O (2.0 g), CaCl₂·2H₂O (0.2 g), ferric ammonium citrate (0.33 g), thiamine·HCl (1.0 mg), yeast extract (0.50 g), and 10 mL of trace elements solution. The pH was adjusted to 6.8 with NH₄OH. The trace elements solution contained: citric acid ·H₂O (4.0 g/L), MnSO·H₂O (3.0 g/L), NaCl (1.0 g/L), FeSO₄·7H₂O (0.10 g/L), CoCl₂·6H₂O (0.10 g/L), ZnSO₄·7H₂O (0.10 g/L), CuSO₄·5H₂O (0.010 g/L), H₃BO₃ (0.010 g/L), and Na₂MoO₄· 2H₂O (0.010 g/L).

The flasks were inoculated at a starting OD₆₀₀ of ≤ 0.01 units and incubated at 34 °C with shaking at 300 rpm. The flasks containing 50 mL of medium were closed with 0.2 µm filter caps; the flasks containing 150 mL of medium were closed with sealed caps. IPTG was added to a final concentration of 0.04 mM when the cells reached an OD₆₀₀ of ≥ 0.4 units. Approximately 18 h after induction, an aliquot of the broth was analyzed by HPLC (Shodex Sugar SH1011 column (Showa Denko America, Inc. NY) with refractive index (RI) detection) and GC (Varian CP-WAX 58(FFAP) CB, 0.25 mm X 0.2 µm X 25 m (Varian, Inc., Palo Alto, CA) with flame ionization detection (FID)) for isobutanol content, as described in the General Methods section. No isobutanol was detected in control strains carrying only the pTrc99A vector (results not shown). Molar selectivities and titers of isobutanol produced by strains carrying pTrc99A::budB-ilvC-ilvD-kivD are shown in Table 13. Significantly higher titers of isobutanol were obtained in the cultures grown under low oxygen conditions.

**Table 13**

| Production of Isobutanol by *E. coli* Strains Grown on Glucose | | | |
|---|---|---|---|
| Strain | O₂ Conditions | Isobutanol mM* | Molar Selectivity (%) |
| MG1655 1.5GI yqhD/ pTrc99A::budB-ilvC-ilvD-kivD | High | 0.4 | 4.2 |
| MG1655 1.5GI yqhD/ pTrc99A::budB-ilvC-ilvD-kivD | Low | 9.9 | 39 |
| BL21 (DE3) 1.5GI yqhD/ pTrc99A::budB-ilvC-ilvD-kivD | High | 0.3 | 3.9 |
| BL21 (DE3)1.5GI yqhD/ pTrc99A::budB-ilvC-ilvD-kivD | Low | 1.2 | 12 |

| | | | |
|---|---|---|---|
| *Determined by HPLC. | | | |

### EXAMPLE 20

### Expression of an Isobutanol Biosynthetic Pathway in E. coli Grown on Sucrose

Since the strains described in Example 19 were not capable of growth on sucrose, an additional plasmid was constructed to allow utilization of sucrose for isobutanol production. A sucrose utilization gene cluster *cscBKA,* given as SEQ ID NO:39, was isolated from genomic DNA of a sucrose-utilizing *E. coli* strain derived from ATCC strain 13281. The sucrose utilization genes *(cscA, cscK, and cscB)* encode a sucrose hydrolase (CscA), given as SEQ ID NO:139, D-fructokinase (CscK), given as SEQ ID NO:140, and sucrose permease (CscB), given as SEQ ID NO:141. The sucrose-specific repressor gene *cscR* was not included so that the three genes *cscBKA* were expressed constitutively from their native promoters in *E. coli.*

Genomic DNA from the sucrose-utilizing *E. coli strain* was digested to completion with *BamHI* and *EcoRI.* Fragments having an average size of about 4 kbp were isolated from an agarose gel and were ligated to plasmid pLitmus28 (New England Biolabs), digested with *BamHI* and *EcoRI* and transformed into ultracompetent *E. coli* TOP10F' cells (Invitrogen). The transformants were streaked onto MacConkey agar plates containing 1% sucrose and ampicillin (100 µg/mL) and screened for the appearance of purple colonies. Plasmid DNA was isolated from the purple transformants, and sequenced with M13 Forward and Reverse primers (Invitrogen), and Scr1-4 (given as SEQ ID NOs:72-75, respectively). The plasmid containing *cscB, cscK,* and *cscA (cscBKA)* genes was designated pScr1.

To create a sucrose utilization plasmid that was compatible with the isobutanol pathway plasmid (Example 18), the operon from pScr1 was subcloned into pBHR1 (MoBiTec, Goettingen, Germany). The *cscBKA* genes were isolated by digestion of pScr1 with Xhol (followed by incubation with Klenow enzyme to generate blunt ends) and then by digestion with *Agel.* The resulting 4.2 kbp fragment was ligated into pBHR1 that had been digested with Nael and Agel, resulting in the 9.3 kbp plasmid pBHR1::cscBKA.

The sucrose plasmid pBHR1::cscBKA was transformed into *E. coli* BL21 (DE3) 1.5 yqhD /pTrc99A::budB-ilvc-ilvD-kivD and *E. coli* MG1655 1.5yqhD /pTrc99A::budB-ilvC-ilvD-kivD (described in Example 19) by electroporation. Transformants were first selected on LB medium containing 100 µg/mL ampicillin and 50 µg/mL kanamycin and then screened on MacConkey sucrose (1%) plates to confirm functional expression of the sucrose operon. For production of isobutanol, strains were grown in TM3a minimal defined medium (described in Example 19) containing 1% sucrose instead of glucose, and the culture medium was analyzed for the amount of isobutanol produced, as described in Example 19, except that samples were taken 14 h after induction. Again, no isobutanol was detected in control strains carrying only the pTrc99A vector (results not shown). Molar selectivities and titers of isobutanol produced by MG1655 1.5yqhD carrying pTrc99A::budB-ilvC-ilvD-kivD are shown in Table 14. Similar results were obtained with the analogous BL21 (DE3) strain.

**Table 14**

| Production of Isobutanol by *E. coli* strain MG1655 1.5yqhD /pTrc99A:: budB-ilvC-ilvD-kivD/PBHR1::cscBKA Grown on Sucrose | | | |
|---|---|---|---|
| O₂ Conditions | IPTG, mM | Isobutanol, mM* | Molar Selectivity, % |
| High | 0.04 | 0.17 | 2 |
| High | 0.4 | 1.59 | 21 |
| Low | 0.04 | 4.03 | 26 |
| Low | 0.4 | 3.95 | 29 |

| | | | |
|---|---|---|---|
| *Determined by HPLC. | | | |

### EXAMPLE 21

### Expression of Isobutanol Pathway Genes in Saccharomyces Cerevisiae

To express isobutanol pathway genes in *Saccharomyces cerevisiae,* a number of *E. coli*-yeast shuttle vectors were constructed. A PCR approach (Yu, et al. Fungal Genet. Biol. 41:973-981(2004)) was used to fuse genes with yeast promoters and terminators. Specifically, the GPD promoter (SEQ ID NO:76) and CYC1 terminator (SEQ ID NO:77) were fused to the *alsS* gene from *Bacillus subtilis* (SEQ ID NO:78), the FBA promoter (SEQ ID NO:79) and CYC1 terminator were fused to the ILV5 gene from *S. cerevisiae* (SEQ ID NO:80), the ADH1 promoter (SEQ ID NO:81) and ADH1 terminator (SEQ ID NO:82) were fused to the ILV3 gene from *S. cerevisiae* (SEQ ID NO:83), and the GPM promoter (SEQ ID NO:84) and ADH1 terminator were fused to the *kivD* gene from *Lactococcus lactis* (SEQ ID NO:7). The primers, given in Table 15, were designed to include restriction sites for cloning promoter/gene/terminator products into *E. coli*-yeast shuttle vectors from the pRS400 series (Christianson et al. Gene 110:119-122 (1992)) and for exchanging promoters between constructs. Primers for the 5' ends of ILV5 and ILV3 (N138 and N155, respectively, given as SEQ ID NOs: 95 and 107, respectively) generated new start codons to eliminate mitochondrial targeting of these enzymes.

All fused PCR products were first cloned into pCR4-Blunt by TOPO cloning reaction (Invitrogen) and the sequences were confirmed (using M13 forward and reverse primers (Invitrogen) and the sequencing primers provided in Table 15. Two additional promoters (CUP1 and GAL1) were cloned by TOPO reaction into pCR4-Blunt and confirmed by sequencing; primer sequences are indicated in Table 15. The plasmids that were constructed are described in Table 16. The plasmids were transformed into either *Saccharomyces cerevisiae* BY4743 (ATCC 201390) or YJR148w (ATCC 4036939) to assess enzyme specific activities using the enzyme assays described in Examples 5-8 and Examples 13-16. For the determination of enzyme activities, cultures were grown to an OD₆₀₀ of 1.0 in synthetic complete medium *(*Methods in Yeast Genetics, 2005, Cold Spring Harbor Laboratory Press, Cold Spring Harbor, NY, pp. 201-202) lacking any metabolite(s) necessary for selection of the expression plasmid(s), harvested by centrifugation (2600 x g for 8 min at 4 °C), washed with buffer, centrifuged again, and frozen at -80 °C. The cells were thawed, resuspended in 20 mM Tris-HCl, pH 8.0 to a final volume of 2 mL, and then disrupted using a bead beater with 1.2 g of glass beads (0.5 mm size). Each sample was processed on high speed for 3 minutes total (with incubation on ice after each minute of beating). Extracts were cleared of cell debris by centrifugation (20,000 x g for 10 min at 4 °C).

**Table 15**

| Primer Sequences for Cloning and Sequencing of *S. cerevisiae* Expression Vectors | | | |
|---|---|---|---|
| Name | Sequence | Description | SEQ ID NO: |
| N98SeqF1 | CGTGTTAGTCACATCAGGA C | *B. subtilis alsS* sequencing primer | 85 |
| N98SeqF2 | GGCCATAGCAAAAATCCAA ACAGC | B. subtilis alsS sequencing primer | 86 |
| N98SeqF3 | CCACGATCAATCATATCGA ACACG | B. subtilis alsS sequencing primer | 87 |
| N98SeqF4 | GGTTTCTGTCTCTGGTGAC G | B. subtilis alsS sequencing primer | 88 |
| N99SeqR1 | GTCTGGTGATTCTACGCGC AAG | B. subtilis alsS sequencing primer | 89 |
| N99SeqR2 | CATCGACTGCATTACGCAA CTC | B. subtilis alsS sequencing primer | 90 |
| N99SeqR3 | CGATCGTCAGAACAACATC TGC | B. subtilis alsS sequencing primer | 91 |
| N99SeqR4 | CCTTCAGTGTTCGCTGTCA G | B. subtilis alsS sequencing primer | 92 |
| N136 | CCGCGGATAGATCTGAAAT GAATAACAATACTGACA | FBA promoter forward primer with SacII/BgIII sites | 93 |
| N137 | TACCACCGAAGTTGATTTG CTTCAACATCCTCAGCTCT AGATTTGAATATGTATTACT TGGTTAT | FBA promoter reverse primer with BbvCl site and ILV5-annealing region | 94 |
| N138 | ATGTTGAAGCAAATCAACT TCGGTGGTA | ILV5 forward primer (creates alternate start codon) | 95 |
| N139 | TTATTGGTTTTCTGGTCTCA AC | ILV5 reverse primer | 96 |
| N140 | AAGTTGAGACCAGAAAACC AATAATTAATTAATCATGTA ATTAGTTATGTCACGCTT | CYC terminator forward primer with Pacl site and ILV5-annealing region | 97 |
| N141 | GCGGCCGCCCGCAAATTA AAGCCTTCGAGC | CYC terminator reverse primer with Notl site | 98 |
| N142 | GGATCCGCATGCTTGCATT TAGTCGTGC | GPM promoter forward primer with BamHI site | 99 |
| N143 | CAGGTAATCCCCCACAGTA TACATCCTCAGCTATTGTA ATATGTGTGTTTGTTTGG | GPM promoter reverse primer with BbvCl site and kivD-annealing region | 100 |
| N144 | ATGTATACTGTGGGGGATT ACC | kivD forward primer | 101 |
| N145 | TTAGCTTTTATTTTGCTCCG CA | kivD reverse primer | 102 |
| N146 | TTTGCGGAGCAAAATAAAA GCTAATTAATTAAGAGTAA GCGAATTTCTTATGATTTA | ADH terminator forward primer with Pacl site and kivD-annealing region | 103 |
| N147 | ACTAGTACCACAGGTGTTG TCCTCTGAG | ADH terminator reverse primer with Spel site | 104 |
| N151 | CTAGAGAGCTTTCGTTTTC ATG | alsS reverse primer | 105 |
| N152 | CTCATGAAAACGAAAGCTC TCTAGTTAATTAATCATGTA ATTAGTTATGTCACGCTT | CYC terminator forward primer with Pacl site and alsS-annealing region | 106 |
| N155 | ATGGCAAAGAAGCTCAACA AGTACT | ILV3 forward primer (alternate start codon) | 107 |
| N156 | TCAAGCATCTAAAACACAA CCG | ILV3 reverse primer | 108 |
| N157 | AACGGTTGTGTTTTAGATG | ADH terminator | 109 |
| | CTTGATTAATTAAGAGTAA GCGAATTTCTTATGATTTA | forward primer with Pacl site and ILV3-annealing region | |
| N158 | GGATCCTTTTCTGGCAACC AAACCCATA | ADH promoter forward primer with BamHI site | 110 |
| N159 | CGAGTACTTGTTGAGCTTC TTTGCCATCCTCAGCGAGA TAGTTGATTGTATGCTTG | ADH promoter reverse primer with BbvCl site and ILV3-annealing region | 111 |
| N160SeqF1 | GAAAACGTGGCATCCTCTC | FBA::ILV5::CYC sequencing primer | 112 |
| N160SeqF2 | GCTGACTGGCCAAGAGAA A | FBA::ILV5::CYC sequencing primer | 113 |
| N160SeqF3 | TGTACTTCTCCCACGGTTT C | FBA::ILV5::CYC sequencing primer | 114 |
| N160SeqF4 | AGCTACCCAATCTCTATAC CCA | FBA:.ILV5::CYC sequencing primer | 115 |
| N160SeqF5 | CCTGAAGTCTAGGTCCCTA TTT | FBA::ILV5::CYC sequencing primer | 116 |
| N160SeqR1 | GCGTGAATGTAAGCGTGA C | FBA::ILV5::CYC sequencing primer | 117 |
| N160SeqR2 | CGTCGTATTGAGCCAAGAA C | FBA::ILV5::CYC sequencing primer | 118 |
| N160SeqR3 | GCATCGGACAACAAGTTCA T | FBA::ILV5::CYC sequencing primer | 119 |
| N160SeqR4 | TCGTTCTTGAAGTAGTCCA ACA | FBA:.ILV5::CYC sequencing primer | 120 |
| N160SeqR5 | TGAGCCCGAAAGAGAGGA T | FBA::ILV5::CYC sequencing primer | 121 |
| N161SeqF1 | ACGGTATACGGCCTTCCTT | ADH::ILV3::ADH sequencing primer | 122 |
| N161SeqF2 | GGGTTTGAAAGCTATGCAG T | ADH::ILV3::ADH sequencing primer | 123 |
| N161SeqF3 | GGTGGTATGTATACTGCCA ACA | ADH::ILV3::ADH sequencing primer | 124 |
| N161SeqF4 | GGTGGTACCCAATCTGTGA TTA | ADH.:ILV3.:ADH sequencing primer | 125 |
| N161SeqF5 | CGGTTTGGGTAAAGATGTT G | ADH::ILV3::ADH sequencing primer | 126 |
| N161SeqF6 | AAACGAAAATTCTTATTCTT GA | ADH::ILV3::ADH sequencing primer | 127 |
| N161SeqR1 | TCGTTTTAAAACCTAAGAG TCA | ADH::ILV3::ADH sequencing primer | 128 |
| N161SeqR2 | CCAAACCGTAACCCATCAG | ADH::ILV3::ADH sequencing primer | 129 |
| N161SeqR3 | CACAGATTGGGTACCACCA | ADH::ILV3::ADH sequencing primer | 130 |
| N161SeqR4 | ACCACAAGAACCAGGACCT G | ADH::ILV3::ADH sequencing primer | 131 |
| N161SeqR5 | CATAGCTTTCAAACCCGCT | ADH::ILV3::ADH sequencing primer | 132 |
| N161SeqR6 | CGTATACCGTTGCTCATTA GAG | ADH::ILV3::ADH sequenceing primer | 133 |
| N162 | ATGTTGACAAAAGCAACAA AAGA | *alsS* forward primer | 134 |
| N189 | ATCCGCGGATAGATCTAGT TCGAGTTTATCATTATCAA | GPD forward primer with *Sac*II/*Bg*/II sites | 135 |
| N190.1 | TTCTTTTGTTGCTTTTGTCA ACATCCTCAGCGTTTATGT GTGTTTATTCGAAA | GPD promoter reverse primer with *BbvC*I site and *alsS-*annealing region | 136 |
| N176 | ATCCGCGGATAGATCTATT AGAAGCCGCCGAGCGGGC G | GAL1 promoter forward primer with *SacI*I/*Bg*/II sites | 137 |
| N177 | ATCCTCAGCTTTTCTCCTT GACGTTAAAGTA | GAL1 promoter reverse with *BbvC*I site | 138 |
| N191 | ATCCGCGGATAGATCTCCC ATTACCGACATTTGGGCGC | CUP1 promoter forward primer with SacII/BgIII sites | 175 |
| N192 | ATCCTCAGCGATGATTGAT TGATTGATTGTA | CUP1 promoter reverse with BbvCl site | 176 |

**Table 16**

| *E. coli*-Yeast Shuttle Vectors Carrying Isobutanol Pathway Genes | |
|---|---|
| Plasmid Name | Construction |
| pRS426 [ATCC No. 77107], URA3 selection | - |
| pRS426::GPD::alsS::CYC | GPD::alsS::CYC PCR product digested with *Sac*II/*No*tI cloned into pRS426 digested with same |
| pRS426::FBA::ILV5::CYC | FBA::ILV5::CYC PCR product digested with *Sac*II/*Not*I cloned into pRS426 digested with same |
| pRS425 [ATCC No. 77106], LEU2 selection | - |
| pRS425::ADH::ILV3::ADH | ADH::ILV3::ADH PCR product digested with *BamH*I/*Spe*I cloned into pRS425 digested with same |
| pRS425::GPM::kivD::ADH | GPM::kivD::ADH PCR product digested with *BamHI*/*SpeI* cloned into pRS425 digested with same |
| pRS426::CUP1::alsS | 7.7 kbp *SacI*I/*BbvCI* fragment from pRS426::GPD::alsS::CYC ligated with SacII/BbvCI CUP1 fragment |
| pRS426::GAL1::ILV5 | 7 kbp *SacII*/*BbvC*I fragment from pRS42B::FBA::ILV5::CYC ligated with *SacII*/*BbvC*I GAL1 fragment |
| pRS425::FBA::ILV3 | 8.9 kbp *BamHI*/*BbvC*I fragment from pRS425::ADH::ILV3::ADH ligated with 0.65 kbp *Bgl*II/*Bbv*CI FBA fragment from pRS426::FBA::ILV5::CYC |
| pRS425::CUP1-alsS+FBA-ILV3 | 2.4 kbp *Sac*II/NotI fragment from pRS426::CUP1::alsS cloned into pRS425::FBA::ILV3 cut with *Sac*II/*Not*I |
| pRS426::FBA-ILV5+GPM-kivD | 2.7 kbp *Bam*HI/*Spe*I fragment from pRS425::GPM::kivD::ADH cloned into pRS426::FBA::ILV5::CYC cut with *Bam*HI/*Spe*I |
| pRS426::GAL1-FBA+GPM-kivD | 8.5 kbp *Sac*II/*Not*I fragment from pRS426:: FBA-ILV5+GPM-kivD ligated with 1.8 kbp SacII/NotI fragment from pR5426::GAL1::ILV5 |
| pRS423 [ATCC No. 77104], HIS3 selection | - |
| pRS423::CUP1-alsS+FBA-ILV3 | 5.2 kbp *Sac*I/*Sa*/I fragment from pRS425::CUP1-alsS+FBA-ILV3 ligated into pRS423 cut with *Sac*I/*Sa*/I |
| pHR81[ATCC No. 87541], URA3 and leu2-d selection | - |
| pHR81::FBA-ILV5+GPM-kivD | 4.7 kbp *Sac*I/*BamH*I fragment from pRS426::FBA-ILV5+GPM-kivD ligated into pHR81 cut with *Sac*I/*BamH*I |

### EXAMPLE 22

### Production of Isobutanol by Recombinant Saccharomyces Cerevisiae

Plasmids pRS423::CUP1-alsS+FBA-ILV3 and pHR81::FBA-ILV5+GPM-kivD (described in Example 21) were transformed into *Saccharomyces cerevisiae* YJR148w to produce strain YJR148w/pRS423::CUP1-alsS+FBA-ILV3/ pHR81::FBA-ILV5+ GPM-kivD. A control strain was prepared by transforming vectors pRS423 and pHR81 (described in Example 21) into *Saccharomyces cerevisiae* YJR148w (strain YJR148w/pRS423/pHR81). Strains were maintained on standard *S. cerevisiae* synthetic complete medium (Methods in Yeast Genetics, 2005, Cold Spring Harbor Laboratory Press, Cold Spring Harbor, NY, pp. 201-202) containing either 2% glucose or sucrose but lacking uracil and histidine to ensure maintenance of plasmids.

For isobutanol production, cells were transferred to synthetic complete medium lacking uracil, histidine and leucine. Removal of leucine from the medium was intended to trigger an increase in copy number of the pHR81-based plasmid due to poor transcription of the leu2-d allele (Erhart and Hollenberg, J. Bacteriol. 156:625-535 (1983)). Aerobic cultures were grown in 175 mL capacity flasks containing 50 mL of medium in an Innova4000 incubator (New Brunswick Scientific, Edison, NJ) at 30 °C and 200 rpm. Low oxygen cultures were prepared by adding 45 mL of medium to 60 mL serum vials that were sealed with crimped caps after inoculation and kept at 30 °C. Sterile syringes were used for sampling and addition of inducer, as needed. Approximately 24 h after inoculation, the inducer CuSO₄ was added to a final concentration of 0.03 mM. Control cultures for each strain without CuSO₄ addition were also prepared. Culture supernatants were analyzed 18 or 19 h and 35 h after CuSO₄ addition by both GC and HPLC for isobutanol content, as described above in Example 19. The results for *S. cerevisiae* YJR148w/pRS423::CUP1-alsS+FBA-ILV3/pHR81::FBA-ILV5+GPM-kivD grown on glucose are presented in Table 17. For the results given in Table 17, the samples from the aerobic cultures were taken at 35 h and the samples from the low oxygen cultures were taken at 19 h and measured by HPLC.

The results for *S. cerevisiae* YJR148w/pRS423::CUP1-alsS+FBA-ILV3/pHR81::FBA-ILV5+GPM-kivD grown on sucrose are presented in Table 18. The results in this table were obtained with samples taken at 18 h and measured by HPLC.

**Table 17**

| Production of Isobutanol by *S. cerevisiae* YJR148w/pRS423::CUP1-alsS+FBA-ILV3/pHR81::FBA-ILV5+GPM-kivD Grown on Glucose | | | |
|---|---|---|---|
| Strain | O₂ level | Isobutanol, mM | Molar Selectivity % |
| YJR148w/pRS423/pHR81 (control) | Aerobic | 0.12 | 0.04 |
| YJR148w/pRS423/pHR81 (control) | Aerobic | 0.11 | 0.04 |
| YJR148w/pRS423::CUP1-alsS+FBA- | Aerobic | 0.97 | 0.34 |
| ILV3/pHR81::FBA-ILV5+ GPM-kivD a | | | |
| YJR148w/pRS423::CUP1-alsS+FBA-ILV3/pHR81::FBA-ILV5+GPM-kivD b | Aerobic | 0.93 | 0.33 |
| YJR148w/pRS423::CUP1-alsS+FBA-ILV3/ pHR81::FBA-ILV5+ GPM-kivD c | Aerobic | 0.85 | 0.30 |
| YJR148w/pRS423/pHR81 (control) | Low | 0.11 | 0.1 |
| YJR148w/pRS423/pHR81 (control) | Low | 0.08 | 0.1 |
| YJR148w/pRS423::CUP1-alsS+FBA-ILV3/ pHR81::FBA-ILV5+ GPM-kivD a | Low | 0.28 | 0.5 |
| YJR148w/pRS423::CUP1-alsS+FBA-ILV3/pHR81::FBA-ILV5+GPM-kivD b | Low | 0.20 | 0.3 |
| YJR148w/pRS423::CUP1-alsS+FBA-ILV3/ pHR81::FBA-ILV5+ GPM-kivD c | Low | 0.33 | 0.6 |

| Table 18 | | | |
|---|---|---|---|
| Production of Isobutanol by *S. cerevisiae* YJR148w/pRS423::CUP1-alsS+FBA-ILV3/pHR81::FBA-ILV5+GPM-kivD Grown on Sucrose | | | |
| Strain | O₂ Level | Isobutanol mM | Molar Selectivity, % |
| YJR148w/pRS423/pHR81 (control) | Aerobic | 0.32 | 0.6 |
| YJR148w/pRS423/pHR81 (control) | Aerobic | 0.17 | 0.3 |
| YJR148w/pRS423::CUP1-alsS+FBA-ILV3/ pHR81::FBA-ILV5+ GPM-kivD a | Aerobic | 0.68 | 1.7 |
| YJR148w/pRS423::CUP1-alsS+FBA-ILV3/ pHR81::FBA-ILV5+ GPM-kivD b | Aerobic | 0.54 | 1.2 |
| YJR148w/pRS423::CUP1-alsS+FBA-ILV3/ pHR81::FBA-ILV5+ GPM-kivD c | Aerobic | 0.92 | 2.0 |
| YJR148w/pRS423/pHR81 (control) | Low | 0.18 | 0.3 |
| YJR148w/pRS423/pHR81 (control) | Low | 0.15 | 0.3 |
| YJR148w/pRS423::CUP1-alsS+FBA-ILV3/ pHR81::FBA-ILV5+ GPM-kivD a | Low | 0.27 | 1.2 |
| YJR148w/pRS423::CUP1-alsS+FBA-ILV3/ pHR81::FBA-ILV5+ GPM-kivD b | Low | 0.30 | 1.1 |
| YJR148w/pRS423::CUP1-alsS+FBA-ILV3/ pHR81::FBA-ILV5+ GPM-kivD c | Low | 0.21 | 0.8 |

| | | | |
|---|---|---|---|
| Strain suffixes "a", "b", and "c" indicate separate isolates. | | | |

The results indicate that, when grown on glucose or sucrose under both aerobic and low oxygen conditions, strain YJR148w/pRS423::CUP1-alsS+FBA-ILV3/ pHR81::FBA-ILV5+ GPM-kivD produced consistently higher levels of isobutanol than the control strain.

### EXAMPLE 23

### Production of Isobutanol by Recombinant Saccharomyces Cerevisiae

Plasmids pRS425::CUP1-alsS+FBA-ILV3 and pRS426::GAL1-ILV5+GPM-kivD (described in Example 21) were transformed into *Saccharomyces cerevisiae* YJR148w to produce strain YJR148w/pRS425::CUP1-alsS+ FBA-ILV3/pRS426::GAL1-ILV5+ GPM-kivD. A control strain was prepared by transforming vectors pRS425 and pRS426 (described in Example 21) into *Saccharomyces cerevisiae* YJR148w (strain YJR148w/pRS425/pRS426). Strains were maintained on synthetic complete medium, as described in Example 22.

For isobutanol production, cells were transferred to synthetic complete medium containing 2% galactose and 1% raffinose, and lacking uracil and leucine. Aerobic and low oxygen cultures were prepared as described in Example 22. Approximately 12 h after inoculation, the inducer CuSO₄ was added up to a final concentration of 0.5 mM. Control cultures for each strain without CuSO₄ addition were also prepared. Culture supernatants were sampled 23 h after CuSO₄ addition for determination of isobutanol by HPLC, as described in Example 22. The results are presented in Table 19. Due to the widely different final optical densities observed and associated with quantifying the residual carbon source, the concentration of isobutanol per OD₆₀₀ unit (instead of molar selectivities) is provided in the table to allow comparison of strains containing the isobutanol biosynthetic pathway genes with the controls.

**Table 19**

| Production of Isobutanol by *S. cerevisiae* YJR148w/pRS425::CUP1-alsS+FBA-ILV3/pRS426::GAL1-ILV5+GPM-kivD Grown on Galactose and Raffinose | | | | |
|---|---|---|---|---|
| Strain | O₂ level | CuSO₄, mM | Isobutanol mM | mM Isobutanol per OD unit |
| YJR148w/pRS425/pRS426 (control) | Aerobic | 0.1 | 0.12 | 0.01 |
| YJR148w/pRS425/pRS426 (control) | Aerobic | 0.5 | 0.13 | 0.01 |
| YJR148w/pRS425::CUP1-alsS+ FBA-ILV3/pRS426::GAL1-ILV5+ GPM-kivD a | Aerobic | 0 | 0.20 | 0.03 |
| YJR148w/pRS425::CUP1-alsS+ FBA-ILV3/pRS426::GAL1-ILV5+ GPM-kivD b | Aerobic | 0.03 | 0.82 | 0.09 |
| YJR148w/pRS425::CUP1-alsS+ FBA-ILV3/pRS426::GAL1-ILV5+ GPM-kivD c | Aerobic | 0.1 | 0.81 | 0.09 |
| YJR148w/pRS425::CUP1-alsS+ FBA-ILV3/pRS426::GAL1-ILV5+ GPM-kivD d | Aerobic | 0.5 | 0.16 | 0.04 |
| YJR148w/pRS425::CUP1-alsS+ . FBA-ILV3/pRS426::GAL1-ILV5+ GPM-kivD e | Aerobic | 0.5 | 0.18 | 0.01 |
| YJR148w/pRS425/pRS426 (control) | Low | 0.1 | 0.042 | 0.007 |
| YJR148w/pRS425/pRS426 (control) | Low | 0.5 | 0.023 | 0.006 |
| YJR148w/pRS425::CUP1-alsS+ FBA-ILV3/pRS426::GAL1-ILV5+ GPM-kivD a | Low | 0 | 0.1 | 0.04 |
| YJR148w/pRS425::CUP1-alsS+ | Low | 0.03 | 0.024 | 0.02 |
| FBA-ILV3/pRS426::GAL1-ILV5+ GPM-kivD b | | | | |
| YJR148w/pRS425::CUP1-alsS+ FBA-ILV3/pRS426::GAL1-ILV5+ GPM-kivD c | Low | 0.1 | 0.030 | 0.04 |
| YJR148w/pRS425::CUP1-alsS+ FBA-ILV3/pRS426::GAL1-ILV5+ GPM-kivD d | Low | 0.5 | 0.008 | 0.02 |
| YJR148w/pRS425.:CUP1-alsS+ FBA-ILV3/pRS426::GAL1-ILV5+ GPM-kivD e | Low | 0.5 | 0.008 | 0.004 |

| | | | | |
|---|---|---|---|---|
| Strain suffixes "a", "b", "c", "d" and "e" indicate separate isolates. | | | | |

The results indicate that in general, higher levels of isobutanol per optical density unit were produced by the YJR148w/pRS425::CUP1-alsS+ FBA-ILV3/pRS426::GAL1-ILV5+ GPM-kivD strain compared to the control strain under both aerobic and low oxygen conditions.

### EXAMPLE 24

### Expression of an Isobutanol Biosynthetic Pathway in Bacillus subtilis

The purpose of this Example was to express an isobutanol biosynthetic pathway in *Bacillus subtilis.* The five genes of the isobutanol pathway (pathway steps (a) through (e) in Figure 1) were split into two operons for expression. The three genes *budB, ilvD,* and *kivD,* encoding acetolactate synthase, acetohydroxy acid dehydratase, and branched-chain keto acid decarboxylase, respectively, were integrated into the chromosome of *B. subtilis* BE1010 (Payne and Jackson, J. Bacteriol. 173:2278-2282 (1991)). The two genes *ilvC* and *bdhB,* encoding acetohydroxy acid isomeroreductase and butanol dehydrogenase, respectively, were cloned into an expression vector and transformed into the *Bacillus* strain carrying the integrated isobutanol genes.

Integration of the three genes, *budB, ilvD* and *kivD* into the chromosome of *B. subtilis* BE1010. *Bacillus* integration vectors pFP988DssPspac and pFP988DssPgroE were used for the chromosomal integration of the three genes, *budB* (SEQ ID NO:1), *ilvD* (SEQ ID NO:5), and *kivD* (SEQ ID NO:7). Both plasmids contain an *E. coli* replicon from pBR322, an ampicillin antibiotic marker for selection in E. *coli* and two sections of homology to the *sacB* gene in the *Bacillus* chromosome that direct integration of the vector and intervening sequence by homologous recombination. Between the *sacB* homology regions is a spac promoter (PgroE) on pFP988DssPspac or a groEL promoter (PgroE) on pFP988DssPgroE, and a selectable marker for *Bacillus,* erythramycin. The promoter region also contains the *lacO* sequence for regulation of expression by a lacI repressor protein. The sequences of pFP988DssPspac (6,341 bp) and pFP988DssPgroE (6,221 bp) are given as SEQ ID NO:142 and SEQ ID NO:143 respectively.

The cassette with three genes *budB-ilvD-kivD* was constructed by deleting the *ilvC* gene from plasmid pTrc99a budB-ilvC-ilvD-kivD. The construction of the plasmid pTrc99A::budB-ilvC-ilvD-kivD is described in Example 18. Plasmid pTrc99A::budB-ilvC-ilvD-kivD was digested with AfIII and Nhel, treated with the Klenow fragment of DNA polymerase to make blunt ends, and the resulting 9.4 kbp fragment containing pTrc99a vector, *budB, ilvD,* and *kivD* was gel-purified. The 9.4 kbp vector fragment was self-ligated to create pTrc99A::budB-ilvD-kivD, and transformed into DH5α competent cells (Invitrogen). A clone of pTrc99a budB-ilvD-kivD was confirmed for the *ilvC* gene deletion by restriction mapping. The resulting plasmid pTrc99A::budB-ilvD-kivD was digested with Sacl and treated with the Klenow fragment of DNA polymerase to make blunt ends. The plasmid was then digested with BamHI and the resulting 5,297 bp *budB-ilvD-kivD* fragment was gel-purified. The 5,297 bp *budB-ilvD-kivD* fragment was ligated into the Smal and BamHI sites of the integration vector pFP988DssPspac. The ligation mixture was transformed into DH5α competent cells. Transformants were screened by PCR amplification of the 5.3 kbp *budB-ilvD-kivD* fragment with primers T-budB(BamHI) (SEQ ID NO:144) and B-kivD(BamHI) (SEQ ID NO:145). The correct clone was named pFP988DssPspac-budB-ilvD-kivD.

Plasmid pFP988DssPspac-budB-ilvD-kivD was prepared from the *E. coli* transformant, and transformed into *B. subtilis* BE1010 competent cells, which had been prepared as described by Doyle et al. (J. Bacteriol. 144:957 (1980)). Competent cells were harvested by centrifugation and the cell pellets were resuspended in a small volume of the supernatant. To one volume of competent cells, two volumes of SPII-EGTA medium *(*Methods for General and Molecular Bacteriology, P. Gerhardt et al., Ed., American Society for Microbiology, Washington, DC (1994)) was added. Aliquots (0.3 mL) of cells were dispensed into test tubes and then 2 to 3 µg of plasmid pFP988DssPspac-budB-ilvD-kivD was added to the tubes. The tubes were incubated for 30 min at 37 °C with shaking, after which 0.1 mL of 10% yeast extract was added to each tube and they were further incubated for 60 min. Transformants were grown for selection on LB plates containing erythromycin (1.0 µg/mL) using the double agar overlay method *(Methods for General and Molecular Bacteriology, supra).* Transformants were screened by PCR amplification with primers N130SeqF1 (SEQ ID NO:40) and N130SeqR1 (SEQ ID NO:44) for *budB,* and N133SeqF1 (SEQ ID NO:62) and N133SeqR1 (SEQ ID NO:66) for *kivD.* Positive integrants showed the expected 1.7 kbp *budB* and 1.7 kbp *kivD* PCR products. Two positive integrants were identified and named B. *subtilis* BE1010 ΔsacB::Pspac-budB-ilvD-kivD #2-3-2 and *B. subtilis* BE1010 ΔsacB::Pspac-budB-ilvD-kivD #6-12-7.

Assay of the enzyme activities in integrants *B. subtilis* BE1010 ΔsacB::Pspac-budB-ilvD-kivD #2-3-2 and *B. subtilis* BE1010 ΔsacB::Pspac-budB-ilvD-kivD #6-12-7 indicated that the activities of BudB, IlvD and KivD were low under the control of the spac promoter (Pspac). To improve expression of functional enzymes, the Pspac promoter was replaced by a PgroE promoter from plasmid pHT01 (MoBitec, Goettingen, Germany).

A 6,039 bp pFP988Dss vector fragment, given as SEQ ID NO:146, was excised from an unrelated plasmid by restriction digestion with Xhol and BamHI, and was gel-purified. The PgroE promoter was PCR-amplified from plasmid pHT01 with primers T-groE(Xhol) (SEQ ID NO:147) and B-groEL(SpeI,BamHI) (SEQ ID NO:148). The PCR product was digested with Xhol and BamHI, ligated with the 6,039 bp pFP988Dss vector fragment, and transformed into DH5α competent cells. Transformants were screened by PCR amplification with primers T-groE(Xhol) and B-groEL(SpeI,BamHI). Positive clones showed the expected 174 bp PgroE PCR product and were named pFP988DssPgroE. The plasmid pFP988DssPgroE was also confirmed by DNA sequence.

Plasmid pFP988DssPspac-budB-ilvD-kivD was digested with SpeI and Pmel and the resulting 5,313 bp budB-ilvD-kivD fragment was gel-purified. The budB-ilvD-kivD fragment was ligated into Spel and Pmel sites of pFP988DssPgroE and transformed into DH5α competent cells. Positive clones were screened for a 1,690 bp PCR product by PCR amplification with primers T-groEL (SEQ ID NO:149) and N111 (SEQ ID NO:20). The positive clone was named pFP988DssPgroE-budB-ilvD-kivD.

Plasmid pFP988DssPgroE-budB-ilvD-kivD was prepared from the *E. coli* transformant, and transformed into *Bacillus subtilis* BE1010 competent cells as described above. Transformants were screened by PCR amplification with primers N130SeqF1 (SEQ ID NO:40) and N130SeqR1 (SEQ ID NO:44) for *budB,* and N133SeqF1 (SEQ ID NO:62) and N133SeqR1 (SEQ ID NO:66) for *kivD.* Positive integrants showed the expected 1.7 kbp *budB* and 1.7 kbp *kivD* PCR products. Two positive integrants were isolated and named *B. subtilis* BE1010 ΔsacB::PgroE-budB-ilvD-kivD #1-7 and *B. subtilis* BE1010 ΔsacB::PgroE-budB-ilvD-kivD #8-16.

Plasmid Expression of *ilvC* and *bdhB* genes. Two remaining isobutanol genes, *ilvC* and *bdhB,* were expressed from a plasmid. Plasmid pHT01 (MoBitec), a *Bacillus-E. coli* shuttle vector, was used to fuse an *ilvC* gene from *B. subtilis* to a PgroE promoter so that the *ilvC* gene was expressed from the PgroE promoter containing a lacO sequence. The *ilvC* gene, given as SEQ ID NO:186, was PCR-amplified from *B. subtilis* BR151 (ATCC 33677) genomic DNA with primers T-ilvCB.s.(BamHI) (SEQ ID NO:150) and B-ilvCB.s.(SpeI BamHI) (SEQ ID NO:151). The 1,067 bp *ilvC* PCR product was digested with BamHI and ligated into the BamHI site of pHT01. The ligation mixture was transformed into DH5α competent cells. Positive clones were screened for a 1,188 bp PCR product by PCR amplification with primers T-groEL and B-ilvB.s.(SpeI BamHI). The positive clone was named pHT01-ilvC(B.s). Plasmid pHT01-ilvC(B.s) was used as a template for PCR amplification of the PgroE-*ilvC* fused fragment.

Plasmid pBD64 (Minton et al., Nucleic Acids Res. 18:1651(1990)) is a fairly stable vector for expression of foreign genes in *B. subtilis* and contains a *repB* gene and chloramphenicol and kanamycin resistance genes for selection in *B. subtilis.* This plasmid was used for expression of *ilvC* and *bdhB* under the control of a PgroE promoter. To clone PgroE-*ilvC*, *bdhB* and a lacI repressor gene into plasmid pBD64, a one-step assembly method was used (Tsuge et al., Nucleic Acids Res. 31:e133 (2003)). A 3,588 bp pBD64 fragment containing a *repB* gene, which included the replication function, and the kanamycin antibiotic marker was PCR-amplified from pBD64 with primers T-BD64(DraIII) (SEQ ID NO:152), which introduced a DraIII sequence (CACCGAGTG), and B-BD64(DraIII) (SEQ ID NO:153), which introduced a DraIII sequence (CACCTGGTG). A 1,327 bp lacI repressor gene was PCR-amplified from pMUTIN4 (Vagner et al., Microbiol. 144:3097-3104 (1998)) with T-laclq(Dralll) (SEQ ID NO:154), which introduced a DraIII sequence (CACCAGGTG) and B-lacIq(DraIII) (SEQ ID NO:155), which introduced a DraIII sequence (CACGGGGTG). A 1,224 bp PgroE-ilvC fused cassette was PCR-amplified from pHT01-ilvC(B.s) with T-groE(DraIII) (SEQ ID NO:156), which introduced a DraIII sequence (CACCCCGTG), and B-B.s.ilvC(DraIII) (SEQ ID NO:157), which introduced a DraIII sequence (CACCGTGTG). A 1.2 kbp *bdhB gene* (SEQ ID NO:158) was PCR-amplified from *Clostridium acetobutylicum* (ATCC 824) genomic DNA with primers T-bdhB(DraIII) (SEQ ID NO:159), which introduced a DraIII sequence (CACACGGTG), and B-bdhB(rrnBT1DraIII) (SEQ ID NO:160), which introduced a DraIII sequence (CACTCGGTG). The three underlined letters in the variable region of the DraIII recognition sequences were designed for specific base-pairing to assemble the four fragments with an order of pBD64-*lacI*-PgroE*ilvC*-*bdhB*. Each PCR product with DraIII sites at both ends was digested separately with DraIII, and the resulting DraIII fragments, 3,588 bp pBD64, *lacI*, PgroE*ilvC*, and *bdhB* were gel-purified using a QIAGEN gel extraction kit (QIAGEN). A mixture containing an equimolar concentration of each fragment with a total DNA concentration of 30 to 50 µg /100 µL was prepared for ligation. The ligation solution was then incubated at 16 °C overnight. The ligation generated high molecular weight tandem repeat DNA. The ligated long, linear DNA mixture was directly transformed into competent *B. subtilis* BE1010, prepared as described above. *B. subtilis* preferentially takes up long repeated linear DNA forms, rather than circular DNA to establish a plasmid. After transformation the culture was spread onto an LB plate containing 10 µg/mL of kanamycin for selection. Positive recombinant plasmids were screened by DraIII digestion, giving four fragments with an expected size of 3,588 bp (pBD64), 1,327 bp *(lacI),* 1,224 bp (PgorE-*ilvC*), and 1,194 bp *(bdhB).* The positive plasmid was named pBDPgroE-ilvC(B.s.)-bdhB.

Demonstration of isobutanol production from glucose or sucrose by *B. subtilis* BE1010 ΔsacB::PgroE-budB-ilvD-kivD/pBDPgroE-ilvC(B.s)-bdhB. To construct the recombinant *B. subtilis* expressing the five genes of the isobutanol biosynthetic pathway, competent cells of the two integrants *B. subtilis* BE1010 ΔsacB-PgroE-budB-ilvD-kivD #1-7 and B. *subtilis* BE1010 ΔsacB::PgroE-budB-ilvD-kivD #8-16 were prepared as described above, and transformed with plasmid pBDPgroE-ilvC(B.s.)-bdhB, yielding *B. subtilis* BE1010 ΔsacB::PgroE-budB-ilvD-kivD #1-7/pBDPgroE-ilvC(B.s.)-bdhB and *B, subtilis* BE1010 ΔsacB::PgroE-budB-ilvD-kivD #8-16/pBDPgroE-ilvC(B.s.)-bdhB.

The two recombinant strains were inoculated in either 25 mL or 100 mL of glucose medium containing kanamycin (10 µg /mL) in 125 mL flasks to simulate high and low oxygen conditions, respectively, and aerobically grown at 37 °C with shaking at 200 rpm. The medium consisted of 10 mM (NH₄)₂SO₄, 5 mM potassium phosphate buffer (pH 7.0), 100 mM MOPS/KOH buffer (pH 7.0), 20 mM glutamic acid/KOH (pH 7.0), 2% S10 metal mix, 1% glucose, 0.01% yeast extract, 0.01% casamino acids, and 50 µg/mL each of L-tryptophan, L-methionine, and L-lysine. The S10 metal mix consisted of 200 mM MgCl₂, 70 mM CaCl₂, 5 mM MnCl₂, 0.1 mM FeCl₃, 0.1 mM ZnCl₂, 0.2 mM thiamine hydrochloride, 0.172 mM CuSO₄, 0.253 mM CoCl₂, and 0.242 mM Na₂MoO₄. The cells were induced with 1.0 mM isopropyl-β-D-thiogalactopyranoiside (IPTG) at early-log phase (OD₆₀₀ of approximately 0.2). At 24 h after inoculation, an aliquot of the broth was analyzed by HPLC (Shodex Sugar SH1011 column) with refractive index (RI) detection for isobutanol content, as described in the General Methods section. The HPLC results are shown in Table 20.

**Table 20**

| Production of Isobutanol from Glucose by *B. subtilis* BE1010 ΔsacB::PgroE-budB-ilvD-kivD/pBDPgroE-ilvC(B.s.)-bdhB Strains | | | |
|---|---|---|---|
| Strain | O₂ Level | isobutanol, mM | molar selectivity, % |
| *B*. *subtilis* a (induced) | high | 1.00 | 1.8 |
| *B. subtilis* b (induced) | high | 0.87 | 1.6 |
| *B*. *subtilis* a (induced) | low | 0.06 | 0.1 |
| *B. subtilis b* (induced) | low | 0.14 | 0.3 |

| | | | |
|---|---|---|---|
| *B. subtilis a* is *B. subtilis* BE1010 ΔsacB::PgroE-budB-ilvD-kivD #1-7/pBDPgroE-ilvc(B.s.)-bdhB *B. subtilis* b is *B. subtilis* BE1010 ΔsacB::PgroE-budB-ilvD-kivD #8-16/pBDPgroE-ilvC(B.s.)-bdhB | | | |

The isolate of *B*. *subtilis* BE1010 ΔsacB::PgroE-budB-ilvD-kivD #1-7/pBDPgroE-ilvC(B.s.)-bdhB was also examined for isobutanol production from sucrose, essentially as described above. The recombinant strain was inoculated in 25 mL or 75 mL of sucrose medium containing kanamycin (10 µg /mL) in 125 mL flasks to simulate high and medium oxygen levels, and grown at 37 °C with shaking at 200 rpm. The sucrose medium was identical to the glucose medium except that glucose (10 g/L) was replaced with 10 g/L of sucrose. The cells were uninduced, or induced with 1.0 mM isopropyl-β-D-thiogalactopyranoiside (IPTG) at early-log phase (OD₆₀₀ of approximately 0.2). At 24 h after inoculation, an aliquot of the broth was analyzed by HPLC (Shodex Sugar SH1011 column) with refractive index (RI) detection for isobutanol content, as described in the General Methods section. The HPLC results are given in Table 21.

**Table 21**

| Production of Isobutanol from Sucrose by *B. subtilis* Strain BE1010 ΔsacB::PgroE-budB-ilvD-kivD/pBDPgroE-ilvC(B.s.)-bdhB | | | |
|---|---|---|---|
| Strain | O₂ Level | isobutanol, mM | molar selectivity, % |
| *B. subtilis* a (uninduced) | high | Not detected | Not detected |
| *B. subtilis* a (induced) | high | 0.44 | 4.9 |
| *B. subtilis* a (induced) | medium | 0.83 | 8.6 |

| | | | |
|---|---|---|---|
| *B. subtilis* a is *B*. *subtilis* BE1010 ΔsacB::PgroE-budB-ilvD-kivD #1-7/pBDPgroE-ilvC(B.s.)-bdhB | | | |

### EXAMPLE 25 (Prophetic)

### Expression of an Isobutanol Biosynthetic Pathway in Lactobacillus plantarum

The purpose of this prophetic Example is to describe how to express an isobutanol biosynthetic pathway in *Lactobacillus plantarum.* The five genes of the isobutanol pathway, encoding five enzyme activities, are divided into two operons for expression. The *budB, ilvD* and *kivD* genes, encoding the enzymes acetolactate synthase, acetohydroxy acid dehydratase, and branched-chain α-keto acid decarboxylase, respectively, are integrated into the chromosome of *Lactobacillus plantarum* by homologous recombination using the method described by Hols et al. (Appl. Environ. Microbiol, 60:1401-1413 (1994)). The remaining two genes *(ilvC* and *bdhB,* encoding the enzymes acetohydroxy acid reductoisomerase and butanol dehydrogenase, respectively) are cloned into an expression plasmid and transformed into the *Lactobacillus* strain carrying the integrated isobutanol genes. *Lactobacillus plantarum* is grown in MRS medium (Difco Laboratories, Detroit, MI) at 37°C, and chromosomal DNA is isolated as described by Moreira et al. (BMC Microbiol. 5:15(2005)).

Integration. The *budB-ilvD-kivD* cassette under the control of the synthetic P11 promoter (Rud et al., Microbiology 152:1011-1019 (2006)) is integrated into the chromosome of *Lactobacillus plantarum* ATCC BAA-793 (NCIMB 8826) at the *IdhL1* locus by homologous recombination. To build the *IdhL* integration targeting vector, a DNA fragment from *Lactobacillus plantarum* (Genbank NC_004567) with homology to *IdhL* is PCR amplified with primers LDH EcoRV F (SEQ ID NO:161) and LDH AatIIR (SEQ ID NO:162). The 1986 bp PCR fragment is cloned into pCR4Blunt-TOPO and sequenced. The pCR4Blunt-TOPO-IdhL1 clone is digested with EcoRV and AatII releasing a 1982 bp IdhL1 fragment that is gel-purified. The integration vector pFP988, given as SEQ ID NO:177, is digested with HindIII and treated with Klenow DNA polymerase to blunt the ends. The linearized plasmid is then digested with AatII and the 2931 bp vector fragment is gel purified. The EcoRV/AatII IdhL1 fragment is ligated with the pFP988 vector fragment and transformed into E. *coli* Top10 cells. Transformants are selected on LB agar plates containing ampicillin (100 µg/mL) and are screened by colony PCR to confirm construction of pFP988-IdhL.

To add a selectable marker to the integrating DNA, the Cm gene with its promoter is PCR amplified from pC194 (GenBank NC_002013, SEQ ID NO:267) with primers Cm F (SEQ ID NO:163) and Cm R (SEQ ID NO:164), amplifying a 836 bp PCR product. This PCR product is cloned into pCR4Blunt-TOPO and transformed into *E*. *coli* Top10 cells, creating pCR4Blunt-TOPO-Cm. After sequencing to confirm that no errors are introduced by PCR, the Cm cassette is digested from pCR4Blunt-TOPO-Cm as an 828 bp MIuI/SwaI fragment and is gel purified. The IdhL-homology containing integration vector pFP988-IdhL is digested with Mlul and SwaI and the 4740 bp vector fragment is gel purified. The Cm cassette fragment is ligated with the pFP988-IdhL vector creating pFP988-DldhL::Cm.

Finally the *budB-ilvD-kivD* cassette from pFP988DssPspac-budB-ilvD-kivD, described in Example 24, is modified to replace the amylase promoter with the synthetic P11 promoter. Then, the whole operon is moved into pFP988-DIdhL::Cm. The P11 promoter is built by oligonucleotide annealing with primer P11 F-Stul (SEQ ID NO:165) and P11 R-Spel (SEQ ID NO:166). The annealed oligonucleotide is gel-purified on a 6% Ultra PAGE gel (Embi Tec, San Diego, CA). The plasmid pFP988DssPspac-budB-ilvD-kivD, containing the amylase promoter, is digested with StuI and Spel and the resulting 10.9 kbp vector fragment is gel-purified. The isolated P11 fragment is ligated with the digested pFP988DssPspac-budB-ilvD-kivD to create pFP988-P11-budB-ilvD-kivD. Plasmid pFP988-P11-budB-ilvD-kivD is then digested with StuI and BamHI and the resulting 5.4 kbp P11-budB-ilvD-kivD fragment is gel-purified. pFP988-DIdhL::Cm is digested with HpaI and BamHI and the 5.5 kbp vector fragment isolated. The budB-ilvD-kivD operon is ligated with the integration vector pFP988-DIdhL::Cm to create pFP988-DIdhL-P11-budB-ilvD-kivD::Cm.

Integration of FPS88-DIdhL-P11-budB-ilvD-kivD::Cm into *L*. *plantarum BAA-793* to form *L. plantarum* ΔldhL1::budB-ilvD-kivD::Cm comprising exogenous *budB, ilvD.* and *kivD* genes. Electrocompetent cells of *L. plantarum* are prepared as described by Aukrust, T.W., et al. (In: Electroporation Protocols for Microorganisms; Nickoloff, J.A., Ed.; Methods in Molecular Biology, Vol. 47; Humana Press, Inc., Totowa, NJ, 1995, pp 201-208). After electroporation, cells are outgrown in MRSSM medium (MRS medium supplemented with 0.5 M sucrose and 0.1 M MgCl₂) as described by Aukrust et al. *supra* for 2 h at 37 °C without shaking. Electroporated cells are plated for selection on MRS plates containing chloramphenicol (10 µg/mL) and incubated at 37 °C. Transformants are initially screened by colony PCR amplification to confirm integration, and initial positive clones are then more rigorously screened by PCR amplification with a battery of primers.

Plasmid Expression of *ilvC* and *bdhB* genes. The remaining two isobutanol genes are expressed from plasmid pTRKH3 (O'Sullivan DJ and Klaenhammer TR, Gene 137:227-231 (1993)) under the control of the *L. plantarum* IdhL promoter (Ferain et al., J. Bacteriol. 176:596-601 (1994)). The IdhL promoter is PCR amplified from the genome of *L, plantarum* ATCC BAA-793 using primers PIdhL F-HindIII (SEQ ID NO:167) and PIdhL R-BamHI (SEQ ID NO:168). The 411 bp PCR product is cloned into pCR4Blunt-TOPO and sequenced. The resulting plasmid, pCR4Blunt-TOPO-PldhL is digested with HindIII and BamHI releasing the PIdhL fragment.

Plasmid pTRKH3 is digested with Hindlll and Sphl and the gel-purified vector fragment is ligated with the PIdhL fragment and the gel-purified 2.4 kbp BamHI/SphI fragment containing ilvC(B.s.)-bdhB from the *Bacillus* expression plasmid pBDPgroE-ilvC(B.s.)-bdhB (Example 24) in a three-way ligation. The ligation mixture is transformed into *E. coli* Top 10 cells and transformants are grown on Brain Heart Infusion (BHI, Difco Laboratories, Detroit, MI) plates containing erythromycin (150 mg/L). Transformants are screened by PCR to confirm construction. The resulting expression plasmid, pTRKH3-ilvC(B.s.)-bdhB is transformed into *L. plantarum* ΔldhL1::budB-ilvO-kivD::Cm by electroporation, as described above.

*L. plantarum* ΔldhL1::budB-ilvD-kivD::Cm containing pTRKH3-ilvC(B.s.)-bdhB is inoculated into a 250 mL shake flask containing 50 mL of MRS medium plus erythromycin (10 µg/mL) and grown at 37 °C for 18 to 24 h without shaking, after which isobutanol is detected by HPLC or GC analysis, as described in the General Methods section.

### EXAMPLE 26 (Prophetic)

### Expression of an Isobutanol Biosynthetic Pathway in Enterococcus faecalis

The purpose of this prophetic Example is to describe how to express an isobutanol biosynthetic pathway in *Enterococcus faecalis.* The complete genome sequence of *Enterococcus faecalis* strain V583, which is used as the host strain for the expression of the isobutanol biosynthetic pathway in this Example, has been published (Paulsen et al., Science 299:2071-2074 (2003)). An *E*. *coli*/Gram-positive shuttle vector, Plasmid pTRKH3 (O'Sullivan DJ and Klaenhammer TR, Gene 137:227-231 (1993)), is used for expression of the five genes *(budB, ilvC, ilvD, kivD, bdhB)* of the isobutanol pathway in one operon. pTRKH3 contains an *E*. *coli* plasmid p15A replication origin, the pAMβ1 replicon, and two antibiotic resistance selection markers for tetracycline and erythromycin. Tetracycline resistance is only expressed in *E*. *coli,* and erythromycin resistance is expressed in both *E, coli and* Gram-positive bacteria. Plasmid pAMβ1 derivatives can replicate in *E. faecalis* (Poyart et al., FEMS Microbiol. Lett. 156:193-198 (1997)), The inducible nisA promoter (PnisA), which has been used for efficient control of gene expression by nisin in a variety of Gram-positive bacteria including *Enterococcus faecalis* (Eichenbaum et al., Appl. Environ. Microbiol. 64:2763-2769 (1998)), is used to control expression of the five desired genes encoding the enzymes of the isobutanol biosynthetic pathway.

The plasmid pTrc99A::budB-ilvC-ilvD-kivD (described in Example 18), which contains the isobutanol pathway operon, is modified to replace the *E*. *coli ilvC* gene (SEQ ID NO:3) with the *B*. *subtilis ilvC* gene (SEQ ID NO:184). Additionally, the *bdhB* gene(SEQ ID NO:158) from *Clostridium acetobutylicum* is added to the end of the operon. First, the *bdhB* gene from pBDPgroE-ilvC(B.s.)-bdhB (described in Example 24) is amplified using primers F-bdhB-AvrII (SEQ ID NO:169) and R-bdhB-BamHI (SEQ ID NO:170), and then TOPO cloned and sequenced. The 1194 bp *bdhB* fragment is isolated by digestion with AvrII and BamHI, followed by gel purification. This *bdhB* fragment is ligated with pTrc99A::budB-ilvC-ilvD-kivD that has previously been digested with AvrII and BamHI and the resulting fragment is gel purified. The ligation mixture is transformed into *E. coli* Top 10 cells by electroporation and transformants are selected following overnight growth at 37 °C on LB agar plates containing ampicillin (100 µg/mL). The transformants are then screened by colony PCR to confirm the correct clone containing pTrc99A::budB₋ilvC-ilvD-kivD-bdhB.

Next, *ilvC*(B.s.) is amplified from pBDPgroE-ilvC(B.s.)-bdhB (described in Example 24) using primers F-ilvC(B.s.)-AfIII (SEQ ID NO:171) and R-ilvC(B.s.)-NotI (SEQ ID NO:172). The PCR product is TOPO cloned and sequenced. The 1051 bp *ilvC(B.s.)* fragment is isolated by digestion with AfIII and Notl followed by gel purification. This fragment is ligated with pTrc99A::budB-ilvC-ilvD-kivD-bdhB that has been cut with AfIII and NotI to release the *E*. *coli ilvC* (the 10.7 kbp vector band is gel purified prior to ligation with *ilvC(B.s.)).* The ligation mixture is transformed into *E*. *coli* Top10 cells by electroporation and transformants are selected following overnight growth at 37 °C on LB agar plates containing ampicillin (100 µg/mL). The transformants are then screened by colony PCR to confirm the correct clone containing pTrc99A::budB-ilvC(B.s.)-ilvD-kivD-bdhB.

To provide a promoter for the *E*. *colil* Gram-positive shuttle vector pTRKH3, the *nisA* promoter (Chandrapati et al., Mol. Microbial. 46(2):467-477 (2002)) is PCR-amplified from *Lactococcus lactis* genomic DNA with primers F-PnisA(HindIII) (SEQ ID NO:173) and R-PnisA(Spel BamHI) (SEQ ID NO:174) and then TOPO cloned. After sequencing, the 213 bp *nisA* promoter fragment is isolated by digestion with HindIII and BamHI followed by gel purification. Plasmid pTRKH3 is digested with HindIII and BamHI and the vector fragment is gel-purified. The linearized pTRKH3 is ligated with the PnisA fragment and transformed into *E. coli* Top10 cells by electroporation. Transformants are selected following overnight growth at 37 °C on LB agar plates containing erythromycin (25 µg/mL). The transformants are then screened by colony PCR to confirm the correct clone of pTRKH3-PnisA.

Plasmid pTRKH3-PnisA is digested with Spel and BamHI, and the vector is gel-purified. Plasmid pTrc99A::budB-ilvC(B.s)-ilvD-kivD-bdhB, described above, is digested with Spel and BamHI, and the 7.5 kbp fragment is gel-purified. The 7.5 kbp *budB-ilvC(B.s)-ilvD-kivD-bdhB* fragment is ligated into the pTRKH3-PnisA vector at the Spel and BamHI sites. The ligation mixture is transformed into *E. coli* Top10 cells by electroporation and transformants are selected following overnight growth on LB agar plates containing erythromycin (25 µg/mL) at 37 °C. The transformants are then screened by colony PCR. The resulting plasmid is named pTRKH3-PnisA-budB-ilvC(B.s)-ilvD-kivD-bdhB. This plasmid is prepared from the *E. coli* transformants and transformed into electrocompetent *E. faecalis* V583 cells by electroporation using methods known in the art (Aukrust, T.W., et al. In: Electroporation Protocols for Microorganisms; Nickoloff, J.A., Ed.; Methods in Molecular Biology, Vol. 47; Humana Press, Inc., Totowa, NJ., 1995, pp 217-226), resulting in *E. faecalis* V583/ pTRKH3-PnisA-budB-ilvC(B.s)-ilvD-kivD-bdhB.

The second plasmid containing *nisA* regulatory genes, *nisR* and *nisK,* the *add9* spectinomycin resistance gene, and the pSH71 origin of replication is transformed into *E. faecalis* V583/ pTRKH3-PnisA-budB-ilvC(B.s)-ilvD-kivD-bdhB by electroporation. The plasmid containing pSH71 origin of replication is compatible with pAMβ1 derivatives in *E. faecalis* (Eichenbaum et al., *supra).* Double drug resistant transformants are selected on LB agar plates containing erythromycin (25 µg/mL) and spectinomycin (100 µg/mL), grown at 37 °C.

The resulting *E. faecalis* strain V583*B* harboring two plasmids, i.e., an expression plasmid (pTRKH3-PnisA-budB-ilvC(B.s)-ilvD-kivD-bdhB) and a regulatory plasmid (pSH71-nisRK), is inoculated into a 250 mL shake flask containing 50 mL of Todd-Hewitt broth supplemented with yeast extract (0.2%) (Fischetti et al., J. Exp. Med. 161:1384-1401 (1985)), nisin (20 µg/mL) (Eichenbaum et al,, *supra),* erythromycin (25 µg/mL), and spectinomycin (100 µg/mL). The flask is incubated without shaking at 37 °C for 18-24 h, after which time, isobutanol production is measured by HPLC or GC analysis, as described in the General Methods section.

### EXAMPLE 27

### Increased tolerance of Saccharomyces cerevisiae to isobutanol at decreased growth temperature

Tolerance levels were determined for yeast strain *Saccharomyces cerevisiae* BY4741 (ATCC 201388) at 25 °C and 30 °C as follows. The strain was cultured in YPD medium. Overnight cultures in the absence of any test compound were started in 25 mL of YPD medium in 150 mL flasks with incubation at 30 °C or at 25 °C in shaking water baths. The next morning, each overnight culture was diluted into a 500 mL flask containing 300 mL of fresh medium to an initial OD₆₀₀ of about 0.1. The flasks were incubated in shaking water baths at 30 °C or 25 °, using the same temperature as used for each overnight culture. The large cultures were incubated for 3 hours and then were split into flasks in the absence (control) and in the presence of 2% or 3% of isobutanol. Growth was followed by measuring OD600 for six hours after addition of the isobutanol. The ΔOD₆₀₀ was calculated by subtracting the initial OD₆₀₀ from the final OD₆₀₀ at 6 hours. The percent growth inhibition relative to the control culture was calculated as follows: % Growth Inhibition = 100 ― [100(Sample ΔOD₆₀₀/Control ΔOD₆₀₀)]. The results are summarized in Table 22 below and indicate that growth of strain BY4741 is less inhibited by 2% isobutanol at 25°C than by 2% isobutanol at 30 °C.

**Table 22. Growth of Saccharomyces cerevisiae Strain BY4741 at 25 °C and 30 °C with Isobutanol.**

| % Isobutanol | Temperature | % Growth Inhibition |
|---|---|---|
| 2 | 30 | 99 |
| 2 | 25 | 84 |
| 3 | 30 | No growth |
| 3 | 25 | No growth |

Embodiments of the invention are also described in the claims of the International Application as filed:
1. A method for the production of isobutanol comprising:
   a) providing a recombinant microbial production host which produces isobutanol;
   b) seeding the production host of (a) into a fermentation medium comprising a fermentable carbon substrate to create a fermentation culture;
   c) growing the production host in the fermentation culture at a first temperature for a first period of time;
   d) lowering the temperature of the fermentation culture to a second temperature; and
   e) incubating the production host at the second temperature of step (d) for a second period of time;
   whereby isobutanol is produced.
2. A method according to Claim 1 wherein the fermentable carbon substrate is derived from a grain or sugar source selected from the group consisting of wheat, corn, barley, oats, rye, sugar cane, sugar beets, cassava, sweet sorghum, and mixtures thereof.
3. A method according to Claim 1 wherein the fermentable carbon substrate is derived from cellulosic or lignocellulosic biomass selected from the group consisting of corn cobs, crop residues, corn husks, corn stover, grasses, wheat straw, barley straw, hay, rice straw, switchgrass, waste paper, sugar cane bagasse, sorghum, soy, components obtained from milling of grains, trees, branches, roots, leaves, wood chips, sawdust, shrubs and bushes, vegetables, fruits, flowers, animal manure, and mixtures thereof.
4. A method according to Claim 1 wherein the fermentable carbon substrate is selected from the group consisting of monosaccharides, oligosaccharides, and polysaccharides.
5. A method according to Claim 1 wherein the fermentation culture is maintained under conditions selected from the group consisting of anaerobic conditions and microaerobic conditions,
6. A method according to Claim 1 wherein while growing the production host in (c) at a first temperature over a first period of time, a metabolic parameter of the fermentation culture is monitored.
7. A method according to Claim 6 wherein the metabolic parameter that is monitored is selected from the group consisting of optical density, pH, respiratory quotient, fermentable carbon substrate utilization, CO₂ production, and isobutanol production.
8. A method according to Claim 1 wherein lowering the temperature of the fermentation culture of step (d) occurs at a predetermined time.
9. A method according to Claim 1 wherein the lowering of the temperature of the fermentation culture of step (d) coincides with a change in a metabolic parameter.
10. A method according to Claim 9 wherein the change in metabolic parameter is a decrease in the rate of isobutanol production.
11. A method according to Claim 1 wherein the first temperature is from about 25 °C to about 40 °C.
12. A method according to Claim 1 wherein the second temperature is from about 3 °C to about 25 °C lower than the first temperature.
13. A method according to Claim 1 wherein steps (d) and (e) are repeated one or more times.
14. A method according to Claim 1 wherein the recombinant microbial production host is selected from the group consisting of *Clostridium, Zymomonas, Escherichia, Salmonella, Rhodococcus, Pseudomonas, Bacillus, Lactobacillus, Enterococcus, Alcaligenes, Klebsiella, Paenibacillus, Arthrobacter, Corynebacterium, Brevibacterium, Saccharomyces,* and *Pichia.*
15. A method according to Claim 1 wherein the recombinant microbial host cell comprises at least one DNA molecule encoding a polypeptide that catalyzes a substrate to product conversion selected from the group consisting of:
   a) pyruvate to acetolactate;
   b) acetolactate to 2,3-dihydroxyisovalerate,
   c) 2,3-dihydroxyisovalerate to α-ketoisovalerate;
   d) α-ketoisovalerate to isobutyraldehyde; and
   e) isobutyraldehyde to isobutanol;
   wherein the at least one DNA molecule is heterologous to said microbial production host cell.
16. A method according to Claim 15 wherein the polypeptide that catalyzes a substrate to product conversion of pyruvate to acetolactate is acetolactate synthase.
17. A method according to Claim 15 wherein the polypeptide that catalyzes a substrate to product conversion of acetolactate to 2,3-dihydroxyisovalerate is acetohydroxy acid isomeroreductase.
18. A method according to Claim 15 wherein the polypeptide that catalyzes a substrate to product conversion of of 2,3-dihydroxyisovalerate to α-ketoisovalerate is acetohydroxy acid dehydratase.
19. A method according to Claim 15 wherein the polypeptide that catalyzes a substrate to product conversion of α-ketoisovalerate to isobutyraldehyde is branched-chain α-keto acid decarboxylase.
20. A method according to Claim 15 wherein the polypeptide that catalyzes a substrate to product conversion of isobutyraldehyde to isobutanol is branched-chain alcohol dehydrogenase.
21. A method according to Claim 16 wherein the acetolactate synthase has an amino acid sequence having at least 95% identity to an amino acid sequence selected from the group consisting of SEQ ID NO:2, SEQ ID NO:178, and SEQ ID NO:180, based on the Clustal W method of alignment using the default parameters of GAP PENALTY=10, GAP LENGTH PENALTY=0.1, and Gonnet 250 series of protein weight matrix.
22. A method according to Claim 17 wherein the acetohydroxy acid isomeroreductase has an amino acid sequence having at least 95% identity to an amino acid sequence selected from the group consisting of SEQ ID NO:43, SEQ ID NO:181, SEQ ID NO:183, and SEQ ID NO:185, based on the Clustal W method of alignment using the default parameters of GAP PENALTY=10, GAP LENGTH PENALTY=0.1, and Gonnet 250 series of protein weight matrix.
23. A method according to Claim 18 wherein the acetohydroxy acid dehydratase has an amino acid sequence having at least 95% identity to an amino acid sequence selected from the group consisting of SEQ ID NO:6, SEQ ID NO:186, SEQ ID NO:188, and SEQ ID NO:190, based on the Clustal W method of alignment using the default parameters of GAP PENALTY=10, GAP LENGTH PENALTY=0.1, and Gonnet 250 series of protein weight matrix.
24. A method according to Claim 19 wherein the branched-chain α-keto acid decarboxylase has an amino acid sequence having at least 95% identity to an amino acid sequence selected from the group consisting of SEQ ID NO:8, SEQ ID NO:193, SEQ ID NO:195, and SEQ ID NO:197, based on the Clustal W method of alignment using the default parameters of GAP PENALTY=10, GAP LENGTH PENALTY=0.1, and Gonnet 250 series of protein weight matrix.
25. A method according to Claim 20 wherein the branched-chain alcohol dehydrogenase has an amino acid sequence having at least 95% identity to an amino acid sequence selected from the group consisting of SEQ ID NO:10, SEQ ID NO:199, SEQ ID NO:201, SEQ ID NO:203, and SEQ ID NO:204, based on the Clustal W method of alignment using the default parameters of GAP PENALTY=10, GAP LENGTH PENALTY=0.1, and Gonnet 250 series of protein weight matrix.

## Claims

1. A recombinant microbial host comprising DNA molecules encoding polypeptides that catalyse each of the following substrate to product conversions:
i) pyruvate to acetolactate;
ii) acetolactate to 2,3-dihydroxyisovalerate;
iii) 2,3-dihydroxyisovalerate to α-ketoisovalerate;
iv) α-ketoisovalerate to isobutyraldehyde; and
v) isobutyraldehyde to isobutanol;
wherein at least one of said DNA molecules is heterologous to said recombinant microbial host cell, and
wherein the polypeptide that catalyzes a substrate to product conversion of pyruvate to acetolactate is acetolactate synthase and the acetolactate synthase has an amino acid sequence having at least 80% identity to an amino acid sequence selected from the group consisting of SEQ ID NO: 2, SEQ ID NO: 178, and SEQ ID NO: 180, based on the Clustal W method of alignment using the default parameters of GAP PENALTY=10, GAP LENGTH PENALTY=0.1, and Gonnet 250 series of protein weight matrix; or
wherein the polypeptide that catalyzes a substrate to product conversion of acetolactate to 2,3-dihydroxyisovalerate is acetohydroxy acid isomeroreductase and the acetohydroxy acid isomeroreductase has an amino acid sequence having at least 80% identity to an amino acid sequence selected from the group consisting of SEQ ID NO: 43, SEQ ID NO: 181, SEQ ID NO: 183, and SEQ ID NO: 185, based on the Clustal W method of alignment using the default parameters of GAP PENALTY=10, GAP LENGTH PENALTY=0.1, and Gonnet 250 series of protein weight matrix; or
wherein the polypeptide that catalyzes a substrate to product conversion of of 2, 3-dihydroxyisovalerate to α-ketoisovalerate is acetohydroxy acid dehydratase and the acetohydroxy acid dehydratase has an amino acid sequence having at least 80% identity to an amino acid sequence selected from the group consisting of SEQ ID NO: 6, SEQ ID NO: 186, SEQ ID NO: 188, and SEQ ID NO: 190, based on the Clustal W method of alignment using the default parameters of GAP PENALTY=10, GAP LENGTH PENALTY=0.1, and Gonnet 250 series of protein weight matrix; or
wherein the polypeptide that catalyzes a substrate to product conversion of α-ketoisovalerate to isobutyraldehyde is branched-chain α-keto acid decarboxylase and the branched-chain α-keto acid decarboxylase has an amino acid sequence having at least 80% identity to an amino acid sequence selected from the group consisting of SEQ ID NO: 8, SEQ ID NO: 193, SEQ ID NO: 195, and SEQ ID NO: 197, based on the Clustal W method of alignment using the default parameters of GAP PENALTY=10, GAP LENGTH PENALTY=0.1, and Gonnet 250 series of protein weight matrix; or
wherein the polypeptide that catalyzes a substrate to product conversion of isobutyraldehyde to isobutanol is branched-chain alcohol dehydrogenase and the branched-chain alcohol dehydrogenase has an amino acid sequence having at least 80% identity to an amino acid sequence selected from the group consisting of SEQ ID NO: 10, SEQ ID NO: 199, SEQ ID NO: 201, SEQ ID NO: 203, and SEQ ID NO: 204, based on the Clustal W method of alignment using the default parameters of GAP PENALTY=10, GAP LENGTH PENALTY=0.1, and Gonnet 250 series of protein weight matrix.

2. A recombinant microbial host comprising DNA molecules encoding polypeptides that catalyse each of the following substrate to product conversions:
i) pyruvate to acetolactate;
ii) acetolactate to 2,3-dihydroxyisovalerate;
iii) 2,3-dihydroxyisovalerate to α-ketoisovalerate;
iv) α-ketoisovalerate to isobutyryl-CoA;
v) isobutyryl-CoA to isobutyraldehyde; and
vi) isobutyraldehyde to isobutanol;
wherein at least one of said DNA molecules is heterologous to said recombinant microbial host cell, and
wherein the polypeptide that catalyzes a substrate to product conversion of pyruvate to acetolactate is acetolactate synthase and the acetolactate synthase has an amino acid sequence having at least 80% identity to an amino acid sequence selected from the group consisting of SEQ ID NO: 2, SEQ ID NO: 178, and SEQ ID NO: 180, based on the Clustal W method of alignment using the default parameters of GAP PENALTY=10, GAP LENGTH PENALTY=0.1, and Gonnet 250 series of protein weight matrix; or
wherein the polypeptide that catalyzes a substrate to product conversion of acetolactate to 2,3-dihydroxyisovalerate is acetohydroxy acid isomeroreductase and the acetohydroxy acid isomeroreductase has an amino acid sequence having at least 80% identity to an amino acid sequence selected from the group consisting of SEQ ID NO: 43, SEQ ID NO: 181, SEQ ID NO: 183, and SEQ ID NO: 185, based on the Clustal W method of alignment using the default parameters of GAP PENALTY=10, GAP LENGTH PENALTY=0.1, and Gonnet 250 series of protein weight matrix; or
wherein the polypeptide that catalyzes a substrate to product conversion of of 2, 3-dihydroxyisovalerate to α-ketoisovalerate is acetohydroxy acid dehydratase and the acetohydroxy acid dehydratase has an amino acid sequence having at least 80% identity to an amino acid sequence selected from the group consisting of SEQ ID NO: 6, SEQ ID NO: 186, SEQ ID NO: 188, and SEQ ID NO: 190, based on the Clustal W method of alignment using the default parameters of GAP PENALTY=10, GAP LENGTH PENALTY=0.1, and Gonnet 250 series of protein weight matrix; or
wherein the polypeptide that catalyzes a substrate to product conversion of isobutyraldehyde to isobutanol is branched-chain alcohol dehydrogenase and the branched-chain alcohol dehydrogenase has an amino acid sequence having at least 80% identity to an amino acid sequence selected from the group consisting of SEQ ID NO: 10, SEQ ID NO: 199, SEQ ID NO: 201, SEQ ID NO: 203, and SEQ ID NO: 204, based on the Clustal W method of alignment using the default parameters of GAP PENALTY=10, GAP LENGTH PENALTY=0.1, and Gonnet 250 series of protein weight matrix; or
wherein the polypeptide that catalyzes a substrate to product conversion of α-ketoisovalerate to isobutyryl-CoA is branched-chain keto acid dehydrogenase and the branched-chain keto acid dehydrogenase has an amino acid sequence selected from the group consisting of SEQ ID NO: 206, SEQ ID NO: 208, SEQ ID NO: 210, SEQ ID NO: 212, SEQ ID NO: 214, SEQ ID NO: 216, SEQ ID NO: 218, and SEQ ID NO: 220; or
wherein the polypeptide that catalyzes a substrate to product conversion of isobutyryl-CoA to isobutyraldehyde is acylating aldehyde dehydrogenase and the acylating aldehyde dehydrogenase has an amino acid sequence selected from the group consisting of SEQ ID NO: 222, SEQ ID NO: 224, SEQ ID NO: 226, SEQ ID NO: 228, and SEQ ID NO: 230.

3. A recombinant microbial host comprising DNA molecules encoding polypeptides that catalyse each of the following substrate to product conversions:
i) pyruvate to acetolactate;
ii) acetolactate to 2,3-dihydroxyisovalerate;
iii) 2,3-dihydroxyisovalerate to α-ketoisovalerate;
iv) α-ketoisovalerate to valine;
v) valine to isobutylamine;
vi) isobutylamine to isobutyraldehyde; and
vii) isobutyraldehyde to isobutanol;
wherein at least one of said DNA molecules is heterologous to said recombinant microbial host cell, and
wherein the polypeptide that catalyzes a substrate to product conversion of pyruvate to acetolactate is acetolactate synthase and the acetolactate synthase has an amino acid sequence having at least 80% identity to an amino acid sequence selected from the group consisting of SEQ ID NO: 2, SEQ ID NO: 178, and SEQ ID NO: 180, based on the Clustal W method of alignment using the default parameters of GAP PENALTY=10, GAP LENGTH PENALTY=0.1, and Gonnet 250 series of protein weight matrix; or
wherein the polypeptide that catalyzes a substrate to product conversion of acetolactate to 2,3-dihydroxyisovalerate is acetohydroxy acid isomeroreductase and the acetohydroxy acid isomeroreductase has an amino acid sequence having at least 80% identity to an amino acid sequence selected from the group consisting of SEQ ID NO: 43, SEQ ID NO: 181, SEQ ID NO: 183, and SEQ ID NO: 185, based on the Clustal W method of alignment using the default parameters of GAP PENALTY=10, GAP LENGTH PENALTY=0.1, and Gonnet 250 series of protein weight matrix; or
wherein the polypeptide that catalyzes a substrate to product conversion of of 2, 3-dihydroxyisovalerate to α-ketoisovalerate is acetohydroxy acid dehydratase and the acetohydroxy acid dehydratase has an amino acid sequence having at least 80% identity to an amino acid sequence selected from the group consisting of SEQ ID NO: 6, SEQ ID NO: 186, SEQ ID NO: 188, and SEQ ID NO: 190, based on the Clustal W method of alignment using the default parameters of GAP PENALTY=10, GAP LENGTH PENALTY=0.1, and Gonnet 250 series of protein weight matrix; or
wherein the polypeptide that catalyzes a substrate to product conversion of isobutyraldehyde to isobutanol is branched-chain alcohol dehydrogenase and the branched-chain alcohol dehydrogenase has an amino acid sequence having at least 80% identity to an amino acid sequence selected from the group consisting of SEQ ID NO: 10, SEQ ID NO: 199, SEQ ID NO: 201, SEQ ID NO: 203, and SEQ ID NO: 204, based on the Clustal W method of alignment using the default parameters of GAP PENALTY=10, GAP LENGTH PENALTY=0.1, and Gonnet 250 series of protein weight matrix; or
wherein the polypeptide that catalyzes a substrate to product conversion of α-ketoisovalerate to valine is transaminase and the transaminase has an amino acid sequence selected from the group consisting of SEQ ID NO: 232, SEQ ID NO: 234, SEQ ID NO: 236, SEQ ID NO: 238, and SEQ ID NO: 240, or valine dehydrogenase and the valine dehydrogenase has an amino acid sequence selected from the group consisting of SEQ ID NO: 242 and SEQ ID NO: 244; or
wherein the polypeptide that catalyzes a substrate to product conversion of valine to isobutylamine is valine decarboxylase and the valine decarboxylase has an amino acid sequence of SEQ ID NO: 246; or
wherein the polypeptide that catalyzes a substrate to product conversion of isobutylamine to isobutyraldehyde is omega transaminase and the omega transaminase has an amino acid sequence selected from the group consisting of SEQ ID NO: 248, SEQ ID NO: 250, SEQ ID NO: 252, and SEQ ID NO; 254.

4. The recombinant microbial host according to any one of claims 1-3, wherein said identity to an amino acid sequence is 80%-85% or 85%-90% identity to an amino acid sequence.

5. The recombinant microbial production host according to any one of claims 1-4, wherein the recombinant microbial production host is selected from the group consisting of *Clostridium, Zymomonas, Escherichia, Salmonella, Rhodococcus, Pseudomonas, Bacillus, Lactobacillus, Enterococcus, Alcaligenes, Klebsiella, Paenibacillus, Arthrobacter, Corynebacterium, Brevibacterium, Saccharomyces,* and *Pichia.*

6. A method for the production of isobutanol comprising:
a) providing a recombinant microbial production host which comprises an isobutanol biosynthetic pathway comprising the substrate to product conversions:
i) pyruvate to acetolactate;
ii) acetolactate to 2,3-dihydroxyisovalerate;
iii) 2,3-dihydroxyisovalerate to a-ketoisovalerate;
iv) α-ketoisovalerate to isobutyraldehyde; and
v) isobutyraldehyde to isobutanol;
or
i) pyruvate to acetolactate,
ii) acetolactate to 2,3-dihydroxy0isovalerate,
iii) 2,3-dihydroxyisovalerate to α-ketoisovalerate,
iv) α-ketoisovalerate to isobutyryl-CoA,
v) isobutyryl-CoA to isobutyraldehyde, and
vi) isobutyraldehyde to isobutanol;
or
i) pyruvate to acetolactate,
ii) acetolactate to 2,3-dihydroxyisovalerate,
iii) 2,3-dihydroxyisovalerate to α-ketoisovalerate,
iv) α-ketoisovalerate to valine,
v) valine to isobutylamine,
vi) isobutylamine to isobutyraldehyde, and
vii) isobutyraldehyde to isobutanol;
b) seeding the production host of (a) into a fermentation medium comprising a fermentable carbon substrate to create a fermentation culture;
c) growing the production host in the fermentation culture at a first temperature for a first period of time, wherein the first temperature is from about 25°C to about 40°C;
d) lowering the temperature of the fermentation culture to a second temperature; and
e) incubating the production host at the second temperature of step (d) for a second period of time;
whereby isobutanol is produced.

7. The method according to claim 6, wherein said recombinant microbial host cell is a recombinant microbial host cell as defined in any one of claims 1-5.

8. The method according to claim 6 or claim 7, wherein while growing the production host in (c) at a first temperature over a first period of time, a metabolic parameter of the fermentation culture is monitored.

9. The method according to claim 8, wherein the metabolic parameter that is monitored is selected from the group consisting of optical density, pH, respiratory quotient, fermentable carbon substrate utilization, CO₂ production, and isobutanol production.

10. The method according to claim 9, wherein the metabolic parameter that is monitored is a decrease in the rate of isobutanol production.

11. The method according to any one of claims 6-10, wherein steps (d) and (e) are repeated one or more times.

12. A method to reduce the sensitivity of a recombinant microbial production host to isobutanol comprising:
a) providing a recombinant microbial production host which produces isobutanol, wherein said recombinant microbial host comprises DNA molecules encoding polypeptides that catalyse each of the following substrate to product conversions:
i) pyruvate to acetolactate;
ii) acetolactate to 2,3-dihydroxyisovalerate;
iii) 2,3-dihydroxyisovalerate to α-ketoisovalerate;
iv) α-ketoisovalerate to isobutyraldehyde; and
v) isobutyraldehyde to isobutanol;
or
i) pyruvate to acetolactate,
ii) acetolactate to 2,3-dihydroxyisovalerate,
iii) 2,3-dihydroxyisovalerate to α-ketoisovalerate,
iv) α-ketoisovalerate to isobutyryl-CoA,
v) isobutyryl-CoA to isobutyraldehyde, and
vi) isobutyraldehyde to isobutanol;
or
i) pyruvate to acetolactate,
ii) acetolactate to 2,3-dihydroxyisovalerate,
iii) 2,3-dihydroxyisovalerate to α-ketoisovalerate,
iv) α-ketoisovalerate to valine,
v) valine to isobutylamine,
vi) isobutylamine to isobutyraldehyde, and
vii) isobutyraldehyde to isobutanol;
wherein at least one of said DNA molecules is heterologous to said recombinant microbial host;
b) growing the recombinant microbial production host in a fermentation culture; and
c) determining the metabolic activity of the fermentation culture by monitoring one or more metabolic parameters selected from optical density, pH, respiratory quotient, fermentable carbon substrate utilization, CO₂ production, and isobutanol production.

13. The method of claim 12, further comprising the step of adjusting the one or more metabolic parameters to support the metabolic activity.

14. The method of claim 12 or claim 13, wherein a decrease in one or more of the metabolic parameters indicates a decrease in metabolic activity.

15. The method of claim 14, wherein the adjusting the one or more metabolic parameters is lowering the temperature of the fermentation culture when a decrease in metabolic activity is detected.


SEQUENCE LISTING

<110>  E.I. du Pont de Nemours and Co.

<120>  Method for the Production of Isobutanol

<130>  CL3892

<160>  267

<170>  PatentIn version 3.4

<210>  1
<211>  1680
<212>  DNA
<213>  K. pneumoniae

<400>  1
atggacaaac agtatccggt acgccagtgg gcgcacggcg ccgatctcgt cgtcagtcag      60

ctggaagctc agggagtacg ccaggtgttc ggcatccccg gcgccaaaat cgacaaggtc     120

tttgattcac tgctggattc ctccattcgc attattccgg tacgccacga agccaacgcc     180

gcatttatgg ccgccgccgt cggacgcatt accggcaaag cgggcgtggc gctggtcacc     240

tccggtccgg gctgttccaa cctgatcacc ggcatggcca ccgcgaacag cgaaggcgac     300

ccggtggtgg ccctgggcgg cgcggtaaaa cgcgccgata aagcgaagca ggtccaccag     360

agtatggata cggtggcgat gttcagcccg gtcaccaaat acgccatcga ggtgacggcg     420

ccggatgcgc tggcggaagt ggtctccaac gccttccgcg ccgccgagca gggccggccg     480

ggcagcgcgt tcgttagcct gccgcaggat gtggtcgatg gcccggtcag cggcaaagtg     540

ctgccggcca gcggggcccc gcagatgggc gccgcgccgg atgatgccat cgaccaggtg     600

gcgaagctta tcgcccaggc gaagaacccg atcttcctgc tcggcctgat ggccagccag     660

ccggaaaaca gcaaggcgct gcgccgtttg ctggagacca gccatattcc agtcaccagc     720

acctatcagg ccgccggagc ggtgaatcag gataacttct ctcgcttcgc cggccgggtt     780

gggctgttta acaaccaggc cggggaccgt ctgctgcagc tcgccgacct ggtgatctgc     840

atcggctaca gcccggtgga atacgaaccg gcgatgtgga acagcggcaa cgcgacgctg     900

gtgcacatcg acgtgctgcc cgcctatgaa gagcgcaact acaccccgga tgtcgagctg     960

gtgggcgata tcgccggcac tctcaacaag ctggcgcaaa atatcgatca tcggctggtg    1020

ctctccccgc aggcggcgga gatcctccgc gaccgccagc accagcgcga gctgctggac    1080

cgccgcggcg cgcagctcaa ccagtttgcc ctgcatcccc tgcgcatcgt tcgcgccatg    1140

caggatatcg tcaacagcga cgtcacgttg accgtggaca tgggcagctt ccatatctgg    1200

attgcccgct acctgtacac gttccgcgcc cgtcaggtga tgatctccaa cggccagcag    1260

accatgggcg tcgccctgcc ctgggctatc ggcgcctggc tggtcaatcc tgagcgcaaa    1320

gtggtctccg tctccggcga cggcggcttc ctgcagtcga gcatggagct ggagaccgcc    1380

gtccgcctga aagccaacgt gctgcatctt atctgggtcg ataacggcta caacatggtc    1440

gctatccagg aagagaaaaa atatcagcgc ctgtccggcg tcgagtttgg gccgatggat    1500

tttaaagcct atgccgaatc cttcggcgcg aaagggtttg ccgtggaaag cgccgaggcg    1560

ctggagccga ccctgcgcgc ggcgatggac gtcgacggcc cggcggtagt ggccatcccg    1620

gtggattatc gcgataaccc gctgctgatg ggccagctgc atctgagtca gattctgtaa    1680

<210>  2
<211>  559
<212>  PRT
<213>  K. pneumoniae

<400>  2

Met Asp Lys Gln Tyr Pro Val Arg Gln Trp Ala His Gly Ala Asp Leu
1               5                   10                  15

Val Val Ser Gln Leu Glu Ala Gln Gly Val Arg Gln Val Phe Gly Ile
            20                  25                  30

Pro Gly Ala Lys Ile Asp Lys Val Phe Asp Ser Leu Leu Asp Ser Ser
        35                  40                  45

Ile Arg Ile Ile Pro Val Arg His Glu Ala Asn Ala Ala Phe Met Ala
    50                  55                  60

Ala Ala Val Gly Arg Ile Thr Gly Lys Ala Gly Val Ala Leu Val Thr
65                  70                  75                  80

Ser Gly Pro Gly Cys Ser Asn Leu Ile Thr Gly Met Ala Thr Ala Asn
                85                  90                  95

Ser Glu Gly Asp Pro Val Val Ala Leu Gly Gly Ala Val Lys Arg Ala
            100                 105                 110

Asp Lys Ala Lys Gln Val His Gln Ser Met Asp Thr Val Ala Met Phe
        115                 120                 125

Ser Pro Val Thr Lys Tyr Ala Ile Glu Val Thr Ala Pro Asp Ala Leu
    130                 135                 140

Ala Glu Val Val Ser Asn Ala Phe Arg Ala Ala Glu Gln Gly Arg Pro
145                 150                 155                 160

Gly Ser Ala Phe Val Ser Leu Pro Gln Asp Val Val Asp Gly Pro Val
                165                 170                 175

Ser Gly Lys Val Leu Pro Ala Ser Gly Ala Pro Gln Met Gly Ala Ala
            180                 185                 190

Pro Asp Asp Ala Ile Asp Gln Val Ala Lys Leu Ile Ala Gln Ala Lys
        195                 200                 205

Asn Pro Ile Phe Leu Leu Gly Leu Met Ala Ser Gln Pro Glu Asn Ser
    210                 215                 220

Lys Ala Leu Arg Arg Leu Leu Glu Thr Ser His Ile Pro Val Thr Ser
225                 230                 235                 240

Thr Tyr Gln Ala Ala Gly Ala Val Asn Gln Asp Asn Phe Ser Arg Phe
                245                 250                 255

Ala Gly Arg Val Gly Leu Phe Asn Asn Gln Ala Gly Asp Arg Leu Leu
            260                 265                 270

Gln Leu Ala Asp Leu Val Ile Cys Ile Gly Tyr Ser Pro Val Glu Tyr
        275                 280                 285

Glu Pro Ala Met Trp Asn Ser Gly Asn Ala Thr Leu Val His Ile Asp
    290                 295                 300

Val Leu Pro Ala Tyr Glu Glu Arg Asn Tyr Thr Pro Asp Val Glu Leu
305                 310                 315                 320

Val Gly Asp Ile Ala Gly Thr Leu Asn Lys Leu Ala Gln Asn Ile Asp
                325                 330                 335

His Arg Leu Val Leu Ser Pro Gln Ala Ala Glu Ile Leu Arg Asp Arg
            340                 345                 350

Gln His Gln Arg Glu Leu Leu Asp Arg Arg Gly Ala Gln Leu Asn Gln
        355                 360                 365

Phe Ala Leu His Pro Leu Arg Ile Val Arg Ala Met Gln Asp Ile Val
    370                 375                 380

Asn Ser Asp Val Thr Leu Thr Val Asp Met Gly Ser Phe His Ile Trp
385                 390                 395                 400

Ile Ala Arg Tyr Leu Tyr Thr Phe Arg Ala Arg Gln Val Met Ile Ser
                405                 410                 415

Asn Gly Gln Gln Thr Met Gly Val Ala Leu Pro Trp Ala Ile Gly Ala
            420                 425                 430

Trp Leu Val Asn Pro Glu Arg Lys Val Val Ser Val Ser Gly Asp Gly
        435                 440                 445

Gly Phe Leu Gln Ser Ser Met Glu Leu Glu Thr Ala Val Arg Leu Lys
    450                 455                 460

Ala Asn Val Leu His Leu Ile Trp Val Asp Asn Gly Tyr Asn Met Val
465                 470                 475                 480

Ala Ile Gln Glu Glu Lys Lys Tyr Gln Arg Leu Ser Gly Val Glu Phe
                485                 490                 495

Gly Pro Met Asp Phe Lys Ala Tyr Ala Glu Ser Phe Gly Ala Lys Gly
            500                 505                 510

Phe Ala Val Glu Ser Ala Glu Ala Leu Glu Pro Thr Leu Arg Ala Ala
        515                 520                 525

Met Asp Val Asp Gly Pro Ala Val Val Ala Ile Pro Val Asp Tyr Arg
    530                 535                 540

Asp Asn Pro Leu Leu Met Gly Gln Leu His Leu Ser Gln Ile Leu
545                 550                 555

<210>  3
<211>  1476
<212>  DNA
<213>  E. coli

<400>  3
atggctaact acttcaatac actgaatctg cgccagcagc tggcacagct gggcaaatgt      60

cgctttatgg gccgcgatga attcgccgat ggcgcgagct accttcaggg taaaaaagta     120

gtcatcgtcg gctgtggcgc acagggtctg aaccagggcc tgaacatgcg tgattctggt     180

ctcgatatct cctacgctct gcgtaaagaa gcgattgccg agaagcgcgc gtcctggcgt     240

aaagcgaccg aaaatggttt taaagtgggt acttacgaag aactgatccc acaggcggat     300

ctggtgatta acctgacgcc ggacaagcag cactctgatg tagtgcgcac cgtacagcca     360

ctgatgaaag acggcgcggc gctgggctac tcgcacggtt tcaacatcgt cgaagtgggc     420

gagcagatcc gtaaagatat caccgtagtg atggttgcgc cgaaatgccc aggcaccgaa     480

gtgcgtgaag agtacaaacg tgggttcggc gtaccgacgc tgattgccgt tcacccggaa     540

aacgatccga aaggcgaagg catggcgatt gccaaagcct gggcggctgc aaccggtggt     600

caccgtgcgg gtgtgctgga atcgtccttc gttgcggaag tgaaatctga cctgatgggc     660

gagcaaacca tcctgtgcgg tatgttgcag gctggctctc tgctgtgctt cgacaagctg     720

gtggaagaag gtaccgatcc agcatacgca gaaaaactga ttcagttcgg ttgggaaacc     780

atcaccgaag cactgaaaca gggcggcatc accctgatga tggaccgtct ctctaacccg     840

gcgaaactgc gtgcttatgc gctttctgaa cagctgaaag agatcatggc acccctgttc     900

cagaaacata tggacgacat catctccggc gaattctctt ccggtatgat ggcggactgg 960

gccaacgatg ataagaaact gctgacctgg cgtgaagaga ccggcaaaac cgcgtttgaa 1020

accgcgccgc agtatgaagg caaaatcggc gagcaggagt acttcgataa aggcgtactg 1080

atgattgcga tggtgaaagc gggcgttgaa ctggcgttcg aaaccatggt cgattccggc 1140

atcattgaag agtctgcata ttatgaatca ctgcacgagc tgccgctgat tgccaacacc 1200

atcgcccgta agcgtctgta cgaaatgaac gtggttatct ctgataccgc tgagtacggt 1260

aactatctgt tctcttacgc ttgtgtgccg ttgctgaaac cgtttatggc agagctgcaa 1320

ccgggcgacc tgggtaaagc tattccggaa ggcgcggtag ataacgggca actgcgtgat 1380

gtgaacgaag cgattcgcag ccatgcgatt gagcaggtag gtaagaaact gcgcggctat 1440

atgacagata tgaaacgtat tgctgttgcg ggttaa 1476

<210> 4
<211> 491
<212> PRT
<213> E. coli

<400> 4

Met Ala Asn Tyr Phe Asn Thr Leu Asn Leu Arg Gln Gln Leu Ala Gln
1               5                   10                  15

Leu Gly Lys Cys Arg Phe Met Gly Arg Asp Glu Phe Ala Asp Gly Ala
            20                  25                  30

Ser Tyr Leu Gln Gly Lys Lys Val Val Ile Val Gly Cys Gly Ala Gln
        35                  40                  45

Gly Leu Asn Gln Gly Leu Asn Met Arg Asp Ser Gly Leu Asp Ile Ser
    50                  55                  60

Tyr Ala Leu Arg Lys Glu Ala Ile Ala Glu Lys Arg Ala Ser Trp Arg
65                  70                  75                  80

Lys Ala Thr Glu Asn Gly Phe Lys Val Gly Thr Tyr Glu Glu Leu Ile
                85                  90                  95

Pro Gln Ala Asp Leu Val Ile Asn Leu Thr Pro Asp Lys Gln His Ser
            100                 105                 110

Asp Val Val Arg Thr Val Gln Pro Leu Met Lys Asp Gly Ala Ala Leu
        115                 120                 125

Gly Tyr Ser His Gly Phe Asn Ile Val Glu Val Gly Glu Gln Ile Arg
    130                 135                 140

Lys Asp Ile Thr Val Val Met Val Ala Pro Lys Cys Pro Gly Thr Glu

145                 150                 155                 160

Val Arg Glu Glu Tyr Lys Arg Gly Phe Gly Val Pro Thr Leu Ile Ala
                165                 170                 175

Val His Pro Glu Asn Asp Pro Lys Gly Glu Gly Met Ala Ile Ala Lys
            180                 185                 190

Ala Trp Ala Ala Ala Thr Gly Gly His Arg Ala Gly Val Leu Glu Ser
        195                 200                 205

Ser Phe Val Ala Glu Val Lys Ser Asp Leu Met Gly Glu Gln Thr Ile
    210                 215                 220

Leu Cys Gly Met Leu Gln Ala Gly Ser Leu Leu Cys Phe Asp Lys Leu
225                 230                 235                 240

Val Glu Glu Gly Thr Asp Pro Ala Tyr Ala Glu Lys Leu Ile Gln Phe
                245                 250                 255

Gly Trp Glu Thr Ile Thr Glu Ala Leu Lys Gln Gly Gly Ile Thr Leu
            260                 265                 270

Met Met Asp Arg Leu Ser Asn Pro Ala Lys Leu Arg Ala Tyr Ala Leu
        275                 280                 285

Ser Glu Gln Leu Lys Glu Ile Met Ala Pro Leu Phe Gln Lys His Met
    290                 295                 300

Asp Asp Ile Ile Ser Gly Glu Phe Ser Ser Gly Met Met Ala Asp Trp
305                 310                 315                 320

Ala Asn Asp Asp Lys Lys Leu Leu Thr Trp Arg Glu Glu Thr Gly Lys
                325                 330                 335

Thr Ala Phe Glu Thr Ala Pro Gln Tyr Glu Gly Lys Ile Gly Glu Gln
            340                 345                 350

Glu Tyr Phe Asp Lys Gly Val Leu Met Ile Ala Met Val Lys Ala Gly
        355                 360                 365

Val Glu Leu Ala Phe Glu Thr Met Val Asp Ser Gly Ile Ile Glu Glu
    370                 375                 380

Ser Ala Tyr Tyr Glu Ser Leu His Glu Leu Pro Leu Ile Ala Asn Thr
385                 390                 395                 400

Ile Ala Arg Lys Arg Leu Tyr Glu Met Asn Val Val Ile Ser Asp Thr
                405                 410                 415

Ala Glu Tyr Gly Asn Tyr Leu Phe Ser Tyr Ala Cys Val Pro Leu Leu
            420                 425                 430

Lys Pro Phe Met Ala Glu Leu Gln Pro Gly Asp Leu Gly Lys Ala Ile
        435                 440                 445

Pro Glu Gly Ala Val Asp Asn Gly Gln Leu Arg Asp Val Asn Glu Ala
    450                 455                 460

Ile Arg Ser His Ala Ile Glu Gln Val Gly Lys Lys Leu Arg Gly Tyr
465                 470                 475                 480

Met Thr Asp Met Lys Arg Ile Ala Val Ala Gly
                485                 490

<210>  5
<211>  1851
<212>  DNA
<213>  E. coli

<400>  5
atgcctaagt accgttccgc caccaccact catggtcgta atatggcggg tgctcgtgcg      60

ctgtggcgcg ccaccggaat gaccgacgcc gatttcggta agccgattat cgcggttgtg     120

aactcgttca cccaatttgt accgggtcac gtccatctgc gcgatctcgg taaactggtc     180

gccgaacaaa ttgaagcggc tggcggcgtt gccaaagagt tcaacaccat tgcggtggat     240

gatgggattg ccatgggcca cggggggatg ctttattcac tgccatctcg cgaactgatc     300

gctgattccg ttgagtatat ggtcaacgcc cactgcgccg acgccatggt ctgcatctct     360

aactgcgaca aaatcacccc ggggatgctg atggcttccc tgcgcctgaa tattccggtg     420

atctttgttt ccggcggccc gatggaggcc gggaaaacca aactttccga tcagatcatc     480

aagctcgatc tggttgatgc gatgatccag ggcgcagacc cgaaagtatc tgactcccag     540

agcgatcagg ttgaacgttc cgcgtgtccg acctgcggtt cctgctccgg gatgtttacc     600

gctaactcaa tgaactgcct gaccgaagcg ctgggcctgt cgcagccggg caacggctcg     660

ctgctggcaa cccacgccga ccgtaagcag ctgttcctta atgctggtaa acgcattgtt     720

gaattgacca aacgttatta cgagcaaaac gacgaaagtg cactgccgcg taatatcgcc     780

agtaaggcgg cgtttgaaaa cgccatgacg ctggatatcg cgatgggtgg atcgactaac     840

accgtacttc acctgctggc ggcggcgcag gaagcggaaa tcgacttcac catgagtgat     900

atcgataagc tttcccgcaa ggttccacag ctgtgtaaag ttgcgccgag cacccagaaa     960

taccatatgg aagatgttca ccgtgctggt ggtgttatcg gtattctcgg cgaactggat    1020

cgcgcggggt tactgaaccg tgatgtgaaa aacgtacttg gcctgacgtt gccgcaaacg    1080

ctggaacaat acgacgttat gctgacccag gatgacgcgg taaaaaatat gttccgcgca    1140

ggtcctgcag gcattcgtac cacacaggca ttctcgcaag attgccgttg ggatacgctg 1200

gacgacgatc gcgccaatgg ctgtatccgc tcgctggaac acgcctacag caaagacggc 1260

ggcctggcgg tgctctacgg taactttgcg gaaaacggct gcatcgtgaa aacggcaggc 1320

gtcgatgaca gcatcctcaa attcaccggc ccggcgaaag tgtacgaaag ccaggacgat 1380

gcggtagaag cgattctcgg cggtaaagtt gtcgccggag atgtggtagt aattcgctat 1440

gaaggcccga aaggcggtcc ggggatgcag gaaatgctct acccaaccag cttcctgaaa 1500

tcaatgggtc tcggcaaagc ctgtgcgctg atcaccgacg gtcgtttctc tggtggcacc 1560

tctggtcttt ccatcggcca cgtctcaccg gaagcggcaa gcggcggcag cattggcctg 1620

attgaagatg gtgacctgat cgctatcgac atcccgaacc gtggcattca gttacaggta 1680

agcgatgccg aactggcggc gcgtcgtgaa gcgcaggacg ctcgaggtga caaagcctgg 1740

acgccgaaaa atcgtgaacg tcaggtctcc tttgccctgc gtgcttatgc cagcctggca 1800

accagcgccg acaaaggcgc ggtgcgcgat aaatcgaaac tggggggtta a 1851

<210> 6
<211> 616
<212> PRT
<213> E. coli

<400> 6

Met Pro Lys Tyr Arg Ser Ala Thr Thr Thr His Gly Arg Asn Met Ala
1               5                   10                  15

Gly Ala Arg Ala Leu Trp Arg Ala Thr Gly Met Thr Asp Ala Asp Phe
            20                  25                  30

Gly Lys Pro Ile Ile Ala Val Val Asn Ser Phe Thr Gln Phe Val Pro
        35                  40                  45

Gly His Val His Leu Arg Asp Leu Gly Lys Leu Val Ala Glu Gln Ile
    50                  55                  60

Glu Ala Ala Gly Gly Val Ala Lys Glu Phe Asn Thr Ile Ala Val Asp
65                  70                  75                  80

Asp Gly Ile Ala Met Gly His Gly Gly Met Leu Tyr Ser Leu Pro Ser
                85                  90                  95

Arg Glu Leu Ile Ala Asp Ser Val Glu Tyr Met Val Asn Ala His Cys
            100                 105                 110

Ala Asp Ala Met Val Cys Ile Ser Asn Cys Asp Lys Ile Thr Pro Gly
        115                 120                 125

Met Leu Met Ala Ser Leu Arg Leu Asn Ile Pro Val Ile Phe Val Ser
    130                 135                 140

Gly Gly Pro Met Glu Ala Gly Lys Thr Lys Leu Ser Asp Gln Ile Ile
145                 150                 155                 160

Lys Leu Asp Leu Val Asp Ala Met Ile Gln Gly Ala Asp Pro Lys Val
                165                 170                 175

Ser Asp Ser Gln Ser Asp Gln Val Glu Arg Ser Ala Cys Pro Thr Cys
            180                 185                 190

Gly Ser Cys Ser Gly Met Phe Thr Ala Asn Ser Met Asn Cys Leu Thr
        195                 200                 205

Glu Ala Leu Gly Leu Ser Gln Pro Gly Asn Gly Ser Leu Leu Ala Thr
    210                 215                 220

His Ala Asp Arg Lys Gln Leu Phe Leu Asn Ala Gly Lys Arg Ile Val
225                 230                 235                 240

Glu Leu Thr Lys Arg Tyr Tyr Glu Gln Asn Asp Glu Ser Ala Leu Pro
                245                 250                 255

Arg Asn Ile Ala Ser Lys Ala Ala Phe Glu Asn Ala Met Thr Leu Asp
            260                 265                 270

Ile Ala Met Gly Gly Ser Thr Asn Thr Val Leu His Leu Leu Ala Ala
        275                 280                 285

Ala Gln Glu Ala Glu Ile Asp Phe Thr Met Ser Asp Ile Asp Lys Leu
    290                 295                 300

Ser Arg Lys Val Pro Gln Leu Cys Lys Val Ala Pro Ser Thr Gln Lys
305                 310                 315                 320

Tyr His Met Glu Asp Val His Arg Ala Gly Gly Val Ile Gly Ile Leu
                325                 330                 335

Gly Glu Leu Asp Arg Ala Gly Leu Leu Asn Arg Asp Val Lys Asn Val
            340                 345                 350

Leu Gly Leu Thr Leu Pro Gln Thr Leu Glu Gln Tyr Asp Val Met Leu
        355                 360                 365

Thr Gln Asp Asp Ala Val Lys Asn Met Phe Arg Ala Gly Pro Ala Gly
    370                 375                 380

Ile Arg Thr Thr Gln Ala Phe Ser Gln Asp Cys Arg Trp Asp Thr Leu

385                 390                 395                 400

Asp Asp Asp Arg Ala Asn Gly Cys Ile Arg Ser Leu Glu His Ala Tyr
                405                 410                 415

Ser Lys Asp Gly Gly Leu Ala Val Leu Tyr Gly Asn Phe Ala Glu Asn
            420                 425                 430

Gly Cys Ile Val Lys Thr Ala Gly Val Asp Asp Ser Ile Leu Lys Phe
        435                 440                 445

Thr Gly Pro Ala Lys Val Tyr Glu Ser Gln Asp Asp Ala Val Glu Ala
    450                 455                 460

Ile Leu Gly Gly Lys Val Val Ala Gly Asp Val Val Val Ile Arg Tyr
465                 470                 475                 480

Glu Gly Pro Lys Gly Gly Pro Gly Met Gln Glu Met Leu Tyr Pro Thr
                485                 490                 495

Ser Phe Leu Lys Ser Met Gly Leu Gly Lys Ala Cys Ala Leu Ile Thr
            500                 505                 510

Asp Gly Arg Phe Ser Gly Gly Thr Ser Gly Leu Ser Ile Gly His Val
        515                 520                 525

Ser Pro Glu Ala Ala Ser Gly Gly Ser Ile Gly Leu Ile Glu Asp Gly
    530                 535                 540

Asp Leu Ile Ala Ile Asp Ile Pro Asn Arg Gly Ile Gln Leu Gln Val
545                 550                 555                 560

Ser Asp Ala Glu Leu Ala Ala Arg Arg Glu Ala Gln Asp Ala Arg Gly
                565                 570                 575

Asp Lys Ala Trp Thr Pro Lys Asn Arg Glu Arg Gln Val Ser Phe Ala
            580                 585                 590

Leu Arg Ala Tyr Ala Ser Leu Ala Thr Ser Ala Asp Lys Gly Ala Val
        595                 600                 605

Arg Asp Lys Ser Lys Leu Gly Gly
    610                 615

<210>  7
<211>  1662
<212>  DNA
<213>  Lactococcus lactis

<400>  7

tctagacata tgtatactgt gggggattac ctgctggatc gcctgcacga actggggatt 60

gaagaaattt tcggtgtgcc aggcgattat aacctgcagt tcctggacca gattatctcg 120

cacaaagata tgaagtgggt cggtaacgcc aacgaactga acgcgagcta tatggcagat 180

ggttatgccc gtaccaaaaa agctgctgcg tttctgacga cctttggcgt tggcgaactg 240

agcgccgtca acggactggc aggaagctac gccgagaacc tgccagttgt cgaaattgtt 300

gggtcgccta cttctaaggt tcagaatgaa ggcaaatttg tgcaccatac tctggctgat 360

ggggatttta aacattttat gaaaatgcat gaaccggtta ctgcggcccg cacgctgctg 420

acagcagaga atgctacggt tgagatcgac cgcgtcctgt ctgcgctgct gaaagagcgc 480

aagccggtat atatcaatct gcctgtcgat gttgccgcag cgaaagccga aaagccgtcg 540

ctgccactga aaaaagaaaa cagcacctcc aatacatcgg accaggaaat tctgaataaa 600

atccaggaat cactgaagaa tgcgaagaaa ccgatcgtca tcaccggaca tgagatcatc 660

tcttttggcc tggaaaaaac ggtcacgcag ttcatttcta agaccaaact gcctatcacc 720

accctgaact tcggcaaatc tagcgtcgat gaagcgctgc cgagttttct gggtatctat 780

aatggtaccc tgtccgaacc gaacctgaaa gaattcgtcg aaagcgcgga ctttatcctg 840

atgctgggcg tgaaactgac ggatagctcc acaggcgcat ttacccacca tctgaacgag 900

aataaaatga tttccctgaa tatcgacgaa ggcaaaatct ttaacgagcg catccagaac 960

ttcgattttg aatctctgat tagttcgctg ctggatctgt ccgaaattga gtataaaggt 1020

aaatatattg ataaaaaaca ggaggatttt gtgccgtcta atgcgctgct gagtcaggat 1080

cgtctgtggc aagccgtaga aaacctgaca cagtctaatg aaacgattgt tgcggaacag 1140

ggaacttcat ttttcggcgc ctcatccatt tttctgaaat ccaaaagcca tttcattggc 1200

caaccgctgt gggggagtat tggttatacc tttccggcgg cgctgggttc acagattgca 1260

gataaggaat cacgccatct gctgtttatt ggtgacggca gcctgcagct gactgtccag 1320

gaactggggc tggcgatccg tgaaaaaatc aatccgattt gctttatcat caataacgac 1380

ggctacaccg tcgaacgcga aattcatgga ccgaatcaaa gttacaatga catcccgatg 1440

tggaactata gcaaactgcc ggaatccttt ggcgcgacag aggatcgcgt ggtgagtaaa 1500

attgtgcgta cggaaaacga atttgtgtcg gttatgaaag aagcgcaggc tgacccgaat 1560

cgcatgtatt ggattgaact gatcctggca aaagaaggcg caccgaaagt tctgaaaaag 1620

atggggaaac tgtttgcgga gcaaaataaa agctaaggat cc 1662

<210> 8
<211> 548
<212> PRT
<213> Lactococcus lactis

<400> 8

Met Tyr Thr Val Gly Asp Tyr Leu Leu Asp Arg Leu His Glu Leu Gly

1               5                   10                  15

Ile Glu Glu Ile Phe Gly Val Pro Gly Asp Tyr Asn Leu Gln Phe Leu
            20                  25                  30

Asp Gln Ile Ile Ser His Lys Asp Met Lys Trp Val Gly Asn Ala Asn
        35                  40                  45

Glu Leu Asn Ala Ser Tyr Met Ala Asp Gly Tyr Ala Arg Thr Lys Lys
    50                  55                  60

Ala Ala Ala Phe Leu Thr Thr Phe Gly Val Gly Glu Leu Ser Ala Val
65                  70                  75                  80

Asn Gly Leu Ala Gly Ser Tyr Ala Glu Asn Leu Pro Val Val Glu Ile
                85                  90                  95

Val Gly Ser Pro Thr Ser Lys Val Gln Asn Glu Gly Lys Phe Val His
            100                 105                 110

His Thr Leu Ala Asp Gly Asp Phe Lys His Phe Met Lys Met His Glu
        115                 120                 125

Pro Val Thr Ala Ala Arg Thr Leu Leu Thr Ala Glu Asn Ala Thr Val
    130                 135                 140

Glu Ile Asp Arg Val Leu Ser Ala Leu Leu Lys Glu Arg Lys Pro Val
145                 150                 155                 160

Tyr Ile Asn Leu Pro Val Asp Val Ala Ala Ala Lys Ala Glu Lys Pro
                165                 170                 175

Ser Leu Pro Leu Lys Lys Glu Asn Ser Thr Ser Asn Thr Ser Asp Gln
            180                 185                 190

Glu Ile Leu Asn Lys Ile Gln Glu Ser Leu Lys Asn Ala Lys Lys Pro
        195                 200                 205

Ile Val Ile Thr Gly His Glu Ile Ile Ser Phe Gly Leu Glu Lys Thr
    210                 215                 220

Val Thr Gln Phe Ile Ser Lys Thr Lys Leu Pro Ile Thr Thr Leu Asn
225                 230                 235                 240

Phe Gly Lys Ser Ser Val Asp Glu Ala Leu Pro Ser Phe Leu Gly Ile
                245                 250                 255

Tyr Asn Gly Thr Leu Ser Glu Pro Asn Leu Lys Glu Phe Val Glu Ser
            260                 265                 270

Ala Asp Phe Ile Leu Met Leu Gly Val Lys Leu Thr Asp Ser Ser Thr
        275                 280                 285

Gly Ala Phe Thr His His Leu Asn Glu Asn Lys Met Ile Ser Leu Asn
    290                 295                 300

Ile Asp Glu Gly Lys Ile Phe Asn Glu Arg Ile Gln Asn Phe Asp Phe
305                 310                 315                 320

Glu Ser Leu Ile Ser Ser Leu Leu Asp Leu Ser Glu Ile Glu Tyr Lys
                325                 330                 335

Gly Lys Tyr Ile Asp Lys Lys Gln Glu Asp Phe Val Pro Ser Asn Ala
            340                 345                 350

Leu Leu Ser Gln Asp Arg Leu Trp Gln Ala Val Glu Asn Leu Thr Gln
        355                 360                 365

Ser Asn Glu Thr Ile Val Ala Glu Gln Gly Thr Ser Phe Phe Gly Ala
    370                 375                 380

Ser Ser Ile Phe Leu Lys Ser Lys Ser His Phe Ile Gly Gln Pro Leu
385                 390                 395                 400

Trp Gly Ser Ile Gly Tyr Thr Phe Pro Ala Ala Leu Gly Ser Gln Ile
                405                 410                 415

Ala Asp Lys Glu Ser Arg His Leu Leu Phe Ile Gly Asp Gly Ser Leu
            420                 425                 430

Gln Leu Thr Val Gln Glu Leu Gly Leu Ala Ile Arg Glu Lys Ile Asn
        435                 440                 445

Pro Ile Cys Phe Ile Ile Asn Asn Asp Gly Tyr Thr Val Glu Arg Glu
    450                 455                 460

Ile His Gly Pro Asn Gln Ser Tyr Asn Asp Ile Pro Met Trp Asn Tyr
465                 470                 475                 480

Ser Lys Leu Pro Glu Ser Phe Gly Ala Thr Glu Asp Arg Val Val Ser
                485                 490                 495

Lys Ile Val Arg Thr Glu Asn Glu Phe Val Ser Val Met Lys Glu Ala
            500                 505                 510

Gln Ala Asp Pro Asn Arg Met Tyr Trp Ile Glu Leu Ile Leu Ala Lys
        515                 520                 525

Glu Gly Ala Pro Lys Val Leu Lys Lys Met Gly Lys Leu Phe Ala Glu
    530                 535                 540

Gln Asn Lys Ser
545

<210>  9
<211>  1164
<212>  DNA
<213>  E. coli

<400>  9
atgaacaact ttaatctgca caccccaacc cgcattctgt ttggtaaagg cgcaatcgct      60

ggtttacgcg aacaaattcc tcacgatgct cgcgtattga ttacctacgg cggcggcagc     120

gtgaaaaaaa ccggcgttct cgatcaagtt ctggatgccc tgaaaggcat ggacgtgctg     180

gaatttggcg gtattgagcc aaacccggct tatgaaacgc tgatgaacgc cgtgaaactg     240

gttcgcgaac agaaagtgac tttcctgctg gcggttggcg gcggttctgt actggacggc     300

accaaattta tcgccgcagc ggctaactat ccggaaaata tcgatccgtg gcacattctg     360

caaacgggcg gtaaagagat taaaagcgcc atcccgatgg gctgtgtgct gacgctgcca     420

gcaaccggtt cagaatccaa cgcaggcgcg gtgatctccc gtaaaaccac aggcgacaag     480

caggcgttcc attctgccca tgttcagccg gtatttgccg tgctcgatcc ggtttatacc     540

tacaccctgc cgccgcgtca ggtggctaac ggcgtagtgg acgcctttgt acacaccgtg     600

gaacagtatg ttaccaaacc ggttgatgcc aaaattcagg accgtttcgc agaaggcatt     660

ttgctgacgc taatcgaaga tggtccgaaa gccctgaaag agccagaaaa ctacgatgtg     720

cgcgccaacg tcatgtgggc ggcgactcag gcgctgaacg gtttgattgg cgctggcgta     780

ccgcaggact gggcaacgca tatgctgggc cacgaactga ctgcgatgca cggtctggat     840

cacgcgcaaa cactggctat cgtcctgcct gcactgtgga atgaaaaacg cgataccaag     900

cgcgctaagc tgctgcaata tgctgaacgc gtctggaaca tcactgaagg ttccgatgat     960

gagcgtattg acgccgcgat tgccgcaacc cgcaatttct ttgagcaatt aggcgtgccg    1020

acccacctct ccgactacgg tctggacggc agctccatcc cggctttgct gaaaaaactg    1080

gaagagcacg gcatgaccca actgggcgaa aatcatgaca ttacgttgga tgtcagccgc    1140

cgtatatacg aagccgcccg ctaa                                           1164

<210>  10
<211>  387
<212>  PRT
<213>  E. coli

<400>  10

Met Asn Asn Phe Asn Leu His Thr Pro Thr Arg Ile Leu Phe Gly Lys
1               5                   10                  15

Gly Ala Ile Ala Gly Leu Arg Glu Gln Ile Pro His Asp Ala Arg Val
            20                  25                  30

Leu Ile Thr Tyr Gly Gly Gly Ser Val Lys Lys Thr Gly Val Leu Asp
        35                  40                  45

Gln Val Leu Asp Ala Leu Lys Gly Met Asp Val Leu Glu Phe Gly Gly
    50                  55                  60

Ile Glu Pro Asn Pro Ala Tyr Glu Thr Leu Met Asn Ala Val Lys Leu
65                  70                  75                  80

Val Arg Glu Gln Lys Val Thr Phe Leu Leu Ala Val Gly Gly Gly Ser
                85                  90                  95

Val Leu Asp Gly Thr Lys Phe Ile Ala Ala Ala Ala Asn Tyr Pro Glu
            100                 105                 110

Asn Ile Asp Pro Trp His Ile Leu Gln Thr Gly Gly Lys Glu Ile Lys
        115                 120                 125

Ser Ala Ile Pro Met Gly Cys Val Leu Thr Leu Pro Ala Thr Gly Ser
    130                 135                 140

Glu Ser Asn Ala Gly Ala Val Ile Ser Arg Lys Thr Thr Gly Asp Lys
145                 150                 155                 160

Gln Ala Phe His Ser Ala His Val Gln Pro Val Phe Ala Val Leu Asp
                165                 170                 175

Pro Val Tyr Thr Tyr Thr Leu Pro Pro Arg Gln Val Ala Asn Gly Val
            180                 185                 190

Val Asp Ala Phe Val His Thr Val Glu Gln Tyr Val Thr Lys Pro Val
        195                 200                 205

Asp Ala Lys Ile Gln Asp Arg Phe Ala Glu Gly Ile Leu Leu Thr Leu
    210                 215                 220

Ile Glu Asp Gly Pro Lys Ala Leu Lys Glu Pro Glu Asn Tyr Asp Val
225                 230                 235                 240

Arg Ala Asn Val Met Trp Ala Ala Thr Gln Ala Leu Asn Gly Leu Ile
                245                 250                 255

Gly Ala Gly Val Pro Gln Asp Trp Ala Thr His Met Leu Gly His Glu
            260                 265                 270

Leu Thr Ala Met His Gly Leu Asp His Ala Gln Thr Leu Ala Ile Val
        275                 280                 285

Leu Pro Ala Leu Trp Asn Glu Lys Arg Asp Thr Lys Arg Ala Lys Leu
    290                 295                 300

Leu Gln Tyr Ala Glu Arg Val Trp Asn Ile Thr Glu Gly Ser Asp Asp
305                 310                 315                 320

Glu Arg Ile Asp Ala Ala Ile Ala Ala Thr Arg Asn Phe Phe Glu Gln
                325                 330                 335

Leu Gly Val Pro Thr His Leu Ser Asp Tyr Gly Leu Asp Gly Ser Ser
            340                 345                 350

Ile Pro Ala Leu Leu Lys Lys Leu Glu Glu His Gly Met Thr Gln Leu
        355                 360                 365

Gly Glu Asn His Asp Ile Thr Leu Asp Val Ser Arg Arg Ile Tyr Glu
    370                 375                 380

Ala Ala Arg
385

<210>  11
<211>  29
<212>  DNA
<213>  Artificial Sequence

<220>
<223>  Primer

<400>  11
caccatggac aaacagtatc cggtacgcc                                        29

<210>  12
<211>  25
<212>  DNA
<213>  Artificial Sequence

<220>
<223>  Primer

<400>  12
cgaagggcga tagctttacc aatcc                                            25

<210>  13
<211>  29
<212>  DNA
<213>  Artificial Sequence

<220>
<223>  Primer

<400>  13
caccatggct aactacttca atacactga                                          29


<210>  14
<211>  28
<212>  DNA
<213>  Artificial Sequence

<220>
<223>  Primer

<400>  14
ccaggagaag gccttgagtg ttttctcc                                           28


<210>  15
<211>  29
<212>  DNA
<213>  Artificial Sequence

<220>
<223>  Primer

<400>  15
caccatgcct aagtaccgtt ccgccacca                                          29


<210>  16
<211>  26
<212>  DNA
<213>  Artificial Sequence

<220>
<223>  Primer

<400>  16
cgcagcactg ctcttaaata ttcggc                                             26


<210>  17
<211>  29
<212>  DNA
<213>  Artificial Sequence

<220>
<223>  Primer

<400>  17
caccatgaac aactttaatc tgcacaccc                                          29


<210>  18
<211>  29
<212>  DNA
<213>  Artificial Sequence

<220>
<223>  Primer

<400>  18
caccatgaac aactttaatc tgcacaccc                                          29


<210>  19

<211>  45
<212>  DNA
<213>  Artificial Sequence

<220>
<223>  Primer

<400>  19
gcatgcctta agaaaggagg ggggtcacat ggacaaacag tatcc                    45


<210>  20
<211>  39
<212>  DNA
<213>  Artificial Sequence

<220>
<223>  Primer

<400>  20
atgcatttaa ttaattacag aatctgactc agatgcagc                           39


<210>  21
<211>  45
<212>  DNA
<213>  Artificial Sequence

<220>
<223>  Primer

<400>  21
gtcgacgcta gcaaaggagg gaatcaccat ggctaactac ttcaa                    45


<210>  22
<211>  31
<212>  DNA
<213>  Artificial Sequence

<220>
<223>  Primer

<400>  22
tctagattaa cccgcaacag caatacgttt c                                   31


<210>  23
<211>  39
<212>  DNA
<213>  Artificial Sequence

<220>
<223>  Primer

<400>  23
tctagaaaag gaggaataaa gtatgcctaa gtaccgttc                           39


<210>  24
<211>  31
<212>  DNA
<213>  Artificial Sequence

<220>

<223>  Primer

<400>  24
ggatccttat taacccccca gtttcgattt a                                        31


<210>  25
<211>  39
<212>  DNA
<213>  Artificial Sequence

<220>
<223>  Primer

<400>  25
ggatccaaag gaggctagac atatgtatac tgtgggga                                39


<210>  26
<211>  31
<212>  DNA
<213>  Artificial Sequence

<220>
<223>  Primer

<400>  26
gagctcttag cttttatttt gctccgcaaa c                                       31


<210>  27
<211>  39
<212>  DNA
<213>  Artificial Sequence

<220>
<223>  Primer

<400>  27
gagctcaaag gaggagcaag taatgaacaa ctttaatct                               39


<210>  28
<211>  43
<212>  DNA
<213>  Artificial Sequence

<220>
<223>  Primer

<400>  28
gaattcacta gtcctaggtt agcgggcggc ttcgtatata cgg                          43


<210>  29
<211>  25
<212>  DNA
<213>  Artificial Sequence

<220>
<223>  Primer

<400>  29
caacattagc gattttcttt tctct                                              25

<210>  30
<211>  45
<212>  DNA
<213>  Artificial Sequence

<220>
<223>  Primer

<400>  30
catgaagctt actagtgggc ttaagttttg aaaataatga aaact                45


<210>  31
<211>  61
<212>  DNA
<213>  Artificial Sequence

<220>
<223>  Primer N110.2

<400>  31
gagctcacta gtcaattgta agtaagtaaa aggaggtggg tcacatggac aaacagtatc      60

c                                                                      61


<210>  32
<211>  50
<212>  DNA
<213>  Artificial Sequence

<220>
<223>  Primer N111.2

<400>  32
ggatccgatc gacttaagcc tcagcttaca gaatctgact cagatgcagc              50


<210>  33
<211>  44
<212>  DNA
<213>  Artificial Sequence

<220>
<223>  Primer N112.2

<400>  33
gagctcctta agaaggaggt aatcaccatg gctaactact tcaa                  44


<210>  34
<211>  51
<212>  DNA
<213>  Artificial Sequence

<220>
<223>  Primer N113.2

<400>  34
ggatccgatc gagctagcgc ggccgcttaa cccgcaacag caatacgttt c            51


<210>  35
<211>  44

<212>  DNA
<213>  Artificial Sequence

<220>
<223>  Primer N114.2

<400>  35
gagctcgcta gcaaggaggt ataaagtatg cctaagtacc gttc                    44


<210>  36
<211>  52
<212>  DNA
<213>  Artificial Sequence

<220>
<223>  Primer N115.2

<400>  36
ggatccgatc gattaattaa cctaaggtta ttaacccccc agtttcgatt ta            52


<210>  37
<211>  46
<212>  DNA
<213>  Artificial Sequence

<220>
<223>  Primer N116.2

<400>  37
gagctcttaa ttaaaaggag gttagacata tgtatactgt gggga                   46


<210>  38
<211>  49
<212>  DNA
<213>  Artificial Sequence

<220>
<223>  Primer 117.2

<400>  38
ggatccagat ctcctaggac atgtttagct tttattttgc tccgcaaac               49


<210>  39
<211>  3883
<212>  DNA
<213>  Escherichia coli

<400>  39
ctatattgct gaaggtacag gcgtttccat aactatttgc tcgcgttttt tactcaagaa    60

gaaaatgcca aatagcaaca tcaggcagac aatacccgaa attgcgaaga aaactgtctg   120

gtagcctgcg tggtcaaaga gtatcccagt cggcgttgaa agcagcacaa tcccaagcga   180

actggcaatt tgaaaaccaa tcagaaagat cgtcgacgac aggcgcttat caaagtttgc   240

cacgctgtat ttgaagacgg atatgacaca aagtggaacc tcaatggcat gtaacaactt   300

cactaatgaa ataatccagg ggttaacgaa cagcgcgcag gaaaggatac gcaacgccat   360

aatcacaact ccgataagta atgcattttt tggccctacc cgattcacaa agaaaggaat   420

aatcgccatg cacagcgctt cgagtaccac ctggaatgag ttgagataac catacaggcg 480

cgttcctaca tcgtgtgatt cgaataaacc tgaataaaag acaggaaaaa gttgttgatc 540

aaaaatgtta tagaaagacc acgtccccac aataaatatg acgaaaaccc agaagtttcg 600

atccttgaaa actgcgataa aatcctcttt ttttacccct cccgcatctg ccgctacgca 660

ctggtgatcc ttatctttaa aacgcatgtt gatcatcata aatacagcgc caaatagcga 720

gaccaaccag aagttgatat ggggactgat actaaaaaat atgccggcaa agaacgcgcc 780

aatagcatag ccaaaagatc cccaggcgcg cgctgttcca tattcgaaat gaaaatttcg 840

cgccattttt tcggtgaagc tatcaagcaa accgcatccc gccagatacc ccaagccaaa 900

aaatagcgcc cccagaatta gacctacaga aaaattgctt tgcagtaacg gttcataaac 960

gtaaatcata aacggtccgg tcaagaccag gatgaaactc atacaccaga tgagcggttt 1020

cttcagaccg agtttatcct gaacgatgcc gtagaacatc ataaatagaa tgctggtaaa 1080

ctggttgacc gaataaagtg tacctaattc cgtccctgtc aaccctagat gtcctttcag 1140

ccaaatagcg tataacgacc accacagcga ccaggaaata aaaaagagaa atgagtaact 1200

ggatgcaaaa cgatagtacg catttctgaa tggaatattc agtgccataa ttacctgcct 1260

gtcgttaaaa aattcacgtc ctatttagag ataagagcga cttcgccgtt tacttctcac 1320

tattccagtt cttgtcgaca tggcagcgct gtcattgccc ctttcgccgt tactgcaagc 1380

gctccgcaac gttgagcgag atcgataatt cgtcgcattt ctctctcatc tgtagataat 1440

cccgtagagg acagacctgt gagtaacccg gcaacgaacg catctcccgc ccccgtgcta 1500

tcgacacaat tcacagacat tccagcaaaa tggtgaactt gtcctcgata acagaccacc 1560

accccttctg cacctttagt caccaacagc atggcgatct catactcttt tgccagggcg 1620

catatatcct gatcgttctg tgtttttcca ctgataagtc gccattcttc ttccgagagc 1680

ttgacgacat ccgccagttg tagcgcctgc cgcaaacaca agcggagcaa atgctcgtct 1740

tgccatagat cttcacgaat attaggatcg aagctgacaa aacctccggc atgccggatc 1800

gccgtcatcg cagtaaatgc gctggtacgc gaaggctcgg cagacaacgc aattgaacag 1860

agatgtaacc attcgccatg tcgccagcag ggcaagtctg tcgtctctaa aaaaagatcg 1920

gcactggggc ggaccataaa cgtaaatgaa cgttcccctt gatcgttcag atcgacaagc 1980

accgtggatg tccggtgcca ttcatcttgc ttcagatacg tgatatcgac tccctcagtt 2040

agcagcgttc tttgcattaa cgcaccaaaa ggatcatccc ccacccgacc tataaaccca 2100

cttgttccgc ctaatctggc gattcccacc gcaacgttag ctggcgcgcc gccaggacaa 2160

ggcagtaggc gcccgtctga ttctggcaag agatctacga ccgcatcccc taaaacccat 2220

actttggctg acattttttt cccttaaatt catctgagtt acgcatagtg ataaacctct 2280

ttttcgcaaa atcgtcatgg atttactaaa acatgcatat tcgatcacaa aacgtcatag 2340

ttaacgttaa catttgtgat attcatcgca tttatgaaag taagggactt tatttttata 2400

aaagttaacg ttaacaattc accaaatttg cttaaccagg atgattaaaa tgacgcaatc 2460

tcgattgcat gcggcgcaaa acgccctagc aaaacttcat gagcaccggg gtaacacttt 2520

ctatccccat tttcacctcg cgcctcctgc cgggtggatg aacgatccaa acggcctgat 2580

ctggtttaac gatcgttatc acgcgtttta tcaacatcat ccgatgagcg aacactgggg 2640

gccaatgcac tggggacatg ccaccagcga cgatatgatc cactggcagc atgagcctat 2700

tgcgctagcg ccaggagacg ataatgacaa agacgggtgt ttttcaggta gtgctgtcga 2760

tgacaatggt gtcctctcac ttatctacac cggacacgtc tggctcgatg gtgcaggtaa 2820

tgacgatgca attcgcgaag tacaatgtct ggctaccagt cgggatggta ttcatttcga 2880

gaaacagggt gtgatcctca ctccaccaga aggaatcatg cacttccgcg atcctaaagt 2940

gtggcgtgaa gccgacacat ggtggatggt agtcggggcg aaagatccag gcaacacggg 3000

gcagatcctg ctttatcgcg gcagttcgtt gcgtgaatgg accttcgatc gcgtactggc 3060

ccacgctgat gcgggtgaaa gctatatgtg ggaatgtccg gactttttca gccttggcga 3120

tcagcattat ctgatgtttt ccccgcaggg aatgaatgcc gagggataca gttaccgaaa 3180

tcgctttcaa agtggcgtaa tacccggaat gtggtcgcca ggacgacttt ttgcacaatc 3240

cgggcatttt actgaacttg ataacgggca tgacttttat gcaccacaaa gctttttagc 3300

gaaggatggt cggcgtattg ttatcggctg gatggatatg tgggaatcgc caatgccctc 3360

aaaacgtgaa ggatgggcag gctgcatgac gctggcgcgc gagctatcag agagcaatgg 3420

caaacttcta caacgcccgg tacacgaagc tgagtcgtta cgccagcagc atcaatctgt 3480

ctctccccgc acaatcagca ataaatatgt tttgcaggaa aacgcgcaag cagttgagat 3540

tcagttgcag tgggcgctga agaacagtga tgccgaacat tacggattac agctcggcac 3600

tggaatgcgg ctgtatattg ataaccaatc tgagcgactt gttttgtggc ggtattaccc 3660

acacgagaat ttagacggct accgtagtat tcccctcccg cagcgtgaca cgctcgccct 3720

aaggatattt atcgatacat catccgtgga agtatttatt aacgacgggg aagcggtgat 3780

gagtagtcga atctatccgc agccagaaga acgggaactg tcgctttatg cctcccacgg 3840

agtggctgtg ctgcaacatg gagcactctg gctactgggt taa 3883

<210> 40
<211> 19
<212> DNA
<213> Artificial Sequence

<220>
<223> Primer N130SeqF1

<400> 40
tgttccaacc tgatcaccg 19

<210>  41
<211>  18
<212>  DNA
<213>  Artificial Sequnce

<220>
<223>  Primer N130SeqF2

<400>  41
ggaaaacagc aaggcgct                                                    18


<210>  42
<211>  18
<212>  DNA
<213>  Artificial Sequence

<220>
<223>  Primer N130SeqF3

<400>  42
cagctgaacc agtttgcc                                                    18


<210>  43
<211>  19
<212>  DNA
<213>  Artificial Sequence

<220>
<223>  Primer N130SeqF4

<400>  43
aaaataccag cgcctgtcc                                                   19


<210>  44
<211>  18
<212>  DNA
<213>  Artificial Sequence

<220>
<223>  Primer N130SeqR1

<400>  44
tgaatggcca ccatgttg                                                    18


<210>  45
<211>  18
<212>  DNA
<213>  Artificial Sequence

<220>
<223>  Primer N130SeqR2

<400>  45
gaggatctcc gccgcctg                                                    18


<210>  46
<211>  18
<212>  DNA
<213>  Artificial Sequence

<220>
<223>  Primer N130SeqR3

<400>  46
aggccgagca ggaagatc                                                    18


<210>  47
<211>  19
<212>  DNA
<213>  Artificial Sequence

<220>
<223>  Primer N130SeqR4

<400>  47
tgatcaggtt ggaacagcc                                                   19


<210>  48
<211>  19
<212>  DNA
<213>  Artificial Sequence

<220>
<223>  Primer N131SeqF1

<400>  48
aagaactgat cccacaggc                                                   19


<210>  49
<211>  19
<212>  DNA
<213>  Artificial Sequence

<220>
<223>  Primer N131SeqF2

<400>  49
atcctgtgcg gtatgttgc                                                   19


<210>  50
<211>  18
<212>  DNA
<213>  Artificial Sequence

<220>
<223>  Primer N131Seqf3

<400>  50
attgcgatgg tgaaagcg                                                    18


<210>  51
<211>  19
<212>  DNA
<213>  Artificial Sequence

<220>
<223>  Primer N131SeqR1

<400>  51
atggtgttgg caatcagcg                                                   19

<210>  52
<211>  18
<212>  DNA
<213>  Artificial Sequence

<220>
<223>  Primer N131SeqR2

<400>  52
gtgcttcggt gatggttt                                                    18


<210>  53
<211>  19
<212>  DNA
<213>  Artificial Sequence

<220>
<223>  Primer N131SeqR3

<400>  53
ttgaaaccgt gcgagtagc                                                   19


<210>  54
<211>  19
<212>  DNA
<213>  Artificial Sequence

<220>
<223>  Primer N132SeqF1

<400>  54
tattcactgc catctcgcg                                                   19


<210>  55
<211>  18
<212>  DNA
<213>  Artificial Sequence

<220>
<223>  Primer N132SeqF2

<400>  55
ccgtaagcag ctgttcct                                                    18


<210>  56
<211>  20
<212>  DNA
<213>  Artificial Sequence

<220>
<223>  Primer N132SeqF3

<400>  56
gctggaacaa tacgacgtta                                                  20


<210>  57
<211>  20
<212>  DNA

<213>  Artificial Sequence

<220>
<223>  Primer N132SeqF4

<400>  57
tgctctaccc aaccagcttc                                                    20


<210>  58
<211>  20
<212>  DNA
<213>  Artificial Sequence

<220>
<223>  Primer N132SeqR1

<400>  58
atggaaagac cagaggtgcc                                                    20


<210>  59
<211>  18
<212>  DNA
<213>  Artificial Sequence

<220>
<223>  Primer N132SeqR2

<400>  59
tgcctgtgtg gtacgaat                                                      18


<210>  60
<211>  19
<212>  DNA
<213>  Artificial Sequence

<220>
<223>  Primer N132SeqR3

<400>  60
tattacgcgg cagtgcact                                                     19


<210>  61
<211>  22
<212>  DNA
<213>  Artificial Sequence

<220>
<223>  Primer N132SeqR4

<400>  61
ggtgattttg tcgcagttag ag                                                 22


<210>  62
<211>  18
<212>  DNA
<213>  Artificial Sequence

<220>
<223>  Primer N133SeqF1

<400>  62
tcgaaattgt tgggtcgc                                                    18


<210>  63
<211>  22
<212>  DNA
<213>  Artificial Sequence

<220>
<223>  Primer N133SeqF2

<400>  63
ggtcacgcag ttcatttcta ag                                               22


<210>  64
<211>  18
<212>  DNA
<213>  Artificial Sequence

<220>
<223>  Primer N133SeqF3

<400>  64
tgtggcaagc cgtagaaa                                                    18


<210>  65
<211>  19
<212>  DNA
<213>  Artificial Sequence

<220>
<223>  Primer N133SeqF4

<400>  65
aggatcgcgt ggtgagtaa                                                   19


<210>  66
<211>  20
<212>  DNA
<213>  Artificial Sequence

<220>
<223>  Primer N133SeqR1

<400>  66
gtagccgtcg ttattgatga                                                  20


<210>  67
<211>  22
<212>  DNA
<213>  Artificial Sequence

<220>
<223>  Primer N133SeqR2

<400>  67
gcagcgaact aatcagagat tc                                               22


<210>  68

<211>  19
<212>  DNA
<213>  Artificial Sequence

<220>
<223>  Primer N133SeqR3

<400>  68
tggtccgatg tattggagg                                                    19


<210>  69
<211>  19
<212>  DNA
<213>  Artificial Sequence

<220>
<223>  Primer N133SeqR4

<400>  69
tctgccatat agctcgcgt                                                    19


<210>  70
<211>  42
<212>  DNA
<213>  Artificial Sequence

<220>
<223>  Promoter 1.6GI Variant

<400>  70
gcccttgaca atgccacatc ctgagcaaat aattcaacca ct                          42


<210>  71
<211>  42
<212>  DNA
<213>  Artificial Sequence

<220>
<223>  Promoter 1.5GI

<400>  71
gcccttgact atgccacatc ctgagcaaat aattcaacca ct                          42


<210>  72
<211>  18
<212>  DNA
<213>  Artificial Sequence

<220>
<223>  Primer Scr1

<400>  72
cctttctttg tgaatcgg                                                     18


<210>  73
<211>  18
<212>  DNA
<213>  Artificial Sequence

<220>

<223>  Primer Scr2

<400>  73
agaaacaggg tgtgatcc                                                    18


<210>  74
<211>  20
<212>  DNA
<213>  Artificial Sequence

<220>
<223>  Primer Scr3

<400>  74
agtgatcatc acctgttgcc                                                  20


<210>  75
<211>  20
<212>  DNA
<213>  Artificial Sequence

<220>
<223>  Primer Scr4

<400>  75
agcacggcga gagtcgacgg                                                  20


<210>  76
<211>  672
<212>  DNA
<213>  Saccharomyces cerevisiae

<400>  76
   agttcgagtt tatcattatc aatactgcca tttcaaagaa tacgtaaata attaatagta      60
   gtgattttcc taactttatt tagtcaaaaa attagccttt taattctgct gtaacccgta     120
   catgcccaaa atagggggcg ggttacacag aatatataac atcgtaggtg tctgggtgaa     180
   cagtttattc ctggcatcca ctaaatataa tggagcccgc tttttaagct ggcatccaga     240
   aaaaaaaaga atcccagcac caaaatattg ttttcttcac caaccatcag ttcataggtc     300
   cattctctta gcgcaactac agagaacagg ggcacaaaca ggcaaaaaac gggcacaacc     360
   tcaatggagt gatgcaacct gcctggagta aatgatgaca caaggcaatt gacccacgca     420
   tgtatctatc tcattttctt acaccttcta ttaccttctg ctctctctga tttggaaaaa     480
   gctgaaaaaa aaggttgaaa ccagttccct gaaattattc ccctacttga ctaataagta     540
   tataaagacg gtaggtattg attgtaattc tgtaaatcta tttcttaaac ttcttaaatt     600
   ctacttttat agttagtctt ttttttagtt ttaaaacacc aagaacttag tttcgaataa     660
   acacacataa ac                                                          672


<210>  77
<211>  270
<212>  DNA
<213>  Saccharomyces cerevisiae

<400>  77
gacctcgagt catgtaatta gttatgtcac gcttacattc acgccctccc cccacatccg      60

ctctaaccga aaaggaagga gttagacaac ctgaagtcta ggtccctatt tattttttta     120

tagttatgtt agtattaaga acgttattta tatttcaaat ttttcttttt tttctgtaca     180

gacgcgtgta cgcatgtaac attatactga aaaccttgct tgagaaggtt ttgggacgct     240

cgaaggcttt aatttgcggc cggtacccaa                                      270


<210>  78
<211>  1716
<212>  DNA
<213>  Bacillus subtilis

<400>  78
atgttgacaa aagcaacaaa agaacaaaaa tcccttgtga aaaacagagg ggcggagctt      60

gttgttgatt gcttagtgga gcaaggtgtc acacatgtat ttggcattcc aggtgcaaaa     120

attgatgcgg tatttgacgc tttacaagat aaaggacctg aaattatcgt tgcccggcac     180

gaacaaaacg cagcattcat ggcccaagca gtcggccgtt taactggaaa accgggagtc     240

gtgttagtca catcaggacc gggtgcctct aacttggcaa caggcctgct gacagcgaac     300

actgaaggag accctgtcgt tgcgcttgct ggaaacgtga tccgtgcaga tcgtttaaaa     360

cggacacatc aatctttgga taatgcggcg ctattccagc cgattacaaa atacagtgta     420

gaagttcaag atgtaaaaaa tataccggaa gctgttacaa atgcatttag gatagcgtca     480

gcagggcagg ctggggccgc ttttgtgagc tttccgcaag atgttgtgaa tgaagtcaca     540

aatacgaaaa acgtgcgtgc tgttgcagcg ccaaaactcg gtcctgcagc agatgatgca     600

atcagtgcgg ccatagcaaa aatccaaaca gcaaaacttc ctgtcgtttt ggtcggcatg     660

aaaggcggaa gaccggaagc aattaaagcg gttcgcaagc ttttgaaaaa ggttcagctt     720

ccatttgttg aaacatatca agctgccggt accctttcta gagatttaga ggatcaatat     780

tttggccgta tcggtttgtt ccgcaaccag cctggcgatt tactgctaga gcaggcagat     840

gttgttctga cgatcggcta tgacccgatt gaatatgatc cgaaattctg gaatatcaat     900

ggagaccgga caattatcca tttagacgag attatcgctg acattgatca tgcttaccag     960

cctgatcttg aattgatcgg tgacattccg tccacgatca atcatatcga acacgatgct    1020

gtgaaagtgg aatttgcaga gcgtgagcag aaaatccttt ctgatttaaa acaatatatg    1080

catgaaggtg agcaggtgcc tgcagattgg aaatcagaca gagcgcaccc tcttgaaatc    1140

gttaaagagt tgcgtaatgc agtcgatgat catgttacag taacttgcga tatcggttcg    1200

cacgccattt ggatgtcacg ttatttccgc agctacgagc cgttaacatt aatgatcagt    1260

aacggtatgc aaacactcgg cgttgcgctt ccttgggcaa tcggcgcttc attggtgaaa    1320

ccgggagaaa aagtggtttc tgtctctggt gacggcggtt tcttattctc agcaatggaa    1380

ttagagacag cagttcgact aaaagcacca attgtacaca ttgtatggaa cgacagcaca 1440

tatgacatgg ttgcattcca gcaattgaaa aaatataacc gtacatctgc ggtcgatttc 1500

ggaaatatcg atatcgtgaa atatgcggaa agcttcggag caactggctt gcgcgtagaa 1560

tcaccagacc agctggcaga tgttctgcgt caaggcatga acgctgaagg tcctgtcatc 1620

atcgatgtcc cggttgacta cagtgataac attaatttag caagtgacaa gcttccgaaa 1680

gaattcgggg aactcatgaa aacgaaagct ctctag 1716

<210> 79
<211> 643
<212> DNA
<213> Saccharomyces cerevisiae

<400> 79
gaaatgaata acaatactga cagtactaaa taattgccta cttggcttca catacgttgc 60

atacgtcgat atagataata atgataatga cagcaggatt atcgtaatac gtaatagttg 120

aaaatctcaa aaatgtgtgg gtcattacgt aaataatgat aggaatggga ttcttctatt 180

tttccttttt ccattctagc agccgtcggg aaaacgtggc atcctctctt tcgggctcaa 240

ttggagtcac gctgccgtga gcatcctctc tttccatatc taacaactga gcacgtaacc 300

aatggaaaag catgagctta gcgttgctcc aaaaaagtat tggatggtta ataccatttg 360

tctgttctct tctgactttg actcctcaaa aaaaaaaaat ctacaatcaa cagatcgctt 420

caattacgcc ctcacaaaaa cttttttcct tcttcttcgc ccacgttaaa ttttatccct 480

catgttgtct aacggatttc tgcacttgat ttattataaa aagacaaaga cataatactt 540

ctctatcaat ttcagttatt gttcttcctt gcgttattct tctgttcttc tttttctttt 600

gtcatatata accataacca agtaatacat attcaaatct aga 643

<210> 80
<211> 1188
<212> DNA
<213> Saccharomyces cerevisiae

<400> 80
atgttgagaa ctcaagccgc cagattgatc tgcaactccc gtgtcatcac tgctaagaga 60

acctttgctt tggccacccg tgctgctgct tacagcagac cagctgcccg tttcgttaag 120

ccaatgatca ctacccgtgg tttgaagcaa atcaacttcg gtggtactgt tgaaaccgtc 180

tacgaaagag ctgactggcc aagagaaaag ttgttggact acttcaagaa cgacactttt 240

gctttgatcg gttacggttc ccaaggttac ggtcaaggtt tgaacttgag agacaacggt 300

ttgaacgtta tcattggtgt ccgtaaagat ggtgcttctt ggaaggctgc catcgaagac 360

ggttgggttc caggcaagaa cttgttcact gttgaagatg ctatcaagag aggtagttac 420

gttatgaact tgttgtccga tgccgctcaa tcagaaacct ggcctgctat caagccattg 480

ttgaccaagg gtaagacttt gtacttctcc cacggtttct ccccagtctt caaggacttg 540

actcacgttg aaccaccaaa ggacttagat gttatcttgg ttgctccaaa gggttccggt 600

agaactgtca gatctttgtt caaggaaggt cgtggtatta actcttctta cgccgtctgg 660

aacgatgtca ccggtaaggc tcacgaaaag gcccaagctt tggccgttgc cattggttcc 720

ggttacgttt accaaaccac tttcgaaaga gaagtcaact ctgacttgta cggtgaaaga 780

ggttgtttaa tgggtggtat ccacggtatg ttcttggctc aatacgacgt cttgagagaa 840

aacggtcact ccccatctga agctttcaac gaaaccgtcg aagaagctac ccaatctcta 900

tacccattga tcggtaagta cggtatggat tacatgtacg atgcttgttc caccaccgcc 960

agaagaggtg ctttggactg gtacccaatc ttcaagaatg ctttgaagcc tgttttccaa 1020

gacttgtacg aatctaccaa gaacggtacc gaaaccaaga gatctttgga attcaactct 1080

caacctgact acagagaaaa gctagaaaag gaattagaca ccatcagaaa catggaaatc 1140

tggaaggttg gtaaggaagt cagaaagttg agaccagaaa accaataa 1188

<210> 81
<211> 760
<212> DNA
<213> Saccharomyces cerevisiae

<400> 81
tcttttccga tttttttcta aaccgtggaa tatttcggat atccttttgt tgtttccggg 60

tgtacaatat ggacttcctc ttttctggca accaaaccca tacatcggga ttcctataat 120

accttcgttg gtctccctaa catgtaggtg gcggagggga gatatacaat agaacagata 180

ccagacaaga cataatgggc taaacaagac tacaccaatt acactgcctc attgatggtg 240

gtacataacg aactaatact gtagccctag acttgatagc catcatcata tcgaagtttc 300

actacccttt ttccatttgc catctattga agtaataata ggcgcatgca acttcttttc 360

tttttttttc ttttctctct cccccgttgt tgtctcacca tatccgcaat gacaaaaaaa 420

tgatggaaga cactaaagga aaaaattaac gacaaagaca gcaccaacag atgtcgttgt 480

tccagagctg atgaggggta tctcgaagca cacgaaactt tttccttcct tcattcacgc 540

acactactct ctaatgagca acggtatacg gccttccttc cagttacttg aatttgaaat 600

aaaaaaaagt ttgctgtctt gctatcaagt ataaatagac ctgcaattat taatcttttg 660

tttcctcgtc attgttctcg ttccctttct tccttgtttc tttttctgca caatatttca 720

agctatacca agcatacaat caactatctc atatacaatg 760

<210> 82
<211> 316
<212> DNA
<213> Saccharomyces cerevisiae

<400> 82
gagtaagcga atttcttatg atttatgatt tttattatta aataagttat aaaaaaaata 60

agtgtataca aattttaaag tgactcttag gttttaaaac gaaaattctt attcttgagt 120

aactctttcc tgtaggtcag gttgctttct caggtatagc atgaggtcgc tcttattgac 180

cacacctcta ccggcatgcc gagcaaatgc ctgcaaatcg ctccccattt cacccaattg 240

tagatatgct aactccagca atgagttgat gaatctcggt gtgtatttta tgtcctcaga 300

ggacaacacc tgtggt 316

<210> 83
<211> 1758
<212> DNA
<213> Saccharomyces cerevisiae

<400> 83
atgggcttgt taacgaaagt tgctacatct agacaattct ctacaacgag atgcgttgca 60

aagaagctca acaagtactc gtatatcatc actgaaccta agggccaagg tgcgtcccag 120

gccatgcttt atgccaccgg tttcaagaag gaagatttca agaagcctca agtcggggtt 180

ggttcctgtt ggtggtccgg taacccatgt aacatgcatc tattggactt gaataacaga 240

tgttctcaat ccattgaaaa agcgggtttg aaagctatgc agttcaacac catcggtgtt 300

tcagacggta tctctatggg tactaaaggt atgagatact cgttacaaag tagagaaatc 360

attgcagact cctttgaaac catcatgatg gcacaacact acgatgctaa catcgccatc 420

ccatcatgtg acaaaaacat gcccggtgtc atgatggcca tgggtagaca taacagacct 480

tccatcatgg tatatggtgg tactatcttg cccggtcatc caacatgtgg ttcttcgaag 540

atctctaaaa acatcgatat cgtctctgcg ttccaatcct acggtgaata tatttccaag 600

caattcactg aagaagaaag agaagatgtt gtggaacatg catgcccagg tcctggttct 660

tgtggtggta tgtatactgc caacacaatg gcttctgccg ctgaagtgct aggtttgacc 720

attccaaact cctcttcctt cccagccgtt tccaaggaga agttagctga gtgtgacaac 780

attggtgaat acatcaagaa gacaatggaa ttgggtattt tacctcgtga tatcctcaca 840

aaagaggctt ttgaaaacgc cattacttat gtcgttgcaa ccggtgggtc cactaatgct 900

gttttgcatt tggtggctgt tgctcactct gcgggtgtca agttgtcacc agatgatttc 960

caaagaatca gtgatactac accattgatc ggtgacttca aaccttctgg taaatacgtc 1020

atggccgatt tgattaacgt tggtggtacc caatctgtga ttaagtatct atatgaaaac 1080

aacatgttgc acggtaacac aatgactgtt accggtgaca ctttggcaga acgtgcaaag 1140

aaagcaccaa gcctacctga aggacaagag attattaagc cactctccca cccaatcaag 1200

gccaacggtc acttgcaaat tctgtacggt tcattggcac caggtggagc tgtgggtaaa 1260

attaccggta aggaaggtac ttacttcaag ggtagagcac gtgtgttcga agaggaaggt 1320

gcctttattg aagccttgga aagaggtgaa atcaagaagg gtgaaaaaac cgttgttgtt 1380

atcagatatg aaggtccaag aggtgcacca ggtatgcctg aaatgctaaa gccttcctct 1440

gctctgatgg gttacggttt gggtaaagat gttgcattgt tgactgatgg tagattctct 1500

ggtggttctc acgggttctt aatcggccac attgttcccg aagccgctga aggtggtcct 1560

atcgggttgg tcagagacgg cgatgagatt atcattgatg ctgataataa caagattgac 1620

ctattagtct ctgataagga aatggctcaa cgtaaacaaa gttgggttgc acctccacct 1680

cgttacacaa gaggtactct atccaagtat gctaagttgg tttccaacgc ttccaacggt 1740

tgtgttttag atgcttga 1758

<210> 84
<211> 753
<212> DNA
<213> Saccharomyces cerevisiae

<400> 84
gcatgcttgc atttagtcgt gcaatgtatg actttaagat ttgtgagcag gaagaaaagg 60

gagaatcttc taacgataaa cccttgaaaa actgggtaga ctacgctatg ttgagttgct 120

acgcaggctg cacaattaca cgagaatgct cccgcctagg atttaaggct aagggacgtg 180

caatgcagac gacagatcta aatgaccgtg tcggtgaagt gttcgccaaa cttttcggtt 240

aacacatgca gtgatgcacg cgcgatggtg ctaagttaca tatatatata tatagccata 300

gtgatgtcta agtaaccttt atggtatatt tcttaatgtg gaaagatact agcgcgcgca 360

cccacacaca agcttcgtct tttcttgaag aaaagaggaa gctcgctaaa tgggattcca 420

ctttccgttc cctgccagct gatggaaaaa ggttagtgga acgatgaaga ataaaaagag 480

agatccactg aggtgaaatt tcagctgaca gcgagtttca tgatcgtgat gaacaatggt 540

aacgagttgt ggctgttgcc agggagggtg gttctcaact tttaatgtat ggccaaatcg 600

ctacttgggt ttgttatata acaaagaaga aataatgaac tgattctctt cctccttctt 660

gtcctttctt aattctgttg taattacctt cctttgtaat tttttttgta attattcttc 720

ttaataatcc aaacaaacac acatattaca ata 753

<210> 85
<211> 20
<212> DNA
<213> Artificial Sequence

<220>
<223> Primer N98SeqF1

<400> 85
cgtgttagtc acatcaggac 20

<210> 86
<211> 24
<212> DNA
<213> Artificial Sequence

<220>
<223> Primer N98SeqF2

<400>  86
ggccatagca aaaatccaaa cagc                                              24


<210>  87
<211>  24
<212>  DNA
<213>  Artificial Sequence

<220>
<223>  Primer N98SeqF3

<400>  87
ccacgatcaa tcatatcgaa cacg                                              24


<210>  88
<211>  20
<212>  DNA
<213>  Artificial Sequence

<220>
<223>  Primer N98SeqF4

<400>  88
ggtttctgtc tctggtgacg                                                   20


<210>  89
<211>  22
<212>  DNA
<213>  Artificial Sequence

<220>
<223>  Primer N99SeqR1

<400>  89
gtctggtgat tctacgcgca ag                                                22


<210>  90
<211>  22
<212>  DNA
<213>  Artificial Sequence

<220>
<223>  Primer N99SeqR2

<400>  90
catcgactgc attacgcaac tc                                                22


<210>  91
<211>  22
<212>  DNA
<213>  Artificial Sequence

<220>
<223>  Primer N99SeqR3

<400>  91
cgatcgtcag aacaacatct gc                                                22

<210>  92
<211>  20
<212>  DNA
<213>  Artificial Sequence

<220>
<223>  Primer N99SeqR4

<400>  92
ccttcagtgt tcgctgtcag                                                   20


<210>  93
<211>  36
<212>  DNA
<213>  Artificial Sequence

<220>
<223>  Primer N136

<400>  93
ccgcggatag atctgaaatg aataacaata ctgaca                                 36


<210>  94
<211>  65
<212>  DNA
<213>  Artificial Sequence

<220>
<223>  Primer N137

<400>  94
taccaccgaa gttgatttgc ttcaacatcc tcagctctag atttgaatat gtattacttg      60

gttat                                                                   65


<210>  95
<211>  28
<212>  DNA
<213>  Artificial Sequence

<220>
<223>  Primer N138

<400>  95
atgttgaagc aaatcaactt cggtggta                                          28


<210>  96
<211>  22
<212>  DNA
<213>  Artificial Sequence

<220>
<223>  Primer N139

<400>  96
ttattggttt tctggtctca ac                                                22


<210>  97
<211>  57
<212>  DNA

<213>  Artificial Sequence

<220>
<223>  Primer N140

<400>  97
aagttgagac cagaaaacca ataattaatt aatcatgtaa ttagttatgt cacgctt          57


<210>  98
<211>  30
<212>  DNA
<213>  Artificial Sequence

<220>
<223>  Primer N141

<400>  98
gcggccgccc gcaaattaaa gccttcgagc                                        30


<210>  99
<211>  28
<212>  DNA
<213>  Artificial Sequence

<220>
<223>  Primer N142

<400>  99
ggatccgcat gcttgcattt agtcgtgc                                          28


<210>  100
<211>  56
<212>  DNA
<213>  Artificial Sequence

<220>
<223>  N143

<400>  100
caggtaatcc cccacagtat acatcctcag ctattgtaat atgtgtgttt gtttgg           56


<210>  101
<211>  22
<212>  DNA
<213>  Artificial Sequence

<220>
<223>  Primer N144

<400>  101
atgtatactg tgggggatta cc                                                22


<210>  102
<211>  22
<212>  DNA
<213>  Artificial Sequence

<220>
<223>  Primer N145

<400>  102
ttagctttta ttttgctccg ca                                                22


<210>  103
<211>  57
<212>  DNA
<213>  Artificial Sequence

<220>
<223>  Primer N146

<400>  103
tttgcggagc aaaataaaag ctaattaatt aagagtaagc gaatttctta tgattta          57


<210>  104
<211>  28
<212>  DNA
<213>  Artificial Sequence

<220>
<223>  Primer N147

<400>  104
actagtacca caggtgttgt cctctgag                                          28


<210>  105
<211>  22
<212>  DNA
<213>  Artificial Sequence

<220>
<223>  Primer N151

<400>  105
ctagagagct ttcgttttca tg                                                22


<210>  106
<211>  57
<212>  DNA
<213>  Artificial Sequence

<220>
<223>  Primer N152

<400>  106
ctcatgaaaa cgaaagctct ctagttaatt aatcatgtaa ttagttatgt cacgctt          57


<210>  107
<211>  25
<212>  DNA
<213>  Artificial Sequence

<220>
<223>  Primer N155

<400>  107
atggcaaaga agctcaacaa gtact                                             25


<210>  108

<211>  22
<212>  DNA
<213>  Artificial Sequence

<220>
<223>  Primer N156

<400>  108
tcaagcatct aaaacacaac cg                                              22


<210>  109
<211>  57
<212>  DNA
<213>  Artificial Sequence

<220>
<223>  Primer N157

<400>  109
aacggttgtg ttttagatgc ttgattaatt aagagtaagc gaatttctta tgattta        57


<210>  110
<211>  28
<212>  DNA
<213>  Artificial Sequence

<220>
<223>  Primer N158

<400>  110
ggatcctttt ctggcaacca aacccata                                        28


<210>  111
<211>  56
<212>  DNA
<213>  Artificial Sequence

<220>
<223>  Primer N159

<400>  111
cgagtacttg ttgagcttct ttgccatcct cagcgagata gttgattgta tgcttg         56


<210>  112
<211>  19
<212>  DNA
<213>  Artificial Sequence

<220>
<223>  Primer N160SeqF1

<400>  112
gaaaacgtgg catcctctc                                                  19


<210>  113
<211>  19
<212>  DNA
<213>  Artificial Sequence

<220>

<223>  Primer N160SeqF2

<400>  113
gctgactggc caagagaaa                                                    19


<210>  114
<211>  20
<212>  DNA
<213>  Artificial Sequence

<220>
<223>  Primer N160SeqF3

<400>  114
tgtacttctc ccacggtttc                                                   20


<210>  115
<211>  22
<212>  DNA
<213>  Artificial Sequence

<220>
<223>  Primer N160SeqF4

<400>  115
agctacccaa tctctatacc ca                                                22


<210>  116
<211>  22
<212>  DNA
<213>  Artificial Sequence

<220>
<223>  Primer N160SeqF5

<400>  116
cctgaagtct aggtccctat tt                                                22


<210>  117
<211>  19
<212>  DNA
<213>  Artificial Sequence

<220>
<223>  N160SeqR1

<400>  117
gcgtgaatgt aagcgtgac                                                    19


<210>  118
<211>  20
<212>  DNA
<213>  Artificial Sequence

<220>
<223>  Primer N160SeqR2

<400>  118
cgtcgtattg agccaagaac                                                   20

<210>  119
<211>  20
<212>  DNA
<213>  Artificial Sequence

<220>
<223>  Primer N160SeqR3

<400>  119
gcatcggaca acaagttcat                                                    20


<210>  120
<211>  22
<212>  DNA
<213>  Artificial Sequence

<220>
<223>  Primer N160SeqR4

<400>  120
tcgttcttga agtagtccaa ca                                                 22


<210>  121
<211>  19
<212>  DNA
<213>  Artificial Sequence

<220>
<223>  Primer N160SeqR5

<400>  121
tgagcccgaa agagaggat                                                     19


<210>  122
<211>  19
<212>  DNA
<213>  Artificial Sequence

<220>
<223>  Primer N161SeqF1

<400>  122
acggtatacg gccttcctt                                                     19


<210>  123
<211>  20
<212>  DNA
<213>  Artificial Sequence

<220>
<223>  Primer N161SeqF2

<400>  123
gggtttgaaa gctatgcagt                                                    20


<210>  124
<211>  22
<212>  DNA
<213>  Artificial Sequence

<220>
<223>  Primer N161SeqF3

<400>  124
ggtggtatgt atactgccaa ca                                                22


<210>  125
<211>  22
<212>  DNA
<213>  Artificial Sequence

<220>
<223>  Primer N161SeqF4

<400>  125
ggtggtaccc aatctgtgat ta                                                22


<210>  126
<211>  20
<212>  DNA
<213>  Artificial Sequence

<220>
<223>  Primer N161SeqF5

<400>  126
cggtttgggt aaagatgttg                                                   20


<210>  127
<211>  22
<212>  DNA
<213>  Artificial Sequence

<220>
<223>  Primer N161SeqF6

<400>  127
aaacgaaaat tcttattctt ga                                                22


<210>  128
<211>  22
<212>  DNA
<213>  Artificial Sequence

<220>
<223>  Primer N161SeqR1

<400>  128
tcgttttaaa acctaagagt ca                                                22


<210>  129
<211>  19
<212>  DNA
<213>  Artificial Sequence

<220>
<223>  Primer N161SeqR2

<400>  129

ccaaaccgta acccatcag                                                    19

<210>  130
<211>  19
<212>  DNA
<213>  Artificial Sequence

<220>
<223>  Primer N161SeqR3

<400>  130
cacagattgg gtaccacca                                                    19

<210>  131
<211>  20
<212>  DNA
<213>  Artificial Sequence

<220>
<223>  Primer N161Seqr4

<400>  131
accacaagaa ccaggacctg                                                   20

<210>  132
<211>  19
<212>  DNA
<213>  Artificial Sequence

<220>
<223>  Primer N161SeqR5

<400>  132
catagctttc aaacccgct                                                    19

<210>  133
<211>  22
<212>  DNA
<213>  Artificial Sequence

<220>
<223>  Primer N161SeqR6

<400>  133
cgtataccgt tgctcattag ag                                                22

<210>  134
<211>  23
<212>  DNA
<213>  Artificial Sequence

<220>
<223>  Primer N162

<400>  134
atgttgacaa aagcaacaaa aga                                               23

<210>  135
<211>  38

<212>  DNA
<213>  Artificial Sequence

<220>
<223>  Primer N189

<400>  135
atccgcggat agatctagtt cgagtttatc attatcaa                              38


<210>  136
<211>  53
<212>  DNA
<213>  Artificial Sequemce

<220>
<223>  Primer N190.1

<400>  136
ttcttttgtt gcttttgtca acatcctcag cgtttatgtg tgtttattcg aaa             53


<210>  137
<211>  38
<212>  DNA
<213>  Artificial Sequence

<220>
<223>  Primer N176

<400>  137
atccgcggat agatctatta gaagccgccg agcgggcg                              38


<210>  138
<211>  31
<212>  DNA
<213>  Artificial Sequence

<220>
<223>  Primer N177

<400>  138
atcctcagct tttctccttg acgttaaagt a                                     31


<210>  139
<211>  477
<212>  PRT
<213>  Escherichia coli

<400>  139

Met Thr Gln Ser Arg Leu His Ala Ala Gln Asn Ala Leu Ala Lys Leu
1               5                   10                  15


His Glu His Arg Gly Asn Thr Phe Tyr Pro His Phe His Leu Ala Pro
            20                  25                  30


Pro Ala Gly Trp Met Asn Asp Pro Asn Gly Leu Ile Trp Phe Asn Asp
        35                  40                  45

Arg Tyr His Ala Phe Tyr Gln His His Pro Met Ser Glu His Trp Gly
    50                  55                  60

Pro Met His Trp Gly His Ala Thr Ser Asp Asp Met Ile His Trp Gln
65                  70                  75                  80

His Glu Pro Ile Ala Leu Ala Pro Gly Asp Asp Asn Asp Lys Asp Gly
                85                  90                  95

Cys Phe Ser Gly Ser Ala Val Asp Asp Asn Gly Val Leu Ser Leu Ile
            100                 105                 110

Tyr Thr Gly His Val Trp Leu Asp Gly Ala Gly Asn Asp Asp Ala Ile
        115                 120                 125

Arg Glu Val Gln Cys Leu Ala Thr Ser Arg Asp Gly Ile His Phe Glu
    130                 135                 140

Lys Gln Gly Val Ile Leu Thr Pro Pro Glu Gly Ile Met His Phe Arg
145                 150                 155                 160

Asp Pro Lys Val Trp Arg Glu Ala Asp Thr Trp Trp Met Val Val Gly
                165                 170                 175

Ala Lys Asp Pro Gly Asn Thr Gly Gln Ile Leu Leu Tyr Arg Gly Ser
            180                 185                 190

Ser Leu Arg Glu Trp Thr Phe Asp Arg Val Leu Ala His Ala Asp Ala
        195                 200                 205

Gly Glu Ser Tyr Met Trp Glu Cys Pro Asp Phe Phe Ser Leu Gly Asp
    210                 215                 220

Gln His Tyr Leu Met Phe Ser Pro Gln Gly Met Asn Ala Glu Gly Tyr
225                 230                 235                 240

Ser Tyr Arg Asn Arg Phe Gln Ser Gly Val Ile Pro Gly Met Trp Ser
                245                 250                 255

Pro Gly Arg Leu Phe Ala Gln Ser Gly His Phe Thr Glu Leu Asp Asn
            260                 265                 270

Gly His Asp Phe Tyr Ala Pro Gln Ser Phe Leu Ala Lys Asp Gly Arg
        275                 280                 285

Arg Ile Val Ile Gly Trp Met Asp Met Trp Glu Ser Pro Met Pro Ser
    290                 295                 300

Lys Arg Glu Gly Trp Ala Gly Cys Met Thr Leu Ala Arg Glu Leu Ser

305                 310                 315                 320

Glu Ser Asn Gly Lys Leu Leu Gln Arg Pro Val His Glu Ala Glu Ser
                325                 330                 335

Leu Arg Gln Gln His Gln Ser Val Ser Pro Arg Thr Ile Ser Asn Lys
            340                 345                 350

Tyr Val Leu Gln Glu Asn Ala Gln Ala Val Glu Ile Gln Leu Gln Trp
        355                 360                 365

Ala Leu Lys Asn Ser Asp Ala Glu His Tyr Gly Leu Gln Leu Gly Thr
    370                 375                 380

Gly Met Arg Leu Tyr Ile Asp Asn Gln Ser Glu Arg Leu Val Leu Trp
385                 390                 395                 400

Arg Tyr Tyr Pro His Glu Asn Leu Asp Gly Tyr Arg Ser Ile Pro Leu
                405                 410                 415

Pro Gln Arg Asp Thr Leu Ala Leu Arg Ile Phe Ile Asp Thr Ser Ser
            420                 425                 430

Val Glu Val Phe Ile Asn Asp Gly Glu Ala Val Met Ser Ser Arg Ile
        435                 440                 445

Tyr Pro Gln Pro Glu Glu Arg Glu Leu Ser Leu Tyr Ala Ser His Gly
    450                 455                 460

Val Ala Val Leu Gln His Gly Ala Leu Trp Leu Leu Gly
465                 470                 475

<210>  140
<211>  304
<212>  PRT
<213>  Escherichia coli

<400>  140

Met Ser Ala Lys Val Trp Val Leu Gly Asp Ala Val Val Asp Leu Leu
1               5                   10                  15

Pro Glu Ser Asp Gly Arg Leu Leu Pro Cys Pro Gly Gly Ala Pro Ala
            20                  25                  30

Asn Val Ala Val Gly Ile Ala Arg Leu Gly Gly Thr Ser Gly Phe Ile
        35                  40                  45

Gly Arg Val Gly Asp Asp Pro Phe Gly Ala Leu Met Gln Arg Thr Leu
    50                  55                  60

Leu Thr Glu Gly Val Asp Ile Thr Tyr Leu Lys Gln Asp Glu Trp His
65                  70                  75                  80

Arg Thr Ser Thr Val Leu Val Asp Leu Asn Asp Gln Gly Glu Arg Ser
                85                  90                  95

Phe Thr Phe Met Val Arg Pro Ser Ala Asp Leu Phe Leu Glu Thr Thr
            100                 105                 110

Asp Leu Pro Cys Trp Arg His Gly Glu Trp Leu His Leu Cys Ser Ile
        115                 120                 125

Ala Leu Ser Ala Glu Pro Ser Arg Thr Ser Ala Phe Thr Ala Met Thr
    130                 135                 140

Ala Ile Arg His Ala Gly Gly Phe Val Ser Phe Asp Pro Asn Ile Arg
145                 150                 155                 160

Glu Asp Leu Trp Gln Asp Glu His Leu Leu Arg Leu Cys Leu Arg Gln
                165                 170                 175

Ala Leu Gln Leu Ala Asp Val Val Lys Leu Ser Glu Glu Glu Trp Arg
            180                 185                 190

Leu Ile Ser Gly Lys Thr Gln Asn Asp Gln Asp Ile Cys Ala Leu Ala
        195                 200                 205

Lys Glu Tyr Glu Ile Ala Met Leu Leu Val Thr Lys Gly Ala Glu Gly
    210                 215                 220

Val Val Val Cys Tyr Arg Gly Gln Val His His Phe Ala Gly Met Ser
225                 230                 235                 240

Val Asn Cys Val Asp Ser Thr Gly Ala Gly Asp Ala Phe Val Ala Gly
                245                 250                 255

Leu Leu Thr Gly Leu Ser Ser Thr Gly Leu Ser Thr Asp Glu Arg Glu
            260                 265                 270

Met Arg Arg Ile Ile Asp Leu Ala Gln Arg Cys Gly Ala Leu Ala Val
        275                 280                 285

Thr Ala Lys Gly Ala Met Thr Ala Leu Pro Cys Arg Gln Glu Leu Glu
    290                 295                 300

<210>  141
<211>  415
<212>  PRT
<213>  Escherichia coli

<400> 141

Met Ala Leu Asn Ile Pro Phe Arg Asn Ala Tyr Tyr Arg Phe Ala Ser
1               5                   10                  15

Ser Tyr Ser Phe Leu Phe Phe Ile Ser Trp Ser Leu Trp Trp Ser Leu
            20                  25                  30

Tyr Ala Ile Trp Leu Lys Gly His Leu Gly Leu Thr Gly Thr Glu Leu
        35                  40                  45

Gly Thr Leu Tyr Ser Val Asn Gln Phe Thr Ser Ile Leu Phe Met Met
    50                  55                  60

Phe Tyr Gly Ile Val Gln Asp Lys Leu Gly Leu Lys Lys Pro Leu Ile
65                  70                  75                  80

Trp Cys Met Ser Phe Ile Leu Val Leu Thr Gly Pro Phe Met Ile Tyr
                85                  90                  95

Val Tyr Glu Pro Leu Leu Gln Ser Asn Phe Ser Val Gly Leu Ile Leu
            100                 105                 110

Gly Ala Leu Phe Phe Gly Leu Gly Tyr Leu Ala Gly Cys Gly Leu Leu
        115                 120                 125

Asp Ser Phe Thr Glu Lys Met Ala Arg Asn Phe His Phe Glu Tyr Gly
    130                 135                 140

Thr Ala Arg Ala Trp Gly Ser Phe Gly Tyr Ala Ile Gly Ala Phe Phe
145                 150                 155                 160

Ala Gly Ile Phe Phe Ser Ile Ser Pro His Ile Asn Phe Trp Leu Val
                165                 170                 175

Ser Leu Phe Gly Ala Val Phe Met Met Ile Asn Met Arg Phe Lys Asp
            180                 185                 190

Lys Asp His Gln Cys Val Ala Ala Asp Ala Gly Gly Val Lys Lys Glu
        195                 200                 205

Asp Phe Ile Ala Val Phe Lys Asp Arg Asn Phe Trp Val Phe Val Ile
    210                 215                 220

Phe Ile Val Gly Thr Trp Ser Phe Tyr Asn Ile Phe Asp Gln Gln Leu
225                 230                 235                 240

Phe Pro Val Phe Tyr Ser Gly Leu Phe Glu Ser His Asp Val Gly Thr
                245                 250                 255

Arg Leu Tyr Gly Tyr Leu Asn Ser Phe Gln Val Val Leu Glu Ala Leu
            260                 265                 270

Cys Met Ala Ile Ile Pro Phe Phe Val Asn Arg Val Gly Pro Lys Asn
        275                 280                 285

Ala Leu Leu Ile Gly Val Val Ile Met Ala Leu Arg Ile Leu Ser Cys
    290                 295                 300

Ala Leu Phe Val Asn Pro Trp Ile Ile Ser Leu Val Lys Leu Leu His
305                 310                 315                 320

Ala Ile Glu Val Pro Leu Cys Val Ile Ser Val Phe Lys Tyr Ser Val
                325                 330                 335

Ala Asn Phe Asp Lys Arg Leu Ser Ser Thr Ile Phe Leu Ile Gly Phe
            340                 345                 350

Gln Ile Ala Ser Ser Leu Gly Ile Val Leu Leu Ser Thr Pro Thr Gly
        355                 360                 365

Ile Leu Phe Asp His Ala Gly Tyr Gln Thr Val Phe Phe Ala Ile Ser
    370                 375                 380

Gly Ile Val Cys Leu Met Leu Leu Phe Gly Ile Phe Phe Leu Ser Lys
385                 390                 395                 400

Lys Arg Glu Gln Ile Val Met Glu Thr Pro Val Pro Ser Ala Ile
                405                 410                 415

<210>  142
<211>  6341
<212>  DNA
<213>  Artificial Sequence

<220>
<223>  Plasmid pFP988DssPspac

<400>  142
gatccaagtt taaactgtac actagatatt tcttctccgc ttaaatcatc aaagaaatct      60

ttatcacttg taaccagtcc gtccacatgt cgaattgcat ctgaccgaat tttacgtttc     120

cctgaataat tctcatcaat cgtttcatca attttatctt tatactttat attttgtgcg     180

ttaatcaaat cataattttt atatgtttcc tcatgattta tgtctttatt attatagttt     240

ttattctctc tttgattatg tctttgtatc ccgtttgtat tacttgatcc tttaactctg     300

gcaaccctca aaattgaatg agacatgcta cacctccgga taataaatat atataaacgt     360

atatagattt cataaagtct aacacactag acttatttac ttcgtaatta agtcgttaaa     420

ccgtgtgctc tacgaccaaa actataaaac ctttaagaac tttctttttt tacaagaaaa 480

aagaaattag ataaatctct catatctttt attcaataat cgcatccgat tgcagtataa 540

atttaacgat cactcatcat gttcatattt atcagagctc gtgctataat tatactaatt 600

ttataaggag gaaaaaatat gggcattttt agtatttttg taatcagcac agttcattat 660

caaccaaaca aaaaataagt ggttataatg aatcgttaat aagcaaaatt catataacca 720

aattaaagag ggttataatg aacgagaaaa atataaaaca cagtcaaaac tttattactt 780

caaaacataa tatagataaa ataatgacaa atataagatt aaatgaacat gataatatct 840

ttgaaatcgg ctcaggaaaa ggccatttta cccttgaatt agtaaagagg tgtaatttcg 900

taactgccat tgaaatagac cataaattat gcaaaactac agaaaataaa cttgttgatc 960

acgataattt ccaagtttta aacaaggata tattgcagtt taaatttcct aaaaaccaat 1020

cctataaaat atatggtaat ataccttata acataagtac ggatataata cgcaaaattg 1080

tttttgatag tatagctaat gagatttatt taatcgtgga atacgggttt gctaaaagat 1140

tattaaatac aaaacgctca ttggcattac ttttaatggc agaagttgat atttctatat 1200

taagtatggt tccaagagaa tattttcatc ctaaacctaa agtgaatagc tcacttatca 1260

gattaagtag aaaaaaatca agaatatcac acaaagataa acaaaagtat aattatttcg 1320

ttatgaaatg ggttaacaaa gaatacaaga aaatatttac aaaaaatcaa tttaacaatt 1380

ccttaaaaca tgcaggaatt gacgatttaa acaatattag ctttgaacaa ttcttatctc 1440

ttttcaatag ctataaatta tttaataagt aagttaaggg atgcagttca tcgatgaagg 1500

caactacagc tcaggcgaca accatacgct gagagatcct cactacgtag aagataaagg 1560

ccacaaatac ttagtatttg aagcaaacac tggaactgaa gatggctacc aaggcgaaga 1620

atctttattt aacaaagcat actatggcaa aagcacatca ttcttccgtc aagaaagtca 1680

aaaacttctg caaagcgata aaaaacgcac ggctgagtta gcaaacggcg ctctcggtat 1740

gattgagcta aacgatgatt acacactgaa aaaagtgatg aaaccgctga ttgcatctaa 1800

cacagtaaca gatgaaattg aacgcgcgaa cgtctttaaa atgaacggca aatggtacct 1860

gttcactgac tcccgcggat caaaaatgac gattgacggc attacgtcta acgatattta 1920

catgcttggt tatgtttcta attctttaac tggcccatac aagccgctga acaaaactgg 1980

ccttgtgtta aaaatggatc ttgatcctaa cgatgtaacc tttacttact cacacttcgc 2040

tgtacctcaa gcgaaaggaa acaatgtcgt gattacaagc tatatgacaa acagaggatt 2100

ctacgcagac aaacaatcaa cgtttgcgcc aagcttgcat gcgagagtag ggaactgcca 2160

ggcatcaaat aaaacgaaag gctcagtcga aagactgggc ctttcgtttt atctgttgtt 2220

tgtcggtgaa cgctctcctg agtaggacaa atccgccggg agcggatttg aacgttgcga 2280

agcaacggcc cggagggtgg cgggcaggac gcccgccata aactgccagg catcaaatta 2340

agcagaaggc catcctgacg gatggccttt ttgcgtttct acaaactctt tttgtttatt 2400

tttctaaata cattcaaata tgtatccgct catgctccgg atctgcatcg caggatgctg 2460
ctggctaccc tgtggaacac ctacatctgt attaacgaag cgctggcatt gaccctgagt 2520
gatttttctc tggtcccgcc gcatccatac cgccagttgt ttaccctcac aacgttccag 2580
taaccgggca tgttcatcat cagtaacccg tatcgtgagc atcctctctc gtttcatcgg 2640
tatcattacc cccatgaaca gaaattcccc cttacacgga ggcatcaagt gaccaaacag 2700
gaaaaaaccg cccttaacat ggcccgcttt atcagaagcc agacattaac gcttctggag 2760
aaactcaacg agctggacgc ggatgaacag gcagacatct gtgaatcgct tcacgaccac 2820
gctgatgagc tttaccgcag ctgcctcgcg cgtttcggtg atgacggtga aaacctctga 2880
cacatgcagc tcccggagac ggtcacagct tgtctgtaag cggatgccgg gagcagacaa 2940
gcccgtcagg gcgcgtcagc gggtgttggc gggtgtcggg gcgcagccat gacccagtca 3000
cgtagcgata gcggagtgta tactggctta actatgcggc atcagagcag attgtactga 3060
gagtgcacca tatgcggtgt gaaataccgc acagatgcgt aaggagaaaa taccgcatca 3120
ggcgctcttc cgcttcctcg ctcactgact cgctgcgctc ggtcgttcgg ctgcggcgag 3180
cggtatcagc tcactcaaag gcggtaatac ggttatccac agaatcaggg gataacgcag 3240
gaaagaacat gtgagcaaaa ggccagcaaa aggccaggaa ccgtaaaaag gccgcgttgc 3300
tggcgttttt ccataggctc cgcccccctg acgagcatca caaaaatcga cgctcaagtc 3360
agaggtggcg aaacccgaca ggactataaa gataccaggc gtttccccct ggaagctccc 3420
tcgtgcgctc tcctgttccg accctgccgc ttaccggata cctgtccgcc tttctccctt 3480
cgggaagcgt ggcgctttct caatgctcac gctgtaggta tctcagttcg gtgtaggtcg 3540
ttcgctccaa gctgggctgt gtgcacgaac cccccgttca gcccgaccgc tgcgccttat 3600
ccggtaacta tcgtcttgag tccaacccgg taagacacga cttatcgcca ctggcagcag 3660
ccactggtaa caggattagc agagcgaggt atgtaggcgg tgctacagag ttcttgaagt 3720
ggtggcctaa ctacggctac actagaagga cagtatttgg tatctgcgct ctgctgaagc 3780
cagttacctt cggaaaaaga gttggtagct cttgatccgg caaacaaacc accgctggta 3840
gcggtggttt ttttgtttgc aagcagcaga ttacgcgcag aaaaaaagga tctcaagaag 3900
atcctttgat cttttctacg gggtctgacg ctcagtggaa cgaaaactca cgttaaggga 3960
ttttggtcat gagattatca aaaaggatct tcacctagat ccttttaaat taaaaatgaa 4020
gttttaaatc aatctaaagt atatatgagt aaacttggtc tgacagttac caatgcttaa 4080
tcagtgaggc acctatctca gcgatctgtc tatttcgttc atccatagtt gcctgactcc 4140
ccgtcgtgta gataactacg atacgggagg gcttaccatc tggccccagt gctgcaatga 4200
taccgcgaga cccacgctca ccggctccag atttatcagc aataaaccag ccagccggaa 4260
gggccgagcg cagaagtggt cctgcaactt tatccgcctc catccagtct attaattgtt 4320

gccgggaagc tagagtaagt agttcgccag ttaatagttt gcgcaacgtt gttgccattg 4380

ctgcaggcat cgtggtgtca cgctcgtcgt ttggtatggc ttcattcagc tccggttccc 4440

aacgatcaag gcgagttaca tgatccccca tgttgtgcaa aaaagcggtt agctccttcg 4500

gtcctccgat cgttgtcaga agtaagttgg ccgcagtgtt atcactcatg gttatggcag 4560

cactgcataa ttctcttact gtcatgccat ccgtaagatg cttttctgtg actggtgagt 4620

actcaaccaa gtcattctga gaatagtgta tgcggcgacc gagttgctct tgcccggcgt 4680

caatacggga taataccgcg ccacatagca gaactttaaa agtgctcatc attggaaaac 4740

gttcttcggg gcgaaaactc tcaaggatct taccgctgtt gagatccagt tcgatgtaac 4800

ccactcgtgc acccaactga tcttcagcat cttttacttt caccagcgtt tctgggtgag 4860

caaaaacagg aaggcaaaat gccgcaaaaa agggaataag ggcgacacgg aaatgttgaa 4920

tactcatact cttccttttt caatattatt gaagcattta tcagggttat tgtctcatga 4980

gcggatacat atttgaatgt atttagaaaa ataaacaaat aggggttccg cgcacatttc 5040

cccgaaaagt gccacctgac gtctaagaaa ccattattat catgacatta acctataaaa 5100

ataggcgtat cacgaggccc tttcgtctcg cgcgtttcgg tgatgacggt gaaaacctct 5160

gacacatgca gctcccggag acggtcacag cttgtctgta agcggatgcc gggagcagac 5220

aagcccgtca gggcgcgtca gcgggtgttc atgtgcgtaa ctaacttgcc atcttcaaac 5280

aggagggctg gaagaagcag accgctaaca cagtacataa aaaaggagac atgaacgatg 5340

aacatcaaaa agtttgcaaa acaagcaaca gtattaacct ttactaccgc actgctggca 5400

ggaggcgcaa ctcaagcgtt tgcgaaagaa acgaaccaaa agccatataa ggaaacatac 5460

ggcatttccc atattacacg ccatgatatg ctgcaaatcc ctgaacagca aaaaaatgaa 5520

aaatatcaag ttcctgaatt cgattcgtcc acaattaaaa atatctcttc tgcaaaaggc 5580

ctggacgttt gggacagctg gccattacaa aacgctgacg gcactgtcgc aaactatcac 5640

ggctaccaca tcgtctttgc attagccgga gatcctaaaa atgcggatga cacatcgatt 5700

tacatgttct atcaaaaagt cggcgaaact tctattgaca gctggaaaaa cgctggccgc 5760

gtctttaaag acagcgacaa attcgatgca aatgattcta tcctaaaaga ccaaacacaa 5820

gaatggtcag gttcagccac atttacatct gacggaaaaa tccgtttatt ctacactgat 5880

ttctccggta aacattacgg caaacaaaca ctgacaactg cacaagttaa cgtatcagca 5940

tcagacagct ctttgaacat caacggtgta gaggattata aatcaatctt tgacggtgac 6000

ggaaaaacgt atcaaaatgt acagaattcg agctctcgag taattctaca cagcccagtc 6060

cagactattc ggcactgaaa ttatgggtga agtggtcaag acctcactag gcaccttaaa 6120

aatagcgcac cctgaagaag atttatttga ggtagccctt gcctacctag cttccaagaa 6180

agatatccta acagcacaag agcggaaaga tgttttgttc tacatccaga acaacctctg 6240

ctaaaattcc tgaaaaattt tgcaaaaagt tgttgacttt atctacaagg tgtggcataa 6300

tgtgtggaat tgtgagcgct cacaattaag cttgaattcc c 6341

<210> 143
<211> 6221
<212> DNA
<213> Artificial Sequence

<220>
<223> Plasmid pFP988DssPgroE

<400> 143
   tcgagagcta ttgtaacata atcggtacgg gggtgaaaaa gctaacggaa aagggagcgg 60
   aaaagaatga tgtaagcgtg aaaaattttt tatcttatca cttgaaattg gaagggagat 120
   tctttattat aagaattgtg gaattgtgag cggataacaa ttcccaatta aaggaggaaa 180
   ctagtggatc caagtttaaa ctgtacacta gatatttctt ctccgcttaa atcatcaaag 240
   aaatctttat cacttgtaac cagtccgtcc acatgtcgaa ttgcatctga ccgaatttta 300
   cgtttccctg aataattctc atcaatcgtt tcatcaattt tatctttata ctttatattt 360
   tgtgcgttaa tcaaatcata atttttatat gtttcctcat gatttatgtc tttattatta 420
   tagtttttat tctctctttg attatgtctt tgtatcccgt ttgtattact tgatccttta 480
   actctggcaa ccctcaaaat tgaatgagac atgctacacc tccggataat aaatatatat 540
   aaacgtatat agatttcata aagtctaaca cactagactt atttacttcg taattaagtc 600
   gttaaaccgt gtgctctacg accaaaacta taaaaccttt aagaactttc tttttttaca 660
   agaaaaaaga aattagataa atctctcata tcttttattc aataatcgca tccgattgca 720
   gtataaattt aacgatcact catcatgttc atatttatca gagctcgtgc tataattata 780
   ctaattttat aaggaggaaa aaatatgggc atttttagta tttttgtaat cagcacagtt 840
   cattatcaac caaacaaaaa ataagtggtt ataatgaatc gttaataagc aaaattcata 900
   taaccaaatt aaagagggtt ataatgaacg agaaaaatat aaaacacagt caaaacttta 960
   ttacttcaaa acataatata gataaaataa tgacaaatat aagattaaat gaacatgata 1020
   atatctttga aatcggctca ggaaaaggcc attttaccct tgaattagta aagaggtgta 1080
   atttcgtaac tgccattgaa atagaccata aattatgcaa aactacagaa aataaacttg 1140
   ttgatcacga taatttccaa gttttaaaca aggatatatt gcagtttaaa tttcctaaaa 1200
   accaatccta taaaatatat ggtaatatac cttataacat aagtacggat ataatacgca 1260
   aaattgtttt tgatagtata gctaatgaga tttatttaat cgtggaatac gggtttgcta 1320
   aaagattatt aaatacaaaa cgctcattgg cattactttt aatggcagaa gttgatattt 1380
   ctatattaag tatggttcca agagaatatt ttcatcctaa acctaaagtg aatagctcac 1440
   ttatcagatt aagtagaaaa aaatcaagaa tatcacacaa agataaacaa aagtataatt 1500
   atttcgttat gaaatgggtt aacaaagaat acaagaaaat atttacaaaa aatcaattta 1560
   acaattcctt aaaacatgca ggaattgacg atttaaacaa tattagcttt gaacaattct 1620
   tatctctttt caatagctat aaattattta ataagtaagt taagggatgc agttcatcga 1680
   tgaaggcaac tacagctcag gcgacaacca tacgctgaga gatcctcact acgtagaaga 1740
   taaaggccac aaatacttag tatttgaagc aaacactgga actgaagatg gctaccaagg 1800
   cgaagaatct ttatttaaca aagcatacta tggcaaaagc acatcattct tccgtcaaga 1860
   aagtcaaaaa cttctgcaaa gcgataaaaa acgcacggct gagttagcaa acggcgctct 1920
   cggtatgatt gagctaaacg atgattacac actgaaaaaa gtgatgaaac cgctgattgc 1980
   atctaacaca gtaacagatg aaattgaacg cgcgaacgtc tttaaaatga acggcaaatg 2040
   gtacctgttc actgactccc gcggatcaaa aatgacgatt gacggcatta cgtctaacga 2100
   tatttacatg cttggttatg tttctaattc tttaactggc ccatacaagc cgctgaacaa 2160
   aactggcctt gtgttaaaaa tggatcttga tcctaacgat gtaaccttta cttactcaca 2220
   cttcgctgta cctcaagcga aaggaaacaa tgtcgtgatt acaagctata tgacaaacag 2280
   aggattctac gcagacaaac aatcaacgtt tgcgccaagc ttgcatgcga gagtagggaa 2340
   ctgccaggca tcaaataaaa cgaaaggctc agtcgaaaga ctgggccttt cgttttatct 2400
   gttgtttgtc ggtgaacgct ctcctgagta ggacaaatcc gccgggagcg gatttgaacg 2460
   ttgcgaagca acggcccgga gggtggcggg caggacgccc gccataaact gccaggcatc 2520
   aaattaagca gaaggccatc ctgacggatg gcctttttgc gtttctacaa actctttttg 2580
   tttatttttc taaatacatt caaatatgta tccgctcatg ctccggatct gcatcgcagg 2640
   atgctgctgg ctaccctgtg gaacacctac atctgtatta acgaagcgct ggcattgacc 2700
   ctgagtgatt tttctctggt cccgccgcat ccataccgcc agttgtttac cctcacaacg 2760
   ttccagtaac cgggcatgtt catcatcagt aacccgtatc gtgagcatcc tctctcgttt 2820
   catcggtatc attaccccca tgaacagaaa ttccccctta cacggaggca tcaagtgacc 2880
   aaacaggaaa aaaccgccct taacatggcc cgctttatca gaagccagac attaacgctt 2940
   ctggagaaac tcaacgagct ggacgcggat gaacaggcag acatctgtga atcgcttcac 3000
   gaccacgctg atgagcttta ccgcagctgc ctcgcgcgtt tcggtgatga cggtgaaaac 3060
   ctctgacaca tgcagctccc ggagacggtc acagcttgtc tgtaagcgga tgccgggagc 3120
   agacaagccc gtcagggcgc gtcagcgggt gttggcgggt gtcggggcgc agccatgacc 3180
   cagtcacgta gcgatagcgg agtgtatact ggcttaacta tgcggcatca gagcagattg 3240
   tactgagagt gcaccatatg cggtgtgaaa taccgcacag atgcgtaagg agaaaatacc 3300
   gcatcaggcg ctcttccgct tcctcgctca ctgactcgct gcgctcggtc gttcggctgc 3360
   ggcgagcggt atcagctcac tcaaaggcgg taatacggtt atccacagaa tcaggggata 3420
   acgcaggaaa gaacatgtga gcaaaaggcc agcaaaaggc caggaaccgt aaaaaggccg 3480
   cgttgctggc gtttttccat aggctccgcc cccctgacga gcatcacaaa aatcgacgct 3540
   caagtcagag gtggcgaaac ccgacaggac tataaagata ccaggcgttt ccccctggaa 3600
   gctccctcgt gcgctctcct gttccgaccc tgccgcttac cggatacctg tccgcctttc 3660
   tcccttcggg aagcgtggcg ctttctcaat gctcacgctg taggtatctc agttcggtgt 3720
   aggtcgttcg ctccaagctg ggctgtgtgc acgaaccccc cgttcagccc gaccgctgcg 3780
   ccttatccgg taactatcgt cttgagtcca acccggtaag acacgactta tcgccactgg 3840
   cagcagccac tggtaacagg attagcagag cgaggtatgt aggcggtgct acagagttct 3900
   tgaagtggtg gcctaactac ggctacacta gaaggacagt atttggtatc tgcgctctgc 3960
   tgaagccagt taccttcgga aaaagagttg gtagctcttg atccggcaaa caaaccaccg 4020
   ctggtagcgg tggttttttt gtttgcaagc agcagattac gcgcagaaaa aaaggatctc 4080
   aagaagatcc tttgatcttt tctacggggt ctgacgctca gtggaacgaa aactcacgtt 4140
   aagggatttt ggtcatgaga ttatcaaaaa ggatcttcac ctagatcctt ttaaattaaa 4200
   aatgaagttt taaatcaatc taaagtatat atgagtaaac ttggtctgac agttaccaat 4260
   gcttaatcag tgaggcacct atctcagcga tctgtctatt tcgttcatcc atagttgcct 4320
   gactccccgt cgtgtagata actacgatac gggagggctt accatctggc cccagtgctg 4380
   caatgatacc gcgagaccca cgctcaccgg ctccagattt atcagcaata aaccagccag 4440
   ccggaagggc cgagcgcaga agtggtcctg caactttatc cgcctccatc cagtctatta 4500
   attgttgccg ggaagctaga gtaagtagtt cgccagttaa tagtttgcgc aacgttgttg 4560
   ccattgctgc aggcatcgtg gtgtcacgct cgtcgtttgg tatggcttca ttcagctccg 4620
   gttcccaacg atcaaggcga gttacatgat cccccatgtt gtgcaaaaaa gcggttagct 4680
   ccttcggtcc tccgatcgtt gtcagaagta agttggccgc agtgttatca ctcatggtta 4740
   tggcagcact gcataattct cttactgtca tgccatccgt aagatgcttt tctgtgactg 4800
   gtgagtactc aaccaagtca ttctgagaat agtgtatgcg gcgaccgagt tgctcttgcc 4860
   cggcgtcaat acgggataat accgcgccac atagcagaac tttaaaagtg ctcatcattg 4920
   gaaaacgttc ttcggggcga aaactctcaa ggatcttacc gctgttgaga tccagttcga 4980
   tgtaacccac tcgtgcaccc aactgatctt cagcatcttt tactttcacc agcgtttctg 5040
   ggtgagcaaa aacaggaagg caaaatgccg caaaaaaggg aataagggcg acacggaaat 5100
   gttgaatact catactcttc ctttttcaat attattgaag catttatcag ggttattgtc 5160
   tcatgagcgg atacatattt gaatgtattt agaaaaataa acaaataggg gttccgcgca 5220
   catttccccg aaaagtgcca cctgacgtct aagaaaccat tattatcatg acattaacct 5280
   ataaaaatag gcgtatcacg aggccctttc gtctcgcgcg tttcggtgat gacggtgaaa 5340
   acctctgaca catgcagctc ccggagacgg tcacagcttg tctgtaagcg gatgccggga 5400
   gcagacaagc ccgtcagggc gcgtcagcgg gtgttcatgt gcgtaactaa cttgccatct 5460
   tcaaacagga gggctggaag aagcagaccg ctaacacagt acataaaaaa ggagacatga 5520
   acgatgaaca tcaaaaagtt tgcaaaacaa gcaacagtat taacctttac taccgcactg 5580
   ctggcaggag gcgcaactca agcgtttgcg aaagaaacga accaaaagcc atataaggaa 5640
   acatacggca tttcccatat tacacgccat gatatgctgc aaatccctga acagcaaaaa 5700
   aatgaaaaat atcaagttcc tgaattcgat tcgtccacaa ttaaaaatat ctcttctgca 5760
   aaaggcctgg acgtttggga cagctggcca ttacaaaacg ctgacggcac tgtcgcaaac 5820
   tatcacggct accacatcgt ctttgcatta gccggagatc ctaaaaatgc ggatgacaca 5880
   tcgatttaca tgttctatca aaaagtcggc gaaacttcta ttgacagctg gaaaaacgct 5940
   ggccgcgtct ttaaagacag cgacaaattc gatgcaaatg attctatcct aaaagaccaa 6000
   acacaagaat ggtcaggttc agccacattt acatctgacg gaaaaatccg tttattctac 6060
   actgatttct ccggtaaaca ttacggcaaa caaacactga caactgcaca agttaacgta 6120
   tcagcatcag acagctcttt gaacatcaac ggtgtagagg attataaatc aatctttgac 6180
   ggtgacggaa aaacgtatca aaatgtacag aattcgagct c 6221

<210> 144
<211> 40
<212> DNA
<213> Artificial Sequence

<220>
<223> Primer T-budB (BamHI)

<400> 144
agatagatgg atccggaggt gggtcacatg gacaaacagt 40

<210> 145
<211> 29
<212> DNA
<213> Artificial Sequence

<220>
<223> Primer B-kivD (BamHI)

<400> 145
ctctagagga tccagactcc taggacatg 29

<210> 146
<211> 6039
<212> DNA
<213> Artificial Sequence

<220>
<223> Vector fragment pFP988Dss

<400> 146
gatccaagtt taaactgtac actagatatt tcttctccgc ttaaatcatc aaagaaatct 60

ttatcacttg taaccagtcc gtccacatgt cgaattgcat ctgaccgaat tttacgtttc 120

cctgaataat tctcatcaat cgtttcatca attttatctt tatactttat attttgtgcg 180

ttaatcaaat cataattttt atatgtttcc tcatgattta tgtctttatt attatagttt 240
ttattctctc tttgattatg tctttgtatc ccgtttgtat tacttgatcc tttaactctg 300
gcaaccctca aaattgaatg agacatgcta cacctccgga taataaatat atataaacgt 360
atatagattt cataaagtct aacacactag acttatttac ttcgtaatta agtcgttaaa 420
ccgtgtgctc tacgaccaaa actataaaac ctttaagaac tttctttttt tacaagaaaa 480
aagaaattag ataaatctct catatctttt attcaataat cgcatccgat tgcagtataa 540
atttaacgat cactcatcat gttcatattt atcagagctc gtgctataat tatactaatt 600
ttataaggag gaaaaaatat gggcattttt agtatttttg taatcagcac agttcattat 660
caaccaaaca aaaaataagt ggttataatg aatcgttaat aagcaaaatt catataacca 720
aattaaagag ggttataatg aacgagaaaa atataaaaca cagtcaaaac tttattactt 780
caaaacataa tatagataaa ataatgacaa atataagatt aaatgaacat gataatatct 840
ttgaaatcgg ctcaggaaaa ggccatttta cccttgaatt agtaaagagg tgtaatttcg 900
taactgccat tgaaatagac cataaattat gcaaaactac agaaaataaa cttgttgatc 960
acgataattt ccaagtttta aacaaggata tattgcagtt taaatttcct aaaaaccaat 1020
cctataaaat atatggtaat ataccttata acataagtac ggatataata cgcaaaattg 1080
tttttgatag tatagctaat gagatttatt taatcgtgga atacgggttt gctaaaagat 1140
tattaaatac aaaacgctca ttggcattac ttttaatggc agaagttgat atttctatat 1200
taagtatggt tccaagagaa tattttcatc ctaaacctaa agtgaatagc tcacttatca 1260
gattaagtag aaaaaaatca agaatatcac acaaagataa acaaaagtat aattatttcg 1320
ttatgaaatg ggttaacaaa gaatacaaga aaatatttac aaaaaatcaa tttaacaatt 1380
ccttaaaaca tgcaggaatt gacgatttaa acaatattag ctttgaacaa ttcttatctc 1440
ttttcaatag ctataaatta tttaataagt aagttaaggg atgcagttca tcgatgaagg 1500
caactacagc tcaggcgaca accatacgct gagagatcct cactacgtag aagataaagg 1560
ccacaaatac ttagtatttg aagcaaacac tggaactgaa gatggctacc aaggcgaaga 1620
atctttattt aacaaagcat actatggcaa aagcacatca ttcttccgtc aagaaagtca 1680
aaaacttctg caaagcgata aaaaacgcac ggctgagtta gcaaacggcg ctctcggtat 1740
gattgagcta aacgatgatt acacactgaa aaaagtgatg aaaccgctga ttgcatctaa 1800
cacagtaaca gatgaaattg aacgcgcgaa cgtctttaaa atgaacggca aatggtacct 1860
gttcactgac tcccgcggat caaaaatgac gattgacggc attacgtcta acgatattta 1920
catgcttggt tatgtttcta attctttaac tggcccatac aagccgctga acaaaactgg 1980
ccttgtgtta aaaatggatc ttgatcctaa cgatgtaacc tttacttact cacacttcgc 2040
tgtacctcaa gcgaaaggaa acaatgtcgt gattacaagc tatatgacaa acagaggatt 2100

ctacgcagac aaacaatcaa cgtttgcgcc aagcttgcat gcgagagtag ggaactgcca 2160

ggcatcaaat aaaacgaaag gctcagtcga aagactgggc ctttcgtttt atctgttgtt 2220

tgtcggtgaa cgctctcctg agtaggacaa atccgccggg agcggatttg aacgttgcga 2280

agcaacggcc cggagggtgg cgggcaggac gcccgccata aactgccagg catcaaatta 2340

agcagaaggc catcctgacg gatggccttt ttgcgtttct acaaactctt tttgtttatt 2400

tttctaaata cattcaaata tgtatccgct catgctccgg atctgcatcg caggatgctg 2460

ctggctaccc tgtggaacac ctacatctgt attaacgaag cgctggcatt gaccctgagt 2520

gatttttctc tggtcccgcc gcatccatac cgccagttgt ttaccctcac aacgttccag 2580

taaccgggca tgttcatcat cagtaacccg tatcgtgagc atcctctctc gtttcatcgg 2640

tatcattacc cccatgaaca gaaattcccc cttacacgga ggcatcaagt gaccaaacag 2700

gaaaaaaccg cccttaacat ggcccgcttt atcagaagcc agacattaac gcttctggag 2760

aaactcaacg agctggacgc ggatgaacag gcagacatct gtgaatcgct tcacgaccac 2820

gctgatgagc tttaccgcag ctgcctcgcg cgtttcggtg atgacggtga aaacctctga 2880

cacatgcagc tcccggagac ggtcacagct tgtctgtaag cggatgccgg gagcagacaa 2940

gcccgtcagg gcgcgtcagc gggtgttggc gggtgtcggg gcgcagccat gacccagtca 3000

cgtagcgata gcggagtgta tactggctta actatgcggc atcagagcag attgtactga 3060

gagtgcacca tatgcggtgt gaaataccgc acagatgcgt aaggagaaaa taccgcatca 3120

ggcgctcttc cgcttcctcg ctcactgact cgctgcgctc ggtcgttcgg ctgcggcgag 3180

cggtatcagc tcactcaaag gcggtaatac ggttatccac agaatcaggg gataacgcag 3240

gaaagaacat gtgagcaaaa ggccagcaaa aggccaggaa ccgtaaaaag gccgcgttgc 3300

tggcgttttt ccataggctc cgcccccctg acgagcatca caaaaatcga cgctcaagtc 3360

agaggtggcg aaacccgaca ggactataaa gataccaggc gtttccccct ggaagctccc 3420

tcgtgcgctc tcctgttccg accctgccgc ttaccggata cctgtccgcc tttctccctt 3480

cgggaagcgt ggcgctttct caatgctcac gctgtaggta tctcagttcg gtgtaggtcg 3540

ttcgctccaa gctgggctgt gtgcacgaac cccccgttca gcccgaccgc tgcgccttat 3600

ccggtaacta tcgtcttgag tccaacccgg taagacacga cttatcgcca ctggcagcag 3660

ccactggtaa caggattagc agagcgaggt atgtaggcgg tgctacagag ttcttgaagt 3720

ggtggcctaa ctacggctac actagaagga cagtatttgg tatctgcgct ctgctgaagc 3780

cagttacctt cggaaaaaga gttggtagct cttgatccgg caaacaaacc accgctggta 3840

gcggtggttt ttttgtttgc aagcagcaga ttacgcgcag aaaaaaagga tctcaagaag 3900

atcctttgat cttttctacg gggtctgacg ctcagtggaa cgaaaactca cgttaaggga 3960

ttttggtcat gagattatca aaaaggatct tcacctagat ccttttaaat taaaaatgaa 4020

gttttaaatc aatctaaagt atatatgagt aaacttggtc tgacagttac caatgcttaa 4080

tcagtgaggc acctatctca gcgatctgtc tatttcgttc atccatagtt gcctgactcc 4140
ccgtcgtgta gataactacg atacgggagg gcttaccatc tggccccagt gctgcaatga 4200
taccgcgaga cccacgctca ccggctccag atttatcagc aataaaccag ccagccggaa 4260
gggccgagcg cagaagtggt cctgcaactt tatccgcctc catccagtct attaattgtt 4320
gccgggaagc tagagtaagt agttcgccag ttaatagttt gcgcaacgtt gttgccattg 4380
ctgcaggcat cgtggtgtca cgctcgtcgt ttggtatggc ttcattcagc tccggttccc 4440
aacgatcaag gcgagttaca tgatccccca tgttgtgcaa aaaagcggtt agctccttcg 4500
gtcctccgat cgttgtcaga agtaagttgg ccgcagtgtt atcactcatg gttatggcag 4560
cactgcataa ttctcttact gtcatgccat ccgtaagatg cttttctgtg actggtgagt 4620
actcaaccaa gtcattctga gaatagtgta tgcggcgacc gagttgctct tgcccggcgt 4680
caatacggga taataccgcg ccacatagca gaactttaaa agtgctcatc attggaaaac 4740
gttcttcggg gcgaaaactc tcaaggatct taccgctgtt gagatccagt tcgatgtaac 4800
ccactcgtgc acccaactga tcttcagcat cttttacttt caccagcgtt tctgggtgag 4860
caaaaacagg aaggcaaaat gccgcaaaaa agggaataag ggcgacacgg aaatgttgaa 4920
tactcatact cttccttttt caatattatt gaagcattta tcagggttat tgtctcatga 4980
gcggatacat atttgaatgt atttagaaaa ataaacaaat aggggttccg cgcacatttc 5040
cccgaaaagt gccacctgac gtctaagaaa ccattattat catgacatta acctataaaa 5100
ataggcgtat cacgaggccc tttcgtctcg cgcgtttcgg tgatgacggt gaaaacctct 5160
gacacatgca gctcccggag acggtcacag cttgtctgta agcggatgcc gggagcagac 5220
aagcccgtca gggcgcgtca gcgggtgttc atgtgcgtaa ctaacttgcc atcttcaaac 5280
aggagggctg gaagaagcag accgctaaca cagtacataa aaaaggagac atgaacgatg 5340
aacatcaaaa agtttgcaaa acaagcaaca gtattaacct ttactaccgc actgctggca 5400
ggaggcgcaa ctcaagcgtt tgcgaaagaa acgaaccaaa agccatataa ggaaacatac 5460
ggcatttccc atattacacg ccatgatatg ctgcaaatcc ctgaacagca aaaaaatgaa 5520
aaatatcaag ttcctgaatt cgattcgtcc acaattaaaa atatctcttc tgcaaaaggc 5580
ctggacgttt gggacagctg gccattacaa aacgctgacg gcactgtcgc aaactatcac 5640
ggctaccaca tcgtctttgc attagccgga gatcctaaaa atgcggatga cacatcgatt 5700
tacatgttct atcaaaaagt cggcgaaact tctattgaca gctggaaaaa cgctggccgc 5760
gtctttaaag acagcgacaa attcgatgca aatgattcta tcctaaaaga ccaaacacaa 5820
gaatggtcag gttcagccac atttacatct gacggaaaaa tccgtttatt ctacactgat 5880
ttctccggta aacattacgg caaacaaaca ctgacaactg cacaagttaa cgtatcagca 5940
tcagacagct ctttgaacat caacggtgta gaggattata aatcaatctt tgacggtgac 6000

ggaaaaacgt atcaaaatgt acagaattcg agctctcga 6039

<210> 147
<211> 44
<212> DNA
<213> Artificial Sequence

<220>
<223> Primer T-groE(XhoI)

<400> 147
agatagatct cgagagctat tgtaacataa tcggtacggg ggtg 44

<210> 148
<211> 51
<212> DNA
<213> Artificial Sequence

<220>
<223> Primer B-groEL (SpeI BamH1)

<400> 148
attatgtcag gatccactag tttcctcctt taattgggaa ttgttatccg c 51

<210> 149
<211> 30
<212> DNA
<213> Artificial Sequence

<220>
<223> Primer T-groEL

<400> 149
agctattgta acataatcgg tacgggggtg 30

<210> 150
<211> 45
<212> DNA
<213> Artificial Sequence

<220>
<223> Primer T-ilvCB.s.(BamHI)

<400> 150
acattgatgg atcccataac aagggagaga ttgaaatggt aaaag 45

<210> 151
<211> 47
<212> DNA
<213> Artificial Sequence

<220>
<223> Primer B-ilvCB.s.(SpeIBamHI)

<400> 151
tagacaacgg atccactagt ttaattttgc gcaacggaga ccaccgc 47

<210> 152
<211> 47

<212>  DNA
<213>  Artificial Sequence

<220>
<223>  Primer T-BD64 (DraIII)

<400>  152
ttaccgtgga ctcaccgagt gggtaactag cctcgccgga aagagcg                47


<210>  153
<211>  48
<212>  DNA
<213>  Artificial Sequence

<220>
<223>  Primer B-BD64 (DraIII)

<400>  153
tcacagttaa gacacctggt gccgttaatg cgccatgaca gccatgat               48


<210>  154
<211>  49
<212>  DNA
<213>  Artificial Sequence

<220>
<223>  Primer T-lacIq (DraIII)

<400>  154
acagatagat caccaggtgc aagctaattc cggtggaaac gaggtcatc              49


<210>  155
<211>  48
<212>  DNA
<213>  Artificial Sequence

<220>
<223>  Primer B-lacIq (DraIII)

<400>  155
acagtacgat acacggggtg tcactgcccg ctttccagtc gggaaacc               48


<210>  156
<211>  49
<212>  DNA
<213>  Artificial Sequence

<220>
<223>  Primer T-groE (DraIII)

<400>  156
tcggattacg caccccgtga gctattgtaa cataatcggt acgggggtg              49


<210>  157
<211>  48
<212>  DNA
<213>  Artificial Sequence

<220>
<223>  Primer B-B.s.ilvC (DraIII)

<400>  157
ctgctgatct cacaccgtgt gttaattttg cgcaacggag accaccgc                    48


<210>  158
<211>  1221
<212>  DNA
<213>  Clostridium acetobutylicum

<400>  158
cacacggtgt aaataataat ctaaacagga ggggttaaaa tggttgattt cgaatattca      60

ataccaacta gaattttttt cggtaaagat aagataaatg tacttggaag agagcttaaa     120

aaatatggtt ctaaagtgct tatagtttat ggtggaggaa gtataaagag aaatggaata     180

tatgataaag ctgtaagtat acttgaaaaa aacagtatta aattttatga acttgcagga     240

gtagagccaa atccaagagt aactacagtt gaaaaaggag ttaaaatatg tagagaaaat     300

ggagttgaag tagtactagc tataggtgga ggaagtgcaa tagattgcgc aaaggttata     360

gcagcagcat gtgaatatga tggaaatcca tgggatattg tgttagatgg ctcaaaaata     420

aaaagggtgc ttcctatagc tagtatatta accattgctg caacaggatc agaaatggat     480

acgtgggcag taataaataa tatggataca aacgaaaaac taattgcggc acatccagat     540

atggctccta agttttctat attagatcca acgtatacgt ataccgtacc taccaatcaa     600

acagcagcag gaacagctga tattatgagt catatatttg aggtgtattt tagtaataca     660

aaaacagcat atttgcagga tagaatggca gaagcgttat taagaacttg tattaaatat     720

ggaggaatag ctcttgagaa gccggatgat tatgaggcaa gagccaatct aatgtgggct     780

tcaagtcttg cgataaatgg acttttaaca tatggtaaag acactaattg gagtgtacac     840

ttaatggaac atgaattaag tgcttattac gacataacac acggcgtagg gcttgcaatt     900

ttaacaccta attggatgga gtatatttta aataatgata cagtgtacaa gtttgttgaa     960

tatggtgtaa atgtttgggg aatagacaaa gaaaaaaatc actatgacat agcacatcaa    1020

gcaatacaaa aaacaagaga ttactttgta aatgtactag gtttaccatc tagactgaga    1080

gatgttggaa ttgaagaaga aaaattggac ataatggcaa aggaatcagt aaagcttaca    1140

ggaggaacca taggaaacct aagaccagta aacgcctccg aagtcctaca aatattcaaa    1200

aaatctgtgt aacaccgagt g                                               1221


<210>  159
<211>  54
<212>  DNA
<213>  Artificial Sequence

<220>
<223>  Primer T-bdhB (DraIII)

<400>  159
tcgatagcat acacacggtg gttaacaaag gaggggttaa aatggttgat ttcg            54

<210>  160
<211>  91
<212>  DNA
<213>  Artificial Sequence

<220>
<223>  Primer B-bdhB (rrnBT1DraIII)

<400>  160
atctacgcac tcggtgataa aacgaaaggc ccagtctttc gactgagcct ttcgttttat      60

cttacacaga ttttttgaat atttgtagga c                                      91


<210>  161
<211>  29
<212>  DNA
<213>  Artificial Sequence

<220>
<223>  Primer LDH EcoRV F

<400>  161
gacgtcatga ccacccgccg atccctttt                                        29


<210>  162
<211>  30
<212>  DNA
<213>  Artificial Sequence

<220>
<223>  Primer LDH AatIIR

<400>  162
gatatccaac accagcgacc gacgtattac                                       30


<210>  163
<211>  47
<212>  DNA
<213>  Artificial Sequence

<220>
<223>  Primer Cm F

<400>  163
atttaaatct cgagtagagg atcccaacaa acgaaaattg gataaag                    47


<210>  164
<211>  29
<212>  DNA
<213>  Artificial Sequence

<220>
<223>  Primer Cm R

<400>  164
acgcgttatt ataaaagcca gtcattagg                                        29


<210>  165
<211>  58

<212>  DNA
<213>  Artificial Sequence

<220>
<223>  Primer P11 F-StuI

<400>  165
cctagcgcta tagttgttga cagaatggac atactatgat atattgttgc tatagcga       58


<210>  166
<211>  62
<212>  DNA
<213>  Artificial Sequence

<220>
<223>  Primer P11 R-SpeI

<400>  166
ctagtcgcta tagcaacaat atatcatagt atgtccattc tgtcaacaac tatagcgcta     60

gg                                                                    62


<210>  167
<211>  38
<212>  DNA
<213>  Artificial Sequence

<220>
<223>  Primer PldhL F-HindIII

<400>  167
aagcttgtcg acaaaccaac attatgacgt gtctgggc                             38


<210>  168
<211>  28
<212>  DNA
<213>  Artificial Sequence

<220>
<223>  Primer PldhL R-BamHI

<400>  168
ggatcctcat cctctcgtag tgaaaatt                                        28


<210>  169
<211>  36
<212>  DNA
<213>  Artificial Sequence

<220>
<223>  Primer F-bdhB-AvrII

<400>  169
ttcctaggaa ggaggtggtt aaaatggttg atttcg                               36


<210>  170
<211>  29
<212>  DNA
<213>  Artificial Sequence

<220>
<223>  Primer R-bdhB-BamHI

<400>  170
ttggatcctt acacagattt tttgaatat                                          29


<210>  171
<211>  39
<212>  DNA
<213>  Artificial Sequence

<220>
<223>  Primer F-ilvC(B.s.)-AflII

<400>  171
aacttaagaa ggaggtgatt gaaatggtaa aagtatatt                               39


<210>  172
<211>  32
<212>  DNA
<213>  Artificial Sequence

<220>
<223>  Primer R-ilvC(B.s.)-NotI

<400>  172
aagcggccgc ttaattttgc gcaacggaga cc                                      32


<210>  173
<211>  30
<212>  DNA
<213>  Artificial Sequence

<220>
<223>  Primer F-PnisA(HindIII)

<400>  173
ttaagcttga catacttgaa tgacctagtc                                         30


<210>  174
<211>  39
<212>  DNA
<213>  Artificial Sequence

<220>
<223>  Primer R-PnisA(SpeI BamHI)

<400>  174
ttggatccaa actagtataa tttattttgt agttccttc                               39


<210>  175
<211>  38
<212>  DNA
<213>  Artificial Sequence

<220>
<223>  Primer N191

<400>  175
atccgcggat agatctccca ttaccgacat ttgggcgc                                38

<210> 176
<211> 31
<212> DNA
<213> Artificial Sequence

<220>
<223> Primer N192

<400> 176
atcctcagcg atgattgatt gattgattgt a 31


<210> 177
<211> 6509
<212> DNA
<213> Artificial Sequence

<220>
<223> Vector pFP988

<400> 177
tcgaggcccc gcacatacga aaagactggc tgaaaacatt gagcctttga tgactgatga 60

tttggctgaa gaagtggatc gattgtttga gaaaagaaga agaccataaa aataccttgt 120

ctgtcatcag acagggtatt ttttatgctg tccagactgt ccgctgtgta aaaaatagga 180

ataaaggggg gttgttatta ttttactgat atgtaaaata taatttgtat aaggaattgt 240

gagcggataa caattcctac gaaaatgaga gggagaggaa acatgattca aaaacgaaag 300

cggacagttt cgttcagact tgtgcttatg tgcacgctgt tatttgtcag tttgccgatt 360

acaaaaacat cagccggatc ccaccatcac catcaccatt aagaattcct agaaactcca 420

agctatcttt aaaaaatcta gtaaatgcac gagcaacatc ttttgttgct cagtgcattt 480

tttattttgt acactagata tttcttctcc gcttaaatca tcaaagaaat ctttatcact 540

tgtaaccagt ccgtccacat gtcgaattgc atctgaccga attttacgtt tccctgaata 600

attctcatca atcgtttcat caattttatc tttatacttt atattttgtg cgttaatcaa 660

atcataattt ttatatgttt cctcatgatt tatgtcttta ttattatagt ttttattctc 720

tctttgatta tgtctttgta tcccgtttgt attacttgat cctttaactc tggcaaccct 780

caaaattgaa tgagacatgc tacacctccg gataataaat atatataaac gtatatagat 840

ttcataaagt ctaacacact agacttattt acttcgtaat taagtcgtta aaccgtgtgc 900

tctacgacca aaactataaa acctttaaga actttctttt tttacaagaa aaaagaaatt 960

agataaatct ctcatatctt ttattcaata atcgcatccg attgcagtat aaatttaacg 1020

atcactcatc atgttcatat ttatcagagc tcgtgctata attatactaa ttttataagg 1080

aggaaaaaat atgggcattt ttagtatttt tgtaatcagc acagttcatt atcaaccaaa 1140

caaaaaataa gtggttataa tgaatcgtta ataagcaaaa ttcatataac caaattaaag 1200

agggttataa tgaacgagaa aaatataaaa cacagtcaaa actttattac ttcaaaacat 1260

aatatagata aaataatgac aaatataaga ttaaatgaac atgataatat ctttgaaatc 1320

ggctcaggaa aaggccattt tacccttgaa ttagtaaaga ggtgtaattt cgtaactgcc 1380

attgaaatag accataaatt atgcaaaact acagaaaata aacttgttga tcacgataat 1440

ttccaagttt taaacaagga tatattgcag tttaaatttc ctaaaaacca atcctataaa 1500

atatatggta atatacctta taacataagt acggatataa tacgcaaaat tgtttttgat 1560

agtatagcta atgagattta tttaatcgtg gaatacgggt ttgctaaaag attattaaat 1620

acaaaacgct cattggcatt acttttaatg gcagaagttg atatttctat attaagtatg 1680

gttccaagag aatattttca tcctaaacct aaagtgaata gctcacttat cagattaagt 1740

agaaaaaaat caagaatatc acacaaagat aaacaaaagt ataattattt cgttatgaaa 1800

tgggttaaca aagaatacaa gaaaatattt acaaaaaatc aatttaacaa ttccttaaaa 1860

catgcaggaa ttgacgattt aaacaatatt agctttgaac aattcttatc tcttttcaat 1920

agctataaat tatttaataa gtaagttaag ggatgcagtt catcgatgaa ggcaactaca 1980

gctcaggcga caaccatacg ctgagagatc ctcactacgt agaagataaa ggccacaaat 2040

acttagtatt tgaagcaaac actggaactg aagatggcta ccaaggcgaa gaatctttat 2100

ttaacaaagc atactatggc aaaagcacat cattcttccg tcaagaaagt caaaaacttc 2160

tgcaaagcga taaaaaacgc acggctgagt tagcaaacgg cgctctcggt atgattgagc 2220

taaacgatga ttacacactg aaaaaagtga tgaaaccgct gattgcatct aacacagtaa 2280

cagatgaaat tgaacgcgcg aacgtcttta aaatgaacgg caaatggtac ctgttcactg 2340

actcccgcgg atcaaaaatg acgattgacg gcattacgtc taacgatatt tacatgcttg 2400

gttatgtttc taattcttta actggcccat acaagccgct gaacaaaact ggccttgtgt 2460

taaaaatgga tcttgatcct aacgatgtaa cctttactta ctcacacttc gctgtacctc 2520

aagcgaaagg aaacaatgtc gtgattacaa gctatatgac aaacagagga ttctacgcag 2580

acaaacaatc aacgtttgcg ccaagcttgc atgcgagagt agggaactgc caggcatcaa 2640

ataaaacgaa aggctcagtc gaaagactgg gcctttcgtt ttatctgttg tttgtcggtg 2700

aacgctctcc tgagtaggac aaatccgccg ggagcggatt tgaacgttgc gaagcaacgg 2760

cccggagggt ggcgggcagg acgcccgcca taaactgcca ggcatcaaat taagcagaag 2820

gccatcctga cggatggcct ttttgcgttt ctacaaactc tttttgttta tttttctaaa 2880

tacattcaaa tatgtatccg ctcatgctcc ggatctgcat cgcaggatgc tgctggctac 2940

cctgtggaac acctacatct gtattaacga agcgctggca ttgaccctga gtgatttttc 3000

tctggtcccg ccgcatccat accgccagtt gtttaccctc acaacgttcc agtaaccggg 3060

catgttcatc atcagtaacc cgtatcgtga gcatcctctc tcgtttcatc ggtatcatta 3120

cccccatgaa cagaaattcc cccttacacg gaggcatcaa gtgaccaaac aggaaaaaac 3180

cgcccttaac atggcccgct ttatcagaag ccagacatta acgcttctgg agaaactcaa 3240

cgagctggac gcggatgaac aggcagacat ctgtgaatcg cttcacgacc acgctgatga 3300

gctttaccgc agctgcctcg cgcgtttcgg tgatgacggt gaaaacctct gacacatgca 3360

gctcccggag acggtcacag cttgtctgta agcggatgcc gggagcagac aagcccgtca 3420

gggcgcgtca gcgggtgttg gcgggtgtcg gggcgcagcc atgacccagt cacgtagcga 3480

tagcggagtg tatactggct taactatgcg gcatcagagc agattgtact gagagtgcac 3540

catatgcggt gtgaaatacc gcacagatgc gtaaggagaa aataccgcat caggcgctct 3600

tccgcttcct cgctcactga ctcgctgcgc tcggtcgttc ggctgcggcg agcggtatca 3660

gctcactcaa aggcggtaat acggttatcc acagaatcag gggataacgc aggaaagaac 3720

atgtgagcaa aaggccagca aaaggccagg aaccgtaaaa aggccgcgtt gctggcgttt 3780

ttccataggc tccgcccccc tgacgagcat cacaaaaatc gacgctcaag tcagaggtgg 3840

cgaaacccga caggactata aagataccag gcgtttcccc ctggaagctc cctcgtgcgc 3900

tctcctgttc cgaccctgcc gcttaccgga tacctgtccg cctttctccc ttcgggaagc 3960

gtggcgcttt ctcaatgctc acgctgtagg tatctcagtt cggtgtaggt cgttcgctcc 4020

aagctgggct gtgtgcacga accccccgtt cagcccgacc gctgcgcctt atccggtaac 4080

tatcgtcttg agtccaaccc ggtaagacac gacttatcgc cactggcagc agccactggt 4140

aacaggatta gcagagcgag gtatgtaggc ggtgctacag agttcttgaa gtggtggcct 4200

aactacggct acactagaag gacagtattt ggtatctgcg ctctgctgaa gccagttacc 4260

ttcggaaaaa gagttggtag ctcttgatcc ggcaaacaaa ccaccgctgg tagcggtggt 4320

ttttttgttt gcaagcagca gattacgcgc agaaaaaaag gatctcaaga agatcctttg 4380

atcttttcta cggggtctga cgctcagtgg aacgaaaact cacgttaagg gattttggtc 4440

atgagattat caaaaaggat cttcacctag atccttttaa attaaaaatg aagttttaaa 4500

tcaatctaaa gtatatatga gtaaacttgg tctgacagtt accaatgctt aatcagtgag 4560

gcacctatct cagcgatctg tctatttcgt tcatccatag ttgcctgact ccccgtcgtg 4620

tagataacta cgatacggga gggcttacca tctggcccca gtgctgcaat gataccgcga 4680

gacccacgct caccggctcc agatttatca gcaataaacc agccagccgg aagggccgag 4740

cgcagaagtg gtcctgcaac tttatccgcc tccatccagt ctattaattg ttgccgggaa 4800

gctagagtaa gtagttcgcc agttaatagt ttgcgcaacg ttgttgccat tgctgcaggc 4860

atcgtggtgt cacgctcgtc gtttggtatg gcttcattca gctccggttc ccaacgatca 4920

aggcgagtta catgatcccc catgttgtgc aaaaaagcgg ttagctcctt cggtcctccg 4980

atcgttgtca gaagtaagtt ggccgcagtg ttatcactca tggttatggc agcactgcat 5040

aattctctta ctgtcatgcc atccgtaaga tgcttttctg tgactggtga gtactcaacc 5100

aagtcattct gagaatagtg tatgcggcga ccgagttgct cttgcccggc gtcaatacgg 5160

gataataccg cgccacatag cagaacttta aaagtgctca tcattggaaa acgttcttcg 5220

gggcgaaaac tctcaaggat cttaccgctg ttgagatcca gttcgatgta acccactcgt 5280

gcacccaact gatcttcagc atcttttact ttcaccagcg tttctgggtg agcaaaaaca 5340

ggaaggcaaa atgccgcaaa aaagggaata agggcgacac ggaaatgttg aatactcata 5400

ctcttccttt ttcaatatta ttgaagcatt tatcagggtt attgtctcat gagcggatac 5460

atatttgaat gtatttagaa aaataaacaa ataggggttc cgcgcacatt tccccgaaaa 5520

gtgccacctg acgtctaaga aaccattatt atcatgacat taacctataa aaataggcgt 5580

atcacgaggc cctttcgtct cgcgcgtttc ggtgatgacg gtgaaaacct ctgacacatg 5640

cagctcccgg agacggtcac agcttgtctg taagcggatg ccgggagcag acaagcccgt 5700

cagggcgcgt cagcgggtgt tcatgtgcgt aactaacttg ccatcttcaa acaggagggc 5760

tggaagaagc agaccgctaa cacagtacat aaaaaaggag acatgaacga tgaacatcaa 5820

aaagtttgca aaacaagcaa cagtattaac ctttactacc gcactgctgg caggaggcgc 5880

aactcaagcg tttgcgaaag aaacgaacca aaagccatat aaggaaacat acggcatttc 5940

ccatattaca cgccatgata tgctgcaaat ccctgaacag caaaaaaatg aaaaatatca 6000

agttcctgaa ttcgattcgt ccacaattaa aaatatctct tctgcaaaag gcctggacgt 6060

ttgggacagc tggccattac aaaacgctga cggcactgtc gcaaactatc acggctacca 6120

catcgtcttt gcattagccg gagatcctaa aaatgcggat gacacatcga tttacatgtt 6180

ctatcaaaaa gtcggcgaaa cttctattga cagctggaaa aacgctggcc gcgtctttaa 6240

agacagcgac aaattcgatg caaatgattc tatcctaaaa gaccaaacac aagaatggtc 6300

aggttcagcc acatttacat ctgacggaaa aatccgttta ttctacactg atttctccgg 6360

taaacattac ggcaaacaaa cactgacaac tgcacaagtt aacgtatcag catcagacag 6420

ctctttgaac atcaacggtg tagaggatta taaatcaatc tttgacggtg acggaaaaac 6480

gtatcaaaat gtacagcatg ccacgcgtc 6509

<210> 178
<211> 571
<212> PRT
<213> Bacillus subtilis

<400> 178

Met Leu Thr Lys Ala Thr Lys Glu Gln Lys Ser Leu Val Lys Asn Arg
1               5                   10                  15

Gly Ala Glu Leu Val Val Asp Cys Leu Val Glu Gln Gly Val Thr His
            20                  25                  30

Val Phe Gly Ile Pro Gly Ala Lys Ile Asp Ala Val Phe Asp Ala Leu
        35                  40                  45

Gln Asp Lys Gly Pro Glu Ile Ile Val Ala Arg His Glu Gln Asn Ala
    50                  55                  60

Ala Phe Met Ala Gln Ala Val Gly Arg Leu Thr Gly Lys Pro Gly Val
65                  70                  75                  80

Val Leu Val Thr Ser Gly Pro Gly Ala Ser Asn Leu Ala Thr Gly Leu
                85                  90                  95

Leu Thr Ala Asn Thr Glu Gly Asp Pro Val Val Ala Leu Ala Gly Asn
            100                 105                 110

Val Ile Arg Ala Asp Arg Leu Lys Arg Thr His Gln Ser Leu Asp Asn
        115                 120                 125

Ala Ala Leu Phe Gln Pro Ile Thr Lys Tyr Ser Val Glu Val Gln Asp
    130                 135                 140

Val Lys Asn Ile Pro Glu Ala Val Thr Asn Ala Phe Arg Ile Ala Ser
145                 150                 155                 160

Ala Gly Gln Ala Gly Ala Ala Phe Val Ser Phe Pro Gln Asp Val Val
                165                 170                 175

Asn Glu Val Thr Asn Thr Lys Asn Val Arg Ala Val Ala Ala Pro Lys
            180                 185                 190

Leu Gly Pro Ala Ala Asp Asp Ala Ile Ser Ala Ala Ile Ala Lys Ile
        195                 200                 205

Gln Thr Ala Lys Leu Pro Val Val Leu Val Gly Met Lys Gly Gly Arg
    210                 215                 220

Pro Glu Ala Ile Lys Ala Val Arg Lys Leu Leu Lys Lys Val Gln Leu
225                 230                 235                 240

Pro Phe Val Glu Thr Tyr Gln Ala Ala Gly Thr Leu Ser Arg Asp Leu
                245                 250                 255

Glu Asp Gln Tyr Phe Gly Arg Ile Gly Leu Phe Arg Asn Gln Pro Gly
            260                 265                 270

Asp Leu Leu Leu Glu Gln Ala Asp Val Val Leu Thr Ile Gly Tyr Asp
        275                 280                 285

Pro Ile Glu Tyr Asp Pro Lys Phe Trp Asn Ile Asn Gly Asp Arg Thr
    290                 295                 300

Ile Ile His Leu Asp Glu Ile Ile Ala Asp Ile Asp His Ala Tyr Gln
305                 310                 315                 320

Pro Asp Leu Glu Leu Ile Gly Asp Ile Pro Ser Thr Ile Asn His Ile
                325                 330                 335

Glu His Asp Ala Val Lys Val Glu Phe Ala Glu Arg Glu Gln Lys Ile
            340                 345                 350

Leu Ser Asp Leu Lys Gln Tyr Met His Glu Gly Glu Gln Val Pro Ala
        355                 360                 365

Asp Trp Lys Ser Asp Arg Ala His Pro Leu Glu Ile Val Lys Glu Leu
    370                 375                 380

Arg Asn Ala Val Asp Asp His Val Thr Val Thr Cys Asp Ile Gly Ser
385                 390                 395                 400

His Ala Ile Trp Met Ser Arg Tyr Phe Arg Ser Tyr Glu Pro Leu Thr
                405                 410                 415

Leu Met Ile Ser Asn Gly Met Gln Thr Leu Gly Val Ala Leu Pro Trp
            420                 425                 430

Ala Ile Gly Ala Ser Leu Val Lys Pro Gly Glu Lys Val Val Ser Val
        435                 440                 445

Ser Gly Asp Gly Gly Phe Leu Phe Ser Ala Met Glu Leu Glu Thr Ala
    450                 455                 460

Val Arg Leu Lys Ala Pro Ile Val His Ile Val Trp Asn Asp Ser Thr
465                 470                 475                 480

Tyr Asp Met Val Ala Phe Gln Gln Leu Lys Lys Tyr Asn Arg Thr Ser
                485                 490                 495

Ala Val Asp Phe Gly Asn Ile Asp Ile Val Lys Tyr Ala Glu Ser Phe
            500                 505                 510

Gly Ala Thr Gly Leu Arg Val Glu Ser Pro Asp Gln Leu Ala Asp Val
        515                 520                 525

Leu Arg Gln Gly Met Asn Ala Glu Gly Pro Val Ile Ile Asp Val Pro
    530                 535                 540

Val Asp Tyr Ser Asp Asn Ile Asn Leu Ala Ser Asp Lys Leu Pro Lys
545                 550                 555                 560

Glu Phe Gly Glu Leu Met Lys Thr Lys Ala Leu

                565                 570

<210> 179
<211> 1665
<212> DNA
<213> Lactococcus lactis

<400> 179
atgtctgaga aacaatttgg ggcgaacttg gttgtcgata gtttgattaa ccataaagtg 60

aagtatgtat ttgggattcc aggagcaaaa attgaccggg tttttgattt attagaaaat 120

gaagaaggcc ctcaaatggt cgtgactcgt catgagcaag gagctgcttt catggctcaa 180

gctgtcggtc gtttaactgg cgaacctggt gtagtagttg ttacgagtgg gcctggtgta 240

tcaaaccttg cgactccgct tttgaccgcg acatcagaag gtgatgctat tttggctatc 300

ggtggacaag ttaaacgaag tgaccgtctt aaacgtgcgc accaatcaat ggataatgct 360

ggaatgatgc aatcagcaac aaaatattca gcagaagttc ttgaccctaa tacactttct 420

gaatcaattg ccaacgctta tcgtattgca aaatcaggac atccaggtgc aactttctta 480

tcaatccccc aagatgtaac ggatgccgaa gtatcaatca aagccattca accactttca 540

gaccctaaaa tggggaatgc ctctattgat gacattaatt atttagcaca agcaattaaa 600

aatgctgtat tgccagtaat tttggttgga gctggtgctt cagatgctaa agtcgcttca 660

tccttgcgta atctattgac tcatgttaat attcctgtcg ttgaaacatt ccaaggtgca 720

ggggttattt cacatgattt agaacatact ttttatggac gtatcggtct tttccgcaat 780

caaccaggcg atatgcttct gaaacgttct gaccttgtta ttgctgttgg ttatgaccca 840

attgaatatg aagctcgtaa ctggaatgca gaaattgata gtcgaattat cgttattgat 900

aatgccattg ctgaaattga tacttactac caaccagagc gtgaattaat tggtgatatc 960

gcagcaacat tggataatct tttaccagct gttcgtggct acaaaattcc aaaaggaaca 1020

aaagattatc tcgatggcct tcatgaagtt gctgagcaac acgaatttga tactgaaaat 1080

actgaagaag gtagaatgca ccctcttgat ttggtcagca ctttccaaga aatcgtcaag 1140

gatgatgaaa cagtaaccgt tgacgtaggt tcactctaca tttggatggc acgtcatttc 1200

aaatcatacg aaccacgtca tctcctcttc tcaaacggaa tgcaaacact cggagttgca 1260

cttccttggg caattacagc cgcattgttg cgcccaggta aaaaagttta ttcacactct 1320

ggtgatggag gcttcctttt cacagggcaa gaattggaaa cagctgtacg tttgaatctt 1380

ccaatcgttc aaattatctg gaatgacggc cattatgata tggttaaatt ccaagaagaa 1440

atgaaatatg gtcgttcagc agccgttgat tttggctatg ttgattacgt aaaatatgct 1500

gaagcaatga gagcaaaagg ttaccgtgca cacagcaaag aagaacttgc tgaaattctc 1560

aaatcaatcc cagatactac tggaccggtg gtaattgacg ttcctttgga ctattctgat 1620

aacattaaat tagcagaaaa attattgcct gaagagtttt attga 1665

<210>  180
<211>  554
<212>  PRT
<213>  Lactococcus lactis

<400>  180
   Met Ser Glu Lys Gln Phe Gly Ala Asn Leu Val Val Asp Ser Leu Ile
   1 5 10 15
   Asn His Lys Val Lys Tyr Val Phe Gly Ile Pro Gly Ala Lys Ile Asp
   20 25 30
   Arg Val Phe Asp Leu Leu Glu Asn Glu Glu Gly Pro Gln Met Val Val
   35 40 45
   Thr Arg His Glu Gln Gly Ala Ala Phe Met Ala Gln Ala Val Gly Arg
   50 55 60
   Leu Thr Gly Glu Pro Gly Val Val Val Val Thr Ser Gly Pro Gly Val
   65 70 75 80
   Ser Asn Leu Ala Thr Pro Leu Leu Thr Ala Thr Ser Glu Gly Asp Ala
   85 90 95
   Ile Leu Ala Ile Gly Gly Gln Val Lys Arg Ser Asp Arg Leu Lys Arg
   100 105 110
   Ala His Gln Ser Met Asp Asn Ala Gly Met Met Gln Ser Ala Thr Lys
   115 120 125
   Tyr Ser Ala Glu Val Leu Asp Pro Asn Thr Leu Ser Glu Ser Ile Ala
   130 135 140
   Asn Ala Tyr Arg Ile Ala Lys Ser Gly His Pro Gly Ala Thr Phe Leu
   145 150 155 160
   Ser Ile Pro Gln Asp Val Thr Asp Ala Glu Val Ser Ile Lys Ala Ile
   165 170 175
   Gln Pro Leu Ser Asp Pro Lys Met Gly Asn Ala Ser Ile Asp Asp Ile
   180 185 190
   Asn Tyr Leu Ala Gln Ala Ile Lys Asn Ala Val Leu Pro Val Ile Leu
   195 200 205
   Val Gly Ala Gly Ala Ser Asp Ala Lys Val Ala Ser Ser Leu Arg Asn
   210 215 220
   Leu Leu Thr His Val Asn Ile Pro Val Val Glu Thr Phe Gln Gly Ala
   225 230 235 240
   Gly Val Ile Ser His Asp Leu Glu His Thr Phe Tyr Gly Arg Ile Gly
   245 250 255
   Leu Phe Arg Asn Gln Pro Gly Asp Met Leu Leu Lys Arg Ser Asp Leu
   260 265 270
   Val Ile Ala Val Gly Tyr Asp Pro Ile Glu Tyr Glu Ala Arg Asn Trp
   275 280 285
   Asn Ala Glu Ile Asp Ser Arg Ile Ile Val Ile Asp Asn Ala Ile Ala
   290 295 300
   Glu Ile Asp Thr Tyr Tyr Gln Pro Glu Arg Glu Leu Ile Gly Asp Ile
   305 310 315 320
   Ala Ala Thr Leu Asp Asn Leu Leu Pro Ala Val Arg Gly Tyr Lys Ile
   325 330 335
   Pro Lys Gly Thr Lys Asp Tyr Leu Asp Gly Leu His Glu Val Ala Glu
   340 345 350
   Gln His Glu Phe Asp Thr Glu Asn Thr Glu Glu Gly Arg Met His Pro
   355 360 365
   Leu Asp Leu Val Ser Thr Phe Gln Glu Ile Val Lys Asp Asp Glu Thr
   370 375 380
   Val Thr Val Asp Val Gly Ser Leu Tyr Ile Trp Met Ala Arg His Phe
   385 390 395 400
   Lys Ser Tyr Glu Pro Arg His Leu Leu Phe Ser Asn Gly Met Gln Thr
   405 410 415
   Leu Gly Val Ala Leu Pro Trp Ala Ile Thr Ala Ala Leu Leu Arg Pro
   420 425 430
   Gly Lys Lys Val Tyr Ser His Ser Gly Asp Gly Gly Phe Leu Phe Thr
   435 440 445
   Gly Gln Glu Leu Glu Thr Ala Val Arg Leu Asn Leu Pro Ile Val Gln
   450 455 460
   Ile Ile Trp Asn Asp Gly His Tyr Asp Met Val Lys Phe Gln Glu Glu
   465 470 475 480
   Met Lys Tyr Gly Arg Ser Ala Ala Val Asp Phe Gly Tyr Val Asp Tyr
   485 490 495
   Val Lys Tyr Ala Glu Ala Met Arg Ala Lys Gly Tyr Arg Ala His Ser
               500                 505                 510
   Lys Glu Glu Leu Ala Glu Ile Leu Lys Ser Ile Pro Asp Thr Thr Gly
           515                 520                 525
   Pro Val Val Ile Asp Val Pro Leu Asp Tyr Ser Asp Asn Ile Lys Leu
       530                 535                 540
   Ala Glu Lys Leu Leu Pro Glu Glu Phe Tyr
   545                 550

<210>  181
<211>  395
<212>  PRT
<213>  Saccharomyces cerevisiae

<400>  181

Met Leu Arg Thr Gln Ala Ala Arg Leu Ile Cys Asn Ser Arg Val Ile
1               5                   10                  15

Thr Ala Lys Arg Thr Phe Ala Leu Ala Thr Arg Ala Ala Ala Tyr Ser
            20                  25                  30

Arg Pro Ala Ala Arg Phe Val Lys Pro Met Ile Thr Thr Arg Gly Leu
        35                  40                  45

Lys Gln Ile Asn Phe Gly Gly Thr Val Glu Thr Val Tyr Glu Arg Ala
    50                  55                  60

Asp Trp Pro Arg Glu Lys Leu Leu Asp Tyr Phe Lys Asn Asp Thr Phe
65                  70                  75                  80

Ala Leu Ile Gly Tyr Gly Ser Gln Gly Tyr Gly Gln Gly Leu Asn Leu
                85                  90                  95

Arg Asp Asn Gly Leu Asn Val Ile Ile Gly Val Arg Lys Asp Gly Ala
            100                 105                 110

Ser Trp Lys Ala Ala Ile Glu Asp Gly Trp Val Pro Gly Lys Asn Leu
        115                 120                 125

Phe Thr Val Glu Asp Ala Ile Lys Arg Gly Ser Tyr Val Met Asn Leu
    130                 135                 140

Leu Ser Asp Ala Ala Gln Ser Glu Thr Trp Pro Ala Ile Lys Pro Leu
145                 150                 155                 160

Leu Thr Lys Gly Lys Thr Leu Tyr Phe Ser His Gly Phe Ser Pro Val
                165                 170                 175

Phe Lys Asp Leu Thr His Val Glu Pro Pro Lys Asp Leu Asp Val Ile
            180                 185                 190

Leu Val Ala Pro Lys Gly Ser Gly Arg Thr Val Arg Ser Leu Phe Lys
        195                 200                 205

Glu Gly Arg Gly Ile Asn Ser Ser Tyr Ala Val Trp Asn Asp Val Thr
    210                 215                 220

Gly Lys Ala His Glu Lys Ala Gln Ala Leu Ala Val Ala Ile Gly Ser
225                 230                 235                 240

Gly Tyr Val Tyr Gln Thr Thr Phe Glu Arg Glu Val Asn Ser Asp Leu
                245                 250                 255

Tyr Gly Glu Arg Gly Cys Leu Met Gly Gly Ile His Gly Met Phe Leu
            260                 265                 270

Ala Gln Tyr Asp Val Leu Arg Glu Asn Gly His Ser Pro Ser Glu Ala
        275                 280                 285

Phe Asn Glu Thr Val Glu Glu Ala Thr Gln Ser Leu Tyr Pro Leu Ile
    290                 295                 300

Gly Lys Tyr Gly Met Asp Tyr Met Tyr Asp Ala Cys Ser Thr Thr Ala
305                 310                 315                 320

Arg Arg Gly Ala Leu Asp Trp Tyr Pro Ile Phe Lys Asn Ala Leu Lys
                325                 330                 335

Pro Val Phe Gln Asp Leu Tyr Glu Ser Thr Lys Asn Gly Thr Glu Thr
            340                 345                 350

Lys Arg Ser Leu Glu Phe Asn Ser Gln Pro Asp Tyr Arg Glu Lys Leu
        355                 360                 365

Glu Lys Glu Leu Asp Thr Ile Arg Asn Met Glu Ile Trp Lys Val Gly
    370                 375                 380

Lys Glu Val Arg Lys Leu Arg Pro Glu Asn Gln
385                 390                 395

<210>  182
<211>  993
<212>  DNA
<213>  Methanococcus maripaludis

<400>  182
atgaaggtat tctatgactc agattttaaa ttagatgctt taaaagaaaa aacaattgca      60

gtaatcggtt atggaagtca aggtagggca cagtccttaa acatgaaaga cagcggatta     120

aacgttgttg ttggtttaag aaaaaacggt gcttcatgga acaacgctaa agcagacggt     180

cacaatgtaa tgaccattga agaagctgct gaaaaagcgg acatcatcca catcttaata     240

cctgatgaat tacaggcaga agtttatgaa agccagataa aaccatacct aaaagaagga     300

aaaacactaa gcttttcaca tggttttaac atccactatg gattcattgt tccaccaaaa     360

ggagttaacg tggttttagt tgctccaaaa tcacctggaa aaatggttag aagaacatac     420

gaagaaggtt tcggtgttcc aggtttaatc tgtattgaaa ttgatgcaac aaacaacgca     480

tttgatattg tttcagcaat ggcaaaagga atcggtttat caagagctgg agttatccag     540

acaactttca aagaagaaac agaaactgac cttttcggtg aacaagctgt tttatgcggt     600

ggagttaccg aattaatcaa ggcaggattt gaaacactcg ttgaagcagg atacgcacca     660

gaaatggcat actttgaaac ctgccacgaa ttgaaattaa tcgttgactt aatctaccaa     720

aaaggattca aaaacatgtg gaacgatgta agtaacactg cagaatacgg cggacttaca     780

agaagaagca gaatcgttac agctgattca aaagctgcaa tgaaagaaat cttaagagaa     840

atccaagatg gaagattcac aaaagaattc cttctcgaaa aacaggtaag ctatgctcat     900

ttaaaatcaa tgagaagact cgaaggagac ttacaaatcg aagaagtcgg cgcaaaatta     960

agaaaaatgt gcggtcttga aaaagaagaa taa                                  993

<210>  183
<211>  330
<212>  PRT
<213>  Methanococcus maripaludis

<400>  183

Met Lys Val Phe Tyr Asp Ser Asp Phe Lys Leu Asp Ala Leu Lys Glu
1               5                   10                  15

Lys Thr Ile Ala Val Ile Gly Tyr Gly Ser Gln Gly Arg Ala Gln Ser
            20                  25                  30

Leu Asn Met Lys Asp Ser Gly Leu Asn Val Val Val Gly Leu Arg Lys
        35                  40                  45

Asn Gly Ala Ser Trp Asn Asn Ala Lys Ala Asp Gly His Asn Val Met
    50                  55                  60

Thr Ile Glu Glu Ala Ala Glu Lys Ala Asp Ile Ile His Ile Leu Ile
65                  70                  75                  80

Pro Asp Glu Leu Gln Ala Glu Val Tyr Glu Ser Gln Ile Lys Pro Tyr
                85                  90                  95

Leu Lys Glu Gly Lys Thr Leu Ser Phe Ser His Gly Phe Asn Ile His
        100                 105                 110

Tyr Gly Phe Ile Val Pro Pro Lys Gly Val Asn Val Val Leu Val Ala
    115                 120                 125

Pro Lys Ser Pro Gly Lys Met Val Arg Arg Thr Tyr Glu Glu Gly Phe
    130                 135                 140

Gly Val Pro Gly Leu Ile Cys Ile Glu Ile Asp Ala Thr Asn Asn Ala
145                 150                 155                 160

Phe Asp Ile Val Ser Ala Met Ala Lys Gly Ile Gly Leu Ser Arg Ala
                165                 170                 175

Gly Val Ile Gln Thr Thr Phe Lys Glu Glu Thr Glu Thr Asp Leu Phe
            180                 185                 190

Gly Glu Gln Ala Val Leu Cys Gly Gly Val Thr Glu Leu Ile Lys Ala
        195                 200                 205

Gly Phe Glu Thr Leu Val Glu Ala Gly Tyr Ala Pro Glu Met Ala Tyr
    210                 215                 220

Phe Glu Thr Cys His Glu Leu Lys Leu Ile Val Asp Leu Ile Tyr Gln
225                 230                 235                 240

Lys Gly Phe Lys Asn Met Trp Asn Asp Val Ser Asn Thr Ala Glu Tyr
                245                 250                 255

Gly Gly Leu Thr Arg Arg Ser Arg Ile Val Thr Ala Asp Ser Lys Ala
            260                 265                 270

Ala Met Lys Glu Ile Leu Arg Glu Ile Gln Asp Gly Arg Phe Thr Lys
        275                 280                 285

Glu Phe Leu Leu Glu Lys Gln Val Ser Tyr Ala His Leu Lys Ser Met
    290                 295                 300

Arg Arg Leu Glu Gly Asp Leu Gln Ile Glu Glu Val Gly Ala Lys Leu
305                 310                 315                 320

Arg Lys Met Cys Gly Leu Glu Lys Glu Glu
                325                 330

<210>  184
<211>  1476
<212>  DNA

<213>  Bacillus subtilis

<400>  184
atggctaact acttcaatac actgaatctg cgccagcagc tggcacagct gggcaaatgt      60

cgctttatgg gccgcgatga attcgccgat ggcgcgagct accttcaggg taaaaaagta     120

gtcatcgtcg gctgtggcgc acagggtctg aaccagggcc tgaacatgcg tgattctggt     180

ctcgatatct cctacgctct gcgtaaagaa gcgattgccg agaagcgcgc gtcctggcgt     240

aaagcgaccg aaaatggttt taaagtgggt acttacgaag aactgatccc acaggcggat     300

ctggtgatta acctgacgcc ggacaagcag cactctgatg tagtgcgcac cgtacagcca     360

ctgatgaaag acggcgcggc gctgggctac tcgcacggtt tcaacatcgt cgaagtgggc     420

gagcagatcc gtaaagatat caccgtagtg atggttgcgc cgaaatgccc aggcaccgaa     480

gtgcgtgaag agtacaaacg tgggttcggc gtaccgacgc tgattgccgt tcacccggaa     540

aacgatccga aaggcgaagg catggcgatt gccaaagcct gggcggctgc aaccggtggt     600

caccgtgcgg gtgtgctgga atcgtccttc gttgcggaag tgaaatctga cctgatgggc     660

gagcaaacca tcctgtgcgg tatgttgcag gctggctctc tgctgtgctt cgacaagctg     720

gtggaagaag gtaccgatcc agcatacgca gaaaaactga ttcagttcgg ttgggaaacc     780

atcaccgaag cactgaaaca gggcggcatc accctgatga tggaccgtct ctctaacccg     840

gcgaaactgc gtgcttatgc gctttctgaa cagctgaaag agatcatggc acccctgttc     900

cagaaacata tggacgacat catctccggc gaattctctt ccggtatgat ggcggactgg     960

gccaacgatg ataagaaact gctgacctgg cgtgaagaga ccggcaaaac cgcgtttgaa    1020

accgcgccgc agtatgaagg caaaatcggc gagcaggagt acttcgataa aggcgtactg    1080

atgattgcga tggtgaaagc gggcgttgaa ctggcgttcg aaaccatggt cgattccggc    1140

atcattgaag agtctgcata ttatgaatca ctgcacgagc tgccgctgat tgccaacacc    1200

atcgcccgta agcgtctgta cgaaatgaac gtggttatct ctgataccgc tgagtacggt    1260

aactatctgt tctcttacgc ttgtgtgccg ttgctgaaac cgtttatggc agagctgcaa    1320

ccgggcgacc tgggtaaagc tattccggaa ggcgcggtag ataacgggca actgcgtgat    1380

gtgaacgaag cgattcgcag ccatgcgatt gagcaggtag gtaagaaact gcgcggctat    1440

atgacagata tgaaacgtat tgctgttgcg ggttaa                              1476


<210>  185
<211>  342
<212>  PRT
<213>  Bacillus subtilis

<400>  185

Met Val Lys Val Tyr Tyr Asn Gly Asp Ile Lys Glu Asn Val Leu Ala
1               5                   10                  15

Gly Lys Thr Val Ala Val Ile Gly Tyr Gly Ser Gln Gly His Ala His
            20                  25                  30

Ala Leu Asn Leu Lys Glu Ser Gly Val Asp Val Ile Val Gly Val Arg
        35                  40                  45

Gln Gly Lys Ser Phe Thr Gln Ala Gln Glu Asp Gly His Lys Val Phe
    50                  55                  60

Ser Val Lys Glu Ala Ala Ala Gln Ala Glu Ile Ile Met Val Leu Leu
65                  70                  75                  80

Pro Asp Glu Gln Gln Gln Lys Val Tyr Glu Ala Glu Ile Lys Asp Glu
                85                  90                  95

Leu Thr Ala Gly Lys Ser Leu Val Phe Ala His Gly Phe Asn Val His
            100                 105                 110

Phe His Gln Ile Val Pro Pro Ala Asp Val Asp Val Phe Leu Val Ala
        115                 120                 125

Pro Lys Gly Pro Gly His Leu Val Arg Arg Thr Tyr Glu Gln Gly Ala
    130                 135                 140

Gly Val Pro Ala Leu Phe Ala Ile Tyr Gln Asp Val Thr Gly Glu Ala
145                 150                 155                 160

Arg Asp Lys Ala Leu Ala Tyr Ala Lys Gly Ile Gly Gly Ala Arg Ala
                165                 170                 175

Gly Val Leu Glu Thr Thr Phe Lys Glu Glu Thr Glu Thr Asp Leu Phe
            180                 185                 190

Gly Glu Gln Ala Val Leu Cys Gly Gly Leu Ser Ala Leu Val Lys Ala
        195                 200                 205

Gly Phe Glu Thr Leu Thr Glu Ala Gly Tyr Gln Pro Glu Leu Ala Tyr
    210                 215                 220

Phe Glu Cys Leu His Glu Leu Lys Leu Ile Val Asp Leu Met Tyr Glu
225                 230                 235                 240

Glu Gly Leu Ala Gly Met Arg Tyr Ser Ile Ser Asp Thr Ala Gln Trp
                245                 250                 255

Gly Asp Phe Val Ser Gly Pro Arg Val Val Asp Ala Lys Val Lys Glu
            260                 265                 270

Ser Met Lys Glu Val Leu Lys Asp Ile Gln Asn Gly Thr Phe Ala Lys

275                 280                 285

Glu Trp Ile Val Glu Asn Gln Val Asn Arg Pro Arg Phe Asn Ala Ile
    290                 295                 300

Asn Ala Ser Glu Asn Glu His Gln Ile Glu Val Val Gly Arg Lys Leu
305                 310                 315                 320

Arg Glu Met Met Pro Phe Val Lys Gln Gly Lys Lys Lys Glu Ala Val
                325                 330                 335

Val Ser Val Ala Gln Asn
            340

<210>  186
<211>  585
<212>  PRT
<213>  Saccharomyces cerevisiae

<400>  186

Met Gly Leu Leu Thr Lys Val Ala Thr Ser Arg Gln Phe Ser Thr Thr
1               5                   10                  15

Arg Cys Val Ala Lys Lys Leu Asn Lys Tyr Ser Tyr Ile Ile Thr Glu
            20                  25                  30

Pro Lys Gly Gln Gly Ala Ser Gln Ala Met Leu Tyr Ala Thr Gly Phe
        35                  40                  45

Lys Lys Glu Asp Phe Lys Lys Pro Gln Val Gly Val Gly Ser Cys Trp
    50                  55                  60

Trp Ser Gly Asn Pro Cys Asn Met His Leu Leu Asp Leu Asn Asn Arg
65                  70                  75                  80

Cys Ser Gln Ser Ile Glu Lys Ala Gly Leu Lys Ala Met Gln Phe Asn
                85                  90                  95

Thr Ile Gly Val Ser Asp Gly Ile Ser Met Gly Thr Lys Gly Met Arg
            100                 105                 110

Tyr Ser Leu Gln Ser Arg Glu Ile Ile Ala Asp Ser Phe Glu Thr Ile
        115                 120                 125

Met Met Ala Gln His Tyr Asp Ala Asn Ile Ala Ile Pro Ser Cys Asp
    130                 135                 140

Lys Asn Met Pro Gly Val Met Met Ala Met Gly Arg His Asn Arg Pro
145                 150                 155                 160

Ser Ile Met Val Tyr Gly Gly Thr Ile Leu Pro Gly His Pro Thr Cys
                165                 170                 175

Gly Ser Ser Lys Ile Ser Lys Asn Ile Asp Ile Val Ser Ala Phe Gln
            180                 185                 190

Ser Tyr Gly Glu Tyr Ile Ser Lys Gln Phe Thr Glu Glu Glu Arg Glu
        195                 200                 205

Asp Val Val Glu His Ala Cys Pro Gly Pro Gly Ser Cys Gly Gly Met
    210                 215                 220

Tyr Thr Ala Asn Thr Met Ala Ser Ala Ala Glu Val Leu Gly Leu Thr
225                 230                 235                 240

Ile Pro Asn Ser Ser Ser Phe Pro Ala Val Ser Lys Glu Lys Leu Ala
                245                 250                 255

Glu Cys Asp Asn Ile Gly Glu Tyr Ile Lys Lys Thr Met Glu Leu Gly
            260                 265                 270

Ile Leu Pro Arg Asp Ile Leu Thr Lys Glu Ala Phe Glu Asn Ala Ile
        275                 280                 285

Thr Tyr Val Val Ala Thr Gly Gly Ser Thr Asn Ala Val Leu His Leu
    290                 295                 300

Val Ala Val Ala His Ser Ala Gly Val Lys Leu Ser Pro Asp Asp Phe
305                 310                 315                 320

Gln Arg Ile Ser Asp Thr Thr Pro Leu Ile Gly Asp Phe Lys Pro Ser
                325                 330                 335

Gly Lys Tyr Val Met Ala Asp Leu Ile Asn Val Gly Gly Thr Gln Ser
            340                 345                 350

Val Ile Lys Tyr Leu Tyr Glu Asn Asn Met Leu His Gly Asn Thr Met
        355                 360                 365

Thr Val Thr Gly Asp Thr Leu Ala Glu Arg Ala Lys Lys Ala Pro Ser
    370                 375                 380

Leu Pro Glu Gly Gln Glu Ile Ile Lys Pro Leu Ser His Pro Ile Lys
385                 390                 395                 400

Ala Asn Gly His Leu Gln Ile Leu Tyr Gly Ser Leu Ala Pro Gly Gly
                405                 410                 415

Ala Val Gly Lys Ile Thr Gly Lys Glu Gly Thr Tyr Phe Lys Gly Arg
            420                 425                 430

Ala Arg Val Phe Glu Glu Glu Gly Ala Phe Ile Glu Ala Leu Glu Arg
        435                 440                 445

Gly Glu Ile Lys Lys Gly Glu Lys Thr Val Val Val Ile Arg Tyr Glu
    450                 455                 460

Gly Pro Arg Gly Ala Pro Gly Met Pro Glu Met Leu Lys Pro Ser Ser
465                 470                 475                 480

Ala Leu Met Gly Tyr Gly Leu Gly Lys Asp Val Ala Leu Leu Thr Asp
                485                 490                 495

Gly Arg Phe Ser Gly Gly Ser His Gly Phe Leu Ile Gly His Ile Val
            500                 505                 510

Pro Glu Ala Ala Glu Gly Gly Pro Ile Gly Leu Val Arg Asp Gly Asp
        515                 520                 525

Glu Ile Ile Ile Asp Ala Asp Asn Asn Lys Ile Asp Leu Leu Val Ser
    530                 535                 540

Asp Lys Glu Met Ala Gln Arg Lys Gln Ser Trp Val Ala Pro Pro Pro
545                 550                 555                 560

Arg Tyr Thr Arg Gly Thr Leu Ser Lys Tyr Ala Lys Leu Val Ser Asn
                565                 570                 575

Ala Ser Asn Gly Cys Val Leu Asp Ala
            580                 585

<210>  187
<211>  1653
<212>  DNA
<213>  Methanococcus maripaludis

<400>  187
atgataagtg ataacgtcaa aaagggagtt ataagaactc caaaccgagc tcttttaaag      60

gcttgcggat atacagacga agacatggaa aaaccattta ttggaattgt aaacagcttt     120

acagaagttg ttcccggcca cattcactta agaacattat cagaagcggc taaacatggt     180

gtttatgcaa acggtggaac accatttgaa tttaatacca ttggaatttg cgacggtatt     240

gcaatgggcc acgaaggtat gaaatactct ttaccttcaa gagaaattat tgcagacgct     300

gttgaatcaa tggcaagagc acatggattt gatggtcttg ttttaattcc tacgtgtgat     360

aaaatcgttc ctggaatgat aatgggtgct ttaagactaa acattccatt tattgtagtt     420

actggaggac caatgcttcc cggagaattc caaggtaaaa aatacgaact tatcagcctt     480

tttgaaggtg tcggagaata ccaagttgga aaaattactg aagaagagtt aaagtgcatt 540

gaagactgtg catgttcagg tgctggaagt tgtgcagggc tttacactgc aaacagtatg 600

gcctgcctta cagaagcttt gggactctct cttccaatgt gtgcaacaac gcatgcagtt 660

gatgcccaaa aagttaggct tgctaaaaaa agtggctcaa aaattgttga tatggtaaaa 720

gaagacctaa aaccaacaga catattaaca aaagaagctt ttgaaaatgc tattttagtt 780

gaccttgcac ttggtggatc aacaaacaca acattacaca ttcctgcaat tgcaaatgaa 840

attgaaaata aattcataac tctcgatgac tttgacaggt taagcgatga agttccacac 900

attgcatcaa tcaaaccagg tggagaacac tacatgattg atttacacaa tgctggaggt 960

attcctgcgg tattgaacgt tttaaaagaa aaaattagag atacaaaaac agttgatgga 1020

agaagcattt tggaaatcgc agaatctgtt aaatacataa attacgacgt tataagaaaa 1080

gtggaagctc cggttcacga aactgctggt ttaagggttt taaagggaaa tcttgctcca 1140

aacggttgcg ttgtaaaaat cggtgcagta catccgaaaa tgtacaaaca cgatggacct 1200

gcaaaagttt acaattccga agatgaagca atttctgcga tacttggcgg aaaaattgta 1260

gaaggggacg ttatagtaat cagatacgaa ggaccatcag gaggccctgg aatgagagaa 1320

atgctctccc caacttcagc aatctgtgga atgggtcttg atgacagcgt tgcattgatt 1380

actgatggaa gattcagtgg tggaagtagg ggcccatgta tcggacacgt ttctccagaa 1440

gctgcagctg gcggagtaat tgctgcaatt gaaaacgggg atatcatcaa aatcgacatg 1500

attgaaaaag aaataaatgt tgatttagat gaatcagtca ttaaagaaag actctcaaaa 1560

ctgggagaat ttgagcctaa aatcaaaaaa ggctatttat caagatactc aaaacttgtc 1620

tcatctgctg acgaaggggc agttttaaaa taa 1653

<210> 188
<211> 550
<212> PRT
<213> Methanococcus maripaludis

<400> 188

Met Ile Ser Asp Asn Val Lys Lys Gly Val Ile Arg Thr Pro Asn Arg
1               5                   10                  15

Ala Leu Leu Lys Ala Cys Gly Tyr Thr Asp Glu Asp Met Glu Lys Pro
            20                  25                  30

Phe Ile Gly Ile Val Asn Ser Phe Thr Glu Val Val Pro Gly His Ile
        35                  40                  45

His Leu Arg Thr Leu Ser Glu Ala Ala Lys His Gly Val Tyr Ala Asn
    50                  55                  60

Gly Gly Thr Pro Phe Glu Phe Asn Thr Ile Gly Ile Cys Asp Gly Ile
65                  70                  75                  80

Ala Met Gly His Glu Gly Met Lys Tyr Ser Leu Pro Ser Arg Glu Ile
                85                  90                  95

Ile Ala Asp Ala Val Glu Ser Met Ala Arg Ala His Gly Phe Asp Gly
            100                 105                 110

Leu Val Leu Ile Pro Thr Cys Asp Lys Ile Val Pro Gly Met Ile Met
        115                 120                 125

Gly Ala Leu Arg Leu Asn Ile Pro Phe Ile Val Val Thr Gly Gly Pro
    130                 135                 140

Met Leu Pro Gly Glu Phe Gln Gly Lys Lys Tyr Glu Leu Ile Ser Leu
145                 150                 155                 160

Phe Glu Gly Val Gly Glu Tyr Gln Val Gly Lys Ile Thr Glu Glu Glu
                165                 170                 175

Leu Lys Cys Ile Glu Asp Cys Ala Cys Ser Gly Ala Gly Ser Cys Ala
            180                 185                 190

Gly Leu Tyr Thr Ala Asn Ser Met Ala Cys Leu Thr Glu Ala Leu Gly
        195                 200                 205

Leu Ser Leu Pro Met Cys Ala Thr Thr His Ala Val Asp Ala Gln Lys
    210                 215                 220

Val Arg Leu Ala Lys Lys Ser Gly Ser Lys Ile Val Asp Met Val Lys
225                 230                 235                 240

Glu Asp Leu Lys Pro Thr Asp Ile Leu Thr Lys Glu Ala Phe Glu Asn
                245                 250                 255

Ala Ile Leu Val Asp Leu Ala Leu Gly Gly Ser Thr Asn Thr Thr Leu
            260                 265                 270

His Ile Pro Ala Ile Ala Asn Glu Ile Glu Asn Lys Phe Ile Thr Leu
        275                 280                 285

Asp Asp Phe Asp Arg Leu Ser Asp Glu Val Pro His Ile Ala Ser Ile
    290                 295                 300

Lys Pro Gly Gly Glu His Tyr Met Ile Asp Leu His Asn Ala Gly Gly
305                 310                 315                 320

Ile Pro Ala Val Leu Asn Val Leu Lys Glu Lys Ile Arg Asp Thr Lys

325                 330                 335

Thr Val Asp Gly Arg Ser Ile Leu Glu Ile Ala Glu Ser Val Lys Tyr
            340                 345                 350

Ile Asn Tyr Asp Val Ile Arg Lys Val Glu Ala Pro Val His Glu Thr
        355                 360                 365

Ala Gly Leu Arg Val Leu Lys Gly Asn Leu Ala Pro Asn Gly Cys Val
    370                 375                 380

Val Lys Ile Gly Ala Val His Pro Lys Met Tyr Lys His Asp Gly Pro
385                 390                 395                 400

Ala Lys Val Tyr Asn Ser Glu Asp Glu Ala Ile Ser Ala Ile Leu Gly
                405                 410                 415

Gly Lys Ile Val Glu Gly Asp Val Ile Val Ile Arg Tyr Glu Gly Pro
            420                 425                 430

Ser Gly Gly Pro Gly Met Arg Glu Met Leu Ser Pro Thr Ser Ala Ile
        435                 440                 445

Cys Gly Met Gly Leu Asp Asp Ser Val Ala Leu Ile Thr Asp Gly Arg
    450                 455                 460

Phe Ser Gly Gly Ser Arg Gly Pro Cys Ile Gly His Val Ser Pro Glu
465                 470                 475                 480

Ala Ala Ala Gly Gly Val Ile Ala Ala Ile Glu Asn Gly Asp Ile Ile
                485                 490                 495

Lys Ile Asp Met Ile Glu Lys Glu Ile Asn Val Asp Leu Asp Glu Ser
            500                 505                 510

Val Ile Lys Glu Arg Leu Ser Lys Leu Gly Glu Phe Glu Pro Lys Ile
        515                 520                 525

Lys Lys Gly Tyr Leu Ser Arg Tyr Ser Lys Leu Val Ser Ser Ala Asp
    530                 535                 540

Glu Gly Ala Val Leu Lys
545                 550

<210>  189
<211>  1677
<212>  DNA
<213>  Bacillus subtilis

<400>  189

atggcagaat tacgcagtaa tatgatcaca caaggaatcg atagagctcc gcaccgcagt 60

ttgcttcgtg cagcaggggt aaaagaagag gatttcggca agccgtttat tgcggtgtgt 120

aattcataca ttgatatcgt tcccggtcat gttcacttgc aggagtttgg gaaaatcgta 180

aaagaagcaa tcagagaagc agggggcgtt ccgtttgaat ttaataccat tggggtagat 240

gatggcatcg caatggggca tatcggtatg agatattcgc tgccaagccg tgaaattatc 300

gcagactctg tggaaacggt tgtatccgca cactggtttg acggaatggt ctgtattccg 360

aactgcgaca aaatcacacc gggaatgctt atggcggcaa tgcgcatcaa cattccgacg 420

atttttgtca gcggcggacc gatggcggca ggaagaacaa gttacgggcg aaaaatctcc 480

ctttcctcag tattcgaagg ggtaggcgcc taccaagcag ggaaaatcaa cgaaaacgag 540

cttcaagaac tagagcagtt cggatgccca acgtgcgggt cttgctcagg catgtttacg 600

gcgaactcaa tgaactgtct gtcagaagca cttggtcttg ctttgccggg taatggaacc 660

attctggcaa catctccgga acgcaaagag tttgtgagaa aatcggctgc gcaattaatg 720

gaaacgattc gcaaagatat caaaccgcgt gatattgtta cagtaaaagc gattgataac 780

gcgtttgcac tcgatatggc gctcggaggt tctacaaata ccgttcttca tacccttgcc 840

cttgcaaacg aagccggcgt tgaatactct ttagaacgca ttaacgaagt cgctgagcgc 900

gtgccgcact tggctaagct ggcgcctgca tcggatgtgt ttattgaaga tcttcacgaa 960

gcgggcggcg tttcagcggc tctgaatgag ctttcgaaga aagaaggagc gcttcattta 1020

gatgcgctga ctgttacagg aaaaactctt ggagaaacca ttgccggaca tgaagtaaag 1080

gattatgacg tcattcaccc gctggatcaa ccattcactg aaaagggagg ccttgctgtt 1140

ttattcggta atctagctcc ggacggcgct atcattaaaa caggcggcgt acagaatggg 1200

attacaagac acgaaggccc ggctgtcgta ttcgattctc aggacgaggc gcttgacggc 1260

attatcaacc gaaaagtaaa agaaggcgac gttgtcatca tcagatacga agggccaaaa 1320

ggcggacctg gcatgccgga aatgctggcg ccaacatccc aaatcgttgg aatgggactc 1380

gggccaaaag tggcattgat tacggacgga cgtttttccg gagcctcccg tggcctctca 1440

atcggccacg tatcacctga ggccgctgag ggcgggccgc ttgcctttgt tgaaaacgga 1500

gaccatatta tcgttgatat tgaaaaacgc atcttggatg tacaagtgcc agaagaagag 1560

tgggaaaaac gaaaagcgaa ctggaaaggt tttgaaccga aagtgaaaac cggctacctg 1620

gcacgttatt ctaaacttgt gacaagtgcc aacaccggcg gtattatgaa aatctag 1677

<210> 190
<211> 558
<212> PRT
<213> Bacillus subtilis

<400> 190

Met Ala Glu Leu Arg Ser Asn Met Ile Thr Gln Gly Ile Asp Arg Ala

1               5                   10                  15

Pro His Arg Ser Leu Leu Arg Ala Ala Gly Val Lys Glu Glu Asp Phe
            20                  25                  30

Gly Lys Pro Phe Ile Ala Val Cys Asn Ser Tyr Ile Asp Ile Val Pro
        35                  40                  45

Gly His Val His Leu Gln Glu Phe Gly Lys Ile Val Lys Glu Ala Ile
    50                  55                  60

Arg Glu Ala Gly Gly Val Pro Phe Glu Phe Asn Thr Ile Gly Val Asp
65                  70                  75                  80

Asp Gly Ile Ala Met Gly His Ile Gly Met Arg Tyr Ser Leu Pro Ser
                85                  90                  95

Arg Glu Ile Ile Ala Asp Ser Val Glu Thr Val Val Ser Ala His Trp
            100                 105                 110

Phe Asp Gly Met Val Cys Ile Pro Asn Cys Asp Lys Ile Thr Pro Gly
        115                 120                 125

Met Leu Met Ala Ala Met Arg Ile Asn Ile Pro Thr Ile Phe Val Ser
    130                 135                 140

Gly Gly Pro Met Ala Ala Gly Arg Thr Ser Tyr Gly Arg Lys Ile Ser
145                 150                 155                 160

Leu Ser Ser Val Phe Glu Gly Val Gly Ala Tyr Gln Ala Gly Lys Ile
                165                 170                 175

Asn Glu Asn Glu Leu Gln Glu Leu Glu Gln Phe Gly Cys Pro Thr Cys
            180                 185                 190

Gly Ser Cys Ser Gly Met Phe Thr Ala Asn Ser Met Asn Cys Leu Ser
        195                 200                 205

Glu Ala Leu Gly Leu Ala Leu Pro Gly Asn Gly Thr Ile Leu Ala Thr
    210                 215                 220

Ser Pro Glu Arg Lys Glu Phe Val Arg Lys Ser Ala Ala Gln Leu Met
225                 230                 235                 240

Glu Thr Ile Arg Lys Asp Ile Lys Pro Arg Asp Ile Val Thr Val Lys
                245                 250                 255

Ala Ile Asp Asn Ala Phe Ala Leu Asp Met Ala Leu Gly Gly Ser Thr
            260                 265                 270

Asn Thr Val Leu His Thr Leu Ala Leu Ala Asn Glu Ala Gly Val Glu
        275                 280                 285

Tyr Ser Leu Glu Arg Ile Asn Glu Val Ala Glu Arg Val Pro His Leu
    290                 295                 300

Ala Lys Leu Ala Pro Ala Ser Asp Val Phe Ile Glu Asp Leu His Glu
305                 310                 315                 320

Ala Gly Gly Val Ser Ala Ala Leu Asn Glu Leu Ser Lys Lys Glu Gly
                325                 330                 335

Ala Leu His Leu Asp Ala Leu Thr Val Thr Gly Lys Thr Leu Gly Glu
            340                 345                 350

Thr Ile Ala Gly His Glu Val Lys Asp Tyr Asp Val Ile His Pro Leu
        355                 360                 365

Asp Gln Pro Phe Thr Glu Lys Gly Gly Leu Ala Val Leu Phe Gly Asn
    370                 375                 380

Leu Ala Pro Asp Gly Ala Ile Ile Lys Thr Gly Gly Val Gln Asn Gly
385                 390                 395                 400

Ile Thr Arg His Glu Gly Pro Ala Val Val Phe Asp Ser Gln Asp Glu
                405                 410                 415

Ala Leu Asp Gly Ile Ile Asn Arg Lys Val Lys Glu Gly Asp Val Val
            420                 425                 430

Ile Ile Arg Tyr Glu Gly Pro Lys Gly Gly Pro Gly Met Pro Glu Met
        435                 440                 445

Leu Ala Pro Thr Ser Gln Ile Val Gly Met Gly Leu Gly Pro Lys Val
    450                 455                 460

Ala Leu Ile Thr Asp Gly Arg Phe Ser Gly Ala Ser Arg Gly Leu Ser
465                 470                 475                 480

Ile Gly His Val Ser Pro Glu Ala Ala Glu Gly Gly Pro Leu Ala Phe
                485                 490                 495

Val Glu Asn Gly Asp His Ile Ile Val Asp Ile Glu Lys Arg Ile Leu
            500                 505                 510

Asp Val Gln Val Pro Glu Glu Glu Trp Glu Lys Arg Lys Ala Asn Trp
        515                 520                 525

Lys Gly Phe Glu Pro Lys Val Lys Thr Gly Tyr Leu Ala Arg Tyr Ser
    530                 535                 540

Lys Leu Val Thr Ser Ala Asn Thr Gly Gly Ile Met Lys Ile
545                 550                 555

<210>  191
<211>  1647
<212>  DNA
<213>  Lactococcus lactis

<400>  191
atgtatacag taggagatta cctattagac cgattacacg agttaggaat tgaagaaatt      60

tttggagtcc ctggagacta taacttacaa tttttagatc aaattatttc ccacaaggat     120

atgaaatggg tcggaaatgc taatgaatta aatgcttcat atatggctga tggctatgct     180

cgtactaaaa aagctgccgc atttcttaca acctttggag taggtgaatt gagtgcagtt     240

aatggattag caggaagtta cgccgaaaat ttaccagtag tagaaatagt gggatcacct     300

acatcaaaag ttcaaaatga aggaaaattt gttcatcata cgctggctga cggtgatttt     360

aaacacttta tgaaaatgca cgaacctgtt acagcagctc gaactttact gacagcagaa     420

aatgcaaccg ttgaaattga ccgagtactt tctgcactat taaaagaaag aaaacctgtc     480

tatatcaact taccagttga tgttgctgct gcaaaagcag agaaaccctc actccctttg     540

aaaaaggaaa actcaacttc aaatacaagt gaccaagaaa ttttgaacaa aattcaagaa     600

agcttgaaaa atgccaaaaa accaatcgtg attacaggac atgaaataat tagttttggc     660

ttagaaaaaa cagtcactca atttatttca aagacaaaac tacctattac gacattaaac     720

tttggtaaaa gttcagttga tgaagccctc ccttcatttt taggaatcta taatggtaca     780

ctctcagagc ctaatcttaa agaattcgtg gaatcagccg acttcatctt gatgcttgga     840

gttaaactca cagactcttc aacaggagcc ttcactcatc atttaaatga aaataaaatg     900

atttcactga atatagatga aggaaaaata tttaacgaaa gaatccaaaa ttttgatttt     960

gaatccctca tctcctctct cttagaccta agcgaaatag aatacaaagg aaaatatatc    1020

gataaaaagc aagaagactt tgttccatca aatgcgcttt tatcacaaga ccgcctatgg    1080

caagcagttg aaaacctaac tcaaagcaat gaaacaatcg ttgctgaaca agggacatca    1140

ttctttggcg cttcatcaat ttttcttaaa tcaaagagtc attttattgg tcaaccctta    1200

tggggatcaa ttggatatac attcccagca gcattaggaa gccaaattgc agataaagaa    1260

agcagacacc ttttatttat tggtgatggt tcacttcaac ttacagtgca agaattagga    1320

ttagcaatca gagaaaaaat taatccaatt tgctttatta tcaataatga tggttataca    1380

gtcgaaagag aaattcatgg accaaatcaa agctacaatg atattccaat gtggaattac    1440

tcaaaattac cagaatcgtt tggagcaaca gaagatcgag tagtctcaaa aatcgttaga    1500

actgaaaatg aatttgtgtc tgtcatgaaa gaagctcaag cagatccaaa tagaatgtac 1560

tggattgagt taattttggc aaaagaaggt gcaccaaaag tactgaaaaa aatgggcaaa 1620

ctatttgctg aacaaaataa atcataa 1647

<210> 192
<211> 1644
<212> DNA
<213> Lactococcus lactis

<400> 192
atgtatacag taggagatta cctgttagac cgattacacg agttgggaat tgaagaaatt 60

tttggagttc ctggtgacta taacttacaa tttttagatc aaattatttc acgcgaagat 120

atgaaatgga ttggaaatgc taatgaatta aatgcttctt atatggctga tggttatgct 180

cgtactaaaa aagctgccgc atttctcacc acatttggag tcggcgaatt gagtgcgatc 240

aatggactgg caggaagtta tgccgaaaat ttaccagtag tagaaattgt tggttcacca 300

acttcaaaag tacaaaatga cggaaaattt gtccatcata cactagcaga tggtgatttt 360

aaacacttta tgaagatgca tgaacctgtt acagcagcgc ggactttact gacagcagaa 420

aatgccacat atgaaattga ccgagtactt tctcaattac taaaagaaag aaaaccagtc 480

tatattaact taccagtcga tgttgctgca gcaaaagcag agaagcctgc attatcttta 540

gaaaaagaaa gctctacaac aaatacaact gaacaagtga ttttgagtaa gattgaagaa 600

agtttgaaaa atgcccaaaa accagtagtg attgcaggac acgaagtaat tagttttggt 660

ttagaaaaaa cggtaactca gtttgtttca gaaacaaaac taccgattac gacactaaat 720

tttggtaaaa gtgctgttga tgaatctttg ccctcatttt taggaatata taacggaaaa 780

ctttcagaaa tcagtcttaa aaattttgtg gagtccgcag actttatcct aatgcttgga 840

gtgaagctta cggactcctc aacaggtgca ttcacacatc atttagatga aaataaaatg 900

atttcactaa acatagatga aggaataatt ttcaataaag tggtagaaga ttttgatttt 960

agagcagtgg tttcttcttt atcagaatta aaaggaatag aatatgaagg acaatatatt 1020

gataagcaat atgaagaatt tattccatca agtgctccct tatcacaaga ccgtctatgg 1080

caggcagttg aaagtttgac tcaaagcaat gaaacaatcg ttgctgaaca aggaacctca 1140

ttttttggag cttcaacaat tttcttaaaa tcaaatagtc gttttattgg acaaccttta 1200

tggggttcta ttggatatac ttttccagcg gctttaggaa gccaaattgc ggataaagag 1260

agcagacacc ttttatttat tggtgatggt tcacttcaac ttaccgtaca agaattagga 1320

ctatcaatca gagaaaaact caatccaatt tgttttatca taaataatga tggttataca 1380

gttgaaagag aaatccacgg acctactcaa agttataacg acattccaat gtggaattac 1440

tcgaaattac cagaaacatt tggagcaaca gaagatcgtg tagtatcaaa aattgttaga 1500

acagagaatg aatttgtgtc tgtcatgaaa gaagcccaag cagatgtcaa tagaatgtat 1560

tggatagaac tagttttgga aaaagaagat gcgccaaaat tactgaaaaa aatgggtaaa 1620

ttatttgctg agcaaaataa atag 1644

<210> 193
<211> 547
<212> PRT
<213> Lactococcus lactis

<400> 193

Met Tyr Thr Val Gly Asp Tyr Leu Leu Asp Arg Leu His Glu Leu Gly
1               5                   10                  15

Ile Glu Glu Ile Phe Gly Val Pro Gly Asp Tyr Asn Leu Gln Phe Leu
            20                  25                  30

Asp Gln Ile Ile Ser Arg Glu Asp Met Lys Trp Ile Gly Asn Ala Asn
        35                  40                  45

Glu Leu Asn Ala Ser Tyr Met Ala Asp Gly Tyr Ala Arg Thr Lys Lys
    50                  55                  60

Ala Ala Ala Phe Leu Thr Thr Phe Gly Val Gly Glu Leu Ser Ala Ile
65                  70                  75                  80

Asn Gly Leu Ala Gly Ser Tyr Ala Glu Asn Leu Pro Val Val Glu Ile
                85                  90                  95

Val Gly Ser Pro Thr Ser Lys Val Gln Asn Asp Gly Lys Phe Val His
            100                 105                 110

His Thr Leu Ala Asp Gly Asp Phe Lys His Phe Met Lys Met His Glu
        115                 120                 125

Pro Val Thr Ala Ala Arg Thr Leu Leu Thr Ala Glu Asn Ala Thr Tyr
    130                 135                 140

Glu Ile Asp Arg Val Leu Ser Gln Leu Leu Lys Glu Arg Lys Pro Val
145                 150                 155                 160

Tyr Ile Asn Leu Pro Val Asp Val Ala Ala Ala Lys Ala Glu Lys Pro
                165                 170                 175

Ala Leu Ser Leu Glu Lys Glu Ser Ser Thr Thr Asn Thr Thr Glu Gln
            180                 185                 190

Val Ile Leu Ser Lys Ile Glu Glu Ser Leu Lys Asn Ala Gln Lys Pro
        195                 200                 205

Val Val Ile Ala Gly His Glu Val Ile Ser Phe Gly Leu Glu Lys Thr

210                 215                 220

Val Thr Gln Phe Val Ser Glu Thr Lys Leu Pro Ile Thr Thr Leu Asn
225                 230                 235                 240

Phe Gly Lys Ser Ala Val Asp Glu Ser Leu Pro Ser Phe Leu Gly Ile
                245                 250                 255

Tyr Asn Gly Lys Leu Ser Glu Ile Ser Leu Lys Asn Phe Val Glu Ser
            260                 265                 270

Ala Asp Phe Ile Leu Met Leu Gly Val Lys Leu Thr Asp Ser Ser Thr
        275                 280                 285

Gly Ala Phe Thr His His Leu Asp Glu Asn Lys Met Ile Ser Leu Asn
    290                 295                 300

Ile Asp Glu Gly Ile Ile Phe Asn Lys Val Val Glu Asp Phe Asp Phe
305                 310                 315                 320

Arg Ala Val Val Ser Ser Leu Ser Glu Leu Lys Gly Ile Glu Tyr Glu
                325                 330                 335

Gly Gln Tyr Ile Asp Lys Gln Tyr Glu Glu Phe Ile Pro Ser Ser Ala
            340                 345                 350

Pro Leu Ser Gln Asp Arg Leu Trp Gln Ala Val Glu Ser Leu Thr Gln
        355                 360                 365

Ser Asn Glu Thr Ile Val Ala Glu Gln Gly Thr Ser Phe Phe Gly Ala
    370                 375                 380

Ser Thr Ile Phe Leu Lys Ser Asn Ser Arg Phe Ile Gly Gln Pro Leu
385                 390                 395                 400

Trp Gly Ser Ile Gly Tyr Thr Phe Pro Ala Ala Leu Gly Ser Gln Ile
                405                 410                 415

Ala Asp Lys Glu Ser Arg His Leu Leu Phe Ile Gly Asp Gly Ser Leu
            420                 425                 430

Gln Leu Thr Val Gln Glu Leu Gly Leu Ser Ile Arg Glu Lys Leu Asn
        435                 440                 445

Pro Ile Cys Phe Ile Ile Asn Asn Asp Gly Tyr Thr Val Glu Arg Glu
    450                 455                 460

Ile His Gly Pro Thr Gln Ser Tyr Asn Asp Ile Pro Met Trp Asn Tyr
465                 470                 475                 480

Ser Lys Leu Pro Glu Thr Phe Gly Ala Thr Glu Asp Arg Val Val Ser
                485                 490                 495

Lys Ile Val Arg Thr Glu Asn Glu Phe Val Ser Val Met Lys Glu Ala
            500                 505                 510

Gln Ala Asp Val Asn Arg Met Tyr Trp Ile Glu Leu Val Leu Glu Lys
        515                 520                 525

Glu Asp Ala Pro Lys Leu Leu Lys Lys Met Gly Lys Leu Phe Ala Glu
    530                 535                 540

Gln Asn Lys
545

<210>  194
<211>  1653
<212>  DNA
<213>  Salmonella typhimurium

<400>  194
ttatcccccg ttgcgggctt ccagcgcccg ggtcacggta cgcagtaatt ccggcagatc      60

ggcttttggc aacatcactt caataaatga cagacgttgt gggcgcgcca accgttcgag     120

gacctctgcc agttggatag cctgcgtcac ccgccagcac tccgcctgtt gcgccgcgtt     180

tagcgccggt ggtatctgcg tccagttcca gctcgcgatg tcgttatacc gctgggccgc     240

gccgtgaatg gcgcgctcta cggtatagcc gtcattgttg agcagcagga tgaccggcgc     300

ctgcccgtcg cgtaacatcg agcccatctc ctgaatcgtg agctgcgccg cgccatcgcc     360

gataatcaga atcacccgcc gatcgggaca ggcggtttgc gcgccaaacg cggcgggcaa     420

ggaatagccg atagaccccc acagcggctg taacacaact tccgcgccgt caggaagcga     480

cagcgcggca gcgccaaaag ctgctgtccc ctggtcgaca aggataatat ctccgggttt     540

gagatactgc tgtaaggttt gccagaagct ttcctgggtc agttctcctt tatcaatccg     600

cactggctgt ccggcggaac gcgtcggcgg cggcgcaaaa gcgcattcca ggcacagttc     660

gcgcagcgta gacaccgcct gcgccatcgg gaggttgaac caggtttcgc cgatgcgcga     720

cgcgtaaggc tgaatctcca gcgtgcgttc cgccggtaat tgttgggtaa atccggccgt     780

aagggtatcg acaaaacggg tgccgacgca gataacccta tcggcgtcct ctatggcctg     840

acgcacttct ttgctgctgg cgccagcgct ataggtgcca acgaagttcg ggtgctgttc     900

atcaaaaagc cccttcccca tcagtagtgt cgcatgagcg atgggcgttt ccgccatcca     960

gcgctgcaac agtggtcgta aaccaaaacg cccggcaaga aagtcggcca atagcgcaat    1020

gcgccgactg ttcatcaggc actgacgggc gtgataacga aaggccgtct ccacgccgct    1080

ttgcgcttca tgcacgggca acgccagcgc ctgcgtaggt gggatggccg tttttttcgc    1140

cacatcggcg ggcaacatga tgtatcctgg cctgcgtgcg gcaagcattt cacccaacac 1200

gcggtcaatc tcgaaacagg cgttctgttc atctaatatt gcgctggcag cggatatcgc 1260

ctgactcatg cgataaaaat gacgaaaatc gccgtcaccg agggtatggt gcatcaattc 1320

gccacgctgc tgcgcagcgc tacagggcgc gccgacgata tgcaagaccg ggacatattc 1380

cgcgtaactg cccgcgatac cgttaatagc gctaagttct cccacgccaa aggtggtgag 1440

tagcgctcca gcgcccgaca tgcgcgcata gccgtccgcg gcataagcgg cgttcagctc 1500

attggcgcat cccacccaac gcagggtcgg gtggtcaatc acatggtcaa gaaactgcaa 1560

gttataatcg cccggtacgc caaaaagatg gccaatgccg catcctgcca gtctgtccag 1620

caaatagtcg gccacggtat aggggttttg cat 1653

<210> 195
<211> 550
<212> PRT
<213> Salmonella typhimurium

<400> 195

Met Gln Asn Pro Tyr Thr Val Ala Asp Tyr Leu Leu Asp Arg Leu Ala
1               5                   10                  15

Gly Cys Gly Ile Gly His Leu Phe Gly Val Pro Gly Asp Tyr Asn Leu
            20                  25                  30

Gln Phe Leu Asp His Val Ile Asp His Pro Thr Leu Arg Trp Val Gly
        35                  40                  45

Cys Ala Asn Glu Leu Asn Ala Ala Tyr Ala Ala Asp Gly Tyr Ala Arg
    50                  55                  60

Met Ser Gly Ala Gly Ala Leu Leu Thr Thr Phe Gly Val Gly Glu Leu
65                  70                  75                  80

Ser Ala Ile Asn Gly Ile Ala Gly Ser Tyr Ala Glu Tyr Val Pro Val
                85                  90                  95

Leu His Ile Val Gly Ala Pro Cys Ser Ala Ala Gln Gln Arg Gly Glu
            100                 105                 110

Leu Met His His Thr Leu Gly Asp Gly Asp Phe Arg His Phe Tyr Arg
        115                 120                 125

Met Ser Gln Ala Ile Ser Ala Ala Ser Ala Ile Leu Asp Glu Gln Asn
    130                 135                 140

Ala Cys Phe Glu Ile Asp Arg Val Leu Gly Glu Met Leu Ala Ala Arg
145                 150                 155                 160

Arg Pro Gly Tyr Ile Met Leu Pro Ala Asp Val Ala Lys Lys Thr Ala
                165                 170                 175

Ile Pro Pro Thr Gln Ala Leu Ala Leu Pro Val His Glu Ala Gln Ser
            180                 185                 190

Gly Val Glu Thr Ala Phe Arg Tyr His Ala Arg Gln Cys Leu Met Asn
        195                 200                 205

Ser Arg Arg Ile Ala Leu Leu Ala Asp Phe Leu Ala Gly Arg Phe Gly
    210                 215                 220

Leu Arg Pro Leu Leu Gln Arg Trp Met Ala Glu Thr Pro Ile Ala His
225                 230                 235                 240

Ala Thr Leu Leu Met Gly Lys Gly Leu Phe Asp Glu Gln His Pro Asn
                245                 250                 255

Phe Val Gly Thr Tyr Ser Ala Gly Ala Ser Ser Lys Glu Val Arg Gln
            260                 265                 270

Ala Ile Glu Asp Ala Asp Arg Val Ile Cys Val Gly Thr Arg Phe Val
        275                 280                 285

Asp Thr Leu Thr Ala Gly Phe Thr Gln Gln Leu Pro Ala Glu Arg Thr
    290                 295                 300

Leu Glu Ile Gln Pro Tyr Ala Ser Arg Ile Gly Glu Thr Trp Phe Asn
305                 310                 315                 320

Leu Pro Met Ala Gln Ala Val Ser Thr Leu Arg Glu Leu Cys Leu Glu
                325                 330                 335

Cys Ala Phe Ala Pro Pro Pro Thr Arg Ser Ala Gly Gln Pro Val Arg
            340                 345                 350

Ile Asp Lys Gly Glu Leu Thr Gln Glu Ser Phe Trp Gln Thr Leu Gln
        355                 360                 365

Gln Tyr Leu Lys Pro Gly Asp Ile Ile Leu Val Asp Gln Gly Thr Ala
    370                 375                 380

Ala Phe Gly Ala Ala Ala Leu Ser Leu Pro Asp Gly Ala Glu Val Val
385                 390                 395                 400

Leu Gln Pro Leu Trp Gly Ser Ile Gly Tyr Ser Leu Pro Ala Ala Phe
                405                 410                 415

Gly Ala Gln Thr Ala Cys Pro Asp Arg Arg Val Ile Leu Ile Ile Gly
            420                 425                 430

Asp Gly Ala Ala Gln Leu Thr Ile Gln Glu Met Gly Ser Met Leu Arg
        435                 440                 445

Asp Gly Gln Ala Pro Val Ile Leu Leu Leu Asn Asn Asp Gly Tyr Thr
    450                 455                 460

Val Glu Arg Ala Ile His Gly Ala Ala Gln Arg Tyr Asn Asp Ile Ala
465                 470                 475                 480

Ser Trp Asn Trp Thr Gln Ile Pro Pro Ala Leu Asn Ala Ala Gln Gln
                485                 490                 495

Ala Glu Cys Trp Arg Val Thr Gln Ala Ile Gln Leu Ala Glu Val Leu
            500                 505                 510

Glu Arg Leu Ala Arg Pro Gln Arg Leu Ser Phe Ile Glu Val Met Leu
        515                 520                 525

Pro Lys Ala Asp Leu Pro Glu Leu Leu Arg Thr Val Thr Arg Ala Leu
    530                 535                 540

Glu Ala Arg Asn Gly Gly
545                 550

<210>  196
<211>  1665
<212>  DNA
<213>  Clostridium acetobutylicum

<400>  196
ttgaagagtg aatacacaat tggaagatat ttgttagacc gtttatcaga gttgggtatt      60

cggcatatct ttggtgtacc tggagattac aatctatcct ttttagacta tataatggag     120

tacaaaggga tagattgggt tggaaattgc aatgaattga atgctgggta tgctgctgat     180

ggatatgcaa gaataaatgg aattggagcc atacttacaa catttggtgt tggagaatta     240

agtgccatta acgcaattgc tggggcatac gctgagcaag ttccagttgt taaaattaca     300

ggtatcccca cagcaaaagt tagggacaat ggattatatg tacaccacac attaggtgac     360

ggaaggtttg atcacttttt tgaaatgttt agagaagtaa cagttgctga ggcattacta     420

agcgaagaaa atgcagcaca agaaattgat cgtgttctta tttcatgctg gagacaaaaa     480

cgtcctgttc ttataaattt accgattgat gtatatgata aaccaattaa caaaccatta     540

aagccattac tcgattatac tatttcaagt aacaaagagg ctgcatgtga atttgttaca     600

gaaatagtac ctataataaa tagggcaaaa aagcctgtta ttcttgcaga ttatggagta     660

tatcgttacc aagttcaaca tgtgcttaaa aacttggccg aaaaaaccgg atttcctgtg 720

gctacactaa gtatgggaaa aggtgttttc aatgaagcac accctcaatt tattggtgtt 780

tataatggtg atgtaagttc tccttattta aggcagcgag ttgatgaagc agactgcatt 840

attagcgttg gtgtaaaatt gacggattca accacagggg gattttctca tggattttct 900

aaaaggaatg taattcacat tgatcctttt tcaataaagg caaaaggtaa aaaatatgca 960

cctattacga tgaaagatgc tttaacagaa ttaacaagta aaattgagca tagaaacttt 1020

gaggatttag atataaagcc ttacaaatca gataatcaaa agtattttgc aaaagagaag 1080

ccaattacac aaaaacgttt ttttgagcgt attgctcact ttataaaaga aaaagatgta 1140

ttattagcag aacagggtac atgctttttt ggtgcgtcaa ccatacaact acccaaagat 1200

gcaactttta ttggtcaacc tttatgggga tctattggat acacacttcc tgctttatta 1260

ggttcacaat tagctgatca aaaaaggcgt aatattcttt taattgggga tggtgcattt 1320

caaatgacag cacaagaaat ttcaacaatg cttcgtttac aaatcaaacc tattattttt 1380

ttaattaata acgatggtta tacaattgaa cgtgctattc atggtagaga acaagtatat 1440

aacaatattc aaatgtggcg atatcataat gttccaaagg ttttaggtcc taaagaatgc 1500

agcttaacct ttaaagtaca aagtgaaact gaacttgaaa aggctctttt agtggcagat 1560

aaggattgtg aacatttgat ttttatagaa gttgttatgg atcgttatga taaacccgag 1620

cctttagaac gtctttcgaa acgttttgca aatcaaaata attag 1665

<210> 197
<211> 554
<212> PRT
<213> Clostridium acetobutylicum

<400> 197

Met Lys Ser Glu Tyr Thr Ile Gly Arg Tyr Leu Leu Asp Arg Leu Ser
1               5                   10                  15

Glu Leu Gly Ile Arg His Ile Phe Gly Val Pro Gly Asp Tyr Asn Leu
            20                  25                  30

Ser Phe Leu Asp Tyr Ile Met Glu Tyr Lys Gly Ile Asp Trp Val Gly
        35                  40                  45

Asn Cys Asn Glu Leu Asn Ala Gly Tyr Ala Ala Asp Gly Tyr Ala Arg
    50                  55                  60

Ile Asn Gly Ile Gly Ala Ile Leu Thr Thr Phe Gly Val Gly Glu Leu
65                  70                  75                  80

Ser Ala Ile Asn Ala Ile Ala Gly Ala Tyr Ala Glu Gln Val Pro Val
                85                  90                  95

Val Lys Ile Thr Gly Ile Pro Thr Ala Lys Val Arg Asp Asn Gly Leu
        100                 105                 110

Tyr Val His His Thr Leu Gly Asp Gly Arg Phe Asp His Phe Phe Glu
        115                 120                 125

Met Phe Arg Glu Val Thr Val Ala Glu Ala Leu Leu Ser Glu Glu Asn
    130                 135                 140

Ala Ala Gln Glu Ile Asp Arg Val Leu Ile Ser Cys Trp Arg Gln Lys
145                 150                 155                 160

Arg Pro Val Leu Ile Asn Leu Pro Ile Asp Val Tyr Asp Lys Pro Ile
                165                 170                 175

Asn Lys Pro Leu Lys Pro Leu Leu Asp Tyr Thr Ile Ser Ser Asn Lys
            180                 185                 190

Glu Ala Ala Cys Glu Phe Val Thr Glu Ile Val Pro Ile Ile Asn Arg
        195                 200                 205

Ala Lys Lys Pro Val Ile Leu Ala Asp Tyr Gly Val Tyr Arg Tyr Gln
    210                 215                 220

Val Gln His Val Leu Lys Asn Leu Ala Glu Lys Thr Gly Phe Pro Val
225                 230                 235                 240

Ala Thr Leu Ser Met Gly Lys Gly Val Phe Asn Glu Ala His Pro Gln
                245                 250                 255

Phe Ile Gly Val Tyr Asn Gly Asp Val Ser Ser Pro Tyr Leu Arg Gln
            260                 265                 270

Arg Val Asp Glu Ala Asp Cys Ile Ile Ser Val Gly Val Lys Leu Thr
        275                 280                 285

Asp Ser Thr Thr Gly Gly Phe Ser His Gly Phe Ser Lys Arg Asn Val
    290                 295                 300

Ile His Ile Asp Pro Phe Ser Ile Lys Ala Lys Gly Lys Lys Tyr Ala
305                 310                 315                 320

Pro Ile Thr Met Lys Asp Ala Leu Thr Glu Leu Thr Ser Lys Ile Glu
                325                 330                 335

His Arg Asn Phe Glu Asp Leu Asp Ile Lys Pro Tyr Lys Ser Asp Asn
            340                 345                 350

Gln Lys Tyr Phe Ala Lys Glu Lys Pro Ile Thr Gln Lys Arg Phe Phe
        355                 360                 365

Glu Arg Ile Ala His Phe Ile Lys Glu Lys Asp Val Leu Leu Ala Glu
    370                 375                 380

Gln Gly Thr Cys Phe Phe Gly Ala Ser Thr Ile Gln Leu Pro Lys Asp
385                 390                 395                 400

Ala Thr Phe Ile Gly Gln Pro Leu Trp Gly Ser Ile Gly Tyr Thr Leu
                405                 410                 415

Pro Ala Leu Leu Gly Ser Gln Leu Ala Asp Gln Lys Arg Arg Asn Ile
            420                 425                 430

Leu Leu Ile Gly Asp Gly Ala Phe Gln Met Thr Ala Gln Glu Ile Ser
        435                 440                 445

Thr Met Leu Arg Leu Gln Ile Lys Pro Ile Ile Phe Leu Ile Asn Asn
    450                 455                 460

Asp Gly Tyr Thr Ile Glu Arg Ala Ile His Gly Arg Glu Gln Val Tyr
465                 470                 475                 480

Asn Asn Ile Gln Met Trp Arg Tyr His Asn Val Pro Lys Val Leu Gly
                485                 490                 495

Pro Lys Glu Cys Ser Leu Thr Phe Lys Val Gln Ser Glu Thr Glu Leu
            500                 505                 510

Glu Lys Ala Leu Leu Val Ala Asp Lys Asp Cys Glu His Leu Ile Phe
        515                 520                 525

Ile Glu Val Val Met Asp Arg Tyr Asp Lys Pro Glu Pro Leu Glu Arg
    530                 535                 540

Leu Ser Lys Arg Phe Ala Asn Gln Asn Asn
545                 550

<210> 198
<211> 939
<212> DNA
<213> Saccharomyces cerevisiae

<400> 198
atgcctgcta cgttaaagaa ttcttctgct acattaaaac taaatactgg tgcctccatt 60

ccagtgttgg gtttcggcac ttggcgttcc gttgacaata acggttacca ttctgtaatt 120

gcagctttga aagctggata cagacacatt gatgctgcgg ctatctattt gaatgaagaa 180

gaagttggca gggctattaa agattccgga gtccctcgtg aggaaatttt tattactact 240

aagctttggg gtacggaaca acgtgatccg gaagctgctc taaacaagtc tttgaaaaga 300

ctaggcttgg attatgttga cctatatctg atgcattggc cagtgccttt gaaaaccgac 360

agagttactg atggtaacgt tctgtgcatt ccaacattag aagatggcac tgttgacatc 420

gatactaagg aatggaattt tatcaagacg tgggagttga tgcaagagtt gccaaagacg 480

ggcaaaacta aagccgttgg tgtctctaat ttttctatta acaacattaa agaattatta 540

gaatctccaa ataacaaggt ggtaccagct actaatcaaa ttgaaattca tccattgcta 600

ccacaagacg aattgattgc cttttgtaag gaaaagggta ttgttgttga agcctactca 660

ccatttggga gtgctaatgc tcctttacta aaagagcaag caattattga tatggctaaa 720

aagcacggcg ttgagccagc acagcttatt atcagttgga gtattcaaag aggctacgtt 780

gttctggcca aatcggttaa tcctgaaaga attgtatcca attttaagat tttcactctg 840

cctgaggatg atttcaagac tattagtaac ctatccaaag tgcatggtac aaagagagtc 900

gttgatatga agtggggatc cttcccaatt ttccaatga 939

<210> 199
<211> 312
<212> PRT
<213> Saccharomyces cerevisiae

<400> 199

Met Pro Ala Thr Leu Lys Asn Ser Ser Ala Thr Leu Lys Leu Asn Thr
1               5                   10                  15

Gly Ala Ser Ile Pro Val Leu Gly Phe Gly Thr Trp Arg Ser Val Asp
            20                  25                  30

Asn Asn Gly Tyr His Ser Val Ile Ala Ala Leu Lys Ala Gly Tyr Arg
        35                  40                  45

His Ile Asp Ala Ala Ala Ile Tyr Leu Asn Glu Glu Glu Val Gly Arg
    50                  55                  60

Ala Ile Lys Asp Ser Gly Val Pro Arg Glu Glu Ile Phe Ile Thr Thr
65                  70                  75                  80

Lys Leu Trp Gly Thr Glu Gln Arg Asp Pro Glu Ala Ala Leu Asn Lys
                85                  90                  95

Ser Leu Lys Arg Leu Gly Leu Asp Tyr Val Asp Leu Tyr Leu Met His
            100                 105                 110

Trp Pro Val Pro Leu Lys Thr Asp Arg Val Thr Asp Gly Asn Val Leu
        115                 120                 125

Cys Ile Pro Thr Leu Glu Asp Gly Thr Val Asp Ile Asp Thr Lys Glu
    130                 135                 140

Trp Asn Phe Ile Lys Thr Trp Glu Leu Met Gln Glu Leu Pro Lys Thr
145                 150                 155                 160

Gly Lys Thr Lys Ala Val Gly Val Ser Asn Phe Ser Ile Asn Asn Ile
                165                 170                 175

Lys Glu Leu Leu Glu Ser Pro Asn Asn Lys Val Val Pro Ala Thr Asn
            180                 185                 190

Gln Ile Glu Ile His Pro Leu Leu Pro Gln Asp Glu Leu Ile Ala Phe
        195                 200                 205

Cys Lys Glu Lys Gly Ile Val Val Glu Ala Tyr Ser Pro Phe Gly Ser
    210                 215                 220

Ala Asn Ala Pro Leu Leu Lys Glu Gln Ala Ile Ile Asp Met Ala Lys
225                 230                 235                 240

Lys His Gly Val Glu Pro Ala Gln Leu Ile Ile Ser Trp Ser Ile Gln
                245                 250                 255

Arg Gly Tyr Val Val Leu Ala Lys Ser Val Asn Pro Glu Arg Ile Val
            260                 265                 270

Ser Asn Phe Lys Ile Phe Thr Leu Pro Glu Asp Asp Phe Lys Thr Ile
        275                 280                 285

Ser Asn Leu Ser Lys Val His Gly Thr Lys Arg Val Val Asp Met Lys
    290                 295                 300

Trp Gly Ser Phe Pro Ile Phe Gln
305                 310

<210>  200
<211>  1083
<212>  DNA
<213>  Saccharomyces cerevisiae

<400>  200
ctagtctgaa aattctttgt cgtagccgac taaggtaaat ctatatctaa cgtcaccctt      60

ttccatcctt tcgaaggctt catggacgcc ggcttcacca acaggtaatg tttccaccca     120

aattttgata tctttttcag agactaattt caagagttgg ttcaattctt tgatggaacc     180

taaagcactg taagaaatgg agacagcctt taagccatat ggctttagcg ataacatttc     240

gtgttgttct ggtatagaga ttgagacaat tctaccacca accttcatag cctttggcat     300

aatgttgaag tcaatgtcgg taagggagga agcacagact acaatcaggt cgaaggtgtc     360

aaagtacttt tcaccccaat caccttcttc taatgtagca atgtagtgat cggcgcccat 420

cttcattgca tcttctcttt ttctcgaaga acgagaaata acatacgtct ctgcccccat 480

ggctttggaa atcaatgtac ccatactgcc gataccacca agaccaacta taccaacttt 540

tttacctgga ccgcaaccgt tacgaaccaa tggagagtac acagtcaaac caccacataa 600

tagtggagca gccaaatgtg atggaatatt ctctgggata ggcaccacaa aatgttcatg 660

aactctgacg tagtttgcat agccaccctg cgacacatag ccgtcttcat aaggctgact 720

gtatgtggta acaaacttgg tgcagtatgg ttcattatca ttcttacaac ggtcacattc 780

caagcatgaa aagacttgag cacctacacc aacacgttga ccgactttca acccactgtt 840

tgacttgggc cctagcttga caactttacc aacgatttca tgaccaacga ctagcggcat 900

cttcatattg ccccaatgac cagctgcaca atgaatatca ctaccgcaga caccacatgc 960

ttcgatctta atgtcaatgt catgatcgta aaatggtttt gggtcatact ttgtcttctt 1020

tgggtttttc caatcttcgt gtgattgaat agcgatacct tcaaatttct caggataaga 1080

cat 1083

<210> 201
<211> 360
<212> PRT
<213> Saccharomyces cerevisiae

<400> 201

Met Ser Tyr Pro Glu Lys Phe Glu Gly Ile Ala Ile Gln Ser His Glu
1               5                   10                  15

Asp Trp Lys Asn Pro Lys Lys Thr Lys Tyr Asp Pro Lys Pro Phe Tyr
            20                  25                  30

Asp His Asp Ile Asp Ile Lys Ile Glu Ala Cys Gly Val Cys Gly Ser
        35                  40                  45

Asp Ile His Cys Ala Ala Gly His Trp Gly Asn Met Lys Met Pro Leu
    50                  55                  60

Val Val Gly His Glu Ile Val Gly Lys Val Val Lys Leu Gly Pro Lys
65                  70                  75                  80

Ser Asn Ser Gly Leu Lys Val Gly Gln Arg Val Gly Val Gly Ala Gln
                85                  90                  95

Val Phe Ser Cys Leu Glu Cys Asp Arg Cys Lys Asn Asp Asn Glu Pro
            100                 105                 110

Tyr Cys Thr Lys Phe Val Thr Thr Tyr Ser Gln Pro Tyr Glu Asp Gly
        115                 120                 125

Tyr Val Ser Gln Gly Gly Tyr Ala Asn Tyr Val Arg Val His Glu His
    130                 135                 140

Phe Val Val Pro Ile Pro Glu Asn Ile Pro Ser His Leu Ala Ala Pro
145                 150                 155                 160

Leu Leu Cys Gly Gly Leu Thr Val Tyr Ser Pro Leu Val Arg Asn Gly
                165                 170                 175

Cys Gly Pro Gly Lys Lys Val Gly Ile Val Gly Leu Gly Gly Ile Gly
            180                 185                 190

Ser Met Gly Thr Leu Ile Ser Lys Ala Met Gly Ala Glu Thr Tyr Val
        195                 200                 205

Ile Ser Arg Ser Ser Arg Lys Arg Glu Asp Ala Met Lys Met Gly Ala
    210                 215                 220

Asp His Tyr Ile Ala Thr Leu Glu Glu Gly Asp Trp Gly Glu Lys Tyr
225                 230                 235                 240

Phe Asp Thr Phe Asp Leu Ile Val Val Cys Ala Ser Ser Leu Thr Asp
                245                 250                 255

Ile Asp Phe Asn Ile Met Pro Lys Ala Met Lys Val Gly Gly Arg Ile
            260                 265                 270

Val Ser Ile Ser Ile Pro Glu Gln His Glu Met Leu Ser Leu Lys Pro
        275                 280                 285

Tyr Gly Leu Lys Ala Val Ser Ile Ser Tyr Ser Ala Leu Gly Ser Ile
    290                 295                 300

Lys Glu Leu Asn Gln Leu Leu Lys Leu Val Ser Glu Lys Asp Ile Lys
305                 310                 315                 320

Ile Trp Val Glu Thr Leu Pro Val Gly Glu Ala Gly Val His Glu Ala
                325                 330                 335

Phe Glu Arg Met Glu Lys Gly Asp Val Arg Tyr Arg Phe Thr Leu Val
            340                 345                 350

Gly Tyr Asp Lys Glu Phe Ser Asp
        355                 360

<210>  202
<211>  1170
<212>  DNA

<213>  Clostridium acetobutylicum

<400>  202
ttaataagat tttttaaata tctcaagaac atcctctgca tttattggtc ttaaacttcc      60

tattgttcct ccagaatttc taacagcttg ctttgccatt agttctagtt tatcttttcc     120

tattccaact tctctaagct ttgaaggaat acccaatgaa ttaaagtatt ctctcgtatt     180

tttaatagcc tctcgtgcta tttcatagtt atctttgttc ttgtctattc cccaaacatt     240

tattccataa gaaacaaatt tatgaagtgt atcgtcattt agaatatatt ccatccaatt     300

aggtgttaaa attgcaagtc ctacaccatg tgttatatca taatatgcac ttaactcgtg     360

ttccatagga tgacaactcc attttctatc cttaccaagt gataatagac catttatagc     420

taaacttgaa gcccacatca aattagctct agcctcgtaa tcatcagtct tctccattgc     480

tatttttcca tactttatac atgttcttaa gattgcttct gctataccgt cctgcacata     540

agcaccttca acaccactaa agtaagattc aaaggtgtga ctcataatgt cagctgttcc     600

cgctgctgtt tgatttttag gtactgtaaa agtatatgta ggatctaaca ctgaaaattt     660

aggtctcata tcatcatgtc ctactccaag cttttcatta gtctccatat ttgaaattac     720

tgcaatttga tccatttcag accctgttgc tgaaagagta agtatacttg caattggaag     780

aactttagtt attttagatg gatctttaac catgtcccat gtatcgccat cataataaac     840

tccagctgca attaccttag aacagtctat tgcacttcct ccccctattg ctaatactaa     900

atccacatta ttttctctac atatttctat gccttttttt actgttgtta tcctaggatt     960

tggctctact cctgaaagtt catagaaagc tatattgttt tcttttaata tagctgttgc    1020

tctatcatat ataccgttcc tttttatact tcctccgcca taaactataa gcactcttga    1080

gccatatttc ttaatttctt ctccaattac gtctattttt ccttttccaa aaaaaacttt    1140

agttggtatt gaataatcaa aacttagcat                                     1170


<210>  203
<211>  389
<212>  PRT
<213>  Clostridium acetobutylicum

<400>  203

Met Leu Ser Phe Asp Tyr Ser Ile Pro Thr Lys Val Phe Phe Gly Lys
1               5                   10                  15


Gly Lys Ile Asp Val Ile Gly Glu Glu Ile Lys Lys Tyr Gly Ser Arg
            20                  25                  30


Val Leu Ile Val Tyr Gly Gly Gly Ser Ile Lys Arg Asn Gly Ile Tyr
        35                  40                  45


Asp Arg Ala Thr Ala Ile Leu Lys Glu Asn Asn Ile Ala Phe Tyr Glu
    50                  55                  60

Leu Ser Gly Val Glu Pro Asn Pro Arg Ile Thr Thr Val Lys Lys Gly
65                  70                  75                  80

Ile Glu Ile Cys Arg Glu Asn Asn Val Asp Leu Val Leu Ala Ile Gly
                85                  90                  95

Gly Gly Ser Ala Ile Asp Cys Ser Lys Val Ile Ala Ala Gly Val Tyr
            100                 105                 110

Tyr Asp Gly Asp Thr Trp Asp Met Val Lys Asp Pro Ser Lys Ile Thr
        115                 120                 125

Lys Val Leu Pro Ile Ala Ser Ile Leu Thr Leu Ser Ala Thr Gly Ser
    130                 135                 140

Glu Met Asp Gln Ile Ala Val Ile Ser Asn Met Glu Thr Asn Glu Lys
145                 150                 155                 160

Leu Gly Val Gly His Asp Asp Met Arg Pro Lys Phe Ser Val Leu Asp
                165                 170                 175

Pro Thr Tyr Thr Phe Thr Val Pro Lys Asn Gln Thr Ala Ala Gly Thr
            180                 185                 190

Ala Asp Ile Met Ser His Thr Phe Glu Ser Tyr Phe Ser Gly Val Glu
        195                 200                 205

Gly Ala Tyr Val Gln Asp Gly Ile Ala Glu Ala Ile Leu Arg Thr Cys
    210                 215                 220

Ile Lys Tyr Gly Lys Ile Ala Met Glu Lys Thr Asp Asp Tyr Glu Ala
225                 230                 235                 240

Arg Ala Asn Leu Met Trp Ala Ser Ser Leu Ala Ile Asn Gly Leu Leu
                245                 250                 255

Ser Leu Gly Lys Asp Arg Lys Trp Ser Cys His Pro Met Glu His Glu
            260                 265                 270

Leu Ser Ala Tyr Tyr Asp Ile Thr His Gly Val Gly Leu Ala Ile Leu
        275                 280                 285

Thr Pro Asn Trp Met Glu Tyr Ile Leu Asn Asp Asp Thr Leu His Lys
    290                 295                 300

Phe Val Ser Tyr Gly Ile Asn Val Trp Gly Ile Asp Lys Asn Lys Asp
305                 310                 315                 320

Asn Tyr Glu Ile Ala Arg Glu Ala Ile Lys Asn Thr Arg Glu Tyr Phe
                325                 330                 335

Asn Ser Leu Gly Ile Pro Ser Lys Leu Arg Glu Val Gly Ile Gly Lys
            340                 345                 350

Asp Lys Leu Glu Leu Met Ala Lys Gln Ala Val Arg Asn Ser Gly Gly
        355                 360                 365

Thr Ile Gly Ser Leu Arg Pro Ile Asn Ala Glu Asp Val Leu Glu Ile
    370                 375                 380

Phe Lys Lys Ser Tyr
385

<210>  204
<211>  390
<212>  PRT
<213>  Clostridium acetobutylicum

<400>  204

Met Val Asp Phe Glu Tyr Ser Ile Pro Thr Arg Ile Phe Phe Gly Lys
1               5                   10                  15

Asp Lys Ile Asn Val Leu Gly Arg Glu Leu Lys Lys Tyr Gly Ser Lys
            20                  25                  30

Val Leu Ile Val Tyr Gly Gly Gly Ser Ile Lys Arg Asn Gly Ile Tyr
        35                  40                  45

Asp Lys Ala Val Ser Ile Leu Glu Lys Asn Ser Ile Lys Phe Tyr Glu
    50                  55                  60

Leu Ala Gly Val Glu Pro Asn Pro Arg Val Thr Thr Val Glu Lys Gly
65                  70                  75                  80

Val Lys Ile Cys Arg Glu Asn Gly Val Glu Val Val Leu Ala Ile Gly
                85                  90                  95

Gly Gly Ser Ala Ile Asp Cys Ala Lys Val Ile Ala Ala Ala Cys Glu
            100                 105                 110

Tyr Asp Gly Asn Pro Trp Asp Ile Val Leu Asp Gly Ser Lys Ile Lys
        115                 120                 125

Arg Val Leu Pro Ile Ala Ser Ile Leu Thr Ile Ala Ala Thr Gly Ser
    130                 135                 140

Glu Met Asp Thr Trp Ala Val Ile Asn Asn Met Asp Thr Asn Glu Lys

145                 150                 155                 160

Leu Ile Ala Ala His Pro Asp Met Ala Pro Lys Phe Ser Ile Leu Asp
                165                 170                 175

Pro Thr Tyr Thr Tyr Thr Val Pro Thr Asn Gln Thr Ala Ala Gly Thr
            180                 185                 190

Ala Asp Ile Met Ser His Ile Phe Glu Val Tyr Phe Ser Asn Thr Lys
        195                 200                 205

Thr Ala Tyr Leu Gln Asp Arg Met Ala Glu Ala Leu Leu Arg Thr Cys
    210                 215                 220

Ile Lys Tyr Gly Gly Ile Ala Leu Glu Lys Pro Asp Asp Tyr Glu Ala
225                 230                 235                 240

Arg Ala Asn Leu Met Trp Ala Ser Ser Leu Ala Ile Asn Gly Leu Leu
                245                 250                 255

Thr Tyr Gly Lys Asp Thr Asn Trp Ser Val His Leu Met Glu His Glu
            260                 265                 270

Leu Ser Ala Tyr Tyr Asp Ile Thr His Gly Val Gly Leu Ala Ile Leu
        275                 280                 285

Thr Pro Asn Trp Met Glu Tyr Ile Leu Asn Asn Asp Thr Val Tyr Lys
    290                 295                 300

Phe Val Glu Tyr Gly Val Asn Val Trp Gly Ile Asp Lys Glu Lys Asn
305                 310                 315                 320

His Tyr Asp Ile Ala His Gln Ala Ile Gln Lys Thr Arg Asp Tyr Phe
                325                 330                 335

Val Asn Val Leu Gly Leu Pro Ser Arg Leu Arg Asp Val Gly Ile Glu
            340                 345                 350

Glu Glu Lys Leu Asp Ile Met Ala Lys Glu Ser Val Lys Leu Thr Gly
        355                 360                 365

Gly Thr Ile Gly Asn Leu Arg Pro Val Asn Ala Ser Glu Val Leu Gln
    370                 375                 380

Ile Phe Lys Lys Ser Val
385                 390

<210>  205
<211>  993

<212>  DNA
<213>  Bacillus subtilis

<400>  205
atgagtacaa accgacatca agcactaggg ctgactgatc aggaagccgt tgatatgtat      60

agaaccatgc tgttagcaag aaaaatcgat gaaagaatgt ggctgttaaa ccgttctggc     120

aaaattccat ttgtaatctc ttgtcaagga caggaagcag cacaggtagg agcggctttc     180

gcacttgacc gtgaaatgga ttatgtattg ccgtactaca gagacatggg tgtcgtgctc     240

gcgtttggca tgacagcaaa ggacttaatg atgtccgggt ttgcaaaagc agcagatccg     300

aactcaggag gccgccagat gccgggacat ttcggacaaa agaaaaaccg cattgtgacg     360

ggatcatctc cggttacaac gcaagtgccg cacgcagtcg gtattgcgct tgcgggacgt     420

atggagaaaa aggatatcgc agcctttgtt acattcgggg aagggtcttc aaaccaaggc     480

gatttccatg aaggggcaaa ctttgccgct gtccataagc tgccggttat tttcatgtgt     540

gaaaacaaca aatacgcaat ctcagtgcct tacgataagc aagtcgcatg tgagaacatt     600

tccgaccgtg ccataggcta tgggatgcct ggcgtaactg tgaatggaaa tgatccgctg     660

gaagtttatc aagcggttaa agaagcacgc gaaagggcac gcagaggaga aggcccgaca     720

ttaattgaaa cgatttctta ccgccttaca ccacattcca gtgatgacga tgacagcagc     780

tacagaggcc gtgaagaagt agaggaagcg aaaaaaagtg atcccctgct tacttatcaa     840

gcttacttaa aggaaacagg cctgctgtcc gatgagatag aacaaaccat gctggatgaa     900

attatggcaa tcgtaaatga agcgacggat gaagcggaga acgccccata tgcagctcct     960

gagtcagcgc ttgattatgt ttatgcgaag tag                                  993

<210>  206
<211>  330
<212>  PRT
<213>  Bacillus subtilis

<400>  206

Met Ser Thr Asn Arg His Gln Ala Leu Gly Leu Thr Asp Gln Glu Ala
1               5                   10                  15

Val Asp Met Tyr Arg Thr Met Leu Leu Ala Arg Lys Ile Asp Glu Arg
            20                  25                  30

Met Trp Leu Leu Asn Arg Ser Gly Lys Ile Pro Phe Val Ile Ser Cys
        35                  40                  45

Gln Gly Gln Glu Ala Ala Gln Val Gly Ala Ala Phe Ala Leu Asp Arg
    50                  55                  60

Glu Met Asp Tyr Val Leu Pro Tyr Tyr Arg Asp Met Gly Val Val Leu
65                  70                  75                  80

Ala Phe Gly Met Thr Ala Lys Asp Leu Met Met Ser Gly Phe Ala Lys
                85                  90                  95

Ala Ala Asp Pro Asn Ser Gly Gly Arg Gln Met Pro Gly His Phe Gly
            100                 105                 110

Gln Lys Lys Asn Arg Ile Val Thr Gly Ser Ser Pro Val Thr Thr Gln
        115                 120                 125

Val Pro His Ala Val Gly Ile Ala Leu Ala Gly Arg Met Glu Lys Lys
    130                 135                 140

Asp Ile Ala Ala Phe Val Thr Phe Gly Glu Gly Ser Ser Asn Gln Gly
145                 150                 155                 160

Asp Phe His Glu Gly Ala Asn Phe Ala Ala Val His Lys Leu Pro Val
                165                 170                 175

Ile Phe Met Cys Glu Asn Asn Lys Tyr Ala Ile Ser Val Pro Tyr Asp
            180                 185                 190

Lys Gln Val Ala Cys Glu Asn Ile Ser Asp Arg Ala Ile Gly Tyr Gly
        195                 200                 205

Met Pro Gly Val Thr Val Asn Gly Asn Asp Pro Leu Glu Val Tyr Gln
    210                 215                 220

Ala Val Lys Glu Ala Arg Glu Arg Ala Arg Arg Gly Glu Gly Pro Thr
225                 230                 235                 240

Leu Ile Glu Thr Ile Ser Tyr Arg Leu Thr Pro His Ser Ser Asp Asp
                245                 250                 255

Asp Asp Ser Ser Tyr Arg Gly Arg Glu Glu Val Glu Glu Ala Lys Lys
            260                 265                 270

Ser Asp Pro Leu Leu Thr Tyr Gln Ala Tyr Leu Lys Glu Thr Gly Leu
        275                 280                 285

Leu Ser Asp Glu Ile Glu Gln Thr Met Leu Asp Glu Ile Met Ala Ile
    290                 295                 300

Val Asn Glu Ala Thr Asp Glu Ala Glu Asn Ala Pro Tyr Ala Ala Pro
305                 310                 315                 320

Glu Ser Ala Leu Asp Tyr Val Tyr Ala Lys
                325                 330

<210>  207
<211>  984
<212>  DNA
<213>  Bacillus subtilis

<400>  207
atgtcagtaa tgtcatatat tgatgcaatc aatttggcga tgaaagaaga aatggaacga      60

gattctcgcg ttttcgtcct tggggaagat gtaggaagaa aaggcggtgt gtttaaagcg     120

acagcgggac tctatgaaca atttggggaa gagcgcgtta tggatacgcc gcttgctgaa     180

tctgcaatcg caggagtcgg tatcggagcg gcaatgtacg gaatgagacc gattgctgaa     240

atgcagtttg ctgatttcat tatgccggca gtcaaccaaa ttatttctga agcggctaaa     300

atccgctacc gcagcaacaa tgactggagc tgtccgattg tcgtcagagc gccatacggc     360

ggaggcgtgc acggagccct gtatcattct caatcagtcg aagcaatttt cgccaaccag     420

cccggactga aaattgtcat gccatcaaca ccatatgacg cgaaagggct cttaaaagcc     480

gcagttcgtg acgaagaccc cgtgctgttt tttgagcaca agcgggcata ccgtctgata     540

aagggcgagg ttccggctga tgattatgtc ctgccaatcg gcaaggcgga cgtaaaaagg     600

gaaggcgacg acatcacagt gatcacatac ggcctgtgtg tccacttcgc cttacaagct     660

gcagaacgtc tcgaaaaaga tggcatttca gcgcatgtgg tggatttaag aacagtttac     720

ccgcttgata aagaagccat catcgaagct gcgtccaaaa ctggaaaggt tcttttggtc     780

acagaagata caaaagaagg cagcatcatg agcgaagtag ccgcaattat atccgagcat     840

tgtctgttcg acttagacgc gccgatcaaa cggcttgcag gtcctgatat tccggctatg     900

ccttatgcgc cgacaatgga aaaatacttt atggtcaacc ctgataaagt ggaagcggcg     960

atgagagaat tagcggagtt ttaa                                            984


<210>  208
<211>  327
<212>  PRT
<213>  Bacillus subtilis

<400>  208

Met Ser Val Met Ser Tyr Ile Asp Ala Ile Asn Leu Ala Met Lys Glu
1               5                   10                  15


Glu Met Glu Arg Asp Ser Arg Val Phe Val Leu Gly Glu Asp Val Gly
            20                  25                  30


Arg Lys Gly Gly Val Phe Lys Ala Thr Ala Gly Leu Tyr Glu Gln Phe
        35                  40                  45


Gly Glu Glu Arg Val Met Asp Thr Pro Leu Ala Glu Ser Ala Ile Ala
    50                  55                  60


Gly Val Gly Ile Gly Ala Ala Met Tyr Gly Met Arg Pro Ile Ala Glu

65                  70                  75                  80

Met Gln Phe Ala Asp Phe Ile Met Pro Ala Val Asn Gln Ile Ile Ser
                85                  90                  95

Glu Ala Ala Lys Ile Arg Tyr Arg Ser Asn Asn Asp Trp Ser Cys Pro
            100                 105                 110

Ile Val Val Arg Ala Pro Tyr Gly Gly Gly Val His Gly Ala Leu Tyr
        115                 120                 125

His Ser Gln Ser Val Glu Ala Ile Phe Ala Asn Gln Pro Gly Leu Lys
    130                 135                 140

Ile Val Met Pro Ser Thr Pro Tyr Asp Ala Lys Gly Leu Leu Lys Ala
145                 150                 155                 160

Ala Val Arg Asp Glu Asp Pro Val Leu Phe Phe Glu His Lys Arg Ala
                165                 170                 175

Tyr Arg Leu Ile Lys Gly Glu Val Pro Ala Asp Asp Tyr Val Leu Pro
            180                 185                 190

Ile Gly Lys Ala Asp Val Lys Arg Glu Gly Asp Asp Ile Thr Val Ile
        195                 200                 205

Thr Tyr Gly Leu Cys Val His Phe Ala Leu Gln Ala Ala Glu Arg Leu
    210                 215                 220

Glu Lys Asp Gly Ile Ser Ala His Val Val Asp Leu Arg Thr Val Tyr
225                 230                 235                 240

Pro Leu Asp Lys Glu Ala Ile Ile Glu Ala Ala Ser Lys Thr Gly Lys
                245                 250                 255

Val Leu Leu Val Thr Glu Asp Thr Lys Glu Gly Ser Ile Met Ser Glu
            260                 265                 270

Val Ala Ala Ile Ile Ser Glu His Cys Leu Phe Asp Leu Asp Ala Pro
        275                 280                 285

Ile Lys Arg Leu Ala Gly Pro Asp Ile Pro Ala Met Pro Tyr Ala Pro
    290                 295                 300

Thr Met Glu Lys Tyr Phe Met Val Asn Pro Asp Lys Val Glu Ala Ala
305                 310                 315                 320

Met Arg Glu Leu Ala Glu Phe
                325

<210>  209
<211>  1275
<212>  DNA
<213>  Bacillus subtilis

<400>  209
atggcaattg aacaaatgac gatgccgcag cttggagaaa gcgtaacaga ggggacgatc      60

agcaaatggc ttgtcgcccc cggtgataaa gtgaacaaat acgatccgat cgcggaagtc     120

atgacagata aggtaaatgc agaggttccg tcttctttta ctggtacgat aacagagctt     180

gtgggagaag aaggccaaac cctgcaagtc ggagaaatga tttgcaaaat tgaaacagaa     240

ggcgcgaatc cggctgaaca aaaacaagaa cagccagcag catcagaagc cgctgagaac     300

cctgttgcaa aaagtgctgg agcagccgat cagcccaata aaaagcgcta ctcgccagct     360

gttctccgtt tggccggaga gcacggcatt gacctcgatc aagtgacagg aactggtgcc     420

ggcgggcgca tcacacgaaa agatattcag cgcttaattg aaacaggcgg cgtgcaagaa     480

cagaatcctg aggagctgaa aacagcagct cctgcaccga agtctgcatc aaaacctgag     540

ccaaaagaag agacgtcata tcctgcgtct gcagccggtg ataaagaaat ccctgtcaca     600

ggtgtaagaa aagcaattgc ttccaatatg aagcgaagca aaacagaaat tccgcatgct     660

tggacgatga tggaagtcga cgtcacaaat atggttgcat atcgcaacag tataaaagat     720

tcttttaaga agacagaagg ctttaattta acgttcttcg ccttttttgt aaaagcggtc     780

gctcaggcgt taaaagaatt cccgcaaatg aatagcatgt gggcggggga caaaattatt     840

cagaaaaagg atatcaatat ttcaattgca gttgccacag aggattcttt atttgttccg     900

gtgattaaaa acgctgatga aaaaacaatt aaaggcattg cgaaagacat taccggccta     960

gctaaaaaag taagagacgg aaaactcact gcagatgaca tgcagggagg cacgtttacc    1020

gtcaacaaca caggttcgtt cgggtctgtt cagtcgatgg gcattatcaa ctaccctcag    1080

gctgcgattc ttcaagtaga atccatcgtc aaacgcccgg ttgtcatgga caatggcatg    1140

attgctgtca gagacatggt taatctgtgc ctgtcattag atcacagagt gcttgacggt    1200

ctcgtgtgcg gacgattcct cggacgagtg aaacaaattt tagaatcgat tgacgagaag    1260

acatctgttt actaa                                                      1275

<210>  210
<211>  424
<212>  PRT
<213>  Bacillus subtilis

<400>  210

Met Ala Ile Glu Gln Met Thr Met Pro Gln Leu Gly Glu Ser Val Thr
1               5                   10                  15

Glu Gly Thr Ile Ser Lys Trp Leu Val Ala Pro Gly Asp Lys Val Asn

20                  25                  30

Lys Tyr Asp Pro Ile Ala Glu Val Met Thr Asp Lys Val Asn Ala Glu
        35                  40                  45

Val Pro Ser Ser Phe Thr Gly Thr Ile Thr Glu Leu Val Gly Glu Glu
    50                  55                  60

Gly Gln Thr Leu Gln Val Gly Glu Met Ile Cys Lys Ile Glu Thr Glu
65                  70                  75                  80

Gly Ala Asn Pro Ala Glu Gln Lys Gln Glu Gln Pro Ala Ala Ser Glu
                85                  90                  95

Ala Ala Glu Asn Pro Val Ala Lys Ser Ala Gly Ala Ala Asp Gln Pro
            100                 105                 110

Asn Lys Lys Arg Tyr Ser Pro Ala Val Leu Arg Leu Ala Gly Glu His
        115                 120                 125

Gly Ile Asp Leu Asp Gln Val Thr Gly Thr Gly Ala Gly Gly Arg Ile
    130                 135                 140

Thr Arg Lys Asp Ile Gln Arg Leu Ile Glu Thr Gly Gly Val Gln Glu
145                 150                 155                 160

Gln Asn Pro Glu Glu Leu Lys Thr Ala Ala Pro Ala Pro Lys Ser Ala
                165                 170                 175

Ser Lys Pro Glu Pro Lys Glu Glu Thr Ser Tyr Pro Ala Ser Ala Ala
            180                 185                 190

Gly Asp Lys Glu Ile Pro Val Thr Gly Val Arg Lys Ala Ile Ala Ser
        195                 200                 205

Asn Met Lys Arg Ser Lys Thr Glu Ile Pro His Ala Trp Thr Met Met
    210                 215                 220

Glu Val Asp Val Thr Asn Met Val Ala Tyr Arg Asn Ser Ile Lys Asp
225                 230                 235                 240

Ser Phe Lys Lys Thr Glu Gly Phe Asn Leu Thr Phe Phe Ala Phe Phe
                245                 250                 255

Val Lys Ala Val Ala Gln Ala Leu Lys Glu Phe Pro Gln Met Asn Ser
            260                 265                 270

Met Trp Ala Gly Asp Lys Ile Ile Gln Lys Lys Asp Ile Asn Ile Ser
        275                 280                 285

Ile Ala Val Ala Thr Glu Asp Ser Leu Phe Val Pro Val Ile Lys Asn
    290                 295                 300

Ala Asp Glu Lys Thr Ile Lys Gly Ile Ala Lys Asp Ile Thr Gly Leu
305                 310                 315                 320

Ala Lys Lys Val Arg Asp Gly Lys Leu Thr Ala Asp Asp Met Gln Gly
                325                 330                 335

Gly Thr Phe Thr Val Asn Asn Thr Gly Ser Phe Gly Ser Val Gln Ser
            340                 345                 350

Met Gly Ile Ile Asn Tyr Pro Gln Ala Ala Ile Leu Gln Val Glu Ser
        355                 360                 365

Ile Val Lys Arg Pro Val Val Met Asp Asn Gly Met Ile Ala Val Arg
    370                 375                 380

Asp Met Val Asn Leu Cys Leu Ser Leu Asp His Arg Val Leu Asp Gly
385                 390                 395                 400

Leu Val Cys Gly Arg Phe Leu Gly Arg Val Lys Gln Ile Leu Glu Ser
                405                 410                 415

Ile Asp Glu Lys Thr Ser Val Tyr
            420

<210>  211
<211>  1374
<212>  DNA
<213>  Bacillus subtilis

<400>  211
atggcaactg agtatgacgt agtcattctg ggcggcggta ccggcggtta tgttgcggcc      60

atcagagccg ctcagctcgg cttaaaaaca gccgttgtgg aaaaggaaaa actcggggga     120

acatgtctgc ataaaggctg tatcccgagt aaagcgctgc ttagaagcgc agaggtatac     180

cggacagctc gtgaagccga tcaattcgga gtggaaacgg ctggcgtgtc cctcaacttt     240

gaaaaagtgc agcagcgtaa gcaagccgtt gttgataagc ttgcagcggg tgtaaatcat     300

ttaatgaaaa aaggaaaaat tgacgtgtac accggatatg gacgtatcct tggaccgtca     360

atcttctctc cgctgccggg aacaatttct gttgagcggg gaaatggcga agaaaatgac     420

atgctgatcc cgaaacaagt gatcattgca acaggatcaa gaccgagaat gcttccgggt     480

cttgaagtgg acggtaagtc tgtactgact tcagatgagg cgctccaaat ggaggagctg     540

ccacagtcaa tcatcattgt cggcggaggg gttatcggta tcgaatgggc gtctatgctt     600

catgattttg gcgttaaggt aacggttatt gaatacgcgg atcgcatatt gccgactgaa     660

gatctagaga tttcaaaaga aatggaaagt cttcttaaga aaaaaggcat ccagttcata 720

acaggggcaa aagtgctgcc tgacacaatg acaaaaacat cagacgatat cagcatacaa 780

gcggaaaaag acggagaaac cgttacctat tctgctgaga aaatgcttgt ttccatcggc 840

agacaggcaa atatcgaagg catcggccta gagaacaccg atattgttac tgaaaatggc 900

atgatttcag tcaatgaaag ctgccaaacg aaggaatctc atatttatgc aatcggagac 960

gtaatcggtg gcctgcagtt agctcacgtt gcttcacatg agggaattat tgctgttgag 1020

cattttgcag gtctcaatcc gcatccgctt gatccgacgc ttgtgccgaa gtgcatttac 1080

tcaagccctg aagctgccag tgtcggctta accgaagacg aagcaaaggc gaacgggcat 1140

aatgtcaaaa tcggcaagtt cccatttatg gcgattggaa aagcgcttgt atacggtgaa 1200

agcgacggtt ttgtcaaaat cgtggctgac cgagatacag atgatattct cggcgttcat 1260

atgattggcc cgcatgtcac cgacatgatt tctgaagcgg gtcttgccaa agtgctggac 1320

gcaacaccgt gggaggtcgg gcaaacgatt tcacccgcat ccaacgcttt ctga 1374

<210> 212
<211> 457
<212> PRT
<213> Bacillus subtilis

<400> 212

Met Ala Thr Glu Tyr Asp Val Val Ile Leu Gly Gly Gly Thr Gly Gly
1               5                   10                  15

Tyr Val Ala Ala Ile Arg Ala Ala Gln Leu Gly Leu Lys Thr Ala Val
            20                  25                  30

Val Glu Lys Glu Lys Leu Gly Gly Thr Cys Leu His Lys Gly Cys Ile
        35                  40                  45

Pro Ser Lys Ala Leu Leu Arg Ser Ala Glu Val Tyr Arg Thr Ala Arg
    50                  55                  60

Glu Ala Asp Gln Phe Gly Val Glu Thr Ala Gly Val Ser Leu Asn Phe
65                  70                  75                  80

Glu Lys Val Gln Gln Arg Lys Gln Ala Val Val Asp Lys Leu Ala Ala
                85                  90                  95

Gly Val Asn His Leu Met Lys Lys Gly Lys Ile Asp Val Tyr Thr Gly
            100                 105                 110

Tyr Gly Arg Ile Leu Gly Pro Ser Ile Phe Ser Pro Leu Pro Gly Thr
        115                 120                 125

Ile Ser Val Glu Arg Gly Asn Gly Glu Glu Asn Asp Met Leu Ile Pro
    130                 135                 140

Lys Gln Val Ile Ile Ala Thr Gly Ser Arg Pro Arg Met Leu Pro Gly
145                 150                 155                 160

Leu Glu Val Asp Gly Lys Ser Val Leu Thr Ser Asp Glu Ala Leu Gln
                165                 170                 175

Met Glu Glu Leu Pro Gln Ser Ile Ile Ile Val Gly Gly Gly Val Ile
            180                 185                 190

Gly Ile Glu Trp Ala Ser Met Leu His Asp Phe Gly Val Lys Val Thr
        195                 200                 205

Val Ile Glu Tyr Ala Asp Arg Ile Leu Pro Thr Glu Asp Leu Glu Ile
    210                 215                 220

Ser Lys Glu Met Glu Ser Leu Leu Lys Lys Lys Gly Ile Gln Phe Ile
225                 230                 235                 240

Thr Gly Ala Lys Val Leu Pro Asp Thr Met Thr Lys Thr Ser Asp Asp
                245                 250                 255

Ile Ser Ile Gln Ala Glu Lys Asp Gly Glu Thr Val Thr Tyr Ser Ala
            260                 265                 270

Glu Lys Met Leu Val Ser Ile Gly Arg Gln Ala Asn Ile Glu Gly Ile
        275                 280                 285

Gly Leu Glu Asn Thr Asp Ile Val Thr Glu Asn Gly Met Ile Ser Val
    290                 295                 300

Asn Glu Ser Cys Gln Thr Lys Glu Ser His Ile Tyr Ala Ile Gly Asp
305                 310                 315                 320

Val Ile Gly Gly Leu Gln Leu Ala His Val Ala Ser His Glu Gly Ile
                325                 330                 335

Ile Ala Val Glu His Phe Ala Gly Leu Asn Pro His Pro Leu Asp Pro
            340                 345                 350

Thr Leu Val Pro Lys Cys Ile Tyr Ser Ser Pro Glu Ala Ala Ser Val
        355                 360                 365

Gly Leu Thr Glu Asp Glu Ala Lys Ala Asn Gly His Asn Val Lys Ile
    370                 375                 380

Gly Lys Phe Pro Phe Met Ala Ile Gly Lys Ala Leu Val Tyr Gly Glu

385                 390                 395                 400

Ser Asp Gly Phe Val Lys Ile Val Ala Asp Arg Asp Thr Asp Asp Ile
                405                 410                 415

Leu Gly Val His Met Ile Gly Pro His Val Thr Asp Met Ile Ser Glu
            420                 425                 430

Ala Gly Leu Ala Lys Val Leu Asp Ala Thr Pro Trp Glu Val Gly Gln
        435                 440                 445

Thr Ile Ser Pro Ala Ser Asn Ala Phe
    450                 455

<210>  213
<211>  1233
<212>  DNA
<213>  Pseudomonas putida

<400>  213
atgaacgagt acgcccccct gcgtttgcat gtgcccgagc ccaccggccg gccaggctgc      60

cagaccgatt tttcctacct gcgcctgaac gatgcaggtc aagcccgtaa accccctgtc     120

gatgtcgacg ctgccgacac cgccgacctg tcctacagcc tggtccgcgt gctcgacgag     180

caaggcgacg cccaaggccc gtgggctgaa gacatcgacc cgcagatcct gcgccaaggc     240

atgcgcgcca tgctcaagac gcggatcttc gacagccgca tggtggttgc ccagcgccag     300

aagaagatgt ccttctacat gcagagcctg ggcgaagaag ccatcggcag cggccaggcg     360

ctggcgctta accgcaccga catgtgcttc cccacctacc gtcagcaaag catcctgatg     420

gcccgcgacg tgtcgctggt ggagatgatc tgccagttgc tgtccaacga acgcgacccc     480

ctcaagggcc gccagctgcc gatcatgtac tcggtacgcg aggccggctt cttcaccatc     540

agcggcaacc tggcgaccca gttcgtgcag gcggtcggct gggccatggc ctcggcgatc     600

aagggcgata ccaagattgc ctcggcctgg atcggcgacg gcgccactgc cgaatcggac     660

ttccacaccg ccctcacctt tgcccacgtt taccgcgccc cggtgatcct caacgtggtc     720

aacaaccagt gggccatctc aaccttccag gccatcgccg gtggcgagtc gaccaccttc     780

gccggccgtg gcgtgggctg cggcatcgct tcgctgcggg tggacggcaa cgacttcgtc     840

gccgtttacg ccgcttcgcg ctgggctgcc gaacgtgccc gccgtggttt gggcccgagc     900

ctgatcgagt gggtcaccta ccgtgccggc ccgcactcga cctcggacga cccgtccaag     960

taccgccctg ccgatgactg gagccacttc ccgctgggtg acccgatcgc ccgcctgaag    1020

cagcacctga tcaagatcgg ccactggtcc gaagaagaac accaggccac cacggccgag    1080

ttcgaagcgg ccgtgattgc tgcgcaaaaa gaagccgagc agtacggcac cctggccaac    1140

ggtcacatcc cgagcgccgc ctcgatgttc gaggacgtgt acaaggagat gcccgaccac    1200

ctgcgccgcc aacgccagga actgggggtt tga                                    1233

<210>  214
<211>  410
<212>  PRT
<213>  Pseudomonas putida

<400>  214

Met Asn Glu Tyr Ala Pro Leu Arg Leu His Val Pro Glu Pro Thr Gly
1               5                   10                  15

Arg Pro Gly Cys Gln Thr Asp Phe Ser Tyr Leu Arg Leu Asn Asp Ala
            20                  25                  30

Gly Gln Ala Arg Lys Pro Pro Val Asp Val Asp Ala Ala Asp Thr Ala
        35                  40                  45

Asp Leu Ser Tyr Ser Leu Val Arg Val Leu Asp Glu Gln Gly Asp Ala
    50                  55                  60

Gln Gly Pro Trp Ala Glu Asp Ile Asp Pro Gln Ile Leu Arg Gln Gly
65                  70                  75                  80

Met Arg Ala Met Leu Lys Thr Arg Ile Phe Asp Ser Arg Met Val Val
                85                  90                  95

Ala Gln Arg Gln Lys Lys Met Ser Phe Tyr Met Gln Ser Leu Gly Glu
            100                 105                 110

Glu Ala Ile Gly Ser Gly Gln Ala Leu Ala Leu Asn Arg Thr Asp Met
        115                 120                 125

Cys Phe Pro Thr Tyr Arg Gln Gln Ser Ile Leu Met Ala Arg Asp Val
    130                 135                 140

Ser Leu Val Glu Met Ile Cys Gln Leu Leu Ser Asn Glu Arg Asp Pro
145                 150                 155                 160

Leu Lys Gly Arg Gln Leu Pro Ile Met Tyr Ser Val Arg Glu Ala Gly
                165                 170                 175

Phe Phe Thr Ile Ser Gly Asn Leu Ala Thr Gln Phe Val Gln Ala Val
            180                 185                 190

Gly Trp Ala Met Ala Ser Ala Ile Lys Gly Asp Thr Lys Ile Ala Ser
        195                 200                 205

Ala Trp Ile Gly Asp Gly Ala Thr Ala Glu Ser Asp Phe His Thr Ala
    210                 215                 220

Leu Thr Phe Ala His Val Tyr Arg Ala Pro Val Ile Leu Asn Val Val
225                 230                 235                 240

Asn Asn Gln Trp Ala Ile Ser Thr Phe Gln Ala Ile Ala Gly Gly Glu
                245                 250                 255

Ser Thr Thr Phe Ala Gly Arg Gly Val Gly Cys Gly Ile Ala Ser Leu
            260                 265                 270

Arg Val Asp Gly Asn Asp Phe Val Ala Val Tyr Ala Ala Ser Arg Trp
        275                 280                 285

Ala Ala Glu Arg Ala Arg Arg Gly Leu Gly Pro Ser Leu Ile Glu Trp
    290                 295                 300

Val Thr Tyr Arg Ala Gly Pro His Ser Thr Ser Asp Asp Pro Ser Lys
305                 310                 315                 320

Tyr Arg Pro Ala Asp Asp Trp Ser His Phe Pro Leu Gly Asp Pro Ile
                325                 330                 335

Ala Arg Leu Lys Gln His Leu Ile Lys Ile Gly His Trp Ser Glu Glu
            340                 345                 350

Glu His Gln Ala Thr Thr Ala Glu Phe Glu Ala Ala Val Ile Ala Ala
        355                 360                 365

Gln Lys Glu Ala Glu Gln Tyr Gly Thr Leu Ala Asn Gly His Ile Pro
    370                 375                 380

Ser Ala Ala Ser Met Phe Glu Asp Val Tyr Lys Glu Met Pro Asp His
385                 390                 395                 400

Leu Arg Arg Gln Arg Gln Glu Leu Gly Val
                405                 410

<210>  215
<211>  1059
<212>  DNA
<213>  Pseudomonas putida

<400>  215
atgaacgacc acaacaacag catcaacccg gaaaccgcca tggccaccac taccatgacc      60

atgatccagg ccctgcgctc ggccatggat gtcatgcttg agcgcgacga caatgtggtg     120

gtgtacggcc aggacgtcgg ctacttcggc ggcgtgttcc gctgcaccga aggcctgcag     180

accaagtacg gcaagtcccg cgtgttcgac gcgcccatct ctgaaagcgg catcgtcggc     240

accgccgtgg gcatgggtgc ctacggcctg cgcccggtgg tggaaatcca gttcgctgac     300

tacttctacc cggcctccga ccagatcgtt tctgaaatgg cccgcctgcg ctaccgttcg 360

gccggcgagt tcatcgcccc gctgaccctg cgtatgccct gcggtggcgg tatctatggc 420

ggccagacac acagccagag cccggaagcg atgttcactc aggtgtgcgg cctgcgcacc 480

gtaatgccat ccaacccgta cgacgccaaa ggcctgctga ttgcctcgat cgaatgcgac 540

gacccggtga tcttcctgga gcccaagcgc ctgtacaacg gcccgttcga cggccaccat 600

gaccgcccgg ttacgccgtg gtcgaaacac ccgcacagcg ccgtgcccga tggctactac 660

accgtgccac tggacaaggc cgccatcacc cgccccggca atgacgtgag cgtgctcacc 720

tatggcacca ccgtgtacgt ggcccaggtg gccgccgaag aaagtggcgt ggatgccgaa 780

gtgatcgacc tgcgcagcct gtggccgcta gacctggaca ccatcgtcga gtcggtgaaa 840

aagaccggcc gttgcgtggt agtacacgag gccacccgta cttgtggctt tggcgcagaa 900

ctggtgtcgc tggtgcagga gcactgcttc caccacctgg aggcgccgat cgagcgcgtc 960

accggttggg acacccccta ccctcacgcg caggaatggg cttacttccc agggccttcg 1020

cgggtaggtg cggcattgaa aaaggtcatg gaggtctga 1059

<210> 216
<211> 352
<212> PRT
<213> Pseudomonas putida

<400> 216

Met Asn Asp His Asn Asn Ser Ile Asn Pro Glu Thr Ala Met Ala Thr
1               5                   10                  15

Thr Thr Met Thr Met Ile Gln Ala Leu Arg Ser Ala Met Asp Val Met
            20                  25                  30

Leu Glu Arg Asp Asp Asn Val Val Val Tyr Gly Gln Asp Val Gly Tyr
        35                  40                  45

Phe Gly Gly Val Phe Arg Cys Thr Glu Gly Leu Gln Thr Lys Tyr Gly
    50                  55                  60

Lys Ser Arg Val Phe Asp Ala Pro Ile Ser Glu Ser Gly Ile Val Gly
65                  70                  75                  80

Thr Ala Val Gly Met Gly Ala Tyr Gly Leu Arg Pro Val Val Glu Ile
                85                  90                  95

Gln Phe Ala Asp Tyr Phe Tyr Pro Ala Ser Asp Gln Ile Val Ser Glu
            100                 105                 110

Met Ala Arg Leu Arg Tyr Arg Ser Ala Gly Glu Phe Ile Ala Pro Leu
        115                 120                 125

Thr Leu Arg Met Pro Cys Gly Gly Gly Ile Tyr Gly Gly Gln Thr His
    130                 135                 140

Ser Gln Ser Pro Glu Ala Met Phe Thr Gln Val Cys Gly Leu Arg Thr
145                 150                 155                 160

Val Met Pro Ser Asn Pro Tyr Asp Ala Lys Gly Leu Leu Ile Ala Ser
                165                 170                 175

Ile Glu Cys Asp Asp Pro Val Ile Phe Leu Glu Pro Lys Arg Leu Tyr
            180                 185                 190

Asn Gly Pro Phe Asp Gly His His Asp Arg Pro Val Thr Pro Trp Ser
        195                 200                 205

Lys His Pro His Ser Ala Val Pro Asp Gly Tyr Tyr Thr Val Pro Leu
    210                 215                 220

Asp Lys Ala Ala Ile Thr Arg Pro Gly Asn Asp Val Ser Val Leu Thr
225                 230                 235                 240

Tyr Gly Thr Thr Val Tyr Val Ala Gln Val Ala Ala Glu Glu Ser Gly
                245                 250                 255

Val Asp Ala Glu Val Ile Asp Leu Arg Ser Leu Trp Pro Leu Asp Leu
            260                 265                 270

Asp Thr Ile Val Glu Ser Val Lys Lys Thr Gly Arg Cys Val Val Val
        275                 280                 285

His Glu Ala Thr Arg Thr Cys Gly Phe Gly Ala Glu Leu Val Ser Leu
    290                 295                 300

Val Gln Glu His Cys Phe His His Leu Glu Ala Pro Ile Glu Arg Val
305                 310                 315                 320

Thr Gly Trp Asp Thr Pro Tyr Pro His Ala Gln Glu Trp Ala Tyr Phe
                325                 330                 335

Pro Gly Pro Ser Arg Val Gly Ala Ala Leu Lys Lys Val Met Glu Val
            340                 345                 350

<210>  217
<211>  1272
<212>  DNA
<213>  Pseudomonas putida

<400>  217
atgggcacgc acgtcatcaa gatgccggac attggcgaag gcatcgcgca ggtcgaattg      60

gtggaatggt tcgtcaaggt gggcgacatc atcgccgagg accaagtggt agccgacgtc 120

atgaccgaca aggccaccgt ggaaatcccg tcgccggtca gcggcaaggt gctggccctg 180

ggtggccagc caggtgaagt gatggcggtc ggcagtgagc tgatccgcat cgaagtggaa 240

ggcagcggca accatgtgga tgtgccgcaa gccaagccgg ccgaagtgcc tgcggcaccg 300

gtagccgcta aacctgaacc acagaaagac gttaaaccgg cggcgtacca ggcgtcagcc 360

agccacgagg cagcgcccat cgtgccgcgc cagccgggcg acaagccgct ggcctcgccg 420

gcggtgcgca aacgcgccct cgatgccggc atcgaattgc gttatgtgca cggcagcggc 480

ccggccgggc gcatcctgca cgaagacctc gacgcgttca tgagcaaacc gcaaagcgct 540

gccgggcaaa cccccaatgg ctatgccagg cgcaccgaca gcgagcaggt gccggtgatc 600

ggcctgcgcc gcaagatcgc ccagcgcatg caggacgcca agcgccgggt cgcgcacttc 660

agctatgtgg aagaaatcga cgtcaccgcc ctggaagccc tgcgccagca gctcaacagc 720

aagcacggcg acagccgcgg caagctgaca ctgctgccgt tcctggtgcg cgccctggtc 780

gtggcactgc gtgacttccc gcagataaac gccacctacg atgacgaagc gcagatcatc 840

acccgccatg gcgcggtgca tgtgggcatc gccacccaag gtgacaacgg cctgatggta 900

cccgtgctgc gccacgccga agcgggcagc ctgtgggcca atgccggtga gatttcacgc 960

ctggccaacg ctgcgcgcaa caacaaggcc agccgcgaag agctgtccgg ttcgaccatt 1020

accctgacca gcctcggcgc cctgggcggc atcgtcagca cgccggtggt caacaccccg 1080

gaagtggcga tcgtcggtgt caaccgcatg gttgagcggc ccgtggtgat cgacggccag 1140

atcgtcgtgc gcaagatgat gaacctgtcc agctcgttcg accaccgcgt ggtcgatggc 1200

atggacgccg ccctgttcat ccaggccgtg cgtggcctgc tcgaacaacc cgcctgcctg 1260

ttcgtggagt ga 1272

<210> 218
<211> 423
<212> PRT
<213> Pseudomonas putida

<400> 218

Met Gly Thr His Val Ile Lys Met Pro Asp Ile Gly Glu Gly Ile Ala
1               5                   10                  15

Gln Val Glu Leu Val Glu Trp Phe Val Lys Val Gly Asp Ile Ile Ala
            20                  25                  30

Glu Asp Gln Val Val Ala Asp Val Met Thr Asp Lys Ala Thr Val Glu
        35                  40                  45

Ile Pro Ser Pro Val Ser Gly Lys Val Leu Ala Leu Gly Gly Gln Pro
    50                  55                  60

Gly Glu Val Met Ala Val Gly Ser Glu Leu Ile Arg Ile Glu Val Glu
65                  70                  75                  80

Gly Ser Gly Asn His Val Asp Val Pro Gln Ala Lys Pro Ala Glu Val
                85                  90                  95

Pro Ala Ala Pro Val Ala Ala Lys Pro Glu Pro Gln Lys Asp Val Lys
            100                 105                 110

Pro Ala Ala Tyr Gln Ala Ser Ala Ser His Glu Ala Ala Pro Ile Val
        115                 120                 125

Pro Arg Gln Pro Gly Asp Lys Pro Leu Ala Ser Pro Ala Val Arg Lys
    130                 135                 140

Arg Ala Leu Asp Ala Gly Ile Glu Leu Arg Tyr Val His Gly Ser Gly
145                 150                 155                 160

Pro Ala Gly Arg Ile Leu His Glu Asp Leu Asp Ala Phe Met Ser Lys
                165                 170                 175

Pro Gln Ser Ala Ala Gly Gln Thr Pro Asn Gly Tyr Ala Arg Arg Thr
            180                 185                 190

Asp Ser Glu Gln Val Pro Val Ile Gly Leu Arg Arg Lys Ile Ala Gln
        195                 200                 205

Arg Met Gln Asp Ala Lys Arg Arg Val Ala His Phe Ser Tyr Val Glu
    210                 215                 220

Glu Ile Asp Val Thr Ala Leu Glu Ala Leu Arg Gln Gln Leu Asn Ser
225                 230                 235                 240

Lys His Gly Asp Ser Arg Gly Lys Leu Thr Leu Leu Pro Phe Leu Val
                245                 250                 255

Arg Ala Leu Val Val Ala Leu Arg Asp Phe Pro Gln Ile Asn Ala Thr
            260                 265                 270

Tyr Asp Asp Glu Ala Gln Ile Ile Thr Arg His Gly Ala Val His Val
        275                 280                 285

Gly Ile Ala Thr Gln Gly Asp Asn Gly Leu Met Val Pro Val Leu Arg
    290                 295                 300

His Ala Glu Ala Gly Ser Leu Trp Ala Asn Ala Gly Glu Ile Ser Arg
305                 310                 315                 320

Leu Ala Asn Ala Ala Arg Asn Asn Lys Ala Ser Arg Glu Glu Leu Ser
                325                 330                 335

Gly Ser Thr Ile Thr Leu Thr Ser Leu Gly Ala Leu Gly Gly Ile Val
            340                 345                 350

Ser Thr Pro Val Val Asn Thr Pro Glu Val Ala Ile Val Gly Val Asn
        355                 360                 365

Arg Met Val Glu Arg Pro Val Val Ile Asp Gly Gln Ile Val Val Arg
    370                 375                 380

Lys Met Met Asn Leu Ser Ser Ser Phe Asp His Arg Val Val Asp Gly
385                 390                 395                 400

Met Asp Ala Ala Leu Phe Ile Gln Ala Val Arg Gly Leu Leu Glu Gln
                405                 410                 415

Pro Ala Cys Leu Phe Val Glu
            420

<210>  219
<211>  1380
<212>  DNA
<213>  Pseudomonas putida

<400>  219
atgcaacaga ctatccagac aaccctgttg atcatcggcg gcggccctgg cggctatgtg      60

gcggccatcc gcgccgggca actgggcatc cctaccgtgc tggtggaagg ccaggcgctg     120

ggcggtacct gcctgaacat cggctgcatt ccgtccaagg cgctgatcca tgtggccgag     180

cagttccacc aggcctcgcg ctttaccgaa ccctcgccgc tgggcatcag cgtggcttcg     240

ccacgcctgg acatcggcca gagcgtggcc tggaaagacg gcatcgtcga tcgcctgacc     300

actggtgtcg ccgccctgct gaaaaagcac ggggtgaagg tggtgcacgg ctgggccaag     360

gtgcttgatg gcaagcaggt cgaggtggat ggccagcgca tccagtgcga gcacctgttg     420

ctggccacgg gctccagcag tgtcgaactg ccgatgctgc cgttgggtgg gccggtgatt     480

tcctcgaccg aggccctggc accgaaagcc ctgccgcaac acctggtggt ggtgggcggt     540

ggctacatcg gcctggagct gggtatcgcc taccgcaagc tcggcgcgca ggtcagcgtg     600

gtggaagcgc gcgagcgcat cctgccgact tacgacagcg aactgaccgc cccggtggcc     660

gagtcgctga aaaagctggg tatcgccctg caccttggcc acagcgtcga aggttacgaa     720

aatggctgcc tgctggccaa cgatggcaag ggcggacaac tgcgcctgga agccgaccgg     780

gtgctggtgg ccgtgggccg ccgcccacgc accaagggct tcaacctgga atgcctggac     840

ctgaagatga atggtgccgc gattgccatc gacgagcgct gccagaccag catgcacaac     900

gtctgggcca tcggcgacgt ggccggcgaa ccgatgctgg cgcaccgggc catggcccag     960

ggcgagatgg tggccgagat catcgccggc aaggcacgcc gcttcgaacc cgctgcgata 1020

gccgccgtgt gcttcaccga cccggaagtg gtcgtggtcg gcaagacgcc ggaacaggcc 1080

agtcagcaag gcctggactg catcgtcgcg cagttcccgt tcgccgccaa cggccgggcc 1140

atgagcctgg agtcgaaaag cggtttcgtg cgcgtggtcg cgcggcgtga caaccacctg 1200

atcctgggct ggcaagcggt tggcgtggcg gtttccgagc tgtccacggc gtttgcccag 1260

tcgctggaga tgggtgcctg cctggaggat gtggccggta ccatccatgc ccacccgacc 1320

ctgggtgaag cggtacagga agcggcactg cgtgccctgg gccacgccct gcatatctga 1380

<210> 220
<211> 459
<212> PRT
<213> Pseudomonas putida

<400> 220

Met Gln Gln Thr Ile Gln Thr Thr Leu Leu Ile Ile Gly Gly Gly Pro
1               5                   10                  15

Gly Gly Tyr Val Ala Ala Ile Arg Ala Gly Gln Leu Gly Ile Pro Thr
            20                  25                  30

Val Leu Val Glu Gly Gln Ala Leu Gly Gly Thr Cys Leu Asn Ile Gly
        35                  40                  45

Cys Ile Pro Ser Lys Ala Leu Ile His Val Ala Glu Gln Phe His Gln
    50                  55                  60

Ala Ser Arg Phe Thr Glu Pro Ser Pro Leu Gly Ile Ser Val Ala Ser
65                  70                  75                  80

Pro Arg Leu Asp Ile Gly Gln Ser Val Ala Trp Lys Asp Gly Ile Val
                85                  90                  95

Asp Arg Leu Thr Thr Gly Val Ala Ala Leu Leu Lys Lys His Gly Val
            100                 105                 110

Lys Val Val His Gly Trp Ala Lys Val Leu Asp Gly Lys Gln Val Glu
        115                 120                 125

Val Asp Gly Gln Arg Ile Gln Cys Glu His Leu Leu Leu Ala Thr Gly
    130                 135                 140

Ser Ser Ser Val Glu Leu Pro Met Leu Pro Leu Gly Gly Pro Val Ile
145                 150                 155                 160

Ser Ser Thr Glu Ala Leu Ala Pro Lys Ala Leu Pro Gln His Leu Val
                165                 170                 175

Val Val Gly Gly Gly Tyr Ile Gly Leu Glu Leu Gly Ile Ala Tyr Arg
        180                 185                 190

Lys Leu Gly Ala Gln Val Ser Val Val Glu Ala Arg Glu Arg Ile Leu
    195                 200                 205

Pro Thr Tyr Asp Ser Glu Leu Thr Ala Pro Val Ala Glu Ser Leu Lys
    210                 215                 220

Lys Leu Gly Ile Ala Leu His Leu Gly His Ser Val Glu Gly Tyr Glu
225                 230                 235                 240

Asn Gly Cys Leu Leu Ala Asn Asp Gly Lys Gly Gly Gln Leu Arg Leu
                245                 250                 255

Glu Ala Asp Arg Val Leu Val Ala Val Gly Arg Arg Pro Arg Thr Lys
            260                 265                 270

Gly Phe Asn Leu Glu Cys Leu Asp Leu Lys Met Asn Gly Ala Ala Ile
        275                 280                 285

Ala Ile Asp Glu Arg Cys Gln Thr Ser Met His Asn Val Trp Ala Ile
    290                 295                 300

Gly Asp Val Ala Gly Glu Pro Met Leu Ala His Arg Ala Met Ala Gln
305                 310                 315                 320

Gly Glu Met Val Ala Glu Ile Ile Ala Gly Lys Ala Arg Arg Phe Glu
                325                 330                 335

Pro Ala Ala Ile Ala Ala Val Cys Phe Thr Asp Pro Glu Val Val Val
            340                 345                 350

Val Gly Lys Thr Pro Glu Gln Ala Ser Gln Gln Gly Leu Asp Cys Ile
        355                 360                 365

Val Ala Gln Phe Pro Phe Ala Ala Asn Gly Arg Ala Met Ser Leu Glu
    370                 375                 380

Ser Lys Ser Gly Phe Val Arg Val Val Ala Arg Arg Asp Asn His Leu
385                 390                 395                 400

Ile Leu Gly Trp Gln Ala Val Gly Val Ala Val Ser Glu Leu Ser Thr
                405                 410                 415

Ala Phe Ala Gln Ser Leu Glu Met Gly Ala Cys Leu Glu Asp Val Ala
            420                 425                 430

Gly Thr Ile His Ala His Pro Thr Leu Gly Glu Ala Val Gln Glu Ala
        435                 440                 445

Ala Leu Arg Ala Leu Gly His Ala Leu His Ile
    450                 455

<210>  221
<211>  1407
<212>  DNA
<213>  Clostridium beijerinckii

<400>  221
atgaataaag acacactaat acctacaact aaagatttaa aattaaaaac aaatgttgaa      60

aacattaatt taaagaacta caaggataat tcttcatgtt tcggagtatt cgaaaatgtt     120

gaaaatgcta taaacagcgc tgtacacgcg caaaagatat tatcccttca ttatacaaaa     180

gaacaaagag aaaaaatcat aactgagata agaaaggccg cattagaaaa taaagaggtt     240

ttagctacca tgattctgga agaaacacat atgggaaggt atgaagataa aatattaaag     300

catgaattag tagctaaata tactcctggt acagaagatt taactactac tgcttggtca     360

ggtgataatg gtcttacagt tgtagaaatg tctccatatg gcgttatagg tgcaataact     420

ccttctacga atccaactga aactgtaata tgtaatagca tcggcatgat agctgctgga     480

aatgctgtag tatttaacgg acacccaggc gctaaaaaat gtgttgcttt tgctattgaa     540

atgataaata aagcaattat ttcatgtggc ggtcctgaga atttagtaac aactataaaa     600

aatccaacta tggaatccct agatgcaatt attaagcatc ctttaataaa acttctttgc     660

ggaactggag gtccaggaat ggtaaaaacc ctcttaaatt ctggcaagaa agctataggt     720

gctggtgctg gaaatccacc agttattgta gatgataccg ctgatataga aaaggctggt     780

aagagtatca ttgaaggctg ttcttttgat aataatttac cttgtattgc agaaaaagaa     840

gtatttgttt ttgagaatgt tgcagatgat ttaatatcta acatgctaaa aaataatgct     900

gtaattataa atgaagatca agtatcaaaa ttaatagatt tagtattaca aaaaaataat     960

gaaactcaag aatactttat aaacaaaaaa tgggtaggaa aagatgcaaa attattctca    1020

gatgaaatag atgttgagtc tccttcaaat attaaatgca tagtctgcga agtaaatgca    1080

aatcatccat ttgtcatgac agaactcatg atgccaatat taccaattgt aagagttaaa    1140

gatatagatg aagctgttaa atatacaaag atagcagaac aaaatagaaa acatagtgcc    1200

tatatttatt ctaaaaatat agacaaccta aatagatttg aaagagaaat tgatactact    1260

atttttgtaa agaatgctaa atcttttgct ggtgttggtt atgaagctga aggatttaca    1320

actttcacta ttgctggatc tactggtgaa ggcataacct ctgcaagaaa ttttacaaga    1380

caaagaagat gtgtacttgc cggctaa                                        1407

<210>  222

<211>  468
<212>  PRT
<213>  Clostridium beijerinckii

<400>  222

Met Asn Lys Asp Thr Leu Ile Pro Thr Thr Lys Asp Leu Lys Leu Lys
1               5                   10                  15

Thr Asn Val Glu Asn Ile Asn Leu Lys Asn Tyr Lys Asp Asn Ser Ser
            20                  25                  30

Cys Phe Gly Val Phe Glu Asn Val Glu Asn Ala Ile Asn Ser Ala Val
        35                  40                  45

His Ala Gln Lys Ile Leu Ser Leu His Tyr Thr Lys Glu Gln Arg Glu
    50                  55                  60

Lys Ile Ile Thr Glu Ile Arg Lys Ala Ala Leu Glu Asn Lys Glu Val
65                  70                  75                  80

Leu Ala Thr Met Ile Leu Glu Glu Thr His Met Gly Arg Tyr Glu Asp
                85                  90                  95

Lys Ile Leu Lys His Glu Leu Val Ala Lys Tyr Thr Pro Gly Thr Glu
            100                 105                 110

Asp Leu Thr Thr Thr Ala Trp Ser Gly Asp Asn Gly Leu Thr Val Val
        115                 120                 125

Glu Met Ser Pro Tyr Gly Val Ile Gly Ala Ile Thr Pro Ser Thr Asn
    130                 135                 140

Pro Thr Glu Thr Val Ile Cys Asn Ser Ile Gly Met Ile Ala Ala Gly
145                 150                 155                 160

Asn Ala Val Val Phe Asn Gly His Pro Gly Ala Lys Lys Cys Val Ala
                165                 170                 175

Phe Ala Ile Glu Met Ile Asn Lys Ala Ile Ile Ser Cys Gly Gly Pro
            180                 185                 190

Glu Asn Leu Val Thr Thr Ile Lys Asn Pro Thr Met Glu Ser Leu Asp
        195                 200                 205

Ala Ile Ile Lys His Pro Leu Ile Lys Leu Leu Cys Gly Thr Gly Gly
    210                 215                 220

Pro Gly Met Val Lys Thr Leu Leu Asn Ser Gly Lys Lys Ala Ile Gly
225                 230                 235                 240

Ala Gly Ala Gly Asn Pro Pro Val Ile Val Asp Asp Thr Ala Asp Ile
                245                 250                 255

Glu Lys Ala Gly Lys Ser Ile Ile Glu Gly Cys Ser Phe Asp Asn Asn
            260                 265                 270

Leu Pro Cys Ile Ala Glu Lys Glu Val Phe Val Phe Glu Asn Val Ala
        275                 280                 285

Asp Asp Leu Ile Ser Asn Met Leu Lys Asn Asn Ala Val Ile Ile Asn
    290                 295                 300

Glu Asp Gln Val Ser Lys Leu Ile Asp Leu Val Leu Gln Lys Asn Asn
305                 310                 315                 320

Glu Thr Gln Glu Tyr Phe Ile Asn Lys Lys Trp Val Gly Lys Asp Ala
                325                 330                 335

Lys Leu Phe Ser Asp Glu Ile Asp Val Glu Ser Pro Ser Asn Ile Lys
            340                 345                 350

Cys Ile Val Cys Glu Val Asn Ala Asn His Pro Phe Val Met Thr Glu
        355                 360                 365

Leu Met Met Pro Ile Leu Pro Ile Val Arg Val Lys Asp Ile Asp Glu
    370                 375                 380

Ala Val Lys Tyr Thr Lys Ile Ala Glu Gln Asn Arg Lys His Ser Ala
385                 390                 395                 400

Tyr Ile Tyr Ser Lys Asn Ile Asp Asn Leu Asn Arg Phe Glu Arg Glu
                405                 410                 415

Ile Asp Thr Thr Ile Phe Val Lys Asn Ala Lys Ser Phe Ala Gly Val
            420                 425                 430

Gly Tyr Glu Ala Glu Gly Phe Thr Thr Phe Thr Ile Ala Gly Ser Thr
        435                 440                 445

Gly Glu Gly Ile Thr Ser Ala Arg Asn Phe Thr Arg Gln Arg Arg Cys
    450                 455                 460

Val Leu Ala Gly
465

<210>  223
<211>  2589
<212>  DNA
<213>  Clostridium acetobutylicum

<400> 223
atgaaagtca caacagtaaa ggaattagat gaaaaactca aggtaattaa agaagctcaa 60

aaaaaattct cttgttactc gcaagaaatg gttgatgaaa tctttagaaa tgcagcaatg 120

gcagcaatcg acgcaaggat agagctagca aaagcagctg ttttggaaac cggtatgggc 180

ttagttgaag acaaggttat aaaaaatcat tttgcaggcg aatacatcta taacaaatat 240

aaggatgaaa aaacctgcgg tataattgaa cgaaatgaac cctacggaat tacaaaaata 300

gcagaaccta taggagttgt agctgctata atccctgtaa caaaccccac atcaacaaca 360

atatttaaat ccttaatatc ccttaaaact agaaatggaa ttttcttttc gcctcaccca 420

agggcaaaaa aatccacaat actagcagct aaaacaatac ttgatgcagc cgttaagagt 480

ggtgccccgg aaaatataat aggttggata gatgaacctt caattgaact aactcaatat 540

ttaatgcaaa aagcagatat aacccttgca actggtggtc cctcactagt taaatctgct 600

tattcttccg gaaaaccagc aataggtgtt ggtccgggta acaccccagt aataattgat 660

gaatctgctc atataaaaat ggcagtaagt tcaattatat tatccaaaac ctatgataat 720

ggtgttatat gtgcttctga acaatctgta atagtcttaa aatccatata taacaaggta 780

aaagatgagt tccaagaaag aggagcttat ataataaaga aaaacgaatt ggataaagtc 840

cgtgaagtga tttttaaaga tggatccgta aaccctaaaa tagtcggaca gtcagcttat 900

actatagcag ctatggctgg cataaaagta cctaaaacca caagaatatt aataggagaa 960

gttacctcct taggtgaaga agaacctttt gcccacgaaa aactatctcc tgttttggct 1020

atgtatgagg ctgacaattt tgatgatgct ttaaaaaaag cagtaactct aataaactta 1080

ggaggcctcg gccatacctc aggaatatat gcagatgaaa taaaagcacg agataaaata 1140

gatagattta gtagtgccat gaaaaccgta agaacctttg taaatatccc aacctcacaa 1200

ggtgcaagtg gagatctata taattttaga ataccacctt ctttcacgct tggctgcgga 1260

ttttggggag gaaattctgt ttccgagaat gttggtccaa aacatctttt gaatattaaa 1320

accgtagctg aaaggagaga aaacatgctt tggtttagag ttccacataa agtatatttt 1380

aagttcggtt gtcttcaatt tgctttaaaa gatttaaaag atctaaagaa aaaaagagcc 1440

tttatagtta ctgatagtga cccctataat ttaaactatg ttgattcaat aataaaaata 1500

cttgagcacc tagatattga ttttaaagta tttaataagg ttggaagaga agctgatctt 1560

aaaaccataa aaaaagcaac tgaagaaatg tcctccttta tgccagacac tataatagct 1620

ttaggtggta cccctgaaat gagctctgca aagctaatgt gggtactata tgaacatcca 1680

gaagtaaaat ttgaagatct tgcaataaaa tttatggaca taagaaagag aatatatact 1740

ttcccaaaac tcggtaaaaa ggctatgtta gttgcaatta caacttctgc tggttccggt 1800

tctgaggtta ctccttttgc tttagtaact gacaataaca ctggaaataa gtacatgtta 1860

gcagattatg aaatgacacc aaatatggca attgtagatg cagaacttat gatgaaaatg 1920

ccaaagggat taaccgctta ttcaggtata gatgcactag taaatagtat agaagcatac 1980

acatccgtat atgcttcaga atacacaaac ggactagcac tagaggcaat acgattaata 2040

tttaaatatt tgcctgaggc ttacaaaaac ggaagaacca atgaaaaagc aagagagaaa 2100

atggctcacg cttcaactat ggcaggtatg gcatccgcta atgcatttct aggtctatgt 2160

cattccatgg caataaaatt aagttcagaa cacaatattc ctagtggcat tgccaatgca 2220

ttactaatag aagaagtaat aaaatttaac gcagttgata atcctgtaaa acaagcccct 2280

tgcccacaat ataagtatcc aaacaccata tttagatatg ctcgaattgc agattatata 2340

aagcttggag gaaatactga tgaggaaaag gtagatctct taattaacaa aatacatgaa 2400

ctaaaaaaag ctttaaatat accaacttca ataaaggatg caggtgtttt ggaggaaaac 2460

ttctattcct cccttgatag aatatctgaa cttgcactag atgatcaatg cacaggcgct 2520

aatcctagat ttcctcttac aagtgagata aaagaaatgt atataaattg ttttaaaaaa 2580

caaccttaa 2589

<210> 224
<211> 862
<212> PRT
<213> Clostridium acetobutylicum

<400> 224

Met Lys Val Thr Thr Val Lys Glu Leu Asp Glu Lys Leu Lys Val Ile
1               5                   10                  15

Lys Glu Ala Gln Lys Lys Phe Ser Cys Tyr Ser Gln Glu Met Val Asp
            20                  25                  30

Glu Ile Phe Arg Asn Ala Ala Met Ala Ala Ile Asp Ala Arg Ile Glu
        35                  40                  45

Leu Ala Lys Ala Ala Val Leu Glu Thr Gly Met Gly Leu Val Glu Asp
    50                  55                  60

Lys Val Ile Lys Asn His Phe Ala Gly Glu Tyr Ile Tyr Asn Lys Tyr
65                  70                  75                  80

Lys Asp Glu Lys Thr Cys Gly Ile Ile Glu Arg Asn Glu Pro Tyr Gly
                85                  90                  95

Ile Thr Lys Ile Ala Glu Pro Ile Gly Val Val Ala Ala Ile Ile Pro
            100                 105                 110

Val Thr Asn Pro Thr Ser Thr Thr Ile Phe Lys Ser Leu Ile Ser Leu
        115                 120                 125

Lys Thr Arg Asn Gly Ile Phe Phe Ser Pro His Pro Arg Ala Lys Lys
    130                 135                 140

Ser Thr Ile Leu Ala Ala Lys Thr Ile Leu Asp Ala Ala Val Lys Ser
145                 150                 155                 160

Gly Ala Pro Glu Asn Ile Ile Gly Trp Ile Asp Glu Pro Ser Ile Glu
                165                 170                 175

Leu Thr Gln Tyr Leu Met Gln Lys Ala Asp Ile Thr Leu Ala Thr Gly
            180                 185                 190

Gly Pro Ser Leu Val Lys Ser Ala Tyr Ser Ser Gly Lys Pro Ala Ile
        195                 200                 205

Gly Val Gly Pro Gly Asn Thr Pro Val Ile Ile Asp Glu Ser Ala His
    210                 215                 220

Ile Lys Met Ala Val Ser Ser Ile Ile Leu Ser Lys Thr Tyr Asp Asn
225                 230                 235                 240

Gly Val Ile Cys Ala Ser Glu Gln Ser Val Ile Val Leu Lys Ser Ile
                245                 250                 255

Tyr Asn Lys Val Lys Asp Glu Phe Gln Glu Arg Gly Ala Tyr Ile Ile
            260                 265                 270

Lys Lys Asn Glu Leu Asp Lys Val Arg Glu Val Ile Phe Lys Asp Gly
        275                 280                 285

Ser Val Asn Pro Lys Ile Val Gly Gln Ser Ala Tyr Thr Ile Ala Ala
    290                 295                 300

Met Ala Gly Ile Lys Val Pro Lys Thr Thr Arg Ile Leu Ile Gly Glu
305                 310                 315                 320

Val Thr Ser Leu Gly Glu Glu Glu Pro Phe Ala His Glu Lys Leu Ser
                325                 330                 335

Pro Val Leu Ala Met Tyr Glu Ala Asp Asn Phe Asp Asp Ala Leu Lys
            340                 345                 350

Lys Ala Val Thr Leu Ile Asn Leu Gly Gly Leu Gly His Thr Ser Gly
        355                 360                 365

Ile Tyr Ala Asp Glu Ile Lys Ala Arg Asp Lys Ile Asp Arg Phe Ser
    370                 375                 380

Ser Ala Met Lys Thr Val Arg Thr Phe Val Asn Ile Pro Thr Ser Gln

385                 390                 395                 400

Gly Ala Ser Gly Asp Leu Tyr Asn Phe Arg Ile Pro Pro Ser Phe Thr
                405                 410                 415

Leu Gly Cys Gly Phe Trp Gly Gly Asn Ser Val Ser Glu Asn Val Gly
            420                 425                 430

Pro Lys His Leu Leu Asn Ile Lys Thr Val Ala Glu Arg Arg Glu Asn
        435                 440                 445

Met Leu Trp Phe Arg Val Pro His Lys Val Tyr Phe Lys Phe Gly Cys
    450                 455                 460

Leu Gln Phe Ala Leu Lys Asp Leu Lys Asp Leu Lys Lys Lys Arg Ala
465                 470                 475                 480

Phe Ile Val Thr Asp Ser Asp Pro Tyr Asn Leu Asn Tyr Val Asp Ser
                485                 490                 495

Ile Ile Lys Ile Leu Glu His Leu Asp Ile Asp Phe Lys Val Phe Asn
            500                 505                 510

Lys Val Gly Arg Glu Ala Asp Leu Lys Thr Ile Lys Lys Ala Thr Glu
        515                 520                 525

Glu Met Ser Ser Phe Met Pro Asp Thr Ile Ile Ala Leu Gly Gly Thr
    530                 535                 540

Pro Glu Met Ser Ser Ala Lys Leu Met Trp Val Leu Tyr Glu His Pro
545                 550                 555                 560

Glu Val Lys Phe Glu Asp Leu Ala Ile Lys Phe Met Asp Ile Arg Lys
                565                 570                 575

Arg Ile Tyr Thr Phe Pro Lys Leu Gly Lys Lys Ala Met Leu Val Ala
            580                 585                 590

Ile Thr Thr Ser Ala Gly Ser Gly Ser Glu Val Thr Pro Phe Ala Leu
        595                 600                 605

Val Thr Asp Asn Asn Thr Gly Asn Lys Tyr Met Leu Ala Asp Tyr Glu
    610                 615                 620

Met Thr Pro Asn Met Ala Ile Val Asp Ala Glu Leu Met Met Lys Met
625                 630                 635                 640

Pro Lys Gly Leu Thr Ala Tyr Ser Gly Ile Asp Ala Leu Val Asn Ser
                645                 650                 655

Ile Glu Ala Tyr Thr Ser Val Tyr Ala Ser Glu Tyr Thr Asn Gly Leu
            660                 665                 670

Ala Leu Glu Ala Ile Arg Leu Ile Phe Lys Tyr Leu Pro Glu Ala Tyr
        675                 680                 685

Lys Asn Gly Arg Thr Asn Glu Lys Ala Arg Glu Lys Met Ala His Ala
    690                 695                 700

Ser Thr Met Ala Gly Met Ala Ser Ala Asn Ala Phe Leu Gly Leu Cys
705                 710                 715                 720

His Ser Met Ala Ile Lys Leu Ser Ser Glu His Asn Ile Pro Ser Gly
                725                 730                 735

Ile Ala Asn Ala Leu Leu Ile Glu Glu Val Ile Lys Phe Asn Ala Val
            740                 745                 750

Asp Asn Pro Val Lys Gln Ala Pro Cys Pro Gln Tyr Lys Tyr Pro Asn
        755                 760                 765

Thr Ile Phe Arg Tyr Ala Arg Ile Ala Asp Tyr Ile Lys Leu Gly Gly
    770                 775                 780

Asn Thr Asp Glu Glu Lys Val Asp Leu Leu Ile Asn Lys Ile His Glu
785                 790                 795                 800

Leu Lys Lys Ala Leu Asn Ile Pro Thr Ser Ile Lys Asp Ala Gly Val
                805                 810                 815

Leu Glu Glu Asn Phe Tyr Ser Ser Leu Asp Arg Ile Ser Glu Leu Ala
            820                 825                 830

Leu Asp Asp Gln Cys Thr Gly Ala Asn Pro Arg Phe Pro Leu Thr Ser
        835                 840                 845

Glu Ile Lys Glu Met Tyr Ile Asn Cys Phe Lys Lys Gln Pro
    850                 855                 860

<210>  225
<211>  2577
<212>  DNA
<213>  Clostridium acetobutylicum

<400>  225
   atgaaagtta caaatcaaaa agaactaaaa caaaagctaa atgaattgag agaagcgcaa      60
   aagaagtttg caacctatac tcaagagcaa gttgataaaa tttttaaaca atgtgccata     120
   gccgcagcta aagaaagaat aaacttagct aaattagcag tagaagaaac aggaataggt     180
   cttgtagaag ataaaattat aaaaaatcat tttgcagcag aatatatata caataaatat 240
   aaaaatgaaa aaacttgtgg cataatagac catgacgatt ctttaggcat aacaaaggtt 300
   gctgaaccaa ttggaattgt tgcagccata gttcctacta ctaatccaac ttccacagca 360
   attttcaaat cattaatttc tttaaaaaca agaaacgcaa tattcttttc accacatcca 420
   cgtgcaaaaa aatctacaat tgctgcagca aaattaattt tagatgcagc tgttaaagca 480
   ggagcaccta aaaatataat aggctggata gatgagccat caatagaact ttctcaagat 540
   ttgatgagtg aagctgatat aatattagca acaggaggtc cttcaatggt taaagcggcc 600
   tattcatctg gaaaacctgc aattggtgtt ggagcaggaa atacaccagc aataatagat 660
   gagagtgcag atatagatat ggcagtaagc tccataattt tatcaaagac ttatgacaat 720
   ggagtaatat gcgcttctga acaatcaata ttagttatga attcaatata cgaaaaagtt 780
   aaagaggaat ttgtaaaacg aggatcatat atactcaatc aaaatgaaat agctaaaata 840
   aaagaaacta tgtttaaaaa tggagctatt aatgctgaca tagttggaaa atctgcttat 900
   ataattgcta aaatggcagg aattgaagtt cctcaaacta caaagatact tataggcgaa 960
   gtacaatctg ttgaaaaaag cgagctgttc tcacatgaaa aactatcacc agtacttgca 1020
   atgtataaag ttaaggattt tgatgaagct ctaaaaaagg cacaaaggct aatagaatta 1080
   ggtggaagtg gacacacgtc atctttatat atagattcac aaaacaataa ggataaagtt 1140
   aaagaatttg gattagcaat gaaaacttca aggacattta ttaacatgcc ttcttcacag 1200
   ggagcaagcg gagatttata caattttgcg atagcaccat catttactct tggatgcggc 1260
   acttggggag gaaactctgt atcgcaaaat gtagagccta aacatttatt aaatattaaa 1320
   agtgttgctg aaagaaggga aaatatgctt tggtttaaag tgccacaaaa aatatatttt 1380
   aaatatggat gtcttagatt tgcattaaaa gaattaaaag atatgaataa gaaaagagcc 1440
   tttatagtaa cagataaaga tctttttaaa cttggatatg ttaataaaat aacaaaggta 1500
   ctagatgaga tagatattaa atacagtata tttacagata ttaaatctga tccaactatt 1560
   gattcagtaa aaaaaggtgc taaagaaatg cttaactttg aacctgatac tataatctct 1620
   attggtggtg gatcgccaat ggatgcagca aaggttatgc acttgttata tgaatatcca 1680
   gaagcagaaa ttgaaaatct agctataaac tttatggata taagaaagag aatatgcaat 1740
   ttccctaaat taggtacaaa ggcgatttca gtagctattc ctacaactgc tggtaccggt 1800
   tcagaggcaa caccttttgc agttataact aatgatgaaa caggaatgaa atacccttta 1860
   acttcttatg aattgacccc aaacatggca ataatagata ctgaattaat gttaaatatg 1920
   cctagaaaat taacagcagc aactggaata gatgcattag ttcatgctat agaagcatat 1980
   gtttcggtta tggctacgga ttatactgat gaattagcct taagagcaat aaaaatgata 2040
   tttaaatatt tgcctagagc ctataaaaat gggactaacg acattgaagc aagagaaaaa 2100
   atggcacatg cctctaatat tgcggggatg gcatttgcaa atgctttctt aggtgtatgc 2160
   cattcaatgg ctcataaact tggggcaatg catcacgttc cacatggaat tgcttgtgct 2220
   gtattaatag aagaagttat taaatataac gctacagact gtccaacaaa gcaaacagca 2280
   ttccctcaat ataaatctcc taatgctaag agaaaatatg ctgaaattgc agagtatttg 2340
   aatttaaagg gtactagcga taccgaaaag gtaacagcct taatagaagc tatttcaaag 2400
   ttaaagatag atttgagtat tccacaaaat ataagtgccg ctggaataaa taaaaaagat 2460
   ttttataata cgctagataa aatgtcagag cttgcttttg atgaccaatg tacaacagct 2520
   aatcctaggt atccacttat aagtgaactt aaggatatct atataaaatc attttaa 2577

<210> 226
<211> 800
<212> PRT
<213> Clostridium acetobutylicum

<400> 226

Met Lys Val Thr Asn Gln Lys Glu Leu Lys Gln Lys Leu Asn Glu Leu
1               5                   10                  15

Arg Glu Ala Gln Lys Lys Phe Ala Thr Tyr Thr Gln Glu Gln Val Asp
            20                  25                  30

Lys Ile Phe Lys Gln Cys Ala Ile Ala Ala Ala Lys Glu Arg Ile Asn
        35                  40                  45

Leu Ala Lys Leu Ala Val Glu Glu Thr Gly Ile Gly Leu Val Glu Asp
    50                  55                  60

Lys Ile Ile Lys Asn His Phe Ala Ala Glu Tyr Ile Tyr Asn Lys Tyr
65                  70                  75                  80

Lys Asn Glu Lys Thr Cys Gly Ile Ile Asp His Asp Asp Ser Leu Gly
                85                  90                  95

Ile Thr Lys Val Ala Glu Pro Ile Gly Ile Val Ala Ala Ile Val Pro
            100                 105                 110

Thr Thr Asn Pro Thr Ser Thr Ala Ile Phe Lys Ser Leu Ile Ser Leu
        115                 120                 125

Lys Thr Arg Asn Ala Ile Phe Phe Ser Pro His Pro Arg Ala Lys Lys
    130                 135                 140

Ser Thr Ile Ala Ala Ala Lys Leu Ile Leu Asp Ala Ala Val Lys Ala
145                 150                 155                 160

Gly Ala Pro Lys Asn Ile Ile Gly Trp Ile Asp Glu Pro Ser Ile Glu

                165                 170                 175

Leu Ser Gln Asp Leu Met Ser Glu Ala Asp Ile Ile Leu Ala Thr Gly
            180                 185                 190

Gly Pro Ser Met Val Lys Ala Ala Tyr Ser Ser Gly Lys Pro Ala Ile
        195                 200                 205

Gly Val Gly Ala Gly Asn Thr Pro Ala Ile Ile Asp Glu Ser Ala Asp
    210                 215                 220

Ile Asp Met Ala Val Ser Ser Ile Ile Leu Ser Lys Thr Tyr Asp Asn
225                 230                 235                 240

Gly Val Ile Cys Ala Ser Glu Gln Ser Ile Leu Val Met Asn Ser Ile
                245                 250                 255

Tyr Glu Lys Val Lys Glu Glu Phe Val Lys Arg Gly Ser Tyr Ile Leu
            260                 265                 270

Asn Gln Asn Glu Ile Ala Lys Ile Lys Glu Thr Met Phe Lys Asn Gly
        275                 280                 285

Ala Ile Asn Ala Asp Ile Val Gly Lys Ser Ala Tyr Ile Ile Ala Lys
    290                 295                 300

Met Ala Gly Ile Glu Val Pro Gln Thr Thr Lys Ile Leu Ile Gly Glu
305                 310                 315                 320

Val Gln Ser Val Glu Lys Ser Glu Leu Phe Ser His Glu Lys Leu Ser
                325                 330                 335

Pro Val Leu Ala Met Tyr Lys Val Lys Asp Phe Asp Glu Ala Leu Lys
            340                 345                 350

Lys Ala Gln Arg Leu Ile Glu Leu Gly Gly Ser Gly His Thr Ser Ser
        355                 360                 365

Leu Tyr Ile Asp Ser Gln Asn Asn Lys Asp Lys Val Lys Glu Phe Gly
    370                 375                 380

Leu Ala Met Lys Thr Ser Arg Thr Phe Ile Asn Met Pro Ser Ser Gln
385                 390                 395                 400

Gly Ala Ser Gly Asp Leu Tyr Asn Phe Ala Ile Ala Pro Ser Phe Thr
                405                 410                 415

Leu Gly Cys Gly Thr Trp Gly Gly Asn Ser Val Ser Gln Asn Val Glu
            420                 425                 430

Pro Lys His Leu Leu Asn Ile Lys Ser Val Ala Glu Arg Arg Glu Asn
        435                 440                 445

Met Leu Trp Phe Lys Val Pro Gln Lys Ile Tyr Phe Lys Tyr Gly Cys
    450                 455                 460

Leu Arg Phe Ala Leu Lys Glu Leu Lys Asp Met Asn Lys Lys Arg Ala
465                 470                 475                 480

Phe Ile Val Thr Asp Lys Asp Leu Phe Lys Leu Gly Tyr Val Asn Lys
                485                 490                 495

Ile Thr Lys Val Leu Asp Glu Ile Asp Ile Lys Tyr Ser Ile Phe Thr
            500                 505                 510

Asp Ile Lys Ser Asp Pro Thr Ile Asp Ser Val Lys Lys Gly Ala Lys
        515                 520                 525

Glu Met Leu Asn Phe Glu Pro Asp Thr Ile Ile Ser Ile Gly Gly Gly
    530                 535                 540

Ser Pro Met Asp Ala Ala Lys Val Met His Leu Leu Tyr Glu Tyr Pro
545                 550                 555                 560

Glu Ala Glu Ile Glu Asn Leu Ala Ile Asn Phe Met Asp Ile Arg Lys
                565                 570                 575

Arg Ile Cys Asn Phe Pro Lys Leu Gly Thr Lys Ala Ile Ser Val Ala
            580                 585                 590

Ile Pro Thr Thr Ala Gly Thr Gly Ser Glu Ala Thr Pro Phe Ala Val
        595                 600                 605

Ile Thr Asn Asp Glu Thr Gly Met Lys Tyr Pro Leu Thr Ser Tyr Glu
    610                 615                 620

Leu Thr Pro Asn Met Ala Ile Ile Asp Thr Glu Leu Met Leu Asn Met
625                 630                 635                 640

Pro Arg Lys Leu Thr Ala Ala Thr Gly Ile Asp Ala Leu Val His Ala
                645                 650                 655

Ile Glu Ala Tyr Val Ser Val Met Ala Thr Asp Tyr Thr Asp Glu Leu
            660                 665                 670

Ala Leu Arg Ala Ile Lys Met Ile Phe Lys Tyr Leu Pro Arg Ala Tyr
        675                 680                 685

Lys Asn Gly Thr Asn Asp Ile Glu Ala Arg Glu Lys Met Ala His Ala
    690                 695                 700

Ser Asn Ile Ala Gly Met Ala Phe Ala Asn Ala Phe Leu Gly Val Cys
705                 710                 715                 720

His Ser Met Ala His Lys Leu Gly Ala Met His His Val Pro His Gly
                725                 730                 735

Ile Ala Cys Ala Val Leu Ile Glu Glu Val Ile Lys Tyr Asn Ala Thr
            740                 745                 750

Asp Cys Pro Thr Lys Gln Thr Ala Phe Pro Gln Tyr Lys Ser Pro Asn
        755                 760                 765

Ala Lys Arg Lys Tyr Ala Glu Ile Ala Glu Tyr Leu Asn Leu Lys Gly
    770                 775                 780

Thr Ser Asp Thr Glu Lys Val Thr Ala Leu Ile Glu Ala Ile Ser Lys
785                 790                 795                 800

<210>  227
<211>  924
<212>  DNA
<213>  Pseudomonas putida

<400>  227
atgagcaaga aactcaaggc ggccatcata ggccccggca atatcggtac cgatctggtg      60

atgaagatgc tccgttccga gtggattgag ccggtgtgga tggtcggcat cgaccccaac     120

tccgacggcc tcaaacgcgc ccgcgatttc ggcatgaaga ccacagccga aggcgtcgac     180

ggcctgctcc cgcacgtgct ggacgacgac atccgcatcg ccttcgacgc cacctcggcc     240

tatgtgcatg ccgagaatag ccgcaagctc aacgcgcttg gcgtgctgat ggtcgacctg     300

accccggcgg ccatcggccc ctactgcgtg ccgccggtca acctcaagca gcatgtcggc     360

cgcctggaaa tgaacgtcaa catggtcacc tgcggcggcc aggccaccat ccccatggtc     420

gccgcggtgt cccgcgtgca gccggtggcc tacgccgaga tcgtcgccac cgtctcctcg     480

cgctcggtcg gcccgggcac gcgcaagaac atcgacgagt tcacccgcac caccgccggc     540

gccatcgagc aggtcggcgg cgccagggaa ggcaaggcga tcatcgtcat caacccggcc     600

gagccgccgc tgatgatgcg cgacaccatc cactgcctga ccgacagcga gccggaccag     660

gctgcgatca ccgcttcggt tcacgcgatg atcgccgagg tgcagaaata cgtgcccggc     720

taccgcctga agaacggccc ggtgttcgac ggcaaccgcg tgtcgatctt catggaagtc     780

gaaggcctgg gcgactacct gcccaagtac gccggcaacc tcgacatcat gaccgccgcc     840

gcgctgcgta ccggcgagat gttcgccgag gaaatcgccg ccggcaccat tcaactgccg     900

cgtcgcgaca tcgcgctggc ttga                                          924

<210>  228
<211>  307
<212>  PRT
<213>  Pseudomonas putida

<400>  228

Met Ser Lys Lys Leu Lys Ala Ala Ile Ile Gly Pro Gly Asn Ile Gly
1               5                   10                  15

Thr Asp Leu Val Met Lys Met Leu Arg Ser Glu Trp Ile Glu Pro Val
            20                  25                  30

Trp Met Val Gly Ile Asp Pro Asn Ser Asp Gly Leu Lys Arg Ala Arg
        35                  40                  45

Asp Phe Gly Met Lys Thr Thr Ala Glu Gly Val Asp Gly Leu Leu Pro
    50                  55                  60

His Val Leu Asp Asp Asp Ile Arg Ile Ala Phe Asp Ala Thr Ser Ala
65                  70                  75                  80

Tyr Val His Ala Glu Asn Ser Arg Lys Leu Asn Ala Leu Gly Val Leu
                85                  90                  95

Met Val Asp Leu Thr Pro Ala Ala Ile Gly Pro Tyr Cys Val Pro Pro
            100                 105                 110

Val Asn Leu Lys Gln His Val Gly Arg Leu Glu Met Asn Val Asn Met
        115                 120                 125

Val Thr Cys Gly Gly Gln Ala Thr Ile Pro Met Val Ala Ala Val Ser
    130                 135                 140

Arg Val Gln Pro Val Ala Tyr Ala Glu Ile Val Ala Thr Val Ser Ser
145                 150                 155                 160

Arg Ser Val Gly Pro Gly Thr Arg Lys Asn Ile Asp Glu Phe Thr Arg
                165                 170                 175

Thr Thr Ala Gly Ala Ile Glu Gln Val Gly Gly Ala Arg Glu Gly Lys
            180                 185                 190

Ala Ile Ile Val Ile Asn Pro Ala Glu Pro Pro Leu Met Met Arg Asp
        195                 200                 205

Thr Ile His Cys Leu Thr Asp Ser Glu Pro Asp Gln Ala Ala Ile Thr
    210                 215                 220

Ala Ser Val His Ala Met Ile Ala Glu Val Gln Lys Tyr Val Pro Gly
225                 230                 235                 240

Tyr Arg Leu Lys Asn Gly Pro Val Phe Asp Gly Asn Arg Val Ser Ile
                245                 250                 255

Phe Met Glu Val Glu Gly Leu Gly Asp Tyr Leu Pro Lys Tyr Ala Gly
            260                 265                 270

Asn Leu Asp Ile Met Thr Ala Ala Ala Leu Arg Thr Gly Glu Met Phe
        275                 280                 285

Ala Glu Glu Ile Ala Ala Gly Thr Ile Gln Leu Pro Arg Arg Asp Ile
    290                 295                 300

Ala Leu Ala
305

<210>  229
<211>  924
<212>  DNA
<213>  Thermus thermophilus

<400>  229
atgtccgaaa gggttaaggt agccatcctg ggctccggca acatcgggac ggacctgatg      60

tacaagctcc tgaagaaccc gggccacatg gagcttgtgg cggtggtggg gatagacccc     120

aagtccgagg gcctggcccg ggcgcgggcc ttagggttag aggcgagcca cgaagggatc     180

gcctacatcc tggagaggcc ggagatcaag atcgtctttg acgccaccag cgccaaggcc     240

cacgtgcgcc acgccaagct cctgagggag gcggggaaga tcgccataga cctcacgccg     300

gcggcccggg gcccttacgt ggtgcccccg gtgaacctga aggaacacct ggacaaggac     360

aacgtgaacc tcatcacctg cggggggcag gccaccatcc ccctggtcta cgcggtgcac     420

cgggtggccc ccgtgctcta cgcggagatg gtctccacgg tggcctcccg ctccgcgggc     480

cccggcaccc ggcagaacat cgacgagttc accttcacca ccgcccgggg cctggaggcc     540

atcggggggg ccaagaaggg gaaggccatc atcatcctga acccggcgga accccccatc     600

ctcatgacca acaccgtgcg ctgcatcccc gaggacgagg gctttgaccg ggaggccgtg     660

gtggcgagcg tccgggccat ggagcgggag gtccaggcct acgtgcccgg ctaccgcctg     720

aaggcggacc cggtgtttga gaggcttccc accccctggg gggagcgcac cgtggtctcc     780

atgctcctgg aggtggaggg ggcgggggac tatttgccca aatacgccgg caacctggac     840

atcatgacgg cttctgcccg gagggtgggg gaggtcttcg cccagcacct cctggggaag     900

cccgtggagg aggtggtggc gtga                                            924

<210>  230

<211>  307
<212>  PRT
<213>  Thermus thermophilus

<400>  230

Met Ser Glu Arg Val Lys Val Ala Ile Leu Gly Ser Gly Asn Ile Gly
1               5                   10                  15

Thr Asp Leu Met Tyr Lys Leu Leu Lys Asn Pro Gly His Met Glu Leu
            20                  25                  30

Val Ala Val Val Gly Ile Asp Pro Lys Ser Glu Gly Leu Ala Arg Ala
        35                  40                  45

Arg Ala Leu Gly Leu Glu Ala Ser His Glu Gly Ile Ala Tyr Ile Leu
    50                  55                  60

Glu Arg Pro Glu Ile Lys Ile Val Phe Asp Ala Thr Ser Ala Lys Ala
65                  70                  75                  80

His Val Arg His Ala Lys Leu Leu Arg Glu Ala Gly Lys Ile Ala Ile
                85                  90                  95

Asp Leu Thr Pro Ala Ala Arg Gly Pro Tyr Val Val Pro Pro Val Asn
            100                 105                 110

Leu Lys Glu His Leu Asp Lys Asp Asn Val Asn Leu Ile Thr Cys Gly
        115                 120                 125

Gly Gln Ala Thr Ile Pro Leu Val Tyr Ala Val His Arg Val Ala Pro
    130                 135                 140

Val Leu Tyr Ala Glu Met Val Ser Thr Val Ala Ser Arg Ser Ala Gly
145                 150                 155                 160

Pro Gly Thr Arg Gln Asn Ile Asp Glu Phe Thr Phe Thr Thr Ala Arg
                165                 170                 175

Gly Leu Glu Ala Ile Gly Gly Ala Lys Lys Gly Lys Ala Ile Ile Ile
            180                 185                 190

Leu Asn Pro Ala Glu Pro Pro Ile Leu Met Thr Asn Thr Val Arg Cys
        195                 200                 205

Ile Pro Glu Asp Glu Gly Phe Asp Arg Glu Ala Val Val Ala Ser Val
    210                 215                 220

Arg Ala Met Glu Arg Glu Val Gln Ala Tyr Val Pro Gly Tyr Arg Leu
225                 230                 235                 240

Lys Ala Asp Pro Val Phe Glu Arg Leu Pro Thr Pro Trp Gly Glu Arg
                245                 250                 255

Thr Val Val Ser Met Leu Leu Glu Val Glu Gly Ala Gly Asp Tyr Leu
            260                 265                 270

Pro Lys Tyr Ala Gly Asn Leu Asp Ile Met Thr Ala Ser Ala Arg Arg
        275                 280                 285

Val Gly Glu Val Phe Ala Gln His Leu Leu Gly Lys Pro Val Glu Glu
    290                 295                 300

Val Val Ala
305

<210>  231
<211>  1254
<212>  DNA
<213>  Escherichia coli

<400>  231
atgacattct ccctttttgg tgacaaattt acccgccact ccggcattac gctgttgatg      60

gaagatctga acgacggttt acgcacgcct ggcgcgatta tgctcggcgg cggtaatccg     120

gcgcagatcc cggaaatgca ggactacttc cagacgctac tgaccgacat gctggaaagt     180

ggcaaagcga ctgatgcact gtgtaactac gacggtccac aggggaaaac ggagctactc     240

acactgcttg ccggaatgct gcgcgagaag ttgggttggg atatcgaacc acagaatatt     300

gcactaacaa acggcagcca gagcgcgttt ttctacttat ttaacctgtt tgccggacgc     360

cgtgccgatg gtcgggtcaa aaaagtgctg ttcccgcttg caccggaata cattggctat     420

gctgacgccg gactggaaga agatctgttt gtctctgcgc gtccgaatat tgaactgctg     480

ccggaaggcc agtttaaata ccacgtcgat tttgagcatc tgcatattgg cgaagaaacc     540

gggatgattt gcgtctcccg gccgacgaat ccaacaggca atgtgattac tgacgaagag     600

ttgctgaagc ttgacgcgct ggcgaatcaa cacggcattc cgctggtgat tgataacgct     660

tatggcgtcc cgttcccggg tatcatcttc agtgaagcgc gcccgctatg gaatccgaat     720

atcgtgctgt gcatgagtct ttccaagctg ggtctacctg gctcccgctg cggcattatc     780

atcgccaatg aaaaaatcat caccgccatc accaatatga acggcattat cagcctggca     840

cctggcggta ttggtccggc gatgatgtgt gaaatgatta agcgtaacga tctgctgcgc     900

ctgtctgaaa cagtcatcaa accgttttac taccagcgtg ttcaggaaac tatcgccatc     960

attcgccgct atttaccgga aaatcgctgc ctgattcata aaccggaagg agccattttc    1020

ctctggctat ggtttaagga tttgcccatt acgaccaagc agctctatca gcgcctgaaa    1080

gcacgcggcg tgctgatggt gccggggcac aacttcttcc cagggctgga taaaccgtgg    1140

ccgcatacgc atcaatgtat gcgcatgaac tacgtaccag agccggagaa aattgaggcg 1200

ggggtgaaga ttctggcgga agagatagaa agagcctggg ctgaaagtca ctaa 1254

<210> 232
<211> 417
<212> PRT
<213> Escherichia coli

<400> 232

Met Thr Phe Ser Leu Phe Gly Asp Lys Phe Thr Arg His Ser Gly Ile
1               5                   10                  15

Thr Leu Leu Met Glu Asp Leu Asn Asp Gly Leu Arg Thr Pro Gly Ala
            20                  25                  30

Ile Met Leu Gly Gly Gly Asn Pro Ala Gln Ile Pro Glu Met Gln Asp
        35                  40                  45

Tyr Phe Gln Thr Leu Leu Thr Asp Met Leu Glu Ser Gly Lys Ala Thr
    50                  55                  60

Asp Ala Leu Cys Asn Tyr Asp Gly Pro Gln Gly Lys Thr Glu Leu Leu
65                  70                  75                  80

Thr Leu Leu Ala Gly Met Leu Arg Glu Lys Leu Gly Trp Asp Ile Glu
                85                  90                  95

Pro Gln Asn Ile Ala Leu Thr Asn Gly Ser Gln Ser Ala Phe Phe Tyr
            100                 105                 110

Leu Phe Asn Leu Phe Ala Gly Arg Arg Ala Asp Gly Arg Val Lys Lys
        115                 120                 125

Val Leu Phe Pro Leu Ala Pro Glu Tyr Ile Gly Tyr Ala Asp Ala Gly
    130                 135                 140

Leu Glu Glu Asp Leu Phe Val Ser Ala Arg Pro Asn Ile Glu Leu Leu
145                 150                 155                 160

Pro Glu Gly Gln Phe Lys Tyr His Val Asp Phe Glu His Leu His Ile
                165                 170                 175

Gly Glu Glu Thr Gly Met Ile Cys Val Ser Arg Pro Thr Asn Pro Thr
            180                 185                 190

Gly Asn Val Ile Thr Asp Glu Glu Leu Leu Lys Leu Asp Ala Leu Ala
        195                 200                 205

Asn Gln His Gly Ile Pro Leu Val Ile Asp Asn Ala Tyr Gly Val Pro

210                 215                 220

Phe Pro Gly Ile Ile Phe Ser Glu Ala Arg Pro Leu Trp Asn Pro Asn
225                 230                 235                 240

Ile Val Leu Cys Met Ser Leu Ser Lys Leu Gly Leu Pro Gly Ser Arg
                245                 250                 255

Cys Gly Ile Ile Ile Ala Asn Glu Lys Ile Ile Thr Ala Ile Thr Asn
            260                 265                 270

Met Asn Gly Ile Ile Ser Leu Ala Pro Gly Gly Ile Gly Pro Ala Met
        275                 280                 285

Met Cys Glu Met Ile Lys Arg Asn Asp Leu Leu Arg Leu Ser Glu Thr
    290                 295                 300

Val Ile Lys Pro Phe Tyr Tyr Gln Arg Val Gln Glu Thr Ile Ala Ile
305                 310                 315                 320

Ile Arg Arg Tyr Leu Pro Glu Asn Arg Cys Leu Ile His Lys Pro Glu
                325                 330                 335

Gly Ala Ile Phe Leu Trp Leu Trp Phe Lys Asp Leu Pro Ile Thr Thr
            340                 345                 350

Lys Gln Leu Tyr Gln Arg Leu Lys Ala Arg Gly Val Leu Met Val Pro
        355                 360                 365

Gly His Asn Phe Phe Pro Gly Leu Asp Lys Pro Trp Pro His Thr His
    370                 375                 380

Gln Cys Met Arg Met Asn Tyr Val Pro Glu Pro Glu Lys Ile Glu Ala
385                 390                 395                 400

Gly Val Lys Ile Leu Ala Glu Glu Ile Glu Arg Ala Trp Ala Glu Ser
                405                 410                 415

His

<210>  233
<211>  1278
<212>  DNA
<213>  Bacillus licheniformis

<400>  233
ttataagtat tcaacctgtt tctcatatac acccttcgca attttagcta aaacatcgat      60

tccccttata atatcttcat ccgccgcggt taggctgatt cgtatacact ggtgtgaatg     120

cgccaggcgc cgggattgac ggtgaaagaa agatgatccg ggaacgataa tgactccatc 180

cgctttcata tactcataca gcgctgcatc ggtcaccggc aggtcttcaa accacagcca 240

tccgaaaagc gatccttccc cttgatgcag ataccatttg atgtcttcag gcatcttgca 300

taaaagcgtt tccttgagca gcatgaattt attgcggtaa tatggcctga cttcattcag 360

cgacacgtcg gcgaggcgcc cgtcattcaa tactgatgca gccatatact gccccagcct 420

tgaagaatgg atcgccgcat tcgactgaaa agcttccatt gcctgaatat accgggacgg 480

cccgatggcg attccgatcc tttcgccagg caggccggct tttgaaaggc tcatacagtg 540

aatgatctgc tcgttgaaaa tcggttccat gtcgataaag tgaatcgccg gaaaaggcgg 600

agcatatgcg gaatcaatga acagcggaac attcgcttct cggcatgcgt ctgaaatgaa 660

tgctacatct tctttaggca agatgtttcc gcaaggattg ttcgggcgcg atagcaagac 720

agcaccgatg cgcatcctct ctaaaaaccc cttacggtcg agctcatatc gaaacgtatg 780

atcatccaat ttcgatatga gcggagggat cccctcaatc atctcccgct ccagtgccgc 840

cccgctgtat cccgaatagt caggcagcat cgggatcaag gcttttttca tcacagatcc 900

gcttcccatt ccgcaaaacg aattgatcgc cagaaaaaac agctgctggc ttccggctgt 960

aatcaacacg ttctcttttc gaatgccggc gctataccgc tctgaaaaga agcggacaac 1020

acttgcaatc agttcatcgg ttccatagct cgatccgtat tggccgatca ccgaagaaaa 1080

cctgtcatcg tcaaggagat cggcaagagc cgacttccac atggctgaca cgccgggcaa 1140

aatcatcgga ttgcccgcac ttaaattaat gtatgaccgt tcaccgccgg ccaggacttc 1200

ctgaatatcg ctcatcacag ccctgacccc tgttttctca atcattttct ctccgatttt 1260

gcttaatggc ggcttcac 1278

<210> 234
<211> 425
<212> PRT
<213> Bacillus licheniformis

<400> 234

Met Lys Pro Pro Leu Ser Lys Ile Gly Glu Lys Met Ile Glu Lys Thr
1               5                   10                  15

Gly Val Arg Ala Val Met Ser Asp Ile Gln Glu Val Leu Ala Gly Gly
            20                  25                  30

Glu Arg Ser Tyr Ile Asn Leu Ser Ala Gly Asn Pro Met Ile Leu Pro
        35                  40                  45

Gly Val Ser Ala Met Trp Lys Ser Ala Leu Ala Asp Leu Leu Asp Asp
    50                  55                  60

Asp Arg Phe Ser Ser Val Ile Gly Gln Tyr Gly Ser Ser Tyr Gly Thr

65                  70                  75                  80

Asp Glu Leu Ile Ala Ser Val Val Arg Phe Phe Ser Glu Arg Tyr Ser
                85                  90                  95

Ala Gly Ile Arg Lys Glu Asn Val Leu Ile Thr Ala Gly Ser Gln Gln
            100                 105                 110

Leu Phe Phe Leu Ala Ile Asn Ser Phe Cys Gly Met Gly Ser Gly Ser
        115                 120                 125

Val Met Lys Lys Ala Leu Ile Pro Met Leu Pro Asp Tyr Ser Gly Tyr
    130                 135                 140

Ser Gly Ala Ala Leu Glu Arg Glu Met Ile Glu Gly Ile Pro Pro Leu
145                 150                 155                 160

Ile Ser Lys Leu Asp Asp His Thr Phe Arg Tyr Glu Leu Asp Arg Lys
                165                 170                 175

Gly Phe Leu Glu Arg Met Arg Ile Gly Ala Val Leu Leu Ser Arg Pro
            180                 185                 190

Asn Asn Pro Cys Gly Asn Ile Leu Pro Lys Glu Asp Val Ala Phe Ile
        195                 200                 205

Ser Asp Ala Cys Arg Glu Ala Asn Val Pro Leu Phe Ile Asp Ser Ala
    210                 215                 220

Tyr Ala Pro Pro Phe Pro Ala Ile His Phe Ile Asp Met Glu Pro Ile
225                 230                 235                 240

Phe Asn Glu Gln Ile Ile His Cys Met Ser Leu Ser Lys Ala Gly Leu
                245                 250                 255

Pro Gly Glu Arg Ile Gly Ile Ala Ile Gly Pro Ser Arg Tyr Ile Gln
            260                 265                 270

Ala Met Glu Ala Phe Gln Ser Asn Ala Ala Ile His Ser Ser Arg Leu
        275                 280                 285

Gly Gln Tyr Met Ala Ala Ser Val Leu Asn Asp Gly Arg Leu Ala Asp
    290                 295                 300

Val Ser Leu Asn Glu Val Arg Pro Tyr Tyr Arg Asn Lys Phe Met Leu
305                 310                 315                 320

Leu Lys Glu Thr Leu Leu Cys Lys Met Pro Glu Asp Ile Lys Trp Tyr
                325                 330                 335

Leu His Gln Gly Glu Gly Ser Leu Phe Gly Trp Leu Trp Phe Glu Asp
            340                 345                 350

Leu Pro Val Thr Asp Ala Ala Leu Tyr Glu Tyr Met Lys Ala Asp Gly
        355                 360                 365

Val Ile Ile Val Pro Gly Ser Ser Phe Phe His Arg Gln Ser Arg Arg
    370                 375                 380

Leu Ala His Ser His Gln Cys Ile Arg Ile Ser Leu Thr Ala Ala Asp
385                 390                 395                 400

Glu Asp Ile Ile Arg Gly Ile Asp Val Leu Ala Lys Ile Ala Lys Gly
                405                 410                 415

Val Tyr Glu Lys Gln Val Glu Tyr Leu
            420                 425

<210>  235
<211>  930
<212>  DNA
<213>  Escherichia coli

<400>  235
atgaccacga agaaagctga ttacatttgg ttcaatgggg agatggttcg ctgggaagac      60

gcgaaggtgc atgtgatgtc gcacgcgctg cactatggca cttcggtttt tgaaggcatc     120

cgttgctacg actcgcacaa aggaccggtt gtattccgcc atcgtgagca tatgcagcgt     180

ctgcatgact ccgccaaaat ctatcgcttc ccggtttcgc agagcattga tgagctgatg     240

gaagcttgtc gtgacgtgat ccgcaaaaac aatctcacca gcgcctatat ccgtccgctg     300

atcttcgtcg gtgatgttgg catgggagta aacccgccag cgggatactc aaccgacgtg     360

attatcgctg ctttcccgtg gggagcgtat ctgggcgcag aagcgctgga gcaggggatc     420

gatgcgatgg tttcctcctg gaaccgcgca gcaccaaaca ccatcccgac ggcggcaaaa     480

gccggtggta actacctctc ttccctgctg gtgggtagcg aagcgcgccg ccacggttat     540

caggaaggta tcgcgctgga tgtgaacggt tatatctctg aaggcgcagg cgaaaacctg     600

tttgaagtga aagatggtgt gctgttcacc ccaccgttca cctcctccgc gctgccgggt     660

attacccgtg atgccatcat caaactggcg aaagagctgg gaattgaagt acgtgagcag     720

gtgctgtcgc gcgaatccct gtacctggcg gatgaagtgt ttatgtccgg tacggcggca     780

gaaatcacgc cagtgcgcag cgtagacggt attcaggttg gcgaaggccg ttgtggcccg     840

gttaccaaac gcattcagca agccttcttc ggcctcttca ctggcgaaac cgaagataaa     900

tggggctggt tagatcaagt taatcaataa                                      930

<210>  236
<211>  309
<212>  PRT
<213>  Escherichia coli

<400>  236

Met Thr Thr Lys Lys Ala Asp Tyr Ile Trp Phe Asn Gly Glu Met Val
1               5                   10                  15

Arg Trp Glu Asp Ala Lys Val His Val Met Ser His Ala Leu His Tyr
            20                  25                  30

Gly Thr Ser Val Phe Glu Gly Ile Arg Cys Tyr Asp Ser His Lys Gly
        35                  40                  45

Pro Val Val Phe Arg His Arg Glu His Met Gln Arg Leu His Asp Ser
    50                  55                  60

Ala Lys Ile Tyr Arg Phe Pro Val Ser Gln Ser Ile Asp Glu Leu Met
65                  70                  75                  80

Glu Ala Cys Arg Asp Val Ile Arg Lys Asn Asn Leu Thr Ser Ala Tyr
                85                  90                  95

Ile Arg Pro Leu Ile Phe Val Gly Asp Val Gly Met Gly Val Asn Pro
            100                 105                 110

Pro Ala Gly Tyr Ser Thr Asp Val Ile Ile Ala Ala Phe Pro Trp Gly
        115                 120                 125

Ala Tyr Leu Gly Ala Glu Ala Leu Glu Gln Gly Ile Asp Ala Met Val
    130                 135                 140

Ser Ser Trp Asn Arg Ala Ala Pro Asn Thr Ile Pro Thr Ala Ala Lys
145                 150                 155                 160

Ala Gly Gly Asn Tyr Leu Ser Ser Leu Leu Val Gly Ser Glu Ala Arg
                165                 170                 175

Arg His Gly Tyr Gln Glu Gly Ile Ala Leu Asp Val Asn Gly Tyr Ile
            180                 185                 190

Ser Glu Gly Ala Gly Glu Asn Leu Phe Glu Val Lys Asp Gly Val Leu
        195                 200                 205

Phe Thr Pro Pro Phe Thr Ser Ser Ala Leu Pro Gly Ile Thr Arg Asp
    210                 215                 220

Ala Ile Ile Lys Leu Ala Lys Glu Leu Gly Ile Glu Val Arg Glu Gln
225                 230                 235                 240

Val Leu Ser Arg Glu Ser Leu Tyr Leu Ala Asp Glu Val Phe Met Ser
                245                 250                 255

Gly Thr Ala Ala Glu Ile Thr Pro Val Arg Ser Val Asp Gly Ile Gln
            260                 265                 270

Val Gly Glu Gly Arg Cys Gly Pro Val Thr Lys Arg Ile Gln Gln Ala
        275                 280                 285

Phe Phe Gly Leu Phe Thr Gly Glu Thr Glu Asp Lys Trp Gly Trp Leu
    290                 295                 300

Asp Gln Val Asn Gln
305

<210>  237
<211>  1131
<212>  DNA
<213>  Saccharomyces cerevisiae

<400>  237
atgaccttgg cacccctaga cgcctccaaa gttaagataa ctaccacaca acatgcatct      60

aagccaaaac cgaacagtga gttagtgttt ggcaagagct tcacggacca catgttaact     120

gcggaatgga cagctgaaaa agggtggggt accccagaga ttaaacctta tcaaaatctg     180

tctttagacc cttccgcggt ggttttccat tatgcttttg agctattcga agggatgaag     240

gcttacagaa cggtggacaa caaaattaca atgtttcgtc cagatatgaa tatgaagcgc     300

atgaataagt ctgctcagag aatctgtttg ccaacgttcg acccagaaga gttgattacc     360

ctaattggga aactgatcca gcaagataag tgcttagttc ctgaaggaaa aggttactct     420

ttatatatca ggcctacatt aatcggcact acggccggtt taggggtttc cacgcctgat     480

agagccttgc tatatgtcat ttgctgccct gtgggtcctt attacaaaac tggatttaag     540

gcggtcagac tggaagccac tgattatgcc acaagagctt ggccaggagg ctgtggtgac     600

aagaaactag gtgcaaacta cgccccctgc gtcctgccac aattgcaagc tgcttcaagg     660

ggttaccaac aaaatttatg gctatttggt ccaaataaca acattactga agtcggcacc     720

atgaatgctt ttttcgtgtt taaagatagt aaaacgggca agaaggaact agttactgct     780

ccactagacg gtaccatttt ggaaggtgtt actagggatt ccattttaaa tcttgctaaa     840

gaaagactcg aaccaagtga atggaccatt agtgaacgct acttcactat aggcgaagtt     900

actgagagat ccaagaacgg tgaactactt gaagcctttg gttctggtac tgctgcgatt     960

gtttctccca ttaaggaaat cggctggaaa ggcgaacaaa ttaatattcc gttgttgccc    1020

ggcgaacaaa ccggtccatt ggccaaagaa gttgcacaat ggattaatgg aatccaatat    1080

ggcgagactg agcatggcaa ttggtcaagg gttgttactg atttgaactg a             1131

<210>  238
<211>  376
<212>  PRT
<213>  Saccharomyces cerevisiae

<400>  238

Met Thr Leu Ala Pro Leu Asp Ala Ser Lys Val Lys Ile Thr Thr Thr
1               5                   10                  15

Gln His Ala Ser Lys Pro Lys Pro Asn Ser Glu Leu Val Phe Gly Lys
            20                  25                  30

Ser Phe Thr Asp His Met Leu Thr Ala Glu Trp Thr Ala Glu Lys Gly
        35                  40                  45

Trp Gly Thr Pro Glu Ile Lys Pro Tyr Gln Asn Leu Ser Leu Asp Pro
    50                  55                  60

Ser Ala Val Val Phe His Tyr Ala Phe Glu Leu Phe Glu Gly Met Lys
65                  70                  75                  80

Ala Tyr Arg Thr Val Asp Asn Lys Ile Thr Met Phe Arg Pro Asp Met
                85                  90                  95

Asn Met Lys Arg Met Asn Lys Ser Ala Gln Arg Ile Cys Leu Pro Thr
            100                 105                 110

Phe Asp Pro Glu Glu Leu Ile Thr Leu Ile Gly Lys Leu Ile Gln Gln
        115                 120                 125

Asp Lys Cys Leu Val Pro Glu Gly Lys Gly Tyr Ser Leu Tyr Ile Arg
    130                 135                 140

Pro Thr Leu Ile Gly Thr Thr Ala Gly Leu Gly Val Ser Thr Pro Asp
145                 150                 155                 160

Arg Ala Leu Leu Tyr Val Ile Cys Cys Pro Val Gly Pro Tyr Tyr Lys
                165                 170                 175

Thr Gly Phe Lys Ala Val Arg Leu Glu Ala Thr Asp Tyr Ala Thr Arg
            180                 185                 190

Ala Trp Pro Gly Gly Cys Gly Asp Lys Lys Leu Gly Ala Asn Tyr Ala
        195                 200                 205

Pro Cys Val Leu Pro Gln Leu Gln Ala Ala Ser Arg Gly Tyr Gln Gln
    210                 215                 220

Asn Leu Trp Leu Phe Gly Pro Asn Asn Asn Ile Thr Glu Val Gly Thr
225                 230                 235                 240

Met Asn Ala Phe Phe Val Phe Lys Asp Ser Lys Thr Gly Lys Lys Glu
                245                 250                 255

Leu Val Thr Ala Pro Leu Asp Gly Thr Ile Leu Glu Gly Val Thr Arg
            260                 265                 270

Asp Ser Ile Leu Asn Leu Ala Lys Glu Arg Leu Glu Pro Ser Glu Trp
        275                 280                 285

Thr Ile Ser Glu Arg Tyr Phe Thr Ile Gly Glu Val Thr Glu Arg Ser
    290                 295                 300

Lys Asn Gly Glu Leu Leu Glu Ala Phe Gly Ser Gly Thr Ala Ala Ile
305                 310                 315                 320

Val Ser Pro Ile Lys Glu Ile Gly Trp Lys Gly Glu Gln Ile Asn Ile
                325                 330                 335

Pro Leu Leu Pro Gly Glu Gln Thr Gly Pro Leu Ala Lys Glu Val Ala
            340                 345                 350

Gln Trp Ile Asn Gly Ile Gln Tyr Gly Glu Thr Glu His Gly Asn Trp
        355                 360                 365

Ser Arg Val Val Thr Asp Leu Asn
    370                 375

<210>  239
<211>  993
<212>  DNA
<213>  Methanobacterium thermoautotrophicum

<400>  239
   tcagatgtag gtgagccatc cgaagctgtc ctctgtctct gccctgatta tcctgaagaa      60
   ctcatcctgc agcagctttg taacgggacc ccttcgcccg gcacctatct ctataccatc     120
   aactgatctg atgggtgtta tctctgcggc tgtacctgtg aagaaggcct catctgcgat     180
   gtagagcatc tccctggtta tgggttcctc atgcacggta acaccctcgg tcctggctat     240
   ctttattacg gagtcccttg ttatccccct cagaagggat gatgaaacag ggggggtgta      300
   aatttcaccc tcactgacga ggaatatgtt ctccccgcta ccctcactta tgtagccatg     360
   gtagtccagc attatggcct catcatagcc gtgtctcaca gcctccatct tggcaagctg     420
   tgagttgagg tagttaccgc cggcctttgc catgttgggc attgtgtttg gtgccatcct     480
   ccgccaggtt gaaacaccag catcgacacc aacctcaagg gcctctgcac ccagataggc     540
   cccccattcc caggcagcca cagcgacgtc cactgggcag ttcaccgggt gaacacccat     600
   ctcaccgtat cccctgaata ccacgggtct tatatagcac tcctcaagtc cgttctccct 660
   gacggtctca actatggcat cacatatctg ctcctgggtg tagggtatgt ccatccggta 720
   tatctttgca gaatcaaaaa ggcgtttaac atgctcccgc aaacggaaga tggctgaccc 780
   cttactgttc ctgtagcacc ttattccctc aaagacagat gatccataat gcacaacatg 840
   tgagagtacg tggacggtgg cttcttccca ttcaaccatt tcaccgttta accatatctt 900
   tccactggct tcgcatgaca tgataataac ctcaggtgat ttactaggat aggttatggt 960
   tggaggccta tataatgctc tccataaccg caa 993

<210> 240
<211> 330
<212> PRT
<213> Methanobacterium thermoautotrophicum

<400> 240

Met Arg Leu Trp Arg Ala Leu Tyr Arg Pro Pro Thr Ile Thr Tyr Pro
1               5                   10                  15

Ser Lys Ser Pro Glu Val Ile Ile Met Ser Cys Glu Ala Ser Gly Lys
            20                  25                  30

Ile Trp Leu Asn Gly Glu Met Val Glu Trp Glu Glu Ala Thr Val His
        35                  40                  45

Val Leu Ser His Val Val His Tyr Gly Ser Ser Val Phe Glu Gly Ile
    50                  55                  60

Arg Cys Tyr Arg Asn Ser Lys Gly Ser Ala Ile Phe Arg Leu Arg Glu
65                  70                  75                  80

His Val Lys Arg Leu Phe Asp Ser Ala Lys Ile Tyr Arg Met Asp Ile
                85                  90                  95

Pro Tyr Thr Gln Glu Gln Ile Cys Asp Ala Ile Val Glu Thr Val Arg
            100                 105                 110

Glu Asn Gly Leu Glu Glu Cys Tyr Ile Arg Pro Val Val Phe Arg Gly
        115                 120                 125

Tyr Gly Glu Met Gly Val His Pro Val Asn Cys Pro Val Asp Val Ala
    130                 135                 140

Val Ala Ala Trp Glu Trp Gly Ala Tyr Leu Gly Ala Glu Ala Leu Glu
145                 150                 155                 160

Val Gly Val Asp Ala Gly Val Ser Thr Trp Arg Arg Met Ala Pro Asn
                165                 170                 175

Thr Met Pro Asn Met Ala Lys Ala Gly Gly Asn Tyr Leu Asn Ser Gln
            180                 185                 190

Leu Ala Lys Met Glu Ala Val Arg His Gly Tyr Asp Glu Ala Ile Met
        195                 200                 205

Leu Asp Tyr His Gly Tyr Ile Ser Glu Gly Ser Gly Glu Asn Ile Phe
    210                 215                 220

Leu Val Ser Glu Gly Glu Ile Tyr Thr Pro Pro Val Ser Ser Ser Leu
225                 230                 235                 240

Leu Arg Gly Ile Thr Arg Asp Ser Val Ile Lys Ile Ala Arg Thr Glu
                245                 250                 255

Gly Val Thr Val His Glu Glu Pro Ile Thr Arg Glu Met Leu Tyr Ile
            260                 265                 270

Ala Asp Glu Ala Phe Phe Thr Gly Thr Ala Ala Glu Ile Thr Pro Ile
        275                 280                 285

Arg Ser Val Asp Gly Ile Glu Ile Gly Ala Gly Arg Arg Gly Pro Val
    290                 295                 300

Thr Lys Leu Leu Gln Asp Glu Phe Phe Arg Ile Ile Arg Ala Glu Thr
305                 310                 315                 320

Glu Asp Ser Phe Gly Trp Leu Thr Tyr Ile
                325                 330

<210>  241
<211>  1095
<212>  DNA
<213>  Streptomyces coelicolor

<400>  241
tcacggccgg ggacgggcct ccgccatccg ctgctcggcg atccggtcgg ccgccgcggc      60

cggcggaata ccgtcctcct tcgcacgtgc gaatatggcc agcgtggtgt cgtagatctt     120

cgaggccttc gccttgcacc ggtcgaagtc gaacccgtgc agctcgtcgg cgacctggat     180

gacaccgccg gcgttcacca catagtccgg cgcgtagagg atcccgcggt cggcgaggtc     240

cttctcgacg cccgggtggg cgagctggtt gttggccgcg ccgcacacca ccttggcggt     300

cagcaccggc acggtgtcgt cgttcagcgc gccgccgagc gcgcagggcg cgtagatgtc     360

caggttctcc acccggatca gcgcgtcggt gtcggcgacg gcgaccaccg acgggtgccg     420

ctccgtgatc ccgcgcacca cgtccttgcg cacgtccgtg acgacgacgt gggcgccctc     480

ggcgagcagg tgctcgacca ggtggtggcc gaccttgccg acgcccgcga tgccgacggt     540

gcggtcgcgc agcgtcgggt cgccccacag gtgctgggcg gcggcccgca tgccctggta 600

gacgccgaag gaggtgagca cggaggagtc gcccgcgccg ccgttctccg gggaacgccc 660

ggtcgtccag cggcactcgc gggccacgac gtccatgtcg gcgacgtagg tgccgacgtc 720

gcacgcggtg acgtagcggc cgcccagcga ggcgacgaac cggccgtagg cgaggagcag 780

ctcctcgctc ttgatctgct ccggatcgcc gatgatcacg gccttgccgc caccgtggtc 840

cagaccggcc atggcgttct tgtacgacat cccgcgggcg aggttcagcg cgtcggcgac 900

ggcctccgcc tcgctcgcgt acgggtagaa gcgggtaccg ccgagcgccg ggcccagggc 960

ggtggagtgg agggcgatca cggccttgag gccgctggca cggtcctggc agagcacgac 1020

ttgctcatgt cccccctgat ccgagtggaa cagggtgtgc agtacatcag caggtgcgcc 1080

gtttacgtcg gtcac 1095

<210> 242
<211> 364
<212> PRT
<213> Streptomyces coelicolor

<400> 242

Met Thr Asp Val Asn Gly Ala Pro Ala Asp Val Leu His Thr Leu Phe
1               5                   10                  15

His Ser Asp Gln Gly Gly His Glu Gln Val Val Leu Cys Gln Asp Arg
            20                  25                  30

Ala Ser Gly Leu Lys Ala Val Ile Ala Leu His Ser Thr Ala Leu Gly
        35                  40                  45

Pro Ala Leu Gly Gly Thr Arg Phe Tyr Pro Tyr Ala Ser Glu Ala Glu
    50                  55                  60

Ala Val Ala Asp Ala Leu Asn Leu Ala Arg Gly Met Ser Tyr Lys Asn
65                  70                  75                  80

Ala Met Ala Gly Leu Asp His Gly Gly Gly Lys Ala Val Ile Ile Gly
                85                  90                  95

Asp Pro Glu Gln Ile Lys Ser Glu Glu Leu Leu Leu Ala Tyr Gly Arg
            100                 105                 110

Phe Val Ala Ser Leu Gly Gly Arg Tyr Val Thr Ala Cys Asp Val Gly
        115                 120                 125

Thr Tyr Val Ala Asp Met Asp Val Val Ala Arg Glu Cys Arg Trp Thr
    130                 135                 140

Thr Gly Arg Ser Pro Glu Asn Gly Gly Ala Gly Asp Ser Ser Val Leu
145                 150                 155                 160

Thr Ser Phe Gly Val Tyr Gln Gly Met Arg Ala Ala Ala Gln His Leu
                165                 170                 175

Trp Gly Asp Pro Thr Leu Arg Asp Arg Thr Val Gly Ile Ala Gly Val
            180                 185                 190

Gly Lys Val Gly His His Leu Val Glu His Leu Leu Ala Glu Gly Ala
        195                 200                 205

His Val Val Val Thr Asp Val Arg Lys Asp Val Val Arg Gly Ile Thr
    210                 215                 220

Glu Arg His Pro Ser Val Val Ala Val Ala Asp Thr Asp Ala Leu Ile
225                 230                 235                 240

Arg Val Glu Asn Leu Asp Ile Tyr Ala Pro Cys Ala Leu Gly Gly Ala
                245                 250                 255

Leu Asn Asp Asp Thr Val Pro Val Leu Thr Ala Lys Val Val Cys Gly
            260                 265                 270

Ala Ala Asn Asn Gln Leu Ala His Pro Gly Val Glu Lys Asp Leu Ala
        275                 280                 285

Asp Arg Gly Ile Leu Tyr Ala Pro Asp Tyr Val Val Asn Ala Gly Gly
    290                 295                 300

Val Ile Gln Val Ala Asp Glu Leu His Gly Phe Asp Phe Asp Arg Cys
305                 310                 315                 320

Lys Ala Lys Ala Ser Lys Ile Tyr Asp Thr Thr Leu Ala Ile Phe Ala
                325                 330                 335

Arg Ala Lys Glu Asp Gly Ile Pro Pro Ala Ala Ala Ala Asp Arg Ile
            340                 345                 350

Ala Glu Gln Arg Met Ala Glu Ala Arg Pro Arg Pro
        355                 360

<210>  243
<211>  1095
<212>  DNA
<213>  Bacillus subtilis

<400>  243
atggaacttt ttaaatatat ggagaaatac gattacgaac aattggtatt ctgccaggat      60

gaacaatctg gattaaaagc gattatcgcc attcatgata caacgcttgg tccggcgctt     120

ggcggaacga gaatgtggac atatgaaaat gaagaagcgg caattgaaga tgcgctcaga 180

ttggcaagag gcatgaccta taagaacgcg gcggcaggct taaaccttgg cggcggaaaa 240

acagtcatta tcggcgatcc gcgcaaagac aaaaatgagg aaatgttccg cgcgtttggc 300

cgctatattc aaggactgaa tggcagatac atcacggctg aagatgtggg cacaacggtc 360

gaggatatgg atatcattca tgatgagaca gactatgtca cagggatttc tcctgctttc 420

ggctcttctg gaaatccgtc cccagtcaca gcgtacgggg tgtacagagg aatgaaggca 480

gcagctaaag ctgctttcgg aaccgattct cttgaaggaa aaaccattgc tgtacagggt 540

gttgggaacg tagcctataa cctttgccgc cacctgcatg aagaaggagc aaacttaatc 600

gttacggata tcaacaaaca atctgtacag cgtgcagttg aagattttgg cgcccgtgcg 660

gtagatcctg atgacattta ttcacaagac tgcgatattt atgcgccgtg tgcccttggt 720

gcgactatta acgacgacac cattaaacag ctgaaggcga aagtgatcgc aggtgcggct 780

aacaaccaat taaaagagac acgccatggt gatcaaattc acgaaatggg catcgtttat 840

gcaccggatt acgtgattaa cgcgggcggt gtcatcaacg tggcagatga gctttacggc 900

tataatgcag aacgtgcatt gaaaaaagtt gaaggcattt acggcaatat cgagcgtgta 960

cttgagattt ctcagcgtga cggcattcct gcatatttag cggctgaccg cttagcagag 1020

gaacggattg aacgcatgcg ccgctcaaga agccagtttt tgcaaaacgg ccacagtgta 1080

ttaagcagac gttaa 1095

<210> 244
<211> 364
<212> PRT
<213> Bacillus subtilis

<400> 244

Met Glu Leu Phe Lys Tyr Met Glu Lys Tyr Asp Tyr Glu Gln Leu Val
1               5                   10                  15

Phe Cys Gln Asp Glu Gln Ser Gly Leu Lys Ala Ile Ile Ala Ile His
            20                  25                  30

Asp Thr Thr Leu Gly Pro Ala Leu Gly Gly Thr Arg Met Trp Thr Tyr
        35                  40                  45

Glu Asn Glu Glu Ala Ala Ile Glu Asp Ala Leu Arg Leu Ala Arg Gly
    50                  55                  60

Met Thr Tyr Lys Asn Ala Ala Ala Gly Leu Asn Leu Gly Gly Gly Lys
65                  70                  75                  80

Thr Val Ile Ile Gly Asp Pro Arg Lys Asp Lys Asn Glu Glu Met Phe
                85                  90                  95

Arg Ala Phe Gly Arg Tyr Ile Gln Gly Leu Asn Gly Arg Tyr Ile Thr
        100                 105                 110

Ala Glu Asp Val Gly Thr Thr Val Glu Asp Met Asp Ile Ile His Asp
    115                 120                 125

Glu Thr Asp Tyr Val Thr Gly Ile Ser Pro Ala Phe Gly Ser Ser Gly
    130                 135                 140

Asn Pro Ser Pro Val Thr Ala Tyr Gly Val Tyr Arg Gly Met Lys Ala
145                 150                 155                 160

Ala Ala Lys Ala Ala Phe Gly Thr Asp Ser Leu Glu Gly Lys Thr Ile
                165                 170                 175

Ala Val Gln Gly Val Gly Asn Val Ala Tyr Asn Leu Cys Arg His Leu
            180                 185                 190

His Glu Glu Gly Ala Asn Leu Ile Val Thr Asp Ile Asn Lys Gln Ser
        195                 200                 205

Val Gln Arg Ala Val Glu Asp Phe Gly Ala Arg Ala Val Asp Pro Asp
    210                 215                 220

Asp Ile Tyr Ser Gln Asp Cys Asp Ile Tyr Ala Pro Cys Ala Leu Gly
225                 230                 235                 240

Ala Thr Ile Asn Asp Asp Thr Ile Lys Gln Leu Lys Ala Lys Val Ile
                245                 250                 255

Ala Gly Ala Ala Asn Asn Gln Leu Lys Glu Thr Arg His Gly Asp Gln
            260                 265                 270

Ile His Glu Met Gly Ile Val Tyr Ala Pro Asp Tyr Val Ile Asn Ala
        275                 280                 285

Gly Gly Val Ile Asn Val Ala Asp Glu Leu Tyr Gly Tyr Asn Ala Glu
    290                 295                 300

Arg Ala Leu Lys Lys Val Glu Gly Ile Tyr Gly Asn Ile Glu Arg Val
305                 310                 315                 320

Leu Glu Ile Ser Gln Arg Asp Gly Ile Pro Ala Tyr Leu Ala Ala Asp
                325                 330                 335

Arg Leu Ala Glu Glu Arg Ile Glu Arg Met Arg Arg Ser Arg Ser Gln
            340                 345                 350

Phe Leu Gln Asn Gly His Ser Val Leu Ser Arg Arg
        355                 360

<210>  245
<211>  1785
<212>  DNA
<213>  Streptomyces viridifaciens

<400>  245
gtgtcaactt cctccgcttc ttccgggccg gacctcccct tcgggcccga ggacacgcca      60

tggcagaagg ccttcagcag gctgcgggcg gtggatggcg tgccgcgcgt caccgcgccg     120

tccagtgatc cgcgtgaggt ctacatggac atcccggaga tccccttctc caaggtccag     180

atccccccgg acggaatgga cgagcagcag tacgcagagg ccgagagcct cttccgccgc     240

tacgtagacg cccagacccg caacttcgcg ggataccagg tcaccagcga cctcgactac     300

cagcacctca gtcactatct caaccggcat ctgaacaacg tcggcgatcc ctatgagtcc     360

agctcctaca cgctgaactc caaggtcctt gagcgagccg ttctcgacta cttcgcctcc     420

ctgtggaacg ccaagtggcc ccatgacgca agcgatccgg aaacgtactg gggttacgtg     480

ctgaccatgg gctccagcga aggcaacctg tacgggttgt ggaacgcacg ggactatctg     540

tcgggcaagc tgctgcggcg ccagcaccgg gaggccggcg gcgacaaggc ctcggtcgtc     600

tacacgcaag cgctgcgaca cgaagggcag agtccgcatg cctacgagcc ggtggcgttc     660

ttctcgcagg acacgcacta ctcgctcacg aaggccgtgc gggttctggg catcgacacc     720

ttccacagca tcggcagcag tcggtatccg gacgagaacc cgctgggccc cggcactccg     780

tggccgaccg aagtgccctc ggttgacggt gccatcgatg tcgacaaact cgcctcgttg     840

gtccgcttct tcgccagcaa gggctacccg atactggtca gcctcaacta cgggtcaacg     900

ttcaagggcg cctacgacga cgtcccggcc gtggcacagg ccgtgcggga catctgcacg     960

gaatacggtc tggatcggcg gcgggtatac cacgaccgca gtaaggacag tgacttcgac    1020

gagcgcagcg gcttctggat ccacatcgat gccgccctgg gggcgggcta cgctccctac    1080

ctgcagatgg cccgggatgc cggcatggtc gaggaggcgc cgcccgtttt cgacttccgg    1140

ctcccggagg tgcactcgct gaccatgagc ggccacaagt ggatgggaac accgtgggca    1200

tgcggtgtct acatgacacg gaccgggctg cagatgaccc cgccgaagtc gtccgagtac    1260

atcggggcgg ccgacaccac cttcgcgggc tcccgcaacg gcttctcgtc actgctgctg    1320

tgggactacc tgtcccggca ttcgtatgac gatctggtgc gcctggccgc cgactgcgac    1380

cggctggccg gctacgccca cgaccggttg ctgaccttgc aggacaaact cggcatggat    1440

ctgtgggtcg cccgcagccc gcagtccctc acggtgcgct tccgtcagcc atgtgcagac    1500

atcgtccgca agtactcgct gtcgtgtgag acggtctacg aagacaacga gcaacggacc    1560

tacgtacatc tctacgccgt tccccacctc actcgggaac tcgtggatga gctcgtgcgc    1620

gatctgcgcc agcccggagc cttcaccaac gctggtgcac tggaggggga ggcctgggcc 1680

ggggtgatcg atgccctcgg ccgcccggac cccgacggaa cctatgccgg cgccttgagc 1740

gctccggctt ccggcccccg ctccgaggac ggcggcggga gctga 1785

<210> 246
<211> 594
<212> PRT
<213> Streptomyces viridifaciens

<400> 246

Met Ser Thr Ser Ser Ala Ser Ser Gly Pro Asp Leu Pro Phe Gly Pro
1               5                   10                  15

Glu Asp Thr Pro Trp Gln Lys Ala Phe Ser Arg Leu Arg Ala Val Asp
            20                  25                  30

Gly Val Pro Arg Val Thr Ala Pro Ser Ser Asp Pro Arg Glu Val Tyr
        35                  40                  45

Met Asp Ile Pro Glu Ile Pro Phe Ser Lys Val Gln Ile Pro Pro Asp
    50                  55                  60

Gly Met Asp Glu Gln Gln Tyr Ala Glu Ala Glu Ser Leu Phe Arg Arg
65                  70                  75                  80

Tyr Val Asp Ala Gln Thr Arg Asn Phe Ala Gly Tyr Gln Val Thr Ser
                85                  90                  95

Asp Leu Asp Tyr Gln His Leu Ser His Tyr Leu Asn Arg His Leu Asn
            100                 105                 110

Asn Val Gly Asp Pro Tyr Glu Ser Ser Ser Tyr Thr Leu Asn Ser Lys
        115                 120                 125

Val Leu Glu Arg Ala Val Leu Asp Tyr Phe Ala Ser Leu Trp Asn Ala
    130                 135                 140

Lys Trp Pro His Asp Ala Ser Asp Pro Glu Thr Tyr Trp Gly Tyr Val
145                 150                 155                 160

Leu Thr Met Gly Ser Ser Glu Gly Asn Leu Tyr Gly Leu Trp Asn Ala
                165                 170                 175

Arg Asp Tyr Leu Ser Gly Lys Leu Leu Arg Arg Gln His Arg Glu Ala
            180                 185                 190

Gly Gly Asp Lys Ala Ser Val Val Tyr Thr Gln Ala Leu Arg His Glu
        195                 200                 205

Gly Gln Ser Pro His Ala Tyr Glu Pro Val Ala Phe Phe Ser Gln Asp
    210                 215                 220

Thr His Tyr Ser Leu Thr Lys Ala Val Arg Val Leu Gly Ile Asp Thr
225                 230                 235                 240

Phe His Ser Ile Gly Ser Ser Arg Tyr Pro Asp Glu Asn Pro Leu Gly
                245                 250                 255

Pro Gly Thr Pro Trp Pro Thr Glu Val Pro Ser Val Asp Gly Ala Ile
            260                 265                 270

Asp Val Asp Lys Leu Ala Ser Leu Val Arg Phe Phe Ala Ser Lys Gly
        275                 280                 285

Tyr Pro Ile Leu Val Ser Leu Asn Tyr Gly Ser Thr Phe Lys Gly Ala
    290                 295                 300

Tyr Asp Asp Val Pro Ala Val Ala Gln Ala Val Arg Asp Ile Cys Thr
305                 310                 315                 320

Glu Tyr Gly Leu Asp Arg Arg Arg Val Tyr His Asp Arg Ser Lys Asp
                325                 330                 335

Ser Asp Phe Asp Glu Arg Ser Gly Phe Trp Ile His Ile Asp Ala Ala
            340                 345                 350

Leu Gly Ala Gly Tyr Ala Pro Tyr Leu Gln Met Ala Arg Asp Ala Gly
        355                 360                 365

Met Val Glu Glu Ala Pro Pro Val Phe Asp Phe Arg Leu Pro Glu Val
    370                 375                 380

His Ser Leu Thr Met Ser Gly His Lys Trp Met Gly Thr Pro Trp Ala
385                 390                 395                 400

Cys Gly Val Tyr Met Thr Arg Thr Gly Leu Gln Met Thr Pro Pro Lys
                405                 410                 415

Ser Ser Glu Tyr Ile Gly Ala Ala Asp Thr Thr Phe Ala Gly Ser Arg
            420                 425                 430

Asn Gly Phe Ser Ser Leu Leu Leu Trp Asp Tyr Leu Ser Arg His Ser
        435                 440                 445

Tyr Asp Asp Leu Val Arg Leu Ala Ala Asp Cys Asp Arg Leu Ala Gly
    450                 455                 460

Tyr Ala His Asp Arg Leu Leu Thr Leu Gln Asp Lys Leu Gly Met Asp
465                 470                 475                 480

Leu Trp Val Ala Arg Ser Pro Gln Ser Leu Thr Val Arg Phe Arg Gln
                485                 490                 495

Pro Cys Ala Asp Ile Val Arg Lys Tyr Ser Leu Ser Cys Glu Thr Val
            500                 505                 510

Tyr Glu Asp Asn Glu Gln Arg Thr Tyr Val His Leu Tyr Ala Val Pro
        515                 520                 525

His Leu Thr Arg Glu Leu Val Asp Glu Leu Val Arg Asp Leu Arg Gln
    530                 535                 540

Pro Gly Ala Phe Thr Asn Ala Gly Ala Leu Glu Gly Glu Ala Trp Ala
545                 550                 555                 560

Gly Val Ile Asp Ala Leu Gly Arg Pro Asp Pro Asp Gly Thr Tyr Ala
                565                 570                 575

Gly Ala Leu Ser Ala Pro Ala Ser Gly Pro Arg Ser Glu Asp Gly Gly
            580                 585                 590

Gly Ser

<210>  247
<211>  1323
<212>  DNA
<213>  Alcaligenes denitrificans

<400>  247
atgagcgctg ccaaactgcc cgacctgtcc cacctctgga tgccctttac cgccaaccgg      60

cagttcaagg cgaacccccg cctgctggcc tcggccaagg gcatgtacta cacgtctttc     120

gacggccgcc agatcctgga cggcacggcc ggcctgtggt gcgtgaacgc cggccactgc     180

cgcgaagaaa tcgtctccgc catcgccagc caggccggcg tcatggacta cgcgccgggg     240

ttccagctcg gccacccgct ggccttcgag gccgccaccg ccgtggccgg cctgatgccg     300

cagggcctgg accgcgtgtt cttcaccaat tcgggctccg aatcggtgga caccgcgctg     360

aagatcgccc tggcctacca ccgcgcgcgc ggcgaggcgc agcgcacccg cctcatcggg     420

cgcgagcgcg gctaccacgg cgtgggcttc ggcggcattt ccgtgggcgg catctcgccc     480

aaccgcaaga ccttctccgg cgcgctgctg ccggccgtgg accacctgcc gcacacccac     540

agcctggaac acaacgcctt cacgcgcggc cagcccgagt ggggcgcgca cctggccgac     600

gagttggaac gcatcatcgc cctgcacgac gcctccacca tcgcggccgt gatcgtcgag     660

cccatggccg gctccaccgg cgtgctcgtc ccgcccaagg gctatctcga aaaactgcgc     720

gaaatcaccg cccgccacgg cattctgctg atcttcgacg aagtcatcac cgcgtacggc 780

cgcctgggcg aggccaccgc cgcggcctat ttcggcgtaa cgcccgacct catcaccatg 840

gccaagggcg tgagcaacgc cgccgttccg gccggcgccg tcgcggtgcg ccgcgaagtg 900

catgacgcca tcgtcaacgg accgcaaggc ggcatcgagt tcttccacgg ctacacctac 960

tcggcccacc cgctggccgc cgccgccgtg ctcgccacgc tggacatcta ccgccgcgaa 1020

gacctgttcg cccgcgcccg caagctgtcg gccgcgttcg aggaagccgc ccacagcctc 1080

aagggcgcgc cgcacgtcat cgacgtgcgc aacatcggcc tggtggccgg catcgagctg 1140

tcgccgcgcg aaggcgcccc gggcgcgcgc gccgccgaag ccttccagaa atgcttcgac 1200

accggcctca tggtgcgcta cacgggcgac atcctcgcgg tgtcgcctcc gctcatcgtc 1260

gacgaaaacc agatcggcca gatcttcgag ggcatcggca aggtgctcaa ggaagtggct 1320

tag 1323

<210> 248
<211> 440
<212> PRT
<213> omega-amino acid:pyruvate transaminase

<400> 248

Met Ser Ala Ala Lys Leu Pro Asp Leu Ser His Leu Trp Met Pro Phe
1               5                   10                  15

Thr Ala Asn Arg Gln Phe Lys Ala Asn Pro Arg Leu Leu Ala Ser Ala
            20                  25                  30

Lys Gly Met Tyr Tyr Thr Ser Phe Asp Gly Arg Gln Ile Leu Asp Gly
        35                  40                  45

Thr Ala Gly Leu Trp Cys Val Asn Ala Gly His Cys Arg Glu Glu Ile
    50                  55                  60

Val Ser Ala Ile Ala Ser Gln Ala Gly Val Met Asp Tyr Ala Pro Gly
65                  70                  75                  80

Phe Gln Leu Gly His Pro Leu Ala Phe Glu Ala Ala Thr Ala Val Ala
                85                  90                  95

Gly Leu Met Pro Gln Gly Leu Asp Arg Val Phe Phe Thr Asn Ser Gly
            100                 105                 110

Ser Glu Ser Val Asp Thr Ala Leu Lys Ile Ala Leu Ala Tyr His Arg
        115                 120                 125

Ala Arg Gly Glu Ala Gln Arg Thr Arg Leu Ile Gly Arg Glu Arg Gly
    130                 135                 140

Tyr His Gly Val Gly Phe Gly Gly Ile Ser Val Gly Gly Ile Ser Pro
145                 150                 155                 160

Asn Arg Lys Thr Phe Ser Gly Ala Leu Leu Pro Ala Val Asp His Leu
                165                 170                 175

Pro His Thr His Ser Leu Glu His Asn Ala Phe Thr Arg Gly Gln Pro
            180                 185                 190

Glu Trp Gly Ala His Leu Ala Asp Glu Leu Glu Arg Ile Ile Ala Leu
        195                 200                 205

His Asp Ala Ser Thr Ile Ala Ala Val Ile Val Glu Pro Met Ala Gly
    210                 215                 220

Ser Thr Gly Val Leu Val Pro Pro Lys Gly Tyr Leu Glu Lys Leu Arg
225                 230                 235                 240

Glu Ile Thr Ala Arg His Gly Ile Leu Leu Ile Phe Asp Glu Val Ile
                245                 250                 255

Thr Ala Tyr Gly Arg Leu Gly Glu Ala Thr Ala Ala Ala Tyr Phe Gly
            260                 265                 270

Val Thr Pro Asp Leu Ile Thr Met Ala Lys Gly Val Ser Asn Ala Ala
        275                 280                 285

Val Pro Ala Gly Ala Val Ala Val Arg Arg Glu Val His Asp Ala Ile
    290                 295                 300

Val Asn Gly Pro Gln Gly Gly Ile Glu Phe Phe His Gly Tyr Thr Tyr
305                 310                 315                 320

Ser Ala His Pro Leu Ala Ala Ala Ala Val Leu Ala Thr Leu Asp Ile
                325                 330                 335

Tyr Arg Arg Glu Asp Leu Phe Ala Arg Ala Arg Lys Leu Ser Ala Ala
            340                 345                 350

Phe Glu Glu Ala Ala His Ser Leu Lys Gly Ala Pro His Val Ile Asp
        355                 360                 365

Val Arg Asn Ile Gly Leu Val Ala Gly Ile Glu Leu Ser Pro Arg Glu
    370                 375                 380

Gly Ala Pro Gly Ala Arg Ala Ala Glu Ala Phe Gln Lys Cys Phe Asp
385                 390                 395                 400

Thr Gly Leu Met Val Arg Tyr Thr Gly Asp Ile Leu Ala Val Ser Pro
                405                 410                 415

Pro Leu Ile Val Asp Glu Asn Gln Ile Gly Gln Ile Phe Glu Gly Ile
            420                 425                 430

Gly Lys Val Leu Lys Glu Val Ala
        435                 440

<210>  249
<211>  1332
<212>  DNA
<213>  Ralstonia eutropha

<400>  249
atggacgccg cgaagaccgt gattcccgat ctcgatgccc tgtggatgcc ctttaccgcg      60

aaccgccagt acaaggcggc gccgcgcctg ctggcctcgg ccagcggcat gtactacacc     120

acccacgacg gacgccagat cctcgacggt tgcgcgggcc tctggtgcgt agcggccggc     180

cactgccgca aggagattgc cgaggccgtg gcccgccagg ccgccacgct cgactacgcg     240

ccgccgttcc agatgggcca tccgctgtcg ttcgaagccg ccaccaaggt ggccgcgatc     300

atgccgcagg gactggaccg catcttcttc acgaattccg gttcggaatc ggtggacacc     360

gcgctgaaga ttgcgctggc ctaccaccgt gcgcgcggcg agggccagcg cacccgcttc     420

atcgggcgcg aacgcggtta ccacggcgtg ggctttggcg gcatggctgt cggtggcatc     480

gggccgaacc gcaaggcgtt ctcggccaac ctgatgccgg gcaccgacca tctgccggcg     540

acgctgaata tcgccgaagc ggcgttctcc aagggtcagc cgacatgggg cgcgcacctt     600

gccgacgaac tcgagcgcat cgtcgcgctg catgatccgt ccacgattgc cgccgtcatc     660

gtggaaccgc tggcgggctc cgccggggtg ctggtgccgc cggtcggcta cctcgacaag     720

ctgcgcgaga tcacgaccaa gcacggcatc ctgctgatct tcgacgaggt catcacggcc     780

tttggtcgcc tgggtaccgc caccgcggcg gaacgcttca aggtcacgcc ggacctgatc     840

accatggcca aggccatcaa caacgccgcc gtgccgatgg gtgccgtggc cgtgcgccgc     900

gaagtccatg acaccgtggt caactcggcc gcgccgggcg cgatcgaact cgcgcatggc     960

tacacctact cgggccaccc gctggccgcc gccgctgcca tcgccacgct ggacctgtat    1020

cagcgcgaga acctgttcgg ccgtgccgcg gagctgtcgc cggtgttcga agcggccgtt    1080

cacagcgtac gcagcgcgcc gcatgtgaag gacatccgca acctcggcat ggtggccggc    1140

atcgagctgg agccgcgtcc gggccagccc ggcgcacgcg cctacgaagc cttcctcaaa    1200

tgccttgagc gtggcgtgct ggtgcgctac accggcgata tcctcgcgtt ctcgccgccg    1260

ctgatcatca gcgaggcgca gattgccgag ctgttcgata cggtcaagca ggccttgcag    1320

gaagtgcagt aa                                                        1332

<210>  250
<211>  443
<212>  PRT
<213>  Ralstonia eutropha

<400>  250

Met Asp Ala Ala Lys Thr Val Ile Pro Asp Leu Asp Ala Leu Trp Met
1               5                   10                  15

Pro Phe Thr Ala Asn Arg Gln Tyr Lys Ala Ala Pro Arg Leu Leu Ala
            20                  25                  30

Ser Ala Ser Gly Met Tyr Tyr Thr Thr His Asp Gly Arg Gln Ile Leu
        35                  40                  45

Asp Gly Cys Ala Gly Leu Trp Cys Val Ala Ala Gly His Cys Arg Lys
    50                  55                  60

Glu Ile Ala Glu Ala Val Ala Arg Gln Ala Ala Thr Leu Asp Tyr Ala
65                  70                  75                  80

Pro Pro Phe Gln Met Gly His Pro Leu Ser Phe Glu Ala Ala Thr Lys
                85                  90                  95

Val Ala Ala Ile Met Pro Gln Gly Leu Asp Arg Ile Phe Phe Thr Asn
            100                 105                 110

Ser Gly Ser Glu Ser Val Asp Thr Ala Leu Lys Ile Ala Leu Ala Tyr
        115                 120                 125

His Arg Ala Arg Gly Glu Gly Gln Arg Thr Arg Phe Ile Gly Arg Glu
    130                 135                 140

Arg Gly Tyr His Gly Val Gly Phe Gly Gly Met Ala Val Gly Gly Ile
145                 150                 155                 160

Gly Pro Asn Arg Lys Ala Phe Ser Ala Asn Leu Met Pro Gly Thr Asp
                165                 170                 175

His Leu Pro Ala Thr Leu Asn Ile Ala Glu Ala Ala Phe Ser Lys Gly
            180                 185                 190

Gln Pro Thr Trp Gly Ala His Leu Ala Asp Glu Leu Glu Arg Ile Val
        195                 200                 205

Ala Leu His Asp Pro Ser Thr Ile Ala Ala Val Ile Val Glu Pro Leu
    210                 215                 220

Ala Gly Ser Ala Gly Val Leu Val Pro Pro Val Gly Tyr Leu Asp Lys

225                 230                 235                 240

Leu Arg Glu Ile Thr Thr Lys His Gly Ile Leu Leu Ile Phe Asp Glu
                245                 250                 255

Val Ile Thr Ala Phe Gly Arg Leu Gly Thr Ala Thr Ala Ala Glu Arg
            260                 265                 270

Phe Lys Val Thr Pro Asp Leu Ile Thr Met Ala Lys Ala Ile Asn Asn
        275                 280                 285

Ala Ala Val Pro Met Gly Ala Val Ala Val Arg Arg Glu Val His Asp
    290                 295                 300

Thr Val Val Asn Ser Ala Ala Pro Gly Ala Ile Glu Leu Ala His Gly
305                 310                 315                 320

Tyr Thr Tyr Ser Gly His Pro Leu Ala Ala Ala Ala Ala Ile Ala Thr
                325                 330                 335

Leu Asp Leu Tyr Gln Arg Glu Asn Leu Phe Gly Arg Ala Ala Glu Leu
            340                 345                 350

Ser Pro Val Phe Glu Ala Ala Val His Ser Val Arg Ser Ala Pro His
        355                 360                 365

Val Lys Asp Ile Arg Asn Leu Gly Met Val Ala Gly Ile Glu Leu Glu
    370                 375                 380

Pro Arg Pro Gly Gln Pro Gly Ala Arg Ala Tyr Glu Ala Phe Leu Lys
385                 390                 395                 400

Cys Leu Glu Arg Gly Val Leu Val Arg Tyr Thr Gly Asp Ile Leu Ala
                405                 410                 415

Phe Ser Pro Pro Leu Ile Ile Ser Glu Ala Gln Ile Ala Glu Leu Phe
            420                 425                 430

Asp Thr Val Lys Gln Ala Leu Gln Glu Val Gln
        435                 440

<210>  251
<211>  1341
<212>  DNA
<213>  Shewanella oneidensis

<400>  251
atggccgact cacccaacaa cctcgctcac gaacatcctt cacttgaaca ctattggatg      60

ccttttaccg ccaatcgcca attcaaagcg agccctcgtt tactcgccca agctgaaggt     120

atgtattaca cagatatcaa tggcaacaag gtattagact ctacagcggg cttatggtgt 180

tgtaatgctg gccatggtcg ccgtgagatc agtgaagccg tcagcaaaca aattcggcag 240

atggattacg ctccctcctt ccaaatgggc catcccatcg cttttgaact ggccgaacgt 300

ttaaccgaac tcagcccaga aggactcaac aaagtattct ttaccaactc aggctctgag 360

tcggttgata ccgcgctaaa aatggctctt tgctaccata gagccaatgg ccaagcgtca 420

cgcacccgct ttattggccg tgaaatgggt taccatggcg taggatttgg tgggatctcg 480

gtgggtggtt taagcaataa ccgtaaagcc ttcagcggcc agctattgca aggcgtggat 540

cacctgcccc acaccttaga cattcaacat gccgccttta gtcgtggctt accgagcctc 600

ggtgctgaaa aagctgaggt attagaacaa ttagtcacac tccatggcgc cgaaaatatt 660

gccgccgtta ttgttgaacc catgtcaggt tctgcagggg taattttacc acctcaaggc 720

tacttaaaac gcttacgtga aatcactaaa aaacacggca tcttattgat tttcgatgaa 780

gtcattaccg catttggccg tgtaggtgca gcattcgcca gccaacgttg gggcgttatt 840

ccagacataa tcaccacggc taaagccatt aataatggcg ccatccccat gggcgcagtg 900

tttgtacagg attatatcca cgatacttgc atgcaagggc caaccgaact gattgaattt 960

ttccacggtt atacctattc gggccaccca gtcgccgcag cagcagcact cgccacgctc 1020

tccatctacc aaaacgagca actgtttgag cgcagttttg agcttgagcg gtatttcgaa 1080

gaagccgttc atagcctcaa agggttaccg aatgtgattg atattcgcaa caccggatta 1140

gtcgcgggtt tccagctagc accgaatagc caaggtgttg gtaaacgcgg atacagcgtg 1200

ttcgagcatt gtttccatca aggcacactc gtgcgggcaa cgggcgatat tatcgccatg 1260

tccccaccac tcattgttga gaaacatcag attgaccaaa tggtaaatag ccttagcgat 1320

gcaattcacg ccgttggatg a 1341

<210> 252
<211> 446
<212> PRT
<213> beta alanine-pyruvate transaminase

<400> 252
   Met Ala Asp Ser Pro Asn Asn Leu Ala His Glu His Pro Ser Leu Glu
   1 5 10 15
   His Tyr Trp Met Pro Phe Thr Ala Asn Arg Gln Phe Lys Ala Ser Pro
   20 25 30
   Arg Leu Leu Ala Gln Ala Glu Gly Met Tyr Tyr Thr Asp Ile Asn Gly
   35 40 45
   Asn Lys Val Leu Asp Ser Thr Ala Gly Leu Trp Cys Cys Asn Ala Gly
   50 55 60
   His Gly Arg Arg Glu Ile Ser Glu Ala Val Ser Lys Gln Ile Arg Gln
   65                  70                  75                  80
   Met Asp Tyr Ala Pro Ser Phe Gln Met Gly His Pro Ile Ala Phe Glu
                   85                  90                  95
   Leu Ala Glu Arg Leu Thr Glu Leu Ser Pro Glu Gly Leu Asn Lys Val
               100                 105                 110
   Phe Phe Thr Asn Ser Gly Ser Glu Ser Val Asp Thr Ala Leu Lys Met
           115                 120                 125
   Ala Leu Cys Tyr His Arg Ala Asn Gly Gln Ala Ser Arg Thr Arg Phe
       130                 135                 140
   Ile Gly Arg Glu Met Gly Tyr His Gly Val Gly Phe Gly Gly Ile Ser
   145                 150                 155                 160
   Val Gly Gly Leu Ser Asn Asn Arg Lys Ala Phe Ser Gly Gln Leu Leu
                   165                 170                 175
   Gln Gly Val Asp His Leu Pro His Thr Leu Asp Ile Gln His Ala Ala
               180                 185                 190
   Phe Ser Arg Gly Leu Pro Ser Leu Gly Ala Glu Lys Ala Glu Val Leu
           195                 200                 205
   Glu Gln Leu Val Thr Leu His Gly Ala Glu Asn Ile Ala Ala Val Ile
       210                 215                 220
   Val Glu Pro Met Ser Gly Ser Ala Gly Val Ile Leu Pro Pro Gln Gly
   225                 230                 235                 240
   Tyr Leu Lys Arg Leu Arg Glu Ile Thr Lys Lys His Gly Ile Leu Leu
                   245                 250                 255
   Ile Phe Asp Glu Val Ile Thr Ala Phe Gly Arg Val Gly Ala Ala Phe
               260                 265                 270
   Ala Ser Gln Arg Trp Gly Val Ile Pro Asp Ile Ile Thr Thr Ala Lys
           275                 280                 285
   Ala Ile Asn Asn Gly Ala Ile Pro Met Gly Ala Val Phe Val Gln Asp
       290                 295                 300
   Tyr Ile His Asp Thr Cys Met Gln Gly Pro Thr Glu Leu Ile Glu Phe
   305                 310                 315                 320
   Phe His Gly Tyr Thr Tyr Ser Gly His Pro Val Ala Ala Ala Ala Ala
   325 330 335
   Leu Ala Thr Leu Ser Ile Tyr Gln Asn Glu Gln Leu Phe Glu Arg Ser
   340 345 350
   Phe Glu Leu Glu Arg Tyr Phe Glu Glu Ala Val His Ser Leu Lys Gly
   355 360 365
   Leu Pro Asn Val Ile Asp Ile Arg Asn Thr Gly Leu Val Ala Gly Phe
   370 375 380
   Gln Leu Ala Pro Asn Ser Gln Gly Val Gly Lys Arg Gly Tyr Ser Val
   385 390 395 400
   Phe Glu His Cys Phe His Gln Gly Thr Leu Val Arg Ala Thr Gly Asp
   405 410 415
   Ile Ile Ala Met Ser Pro Pro Leu Ile Val Glu Lys His Gln Ile Asp
   420 425 430
   Gln Met Val Asn Ser Leu Ser Asp Ala Ile His Ala Val Gly
   435 440 445

<210>  253
<211>  1347
<212>  DNA
<213>  Pseudomonas putida

<400>  253
atgaacatgc ccgaaactgg tcctgccggt atcgccagcc agctcaagct ggacgcccac      60

tggatgccct acaccgccaa ccgcaacttc cagcgcgacc cacgcctgat cgtggcggcc     120

gaaggcaact acctggtcga tgaccacggg cgcaagatct tcgacgccct gtccggcctg     180

tggacctgcg gcgcagggca cactcgcaag gaaatcgctg acgcggtgac ccgtcaactg     240

agtacgctgg actactcccc agcgttccag ttcggccacc cgctgtcgtt ccagctggcg     300

gaaaagatcg ccgagctggt tccgggcaat ctgaatcacg tcttctatac caactccggt     360

tccgagtgcg ccgataccgc actgaagatg gtgcgtgcct actggcgcct gaaaggccag     420

gcaaccaaga ccaagatcat cggccgtgcc cgtggttacc atggcgtgaa catcgccggt     480

accagcctgg gtggcgtcaa cggtaaccgc aagatgtttg gccagctgct ggacgtcgac     540

cacctgcctc acactgtatt gccggtgaac gccttctcga aaggcttgcc ggaagagggc     600

ggtatcgcgc tggctgacga aatgctcaag ctgatcgagc tgcacgatgc ctccaacatc     660

gcagcagtca tcgtcgagcc gctggccggt tcggccggtg tgctgccgcc gccaaagggt     720

tacctgaagc gcctgcgtga aatctgcacc cagcacaaca ttctgctgat cttcgacgaa     780

gtgatcacag gcttcggccg catgggcgcg atgaccggct cggaagcctt cggcgttacc     840

ccggacctga tgtgcatcgc caagcaggtg accaacggcg ccatcccgat gggcgcagtg 900

attgccagca gcgagatcta ccagaccttc atgaaccagc cgaccccgga atacgccgtg 960

gaattcccac acggctacac ctattcggcg cacccggtag cctgtgccgc cggtctcgcc 1020

gcgctggacc tgctgcagaa ggaaaacctg gtgcagtccg cggctgaact ggcgccgcat 1080

ttcgagaagc tgctgcacgg cgtgaagggc accaagaata tcgtcgatat ccgcaactac 1140

ggcctggccg gcgccatcca gatcgccgcc cgtgacggtg atgccatcgt tcgcccttac 1200

gaagcggcca tgaagctgtg gaaagcgggc ttctatgtac gctttggtgg cgacaccctg 1260

cagttcggcc caaccttcaa taccaagccg caggaactgg accgcttgtt cgatgctgtt 1320

ggcgaaaccc tgaacctgat cgactga 1347

<210> 254
<211> 448
<212> PRT
<213> Pseudomonas putida

<400> 254

Met Asn Met Pro Glu Thr Gly Pro Ala Gly Ile Ala Ser Gln Leu Lys
1               5                   10                  15

Leu Asp Ala His Trp Met Pro Tyr Thr Ala Asn Arg Asn Phe Gln Arg
            20                  25                  30

Asp Pro Arg Leu Ile Val Ala Ala Glu Gly Asn Tyr Leu Val Asp Asp
        35                  40                  45

His Gly Arg Lys Ile Phe Asp Ala Leu Ser Gly Leu Trp Thr Cys Gly
    50                  55                  60

Ala Gly His Thr Arg Lys Glu Ile Ala Asp Ala Val Thr Arg Gln Leu
65                  70                  75                  80

Ser Thr Leu Asp Tyr Ser Pro Ala Phe Gln Phe Gly His Pro Leu Ser
                85                  90                  95

Phe Gln Leu Ala Glu Lys Ile Ala Glu Leu Val Pro Gly Asn Leu Asn
            100                 105                 110

His Val Phe Tyr Thr Asn Ser Gly Ser Glu Cys Ala Asp Thr Ala Leu
        115                 120                 125

Lys Met Val Arg Ala Tyr Trp Arg Leu Lys Gly Gln Ala Thr Lys Thr
    130                 135                 140

Lys Ile Ile Gly Arg Ala Arg Gly Tyr His Gly Val Asn Ile Ala Gly
145                 150                 155                 160

Thr Ser Leu Gly Gly Val Asn Gly Asn Arg Lys Met Phe Gly Gln Leu
                165                 170                 175

Leu Asp Val Asp His Leu Pro His Thr Val Leu Pro Val Asn Ala Phe
            180                 185                 190

Ser Lys Gly Leu Pro Glu Glu Gly Gly Ile Ala Leu Ala Asp Glu Met
        195                 200                 205

Leu Lys Leu Ile Glu Leu His Asp Ala Ser Asn Ile Ala Ala Val Ile
    210                 215                 220

Val Glu Pro Leu Ala Gly Ser Ala Gly Val Leu Pro Pro Pro Lys Gly
225                 230                 235                 240

Tyr Leu Lys Arg Leu Arg Glu Ile Cys Thr Gln His Asn Ile Leu Leu
                245                 250                 255

Ile Phe Asp Glu Val Ile Thr Gly Phe Gly Arg Met Gly Ala Met Thr
            260                 265                 270

Gly Ser Glu Ala Phe Gly Val Thr Pro Asp Leu Met Cys Ile Ala Lys
        275                 280                 285

Gln Val Thr Asn Gly Ala Ile Pro Met Gly Ala Val Ile Ala Ser Ser
    290                 295                 300

Glu Ile Tyr Gln Thr Phe Met Asn Gln Pro Thr Pro Glu Tyr Ala Val
305                 310                 315                 320

Glu Phe Pro His Gly Tyr Thr Tyr Ser Ala His Pro Val Ala Cys Ala
                325                 330                 335

Ala Gly Leu Ala Ala Leu Asp Leu Leu Gln Lys Glu Asn Leu Val Gln
            340                 345                 350

Ser Ala Ala Glu Leu Ala Pro His Phe Glu Lys Leu Leu His Gly Val
        355                 360                 365

Lys Gly Thr Lys Asn Ile Val Asp Ile Arg Asn Tyr Gly Leu Ala Gly
    370                 375                 380

Ala Ile Gln Ile Ala Ala Arg Asp Gly Asp Ala Ile Val Arg Pro Tyr
385                 390                 395                 400

Glu Ala Ala Met Lys Leu Trp Lys Ala Gly Phe Tyr Val Arg Phe Gly
                405                 410                 415

Gly Asp Thr Leu Gln Phe Gly Pro Thr Phe Asn Thr Lys Pro Gln Glu
            420                 425                 430

Leu Asp Arg Leu Phe Asp Ala Val Gly Glu Thr Leu Asn Leu Ile Asp
        435                 440                 445

<210>  255
<211>  1701
<212>  DNA
<213>  Streptomyces cinnamonensis

<400>  255
atggacgctg acgcgatcga ggaaggccgc cgacgctggc aggcccgtta cgacaaggcc      60

cgcaagcgcg acgcggactt caccacgctc tccggggacc ccgtcgaccc cgtctacggc     120

ccccggcccg gggacacgta cgacgggttc gagcggatcg gctggccggg ggagtacccc     180

ttcacccgcg ggctctacgc caccgggtac cgcggccgca cctggaccat ccgccagttc     240

gccggcttcg gcaacgccga gcagacgaac gagcgctaca agatgatcct ggccaacggc     300

ggcggcggcc tctccgtcgc cttcgacatg ccgaccctca tgggccgcga ctccgacgac     360

ccgcgctcgc tcggcgaggt cggccactgc ggtgtcgcca tcgactccgc cgccgacatg     420

gaggtcctct tcaaggacat cccgctcggc gacgtcacga cgtccatgac catcagcggg     480

cccgccgtgc ccgtcttctg catgtacctc gtcgcggccg agcgccaggg cgtcgacccg     540

gccgtcctca acggcacgct gcagaccgac atcttcaagg agtacatcgc ccagaaggag     600

tggctcttcc agcccgagcc gcacctgcgc ctcatcggcg acctgatgga gcactgcgcg     660

cgcgacatcc ccgcgtacaa gccgctctcg gtctccggct accacatccg cgaggccggg     720

gcgacggccg cgcaggagct cgcgtacacc ctcgcggacg gcttcgggta cgtggaactg     780

ggcctctcgc gcggcctgga cgtggacgtc ttcgcgcccg gcctctcctt cttcttcgac     840

gcgcacgtcg acttcttcga ggagatcgcg aagttccgcg ccgcacgccg catctgggcg     900

cgctggctcc gggacgagta cggagcgaag accgagaagg cacagtggct gcgcttccac     960

acgcagaccg cgggggtctc gctcacggcc cagcagccgt acaacaacgt ggtgcggacg    1020

gcggtggagg ccctcgccgc ggtgctcggc ggcacgaact ccctgcacac caacgctctc    1080

gacgagaccc ttgccctccc cagcgagcag gccgcggaga tcgcgctgcg cacccagcag    1140

gtgctgatgg aggagaccgg cgtcgccaac gtcgcggacc cgctgggcgg ctcctggtac    1200

atcgagcagc tcaccgaccg catcgaggcc gacgccgaga agatcttcga gcagatcagg    1260

gagcgggggc ggcgggcctg ccccgacggg cagcacccga tcgggccgat cacctccggc    1320

atcctgcgcg gcatcgagga cggctggttc accggcgaga tcgccgagtc cgccttccag    1380

taccagcggt ccctggagaa gggcgacaag cgggtcgtcg gcgtcaactg cctcgaaggc    1440

tccgtcaccg gcgacctgga gatcctgcgc gtcagccacg aggtcgagcg cgagcaggtg    1500

cgggagcttg cggggcgcaa ggggcggcgt gacgatgcgc gggtgcgggc ctcgctcgac 1560

gcgatgctcg ccgctgcgcg ggacgggtcg aacatgattg cccccatgct ggaggcggtg 1620

cgggccgagg cgaccctcgg ggagatctgc ggggtgcttc gcgatgagtg gggggtctac 1680

gtggagccgc ccgggttctg a 1701

<210> 256
<211> 566
<212> PRT
<213> Streptomyces cinnamonensis

<400> 256

Met Asp Ala Asp Ala Ile Glu Glu Gly Arg Arg Arg Trp Gln Ala Arg
1               5                   10                  15

Tyr Asp Lys Ala Arg Lys Arg Asp Ala Asp Phe Thr Thr Leu Ser Gly
            20                  25                  30

Asp Pro Val Asp Pro Val Tyr Gly Pro Arg Pro Gly Asp Thr Tyr Asp
        35                  40                  45

Gly Phe Glu Arg Ile Gly Trp Pro Gly Glu Tyr Pro Phe Thr Arg Gly
    50                  55                  60

Leu Tyr Ala Thr Gly Tyr Arg Gly Arg Thr Trp Thr Ile Arg Gln Phe
65                  70                  75                  80

Ala Gly Phe Gly Asn Ala Glu Gln Thr Asn Glu Arg Tyr Lys Met Ile
                85                  90                  95

Leu Ala Asn Gly Gly Gly Gly Leu Ser Val Ala Phe Asp Met Pro Thr
            100                 105                 110

Leu Met Gly Arg Asp Ser Asp Asp Pro Arg Ser Leu Gly Glu Val Gly
        115                 120                 125

His Cys Gly Val Ala Ile Asp Ser Ala Ala Asp Met Glu Val Leu Phe
    130                 135                 140

Lys Asp Ile Pro Leu Gly Asp Val Thr Thr Ser Met Thr Ile Ser Gly
145                 150                 155                 160

Pro Ala Val Pro Val Phe Cys Met Tyr Leu Val Ala Ala Glu Arg Gln
                165                 170                 175

Gly Val Asp Pro Ala Val Leu Asn Gly Thr Leu Gln Thr Asp Ile Phe
            180                 185                 190

Lys Glu Tyr Ile Ala Gln Lys Glu Trp Leu Phe Gln Pro Glu Pro His

        195                 200                 205

Leu Arg Leu Ile Gly Asp Leu Met Glu His Cys Ala Arg Asp Ile Pro
    210                 215                 220

Ala Tyr Lys Pro Leu Ser Val Ser Gly Tyr His Ile Arg Glu Ala Gly
225                 230                 235                 240

Ala Thr Ala Ala Gln Glu Leu Ala Tyr Thr Leu Ala Asp Gly Phe Gly
                245                 250                 255

Tyr Val Glu Leu Gly Leu Ser Arg Gly Leu Asp Val Asp Val Phe Ala
            260                 265                 270

Pro Gly Leu Ser Phe Phe Phe Asp Ala His Val Asp Phe Phe Glu Glu
        275                 280                 285

Ile Ala Lys Phe Arg Ala Ala Arg Arg Ile Trp Ala Arg Trp Leu Arg
    290                 295                 300

Asp Glu Tyr Gly Ala Lys Thr Glu Lys Ala Gln Trp Leu Arg Phe His
305                 310                 315                 320

Thr Gln Thr Ala Gly Val Ser Leu Thr Ala Gln Gln Pro Tyr Asn Asn
                325                 330                 335

Val Val Arg Thr Ala Val Glu Ala Leu Ala Ala Val Leu Gly Gly Thr
            340                 345                 350

Asn Ser Leu His Thr Asn Ala Leu Asp Glu Thr Leu Ala Leu Pro Ser
        355                 360                 365

Glu Gln Ala Ala Glu Ile Ala Leu Arg Thr Gln Gln Val Leu Met Glu
    370                 375                 380

Glu Thr Gly Val Ala Asn Val Ala Asp Pro Leu Gly Gly Ser Trp Tyr
385                 390                 395                 400

Ile Glu Gln Leu Thr Asp Arg Ile Glu Ala Asp Ala Glu Lys Ile Phe
                405                 410                 415

Glu Gln Ile Arg Glu Arg Gly Arg Arg Ala Cys Pro Asp Gly Gln His
            420                 425                 430

Pro Ile Gly Pro Ile Thr Ser Gly Ile Leu Arg Gly Ile Glu Asp Gly
        435                 440                 445

Trp Phe Thr Gly Glu Ile Ala Glu Ser Ala Phe Gln Tyr Gln Arg Ser
    450                 455                 460

Leu Glu Lys Gly Asp Lys Arg Val Val Gly Val Asn Cys Leu Glu Gly
465                 470                 475                 480

Ser Val Thr Gly Asp Leu Glu Ile Leu Arg Val Ser His Glu Val Glu
                485                 490                 495

Arg Glu Gln Val Arg Glu Leu Ala Gly Arg Lys Gly Arg Arg Asp Asp
            500                 505                 510

Ala Arg Val Arg Ala Ser Leu Asp Ala Met Leu Ala Ala Ala Arg Asp
        515                 520                 525

Gly Ser Asn Met Ile Ala Pro Met Leu Glu Ala Val Arg Ala Glu Ala
    530                 535                 540

Thr Leu Gly Glu Ile Cys Gly Val Leu Arg Asp Glu Trp Gly Val Tyr
545                 550                 555                 560

Val Glu Pro Pro Gly Phe
                565

<210>  257
<211>  411
<212>  DNA
<213>  Streptomyces cinnamonensis

<400>  257
atgggtgtgg cagccgggcc gatccgcgtg gtggtcgcca agccggggct cgacgggcac      60

gatcgcgggg ccaaggtgat cgcgcgggcg ttgcgtgacg cgggtatgga ggtcatctac     120

accgggctgc accagacgcc cgagcaggtg gtggacaccg cgatccagga ggacgccgac     180

gcgatcggcc tctccatcct ctccggagcg cacaacacgc tgttcgcgcg cgtgttggag     240

ctcttgaagg agcgggacgc ggaggacatc aaggtgtttg gtggcggcat catcccggag     300

gcggacatcg cgccgctgaa ggagaagggc gtcgcggaga tcttcacgcc cggggccacc     360

accacgtcga tcgtggagtg ggttcggggg aacgtgcgac aggccgtctg a              411

<210>  258
<211>  136
<212>  PRT
<213>  Streptomyces cinnamonensis

<400>  258

Met Gly Val Ala Ala Gly Pro Ile Arg Val Val Val Ala Lys Pro Gly
1               5                   10                  15

Leu Asp Gly His Asp Arg Gly Ala Lys Val Ile Ala Arg Ala Leu Arg
            20                  25                  30

Asp Ala Gly Met Glu Val Ile Tyr Thr Gly Leu His Gln Thr Pro Glu
        35                  40                  45

Gln Val Val Asp Thr Ala Ile Gln Glu Asp Ala Asp Ala Ile Gly Leu
    50                  55                  60

Ser Ile Leu Ser Gly Ala His Asn Thr Leu Phe Ala Arg Val Leu Glu
65                  70                  75                  80

Leu Leu Lys Glu Arg Asp Ala Glu Asp Ile Lys Val Phe Gly Gly Gly
                85                  90                  95

Ile Ile Pro Glu Ala Asp Ile Ala Pro Leu Lys Glu Lys Gly Val Ala
            100                 105                 110

Glu Ile Phe Thr Pro Gly Ala Thr Thr Thr Ser Ile Val Glu Trp Val
        115                 120                 125

Arg Gly Asn Val Arg Gln Ala Val
    130                 135

<210> 259
<211> 1701
<212> DNA
<213> Streptomyces coelicolor

<400> 259
atggacgctc atgccataga ggagggccgc cttcgctggc aggcccggta cgacgcggcg 60

cgcaagcgcg acgcggactt caccacgctc tccggagacc ccgtggagcc ggtgtacggg 120

ccccgccccg gggacgagta cgagggcttc gagcggatcg gctggccggg cgagtacccc 180

ttcacccgcg gcctgtatcc gaccgggtac cgggggcgta cgtggaccat ccggcagttc 240

gccgggttcg gcaacgccga gcagaccaac gagcgctaca agatgatcct ccgcaacggc 300

ggcggcgggc tctcggtcgc cttcgacatg ccgaccctga tgggccgcga ctccgacgac 360

ccgcgctcgc tgggcgaggt cgggcactgc ggggtggcca tcgactcggc cgccgacatg 420

gaagtgctgt tcaaggacat cccgctcggg gacgtgacga cctccatgac gatcagcggg 480

cccgccgtgc ccgtgttctg catgtacctc gtcgccgccg agcgccaggg cgtcgacgca 540

tccgtgctca acggcacgct gcagaccgac atcttcaagg agtacatcgc ccagaaggag 600

tggctcttcc agcccgagcc ccacctccgg ctcatcggcg acctcatgga gtactgcgcg 660

gccggcatcc ccgcctacaa gccgctctcc gtctccggct accacatccg cgaggcgggc 720

gcgacggccg cgcaggagct ggcgtacacg ctcgccgacg gcttcggata cgtggagctg 780

ggcctcagcc gcgggctcga cgtggacgtc ttcgcgcccg gcctctcctt cttcttcgac 840

gcgcacctcg acttcttcga ggagatcgcc aagttccgcg cggcccgcag gatctgggcc 900

cgctggatgc gcgacgtgta cggcgcgcgg accgacaagg cccagtggct gcggttccac 960

acccagaccg ccggagtctc gctcaccgcg cagcagccgt acaacaacgt cgtacgcacc 1020

gcggtggagg cgctggcggc cgtgctcggc ggcaccaact ccctgcacac caacgcgctc 1080

gacgagaccc tcgccctgcc cagcgagcag gccgccgaga tcgccctgcg cacccagcag 1140

gtgctgatgg aggagaccgg cgtcgccaac gtcgccgacc cgctgggcgg ttcctggttc 1200

atcgagcagc tgaccgaccg catcgaggcc gacgccgaga agatcttcga gcagatcaag 1260

gagcgggggc tgcgcgccca ccccgacggg cagcaccccg tcggaccgat cacctccggc 1320

ctgctgcgcg gcatcgagga cggctggttc accggcgaga tcgccgagtc cgccttccgc 1380

taccagcagt ccttggagaa ggacgacaag aaggtggtcg gcgtcaacgt ccacaccggc 1440

tccgtcaccg gcgacctgga gatcctgcgg gtcagccacg aggtcgagcg cgagcaggtg 1500

cgggtcctgg gcgagcgcaa ggacgcccgg gacgacgccg ccgtgcgcgg cgccctggac 1560

gccatgctgg ccgcggcccg ctccggcggc aacatgatcg ggccgatgct ggacgcggtg 1620

cgcgcggagg cgacgctggg cgagatctgc ggtgtgctgc gcgacgagtg gggggtgtac 1680

acggaaccgg cggggttctg a 1701

<210> 260
<211> 566
<212> PRT
<213> Streptomyces coelicolor

<400> 260

Met Asp Ala His Ala Ile Glu Glu Gly Arg Leu Arg Trp Gln Ala Arg
1               5                   10                  15

Tyr Asp Ala Ala Arg Lys Arg Asp Ala Asp Phe Thr Thr Leu Ser Gly
            20                  25                  30

Asp Pro Val Glu Pro Val Tyr Gly Pro Arg Pro Gly Asp Glu Tyr Glu
        35                  40                  45

Gly Phe Glu Arg Ile Gly Trp Pro Gly Glu Tyr Pro Phe Thr Arg Gly
    50                  55                  60

Leu Tyr Pro Thr Gly Tyr Arg Gly Arg Thr Trp Thr Ile Arg Gln Phe
65                  70                  75                  80

Ala Gly Phe Gly Asn Ala Glu Gln Thr Asn Glu Arg Tyr Lys Met Ile
                85                  90                  95

Leu Arg Asn Gly Gly Gly Gly Leu Ser Val Ala Phe Asp Met Pro Thr
            100                 105                 110

Leu Met Gly Arg Asp Ser Asp Asp Pro Arg Ser Leu Gly Glu Val Gly

115                 120                 125

His Cys Gly Val Ala Ile Asp Ser Ala Ala Asp Met Glu Val Leu Phe
    130                 135                 140

Lys Asp Ile Pro Leu Gly Asp Val Thr Thr Ser Met Thr Ile Ser Gly
145                 150                 155                 160

Pro Ala Val Pro Val Phe Cys Met Tyr Leu Val Ala Ala Glu Arg Gln
                165                 170                 175

Gly Val Asp Ala Ser Val Leu Asn Gly Thr Leu Gln Thr Asp Ile Phe
            180                 185                 190

Lys Glu Tyr Ile Ala Gln Lys Glu Trp Leu Phe Gln Pro Glu Pro His
        195                 200                 205

Leu Arg Leu Ile Gly Asp Leu Met Glu Tyr Cys Ala Ala Gly Ile Pro
    210                 215                 220

Ala Tyr Lys Pro Leu Ser Val Ser Gly Tyr His Ile Arg Glu Ala Gly
225                 230                 235                 240

Ala Thr Ala Ala Gln Glu Leu Ala Tyr Thr Leu Ala Asp Gly Phe Gly
                245                 250                 255

Tyr Val Glu Leu Gly Leu Ser Arg Gly Leu Asp Val Asp Val Phe Ala
            260                 265                 270

Pro Gly Leu Ser Phe Phe Phe Asp Ala His Leu Asp Phe Phe Glu Glu
        275                 280                 285

Ile Ala Lys Phe Arg Ala Ala Arg Arg Ile Trp Ala Arg Trp Met Arg
    290                 295                 300

Asp Val Tyr Gly Ala Arg Thr Asp Lys Ala Gln Trp Leu Arg Phe His
305                 310                 315                 320

Thr Gln Thr Ala Gly Val Ser Leu Thr Ala Gln Gln Pro Tyr Asn Asn
                325                 330                 335

Val Val Arg Thr Ala Val Glu Ala Leu Ala Ala Val Leu Gly Gly Thr
            340                 345                 350

Asn Ser Leu His Thr Asn Ala Leu Asp Glu Thr Leu Ala Leu Pro Ser
        355                 360                 365

Glu Gln Ala Ala Glu Ile Ala Leu Arg Thr Gln Gln Val Leu Met Glu
    370                 375                 380

Glu Thr Gly Val Ala Asn Val Ala Asp Pro Leu Gly Gly Ser Trp Phe
385                 390                 395                 400

Ile Glu Gln Leu Thr Asp Arg Ile Glu Ala Asp Ala Glu Lys Ile Phe
                405                 410                 415

Glu Gln Ile Lys Glu Arg Gly Leu Arg Ala His Pro Asp Gly Gln His
            420                 425                 430

Pro Val Gly Pro Ile Thr Ser Gly Leu Leu Arg Gly Ile Glu Asp Gly
        435                 440                 445

Trp Phe Thr Gly Glu Ile Ala Glu Ser Ala Phe Arg Tyr Gln Gln Ser
    450                 455                 460

Leu Glu Lys Asp Asp Lys Lys Val Val Gly Val Asn Val His Thr Gly
465                 470                 475                 480

Ser Val Thr Gly Asp Leu Glu Ile Leu Arg Val Ser His Glu Val Glu
                485                 490                 495

Arg Glu Gln Val Arg Val Leu Gly Glu Arg Lys Asp Ala Arg Asp Asp
            500                 505                 510

Ala Ala Val Arg Gly Ala Leu Asp Ala Met Leu Ala Ala Ala Arg Ser
        515                 520                 525

Gly Gly Asn Met Ile Gly Pro Met Leu Asp Ala Val Arg Ala Glu Ala
    530                 535                 540

Thr Leu Gly Glu Ile Cys Gly Val Leu Arg Asp Glu Trp Gly Val Tyr
545                 550                 555                 560

Thr Glu Pro Ala Gly Phe
                565

<210>  261
<211>  417
<212>  DNA
<213>  Streptomyces coelicolor

<400>  261
atgggtgtgg cagccggtcc gatccgcgtg gtggtggcca agccggggct cgacggccac      60

gatcgcgggg ccaaggtgat cgcgagggcc ctgcgtgacg ccggtatgga ggtgatctac     120

accgggctcc accagacgcc cgagcagatc gtcgacaccg cgatccagga ggacgccgac     180

gcgatcgggc tgtccatcct ctccggtgcg cacaacacgc tcttcgccgc cgtgatcgag     240

ctgctccggg agcgggacgc cgcggacatc ctggtcttcg gcggcgggat catccccgag     300

gcggacatcg ccccgctgaa ggagaagggc gtcgcggaga tcttcacgcc cggcgccacc 360

acggcgtcca tcgtggactg ggtccgggcg aacgtgcggg agcccgcggg agcatag 417

<210> 262
<211> 138
<212> PRT
<213> Streptomyces coelicolor

<400> 262

Met Gly Val Ala Ala Gly Pro Ile Arg Val Val Val Ala Lys Pro Gly
1               5                   10                  15

Leu Asp Gly His Asp Arg Gly Ala Lys Val Ile Ala Arg Ala Leu Arg
            20                  25                  30

Asp Ala Gly Met Glu Val Ile Tyr Thr Gly Leu His Gln Thr Pro Glu
        35                  40                  45

Gln Ile Val Asp Thr Ala Ile Gln Glu Asp Ala Asp Ala Ile Gly Leu
    50                  55                  60

Ser Ile Leu Ser Gly Ala His Asn Thr Leu Phe Ala Ala Val Ile Glu
65                  70                  75                  80

Leu Leu Arg Glu Arg Asp Ala Ala Asp Ile Leu Val Phe Gly Gly Gly
                85                  90                  95

Ile Ile Pro Glu Ala Asp Ile Ala Pro Leu Lys Glu Lys Gly Val Ala
            100                 105                 110

Glu Ile Phe Thr Pro Gly Ala Thr Thr Ala Ser Ile Val Asp Trp Val
        115                 120                 125

Arg Ala Asn Val Arg Glu Pro Ala Gly Ala
    130                 135

<210> 263
<211> 1701
<212> DNA
<213> Streptomyces avermitilis

<400> 263
tcagaaaccg gcgggctccg tgtagacccc ccactcctcc cggaggacat cgcagatctc 60

gcccagcgtg gcctccgcgc ggaccgcgtc cagcatcggg gcgatcatgt tcgacccgtc 120

gcgcgcggcg gcgagcatcg cgtccagggc cgcggttacg gccgtgtcgt cgcgccccga 180

cttccgctcg cccagcaccc gcacctgctc gcgctccacc tcgtggctga cgcgcaggat 240

ctccaggtcg cccgtcacgg acccgtggtg gacgttgacg ccgacgaccc gcttgtcgcc 300

cttctccagc gcctgctggt actggaaggc cgactcggcg atctccccgg tgaaccagcc 360

gtcctcgatg ccgcgcagga tgccggaggt gatgggcccg atcgggtgcc gcccgtccgg 420

gtgggcccgc agcccgcgct ccctgatctg ttcgaagatc ttctcggcgt cggcctcgat 480

ccggtcggtc agctgctcca cgtaccagga accgcccagc ggatcggcca cgttggcgac 540

gcccgtctcc tccatcagca cctgctgggt gcgcagggcg atctcggccg cctgctcgga 600

cggcagggcg agggtctcgt cgagggcgtt ggtgtgcagc gagttcgtcc cgccgagcac 660

cgcggcgagg gcctccacgg ccgtccgtac gacgttgttg tacggctgct gcgcggtgag 720

cgagacgccc gcggtctggg tgtggaagcg cagccactgc gccttctccg acttcgcccc 780

gtacacgtcc cgcagccagc gcgcccagat gcgccgcgcc gcacggaact tggcgatctc 840

ctcgaagaag tcgacgtgcg cgtcgaagaa gaaggagagc ccgggcgcga acacgtccac 900

gtccaggccg cggctcagcc ccagctccac gtatccgaaa ccgtcggcga gggtgtacgc 960

cagctcctgg gcggccgtgg caccggcctc ccggatgtgg tacccggaga cggacagcgg 1020

cttgtacgcg gggatcttcg aggcgcagtg ctccatcagg tcgccgatga gccgcagatg 1080

gggctcgggc tggaagagcc actccttctg cgcgatgtac tccttgaaga tgtcggtctg 1140

gagggtgccg ttgaggacgg aggggtcgac gccctgccgc tcggccgcga ccaggtacat 1200

gcagaagacg ggcacggcgg gcccgctgat cgtcatcgac gtcgtcacgt cacccagcgg 1260

gatgtccttg aacaggacct ccatgtcggc cgccgagtcg atcgcgaccc cgcagtgccc 1320

gacctcgccg agcgcgcggc ggtcgtcgga gtcgcgcccc atgagcgtcg gcatgtcgaa 1380

ggccacggac agcccaccgc cgccgttggc gaggatcttc ttgtagcgct cgttggtctg 1440

ctcggcgttg ccgaacccgg cgaactgccg gatggtccag gtccggcccc ggtagccggt 1500

cggatacaga ccgcgcgtga aggggtactc acccggccag ccgatccgct cgaaaccctc 1560

gtacgcgtcc ccgggccggg gcccgtacgc cggctccacg ggatcgccgg agagcgtggt 1620

gaaatcggcc tcgcgcttgc gtgaggcgtc gtagcgggcc tgccagcgtc ggcggccttc 1680

ctcgatggcg tcagcgtcca t 1701

<210> 264
<211> 566
<212> PRT
<213> Streptomyces avermitilis

<400> 264

Met Asp Ala Asp Ala Ile Glu Glu Gly Arg Arg Arg Trp Gln Ala Arg
1               5                   10                  15

Tyr Asp Ala Ser Arg Lys Arg Glu Ala Asp Phe Thr Thr Leu Ser Gly
            20                  25                  30

Asp Pro Val Glu Pro Ala Tyr Gly Pro Arg Pro Gly Asp Ala Tyr Glu

35                  40                  45

Gly Phe Glu Arg Ile Gly Trp Pro Gly Glu Tyr Pro Phe Thr Arg Gly
    50                  55                  60

Leu Tyr Pro Thr Gly Tyr Arg Gly Arg Thr Trp Thr Ile Arg Gln Phe
65                  70                  75                  80

Ala Gly Phe Gly Asn Ala Glu Gln Thr Asn Glu Arg Tyr Lys Lys Ile
                85                  90                  95

Leu Ala Asn Gly Gly Gly Gly Leu Ser Val Ala Phe Asp Met Pro Thr
            100                 105                 110

Leu Met Gly Arg Asp Ser Asp Asp Arg Arg Ala Leu Gly Glu Val Gly
        115                 120                 125

His Cys Gly Val Ala Ile Asp Ser Ala Ala Asp Met Glu Val Leu Phe
    130                 135                 140

Lys Asp Ile Pro Leu Gly Asp Val Thr Thr Ser Met Thr Ile Ser Gly
145                 150                 155                 160

Pro Ala Val Pro Val Phe Cys Met Tyr Leu Val Ala Ala Glu Arg Gln
                165                 170                 175

Gly Val Asp Pro Ser Val Leu Asn Gly Thr Leu Gln Thr Asp Ile Phe
            180                 185                 190

Lys Glu Tyr Ile Ala Gln Lys Glu Trp Leu Phe Gln Pro Glu Pro His
        195                 200                 205

Leu Arg Leu Ile Gly Asp Leu Met Glu His Cys Ala Ser Lys Ile Pro
    210                 215                 220

Ala Tyr Lys Pro Leu Ser Val Ser Gly Tyr His Ile Arg Glu Ala Gly
225                 230                 235                 240

Ala Thr Ala Ala Gln Glu Leu Ala Tyr Thr Leu Ala Asp Gly Phe Gly
                245                 250                 255

Tyr Val Glu Leu Gly Leu Ser Arg Gly Leu Asp Val Asp Val Phe Ala
            260                 265                 270

Pro Gly Leu Ser Phe Phe Phe Asp Ala His Val Asp Phe Phe Glu Glu
        275                 280                 285

Ile Ala Lys Phe Arg Ala Ala Arg Arg Ile Trp Ala Arg Trp Leu Arg
    290                 295                 300

Asp Val Tyr Gly Ala Lys Ser Glu Lys Ala Gln Trp Leu Arg Phe His
305                 310                 315                 320

Thr Gln Thr Ala Gly Val Ser Leu Thr Ala Gln Gln Pro Tyr Asn Asn
                325                 330                 335

Val Val Arg Thr Ala Val Glu Ala Leu Ala Ala Val Leu Gly Gly Thr
            340                 345                 350

Asn Ser Leu His Thr Asn Ala Leu Asp Glu Thr Leu Ala Leu Pro Ser
        355                 360                 365

Glu Gln Ala Ala Glu Ile Ala Leu Arg Thr Gln Gln Val Leu Met Glu
    370                 375                 380

Glu Thr Gly Val Ala Asn Val Ala Asp Pro Leu Gly Gly Ser Trp Tyr
385                 390                 395                 400

Val Glu Gln Leu Thr Asp Arg Ile Glu Ala Asp Ala Glu Lys Ile Phe
                405                 410                 415

Glu Gln Ile Arg Glu Arg Gly Leu Arg Ala His Pro Asp Gly Arg His
            420                 425                 430

Pro Ile Gly Pro Ile Thr Ser Gly Ile Leu Arg Gly Ile Glu Asp Gly
        435                 440                 445

Trp Phe Thr Gly Glu Ile Ala Glu Ser Ala Phe Gln Tyr Gln Gln Ala
    450                 455                 460

Leu Glu Lys Gly Asp Lys Arg Val Val Gly Val Asn Val His His Gly
465                 470                 475                 480

Ser Val Thr Gly Asp Leu Glu Ile Leu Arg Val Ser His Glu Val Glu
                485                 490                 495

Arg Glu Gln Val Arg Val Leu Gly Glu Arg Lys Ser Gly Arg Asp Asp
            500                 505                 510

Thr Ala Val Thr Ala Ala Leu Asp Ala Met Leu Ala Ala Ala Arg Asp
        515                 520                 525

Gly Ser Asn Met Ile Ala Pro Met Leu Asp Ala Val Arg Ala Glu Ala
    530                 535                 540

Thr Leu Gly Glu Ile Cys Asp Val Leu Arg Glu Glu Trp Gly Val Tyr
545                 550                 555                 560

Thr Glu Pro Ala Gly Phe
                565

<210>  265
<211>  417
<212>  DNA
<213>  Streptomyces avermitilis

<400>  265
ctacgccccg gcaggctgcc gcacgttcgc ccgcacccac tccacgatcg acgccgtggt      60

cgcccccgga gtgaagatct ccgcgacacc cttctccttc agcggcgcga tgtccgcctc     120

ggggatgatg ccgccaccga acaccttgat gtcctcggca tcgcgctcct tgagcagatc     180

gatgaccgcc gcgaacaacg tgttgtgcgc cccggacagg atcgacagcc cgatcgcgtc     240

ggcgtcctcc tggatggccg tgcccacgat ctgctccggc gtctggtgca gccccgtgta     300

aatgacctcc ataccggcat cgcgcagcgc ccgcgcgatc accttggccc cgcgatcgtg     360

gccatcgagc ccggcttggc ccaccaccac gcggatcgga ccggctgcca cacccat        417

<210>  266
<211>  138
<212>  PRT
<213>  Streptomyces avermitilis

<400>  266

Met Gly Val Ala Ala Gly Pro Ile Arg Val Val Val Ala Lys Pro Gly
1               5                   10                  15

Leu Asp Gly His Asp Arg Gly Ala Lys Val Ile Ala Arg Ala Leu Arg
            20                  25                  30

Asp Ala Gly Met Glu Val Ile Tyr Thr Gly Leu His Gln Thr Pro Glu
        35                  40                  45

Gln Ile Val Gly Thr Ala Ile Gln Glu Asp Ala Asp Ala Ile Gly Leu
    50                  55                  60

Ser Ile Leu Ser Gly Ala His Asn Thr Leu Phe Ala Ala Val Ile Asp
65                  70                  75                  80

Leu Leu Lys Glu Arg Asp Ala Glu Asp Ile Lys Val Phe Gly Gly Gly
                85                  90                  95

Ile Ile Pro Glu Ala Asp Ile Ala Pro Leu Lys Glu Lys Gly Val Ala
            100                 105                 110

Glu Ile Phe Thr Pro Gly Ala Thr Thr Ala Ser Ile Val Glu Trp Val
        115                 120                 125

Arg Ala Asn Val Arg Gln Pro Ala Gly Ala
    130                 135

<210>  267
<211>  2910
<212>  DNA
<213>  Staphylococcus aureus

<400>  267
gatcaatttc ttttaagtaa tctaaatccc cattttttaa tttcttttta gcctctttaa      60

ataatcctga ataaactaat acctgtttac ctttaagtga tttataaaat gcatcaaaga     120

ctttttgatt tatttattaa ataatcacta tctttaccag aatacttagc catttcatat     180

aattctttat tattattttg tcttattttt tgaacttgaa cttgtgttat ttctgaaatg     240

cccgttacat cacgccataa atctaaccat tcttgttggc taatataata tcttttatct     300

gtgaaatacg atttatttac tgcaattaac acatgaaaat gaggattata atcatctctt     360

tttttattat atgtaatctc taacttacga acatatccct ttataacact acctactttt     420

tttctcttta taagttttct aaaagaatta ttataacgtt ttatttcatt ttctaattca     480

tcactcatta cattaggtgt agtcaaagtt aaaaagataa actccttttt ctcttgctgc     540

ttaatatatt gcatcatcaa agataaaccc aatgcatctt ttctagcttt tctccaagca     600

cagacaggac aaaatcgatt tttacaagaa ttagctttat ataatttctg tttttctaaa     660

gttttatcag ctacaaaaga cagaaatgta ttgcaatctt caactaaatc catttgattc     720

tctccaatat gacgtttaat aaatttctga aatacttgat ttctttgttt tttctcagta     780

tacttttcca tgttataaca cataaaaaca acttagtttt cacaaactat gacaataaaa     840

aaagttgctt tttccccttt ctatgtatgt tttttactag tcatttaaaa cgatacatta     900

ataggtacga aaaagcaact ttttttgcgc ttaaaaccag tcataccaat aacttaaggg     960

taactagcct cgccggcaat agttaccctt attatcaaga taagaaagaa aaggattttt    1020

cgctacgctc aaatccttta aaaaaacaca aaagaccaca ttttttaatg tggtctttat    1080

tcttcaacta aagcacccat tagttcaaca aacgaaaatt ggataaagtg ggatattttt    1140

aaaatatata tttatgttac agtaatattg acttttaaaa aaggattgat tctaatgaag    1200

aaagcagaca agtaagcctc ctaaattcac tttagataaa aatttaggag gcatatcaaa    1260

tgaactttaa taaaattgat ttagacaatt ggaagagaaa agagatattt aatcattatt    1320

tgaaccaaca aacgactttt agtataacca cagaaattga tattagtgtt ttataccgaa    1380

acataaaaca agaaggatat aaattttacc ctgcatttat tttcttagtg acaagggtga    1440

taaactcaaa tacagctttt agaactggtt acaatagcga cggagagtta ggttattggg    1500

ataagttaga gccactttat acaatttttg atggtgtatc taaaacattc tctggtattt    1560

ggactcctgt aaagaatgac ttcaaagagt tttatgattt atacctttct gatgtagaga    1620

aatataatgg ttcggggaaa ttgtttccca aaacacctat acctgaaaat gctttttctc    1680

tttctattat tccatggact tcatttactg ggtttaactt aaatatcaat aataatagta 1740

attaccttct acccattatt acagcaggaa aattcattaa taaaggtaat tcaatatatt 1800

taccgctatc tttacaggta catcattctg tttgtgatgg ttatcatgca ggattgttta 1860

tgaactctat tcaggaattg tcagataggc ctaatgactg gcttttataa tatgagataa 1920

tgccgactgt actttttaca gtcggttttc taatgtcact aacctgcccc gttagttgaa 1980

gaaggttttt atattacagc tccagatcca tatccttctt tttctgaacc gacttctcct 2040

ttttcgcttc tttattccaa ttgctttatt gacgttgagc ctcggaaccc ttaacaatcc 2100

caaaacttgt cgaatggtcg gcttaatagc tcacgctatg ccgacattcg tctgcaagtt 2160

tagttaaggg ttcttctcaa cgcacaataa attttctcgg cataaatgcg tggtctaatt 2220

tttattttta ataaccttga tagcaaaaaa tgccattcca atacaaaacc acatacctat 2280

aatcgataac cacataacag tcataaaacc actccttttt aacaaacttt atcacaagaa 2340

atatttaaat tttaaatgcc tttattttga attttaaggg gcattttaaa gatttagggg 2400

taaatcatat agttttatgc ctaaaaacct acagaagctt ttaaaaagca aatatgagcc 2460

aaataaatat attctaattc tacaaacaaa aatttgagca aattcagtgt cgatttttta 2520

agacactgcc cagttacatg caaattaaaa ttttcatgat tttttatagt tcctaacagg 2580

gttaaaattt gtataacgaa agtataatgt ttatataacg ttagtataat aaagcatttt 2640

aacattatac ttttgataat cgtttatcgt cgtcatcaca ataactttta aaatactcgt 2700

gcataattca cgctgacctc ccaataacta catggtgtta tcgggaggtc agctgttagc 2760

acttatattt tgttattgtt cttcctcgat ttcgtctatc attttgtgat taatttctct 2820

tttttcttgt tctgttaagt cataaagttc actagctaaa tactcttttt gtttccaaat 2880

ataaaaaatt tgatagatat attacggttg 2910

SEQUENCE LISTING

<110>  Butamax (TM) Advanced Biofuels LLC

<120>  Method for the Production of Isobutanol

<130>  69.15.104007/01

<140>  EP12173233.3
<141>  2008-04-29

<150>  EP08780577.6
<151>  2008-04-29

<150>  PCT/US2008/061861
<151>  2008-04-29

<150>  60/915,467
<151>  2007-05-02

<160>  267

<170>  PatentIn version 3.4

<210>  1
<211>  1680
<212>  DNA
<213>  Klebsiella pneumoniae

<400>  1
atggacaaac agtatccggt acgccagtgg gcgcacggcg ccgatctcgt cgtcagtcag      60

ctggaagctc agggagtacg ccaggtgttc ggcatccccg gcgccaaaat cgacaaggtc     120

tttgattcac tgctggattc ctccattcgc attattccgg tacgccacga agccaacgcc     180

gcatttatgg ccgccgccgt cggacgcatt accggcaaag cgggcgtggc gctggtcacc     240

tccggtccgg gctgttccaa cctgatcacc ggcatggcca ccgcgaacag cgaaggcgac     300

ccggtggtgg ccctgggcgg cgcggtaaaa cgcgccgata aagcgaagca ggtccaccag     360

agtatggata cggtggcgat gttcagcccg gtcaccaaat acgccatcga ggtgacggcg     420

ccggatgcgc tggcggaagt ggtctccaac gccttccgcg ccgccgagca gggccggccg     480

ggcagcgcgt tcgttagcct gccgcaggat gtggtcgatg gcccggtcag cggcaaagtg     540

ctgccggcca gcggggcccc gcagatgggc gccgcgccgg atgatgccat cgaccaggtg     600

gcgaagctta tcgcccaggc gaagaacccg atcttcctgc tcggcctgat ggccagccag     660

ccggaaaaca gcaaggcgct gcgccgtttg ctggagacca gccatattcc agtcaccagc     720

acctatcagg ccgccggagc ggtgaatcag gataacttct ctcgcttcgc cggccgggtt     780

gggctgttta acaaccaggc cggggaccgt ctgctgcagc tcgccgacct ggtgatctgc     840

atcggctaca gcccggtgga atacgaaccg gcgatgtgga acagcggcaa cgcgacgctg     900

gtgcacatcg acgtgctgcc cgcctatgaa gagcgcaact acaccccgga tgtcgagctg     960

gtgggcgata tcgccggcac tctcaacaag ctggcgcaaa atatcgatca tcggctggtg    1020

ctctccccgc aggcggcgga gatcctccgc gaccgccagc accagcgcga gctgctggac 1080

cgccgcggcg cgcagctcaa ccagtttgcc ctgcatcccc tgcgcatcgt tcgcgccatg 1140

caggatatcg tcaacagcga cgtcacgttg accgtggaca tgggcagctt ccatatctgg 1200

attgcccgct acctgtacac gttccgcgcc cgtcaggtga tgatctccaa cggccagcag 1260

accatgggcg tcgccctgcc ctgggctatc ggcgcctggc tggtcaatcc tgagcgcaaa 1320

gtggtctccg tctccggcga cggcggcttc ctgcagtcga gcatggagct ggagaccgcc 1380

gtccgcctga aagccaacgt gctgcatctt atctgggtcg ataacggcta caacatggtc 1440

gctatccagg aagagaaaaa atatcagcgc ctgtccggcg tcgagtttgg gccgatggat 1500

tttaaagcct atgccgaatc cttcggcgcg aaagggtttg ccgtggaaag cgccgaggcg 1560

ctggagccga ccctgcgcgc ggcgatggac gtcgacggcc cggcggtagt ggccatcccg 1620

gtggattatc gcgataaccc gctgctgatg ggccagctgc atctgagtca gattctgtaa 1680

<210> 2
<211> 559
<212> PRT
<213> Klebsiella pneumoniae

<400> 2

Met Asp Lys Gln Tyr Pro Val Arg Gln Trp Ala His Gly Ala Asp Leu
1               5                   10                  15

Val Val Ser Gln Leu Glu Ala Gln Gly Val Arg Gln Val Phe Gly Ile
            20                  25                  30

Pro Gly Ala Lys Ile Asp Lys Val Phe Asp Ser Leu Leu Asp Ser Ser
        35                  40                  45

Ile Arg Ile Ile Pro Val Arg His Glu Ala Asn Ala Ala Phe Met Ala
    50                  55                  60

Ala Ala Val Gly Arg Ile Thr Gly Lys Ala Gly Val Ala Leu Val Thr
65                  70                  75                  80

Ser Gly Pro Gly Cys Ser Asn Leu Ile Thr Gly Met Ala Thr Ala Asn
                85                  90                  95

Ser Glu Gly Asp Pro Val Val Ala Leu Gly Gly Ala Val Lys Arg Ala
            100                 105                 110

Asp Lys Ala Lys Gln Val His Gln Ser Met Asp Thr Val Ala Met Phe
        115                 120                 125

Ser Pro Val Thr Lys Tyr Ala Ile Glu Val Thr Ala Pro Asp Ala Leu
    130                 135                 140

Ala Glu Val Val Ser Asn Ala Phe Arg Ala Ala Glu Gln Gly Arg Pro
145                 150                 155                 160

Gly Ser Ala Phe Val Ser Leu Pro Gln Asp Val Val Asp Gly Pro Val
                165                 170                 175

Ser Gly Lys Val Leu Pro Ala Ser Gly Ala Pro Gln Met Gly Ala Ala
            180                 185                 190

Pro Asp Asp Ala Ile Asp Gln Val Ala Lys Leu Ile Ala Gln Ala Lys
        195                 200                 205

Asn Pro Ile Phe Leu Leu Gly Leu Met Ala Ser Gln Pro Glu Asn Ser
    210                 215                 220

Lys Ala Leu Arg Arg Leu Leu Glu Thr Ser His Ile Pro Val Thr Ser
225                 230                 235                 240

Thr Tyr Gln Ala Ala Gly Ala Val Asn Gln Asp Asn Phe Ser Arg Phe
                245                 250                 255

Ala Gly Arg Val Gly Leu Phe Asn Asn Gln Ala Gly Asp Arg Leu Leu
            260                 265                 270

Gln Leu Ala Asp Leu Val Ile Cys Ile Gly Tyr Ser Pro Val Glu Tyr
        275                 280                 285

Glu Pro Ala Met Trp Asn Ser Gly Asn Ala Thr Leu Val His Ile Asp
    290                 295                 300

Val Leu Pro Ala Tyr Glu Glu Arg Asn Tyr Thr Pro Asp Val Glu Leu
305                 310                 315                 320

Val Gly Asp Ile Ala Gly Thr Leu Asn Lys Leu Ala Gln Asn Ile Asp
                325                 330                 335

His Arg Leu Val Leu Ser Pro Gln Ala Ala Glu Ile Leu Arg Asp Arg
            340                 345                 350

Gln His Gln Arg Glu Leu Leu Asp Arg Arg Gly Ala Gln Leu Asn Gln
        355                 360                 365

Phe Ala Leu His Pro Leu Arg Ile Val Arg Ala Met Gln Asp Ile Val
    370                 375                 380

Asn Ser Asp Val Thr Leu Thr Val Asp Met Gly Ser Phe His Ile Trp
385                 390                 395                 400

Ile Ala Arg Tyr Leu Tyr Thr Phe Arg Ala Arg Gln Val Met Ile Ser
                405                 410                 415

Asn Gly Gln Gln Thr Met Gly Val Ala Leu Pro Trp Ala Ile Gly Ala
            420                 425                 430

Trp Leu Val Asn Pro Glu Arg Lys Val Val Ser Val Ser Gly Asp Gly
        435                 440                 445

Gly Phe Leu Gln Ser Ser Met Glu Leu Glu Thr Ala Val Arg Leu Lys
    450                 455                 460

Ala Asn Val Leu His Leu Ile Trp Val Asp Asn Gly Tyr Asn Met Val
465                 470                 475                 480

Ala Ile Gln Glu Glu Lys Lys Tyr Gln Arg Leu Ser Gly Val Glu Phe
                485                 490                 495

Gly Pro Met Asp Phe Lys Ala Tyr Ala Glu Ser Phe Gly Ala Lys Gly
            500                 505                 510

Phe Ala Val Glu Ser Ala Glu Ala Leu Glu Pro Thr Leu Arg Ala Ala
        515                 520                 525

Met Asp Val Asp Gly Pro Ala Val Val Ala Ile Pro Val Asp Tyr Arg
    530                 535                 540

Asp Asn Pro Leu Leu Met Gly Gln Leu His Leu Ser Gln Ile Leu
545                 550                 555

<210>  3
<211>  1476
<212>  DNA
<213>  Escherichia coli

<400>  3
atggctaact acttcaatac actgaatctg cgccagcagc tggcacagct gggcaaatgt      60

cgctttatgg gccgcgatga attcgccgat ggcgcgagct accttcaggg taaaaaagta     120

gtcatcgtcg gctgtggcgc acagggtctg aaccagggcc tgaacatgcg tgattctggt     180

ctcgatatct cctacgctct gcgtaaagaa gcgattgccg agaagcgcgc gtcctggcgt     240

aaagcgaccg aaaatggttt taaagtgggt acttacgaag aactgatccc acaggcggat     300

ctggtgatta acctgacgcc ggacaagcag cactctgatg tagtgcgcac cgtacagcca     360

ctgatgaaag acggcgcggc gctgggctac tcgcacggtt tcaacatcgt cgaagtgggc     420

gagcagatcc gtaaagatat caccgtagtg atggttgcgc cgaaatgccc aggcaccgaa     480

gtgcgtgaag agtacaaacg tgggttcggc gtaccgacgc tgattgccgt tcacccggaa     540

aacgatccga aaggcgaagg catggcgatt gccaaagcct gggcggctgc aaccggtggt     600

caccgtgcgg gtgtgctgga atcgtccttc gttgcggaag tgaaatctga cctgatgggc 660

gagcaaacca tcctgtgcgg tatgttgcag gctggctctc tgctgtgctt cgacaagctg 720

gtggaagaag gtaccgatcc agcatacgca gaaaaactga ttcagttcgg ttgggaaacc 780

atcaccgaag cactgaaaca gggcggcatc accctgatga tggaccgtct ctctaacccg 840

gcgaaactgc gtgcttatgc gctttctgaa cagctgaaag agatcatggc acccctgttc 900

cagaaacata tggacgacat catctccggc gaattctctt ccggtatgat ggcggactgg 960

gccaacgatg ataagaaact gctgacctgg cgtgaagaga ccggcaaaac cgcgtttgaa 1020

accgcgccgc agtatgaagg caaaatcggc gagcaggagt acttcgataa aggcgtactg 1080

atgattgcga tggtgaaagc gggcgttgaa ctggcgttcg aaaccatggt cgattccggc 1140

atcattgaag agtctgcata ttatgaatca ctgcacgagc tgccgctgat tgccaacacc 1200

atcgcccgta agcgtctgta cgaaatgaac gtggttatct ctgataccgc tgagtacggt 1260

aactatctgt tctcttacgc ttgtgtgccg ttgctgaaac cgtttatggc agagctgcaa 1320

ccgggcgacc tgggtaaagc tattccggaa ggcgcggtag ataacgggca actgcgtgat 1380

gtgaacgaag cgattcgcag ccatgcgatt gagcaggtag gtaagaaact gcgcggctat 1440

atgacagata tgaaacgtat tgctgttgcg ggttaa 1476

<210> 4
<211> 491
<212> PRT
<213> Escherichia coli

<400> 4

Met Ala Asn Tyr Phe Asn Thr Leu Asn Leu Arg Gln Gln Leu Ala Gln
1               5                   10                  15

Leu Gly Lys Cys Arg Phe Met Gly Arg Asp Glu Phe Ala Asp Gly Ala
            20                  25                  30

Ser Tyr Leu Gln Gly Lys Lys Val Val Ile Val Gly Cys Gly Ala Gln
        35                  40                  45

Gly Leu Asn Gln Gly Leu Asn Met Arg Asp Ser Gly Leu Asp Ile Ser
    50                  55                  60

Tyr Ala Leu Arg Lys Glu Ala Ile Ala Glu Lys Arg Ala Ser Trp Arg
65                  70                  75                  80

Lys Ala Thr Glu Asn Gly Phe Lys Val Gly Thr Tyr Glu Glu Leu Ile
                85                  90                  95

Pro Gln Ala Asp Leu Val Ile Asn Leu Thr Pro Asp Lys Gln His Ser
            100                 105                 110

Asp Val Val Arg Thr Val Gln Pro Leu Met Lys Asp Gly Ala Ala Leu
        115                 120                 125

Gly Tyr Ser His Gly Phe Asn Ile Val Glu Val Gly Glu Gln Ile Arg
    130                 135                 140

Lys Asp Ile Thr Val Val Met Val Ala Pro Lys Cys Pro Gly Thr Glu
145                 150                 155                 160

Val Arg Glu Glu Tyr Lys Arg Gly Phe Gly Val Pro Thr Leu Ile Ala
                165                 170                 175

Val His Pro Glu Asn Asp Pro Lys Gly Glu Gly Met Ala Ile Ala Lys
            180                 185                 190

Ala Trp Ala Ala Ala Thr Gly Gly His Arg Ala Gly Val Leu Glu Ser
        195                 200                 205

Ser Phe Val Ala Glu Val Lys Ser Asp Leu Met Gly Glu Gln Thr Ile
    210                 215                 220

Leu Cys Gly Met Leu Gln Ala Gly Ser Leu Leu Cys Phe Asp Lys Leu
225                 230                 235                 240

Val Glu Glu Gly Thr Asp Pro Ala Tyr Ala Glu Lys Leu Ile Gln Phe
                245                 250                 255

Gly Trp Glu Thr Ile Thr Glu Ala Leu Lys Gln Gly Gly Ile Thr Leu
            260                 265                 270

Met Met Asp Arg Leu Ser Asn Pro Ala Lys Leu Arg Ala Tyr Ala Leu
        275                 280                 285

Ser Glu Gln Leu Lys Glu Ile Met Ala Pro Leu Phe Gln Lys His Met
    290                 295                 300

Asp Asp Ile Ile Ser Gly Glu Phe Ser Ser Gly Met Met Ala Asp Trp
305                 310                 315                 320

Ala Asn Asp Asp Lys Lys Leu Leu Thr Trp Arg Glu Glu Thr Gly Lys
                325                 330                 335

Thr Ala Phe Glu Thr Ala Pro Gln Tyr Glu Gly Lys Ile Gly Glu Gln
            340                 345                 350

Glu Tyr Phe Asp Lys Gly Val Leu Met Ile Ala Met Val Lys Ala Gly
        355                 360                 365

Val Glu Leu Ala Phe Glu Thr Met Val Asp Ser Gly Ile Ile Glu Glu
    370                 375                 380

Ser Ala Tyr Tyr Glu Ser Leu His Glu Leu Pro Leu Ile Ala Asn Thr
385                 390                 395                 400

Ile Ala Arg Lys Arg Leu Tyr Glu Met Asn Val Val Ile Ser Asp Thr
                405                 410                 415

Ala Glu Tyr Gly Asn Tyr Leu Phe Ser Tyr Ala Cys Val Pro Leu Leu
            420                 425                 430

Lys Pro Phe Met Ala Glu Leu Gln Pro Gly Asp Leu Gly Lys Ala Ile
        435                 440                 445

Pro Glu Gly Ala Val Asp Asn Gly Gln Leu Arg Asp Val Asn Glu Ala
    450                 455                 460

Ile Arg Ser His Ala Ile Glu Gln Val Gly Lys Lys Leu Arg Gly Tyr
465                 470                 475                 480

Met Thr Asp Met Lys Arg Ile Ala Val Ala Gly
                485                 490

<210>  5
<211>  1851
<212>  DNA
<213>  Escherichia coli

<400>  5
atgcctaagt accgttccgc caccaccact catggtcgta atatggcggg tgctcgtgcg      60

ctgtggcgcg ccaccggaat gaccgacgcc gatttcggta agccgattat cgcggttgtg     120

aactcgttca cccaatttgt accgggtcac gtccatctgc gcgatctcgg taaactggtc     180

gccgaacaaa ttgaagcggc tggcggcgtt gccaaagagt tcaacaccat tgcggtggat     240

gatgggattg ccatgggcca cggggggatg ctttattcac tgccatctcg cgaactgatc     300

gctgattccg ttgagtatat ggtcaacgcc cactgcgccg acgccatggt ctgcatctct     360

aactgcgaca aaatcacccc ggggatgctg atggcttccc tgcgcctgaa tattccggtg     420

atctttgttt ccggcggccc gatggaggcc gggaaaacca aactttccga tcagatcatc     480

aagctcgatc tggttgatgc gatgatccag ggcgcagacc cgaaagtatc tgactcccag     540

agcgatcagg ttgaacgttc cgcgtgtccg acctgcggtt cctgctccgg gatgtttacc     600

gctaactcaa tgaactgcct gaccgaagcg ctgggcctgt cgcagccggg caacggctcg     660

ctgctggcaa cccacgccga ccgtaagcag ctgttcctta atgctggtaa acgcattgtt     720

gaattgacca aacgttatta cgagcaaaac gacgaaagtg cactgccgcg taatatcgcc     780

agtaaggcgg cgtttgaaaa cgccatgacg ctggatatcg cgatgggtgg atcgactaac 840

accgtacttc acctgctggc ggcggcgcag gaagcggaaa tcgacttcac catgagtgat 900

atcgataagc tttcccgcaa ggttccacag ctgtgtaaag ttgcgccgag cacccagaaa 960

taccatatgg aagatgttca ccgtgctggt ggtgttatcg gtattctcgg cgaactggat 1020

cgcgcggggt tactgaaccg tgatgtgaaa aacgtacttg gcctgacgtt gccgcaaacg 1080

ctggaacaat acgacgttat gctgacccag gatgacgcgg taaaaaatat gttccgcgca 1140

ggtcctgcag gcattcgtac cacacaggca ttctcgcaag attgccgttg ggatacgctg 1200

gacgacgatc gcgccaatgg ctgtatccgc tcgctggaac acgcctacag caaagacggc 1260

ggcctggcgg tgctctacgg taactttgcg gaaaacggct gcatcgtgaa aacggcaggc 1320

gtcgatgaca gcatcctcaa attcaccggc ccggcgaaag tgtacgaaag ccaggacgat 1380

gcggtagaag cgattctcgg cggtaaagtt gtcgccggag atgtggtagt aattcgctat 1440

gaaggcccga aaggcggtcc ggggatgcag gaaatgctct acccaaccag cttcctgaaa 1500

tcaatgggtc tcggcaaagc ctgtgcgctg atcaccgacg gtcgtttctc tggtggcacc 1560

tctggtcttt ccatcggcca cgtctcaccg gaagcggcaa gcggcggcag cattggcctg 1620

attgaagatg gtgacctgat cgctatcgac atcccgaacc gtggcattca gttacaggta 1680

agcgatgccg aactggcggc gcgtcgtgaa gcgcaggacg ctcgaggtga caaagcctgg 1740

acgccgaaaa atcgtgaacg tcaggtctcc tttgccctgc gtgcttatgc cagcctggca 1800

accagcgccg acaaaggcgc ggtgcgcgat aaatcgaaac tggggggtta a 1851

<210> 6
<211> 616
<212> PRT
<213> Escherichia coli

<400> 6

Met Pro Lys Tyr Arg Ser Ala Thr Thr Thr His Gly Arg Asn Met Ala
1               5                   10                  15

Gly Ala Arg Ala Leu Trp Arg Ala Thr Gly Met Thr Asp Ala Asp Phe
            20                  25                  30

Gly Lys Pro Ile Ile Ala Val Val Asn Ser Phe Thr Gln Phe Val Pro
        35                  40                  45

Gly His Val His Leu Arg Asp Leu Gly Lys Leu Val Ala Glu Gln Ile
    50                  55                  60

Glu Ala Ala Gly Gly Val Ala Lys Glu Phe Asn Thr Ile Ala Val Asp
65                  70                  75                  80

Asp Gly Ile Ala Met Gly His Gly Gly Met Leu Tyr Ser Leu Pro Ser

85                  90                  95

Arg Glu Leu Ile Ala Asp Ser Val Glu Tyr Met Val Asn Ala His Cys
            100                 105                 110

Ala Asp Ala Met Val Cys Ile Ser Asn Cys Asp Lys Ile Thr Pro Gly
        115                 120                 125

Met Leu Met Ala Ser Leu Arg Leu Asn Ile Pro Val Ile Phe Val Ser
    130                 135                 140

Gly Gly Pro Met Glu Ala Gly Lys Thr Lys Leu Ser Asp Gln Ile Ile
145                 150                 155                 160

Lys Leu Asp Leu Val Asp Ala Met Ile Gln Gly Ala Asp Pro Lys Val
                165                 170                 175

Ser Asp Ser Gln Ser Asp Gln Val Glu Arg Ser Ala Cys Pro Thr Cys
            180                 185                 190

Gly Ser Cys Ser Gly Met Phe Thr Ala Asn Ser Met Asn Cys Leu Thr
        195                 200                 205

Glu Ala Leu Gly Leu Ser Gln Pro Gly Asn Gly Ser Leu Leu Ala Thr
    210                 215                 220

His Ala Asp Arg Lys Gln Leu Phe Leu Asn Ala Gly Lys Arg Ile Val
225                 230                 235                 240

Glu Leu Thr Lys Arg Tyr Tyr Glu Gln Asn Asp Glu Ser Ala Leu Pro
                245                 250                 255

Arg Asn Ile Ala Ser Lys Ala Ala Phe Glu Asn Ala Met Thr Leu Asp
            260                 265                 270

Ile Ala Met Gly Gly Ser Thr Asn Thr Val Leu His Leu Leu Ala Ala
        275                 280                 285

Ala Gln Glu Ala Glu Ile Asp Phe Thr Met Ser Asp Ile Asp Lys Leu
    290                 295                 300

Ser Arg Lys Val Pro Gln Leu Cys Lys Val Ala Pro Ser Thr Gln Lys
305                 310                 315                 320

Tyr His Met Glu Asp Val His Arg Ala Gly Gly Val Ile Gly Ile Leu
                325                 330                 335

Gly Glu Leu Asp Arg Ala Gly Leu Leu Asn Arg Asp Val Lys Asn Val
            340                 345                 350

Leu Gly Leu Thr Leu Pro Gln Thr Leu Glu Gln Tyr Asp Val Met Leu
        355                 360                 365

Thr Gln Asp Asp Ala Val Lys Asn Met Phe Arg Ala Gly Pro Ala Gly
    370                 375                 380

Ile Arg Thr Thr Gln Ala Phe Ser Gln Asp Cys Arg Trp Asp Thr Leu
385                 390                 395                 400

Asp Asp Asp Arg Ala Asn Gly Cys Ile Arg Ser Leu Glu His Ala Tyr
                405                 410                 415

Ser Lys Asp Gly Gly Leu Ala Val Leu Tyr Gly Asn Phe Ala Glu Asn
            420                 425                 430

Gly Cys Ile Val Lys Thr Ala Gly Val Asp Asp Ser Ile Leu Lys Phe
        435                 440                 445

Thr Gly Pro Ala Lys Val Tyr Glu Ser Gln Asp Asp Ala Val Glu Ala
    450                 455                 460

Ile Leu Gly Gly Lys Val Val Ala Gly Asp Val Val Val Ile Arg Tyr
465                 470                 475                 480

Glu Gly Pro Lys Gly Gly Pro Gly Met Gln Glu Met Leu Tyr Pro Thr
                485                 490                 495

Ser Phe Leu Lys Ser Met Gly Leu Gly Lys Ala Cys Ala Leu Ile Thr
            500                 505                 510

Asp Gly Arg Phe Ser Gly Gly Thr Ser Gly Leu Ser Ile Gly His Val
        515                 520                 525

Ser Pro Glu Ala Ala Ser Gly Gly Ser Ile Gly Leu Ile Glu Asp Gly
    530                 535                 540

Asp Leu Ile Ala Ile Asp Ile Pro Asn Arg Gly Ile Gln Leu Gln Val
545                 550                 555                 560

Ser Asp Ala Glu Leu Ala Ala Arg Arg Glu Ala Gln Asp Ala Arg Gly
                565                 570                 575

Asp Lys Ala Trp Thr Pro Lys Asn Arg Glu Arg Gln Val Ser Phe Ala
            580                 585                 590

Leu Arg Ala Tyr Ala Ser Leu Ala Thr Ser Ala Asp Lys Gly Ala Val
        595                 600                 605

Arg Asp Lys Ser Lys Leu Gly Gly
    610                 615

<210>  7
<211>  1662
<212>  DNA
<213>  Lactococcus lactis

<400>  7
tctagacata tgtatactgt gggggattac ctgctggatc gcctgcacga actggggatt      60

gaagaaattt tcggtgtgcc aggcgattat aacctgcagt tcctggacca gattatctcg     120

cacaaagata tgaagtgggt cggtaacgcc aacgaactga acgcgagcta tatggcagat     180

ggttatgccc gtaccaaaaa agctgctgcg tttctgacga cctttggcgt tggcgaactg     240

agcgccgtca acggactggc aggaagctac gccgagaacc tgccagttgt cgaaattgtt     300

gggtcgccta cttctaaggt tcagaatgaa ggcaaatttg tgcaccatac tctggctgat     360

ggggatttta aacattttat gaaaatgcat gaaccggtta ctgcggcccg cacgctgctg     420

acagcagaga atgctacggt tgagatcgac cgcgtcctgt ctgcgctgct gaaagagcgc     480

aagccggtat atatcaatct gcctgtcgat gttgccgcag cgaaagccga aaagccgtcg     540

ctgccactga aaaaagaaaa cagcacctcc aatacatcgg accaggaaat tctgaataaa     600

atccaggaat cactgaagaa tgcgaagaaa ccgatcgtca tcaccggaca tgagatcatc     660

tcttttggcc tggaaaaaac ggtcacgcag ttcatttcta agaccaaact gcctatcacc     720

accctgaact tcggcaaatc tagcgtcgat gaagcgctgc cgagttttct gggtatctat     780

aatggtaccc tgtccgaacc gaacctgaaa gaattcgtcg aaagcgcgga ctttatcctg     840

atgctgggcg tgaaactgac ggatagctcc acaggcgcat ttacccacca tctgaacgag     900

aataaaatga tttccctgaa tatcgacgaa ggcaaaatct ttaacgagcg catccagaac     960

ttcgattttg aatctctgat tagttcgctg ctggatctgt ccgaaattga gtataaaggt    1020

aaatatattg ataaaaaaca ggaggatttt gtgccgtcta atgcgctgct gagtcaggat    1080

cgtctgtggc aagccgtaga aaacctgaca cagtctaatg aaacgattgt tgcggaacag    1140

ggaacttcat ttttcggcgc ctcatccatt tttctgaaat ccaaaagcca tttcattggc    1200

caaccgctgt gggggagtat tggttatacc tttccggcgg cgctgggttc acagattgca    1260

gataaggaat cacgccatct gctgtttatt ggtgacggca gcctgcagct gactgtccag    1320

gaactggggc tggcgatccg tgaaaaaatc aatccgattt gctttatcat caataacgac    1380

ggctacaccg tcgaacgcga aattcatgga ccgaatcaaa gttacaatga catcccgatg    1440

tggaactata gcaaactgcc ggaatccttt ggcgcgacag aggatcgcgt ggtgagtaaa    1500

attgtgcgta cggaaaacga atttgtgtcg gttatgaaag aagcgcaggc tgacccgaat    1560

cgcatgtatt ggattgaact gatcctggca aaagaaggcg caccgaaagt tctgaaaaag    1620

atggggaaac tgtttgcgga gcaaaataaa agctaaggat cc                    1662

<210>  8
<211>  548
<212>  PRT
<213>  Lactococcus lactis

<400>  8

Met Tyr Thr Val Gly Asp Tyr Leu Leu Asp Arg Leu His Glu Leu Gly
1               5                   10                  15

Ile Glu Glu Ile Phe Gly Val Pro Gly Asp Tyr Asn Leu Gln Phe Leu
            20                  25                  30

Asp Gln Ile Ile Ser His Lys Asp Met Lys Trp Val Gly Asn Ala Asn
        35                  40                  45

Glu Leu Asn Ala Ser Tyr Met Ala Asp Gly Tyr Ala Arg Thr Lys Lys
    50                  55                  60

Ala Ala Ala Phe Leu Thr Thr Phe Gly Val Gly Glu Leu Ser Ala Val
65                  70                  75                  80

Asn Gly Leu Ala Gly Ser Tyr Ala Glu Asn Leu Pro Val Val Glu Ile
                85                  90                  95

Val Gly Ser Pro Thr Ser Lys Val Gln Asn Glu Gly Lys Phe Val His
            100                 105                 110

His Thr Leu Ala Asp Gly Asp Phe Lys His Phe Met Lys Met His Glu
        115                 120                 125

Pro Val Thr Ala Ala Arg Thr Leu Leu Thr Ala Glu Asn Ala Thr Val
    130                 135                 140

Glu Ile Asp Arg Val Leu Ser Ala Leu Leu Lys Glu Arg Lys Pro Val
145                 150                 155                 160

Tyr Ile Asn Leu Pro Val Asp Val Ala Ala Ala Lys Ala Glu Lys Pro
                165                 170                 175

Ser Leu Pro Leu Lys Lys Glu Asn Ser Thr Ser Asn Thr Ser Asp Gln
            180                 185                 190

Glu Ile Leu Asn Lys Ile Gln Glu Ser Leu Lys Asn Ala Lys Lys Pro
        195                 200                 205

Ile Val Ile Thr Gly His Glu Ile Ile Ser Phe Gly Leu Glu Lys Thr
    210                 215                 220

Val Thr Gln Phe Ile Ser Lys Thr Lys Leu Pro Ile Thr Thr Leu Asn
225                 230                 235                 240

Phe Gly Lys Ser Ser Val Asp Glu Ala Leu Pro Ser Phe Leu Gly Ile
                245                 250                 255

Tyr Asn Gly Thr Leu Ser Glu Pro Asn Leu Lys Glu Phe Val Glu Ser
            260                 265                 270

Ala Asp Phe Ile Leu Met Leu Gly Val Lys Leu Thr Asp Ser Ser Thr
        275                 280                 285

Gly Ala Phe Thr His His Leu Asn Glu Asn Lys Met Ile Ser Leu Asn
    290                 295                 300

Ile Asp Glu Gly Lys Ile Phe Asn Glu Arg Ile Gln Asn Phe Asp Phe
305                 310                 315                 320

Glu Ser Leu Ile Ser Ser Leu Leu Asp Leu Ser Glu Ile Glu Tyr Lys
                325                 330                 335

Gly Lys Tyr Ile Asp Lys Lys Gln Glu Asp Phe Val Pro Ser Asn Ala
            340                 345                 350

Leu Leu Ser Gln Asp Arg Leu Trp Gln Ala Val Glu Asn Leu Thr Gln
        355                 360                 365

Ser Asn Glu Thr Ile Val Ala Glu Gln Gly Thr Ser Phe Phe Gly Ala
    370                 375                 380

Ser Ser Ile Phe Leu Lys Ser Lys Ser His Phe Ile Gly Gln Pro Leu
385                 390                 395                 400

Trp Gly Ser Ile Gly Tyr Thr Phe Pro Ala Ala Leu Gly Ser Gln Ile
                405                 410                 415

Ala Asp Lys Glu Ser Arg His Leu Leu Phe Ile Gly Asp Gly Ser Leu
            420                 425                 430

Gln Leu Thr Val Gln Glu Leu Gly Leu Ala Ile Arg Glu Lys Ile Asn
        435                 440                 445

Pro Ile Cys Phe Ile Ile Asn Asn Asp Gly Tyr Thr Val Glu Arg Glu
    450                 455                 460

Ile His Gly Pro Asn Gln Ser Tyr Asn Asp Ile Pro Met Trp Asn Tyr
465                 470                 475                 480

Ser Lys Leu Pro Glu Ser Phe Gly Ala Thr Glu Asp Arg Val Val Ser
                485                 490                 495

Lys Ile Val Arg Thr Glu Asn Glu Phe Val Ser Val Met Lys Glu Ala
            500                 505                 510

Gln Ala Asp Pro Asn Arg Met Tyr Trp Ile Glu Leu Ile Leu Ala Lys
        515                 520                 525

Glu Gly Ala Pro Lys Val Leu Lys Lys Met Gly Lys Leu Phe Ala Glu
    530                 535                 540

Gln Asn Lys Ser
545

<210>  9
<211>  1164
<212>  DNA
<213>  Escherichia coli

<400>  9
atgaacaact ttaatctgca cacccaaacc cgcattctgt ttggtaaagg cgcaatcgct      60

ggtttacgcg aacaaattcc tcacgatgct cgcgtattga ttacctacgg cggcggcagc     120

gtgaaaaaaa ccggcgttct cgatcaagtt ctggatgccc tgaaaggcat ggacgtgctg     180

gaatttggcg gtattgagcc aaacccggct tatgaaacgc tgatgaacgc cgtgaaactg     240

gttcgcgaac agaaagtgac tttcctgctg gcggttggcg gcggttctgt actggacggc     300

accaaattta tcgccgcagc ggctaactat ccggaaaata tcgatccgtg gcacattctg     360

caaacgggcg gtaaagagat taaaagcgcc atcccgatgg gctgtgtgct gacgctgcca     420

gcaaccggtt cagaatccaa cgcaggcgcg gtgatctccc gtaaaaccac aggcgacaag     480

caggcgttcc attctgccca tgttcagccg gtatttgccg tgctcgatcc ggtttatacc     540

tacaccctgc cgccgcgtca ggtggctaac ggcgtagtgg acgcctttgt acacaccgtg     600

gaacagtatg ttaccaaacc ggttgatgcc aaaattcagg accgtttcgc agaaggcatt     660

ttgctgacgc taatcgaaga tggtccgaaa gccctgaaag agccagaaaa ctacgatgtg     720

cgcgccaacg tcatgtgggc ggcgactcag gcgctgaacg gtttgattgg cgctggcgta     780

ccgcaggact gggcaacgca tatgctgggc cacgaactga ctgcgatgca cggtctggat     840

cacgcgcaaa cactggctat cgtcctgcct gcactgtgga atgaaaaacg cgataccaag     900

cgcgctaagc tgctgcaata tgctgaacgc gtctggaaca tcactgaagg ttccgatgat     960

gagcgtattg acgccgcgat tgccgcaacc cgcaatttct ttgagcaatt aggcgtgccg    1020

acccacctct ccgactacgg tctggacggc agctccatcc cggctttgct gaaaaaactg    1080

gaagagcacg gcatgaccca actgggcgaa aatcatgaca ttacgttgga tgtcagccgc    1140

cgtatatacg aagccgcccg ctaa                                           1164

<210>  10
<211>  387
<212>  PRT
<213>  Escherichia coli

<400>  10

Met Asn Asn Phe Asn Leu His Thr Pro Thr Arg Ile Leu Phe Gly Lys
1               5                   10                  15

Gly Ala Ile Ala Gly Leu Arg Glu Gln Ile Pro His Asp Ala Arg Val
            20                  25                  30

Leu Ile Thr Tyr Gly Gly Gly Ser Val Lys Lys Thr Gly Val Leu Asp
        35                  40                  45

Gln Val Leu Asp Ala Leu Lys Gly Met Asp Val Leu Glu Phe Gly Gly
    50                  55                  60

Ile Glu Pro Asn Pro Ala Tyr Glu Thr Leu Met Asn Ala Val Lys Leu
65                  70                  75                  80

Val Arg Glu Gln Lys Val Thr Phe Leu Leu Ala Val Gly Gly Gly Ser
                85                  90                  95

Val Leu Asp Gly Thr Lys Phe Ile Ala Ala Ala Ala Asn Tyr Pro Glu
            100                 105                 110

Asn Ile Asp Pro Trp His Ile Leu Gln Thr Gly Gly Lys Glu Ile Lys
        115                 120                 125

Ser Ala Ile Pro Met Gly Cys Val Leu Thr Leu Pro Ala Thr Gly Ser
    130                 135                 140

Glu Ser Asn Ala Gly Ala Val Ile Ser Arg Lys Thr Thr Gly Asp Lys
145                 150                 155                 160

Gln Ala Phe His Ser Ala His Val Gln Pro Val Phe Ala Val Leu Asp
                165                 170                 175

Pro Val Tyr Thr Tyr Thr Leu Pro Pro Arg Gln Val Ala Asn Gly Val
            180                 185                 190

Val Asp Ala Phe Val His Thr Val Glu Gln Tyr Val Thr Lys Pro Val
        195                 200                 205

Asp Ala Lys Ile Gln Asp Arg Phe Ala Glu Gly Ile Leu Leu Thr Leu
    210                 215                 220

Ile Glu Asp Gly Pro Lys Ala Leu Lys Glu Pro Glu Asn Tyr Asp Val
225                 230                 235                 240

Arg Ala Asn Val Met Trp Ala Ala Thr Gln Ala Leu Asn Gly Leu Ile
                245                 250                 255

Gly Ala Gly Val Pro Gln Asp Trp Ala Thr His Met Leu Gly His Glu
            260                 265                 270

Leu Thr Ala Met His Gly Leu Asp His Ala Gln Thr Leu Ala Ile Val
        275                 280                 285

Leu Pro Ala Leu Trp Asn Glu Lys Arg Asp Thr Lys Arg Ala Lys Leu
    290                 295                 300

Leu Gln Tyr Ala Glu Arg Val Trp Asn Ile Thr Glu Gly Ser Asp Asp
305                 310                 315                 320

Glu Arg Ile Asp Ala Ala Ile Ala Ala Thr Arg Asn Phe Phe Glu Gln
                325                 330                 335

Leu Gly Val Pro Thr His Leu Ser Asp Tyr Gly Leu Asp Gly Ser Ser
            340                 345                 350

Ile Pro Ala Leu Leu Lys Lys Leu Glu Glu His Gly Met Thr Gln Leu
        355                 360                 365

Gly Glu Asn His Asp Ile Thr Leu Asp Val Ser Arg Arg Ile Tyr Glu
    370                 375                 380

Ala Ala Arg
385

<210>  11
<211>  29
<212>  DNA
<213>  Artificial Sequence

<220>
<223>  Primer

<400>  11
caccatggac aaacagtatc cggtacgcc                                    29

<210>  12
<211>  25
<212>  DNA
<213>  Artificial Sequence

<220>
<223>  Primer

<400>  12

cgaagggcga tagctttacc aatcc                                              25

<210>  13
<211>  29
<212>  DNA
<213>  Artificial Sequence

<220>
<223>  Primer

<400>  13
caccatggct aactacttca atacactga                                          29

<210>  14
<211>  28
<212>  DNA
<213>  Artificial Sequence

<220>
<223>  Primer

<400>  14
ccaggagaag gccttgagtg ttttctcc                                           28

<210>  15
<211>  29
<212>  DNA
<213>  Artificial Sequence

<220>
<223>  Primer

<400>  15
caccatgcct aagtaccgtt ccgccacca                                          29

<210>  16
<211>  26
<212>  DNA
<213>  Artificial Sequence

<220>
<223>  Primer

<400>  16
cgcagcactg ctcttaaata ttcggc                                             26

<210>  17
<211>  29
<212>  DNA
<213>  Artificial Sequence

<220>
<223>  Primer

<400>  17
caccatgaac aactttaatc tgcacaccc                                          29

<210>  18
<211>  29

<212>  DNA
<213>  Artificial Sequence

<220>
<223>  Primer

<400>  18
caccatgaac aactttaatc tgcacaccc                                          29


<210>  19
<211>  45
<212>  DNA
<213>  Artificial Sequence

<220>
<223>  Primer

<400>  19
gcatgcctta agaaaggagg ggggtcacat ggacaaacag tatcc                        45


<210>  20
<211>  39
<212>  DNA
<213>  Artificial Sequence

<220>
<223>  Primer

<400>  20
atgcatttaa ttaattacag aatctgactc agatgcagc                               39


<210>  21
<211>  45
<212>  DNA
<213>  Artificial Sequence

<220>
<223>  Primer

<400>  21
gtcgacgcta gcaaaggagg gaatcaccat ggctaactac ttcaa                        45


<210>  22
<211>  31
<212>  DNA
<213>  Artificial Sequence

<220>
<223>  Primer

<400>  22
tctagattaa cccgcaacag caatacgttt c                                       31


<210>  23
<211>  39
<212>  DNA
<213>  Artificial Sequence

<220>
<223>  Primer

<400>  23
tctagaaaag gaggaataaa gtatgcctaa gtaccgttc                              39


<210>  24
<211>  31
<212>  DNA
<213>  Artificial Sequence

<220>
<223>  Primer

<400>  24
ggatccttat taacccccca gtttcgattt a                                       31


<210>  25
<211>  39
<212>  DNA
<213>  Artificial Sequence

<220>
<223>  Primer

<400>  25
ggatccaaag gaggctagac atatgtatac tgtgggga                               39


<210>  26
<211>  31
<212>  DNA
<213>  Artificial Sequence

<220>
<223>  Primer

<400>  26
gagctcttag cttttatttt gctccgcaaa c                                      31


<210>  27
<211>  39
<212>  DNA
<213>  Artificial Sequence

<220>
<223>  Primer

<400>  27
gagctcaaag gaggagcaag taatgaacaa ctttaatct                              39


<210>  28
<211>  43
<212>  DNA
<213>  Artificial Sequence

<220>
<223>  Primer

<400>  28
gaattcacta gtcctaggtt agcgggcggc ttcgtatata cgg                         43

<210>  29
<211>  25
<212>  DNA
<213>  Artificial Sequence

<220>
<223>  Primer

<400>  29
caacattagc gattttcttt tctct                                             25


<210>  30
<211>  45
<212>  DNA
<213>  Artificial Sequence

<220>
<223>  Primer

<400>  30
catgaagctt actagtgggc ttaagttttg aaaataatga aaact                       45


<210>  31
<211>  61
<212>  DNA
<213>  Artificial Sequence

<220>
<223>  Primer N110.2

<400>  31
gagctcacta gtcaattgta agtaagtaaa aggaggtggg tcacatggac aaacagtatc      60

c                                                                       61


<210>  32
<211>  50
<212>  DNA
<213>  Artificial Sequence

<220>
<223>  Primer N111.2

<400>  32
ggatccgatc gacttaagcc tcagcttaca gaatctgact cagatgcagc                 50


<210>  33
<211>  44
<212>  DNA
<213>  Artificial Sequence

<220>
<223>  Primer N112.2

<400>  33
gagctcctta agaaggaggt aatcaccatg gctaactact tcaa                        44


<210>  34
<211>  51
<212>  DNA

<213>  Artificial Sequence

<220>
<223>  Primer N113.2

<400>  34
ggatccgatc gagctagcgc ggccgcttaa cccgcaacag caatacgttt c                51


<210>  35
<211>  44
<212>  DNA
<213>  Artificial Sequence

<220>
<223>  Primer N114.2

<400>  35
gagctcgcta gcaaggaggt ataaagtatg cctaagtacc gttc                        44


<210>  36
<211>  52
<212>  DNA
<213>  Artificial Sequence

<220>
<223>  Primer N115.2

<400>  36
ggatccgatc gattaattaa cctaaggtta ttaacccccc agtttcgatt ta               52


<210>  37
<211>  46
<212>  DNA
<213>  Artificial Sequence

<220>
<223>  Primer N116.2

<400>  37
gagctcttaa ttaaaaggag gttagacata tgtatactgt gggggga                     46


<210>  38
<211>  49
<212>  DNA
<213>  Artificial Sequence

<220>
<223>  Primer 117.2

<400>  38
ggatccagat ctcctaggac atgtttagct tttattttgc tccgcaaac                   49


<210>  39
<211>  3883
<212>  DNA
<213>  Escherichia coli

<400>  39
ctatattgct gaaggtacag gcgtttccat aactatttgc tcgcgttttt tactcaagaa      60

gaaaatgcca aatagcaaca tcaggcagac aatacccgaa attgcgaaga aaactgtctg 120

gtagcctgcg tggtcaaaga gtatcccagt cggcgttgaa agcagcacaa tcccaagcga 180

actggcaatt tgaaaaccaa tcagaaagat cgtcgacgac aggcgcttat caaagtttgc 240

cacgctgtat ttgaagacgg atatgacaca aagtggaacc tcaatggcat gtaacaactt 300

cactaatgaa ataatccagg ggttaacgaa cagcgcgcag gaaaggatac gcaacgccat 360

aatcacaact ccgataagta atgcattttt tggccctacc cgattcacaa agaaaggaat 420

aatcgccatg cacagcgctt cgagtaccac ctggaatgag ttgagataac catacaggcg 480

cgttcctaca tcgtgtgatt cgaataaacc tgaataaaag acaggaaaaa gttgttgatc 540

aaaaatgtta tagaaagacc acgtccccac aataaatatg acgaaaaccc agaagtttcg 600

atccttgaaa actgcgataa aatcctcttt ttttacccct cccgcatctg ccgctacgca 660

ctggtgatcc ttatctttaa aacgcatgtt gatcatcata aatacagcgc caaatagcga 720

gaccaaccag aagttgatat ggggactgat actaaaaaat atgccggcaa agaacgcgcc 780

aatagcatag ccaaaagatc cccaggcgcg cgctgttcca tattcgaaat gaaaatttcg 840

cgccattttt tcggtgaagc tatcaagcaa accgcatccc gccagatacc ccaagccaaa 900

aaatagcgcc cccagaatta gacctacaga aaaattgctt tgcagtaacg gttcataaac 960

gtaaatcata aacggtccgg tcaagaccag gatgaaactc atacaccaga tgagcggttt 1020

cttcagaccg agtttatcct gaacgatgcc gtagaacatc ataaatagaa tgctggtaaa 1080

ctggttgacc gaataaagtg tacctaattc cgtccctgtc aaccctagat gtcctttcag 1140

ccaaatagcg tataacgacc accacagcga ccaggaaata aaaaagagaa atgagtaact 1200

ggatgcaaaa cgatagtacg catttctgaa tggaatattc agtgccataa ttacctgcct 1260

gtcgttaaaa aattcacgtc ctatttagag ataagagcga cttcgccgtt tacttctcac 1320

tattccagtt cttgtcgaca tggcagcgct gtcattgccc ctttcgccgt tactgcaagc 1380

gctccgcaac gttgagcgag atcgataatt cgtcgcattt ctctctcatc tgtagataat 1440

cccgtagagg acagacctgt gagtaacccg gcaacgaacg catctcccgc ccccgtgcta 1500

tcgacacaat tcacagacat tccagcaaaa tggtgaactt gtcctcgata acagaccacc 1560

accccttctg cacctttagt caccaacagc atggcgatct catactcttt tgccagggcg 1620

catatatcct gatcgttctg tgtttttcca ctgataagtc gccattcttc ttccgagagc 1680

ttgacgacat ccgccagttg tagcgcctgc cgcaaacaca agcggagcaa atgctcgtct 1740

tgccatagat cttcacgaat attaggatcg aagctgacaa aacctccggc atgccggatc 1800

gccgtcatcg cagtaaatgc gctggtacgc gaaggctcgg cagacaacgc aattgaacag 1860

agatgtaacc attcgccatg tcgccagcag ggcaagtctg tcgtctctaa aaaaagatcg 1920

gcactggggc ggaccataaa cgtaaatgaa cgttcccctt gatcgttcag atcgacaagc 1980

accgtggatg tccggtgcca ttcatcttgc ttcagatacg tgatatcgac tccctcagtt 2040

agcagcgttc tttgcattaa cgcaccaaaa ggatcatccc ccacccgacc tataaaccca 2100

cttgttccgc ctaatctggc gattcccacc gcaacgttag ctggcgcgcc gccaggacaa 2160

ggcagtaggc gcccgtctga ttctggcaag agatctacga ccgcatcccc taaaacccat 2220

actttggctg acattttttt cccttaaatt catctgagtt acgcatagtg ataaacctct 2280

ttttcgcaaa atcgtcatgg atttactaaa acatgcatat tcgatcacaa aacgtcatag 2340

ttaacgttaa catttgtgat attcatcgca tttatgaaag taaggacttt tatttttata 2400

aaagttaacg ttaacaattc accaaatttg cttaaccagg atgattaaaa tgacgcaatc 2460

tcgattgcat gcggcgcaaa acgccctagc aaaacttcat gagcaccggg gtaacacttt 2520

ctatccccat tttcacctcg cgcctcctgc cgggtggatg aacgatccaa acggcctgat 2580

ctggtttaac gatcgttatc acgcgtttta tcaacatcat ccgatgagcg aacactgggg 2640

gccaatgcac tggggacatg ccaccagcga cgatatgatc cactggcagc atgagcctat 2700

tgcgctagcg ccaggagacg ataatgacaa agacgggtgt ttttcaggta gtgctgtcga 2760

tgacaatggt gtcctctcac ttatctacac cggacacgtc tggctcgatg gtgcaggtaa 2820

tgacgatgca attcgcgaag tacaatgtct ggctaccagt cgggatggta ttcatttcga 2880

gaaacagggt gtgatcctca ctccaccaga aggaatcatg cacttccgcg atcctaaagt 2940

gtggcgtgaa gccgacacat ggtggatggt agtcggggcg aaagatccag gcaacacggg 3000

gcagatcctg ctttatcgcg gcagttcgtt gcgtgaatgg accttcgatc gcgtactggc 3060

ccacgctgat gcgggtgaaa gctatatgtg ggaatgtccg gactttttca gccttggcga 3120

tcagcattat ctgatgtttt ccccgcaggg aatgaatgcc gagggataca gttaccgaaa 3180

tcgctttcaa agtggcgtaa tacccggaat gtggtcgcca ggacgacttt ttgcacaatc 3240

cgggcatttt actgaacttg ataacggcca tgacttttat gcaccacaaa gctttttagc 3300

gaaggatggt cggcgtattg ttatcggctg gatggatatg tgggaatcgc caatgccctc 3360

aaaacgtgaa ggatgggcag gctgcatgac gctggcgcgc gagctatcag agagcaatgg 3420

caaacttcta caacgcccgg tacacgaagc tgagtcgtta cgccagcagc atcaatctgt 3480

ctctccccgc acaatcagca ataaatatgt tttgcaggaa aacgcgcaag cagttgagat 3540

tcagttgcag tgggcgctga agaacagtga tgccgaacat tacggattac agctcggcac 3600

tggaatgcgg ctgtatattg ataaccaatc tgagcgactt gttttgtggc ggtattaccc 3660

acacgagaat ttagacggct accgtagtat tcccctcccg cagcgtgaca cgctcgccct 3720

aaggatattt atcgatacat catccgtgga agtatttatt aacgacgggg aagcggtgat 3780

gagtagtcga atctatccgc agccagaaga acgggaactg tcgctttatg cctcccacgg 3840

agtggctgtg ctgcaacatg gagcactctg gctactgggt taa 3883

<210> 40

<211>  19
<212>  DNA
<213>  Artificial Sequence

<220>
<223>  Primer N130SeqF1

<400>  40
tgttccaacc tgatcaccg                                                    19


<210>  41
<211>  18
<212>  DNA
<213>  Artificial Sequence

<220>
<223>  Primer N130SeqF2

<400>  41
ggaaaacagc aaggcgct                                                     18


<210>  42
<211>  18
<212>  DNA
<213>  Artificial Sequence

<220>
<223>  Primer N130SeqF3

<400>  42
cagctgaacc agtttgcc                                                     18


<210>  43
<211>  19
<212>  DNA
<213>  Artificial Sequence

<220>
<223>  Primer N130SeqF4

<400>  43
aaaataccag cgcctgtcc                                                    19


<210>  44
<211>  18
<212>  DNA
<213>  Artificial Sequence

<220>
<223>  Primer N130SeqR1

<400>  44
tgaatggcca ccatgttg                                                     18


<210>  45
<211>  18
<212>  DNA
<213>  Artificial Sequence

<220>

<223>  Primer N130SeqR2

<400>  45
gaggatctcc gccgcctg                                                    18


<210>  46
<211>  18
<212>  DNA
<213>  Artificial Sequence

<220>
<223>  Primer N130SeqR3

<400>  46
aggccgagca ggaagatc                                                    18


<210>  47
<211>  19
<212>  DNA
<213>  Artificial Sequence

<220>
<223>  Primer N130SeqR4

<400>  47
tgatcaggtt ggaacagcc                                                   19


<210>  48
<211>  19
<212>  DNA
<213>  Artificial Sequence

<220>
<223>  Primer N131SeqF1

<400>  48
aagaactgat cccacaggc                                                   19


<210>  49
<211>  19
<212>  DNA
<213>  Artificial Sequence

<220>
<223>  Primer N131SeqF2

<400>  49
atcctgtgcg gtatgttgc                                                   19


<210>  50
<211>  18
<212>  DNA
<213>  Artificial Sequence

<220>
<223>  Primer N131Seqf3

<400>  50
attgcgatgg tgaaagcg                                                    18

<210>  51
<211>  19
<212>  DNA
<213>  Artificial Sequence

<220>
<223>  Primer N131SeqR1

<400>  51
atggtgttgg caatcagcg                                                    19


<210>  52
<211>  18
<212>  DNA
<213>  Artificial Sequence

<220>
<223>  Primer N131SeqR2

<400>  52
gtgcttcggt gatggttt                                                     18


<210>  53
<211>  19
<212>  DNA
<213>  Artificial Sequence

<220>
<223>  Primer N131SeqR3

<400>  53
ttgaaaccgt gcgagtagc                                                    19


<210>  54
<211>  19
<212>  DNA
<213>  Artificial Sequence

<220>
<223>  Primer N132SeqF1

<400>  54
tattcactgc catctcgcg                                                    19


<210>  55
<211>  18
<212>  DNA
<213>  Artificial Sequence

<220>
<223>  Primer N132SeqF2

<400>  55
ccgtaagcag ctgttcct                                                     18


<210>  56
<211>  20
<212>  DNA
<213>  Artificial Sequence

<220>
<223>  Primer N132SeqF3

<400>  56
gctggaacaa tacgacgtta                                                    20


<210>  57
<211>  20
<212>  DNA
<213>  Artificial Sequence

<220>
<223>  Primer N132SeqF4

<400>  57
tgctctaccc aaccagcttc                                                    20


<210>  58
<211>  20
<212>  DNA
<213>  Artificial Sequence

<220>
<223>  Primer N132SeqR1

<400>  58
atggaaagac cagaggtgcc                                                    20


<210>  59
<211>  18
<212>  DNA
<213>  Artificial Sequence

<220>
<223>  Primer N132SeqR2

<400>  59
tgcctgtgtg gtacgaat                                                      18


<210>  60
<211>  19
<212>  DNA
<213>  Artificial Sequence

<220>
<223>  Primer N132SeqR3

<400>  60
tattacgcgg cagtgcact                                                     19


<210>  61
<211>  22
<212>  DNA
<213>  Artificial Sequence

<220>
<223>  Primer N132SeqR4

<400>  61

ggtgattttg tcgcagttag ag                                                22

<210>  62
<211>  18
<212>  DNA
<213>  Artificial Sequence

<220>
<223>  Primer N133SeqF1

<400>  62
tcgaaattgt tgggtcgc                                                    18

<210>  63
<211>  22
<212>  DNA
<213>  Artificial Sequence

<220>
<223>  Primer N133SeqF2

<400>  63
ggtcacgcag ttcatttcta ag                                                22

<210>  64
<211>  18
<212>  DNA
<213>  Artificial Sequence

<220>
<223>  Primer N133SeqF3

<400>  64
tgtggcaagc cgtagaaa                                                    18

<210>  65
<211>  19
<212>  DNA
<213>  Artificial Sequence

<220>
<223>  Primer N133SeqF4

<400>  65
aggatcgcgt ggtgagtaa                                                   19

<210>  66
<211>  20
<212>  DNA
<213>  Artificial Sequence

<220>
<223>  Primer N133SeqR1

<400>  66
gtagccgtcg ttattgatga                                                  20

<210>  67
<211>  22

<212>  DNA
<213>  Artificial Sequence

<220>
<223>  Primer N133SeqR2

<400>  67
gcagcgaact aatcagagat tc                                                22


<210>  68
<211>  19
<212>  DNA
<213>  Artificial Sequence

<220>
<223>  Primer N133SeqR3

<400>  68
tggtccgatg tattggagg                                                    19


<210>  69
<211>  19
<212>  DNA
<213>  Artificial Sequence

<220>
<223>  Primer N133SeqR4

<400>  69
tctgccatat agctcgcgt                                                    19


<210>  70
<211>  42
<212>  DNA
<213>  Artificial Sequence

<220>
<223>  Promoter 1.6GI Variant

<400>  70
gcccttgaca atgccacatc ctgagcaaat aattcaacca ct                          42


<210>  71
<211>  42
<212>  DNA
<213>  Artificial Sequence

<220>
<223>  Promoter 1.5GI

<400>  71
gcccttgact atgccacatc ctgagcaaat aattcaacca ct                          42


<210>  72
<211>  18
<212>  DNA
<213>  Artificial Sequence

<220>
<223>  Primer Scr1

<400>  72
cctttctttg tgaatcgg                                                    18


<210>  73
<211>  18
<212>  DNA
<213>  Artificial Sequence

<220>
<223>  Primer Scr2

<400>  73
agaaacaggg tgtgatcc                                                    18


<210>  74
<211>  20
<212>  DNA
<213>  Artificial Sequence

<220>
<223>  Primer Scr3

<400>  74
agtgatcatc acctgttgcc                                                  20


<210>  75
<211>  20
<212>  DNA
<213>  Artificial Sequence

<220>
<223>  Primer Scr4

<400>  75
agcacggcga gagtcgacgg                                                  20


<210>  76
<211>  672
<212>  DNA
<213>  Saccharomyces cerevisiae

<400>  76
agttcgagtt tatcattatc aatactgcca tttcaaagaa tacgtaaata attaatagta      60

gtgattttcc taactttatt tagtcaaaaa attagccttt taattctgct gtaacccgta     120

catgcccaaa atagggggcg ggttacacag aatatataac atcgtaggtg tctgggtgaa     180

cagtttattc ctggcatcca ctaaatataa tggagcccgc tttttaagct ggcatccaga     240

aaaaaaaaga atcccagcac caaaatattg ttttcttcac caaccatcag ttcataggtc     300

cattctctta gcgcaactac agagaacagg ggcacaaaca ggcaaaaaac gggcacaacc     360

tcaatggagt gatgcaacct gcctggagta aatgatgaca caaggcaatt gacccacgca     420

tgtatctatc tcattttctt acaccttcta ttaccttctg ctctctctga tttggaaaaa     480

gctgaaaaaa aaggttgaaa ccagttccct gaaattattc ccctacttga ctaataagta     540

tataaagacg gtaggtattg attgtaattc tgtaaatcta tttcttaaac ttcttaaatt 600

ctacttttat agttagtctt ttttttagtt ttaaaacacc aagaacttag tttcgaataa 660

acacacataa ac 672

<210> 77
<211> 270
<212> DNA
<213> Saccharomyces cerevisiae

<400> 77
gacctcgagt catgtaatta gttatgtcac gcttacattc acgccctccc cccacatccg 60

ctctaaccga aaaggaagga gttagacaac ctgaagtcta ggtccctatt tattttttta 120

tagttatgtt agtattaaga acgttattta tatttcaaat ttttcttttt tttctgtaca 180

gacgcgtgta cgcatgtaac attatactga aaaccttgct tgagaaggtt ttgggacgct 240

cgaaggcttt aatttgcggc cggtacccaa 270

<210> 78
<211> 1716
<212> DNA
<213> Bacillus subtilis

<400> 78
atgttgacaa aagcaacaaa agaacaaaaa tcccttgtga aaaacagagg ggcggagctt 60

gttgttgatt gcttagtgga gcaaggtgtc acacatgtat ttggcattcc aggtgcaaaa 120

attgatgcgg tatttgacgc tttacaagat aaaggacctg aaattatcgt tgcccggcac 180

gaacaaaacg cagcattcat ggcccaagca gtcggccgtt taactggaaa accgggagtc 240

gtgttagtca catcaggacc gggtgcctct aacttggcaa caggcctgct gacagcgaac 300

actgaaggag accctgtcgt tgcgcttgct ggaaacgtga tccgtgcaga tcgtttaaaa 360

cggacacatc aatctttgga taatgcggcg ctattccagc cgattacaaa atacagtgta 420

gaagttcaag atgtaaaaaa tataccggaa gctgttacaa atgcatttag gatagcgtca 480

gcagggcagg ctggggccgc ttttgtgagc tttccgcaag atgttgtgaa tgaagtcaca 540

aatacgaaaa acgtgcgtgc tgttgcagcg ccaaaactcg gtcctgcagc agatgatgca 600

atcagtgcgg ccatagcaaa aatccaaaca gcaaaacttc ctgtcgtttt ggtcggcatg 660

aaaggcggaa gaccggaagc aattaaagcg gttcgcaagc ttttgaaaaa ggttcagctt 720

ccatttgttg aaacatatca agctgccggt accctttcta gagatttaga ggatcaatat 780

tttggccgta tcggtttgtt ccgcaaccag cctggcgatt tactgctaga gcaggcagat 840

gttgttctga cgatcggcta tgacccgatt gaatatgatc cgaaattctg gaatatcaat 900

ggagaccgga caattatcca tttagacgag attatcgctg acattgatca tgcttaccag 960

cctgatcttg aattgatcgg tgacattccg tccacgatca atcatatcga acacgatgct 1020

gtgaaagtgg aatttgcaga gcgtgagcag aaaatccttt ctgatttaaa acaatatatg 1080

catgaaggtg agcaggtgcc tgcagattgg aaatcagaca gagcgcaccc tcttgaaatc 1140

gttaaagagt tgcgtaatgc agtcgatgat catgttacag taacttgcga tatcggttcg 1200

cacgccattt ggatgtcacg ttatttccgc agctacgagc cgttaacatt aatgatcagt 1260

aacggtatgc aaacactcgg cgttgcgctt ccttgggcaa tcggcgcttc attggtgaaa 1320

ccgggagaaa aagtggtttc tgtctctggt gacggcggtt tcttattctc agcaatggaa 1380

ttagagacag cagttcgact aaaagcacca attgtacaca ttgtatggaa cgacagcaca 1440

tatgacatgg ttgcattcca gcaattgaaa aaatataacc gtacatctgc ggtcgatttc 1500

ggaaatatcg atatcgtgaa atatgcggaa agcttcggag caactggctt gcgcgtagaa 1560

tcaccagacc agctggcaga tgttctgcgt caaggcatga acgctgaagg tcctgtcatc 1620

atcgatgtcc cggttgacta cagtgataac attaatttag caagtgacaa gcttccgaaa 1680

gaattcgggg aactcatgaa aacgaaagct ctctag 1716

<210> 79
<211> 643
<212> DNA
<213> Saccharomyces cerevisiae

<400> 79
gaaatgaata acaatactga cagtactaaa taattgccta cttggcttca catacgttgc 60

atacgtcgat atagataata atgataatga cagcaggatt atcgtaatac gtaatagttg 120

aaaatctcaa aaatgtgtgg gtcattacgt aaataatgat aggaatggga ttcttctatt 180

tttccttttt ccattctagc agccgtcggg aaaacgtggc atcctctctt tcgggctcaa 240

ttggagtcac gctgccgtga gcatcctctc tttccatatc taacaactga gcacgtaacc 300

aatggaaaag catgagctta gcgttgctcc aaaaaagtat tggatggtta ataccatttg 360

tctgttctct tctgactttg actcctcaaa aaaaaaaaat ctacaatcaa cagatcgctt 420

caattacgcc ctcacaaaaa cttttttcct tcttcttcgc ccacgttaaa ttttatccct 480

catgttgtct aacggatttc tgcacttgat ttattataaa aagacaaaga cataatactt 540

ctctatcaat ttcagttatt gttcttcctt gcgttattct tctgttcttc tttttctttt 600

gtcatatata accataacca agtaatacat attcaaatct aga 643

<210> 80
<211> 1188
<212> DNA
<213> Saccharomyces cerevisiae

<400> 80
atgttgagaa ctcaagccgc cagattgatc tgcaactccc gtgtcatcac tgctaagaga 60

acctttgctt tggccacccg tgctgctgct tacagcagac cagctgcccg tttcgttaag 120

ccaatgatca ctacccgtgg tttgaagcaa atcaacttcg gtggtactgt tgaaaccgtc 180

tacgaaagag ctgactggcc aagagaaaag ttgttggact acttcaagaa cgacactttt 240

gctttgatcg gttacggttc ccaaggttac ggtcaaggtt tgaacttgag agacaacggt 300

ttgaacgtta tcattggtgt ccgtaaagat ggtgcttctt ggaaggctgc catcgaagac 360

ggttgggttc caggcaagaa cttgttcact gttgaagatg ctatcaagag aggtagttac 420

gttatgaact tgttgtccga tgccgctcaa tcagaaacct ggcctgctat caagccattg 480

ttgaccaagg gtaagacttt gtacttctcc cacggtttct ccccagtctt caaggacttg 540

actcacgttg aaccaccaaa ggacttagat gttatcttgg ttgctccaaa gggttccggt 600

agaactgtca gatctttgtt caaggaaggt cgtggtatta actcttctta cgccgtctgg 660

aacgatgtca ccggtaaggc tcacgaaaag gcccaagctt tggccgttgc cattggttcc 720

ggttacgttt accaaaccac tttcgaaaga gaagtcaact ctgacttgta cggtgaaaga 780

ggttgtttaa tgggtggtat ccacggtatg ttcttggctc aatacgacgt cttgagagaa 840

aacggtcact ccccatctga agctttcaac gaaaccgtcg aagaagctac ccaatctcta 900

tacccattga tcggtaagta cggtatggat tacatgtacg atgcttgttc caccaccgcc 960

agaagaggtg ctttggactg gtacccaatc ttcaagaatg ctttgaagcc tgttttccaa 1020

gacttgtacg aatctaccaa gaacggtacc gaaaccaaga gatctttgga attcaactct 1080

caacctgact acagagaaaa gctagaaaag gaattagaca ccatcagaaa catggaaatc 1140

tggaaggttg gtaaggaagt cagaaagttg agaccagaaa accaataa 1188

<210> 81
<211> 760
<212> DNA
<213> Saccharomyces cerevisiae

<400> 81
tcttttccga tttttttcta aaccgtggaa tatttcggat atccttttgt tgtttccggg 60

tgtacaatat ggacttcctc ttttctggca accaaaccca tacatcggga ttcctataat 120

accttcgttg gtctccctaa catgtaggtg gcggagggga gatatacaat agaacagata 180

ccagacaaga cataatgggc taaacaagac tacaccaatt acactgcctc attgatggtg 240

gtacataacg aactaatact gtagccctag acttgatagc catcatcata tcgaagtttc 300

actacccttt ttccatttgc catctattga agtaataata ggcgcatgca acttcttttc 360

tttttttttc ttttctctct cccccgttgt tgtctcacca tatccgcaat gacaaaaaaa 420

tgatggaaga cactaaagga aaaaattaac gacaaagaca gcaccaacag atgtcgttgt 480

tccagagctg atgaggggta tctcgaagca cacgaaactt tttccttcct tcattcacgc 540

acactactct ctaatgagca acggtatacg gccttccttc cagttacttg aatttgaaat 600

aaaaaaaagt ttgctgtctt gctatcaagt ataaatagac ctgcaattat taatcttttg 660

tttcctcgtc attgttctcg ttccctttct tccttgtttc tttttctgca caatatttca 720

agctatacca agcatacaat caactatctc atatacaatg 760

<210> 82
<211> 316
<212> DNA
<213> Saccharomyces cerevisiae

<400> 82
gagtaagcga atttcttatg atttatgatt tttattatta aataagttat aaaaaaaata 60

agtgtataca aattttaaag tgactcttag gttttaaaac gaaaattctt attcttgagt 120

aactctttcc tgtaggtcag gttgctttct caggtatagc atgaggtcgc tcttattgac 180

cacacctcta ccggcatgcc gagcaaatgc ctgcaaatcg ctccccattt cacccaattg 240

tagatatgct aactccagca atgagttgat gaatctcggt gtgtatttta tgtcctcaga 300

ggacaacacc tgtggt 316

<210> 83
<211> 1758
<212> DNA
<213> Saccharomyces cerevisiae

<400> 83
atgggcttgt taacgaaagt tgctacatct agacaattct ctacaacgag atgcgttgca 60

aagaagctca acaagtactc gtatatcatc actgaaccta agggccaagg tgcgtcccag 120

gccatgcttt atgccaccgg tttcaagaag gaagatttca agaagcctca agtcggggtt 180

ggttcctgtt ggtggtccgg taacccatgt aacatgcatc tattggactt gaataacaga 240

tgttctcaat ccattgaaaa agcgggtttg aaagctatgc agttcaacac catcggtgtt 300

tcagacggta tctctatggg tactaaaggt atgagatact cgttacaaag tagagaaatc 360

attgcagact cctttgaaac catcatgatg gcacaacact acgatgctaa catcgccatc 420

ccatcatgtg acaaaaacat gcccggtgtc atgatggcca tgggtagaca taacagacct 480

tccatcatgg tatatggtgg tactatcttg cccggtcatc caacatgtgg ttcttcgaag 540

atctctaaaa acatcgatat cgtctctgcg ttccaatcct acggtgaata tatttccaag 600

caattcactg aagaagaaag agaagatgtt gtggaacatg catgcccagg tcctggttct 660

tgtggtggta tgtatactgc caacacaatg gcttctgccg ctgaagtgct aggtttgacc 720

attccaaact cctcttcctt cccagccgtt tccaaggaga agttagctga gtgtgacaac 780

attggtgaat acatcaagaa gacaatggaa ttgggtattt tacctcgtga tatcctcaca 840

aaagaggctt ttgaaaacgc cattacttat gtcgttgcaa ccggtgggtc cactaatgct 900

gttttgcatt tggtggctgt tgctcactct gcgggtgtca agttgtcacc agatgatttc 960

caaagaatca gtgatactac accattgatc ggtgacttca aaccttctgg taaatacgtc 1020

atggccgatt tgattaacgt tggtggtacc caatctgtga ttaagtatct atatgaaaac 1080

aacatgttgc acggtaacac aatgactgtt accggtgaca ctttggcaga acgtgcaaag 1140

aaagcaccaa gcctacctga aggacaagag attattaagc cactctccca cccaatcaag    1200

gccaacggtc acttgcaaat tctgtacggt tcattggcac caggtggagc tgtgggtaaa    1260

attaccggta aggaaggtac ttacttcaag ggtagagcac gtgtgttcga agaggaaggt    1320

gcctttattg aagccttgga aagaggtgaa atcaagaagg gtgaaaaaac cgttgttgtt    1380

atcagatatg aaggtccaag aggtgcacca ggtatgcctg aaatgctaaa gccttcctct    1440

gctctgatgg gttacggttt gggtaaagat gttgcattgt tgactgatgg tagattctct    1500

ggtggttctc acgggttctt aatcggccac attgttcccg aagccgctga aggtggtcct    1560

atcgggttgg tcagagacgg cgatgagatt atcattgatg ctgataataa caagattgac    1620

ctattagtct ctgataagga aatggctcaa cgtaaacaaa gttgggttgc acctccacct    1680

cgttacacaa gaggtactct atccaagtat gctaagttgg tttccaacgc ttccaacggt    1740

tgtgttttag atgcttga                                                  1758

<210>  84
<211>  753
<212>  DNA
<213>  Saccharomyces cerevisiae

<400>  84
gcatgcttgc atttagtcgt gcaatgtatg actttaagat ttgtgagcag gaagaaaagg      60

gagaatcttc taacgataaa cccttgaaaa actgggtaga ctacgctatg ttgagttgct     120

acgcaggctg cacaattaca cgagaatgct cccgcctagg atttaaggct aagggacgtg     180

caatgcagac gacagatcta aatgaccgtg tcggtgaagt gttcgccaaa cttttcggtt     240

aacacatgca gtgatgcacg cgcgatggtg ctaagttaca tatatatata tatagccata     300

gtgatgtcta agtaaccttt atggtatatt tcttaatgtg gaaagatact agcgcgcgca     360

cccacacaca agcttcgtct tttcttgaag aaaagaggaa gctcgctaaa tgggattcca     420

ctttccgttc cctgccagct gatggaaaaa ggttagtgga acgatgaaga ataaaaagag     480

agatccactg aggtgaaatt tcagctgaca gcgagtttca tgatcgtgat gaacaatggt     540

aacgagttgt ggctgttgcc agggagggtg gttctcaact tttaatgtat ggccaaatcg     600

ctacttgggt ttgttatata acaaagaaga aataatgaac tgattctctt cctccttctt     660

gtcctttctt aattctgttg taattacctt cctttgtaat tttttttgta attattcttc     720

ttaataatcc aaacaaacac acatattaca ata                                 753

<210>  85
<211>  20
<212>  DNA
<213>  Artificial Sequence

<220>
<223>  Primer N98SeqF1

<400>  85
cgtgttagtc acatcaggac                                                    20


<210>  86
<211>  24
<212>  DNA
<213>  Artificial Sequence

<220>
<223>  Primer N98SeqF2

<400>  86
ggccatagca aaaatccaaa cagc                                               24


<210>  87
<211>  24
<212>  DNA
<213>  Artificial Sequence

<220>
<223>  Primer N98SeqF3

<400>  87
ccacgatcaa tcatatcgaa cacg                                               24


<210>  88
<211>  20
<212>  DNA
<213>  Artificial Sequence

<220>
<223>  Primer N98SeqF4

<400>  88
ggtttctgtc tctggtgacg                                                    20


<210>  89
<211>  22
<212>  DNA
<213>  Artificial Sequence

<220>
<223>  Primer N99SeqR1

<400>  89
gtctggtgat tctacgcgca ag                                                 22


<210>  90
<211>  22
<212>  DNA
<213>  Artificial Sequence

<220>
<223>  Primer N99SeqR2

<400>  90
catcgactgc attacgcaac tc                                                 22


<210>  91

<211>  22
<212>  DNA
<213>  Artificial Sequence

<220>
<223>  Primer N99SeqR3

<400>  91
cgatcgtcag aacaacatct gc                                                22


<210>  92
<211>  20
<212>  DNA
<213>  Artificial Sequence

<220>
<223>  Primer N99SeqR4

<400>  92
ccttcagtgt tcgctgtcag                                                   20


<210>  93
<211>  36
<212>  DNA
<213>  Artificial Sequence

<220>
<223>  Primer N136

<400>  93
ccgcggatag atctgaaatg aataacaata ctgaca                                 36


<210>  94
<211>  65
<212>  DNA
<213>  Artificial Sequence

<220>
<223>  Primer N137

<400>  94
taccaccgaa gttgatttgc ttcaacatcc tcagctctag atttgaatat gtattacttg      60

gttat                                                                   65


<210>  95
<211>  28
<212>  DNA
<213>  Artificial Sequence

<220>
<223>  Primer N138

<400>  95
atgttgaagc aaatcaactt cggtggta                                          28


<210>  96
<211>  22
<212>  DNA
<213>  Artificial Sequence

<220>
<223>  Primer N139

<400>  96
ttattggttt tctggtctca ac                                              22


<210>  97
<211>  57
<212>  DNA
<213>  Artificial Sequence

<220>
<223>  Primer N140

<400>  97
aagttgagac cagaaaacca ataattaatt aatcatgtaa ttagttatgt cacgctt        57


<210>  98
<211>  30
<212>  DNA
<213>  Artificial Sequence

<220>
<223>  Primer N141

<400>  98
gcggccgccc gcaaattaaa gccttcgagc                                      30


<210>  99
<211>  28
<212>  DNA
<213>  Artificial Sequence

<220>
<223>  Primer N142

<400>  99
ggatccgcat gcttgcattt agtcgtgc                                        28


<210>  100
<211>  56
<212>  DNA
<213>  Artificial Sequence

<220>
<223>  N143

<400>  100
caggtaatcc cccacagtat acatcctcag ctattgtaat atgtgtgttt gtttgg         56


<210>  101
<211>  22
<212>  DNA
<213>  Artificial Sequence

<220>
<223>  Primer N144

<400>  101
   atgtatactg tgggggatta cc                                                22

<210>  102
<211>  22
<212>  DNA
<213>  Artificial Sequence

<220>
<223>  Primer N145

<400>  102
ttagctttta ttttgctccg ca                                                22

<210>  103
<211>  57
<212>  DNA
<213>  Artificial Sequence

<220>
<223>  Primer N146

<400>  103
tttgcggagc aaaataaaag ctaattaatt aagagtaagc gaatttctta tgattta          57

<210>  104
<211>  28
<212>  DNA
<213>  Artificial Sequence

<220>
<223>  Primer N147

<400>  104
actagtacca caggtgttgt cctctgag                                          28

<210>  105
<211>  22
<212>  DNA
<213>  Artificial Sequence

<220>
<223>  Primer N151

<400>  105
ctagagagct ttcgttttca tg                                                22

<210>  106
<211>  57
<212>  DNA
<213>  Artificial Sequence

<220>
<223>  Primer N152

<400>  106
ctcatgaaaa cgaaagctct ctagttaatt aatcatgtaa ttagttatgt cacgctt          57

<210>  107
<211>  25

<212>  DNA
<213>  Artificial Sequence

<220>
<223>  Primer N155

<400>  107
atggcaaaga agctcaacaa gtact                                          25


<210>  108
<211>  22
<212>  DNA
<213>  Artificial Sequence

<220>
<223>  Primer N156

<400>  108
tcaagcatct aaaacacaac cg                                             22


<210>  109
<211>  57
<212>  DNA
<213>  Artificial Sequence

<220>
<223>  Primer N157

<400>  109
aacggttgtg ttttagatgc ttgattaatt aagagtaagc gaatttctta tgattta       57


<210>  110
<211>  28
<212>  DNA
<213>  Artificial Sequence

<220>
<223>  Primer N158

<400>  110
ggatcctttt ctggcaacca aacccata                                       28


<210>  111
<211>  56
<212>  DNA
<213>  Artificial Sequence

<220>
<223>  Primer N159

<400>  111
cgagtacttg ttgagcttct ttgccatcct cagcgagata gttgattgta tgcttg        56


<210>  112
<211>  19
<212>  DNA
<213>  Artificial Sequence

<220>
<223>  Primer N160SeqF1

<400>  112
gaaaacgtgg catcctctc                                                    19


<210>  113
<211>  19
<212>  DNA
<213>  Artificial Sequence

<220>
<223>  Primer N160SeqF2

<400>  113
gctgactggc caagagaaa                                                    19


<210>  114
<211>  20
<212>  DNA
<213>  Artificial Sequence

<220>
<223>  Primer N160SeqF3

<400>  114
tgtacttctc ccacggtttc                                                   20


<210>  115
<211>  22
<212>  DNA
<213>  Artificial Sequence

<220>
<223>  Primer N160SeqF4

<400>  115
agctacccaa tctctatacc ca                                                22


<210>  116
<211>  22
<212>  DNA
<213>  Artificial Sequence

<220>
<223>  Primer N160SeqF5

<400>  116
cctgaagtct aggtccctat tt                                                22


<210>  117
<211>  19
<212>  DNA
<213>  Artificial Sequence

<220>
<223>  N160SeqR1

<400>  117
gcgtgaatgt aagcgtgac                                                    19

<210>  118
<211>  20
<212>  DNA
<213>  Artificial Sequence

<220>
<223>  Primer N160SeqR2

<400>  118
cgtcgtattg agccaagaac                                                   20


<210>  119
<211>  20
<212>  DNA
<213>  Artificial Sequence

<220>
<223>  Primer N160SeqR3

<400>  119
gcatcggaca acaagttcat                                                   20


<210>  120
<211>  22
<212>  DNA
<213>  Artificial Sequence

<220>
<223>  Primer N160SeqR4

<400>  120
tcgttcttga agtagtccaa ca                                                22


<210>  121
<211>  19
<212>  DNA
<213>  Artificial Sequence

<220>
<223>  Primer N160SeqR5

<400>  121
tgagcccgaa agagaggat                                                    19


<210>  122
<211>  19
<212>  DNA
<213>  Artificial Sequence

<220>
<223>  Primer N161SeqF1

<400>  122
acggtatacg gccttcctt                                                    19


<210>  123
<211>  20
<212>  DNA
<213>  Artificial Sequence

<220>
<223>  Primer N161SeqF2

<400>  123
gggtttgaaa gctatgcagt                                                    20


<210>  124
<211>  22
<212>  DNA
<213>  Artificial Sequence

<220>
<223>  Primer N161SeqF3

<400>  124
ggtggtatgt atactgccaa ca                                                 22


<210>  125
<211>  22
<212>  DNA
<213>  Artificial Sequence

<220>
<223>  Primer N161SeqF4

<400>  125
ggtggtaccc aatctgtgat ta                                                 22


<210>  126
<211>  20
<212>  DNA
<213>  Artificial Sequence

<220>
<223>  Primer N161SeqF5

<400>  126
cggtttgggt aaagatgttg                                                    20


<210>  127
<211>  22
<212>  DNA
<213>  Artificial Sequence

<220>
<223>  Primer N161SeqF6

<400>  127
aaacgaaaat tcttattctt ga                                                 22


<210>  128
<211>  22
<212>  DNA
<213>  Artificial Sequence

<220>
<223>  Primer N161SeqR1

<400>  128
tcgttttaaa acctaagagt ca                                                 22

<210>  129
<211>  19
<212>  DNA
<213>  Artificial Sequence

<220>
<223>  Primer N161SeqR2

<400>  129
ccaaaccgta acccatcag                                                    19


<210>  130
<211>  19
<212>  DNA
<213>  Artificial Sequence

<220>
<223>  Primer N161SeqR3

<400>  130
cacagattgg gtaccacca                                                    19


<210>  131
<211>  20
<212>  DNA
<213>  Artificial Sequence

<220>
<223>  Primer N161Seqr4

<400>  131
accacaagaa ccaggacctg                                                   20


<210>  132
<211>  19
<212>  DNA
<213>  Artificial Sequence

<220>
<223>  Primer N161SeqR5

<400>  132
catagctttc aaacccgct                                                    19


<210>  133
<211>  22
<212>  DNA
<213>  Artificial Sequence

<220>
<223>  Primer N161SeqR6

<400>  133
cgtataccgt tgctcattag ag                                                22


<210>  134
<211>  23
<212>  DNA

<213>  Artificial Sequence

<220>
<223>  Primer N162

<400>  134
atgttgacaa aagcaacaaa aga                                              23


<210>  135
<211>  38
<212>  DNA
<213>  Artificial Sequence

<220>
<223>  Primer N189

<400>  135
atccgcggat agatctagtt cgagtttatc attatcaa                              38


<210>  136
<211>  53
<212>  DNA
<213>  Artificial Sequence

<220>
<223>  Primer N190.1

<400>  136
ttcttttgtt gcttttgtca acatcctcag cgtttatgtg tgtttattcg aaa             53


<210>  137
<211>  38
<212>  DNA
<213>  Artificial Sequence

<220>
<223>  Primer N176

<400>  137
atccgcggat agatctatta gaagccgccg agcgggcg                              38


<210>  138
<211>  31
<212>  DNA
<213>  Artificial Sequence

<220>
<223>  Primer N177

<400>  138
atcctcagct tttctccttg acgttaaagt a                                     31


<210>  139
<211>  477
<212>  PRT
<213>  Escherichia coli

<400>  139

Met Thr Gln Ser Arg Leu His Ala Ala Gln Asn Ala Leu Ala Lys Leu

1               5                   10                  15

His Glu His Arg Gly Asn Thr Phe Tyr Pro His Phe His Leu Ala Pro
            20                  25                  30

Pro Ala Gly Trp Met Asn Asp Pro Asn Gly Leu Ile Trp Phe Asn Asp
        35                  40                  45

Arg Tyr His Ala Phe Tyr Gln His His Pro Met Ser Glu His Trp Gly
    50                  55                  60

Pro Met His Trp Gly His Ala Thr Ser Asp Asp Met Ile His Trp Gln
65                  70                  75                  80

His Glu Pro Ile Ala Leu Ala Pro Gly Asp Asp Asn Asp Lys Asp Gly
                85                  90                  95

Cys Phe Ser Gly Ser Ala Val Asp Asp Asn Gly Val Leu Ser Leu Ile
            100                 105                 110

Tyr Thr Gly His Val Trp Leu Asp Gly Ala Gly Asn Asp Asp Ala Ile
        115                 120                 125

Arg Glu Val Gln Cys Leu Ala Thr Ser Arg Asp Gly Ile His Phe Glu
    130                 135                 140

Lys Gln Gly Val Ile Leu Thr Pro Pro Glu Gly Ile Met His Phe Arg
145                 150                 155                 160

Asp Pro Lys Val Trp Arg Glu Ala Asp Thr Trp Trp Met Val Val Gly
                165                 170                 175

Ala Lys Asp Pro Gly Asn Thr Gly Gln Ile Leu Leu Tyr Arg Gly Ser
            180                 185                 190

Ser Leu Arg Glu Trp Thr Phe Asp Arg Val Leu Ala His Ala Asp Ala
        195                 200                 205

Gly Glu Ser Tyr Met Trp Glu Cys Pro Asp Phe Phe Ser Leu Gly Asp
    210                 215                 220

Gln His Tyr Leu Met Phe Ser Pro Gln Gly Met Asn Ala Glu Gly Tyr
225                 230                 235                 240

Ser Tyr Arg Asn Arg Phe Gln Ser Gly Val Ile Pro Gly Met Trp Ser
                245                 250                 255

Pro Gly Arg Leu Phe Ala Gln Ser Gly His Phe Thr Glu Leu Asp Asn
            260                 265                 270

Gly His Asp Phe Tyr Ala Pro Gln Ser Phe Leu Ala Lys Asp Gly Arg
        275                 280                 285

Arg Ile Val Ile Gly Trp Met Asp Met Trp Glu Ser Pro Met Pro Ser
    290                 295                 300

Lys Arg Glu Gly Trp Ala Gly Cys Met Thr Leu Ala Arg Glu Leu Ser
305                 310                 315                 320

Glu Ser Asn Gly Lys Leu Leu Gln Arg Pro Val His Glu Ala Glu Ser
                325                 330                 335

Leu Arg Gln Gln His Gln Ser Val Ser Pro Arg Thr Ile Ser Asn Lys
            340                 345                 350

Tyr Val Leu Gln Glu Asn Ala Gln Ala Val Glu Ile Gln Leu Gln Trp
        355                 360                 365

Ala Leu Lys Asn Ser Asp Ala Glu His Tyr Gly Leu Gln Leu Gly Thr
    370                 375                 380

Gly Met Arg Leu Tyr Ile Asp Asn Gln Ser Glu Arg Leu Val Leu Trp
385                 390                 395                 400

Arg Tyr Tyr Pro His Glu Asn Leu Asp Gly Tyr Arg Ser Ile Pro Leu
                405                 410                 415

Pro Gln Arg Asp Thr Leu Ala Leu Arg Ile Phe Ile Asp Thr Ser Ser
            420                 425                 430

Val Glu Val Phe Ile Asn Asp Gly Glu Ala Val Met Ser Ser Arg Ile
        435                 440                 445

Tyr Pro Gln Pro Glu Glu Arg Glu Leu Ser Leu Tyr Ala Ser His Gly
    450                 455                 460

Val Ala Val Leu Gln His Gly Ala Leu Trp Leu Leu Gly
465                 470                 475

<210>  140
<211>  304
<212>  PRT
<213>  Escherichia coli

<400>  140

Met Ser Ala Lys Val Trp Val Leu Gly Asp Ala Val Val Asp Leu Leu
1               5                   10                  15

Pro Glu Ser Asp Gly Arg Leu Leu Pro Cys Pro Gly Gly Ala Pro Ala
            20                  25                  30

Asn Val Ala Val Gly Ile Ala Arg Leu Gly Gly Thr Ser Gly Phe Ile
        35                  40                  45

Gly Arg Val Gly Asp Asp Pro Phe Gly Ala Leu Met Gln Arg Thr Leu
    50                  55                  60

Leu Thr Glu Gly Val Asp Ile Thr Tyr Leu Lys Gln Asp Glu Trp His
65                  70                  75                  80

Arg Thr Ser Thr Val Leu Val Asp Leu Asn Asp Gln Gly Glu Arg Ser
                85                  90                  95

Phe Thr Phe Met Val Arg Pro Ser Ala Asp Leu Phe Leu Glu Thr Thr
            100                 105                 110

Asp Leu Pro Cys Trp Arg His Gly Glu Trp Leu His Leu Cys Ser Ile
        115                 120                 125

Ala Leu Ser Ala Glu Pro Ser Arg Thr Ser Ala Phe Thr Ala Met Thr
    130                 135                 140

Ala Ile Arg His Ala Gly Gly Phe Val Ser Phe Asp Pro Asn Ile Arg
145                 150                 155                 160

Glu Asp Leu Trp Gln Asp Glu His Leu Leu Arg Leu Cys Leu Arg Gln
                165                 170                 175

Ala Leu Gln Leu Ala Asp Val Val Lys Leu Ser Glu Glu Glu Trp Arg
            180                 185                 190

Leu Ile Ser Gly Lys Thr Gln Asn Asp Gln Asp Ile Cys Ala Leu Ala
        195                 200                 205

Lys Glu Tyr Glu Ile Ala Met Leu Leu Val Thr Lys Gly Ala Glu Gly
    210                 215                 220

Val Val Val Cys Tyr Arg Gly Gln Val His His Phe Ala Gly Met Ser
225                 230                 235                 240

Val Asn Cys Val Asp Ser Thr Gly Ala Gly Asp Ala Phe Val Ala Gly
                245                 250                 255

Leu Leu Thr Gly Leu Ser Ser Thr Gly Leu Ser Thr Asp Glu Arg Glu
            260                 265                 270

Met Arg Arg Ile Ile Asp Leu Ala Gln Arg Cys Gly Ala Leu Ala Val

275                 280                 285

Thr Ala Lys Gly Ala Met Thr Ala Leu Pro Cys Arg Gln Glu Leu Glu
    290                 295                 300

<210>  141
<211>  415
<212>  PRT
<213>  Escherichia coli

<400>  141

Met Ala Leu Asn Ile Pro Phe Arg Asn Ala Tyr Tyr Arg Phe Ala Ser
1               5                   10                  15

Ser Tyr Ser Phe Leu Phe Phe Ile Ser Trp Ser Leu Trp Trp Ser Leu
            20                  25                  30

Tyr Ala Ile Trp Leu Lys Gly His Leu Gly Leu Thr Gly Thr Glu Leu
        35                  40                  45

Gly Thr Leu Tyr Ser Val Asn Gln Phe Thr Ser Ile Leu Phe Met Met
    50                  55                  60

Phe Tyr Gly Ile Val Gln Asp Lys Leu Gly Leu Lys Lys Pro Leu Ile
65                  70                  75                  80

Trp Cys Met Ser Phe Ile Leu Val Leu Thr Gly Pro Phe Met Ile Tyr
                85                  90                  95

Val Tyr Glu Pro Leu Leu Gln Ser Asn Phe Ser Val Gly Leu Ile Leu
            100                 105                 110

Gly Ala Leu Phe Phe Gly Leu Gly Tyr Leu Ala Gly Cys Gly Leu Leu
        115                 120                 125

Asp Ser Phe Thr Glu Lys Met Ala Arg Asn Phe His Phe Glu Tyr Gly
    130                 135                 140

Thr Ala Arg Ala Trp Gly Ser Phe Gly Tyr Ala Ile Gly Ala Phe Phe
145                 150                 155                 160

Ala Gly Ile Phe Phe Ser Ile Ser Pro His Ile Asn Phe Trp Leu Val
                165                 170                 175

Ser Leu Phe Gly Ala Val Phe Met Met Ile Asn Met Arg Phe Lys Asp
            180                 185                 190

Lys Asp His Gln Cys Val Ala Ala Asp Ala Gly Gly Val Lys Lys Glu
        195                 200                 205

Asp Phe Ile Ala Val Phe Lys Asp Arg Asn Phe Trp Val Phe Val Ile
    210                 215                 220

Phe Ile Val Gly Thr Trp Ser Phe Tyr Asn Ile Phe Asp Gln Gln Leu
225                 230                 235                 240

Phe Pro Val Phe Tyr Ser Gly Leu Phe Glu Ser His Asp Val Gly Thr
                245                 250                 255

Arg Leu Tyr Gly Tyr Leu Asn Ser Phe Gln Val Val Leu Glu Ala Leu
            260                 265                 270

Cys Met Ala Ile Ile Pro Phe Phe Val Asn Arg Val Gly Pro Lys Asn
        275                 280                 285

Ala Leu Leu Ile Gly Val Val Ile Met Ala Leu Arg Ile Leu Ser Cys
    290                 295                 300

Ala Leu Phe Val Asn Pro Trp Ile Ile Ser Leu Val Lys Leu Leu His
305                 310                 315                 320

Ala Ile Glu Val Pro Leu Cys Val Ile Ser Val Phe Lys Tyr Ser Val
                325                 330                 335

Ala Asn Phe Asp Lys Arg Leu Ser Ser Thr Ile Phe Leu Ile Gly Phe
            340                 345                 350

Gln Ile Ala Ser Ser Leu Gly Ile Val Leu Leu Ser Thr Pro Thr Gly
        355                 360                 365

Ile Leu Phe Asp His Ala Gly Tyr Gln Thr Val Phe Phe Ala Ile Ser
    370                 375                 380

Gly Ile Val Cys Leu Met Leu Leu Phe Gly Ile Phe Phe Leu Ser Lys
385                 390                 395                 400

Lys Arg Glu Gln Ile Val Met Glu Thr Pro Val Pro Ser Ala Ile
                405                 410                 415

<210>  142
<211>  6341
<212>  DNA
<213>  Artificial Sequence

<220>
<223>  Plasmid pFP988DssPspac

<400>  142
gatccaagtt taaactgtac actagatatt tcttctccgc ttaaatcatc aaagaaatct      60

ttatcacttg taaccagtcc gtccacatgt cgaattgcat ctgaccgaat tttacgtttc     120

cctgaataat tctcatcaat cgtttcatca attttatctt tatactttat attttgtgcg 180

ttaatcaaat cataattttt atatgtttcc tcatgattta tgtctttatt attatagttt 240

ttattctctc tttgattatg tctttgtatc ccgtttgtat tacttgatcc tttaactctg 300

gcaaccctca aaattgaatg agacatgcta cacctccgga taataaatat atataaacgt 360

atatagattt cataaagtct aacacactag acttatttac ttcgtaatta agtcgttaaa 420

ccgtgtgctc tacgaccaaa actataaaac ctttaagaac tttctttttt tacaagaaaa 480

aagaaattag ataaatctct catatctttt attcaataat cgcatccgat tgcagtataa 540

atttaacgat cactcatcat gttcatattt atcagagctc gtgctataat tatactaatt 600

ttataaggag gaaaaaatat gggcattttt agtatttttg taatcagcac agttcattat 660

caaccaaaca aaaaataagt ggttataatg aatcgttaat aagcaaaatt catataacca 720

aattaaagag ggttataatg aacgagaaaa atataaaaca cagtcaaaac tttattactt 780

caaaacataa tatagataaa ataatgacaa atataagatt aaatgaacat gataatatct 840

ttgaaatcgg ctcaggaaaa ggccatttta cccttgaatt agtaaagagg tgtaatttcg 900

taactgccat tgaaatagac cataaattat gcaaaactac agaaaataaa cttgttgatc 960

acgataattt ccaagtttta aacaaggata tattgcagtt taaatttcct aaaaaccaat 1020

cctataaaat atatggtaat ataccttata acataagtac ggatataata cgcaaaattg 1080

tttttgatag tatagctaat gagatttatt taatcgtgga atacgggttt gctaaaagat 1140

tattaaatac aaaacgctca ttggcattac ttttaatggc agaagttgat atttctatat 1200

taagtatggt tccaagagaa tattttcatc ctaaacctaa agtgaatagc tcacttatca 1260

gattaagtag aaaaaaatca agaatatcac acaaagataa acaaaagtat aattatttcg 1320

ttatgaaatg ggttaacaaa gaatacaaga aaatatttac aaaaaatcaa tttaacaatt 1380

ccttaaaaca tgcaggaatt gacgatttaa acaatattag ctttgaacaa ttcttatctc 1440

ttttcaatag ctataaatta tttaataagt aagttaaggg atgcagttca tcgatgaagg 1500

caactacagc tcaggcgaca accatacgct gagagatcct cactacgtag aagataaagg 1560

ccacaaatac ttagtatttg aagcaaacac tggaactgaa gatggctacc aaggcgaaga 1620

atctttattt aacaaagcat actatggcaa aagcacatca ttcttccgtc aagaaagtca 1680

aaaacttctg caaagcgata aaaaacgcac ggctgagtta gcaaacggcg ctctcggtat 1740

gattgagcta aacgatgatt acacactgaa aaaagtgatg aaaccgctga ttgcatctaa 1800

cacagtaaca gatgaaattg aacgcgcgaa cgtctttaaa atgaacggca aatggtacct 1860

gttcactgac tcccgcggat caaaaatgac gattgacggc attacgtcta acgatattta 1920

catgcttggt tatgtttcta attctttaac tggcccatac aagccgctga acaaaactgg 1980

ccttgtgtta aaaatggatc ttgatcctaa cgatgtaacc tttacttact cacacttcgc 2040

tgtacctcaa gcgaaaggaa acaatgtcgt gattacaagc tatatgacaa acagaggatt 2100

ctacgcagac aaacaatcaa cgtttgcgcc aagcttgcat gcgagagtag ggaactgcca 2160

ggcatcaaat aaaacgaaag gctcagtcga aagactgggc ctttcgtttt atctgttgtt 2220

tgtcggtgaa cgctctcctg agtaggacaa atccgccggg agcggatttg aacgttgcga 2280

agcaacggcc cggagggtgg cgggcaggac gcccgccata aactgccagg catcaaatta 2340

agcagaaggc catcctgacg gatggccttt ttgcgtttct acaaactctt tttgtttatt 2400

tttctaaata cattcaaata tgtatccgct catgctccgg atctgcatcg caggatgctg 2460

ctggctaccc tgtggaacac ctacatctgt attaacgaag cgctggcatt gaccctgagt 2520

gatttttctc tggtcccgcc gcatccatac cgccagttgt ttaccctcac aacgttccag 2580

taaccgggca tgttcatcat cagtaacccg tatcgtgagc atcctctctc gtttcatcgg 2640

tatcattacc cccatgaaca gaaattcccc cttacacgga ggcatcaagt gaccaaacag 2700

gaaaaaaccg cccttaacat ggcccgcttt atcagaagcc agacattaac gcttctggag 2760

aaactcaacg agctggacgc ggatgaacag gcagacatct gtgaatcgct tcacgaccac 2820

gctgatgagc tttaccgcag ctgcctcgcg cgtttcggtg atgacggtga aaacctctga 2880

cacatgcagc tcccggagac ggtcacagct tgtctgtaag cggatgccgg gagcagacaa 2940

gcccgtcagg gcgcgtcagc gggtgttggc gggtgtcggg gcgcagccat gacccagtca 3000

cgtagcgata gcggagtgta tactggctta actatgcggc atcagagcag attgtactga 3060

gagtgcacca tatgcggtgt gaaataccgc acagatgcgt aaggagaaaa taccgcatca 3120

ggcgctcttc cgcttcctcg ctcactgact cgctgcgctc ggtcgttcgg ctgcggcgag 3180

cggtatcagc tcactcaaag gcggtaatac ggttatccac agaatcaggg gataacgcag 3240

gaaagaacat gtgagcaaaa ggccagcaaa aggccaggaa ccgtaaaaag gccgcgttgc 3300

tggcgttttt ccataggctc cgcccccctg acgagcatca caaaaatcga cgctcaagtc 3360

agaggtggcg aaacccgaca ggactataaa gataccaggc gtttccccct ggaagctccc 3420

tcgtgcgctc tcctgttccg accctgccgc ttaccggata cctgtccgcc tttctccctt 3480

cgggaagcgt ggcgctttct caatgctcac gctgtaggta tctcagttcg gtgtaggtcg 3540

ttcgctccaa gctgggctgt gtgcacgaac cccccgttca gcccgaccgc tgcgccttat 3600

ccggtaacta tcgtcttgag tccaacccgg taagacacga cttatcgcca ctggcagcag 3660

ccactggtaa caggattagc agagcgaggt atgtaggcgg tgctacagag ttcttgaagt 3720

ggtggcctaa ctacggctac actagaagga cagtatttgg tatctgcgct ctgctgaagc 3780

cagttacctt cggaaaaaga gttggtagct cttgatccgg caaacaaacc accgctggta 3840

gcggtggttt ttttgtttgc aagcagcaga ttacgcgcag aaaaaaagga tctcaagaag 3900

atcctttgat cttttctacg gggtctgacg ctcagtggaa cgaaaactca cgttaaggga 3960

ttttggtcat gagattatca aaaaggatct tcacctagat ccttttaaat taaaaatgaa 4020

gttttaaatc aatctaaagt atatatgagt aaacttggtc tgacagttac caatgcttaa 4080

tcagtgaggc acctatctca gcgatctgtc tatttcgttc atccatagtt gcctgactcc 4140

ccgtcgtgta gataactacg atacgggagg gcttaccatc tggccccagt gctgcaatga 4200

taccgcgaga cccacgctca ccggctccag atttatcagc aataaaccag ccagccggaa 4260

gggccgagcg cagaagtggt cctgcaactt tatccgcctc catccagtct attaattgtt 4320

gccgggaagc tagagtaagt agttcgccag ttaatagttt gcgcaacgtt gttgccattg 4380

ctgcaggcat cgtggtgtca cgctcgtcgt ttggtatggc ttcattcagc tccggttccc 4440

aacgatcaag gcgagttaca tgatccccca tgttgtgcaa aaaagcggtt agctccttcg 4500

gtcctccgat cgttgtcaga agtaagttgg ccgcagtgtt atcactcatg gttatggcag 4560

cactgcataa ttctcttact gtcatgccat ccgtaagatg cttttctgtg actggtgagt 4620

actcaaccaa gtcattctga gaatagtgta tgcggcgacc gagttgctct tgcccggcgt 4680

caatacggga taataccgcg ccacatagca gaactttaaa agtgctcatc attggaaaac 4740

gttcttcggg gcgaaaactc tcaaggatct taccgctgtt gagatccagt tcgatgtaac 4800

ccactcgtgc acccaactga tcttcagcat cttttacttt caccagcgtt tctgggtgag 4860

caaaaacagg aaggcaaaat gccgcaaaaa agggaataag ggcgacacgg aaatgttgaa 4920

tactcatact cttccttttt caatattatt gaagcattta tcagggttat tgtctcatga 4980

gcggatacat atttgaatgt atttagaaaa ataaacaaat aggggttccg cgcacatttc 5040

cccgaaaagt gccacctgac gtctaagaaa ccattattat catgacatta acctataaaa 5100

ataggcgtat cacgaggccc tttcgtctcg cgcgtttcgg tgatgacggt gaaaacctct 5160

gacacatgca gctcccggag acggtcacag cttgtctgta agcggatgcc gggagcagac 5220

aagcccgtca gggcgcgtca gcgggtgttc atgtgcgtaa ctaacttgcc atcttcaaac 5280

aggagggctg gaagaagcag accgctaaca cagtacataa aaaaggagac atgaacgatg 5340

aacatcaaaa agtttgcaaa acaagcaaca gtattaacct ttactaccgc actgctggca 5400

ggaggcgcaa ctcaagcgtt tgcgaaagaa acgaaccaaa agccatataa ggaaacatac 5460

ggcatttccc atattacacg ccatgatatg ctgcaaatcc ctgaacagca aaaaaatgaa 5520

aaatatcaag ttcctgaatt cgattcgtcc acaattaaaa atatctcttc tgcaaaaggc 5580

ctggacgttt gggacagctg gccattacaa aacgctgacg gcactgtcgc aaactatcac 5640

ggctaccaca tcgtctttgc attagccgga gatcctaaaa atgcggatga cacatcgatt 5700

tacatgttct atcaaaaagt cggcgaaact tctattgaca gctggaaaaa cgctggccgc 5760

gtctttaaag acagcgacaa attcgatgca aatgattcta tcctaaaaga ccaaacacaa 5820

gaatggtcag gttcagccac atttacatct gacggaaaaa tccgtttatt ctacactgat 5880

ttctccggta aacattacgg caaacaaaca ctgacaactg cacaagttaa cgtatcagca 5940

tcagacagct ctttgaacat caacggtgta gaggattata aatcaatctt tgacggtgac 6000

ggaaaaacgt atcaaaatgt acagaattcg agctctcgag taattctaca cagcccagtc 6060

cagactattc ggcactgaaa ttatgggtga agtggtcaag acctcactag gcaccttaaa 6120

aatagcgcac cctgaagaag atttatttga ggtagccctt gcctacctag cttccaagaa 6180

agatatccta acagcacaag agcggaaaga tgttttgttc tacatccaga acaacctctg 6240

ctaaaattcc tgaaaaattt tgcaaaaagt tgttgacttt atctacaagg tgtggcataa 6300

tgtgtggaat tgtgagcgct cacaattaag cttgaattcc c 6341

<210> 143
<211> 6221
<212> DNA
<213> Artificial Sequence

<220>
<223> Plasmid pFP988DssPgroE

<400> 143
tcgagagcta ttgtaacata atcggtacgg gggtgaaaaa gctaacggaa aagggagcgg 60

aaaagaatga tgtaagcgtg aaaaattttt tatcttatca cttgaaattg gaagggagat 120

tctttattat aagaattgtg gaattgtgag cggataacaa ttcccaatta aaggaggaaa 180

ctagtggatc caagtttaaa ctgtacacta gatatttctt ctccgcttaa atcatcaaag 240

aaatctttat cacttgtaac cagtccgtcc acatgtcgaa ttgcatctga ccgaatttta 300

cgtttccctg aataattctc atcaatcgtt tcatcaattt tatctttata ctttatattt 360

tgtgcgttaa tcaaatcata atttttatat gtttcctcat gatttatgtc tttattatta 420

tagtttttat tctctctttg attatgtctt tgtatcccgt ttgtattact tgatccttta 480

actctggcaa ccctcaaaat tgaatgagac atgctacacc tccggataat aaatatatat 540

aaacgtatat agatttcata aagtctaaca cactagactt atttacttcg taattaagtc 600

gttaaaccgt gtgctctacg accaaaacta taaaaccttt aagaactttc tttttttaca 660

agaaaaaaga aattagataa atctctcata tcttttattc aataatcgca tccgattgca 720

gtataaattt aacgatcact catcatgttc atatttatca gagctcgtgc tataattata 780

ctaattttat aaggaggaaa aaatatgggc atttttagta tttttgtaat cagcacagtt 840

cattatcaac caaacaaaaa ataagtggtt ataatgaatc gttaataagc aaaattcata 900

taaccaaatt aaagagggtt ataatgaacg agaaaaatat aaaacacagt caaaacttta 960

ttacttcaaa acataatata gataaaataa tgacaaatat aagattaaat gaacatgata 1020

atatctttga aatcggctca ggaaaaggcc attttaccct tgaattagta aagaggtgta 1080

atttcgtaac tgccattgaa atagaccata aattatgcaa aactacagaa aataaacttg 1140

ttgatcacga taatttccaa gttttaaaca aggatatatt gcagtttaaa tttcctaaaa 1200

accaatccta taaaatatat ggtaatatac cttataacat aagtacggat ataatacgca 1260

aaattgtttt tgatagtata gctaatgaga tttatttaat cgtggaatac gggtttgcta 1320

aaagattatt aaatacaaaa cgctcattgg cattactttt aatggcagaa gttgatattt 1380

ctatattaag tatggttcca agagaatatt ttcatcctaa acctaaagtg aatagctcac 1440

ttatcagatt aagtagaaaa aaatcaagaa tatcacacaa agataaacaa aagtataatt 1500

atttcgttat gaaatgggtt aacaaagaat acaagaaaat atttacaaaa aatcaattta 1560

acaattcctt aaaacatgca ggaattgacg atttaaacaa tattagcttt gaacaattct 1620

tatctctttt caatagctat aaattattta ataagtaagt taagggatgc agttcatcga 1680

tgaaggcaac tacagctcag gcgacaacca tacgctgaga gatcctcact acgtagaaga 1740

taaaggccac aaatacttag tatttgaagc aaacactgga actgaagatg gctaccaagg 1800

cgaagaatct ttatttaaca aagcatacta tggcaaaagc acatcattct tccgtcaaga 1860

aagtcaaaaa cttctgcaaa gcgataaaaa acgcacggct gagttagcaa acggcgctct 1920

cggtatgatt gagctaaacg atgattacac actgaaaaaa gtgatgaaac cgctgattgc 1980

atctaacaca gtaacagatg aaattgaacg cgcgaacgtc tttaaaatga acggcaaatg 2040

gtacctgttc actgactccc gcggatcaaa aatgacgatt gacggcatta cgtctaacga 2100

tatttacatg cttggttatg tttctaattc tttaactggc ccatacaagc cgctgaacaa 2160

aactggcctt gtgttaaaaa tggatcttga tcctaacgat gtaaccttta cttactcaca 2220

cttcgctgta cctcaagcga aaggaaacaa tgtcgtgatt acaagctata tgacaaacag 2280

aggattctac gcagacaaac aatcaacgtt tgcgccaagc ttgcatgcga gagtagggaa 2340

ctgccaggca tcaaataaaa cgaaaggctc agtcgaaaga ctgggccttt cgttttatct 2400

gttgtttgtc ggtgaacgct ctcctgagta ggacaaatcc gccgggagcg gatttgaacg 2460

ttgcgaagca acggcccgga gggtggcggg caggacgccc gccataaact gccaggcatc 2520

aaattaagca gaaggccatc ctgacggatg gcctttttgc gtttctacaa actctttttg 2580

tttatttttc taaatacatt caaatatgta tccgctcatg ctccggatct gcatcgcagg 2640

atgctgctgg ctaccctgtg gaacacctac atctgtatta acgaagcgct ggcattgacc 2700

ctgagtgatt tttctctggt cccgccgcat ccataccgcc agttgtttac cctcacaacg 2760

ttccagtaac cgggcatgtt catcatcagt aacccgtatc gtgagcatcc tctctcgttt 2820

catcggtatc attaccccca tgaacagaaa ttccccctta cacggaggca tcaagtgacc 2880

aaacaggaaa aaaccgccct taacatggcc cgctttatca gaagccagac attaacgctt 2940

ctggagaaac tcaacgagct ggacgcggat gaacaggcag acatctgtga atcgcttcac 3000

gaccacgctg atgagcttta ccgcagctgc ctcgcgcgtt tcggtgatga cggtgaaaac 3060

ctctgacaca tgcagctccc ggagacggtc acagcttgtc tgtaagcgga tgccgggagc 3120

agacaagccc gtcagggcgc gtcagcgggt gttggcgggt gtcggggcgc agccatgacc 3180

cagtcacgta gcgatagcgg agtgtatact ggcttaacta tgcggcatca gagcagattg 3240

tactgagagt gcaccatatg cggtgtgaaa taccgcacag atgcgtaagg agaaaatacc 3300

gcatcaggcg ctcttccgct tcctcgctca ctgactcgct gcgctcggtc gttcggctgc 3360

ggcgagcggt atcagctcac tcaaaggcgg taatacggtt atccacagaa tcaggggata 3420

acgcaggaaa gaacatgtga gcaaaaggcc agcaaaaggc caggaaccgt aaaaaggccg 3480

cgttgctggc gtttttccat aggctccgcc cccctgacga gcatcacaaa aatcgacgct 3540

caagtcagag gtggcgaaac ccgacaggac tataaagata ccaggcgttt ccccctggaa 3600

gctccctcgt gcgctctcct gttccgaccc tgccgcttac cggatacctg tccgcctttc 3660

tcccttcggg aagcgtggcg ctttctcaat gctcacgctg taggtatctc agttcggtgt 3720

aggtcgttcg ctccaagctg ggctgtgtgc acgaaccccc cgttcagccc gaccgctgcg 3780

ccttatccgg taactatcgt cttgagtcca acccggtaag acacgactta tcgccactgg 3840

cagcagccac tggtaacagg attagcagag cgaggtatgt aggcggtgct acagagttct 3900

tgaagtggtg gcctaactac ggctacacta gaaggacagt atttggtatc tgcgctctgc 3960

tgaagccagt taccttcgga aaaagagttg gtagctcttg atccggcaaa caaaccaccg 4020

ctggtagcgg tggttttttt gtttgcaagc agcagattac gcgcagaaaa aaaggatctc 4080

aagaagatcc tttgatcttt tctacggggt ctgacgctca gtggaacgaa aactcacgtt 4140

aagggatttt ggtcatgaga ttatcaaaaa ggatcttcac ctagatcctt ttaaattaaa 4200

aatgaagttt taaatcaatc taaagtatat atgagtaaac ttggtctgac agttaccaat 4260

gcttaatcag tgaggcacct atctcagcga tctgtctatt tcgttcatcc atagttgcct 4320

gactccccgt cgtgtagata actacgatac gggagggctt accatctggc cccagtgctg 4380

caatgatacc gcgagaccca cgctcaccgg ctccagattt atcagcaata aaccagccag 4440

ccggaagggc cgagcgcaga agtggtcctg caactttatc cgcctccatc cagtctatta 4500

attgttgccg ggaagctaga gtaagtagtt cgccagttaa tagtttgcgc aacgttgttg 4560

ccattgctgc aggcatcgtg gtgtcacgct cgtcgtttgg tatggcttca ttcagctccg 4620

gttcccaacg atcaaggcga gttacatgat cccccatgtt gtgcaaaaaa gcggttagct 4680

ccttcggtcc tccgatcgtt gtcagaagta agttggccgc agtgttatca ctcatggtta 4740

tggcagcact gcataattct cttactgtca tgccatccgt aagatgcttt tctgtgactg 4800

gtgagtactc aaccaagtca ttctgagaat agtgtatgcg gcgaccgagt tgctcttgcc 4860

cggcgtcaat acgggataat accgcgccac atagcagaac tttaaaagtg ctcatcattg 4920

gaaaacgttc ttcggggcga aaactctcaa ggatcttacc gctgttgaga tccagttcga 4980

tgtaacccac tcgtgcaccc aactgatctt cagcatcttt tactttcacc agcgtttctg 5040

ggtgagcaaa aacaggaagg caaaatgccg caaaaaaggg aataagggcg acacggaaat 5100

gttgaatact catactcttc ctttttcaat attattgaag catttatcag ggttattgtc 5160

tcatgagcgg atacatattt gaatgtattt agaaaaataa acaaataggg gttccgcgca 5220

catttccccg aaaagtgcca cctgacgtct aagaaaccat tattatcatg acattaacct 5280

ataaaaatag gcgtatcacg aggccctttc gtctcgcgcg tttcggtgat gacggtgaaa 5340

acctctgaca catgcagctc ccggagacgg tcacagcttg tctgtaagcg gatgccggga 5400

gcagacaagc ccgtcagggc gcgtcagcgg gtgttcatgt gcgtaactaa cttgccatct 5460

tcaaacagga gggctggaag aagcagaccg ctaacacagt acataaaaaa ggagacatga 5520

acgatgaaca tcaaaaagtt tgcaaaacaa gcaacagtat taacctttac taccgcactg 5580

ctggcaggag gcgcaactca agcgtttgcg aaagaaacga accaaaagcc atataaggaa 5640

acatacggca tttcccatat tacacgccat gatatgctgc aaatccctga acagcaaaaa 5700

aatgaaaaat atcaagttcc tgaattcgat tcgtccacaa ttaaaaatat ctcttctgca 5760

aaaggcctgg acgtttggga cagctggcca ttacaaaacg ctgacggcac tgtcgcaaac 5820

tatcacggct accacatcgt ctttgcatta gccggagatc ctaaaaatgc ggatgacaca 5880

tcgatttaca tgttctatca aaaagtcggc gaaacttcta ttgacagctg gaaaaacgct 5940

ggccgcgtct ttaaagacag cgacaaattc gatgcaaatg attctatcct aaaagaccaa 6000

acacaagaat ggtcaggttc agccacattt acatctgacg gaaaaatccg tttattctac 6060

actgatttct ccggtaaaca ttacggcaaa caaacactga caactgcaca agttaacgta 6120

tcagcatcag acagctcttt gaacatcaac ggtgtagagg attataaatc aatctttgac 6180

ggtgacggaa aaacgtatca aaatgtacag aattcgagct c 6221

<210> 144
<211> 40
<212> DNA
<213> Artificial Sequence

<220>
<223> Primer T-budB (BamHI)

<400> 144
agatagatgg atccggaggt gggtcacatg gacaaacagt 40

<210> 145
<211> 29
<212> DNA
<213> Artificial Sequence

<220>
<223> Primer B-kivD (BamHI)

<400> 145
ctctagagga tccagactcc taggacatg 29

<210> 146
<211> 6039
<212> DNA

<213>  Artificial Sequence

<220>
<223>  Vector fragment pFP988Dss

<400>  146
gatccaagtt taaactgtac actagatatt tcttctccgc ttaaatcatc aaagaaatct      60

ttatcacttg taaccagtcc gtccacatgt cgaattgcat ctgaccgaat tttacgtttc     120

cctgaataat tctcatcaat cgtttcatca attttatctt tatactttat attttgtgcg     180

ttaatcaaat cataattttt atatgtttcc tcatgattta tgtctttatt attatagttt     240

ttattctctc tttgattatg tctttgtatc ccgtttgtat tacttgatcc tttaactctg     300

gcaaccctca aaattgaatg agacatgcta cacctccgga taataaatat atataaacgt     360

atatagattt cataaagtct aacacactag acttatttac ttcgtaatta agtcgttaaa     420

ccgtgtgctc tacgaccaaa actataaaac ctttaagaac tttctttttt tacaagaaaa     480

aagaaattag ataaatctct catatctttt attcaataat cgcatccgat tgcagtataa     540

atttaacgat cactcatcat gttcatattt atcagagctc gtgctataat tatactaatt     600

ttataaggag gaaaaaatat gggcattttt agtatttttg taatcagcac agttcattat     660

caaccaaaca aaaaataagt ggttataatg aatcgttaat aagcaaaatt catataacca     720

aattaaagag ggttataatg aacgagaaaa atataaaaca cagtcaaaac tttattactt     780

caaaacataa tatagataaa ataatgacaa atataagatt aaatgaacat gataatatct     840

ttgaaatcgg ctcaggaaaa ggccatttta cccttgaatt agtaaagagg tgtaatttcg     900

taactgccat tgaaatagac cataaattat gcaaaactac agaaaataaa cttgttgatc     960

acgataattt ccaagtttta aacaaggata tattgcagtt taaatttcct aaaaaccaat    1020

cctataaaat atatggtaat ataccttata acataagtac ggatataata cgcaaaattg    1080

tttttgatag tatagctaat gagatttatt taatcgtgga atacgggttt gctaaaagat    1140

tattaaatac aaaacgctca ttggcattac ttttaatggc agaagttgat atttctatat    1200

taagtatggt tccaagagaa tattttcatc ctaaacctaa agtgaatagc tcacttatca    1260

gattaagtag aaaaaaatca agaatatcac acaaagataa acaaaagtat aattatttcg    1320

ttatgaaatg ggttaacaaa gaatacaaga aaatatttac aaaaaatcaa tttaacaatt    1380

ccttaaaaca tgcaggaatt gacgatttaa acaatattag ctttgaacaa ttcttatctc    1440

ttttcaatag ctataaatta tttaataagt aagttaaggg atgcagttca tcgatgaagg    1500

caactacagc tcaggcgaca accatacgct gagagatcct cactacgtag aagataaagg    1560

ccacaaatac ttagtatttg aagcaaacac tggaactgaa gatggctacc aaggcgaaga    1620

atctttattt aacaaagcat actatggcaa aagcacatca ttcttccgtc aagaaagtca    1680

aaaacttctg caaagcgata aaaaacgcac ggctgagtta gcaaacggcg ctctcggtat    1740

gattgagcta aacgatgatt acacactgaa aaaagtgatg aaaccgctga ttgcatctaa    1800

cacagtaaca gatgaaattg aacgcgcgaa cgtctttaaa atgaacggca aatggtacct 1860

gttcactgac tcccgcggat caaaaatgac gattgacggc attacgtcta acgatattta 1920

catgcttggt tatgtttcta attctttaac tggcccatac aagccgctga acaaaactgg 1980

ccttgtgtta aaaatggatc ttgatcctaa cgatgtaacc tttacttact cacacttcgc 2040

tgtacctcaa gcgaaaggaa acaatgtcgt gattacaagc tatatgacaa acagaggatt 2100

ctacgcagac aaacaatcaa cgtttgcgcc aagcttgcat gcgagagtag ggaactgcca 2160

ggcatcaaat aaaacgaaag gctcagtcga aagactgggc ctttcgtttt atctgttgtt 2220

tgtcggtgaa cgctctcctg agtaggacaa atccgccggg agcggatttg aacgttgcga 2280

agcaacggcc cggagggtgg cgggcaggac gcccgccata aactgccagg catcaaatta 2340

agcagaaggc catcctgacg gatggccttt ttgcgtttct acaaactctt tttgtttatt 2400

tttctaaata cattcaaata tgtatccgct catgctccgg atctgcatcg caggatgctg 2460

ctggctaccc tgtggaacac ctacatctgt attaacgaag cgctggcatt gaccctgagt 2520

gatttttctc tggtcccgcc gcatccatac cgccagttgt ttaccctcac aacgttccag 2580

taaccgggca tgttcatcat cagtaacccg tatcgtgagc atcctctctc gtttcatcgg 2640

tatcattacc cccatgaaca gaaattcccc cttacacgga ggcatcaagt gaccaaacag 2700

gaaaaaaccg cccttaacat ggcccgcttt atcagaagcc agacattaac gcttctggag 2760

aaactcaacg agctggacgc ggatgaacag gcagacatct gtgaatcgct tcacgaccac 2820

gctgatgagc tttaccgcag ctgcctcgcg cgtttcggtg atgacggtga aaacctctga 2880

cacatgcagc tcccggagac ggtcacagct tgtctgtaag cggatgccgg gagcagacaa 2940

gcccgtcagg gcgcgtcagc gggtgttggc gggtgtcggg gcgcagccat gacccagtca 3000

cgtagcgata gcggagtgta tactggctta actatgcggc atcagagcag attgtactga 3060

gagtgcacca tatgcggtgt gaaataccgc acagatgcgt aaggagaaaa taccgcatca 3120

ggcgctcttc cgcttcctcg ctcactgact cgctgcgctc ggtcgttcgg ctgcggcgag 3180

cggtatcagc tcactcaaag gcggtaatac ggttatccac agaatcaggg gataacgcag 3240

gaaagaacat gtgagcaaaa ggccagcaaa aggccaggaa ccgtaaaaag gccgcgttgc 3300

tggcgttttt ccataggctc cgcccccctg acgagcatca caaaaatcga cgctcaagtc 3360

agaggtggcg aaacccgaca ggactataaa gataccaggc gtttccccct ggaagctccc 3420

tcgtgcgctc tcctgttccg accctgccgc ttaccggata cctgtccgcc tttctccctt 3480

cgggaagcgt ggcgctttct caatgctcac gctgtaggta tctcagttcg gtgtaggtcg 3540

ttcgctccaa gctgggctgt gtgcacgaac cccccgttca gcccgaccgc tgcgccttat 3600

ccggtaacta tcgtcttgag tccaacccgg taagacacga cttatcgcca ctggcagcag 3660

ccactggtaa caggattagc agagcgaggt atgtaggcgg tgctacagag ttcttgaagt 3720

ggtggcctaa ctacggctac actagaagga cagtatttgg tatctgcgct ctgctgaagc 3780
cagttacctt cggaaaaaga gttggtagct cttgatccgg caaacaaacc accgctggta 3840
gcggtggttt ttttgtttgc aagcagcaga ttacgcgcag aaaaaaagga tctcaagaag 3900
atcctttgat cttttctacg gggtctgacg ctcagtggaa cgaaaactca cgttaaggga 3960
ttttggtcat gagattatca aaaaggatct tcacctagat ccttttaaat taaaaatgaa 4020
gttttaaatc aatctaaagt atatatgagt aaacttggtc tgacagttac caatgcttaa 4080
tcagtgaggc acctatctca gcgatctgtc tatttcgttc atccatagtt gcctgactcc 4140
ccgtcgtgta gataactacg atacgggagg gcttaccatc tggccccagt gctgcaatga 4200
taccgcgaga cccacgctca ccggctccag atttatcagc aataaaccag ccagccggaa 4260
gggccgagcg cagaagtggt cctgcaactt tatccgcctc catccagtct attaattgtt 4320
gccgggaagc tagagtaagt agttcgccag ttaatagttt gcgcaacgtt gttgccattg 4380
ctgcaggcat cgtggtgtca cgctcgtcgt ttggtatggc ttcattcagc tccggttccc 4440
aacgatcaag gcgagttaca tgatccccca tgttgtgcaa aaaagcggtt agctccttcg 4500
gtcctccgat cgttgtcaga agtaagttgg ccgcagtgtt atcactcatg gttatggcag 4560
cactgcataa ttctcttact gtcatgccat ccgtaagatg cttttctgtg actggtgagt 4620
actcaaccaa gtcattctga gaatagtgta tgcggcgacc gagttgctct tgcccggcgt 4680
caatacggga taataccgcg ccacatagca gaactttaaa agtgctcatc attggaaaac 4740
gttcttcggg gcgaaaactc tcaaggatct taccgctgtt gagatccagt tcgatgtaac 4800
ccactcgtgc acccaactga tcttcagcat cttttacttt caccagcgtt tctgggtgag 4860
caaaaacagg aaggcaaaat gccgcaaaaa agggaataag ggcgacacgg aaatgttgaa 4920
tactcatact cttccttttt caatattatt gaagcattta tcagggttat tgtctcatga 4980
gcggatacat atttgaatgt atttagaaaa ataaacaaat aggggttccg cgcacatttc 5040
cccgaaaagt gccacctgac gtctaagaaa ccattattat catgacatta acctataaaa 5100
ataggcgtat cacgaggccc tttcgtctcg cgcgtttcgg tgatgacggt gaaaacctct 5160
gacacatgca gctcccggag acggtcacag cttgtctgta agcggatgcc gggagcagac 5220
aagcccgtca gggcgcgtca gcgggtgttc atgtgcgtaa ctaacttgcc atcttcaaac 5280
aggagggctg gaagaagcag accgctaaca cagtacataa aaaaggagac atgaacgatg 5340
aacatcaaaa agtttgcaaa acaagcaaca gtattaacct ttactaccgc actgctggca 5400
ggaggcgcaa ctcaagcgtt tgcgaaagaa acgaaccaaa agccatataa ggaaacatac 5460
ggcatttccc atattacacg ccatgatatg ctgcaaatcc ctgaacagca aaaaaatgaa 5520
aaatatcaag ttcctgaatt cgattcgtcc acaattaaaa atatctcttc tgcaaaaggc 5580
ctggacgttt gggacagctg gccattacaa aacgctgacg gcactgtcgc aaactatcac 5640
ggctaccaca tcgtctttgc attagccgga gatcctaaaa atgcggatga cacatcgatt 5700

tacatgttct atcaaaaagt cggcgaaact tctattgaca gctggaaaaa cgctggccgc 5760

gtctttaaag acagcgacaa attcgatgca aatgattcta tcctaaaaga ccaaacacaa 5820

gaatggtcag gttcagccac atttacatct gacggaaaaa tccgtttatt ctacactgat 5880

ttctccggta aacattacgg caaacaaaca ctgacaactg cacaagttaa cgtatcagca 5940

tcagacagct ctttgaacat caacggtgta gaggattata aatcaatctt tgacggtgac 6000

ggaaaaacgt atcaaaatgt acagaattcg agctctcga 6039

<210> 147
<211> 44
<212> DNA
<213> Artificial Sequence

<220>
<223> Primer T-groE(XhoI)

<400> 147
agatagatct cgagagctat tgtaacataa tcggtacggg ggtg 44

<210> 148
<211> 51
<212> DNA
<213> Artificial Sequence

<220>
<223> Primer B-groEL (SpeI BamH1)

<400> 148
attatgtcag gatccactag tttcctcctt taattgggaa ttgttatccg c 51

<210> 149
<211> 30
<212> DNA
<213> Artificial Sequence

<220>
<223> Primer T-groEL

<400> 149
agctattgta acataatcgg tacgggggtg 30

<210> 150
<211> 45
<212> DNA
<213> Artificial Sequence

<220>
<223> Primer T-ilvCB.s.(BamHI)

<400> 150
acattgatgg atcccataac aagggagaga ttgaaatggt aaaag 45

<210> 151
<211> 47
<212> DNA

<213>  Artificial Sequence

<220>
<223>  Primer B-ilvCB.s.(SpeIBamHI)

<400>  151
tagacaacgg atccactagt ttaattttgc gcaacggaga ccaccgc                    47


<210>  152
<211>  47
<212>  DNA
<213>  Artificial Sequence

<220>
<223>  Primer T-BD64 (DraIII)

<400>  152
ttaccgtgga ctcaccgagt gggtaactag cctcgccgga aagagcg                    47


<210>  153
<211>  48
<212>  DNA
<213>  Artificial Sequence

<220>
<223>  Primer B-BD64 (DraIII)

<400>  153
tcacagttaa gacacctggt gccgttaatg cgccatgaca gccatgat                   48


<210>  154
<211>  49
<212>  DNA
<213>  Artificial Sequence

<220>
<223>  Primer T-lacIq (DraIII)

<400>  154
acagatagat caccaggtgc aagctaattc cggtggaaac gaggtcatc                  49


<210>  155
<211>  48
<212>  DNA
<213>  Artificial Sequence

<220>
<223>  Primer B-lacIq (DraIII)

<400>  155
acagtacgat acacggggtg tcactgcccg ctttccagtc gggaaacc                   48


<210>  156
<211>  49
<212>  DNA
<213>  Artificial Sequence

<220>
<223>  Primer T-groE (DraIII)

<400>  156
tcggattacg caccccgtga gctattgtaa cataatcggt acgggggtg                49

<210>  157
<211>  48
<212>  DNA
<213>  Artificial Sequence

<220>
<223>  Primer B-B.s.ilvC (DraIII)

<400>  157
ctgctgatct cacaccgtgt gttaattttg cgcaacggag accaccgc                 48

<210>  158
<211>  1221
<212>  DNA
<213>  Clostridium acetobutylicum

<400>  158
cacacggtgt aaataataat ctaaacagga ggggttaaaa tggttgattt cgaatattca      60

ataccaacta gaattttttt cggtaaagat aagataaatg tacttggaag agagcttaaa     120

aaatatggtt ctaaagtgct tatagtttat ggtggaggaa gtataaagag aaatggaata     180

tatgataaag ctgtaagtat acttgaaaaa aacagtatta aattttatga acttgcagga     240

gtagagccaa atccaagagt aactacagtt gaaaaaggag ttaaaatatg tagagaaaat     300

ggagttgaag tagtactagc tataggtgga ggaagtgcaa tagattgcgc aaaggttata     360

gcagcagcat gtgaatatga tggaaatcca tgggatattg tgttagatgg ctcaaaaata     420

aaaagggtgc ttcctatagc tagtatatta accattgctg caacaggatc agaaatggat     480

acgtgggcag taataaataa tatggataca aacgaaaaac taattgcggc acatccagat     540

atggctccta agttttctat attagatcca acgtatacgt ataccgtacc taccaatcaa     600

acagcagcag gaacagctga tattatgagt catatatttg aggtgtattt tagtaataca     660

aaaacagcat atttgcagga tagaatggca gaagcgttat taagaacttg tattaaatat     720

ggaggaatag ctcttgagaa gccggatgat tatgaggcaa gagccaatct aatgtgggct     780

tcaagtcttg cgataaatgg acttttaaca tatggtaaag acactaattg gagtgtacac     840

ttaatggaac atgaattaag tgcttattac gacataacac acggcgtagg gcttgcaatt     900

ttaacaccta attggatgga gtatatttta aataatgata cagtgtacaa gtttgttgaa     960

tatggtgtaa atgtttgggg aatagacaaa gaaaaaaatc actatgacat agcacatcaa    1020

gcaatacaaa aaacaagaga ttactttgta aatgtactag gtttaccatc tagactgaga    1080

gatgttggaa ttgaagaaga aaaattggac ataatggcaa aggaatcagt aaagcttaca    1140

ggaggaacca taggaaacct aagaccagta aacgcctccg aagtcctaca aatattcaaa    1200

aaatctgtgt aacaccgagt g                                              1221

<210>  159
<211>  54
<212>  DNA
<213>  Artificial Sequence

<220>
<223>  Primer T-bdhB (DraIII)

<400>  159
tcgatagcat acacacggtg gttaacaaag gaggggttaa aatggttgat ttcg          54


<210>  160
<211>  91
<212>  DNA
<213>  Artificial Sequence

<220>
<223>  Primer B-bdhB (rrnBT1DraIII)

<400>  160
atctacgcac tcggtgataa aacgaaaggc ccagtctttc gactgagcct ttcgttttat     60

cttacacaga ttttttgaat atttgtagga c                                     91


<210>  161
<211>  29
<212>  DNA
<213>  Artificial Sequence

<220>
<223>  Primer LDH EcoRV F

<400>  161
gacgtcatga ccacccgccg atccctttt                                       29


<210>  162
<211>  30
<212>  DNA
<213>  Artificial Sequence

<220>
<223>  Primer LDH AatIIR

<400>  162
gatatccaac accagcgacc gacgtattac                                      30


<210>  163
<211>  47
<212>  DNA
<213>  Artificial Sequence

<220>
<223>  Primer Cm F

<400>  163
atttaaatct cgagtagagg atcccaacaa acgaaaattg gataaag                   47


<210>  164
<211>  29
<212>  DNA

<213>  Artificial Sequence

<220>
<223>  Primer Cm R

<400>  164
acgcgttatt ataaaagcca gtcattagg                                          29


<210>  165
<211>  58
<212>  DNA
<213>  Artificial Sequence

<220>
<223>  Primer P11 F-StuI

<400>  165
cctagcgcta tagttgttga cagaatggac atactatgat atattgttgc tatagcga          58


<210>  166
<211>  62
<212>  DNA
<213>  Artificial Sequence

<220>
<223>  Primer P11 R-SpeI

<400>  166
ctagtcgcta tagcaacaat atatcatagt atgtccattc tgtcaacaac tatagcgcta        60

gg                                                                       62


<210>  167
<211>  38
<212>  DNA
<213>  Artificial Sequence

<220>
<223>  Primer PldhL F-HindIII

<400>  167
aagcttgtcg acaaaccaac attatgacgt gtctgggc                                38


<210>  168
<211>  28
<212>  DNA
<213>  Artificial Sequence

<220>
<223>  Primer PldhL R-BamHI

<400>  168
ggatcctcat cctctcgtag tgaaaatt                                           28


<210>  169
<211>  36
<212>  DNA
<213>  Artificial Sequence

<220>

<223>  Primer F-bdhB-AvrII

<400>  169
ttcctaggaa ggaggtggtt aaaatggttg atttcg                                36


<210>  170
<211>  29
<212>  DNA
<213>  Artificial Sequence

<220>
<223>  Primer R-bdhB-BamHI

<400>  170
ttggatcctt acacagattt tttgaatat                                        29


<210>  171
<211>  39
<212>  DNA
<213>  Artificial Sequence

<220>
<223>  Primer F-ilvC(B.s.)-AflII

<400>  171
aacttaagaa ggaggtgatt gaaatggtaa aagtatatt                             39


<210>  172
<211>  32
<212>  DNA
<213>  Artificial Sequence

<220>
<223>  Primer R-ilvC(B.s.)-NotI

<400>  172
aagcggccgc ttaattttgc gcaacggaga cc                                     32


<210>  173
<211>  30
<212>  DNA
<213>  Artificial Sequence

<220>
<223>  Primer F-PnisA(HindIII)

<400>  173
ttaagcttga catacttgaa tgacctagtc                                       30


<210>  174
<211>  39
<212>  DNA
<213>  Artificial Sequence

<220>
<223>  Primer R-PnisA(SpeI BamHI)

<400>  174
ttggatccaa actagtataa tttattttgt agttccttc                             39

<210>  175
<211>  38
<212>  DNA
<213>  Artificial Sequence

<220>
<223>  Primer N191

<400>  175
atccgcggat agatctccca ttaccgacat ttgggcgc                              38


<210>  176
<211>  31
<212>  DNA
<213>  Artificial Sequence

<220>
<223>  Primer N192

<400>  176
atcctcagcg atgattgatt gattgattgt a                                     31


<210>  177
<211>  6509
<212>  DNA
<213>  Artificial Sequence

<220>
<223>  Vector pFP988

<400>  177
tcgaggcccc gcacatacga aaagactggc tgaaaacatt gagcctttga tgactgatga      60

tttggctgaa gaagtggatc gattgtttga gaaaagaaga agaccataaa aataccttgt     120

ctgtcatcag acagggtatt ttttatgctg tccagactgt ccgctgtgta aaaaatagga     180

ataaaggggg gttgttatta ttttactgat atgtaaaata taatttgtat aaggaattgt     240

gagcggataa caattcctac gaaaatgaga gggagaggaa acatgattca aaaacgaaag     300

cggacagttt cgttcagact tgtgcttatg tgcacgctgt tatttgtcag tttgccgatt     360

acaaaaacat cagccggatc ccaccatcac catcaccatt aagaattcct agaaactcca     420

agctatcttt aaaaaatcta gtaaatgcac gagcaacatc ttttgttgct cagtgcattt     480

tttattttgt acactagata tttcttctcc gcttaaatca tcaaagaaat ctttatcact     540

tgtaaccagt ccgtccacat gtcgaattgc atctgaccga attttacgtt tccctgaata     600

attctcatca atcgtttcat caattttatc tttatacttt atattttgtg cgttaatcaa     660

atcataattt ttatatgttt cctcatgatt tatgtcttta ttattatagt ttttattctc     720

tctttgatta tgtctttgta tcccgtttgt attacttgat cctttaactc tggcaaccct     780

caaaattgaa tgagacatgc tacacctccg gataataaat atatataaac gtatatagat     840

ttcataaagt ctaacacact agacttattt acttcgtaat taagtcgtta aaccgtgtgc     900

tctacgacca aaactataaa acctttaaga actttctttt tttacaagaa aaaagaaatt     960

agataaatct ctcatatctt ttattcaata atcgcatccg attgcagtat aaatttaacg 1020

atcactcatc atgttcatat ttatcagagc tcgtgctata attatactaa ttttataagg 1080

aggaaaaaat atgggcattt ttagtatttt tgtaatcagc acagttcatt atcaaccaaa 1140

caaaaaataa gtggttataa tgaatcgtta ataagcaaaa ttcatataac caaattaaag 1200

agggttataa tgaacgagaa aaatataaaa cacagtcaaa actttattac ttcaaaacat 1260

aatatagata aaataatgac aaatataaga ttaaatgaac atgataatat ctttgaaatc 1320

ggctcaggaa aaggccattt tacccttgaa ttagtaaaga ggtgtaattt cgtaactgcc 1380

attgaaatag accataaatt atgcaaaact acagaaaata aacttgttga tcacgataat 1440

ttccaagttt taaacaagga tatattgcag tttaaatttc ctaaaaacca atcctataaa 1500

atatatggta atatacctta taacataagt acggatataa tacgcaaaat tgtttttgat 1560

agtatagcta atgagattta tttaatcgtg gaatacgggt ttgctaaaag attattaaat 1620

acaaaacgct cattggcatt acttttaatg gcagaagttg atatttctat attaagtatg 1680

gttccaagag aatattttca tcctaaacct aaagtgaata gctcacttat cagattaagt 1740

agaaaaaaat caagaatatc acacaaagat aaacaaaagt ataattattt cgttatgaaa 1800

tgggttaaca aagaatacaa gaaaatattt acaaaaaatc aatttaacaa ttccttaaaa 1860

catgcaggaa ttgacgattt aaacaatatt agctttgaac aattcttatc tcttttcaat 1920

agctataaat tatttaataa gtaagttaag ggatgcagtt catcgatgaa ggcaactaca 1980

gctcaggcga caaccatacg ctgagagatc ctcactacgt agaagataaa ggccacaaat 2040

acttagtatt tgaagcaaac actggaactg aagatggcta ccaaggcgaa gaatctttat 2100

ttaacaaagc atactatggc aaaagcacat cattcttccg tcaagaaagt caaaaacttc 2160

tgcaaagcga taaaaaacgc acggctgagt tagcaaacgg cgctctcggt atgattgagc 2220

taaacgatga ttacacactg aaaaaagtga tgaaaccgct gattgcatct aacacagtaa 2280

cagatgaaat tgaacgcgcg aacgtcttta aaatgaacgg caaatggtac ctgttcactg 2340

actcccgcgg atcaaaaatg acgattgacg gcattacgtc taacgatatt tacatgcttg 2400

gttatgtttc taattcttta actggcccat acaagccgct gaacaaaact ggccttgtgt 2460

taaaaatgga tcttgatcct aacgatgtaa cctttactta ctcacacttc gctgtacctc 2520

aagcgaaagg aaacaatgtc gtgattacaa gctatatgac aaacagagga ttctacgcag 2580

acaaacaatc aacgtttgcg ccaagcttgc atgcgagagt agggaactgc caggcatcaa 2640

ataaaacgaa aggctcagtc gaaagactgg gcctttcgtt ttatctgttg tttgtcggtg 2700

aacgctctcc tgagtaggac aaatccgccg ggagcggatt tgaacgttgc gaagcaacgg 2760

cccggagggt ggcgggcagg acgcccgcca taaactgcca ggcatcaaat taagcagaag 2820

gccatcctga cggatggcct ttttgcgttt ctacaaactc tttttgttta tttttctaaa 2880

tacattcaaa tatgtatccg ctcatgctcc ggatctgcat cgcaggatgc tgctggctac 2940
cctgtggaac acctacatct gtattaacga agcgctggca ttgaccctga gtgatttttc 3000
tctggtcccg ccgcatccat accgccagtt gtttaccctc acaacgttcc agtaaccggg 3060
catgttcatc atcagtaacc cgtatcgtga gcatcctctc tcgtttcatc ggtatcatta 3120
cccccatgaa cagaaattcc cccttacacg gaggcatcaa gtgaccaaac aggaaaaaac 3180
cgcccttaac atggcccgct ttatcagaag ccagacatta acgcttctgg agaaactcaa 3240
cgagctggac gcggatgaac aggcagacat ctgtgaatcg cttcacgacc acgctgatga 3300
gctttaccgc agctgcctcg cgcgtttcgg tgatgacggt gaaaacctct gacacatgca 3360
gctcccggag acggtcacag cttgtctgta agcggatgcc gggagcagac aagcccgtca 3420
gggcgcgtca gcgggtgttg gcgggtgtcg gggcgcagcc atgacccagt cacgtagcga 3480
tagcggagtg tatactggct taactatgcg gcatcagagc agattgtact gagagtgcac 3540
catatgcggt gtgaaatacc gcacagatgc gtaaggagaa aataccgcat caggcgctct 3600
tccgcttcct cgctcactga ctcgctgcgc tcggtcgttc ggctgcggcg agcggtatca 3660
gctcactcaa aggcggtaat acggttatcc acagaatcag gggataacgc aggaaagaac 3720
atgtgagcaa aaggccagca aaaggccagg aaccgtaaaa aggccgcgtt gctggcgttt 3780
ttccataggc tccgcccccc tgacgagcat cacaaaaatc gacgctcaag tcagaggtgg 3840
cgaaacccga caggactata aagataccag gcgtttcccc ctggaagctc cctcgtgcgc 3900
tctcctgttc cgaccctgcc gcttaccgga tacctgtccg cctttctccc ttcgggaagc 3960
gtggcgcttt ctcaatgctc acgctgtagg tatctcagtt cggtgtaggt cgttcgctcc 4020
aagctgggct gtgtgcacga accccccgtt cagcccgacc gctgcgcctt atccggtaac 4080
tatcgtcttg agtccaaccc ggtaagacac gacttatcgc cactggcagc agccactggt 4140
aacaggatta gcagagcgag gtatgtaggc ggtgctacag agttcttgaa gtggtggcct 4200
aactacggct acactagaag gacagtattt ggtatctgcg ctctgctgaa gccagttacc 4260
ttcggaaaaa gagttggtag ctcttgatcc ggcaaacaaa ccaccgctgg tagcggtggt 4320
ttttttgttt gcaagcagca gattacgcgc agaaaaaaag gatctcaaga agatcctttg 4380
atcttttcta cggggtctga cgctcagtgg aacgaaaact cacgttaagg gattttggtc 4440
atgagattat caaaaaggat cttcacctag atccttttaa attaaaaatg aagttttaaa 4500
tcaatctaaa gtatatatga gtaaacttgg tctgacagtt accaatgctt aatcagtgag 4560
gcacctatct cagcgatctg tctatttcgt tcatccatag ttgcctgact ccccgtcgtg 4620
tagataacta cgatacggga gggcttacca tctggcccca gtgctgcaat gataccgcga 4680
gacccacgct caccggctcc agatttatca gcaataaacc agccagccgg aagggccgag 4740
cgcagaagtg gtcctgcaac tttatccgcc tccatccagt ctattaattg ttgccgggaa 4800
gctagagtaa gtagttcgcc agttaatagt ttgcgcaacg ttgttgccat tgctgcaggc 4860

atcgtggtgt cacgctcgtc gtttggtatg gcttcattca gctccggttc ccaacgatca 4920

aggcgagtta catgatcccc catgttgtgc aaaaaagcgg ttagctcctt cggtcctccg 4980

atcgttgtca gaagtaagtt ggccgcagtg ttatcactca tggttatggc agcactgcat 5040

aattctctta ctgtcatgcc atccgtaaga tgcttttctg tgactggtga gtactcaacc 5100

aagtcattct gagaatagtg tatgcggcga ccgagttgct cttgcccggc gtcaatacgg 5160

gataataccg cgccacatag cagaacttta aaagtgctca tcattggaaa acgttcttcg 5220

gggcgaaaac tctcaaggat cttaccgctg ttgagatcca gttcgatgta acccactcgt 5280

gcacccaact gatcttcagc atcttttact ttcaccagcg tttctgggtg agcaaaaaca 5340

ggaaggcaaa atgccgcaaa aaagggaata agggcgacac ggaaatgttg aatactcata 5400

ctcttccttt ttcaatatta ttgaagcatt tatcagggtt attgtctcat gagcggatac 5460

atatttgaat gtatttagaa aaataaacaa ataggggttc cgcgcacatt tccccgaaaa 5520

gtgccacctg acgtctaaga aaccattatt atcatgacat taacctataa aaataggcgt 5580

atcacgaggc cctttcgtct cgcgcgtttc ggtgatgacg gtgaaaacct ctgacacatg 5640

cagctcccgg agacggtcac agcttgtctg taagcggatg ccgggagcag acaagcccgt 5700

cagggcgcgt cagcgggtgt tcatgtgcgt aactaacttg ccatcttcaa acaggagggc 5760

tggaagaagc agaccgctaa cacagtacat aaaaaaggag acatgaacga tgaacatcaa 5820

aaagtttgca aaacaagcaa cagtattaac ctttactacc gcactgctgg caggaggcgc 5880

aactcaagcg tttgcgaaag aaacgaacca aaagccatat aaggaaacat acggcatttc 5940

ccatattaca cgccatgata tgctgcaaat ccctgaacag caaaaaaatg aaaaatatca 6000

agttcctgaa ttcgattcgt ccacaattaa aaatatctct tctgcaaaag gcctggacgt 6060

ttgggacagc tggccattac aaaacgctga cggcactgtc gcaaactatc acggctacca 6120

catcgtcttt gcattagccg gagatcctaa aaatgcggat gacacatcga tttacatgtt 6180

ctatcaaaaa gtcggcgaaa cttctattga cagctggaaa aacgctggcc gcgtctttaa 6240

agacagcgac aaattcgatg caaatgattc tatcctaaaa gaccaaacac aagaatggtc 6300

aggttcagcc acatttacat ctgacggaaa aatccgttta ttctacactg atttctccgg 6360

taaacattac ggcaaacaaa cactgacaac tgcacaagtt aacgtatcag catcagacag 6420

ctctttgaac atcaacggtg tagaggatta taaatcaatc tttgacggtg acggaaaaac 6480

gtatcaaaat gtacagcatg ccacgcgtc 6509

<210> 178
<211> 571
<212> PRT
<213> Bacillus subtilis

<400> 178

Met Leu Thr Lys Ala Thr Lys Glu Gln Lys Ser Leu Val Lys Asn Arg
1               5                   10                  15

Gly Ala Glu Leu Val Val Asp Cys Leu Val Glu Gln Gly Val Thr His
            20                  25                  30

Val Phe Gly Ile Pro Gly Ala Lys Ile Asp Ala Val Phe Asp Ala Leu
        35                  40                  45

Gln Asp Lys Gly Pro Glu Ile Ile Val Ala Arg His Glu Gln Asn Ala
    50                  55                  60

Ala Phe Met Ala Gln Ala Val Gly Arg Leu Thr Gly Lys Pro Gly Val
65                  70                  75                  80

Val Leu Val Thr Ser Gly Pro Gly Ala Ser Asn Leu Ala Thr Gly Leu
                85                  90                  95

Leu Thr Ala Asn Thr Glu Gly Asp Pro Val Val Ala Leu Ala Gly Asn
            100                 105                 110

Val Ile Arg Ala Asp Arg Leu Lys Arg Thr His Gln Ser Leu Asp Asn
        115                 120                 125

Ala Ala Leu Phe Gln Pro Ile Thr Lys Tyr Ser Val Glu Val Gln Asp
    130                 135                 140

Val Lys Asn Ile Pro Glu Ala Val Thr Asn Ala Phe Arg Ile Ala Ser
145                 150                 155                 160

Ala Gly Gln Ala Gly Ala Ala Phe Val Ser Phe Pro Gln Asp Val Val
                165                 170                 175

Asn Glu Val Thr Asn Thr Lys Asn Val Arg Ala Val Ala Ala Pro Lys
            180                 185                 190

Leu Gly Pro Ala Ala Asp Asp Ala Ile Ser Ala Ala Ile Ala Lys Ile
        195                 200                 205

Gln Thr Ala Lys Leu Pro Val Val Leu Val Gly Met Lys Gly Gly Arg
    210                 215                 220

Pro Glu Ala Ile Lys Ala Val Arg Lys Leu Leu Lys Lys Val Gln Leu
225                 230                 235                 240

Pro Phe Val Glu Thr Tyr Gln Ala Ala Gly Thr Leu Ser Arg Asp Leu
                245                 250                 255

Glu Asp Gln Tyr Phe Gly Arg Ile Gly Leu Phe Arg Asn Gln Pro Gly

260                 265                 270

Asp Leu Leu Leu Glu Gln Ala Asp Val Val Leu Thr Ile Gly Tyr Asp
        275                 280                 285

Pro Ile Glu Tyr Asp Pro Lys Phe Trp Asn Ile Asn Gly Asp Arg Thr
    290                 295                 300

Ile Ile His Leu Asp Glu Ile Ile Ala Asp Ile Asp His Ala Tyr Gln
305                 310                 315                 320

Pro Asp Leu Glu Leu Ile Gly Asp Ile Pro Ser Thr Ile Asn His Ile
                325                 330                 335

Glu His Asp Ala Val Lys Val Glu Phe Ala Glu Arg Glu Gln Lys Ile
            340                 345                 350

Leu Ser Asp Leu Lys Gln Tyr Met His Glu Gly Glu Gln Val Pro Ala
        355                 360                 365

Asp Trp Lys Ser Asp Arg Ala His Pro Leu Glu Ile Val Lys Glu Leu
    370                 375                 380

Arg Asn Ala Val Asp Asp His Val Thr Val Thr Cys Asp Ile Gly Ser
385                 390                 395                 400

His Ala Ile Trp Met Ser Arg Tyr Phe Arg Ser Tyr Glu Pro Leu Thr
                405                 410                 415

Leu Met Ile Ser Asn Gly Met Gln Thr Leu Gly Val Ala Leu Pro Trp
            420                 425                 430

Ala Ile Gly Ala Ser Leu Val Lys Pro Gly Glu Lys Val Val Ser Val
        435                 440                 445

Ser Gly Asp Gly Gly Phe Leu Phe Ser Ala Met Glu Leu Glu Thr Ala
    450                 455                 460

Val Arg Leu Lys Ala Pro Ile Val His Ile Val Trp Asn Asp Ser Thr
465                 470                 475                 480

Tyr Asp Met Val Ala Phe Gln Gln Leu Lys Lys Tyr Asn Arg Thr Ser
                485                 490                 495

Ala Val Asp Phe Gly Asn Ile Asp Ile Val Lys Tyr Ala Glu Ser Phe
            500                 505                 510

Gly Ala Thr Gly Leu Arg Val Glu Ser Pro Asp Gln Leu Ala Asp Val
        515                 520                 525

Leu Arg Gln Gly Met Asn Ala Glu Gly Pro Val Ile Ile Asp Val Pro
    530                 535                 540

Val Asp Tyr Ser Asp Asn Ile Asn Leu Ala Ser Asp Lys Leu Pro Lys
545                 550                 555                 560

Glu Phe Gly Glu Leu Met Lys Thr Lys Ala Leu
                565                 570

<210>  179
<211>  1665
<212>  DNA
<213>  Lactococcus lactis

<400>  179
atgtctgaga aacaatttgg ggcgaacttg gttgtcgata gtttgattaa ccataaagtg      60

aagtatgtat ttgggattcc aggagcaaaa attgaccggg tttttgattt attagaaaat     120

gaagaaggcc ctcaaatggt cgtgactcgt catgagcaag gagctgcttt catggctcaa     180

gctgtcggtc gtttaactgg cgaacctggt gtagtagttg ttacgagtgg gcctggtgta     240

tcaaaccttg cgactccgct tttgaccgcg acatcagaag gtgatgctat tttggctatc     300

ggtggacaag ttaaacgaag tgaccgtctt aaacgtgcgc accaatcaat ggataatgct     360

ggaatgatgc aatcagcaac aaaatattca gcagaagttc ttgaccctaa tacactttct     420

gaatcaattg ccaacgctta tcgtattgca aaatcaggac atccaggtgc aactttctta     480

tcaatccccc aagatgtaac ggatgccgaa gtatcaatca aagccattca accactttca     540

gaccctaaaa tggggaatgc ctctattgat gacattaatt atttagcaca agcaattaaa     600

aatgctgtat tgccagtaat tttggttgga gctggtgctt cagatgctaa agtcgcttca     660

tccttgcgta atctattgac tcatgttaat attcctgtcg ttgaaacatt ccaaggtgca     720

ggggttattt cacatgattt agaacatact ttttatggac gtatcggtct tttccgcaat     780

caaccaggcg atatgcttct gaaacgttct gaccttgtta ttgctgttgg ttatgaccca     840

attgaatatg aagctcgtaa ctggaatgca gaaattgata gtcgaattat cgttattgat     900

aatgccattg ctgaaattga tacttactac caaccagagc gtgaattaat tggtgatatc     960

gcagcaacat tggataatct tttaccagct gttcgtggct acaaaattcc aaaaggaaca    1020

aaagattatc tcgatggcct tcatgaagtt gctgagcaac acgaatttga tactgaaaat    1080

actgaagaag gtagaatgca ccctcttgat ttggtcagca ctttccaaga aatcgtcaag    1140

gatgatgaaa cagtaaccgt tgacgtaggt tcactctaca tttggatggc acgtcatttc    1200

aaatcatacg aaccacgtca tctcctcttc tcaaacggaa tgcaaacact cggagttgca    1260

cttccttggg caattacagc cgcattgttg cgcccaggta aaaaagttta ttcacactct    1320

ggtgatggag gcttcctttt cacagggcaa gaattggaaa cagctgtacg tttgaatctt    1380

ccaatcgttc aaattatctg gaatgacggc cattatgata tggttaaatt ccaagaagaa 1440

atgaaatatg gtcgttcagc agccgttgat tttggctatg ttgattacgt aaaatatgct 1500

gaagcaatga gagcaaaagg ttaccgtgca cacagcaaag aagaacttgc tgaaattctc 1560

aaatcaatcc cagatactac tggaccggtg gtaattgacg ttcctttgga ctattctgat 1620

aacattaaat tagcagaaaa attattgcct gaagagtttt attga 1665

<210> 180
<211> 554
<212> PRT
<213> Lactococcus lactis

<400> 180

Met Ser Glu Lys Gln Phe Gly Ala Asn Leu Val Val Asp Ser Leu Ile
1               5                   10                  15

Asn His Lys Val Lys Tyr Val Phe Gly Ile Pro Gly Ala Lys Ile Asp
            20                  25                  30

Arg Val Phe Asp Leu Leu Glu Asn Glu Glu Gly Pro Gln Met Val Val
        35                  40                  45

Thr Arg His Glu Gln Gly Ala Ala Phe Met Ala Gln Ala Val Gly Arg
    50                  55                  60

Leu Thr Gly Glu Pro Gly Val Val Val Val Thr Ser Gly Pro Gly Val
65                  70                  75                  80

Ser Asn Leu Ala Thr Pro Leu Leu Thr Ala Thr Ser Glu Gly Asp Ala
                85                  90                  95

Ile Leu Ala Ile Gly Gly Gln Val Lys Arg Ser Asp Arg Leu Lys Arg
            100                 105                 110

Ala His Gln Ser Met Asp Asn Ala Gly Met Met Gln Ser Ala Thr Lys
        115                 120                 125

Tyr Ser Ala Glu Val Leu Asp Pro Asn Thr Leu Ser Glu Ser Ile Ala
    130                 135                 140

Asn Ala Tyr Arg Ile Ala Lys Ser Gly His Pro Gly Ala Thr Phe Leu
145                 150                 155                 160

Ser Ile Pro Gln Asp Val Thr Asp Ala Glu Val Ser Ile Lys Ala Ile
                165                 170                 175

Gln Pro Leu Ser Asp Pro Lys Met Gly Asn Ala Ser Ile Asp Asp Ile
            180                 185                 190

Asn Tyr Leu Ala Gln Ala Ile Lys Asn Ala Val Leu Pro Val Ile Leu
        195                 200                 205

Val Gly Ala Gly Ala Ser Asp Ala Lys Val Ala Ser Ser Leu Arg Asn
    210                 215                 220

Leu Leu Thr His Val Asn Ile Pro Val Val Glu Thr Phe Gln Gly Ala
225                 230                 235                 240

Gly Val Ile Ser His Asp Leu Glu His Thr Phe Tyr Gly Arg Ile Gly
                245                 250                 255

Leu Phe Arg Asn Gln Pro Gly Asp Met Leu Leu Lys Arg Ser Asp Leu
            260                 265                 270

Val Ile Ala Val Gly Tyr Asp Pro Ile Glu Tyr Glu Ala Arg Asn Trp
        275                 280                 285

Asn Ala Glu Ile Asp Ser Arg Ile Ile Val Ile Asp Asn Ala Ile Ala
    290                 295                 300

Glu Ile Asp Thr Tyr Tyr Gln Pro Glu Arg Glu Leu Ile Gly Asp Ile
305                 310                 315                 320

Ala Ala Thr Leu Asp Asn Leu Leu Pro Ala Val Arg Gly Tyr Lys Ile
                325                 330                 335

Pro Lys Gly Thr Lys Asp Tyr Leu Asp Gly Leu His Glu Val Ala Glu
            340                 345                 350

Gln His Glu Phe Asp Thr Glu Asn Thr Glu Glu Gly Arg Met His Pro
        355                 360                 365

Leu Asp Leu Val Ser Thr Phe Gln Glu Ile Val Lys Asp Asp Glu Thr
    370                 375                 380

Val Thr Val Asp Val Gly Ser Leu Tyr Ile Trp Met Ala Arg His Phe
385                 390                 395                 400

Lys Ser Tyr Glu Pro Arg His Leu Leu Phe Ser Asn Gly Met Gln Thr
                405                 410                 415

Leu Gly Val Ala Leu Pro Trp Ala Ile Thr Ala Ala Leu Leu Arg Pro
            420                 425                 430

Gly Lys Lys Val Tyr Ser His Ser Gly Asp Gly Gly Phe Leu Phe Thr
        435                 440                 445

Gly Gln Glu Leu Glu Thr Ala Val Arg Leu Asn Leu Pro Ile Val Gln
    450                 455                 460

Ile Ile Trp Asn Asp Gly His Tyr Asp Met Val Lys Phe Gln Glu Glu
465                 470                 475                 480

Met Lys Tyr Gly Arg Ser Ala Ala Val Asp Phe Gly Tyr Val Asp Tyr
                485                 490                 495

Val Lys Tyr Ala Glu Ala Met Arg Ala Lys Gly Tyr Arg Ala His Ser
            500                 505                 510

Lys Glu Glu Leu Ala Glu Ile Leu Lys Ser Ile Pro Asp Thr Thr Gly
        515                 520                 525

Pro Val Val Ile Asp Val Pro Leu Asp Tyr Ser Asp Asn Ile Lys Leu
    530                 535                 540

Ala Glu Lys Leu Leu Pro Glu Glu Phe Tyr
545                 550

<210>  181
<211>  395
<212>  PRT
<213>  Saccharomyces cerevisiae

<400>  181

Met Leu Arg Thr Gln Ala Ala Arg Leu Ile Cys Asn Ser Arg Val Ile
1               5                   10                  15

Thr Ala Lys Arg Thr Phe Ala Leu Ala Thr Arg Ala Ala Ala Tyr Ser
            20                  25                  30

Arg Pro Ala Ala Arg Phe Val Lys Pro Met Ile Thr Thr Arg Gly Leu
        35                  40                  45

Lys Gln Ile Asn Phe Gly Gly Thr Val Glu Thr Val Tyr Glu Arg Ala
    50                  55                  60

Asp Trp Pro Arg Glu Lys Leu Leu Asp Tyr Phe Lys Asn Asp Thr Phe
65                  70                  75                  80

Ala Leu Ile Gly Tyr Gly Ser Gln Gly Tyr Gly Gln Gly Leu Asn Leu
                85                  90                  95

Arg Asp Asn Gly Leu Asn Val Ile Ile Gly Val Arg Lys Asp Gly Ala
            100                 105                 110

Ser Trp Lys Ala Ala Ile Glu Asp Gly Trp Val Pro Gly Lys Asn Leu

115                 120                 125

Phe Thr Val Glu Asp Ala Ile Lys Arg Gly Ser Tyr Val Met Asn Leu
    130                 135                 140

Leu Ser Asp Ala Ala Gln Ser Glu Thr Trp Pro Ala Ile Lys Pro Leu
145                 150                 155                 160

Leu Thr Lys Gly Lys Thr Leu Tyr Phe Ser His Gly Phe Ser Pro Val
                165                 170                 175

Phe Lys Asp Leu Thr His Val Glu Pro Pro Lys Asp Leu Asp Val Ile
            180                 185                 190

Leu Val Ala Pro Lys Gly Ser Gly Arg Thr Val Arg Ser Leu Phe Lys
        195                 200                 205

Glu Gly Arg Gly Ile Asn Ser Ser Tyr Ala Val Trp Asn Asp Val Thr
    210                 215                 220

Gly Lys Ala His Glu Lys Ala Gln Ala Leu Ala Val Ala Ile Gly Ser
225                 230                 235                 240

Gly Tyr Val Tyr Gln Thr Thr Phe Glu Arg Glu Val Asn Ser Asp Leu
                245                 250                 255

Tyr Gly Glu Arg Gly Cys Leu Met Gly Gly Ile His Gly Met Phe Leu
            260                 265                 270

Ala Gln Tyr Asp Val Leu Arg Glu Asn Gly His Ser Pro Ser Glu Ala
        275                 280                 285

Phe Asn Glu Thr Val Glu Glu Ala Thr Gln Ser Leu Tyr Pro Leu Ile
    290                 295                 300

Gly Lys Tyr Gly Met Asp Tyr Met Tyr Asp Ala Cys Ser Thr Thr Ala
305                 310                 315                 320

Arg Arg Gly Ala Leu Asp Trp Tyr Pro Ile Phe Lys Asn Ala Leu Lys
                325                 330                 335

Pro Val Phe Gln Asp Leu Tyr Glu Ser Thr Lys Asn Gly Thr Glu Thr
            340                 345                 350

Lys Arg Ser Leu Glu Phe Asn Ser Gln Pro Asp Tyr Arg Glu Lys Leu
        355                 360                 365

Glu Lys Glu Leu Asp Thr Ile Arg Asn Met Glu Ile Trp Lys Val Gly
    370                 375                 380

Lys Glu Val Arg Lys Leu Arg Pro Glu Asn Gln
385                 390                 395

<210>  182
<211>  993
<212>  DNA
<213>  Methanococcus maripaludis

<400>  182
atgaaggtat tctatgactc agattttaaa ttagatgctt taaaagaaaa aacaattgca      60

gtaatcggtt atggaagtca aggtagggca cagtccttaa acatgaaaga cagcggatta     120

aacgttgttg ttggtttaag aaaaaacggt gcttcatgga acaacgctaa agcagacggt     180

cacaatgtaa tgaccattga agaagctgct gaaaaagcgg acatcatcca catcttaata     240

cctgatgaat tacaggcaga agtttatgaa agccagataa aaccatacct aaaagaagga     300

aaaacactaa gcttttcaca tggttttaac atccactatg gattcattgt tccaccaaaa     360

ggagttaacg tggttttagt tgctccaaaa tcacctggaa aaatggttag aagaacatac     420

gaagaaggtt tcggtgttcc aggtttaatc tgtattgaaa ttgatgcaac aaacaacgca     480

tttgatattg tttcagcaat ggcaaaagga atcggtttat caagagctgg agttatccag     540

acaactttca aagaagaaac agaaactgac cttttcggtg aacaagctgt tttatgcggt     600

ggagttaccg aattaatcaa ggcaggattt gaaacactcg ttgaagcagg atacgcacca     660

gaaatggcat actttgaaac ctgccacgaa ttgaaattaa tcgttgactt aatctaccaa     720

aaaggattca aaaacatgtg gaacgatgta agtaacactg cagaatacgg cggacttaca     780

agaagaagca gaatcgttac agctgattca aaagctgcaa tgaaagaaat cttaagagaa     840

atccaagatg gaagattcac aaaagaattc cttctcgaaa aacaggtaag ctatgctcat     900

ttaaaatcaa tgagaagact cgaaggagac ttacaaatcg aagaagtcgg cgcaaaatta     960

agaaaaatgt gcggtcttga aaaagaagaa taa                                 993

<210>  183
<211>  330
<212>  PRT
<213>  Methanococcus maripaludis

<400>  183

Met Lys Val Phe Tyr Asp Ser Asp Phe Lys Leu Asp Ala Leu Lys Glu
1               5                   10                  15

Lys Thr Ile Ala Val Ile Gly Tyr Gly Ser Gln Gly Arg Ala Gln Ser
            20                  25                  30

Leu Asn Met Lys Asp Ser Gly Leu Asn Val Val Val Gly Leu Arg Lys
        35                  40                  45

Asn Gly Ala Ser Trp Asn Asn Ala Lys Ala Asp Gly His Asn Val Met
    50                  55                  60

Thr Ile Glu Glu Ala Ala Glu Lys Ala Asp Ile Ile His Ile Leu Ile
65                  70                  75                  80

Pro Asp Glu Leu Gln Ala Glu Val Tyr Glu Ser Gln Ile Lys Pro Tyr
                85                  90                  95

Leu Lys Glu Gly Lys Thr Leu Ser Phe Ser His Gly Phe Asn Ile His
            100                 105                 110

Tyr Gly Phe Ile Val Pro Pro Lys Gly Val Asn Val Val Leu Val Ala
        115                 120                 125

Pro Lys Ser Pro Gly Lys Met Val Arg Arg Thr Tyr Glu Glu Gly Phe
    130                 135                 140

Gly Val Pro Gly Leu Ile Cys Ile Glu Ile Asp Ala Thr Asn Asn Ala
145                 150                 155                 160

Phe Asp Ile Val Ser Ala Met Ala Lys Gly Ile Gly Leu Ser Arg Ala
                165                 170                 175

Gly Val Ile Gln Thr Thr Phe Lys Glu Glu Thr Glu Thr Asp Leu Phe
            180                 185                 190

Gly Glu Gln Ala Val Leu Cys Gly Gly Val Thr Glu Leu Ile Lys Ala
        195                 200                 205

Gly Phe Glu Thr Leu Val Glu Ala Gly Tyr Ala Pro Glu Met Ala Tyr
    210                 215                 220

Phe Glu Thr Cys His Glu Leu Lys Leu Ile Val Asp Leu Ile Tyr Gln
225                 230                 235                 240

Lys Gly Phe Lys Asn Met Trp Asn Asp Val Ser Asn Thr Ala Glu Tyr
                245                 250                 255

Gly Gly Leu Thr Arg Arg Ser Arg Ile Val Thr Ala Asp Ser Lys Ala
            260                 265                 270

Ala Met Lys Glu Ile Leu Arg Glu Ile Gln Asp Gly Arg Phe Thr Lys
        275                 280                 285

Glu Phe Leu Leu Glu Lys Gln Val Ser Tyr Ala His Leu Lys Ser Met
    290                 295                 300

Arg Arg Leu Glu Gly Asp Leu Gln Ile Glu Glu Val Gly Ala Lys Leu
305                 310                 315                 320

Arg Lys Met Cys Gly Leu Glu Lys Glu Glu
                325                 330

<210>  184
<211>  1476
<212>  DNA
<213>  Bacillus subtilis

<400>  184
atggctaact acttcaatac actgaatctg cgccagcagc tggcacagct gggcaaatgt      60

cgctttatgg gccgcgatga attcgccgat ggcgcgagct accttcaggg taaaaaagta     120

gtcatcgtcg gctgtggcgc acagggtctg aaccagggcc tgaacatgcg tgattctggt     180

ctcgatatct cctacgctct gcgtaaagaa gcgattgccg agaagcgcgc gtcctggcgt     240

aaagcgaccg aaaatggttt taaagtgggt acttacgaag aactgatccc acaggcggat     300

ctggtgatta acctgacgcc ggacaagcag cactctgatg tagtgcgcac cgtacagcca     360

ctgatgaaag acggcgcggc gctgggctac tcgcacggtt tcaacatcgt cgaagtgggc     420

gagcagatcc gtaaagatat caccgtagtg atggttgcgc cgaaatgccc aggcaccgaa     480

gtgcgtgaag agtacaaacg tgggttcggc gtaccgacgc tgattgccgt tcacccggaa     540

aacgatccga aaggcgaagg catggcgatt gccaaagcct gggcggctgc aaccggtggt     600

caccgtgcgg gtgtgctgga atcgtccttc gttgcggaag tgaaatctga cctgatgggc     660

gagcaaacca tcctgtgcgg tatgttgcag gctggctctc tgctgtgctt cgacaagctg     720

gtggaagaag gtaccgatcc agcatacgca gaaaaactga ttcagttcgg ttgggaaacc     780

atcaccgaag cactgaaaca gggcggcatc accctgatga tggaccgtct ctctaacccg     840

gcgaaactgc gtgcttatgc gctttctgaa cagctgaaag agatcatggc acccctgttc     900

cagaaacata tggacgacat catctccggc gaattctctt ccggtatgat ggcggactgg     960

gccaacgatg ataagaaact gctgacctgg cgtgaagaga ccggcaaaac cgcgtttgaa    1020

accgcgccgc agtatgaagg caaaatcggc gagcaggagt acttcgataa aggcgtactg    1080

atgattgcga tggtgaaagc gggcgttgaa ctggcgttcg aaaccatggt cgattccggc    1140

atcattgaag agtctgcata ttatgaatca ctgcacgagc tgccgctgat tgccaacacc    1200

atcgcccgta agcgtctgta cgaaatgaac gtggttatct ctgataccgc tgagtacggt    1260

aactatctgt tctcttacgc ttgtgtgccg ttgctgaaac cgtttatggc agagctgcaa    1320

ccgggcgacc tgggtaaagc tattccggaa ggcgcggtag ataacggcca actgcgtgat    1380

gtgaacgaag cgattcgcag ccatgcgatt gagcaggtag gtaagaaact gcgcggctat    1440

atgacagata tgaaacgtat tgctgttgcg ggttaa                              1476

<210>  185
<211>  342
<212>  PRT
<213>  Bacillus subtilis

<400>  185

Met Val Lys Val Tyr Tyr Asn Gly Asp Ile Lys Glu Asn Val Leu Ala
1               5                   10                  15

Gly Lys Thr Val Ala Val Ile Gly Tyr Gly Ser Gln Gly His Ala His
            20                  25                  30

Ala Leu Asn Leu Lys Glu Ser Gly Val Asp Val Ile Val Gly Val Arg
        35                  40                  45

Gln Gly Lys Ser Phe Thr Gln Ala Gln Glu Asp Gly His Lys Val Phe
    50                  55                  60

Ser Val Lys Glu Ala Ala Ala Gln Ala Glu Ile Ile Met Val Leu Leu
65                  70                  75                  80

Pro Asp Glu Gln Gln Gln Lys Val Tyr Glu Ala Glu Ile Lys Asp Glu
                85                  90                  95

Leu Thr Ala Gly Lys Ser Leu Val Phe Ala His Gly Phe Asn Val His
            100                 105                 110

Phe His Gln Ile Val Pro Pro Ala Asp Val Asp Val Phe Leu Val Ala
        115                 120                 125

Pro Lys Gly Pro Gly His Leu Val Arg Arg Thr Tyr Glu Gln Gly Ala
    130                 135                 140

Gly Val Pro Ala Leu Phe Ala Ile Tyr Gln Asp Val Thr Gly Glu Ala
145                 150                 155                 160

Arg Asp Lys Ala Leu Ala Tyr Ala Lys Gly Ile Gly Gly Ala Arg Ala
                165                 170                 175

Gly Val Leu Glu Thr Thr Phe Lys Glu Glu Thr Glu Thr Asp Leu Phe
            180                 185                 190

Gly Glu Gln Ala Val Leu Cys Gly Gly Leu Ser Ala Leu Val Lys Ala
        195                 200                 205

Gly Phe Glu Thr Leu Thr Glu Ala Gly Tyr Gln Pro Glu Leu Ala Tyr
    210                 215                 220

Phe Glu Cys Leu His Glu Leu Lys Leu Ile Val Asp Leu Met Tyr Glu
225                 230                 235                 240

Glu Gly Leu Ala Gly Met Arg Tyr Ser Ile Ser Asp Thr Ala Gln Trp
                245                 250                 255

Gly Asp Phe Val Ser Gly Pro Arg Val Val Asp Ala Lys Val Lys Glu
            260                 265                 270

Ser Met Lys Glu Val Leu Lys Asp Ile Gln Asn Gly Thr Phe Ala Lys
        275                 280                 285

Glu Trp Ile Val Glu Asn Gln Val Asn Arg Pro Arg Phe Asn Ala Ile
    290                 295                 300

Asn Ala Ser Glu Asn Glu His Gln Ile Glu Val Val Gly Arg Lys Leu
305                 310                 315                 320

Arg Glu Met Met Pro Phe Val Lys Gln Gly Lys Lys Lys Glu Ala Val
                325                 330                 335

Val Ser Val Ala Gln Asn
            340

<210>  186
<211>  585
<212>  PRT
<213>  Saccharomyces cerevisiae

<400>  186

Met Gly Leu Leu Thr Lys Val Ala Thr Ser Arg Gln Phe Ser Thr Thr
1               5                   10                  15

Arg Cys Val Ala Lys Lys Leu Asn Lys Tyr Ser Tyr Ile Ile Thr Glu
            20                  25                  30

Pro Lys Gly Gln Gly Ala Ser Gln Ala Met Leu Tyr Ala Thr Gly Phe
        35                  40                  45

Lys Lys Glu Asp Phe Lys Lys Pro Gln Val Gly Val Gly Ser Cys Trp
    50                  55                  60

Trp Ser Gly Asn Pro Cys Asn Met His Leu Leu Asp Leu Asn Asn Arg
65                  70                  75                  80

Cys Ser Gln Ser Ile Glu Lys Ala Gly Leu Lys Ala Met Gln Phe Asn
                85                  90                  95

Thr Ile Gly Val Ser Asp Gly Ile Ser Met Gly Thr Lys Gly Met Arg
            100                 105                 110

Tyr Ser Leu Gln Ser Arg Glu Ile Ile Ala Asp Ser Phe Glu Thr Ile
        115                 120                 125

Met Met Ala Gln His Tyr Asp Ala Asn Ile Ala Ile Pro Ser Cys Asp
    130                 135                 140

Lys Asn Met Pro Gly Val Met Met Ala Met Gly Arg His Asn Arg Pro
145                 150                 155                 160

Ser Ile Met Val Tyr Gly Gly Thr Ile Leu Pro Gly His Pro Thr Cys
                165                 170                 175

Gly Ser Ser Lys Ile Ser Lys Asn Ile Asp Ile Val Ser Ala Phe Gln
            180                 185                 190

Ser Tyr Gly Glu Tyr Ile Ser Lys Gln Phe Thr Glu Glu Glu Arg Glu
        195                 200                 205

Asp Val Val Glu His Ala Cys Pro Gly Pro Gly Ser Cys Gly Gly Met
    210                 215                 220

Tyr Thr Ala Asn Thr Met Ala Ser Ala Ala Glu Val Leu Gly Leu Thr
225                 230                 235                 240

Ile Pro Asn Ser Ser Ser Phe Pro Ala Val Ser Lys Glu Lys Leu Ala
                245                 250                 255

Glu Cys Asp Asn Ile Gly Glu Tyr Ile Lys Lys Thr Met Glu Leu Gly
            260                 265                 270

Ile Leu Pro Arg Asp Ile Leu Thr Lys Glu Ala Phe Glu Asn Ala Ile
        275                 280                 285

Thr Tyr Val Val Ala Thr Gly Gly Ser Thr Asn Ala Val Leu His Leu
    290                 295                 300

Val Ala Val Ala His Ser Ala Gly Val Lys Leu Ser Pro Asp Asp Phe
305                 310                 315                 320

Gln Arg Ile Ser Asp Thr Thr Pro Leu Ile Gly Asp Phe Lys Pro Ser
                325                 330                 335

Gly Lys Tyr Val Met Ala Asp Leu Ile Asn Val Gly Gly Thr Gln Ser
            340                 345                 350

Val Ile Lys Tyr Leu Tyr Glu Asn Asn Met Leu His Gly Asn Thr Met
        355                 360                 365

Thr Val Thr Gly Asp Thr Leu Ala Glu Arg Ala Lys Lys Ala Pro Ser

370                 375                 380

Leu Pro Glu Gly Gln Glu Ile Ile Lys Pro Leu Ser His Pro Ile Lys
385                 390                 395                 400

Ala Asn Gly His Leu Gln Ile Leu Tyr Gly Ser Leu Ala Pro Gly Gly
                405                 410                 415

Ala Val Gly Lys Ile Thr Gly Lys Glu Gly Thr Tyr Phe Lys Gly Arg
            420                 425                 430

Ala Arg Val Phe Glu Glu Glu Gly Ala Phe Ile Glu Ala Leu Glu Arg
        435                 440                 445

Gly Glu Ile Lys Lys Gly Glu Lys Thr Val Val Val Ile Arg Tyr Glu
    450                 455                 460

Gly Pro Arg Gly Ala Pro Gly Met Pro Glu Met Leu Lys Pro Ser Ser
465                 470                 475                 480

Ala Leu Met Gly Tyr Gly Leu Gly Lys Asp Val Ala Leu Leu Thr Asp
                485                 490                 495

Gly Arg Phe Ser Gly Gly Ser His Gly Phe Leu Ile Gly His Ile Val
            500                 505                 510

Pro Glu Ala Ala Glu Gly Gly Pro Ile Gly Leu Val Arg Asp Gly Asp
        515                 520                 525

Glu Ile Ile Ile Asp Ala Asp Asn Asn Lys Ile Asp Leu Leu Val Ser
    530                 535                 540

Asp Lys Glu Met Ala Gln Arg Lys Gln Ser Trp Val Ala Pro Pro Pro
545                 550                 555                 560

Arg Tyr Thr Arg Gly Thr Leu Ser Lys Tyr Ala Lys Leu Val Ser Asn
                565                 570                 575

Ala Ser Asn Gly Cys Val Leu Asp Ala
            580                 585

<210>  187
<211>  1653
<212>  DNA
<213>  Methanococcus maripaludis

<400>  187
atgataagtg ataacgtcaa aaagggagtt ataagaactc caaaccgagc tcttttaaag      60

gcttgcggat atacagacga agacatggaa aaaccattta ttggaattgt aaacagcttt     120

acagaagttg ttcccggcca cattcactta agaacattat cagaagcggc taaacatggt 180

gtttatgcaa acggtggaac accatttgaa tttaatacca ttggaatttg cgacggtatt 240

gcaatgggcc acgaaggtat gaaatactct ttaccttcaa gagaaattat tgcagacgct 300

gttgaatcaa tggcaagagc acatggattt gatggtcttg ttttaattcc tacgtgtgat 360

aaaatcgttc ctggaatgat aatgggtgct ttaagactaa acattccatt tattgtagtt 420

actggaggac caatgcttcc cggagaattc caaggtaaaa aatacgaact tatcagcctt 480

tttgaaggtg tcggagaata ccaagttgga aaaattactg aagaagagtt aaagtgcatt 540

gaagactgtg catgttcagg tgctggaagt tgtgcagggc tttacactgc aaacagtatg 600

gcctgcctta cagaagcttt gggactctct cttccaatgt gtgcaacaac gcatgcagtt 660

gatgcccaaa aagttaggct tgctaaaaaa agtggctcaa aaattgttga tatggtaaaa 720

gaagacctaa aaccaacaga catattaaca aaagaagctt ttgaaaatgc tattttagtt 780

gaccttgcac ttggtggatc aacaaacaca acattacaca ttcctgcaat tgcaaatgaa 840

attgaaaata aattcataac tctcgatgac tttgacaggt taagcgatga agttccacac 900

attgcatcaa tcaaaccagg tggagaacac tacatgattg atttacacaa tgctggaggt 960

attcctgcgg tattgaacgt tttaaaagaa aaaattagag atacaaaaac agttgatgga 1020

agaagcattt tggaaatcgc agaatctgtt aaatacataa attacgacgt tataagaaaa 1080

gtggaagctc cggttcacga aactgctggt ttaagggttt taaagggaaa tcttgctcca 1140

aacggttgcg ttgtaaaaat cggtgcagta catccgaaaa tgtacaaaca cgatggacct 1200

gcaaaagttt acaattccga agatgaagca atttctgcga tacttggcgg aaaaattgta 1260

gaaggggacg ttatagtaat cagatacgaa ggaccatcag gaggccctgg aatgagagaa 1320

atgctctccc caacttcagc aatctgtgga atgggtcttg atgacagcgt tgcattgatt 1380

actgatggaa gattcagtgg tggaagtagg ggcccatgta tcggacacgt ttctccagaa 1440

gctgcagctg gcggagtaat tgctgcaatt gaaaacgggg atatcatcaa aatcgacatg 1500

attgaaaaag aaataaatgt tgatttagat gaatcagtca ttaaagaaag actctcaaaa 1560

ctgggagaat ttgagcctaa aatcaaaaaa ggctatttat caagatactc aaaacttgtc 1620

tcatctgctg acgaaggggc agttttaaaa taa 1653

<210> 188
<211> 550
<212> PRT
<213> Methanococcus maripaludis

<400> 188

Met Ile Ser Asp Asn Val Lys Lys Gly Val Ile Arg Thr Pro Asn Arg
1               5                   10                  15

Ala Leu Leu Lys Ala Cys Gly Tyr Thr Asp Glu Asp Met Glu Lys Pro

20                  25                  30

Phe Ile Gly Ile Val Asn Ser Phe Thr Glu Val Val Pro Gly His Ile
        35                  40                  45

His Leu Arg Thr Leu Ser Glu Ala Ala Lys His Gly Val Tyr Ala Asn
    50                  55                  60

Gly Gly Thr Pro Phe Glu Phe Asn Thr Ile Gly Ile Cys Asp Gly Ile
65                  70                  75                  80

Ala Met Gly His Glu Gly Met Lys Tyr Ser Leu Pro Ser Arg Glu Ile
                85                  90                  95

Ile Ala Asp Ala Val Glu Ser Met Ala Arg Ala His Gly Phe Asp Gly
            100                 105                 110

Leu Val Leu Ile Pro Thr Cys Asp Lys Ile Val Pro Gly Met Ile Met
        115                 120                 125

Gly Ala Leu Arg Leu Asn Ile Pro Phe Ile Val Val Thr Gly Gly Pro
    130                 135                 140

Met Leu Pro Gly Glu Phe Gln Gly Lys Lys Tyr Glu Leu Ile Ser Leu
145                 150                 155                 160

Phe Glu Gly Val Gly Glu Tyr Gln Val Gly Lys Ile Thr Glu Glu Glu
                165                 170                 175

Leu Lys Cys Ile Glu Asp Cys Ala Cys Ser Gly Ala Gly Ser Cys Ala
            180                 185                 190

Gly Leu Tyr Thr Ala Asn Ser Met Ala Cys Leu Thr Glu Ala Leu Gly
        195                 200                 205

Leu Ser Leu Pro Met Cys Ala Thr Thr His Ala Val Asp Ala Gln Lys
    210                 215                 220

Val Arg Leu Ala Lys Lys Ser Gly Ser Lys Ile Val Asp Met Val Lys
225                 230                 235                 240

Glu Asp Leu Lys Pro Thr Asp Ile Leu Thr Lys Glu Ala Phe Glu Asn
                245                 250                 255

Ala Ile Leu Val Asp Leu Ala Leu Gly Gly Ser Thr Asn Thr Thr Leu
            260                 265                 270

His Ile Pro Ala Ile Ala Asn Glu Ile Glu Asn Lys Phe Ile Thr Leu
        275                 280                 285

Asp Asp Phe Asp Arg Leu Ser Asp Glu Val Pro His Ile Ala Ser Ile
    290                 295                 300

Lys Pro Gly Gly Glu His Tyr Met Ile Asp Leu His Asn Ala Gly Gly
305                 310                 315                 320

Ile Pro Ala Val Leu Asn Val Leu Lys Glu Lys Ile Arg Asp Thr Lys
                325                 330                 335

Thr Val Asp Gly Arg Ser Ile Leu Glu Ile Ala Glu Ser Val Lys Tyr
            340                 345                 350

Ile Asn Tyr Asp Val Ile Arg Lys Val Glu Ala Pro Val His Glu Thr
        355                 360                 365

Ala Gly Leu Arg Val Leu Lys Gly Asn Leu Ala Pro Asn Gly Cys Val
    370                 375                 380

Val Lys Ile Gly Ala Val His Pro Lys Met Tyr Lys His Asp Gly Pro
385                 390                 395                 400

Ala Lys Val Tyr Asn Ser Glu Asp Glu Ala Ile Ser Ala Ile Leu Gly
                405                 410                 415

Gly Lys Ile Val Glu Gly Asp Val Ile Val Ile Arg Tyr Glu Gly Pro
            420                 425                 430

Ser Gly Gly Pro Gly Met Arg Glu Met Leu Ser Pro Thr Ser Ala Ile
        435                 440                 445

Cys Gly Met Gly Leu Asp Asp Ser Val Ala Leu Ile Thr Asp Gly Arg
    450                 455                 460

Phe Ser Gly Gly Ser Arg Gly Pro Cys Ile Gly His Val Ser Pro Glu
465                 470                 475                 480

Ala Ala Ala Gly Gly Val Ile Ala Ala Ile Glu Asn Gly Asp Ile Ile
                485                 490                 495

Lys Ile Asp Met Ile Glu Lys Glu Ile Asn Val Asp Leu Asp Glu Ser
            500                 505                 510

Val Ile Lys Glu Arg Leu Ser Lys Leu Gly Glu Phe Glu Pro Lys Ile
        515                 520                 525

Lys Lys Gly Tyr Leu Ser Arg Tyr Ser Lys Leu Val Ser Ser Ala Asp
    530                 535                 540

Glu Gly Ala Val Leu Lys
545                 550

<210>  189
<211>  1677
<212>  DNA
<213>  Bacillus subtilis

<400>  189
atggcagaat tacgcagtaa tatgatcaca caaggaatcg atagagctcc gcaccgcagt      60

ttgcttcgtg cagcaggggt aaaagaagag gatttcggca agccgtttat tgcggtgtgt     120

aattcataca ttgatatcgt tcccggtcat gttcacttgc aggagtttgg gaaaatcgta     180

aaagaagcaa tcagagaagc agggggcgtt ccgtttgaat ttaataccat tggggtagat     240

gatggcatcg caatggggca tatcggtatg agatattcgc tgccaagccg tgaaattatc     300

gcagactctg tggaaacggt tgtatccgca cactggtttg acggaatggt ctgtattccg     360

aactgcgaca aaatcacacc gggaatgctt atggcggcaa tgcgcatcaa cattccgacg     420

atttttgtca gcggcggacc gatggcggca ggaagaacaa gttacgggcg aaaaatctcc     480

ctttcctcag tattcgaagg ggtaggcgcc taccaagcag ggaaaatcaa cgaaaacgag     540

cttcaagaac tagagcagtt cggatgccca acgtgcgggt cttgctcagg catgtttacg     600

gcgaactcaa tgaactgtct gtcagaagca cttggtcttg ctttgccggg taatggaacc     660

attctggcaa catctccgga acgcaaagag tttgtgagaa aatcggctgc gcaattaatg     720

gaaacgattc gcaaagatat caaaccgcgt gatattgtta cagtaaaagc gattgataac     780

gcgtttgcac tcgatatggc gctcggaggt tctacaaata ccgttcttca tacccttgcc     840

cttgcaaacg aagccggcgt tgaatactct ttagaacgca ttaacgaagt cgctgagcgc     900

gtgccgcact tggctaagct ggcgcctgca tcggatgtgt ttattgaaga tcttcacgaa     960

gcgggcggcg tttcagcggc tctgaatgag ctttcgaaga aagaaggagc gcttcattta    1020

gatgcgctga ctgttacagg aaaaactctt ggagaaacca ttgccggaca tgaagtaaag    1080

gattatgacg tcattcaccc gctggatcaa ccattcactg aaaagggagg ccttgctgtt    1140

ttattcggta atctagctcc ggacggcgct atcattaaaa caggcggcgt acagaatggg    1200

attacaagac acgaagggcc ggctgtcgta ttcgattctc aggacgaggc gcttgacggc    1260

attatcaacc gaaaagtaaa agaaggcgac gttgtcatca tcagatacga agggccaaaa    1320

ggcggacctg gcatgccgga aatgctggcg ccaacatccc aaatcgttgg aatgggactc    1380

gggccaaaag tggcattgat tacggacgga cgtttttccg gagcctcccg tggcctctca    1440

atcggccacg tatcacctga ggccgctgag ggcgggccgc ttgcctttgt tgaaaacgga    1500

gaccatatta tcgttgatat tgaaaaacgc atcttggatg tacaagtgcc agaagaagag    1560

tgggaaaaac gaaaagcgaa ctggaaaggt tttgaaccga aagtgaaaac cggctacctg    1620

gcacgttatt ctaaacttgt gacaagtgcc aacaccggcg gtattatgaa aatctag      1677

<210>  190
<211>  558
<212>  PRT
<213>  Bacillus subtilis

<400>  190

Met Ala Glu Leu Arg Ser Asn Met Ile Thr Gln Gly Ile Asp Arg Ala
1               5                   10                  15

Pro His Arg Ser Leu Leu Arg Ala Ala Gly Val Lys Glu Glu Asp Phe
            20                  25                  30

Gly Lys Pro Phe Ile Ala Val Cys Asn Ser Tyr Ile Asp Ile Val Pro
        35                  40                  45

Gly His Val His Leu Gln Glu Phe Gly Lys Ile Val Lys Glu Ala Ile
    50                  55                  60

Arg Glu Ala Gly Gly Val Pro Phe Glu Phe Asn Thr Ile Gly Val Asp
65                  70                  75                  80

Asp Gly Ile Ala Met Gly His Ile Gly Met Arg Tyr Ser Leu Pro Ser
                85                  90                  95

Arg Glu Ile Ile Ala Asp Ser Val Glu Thr Val Val Ser Ala His Trp
            100                 105                 110

Phe Asp Gly Met Val Cys Ile Pro Asn Cys Asp Lys Ile Thr Pro Gly
        115                 120                 125

Met Leu Met Ala Ala Met Arg Ile Asn Ile Pro Thr Ile Phe Val Ser
    130                 135                 140

Gly Gly Pro Met Ala Ala Gly Arg Thr Ser Tyr Gly Arg Lys Ile Ser
145                 150                 155                 160

Leu Ser Ser Val Phe Glu Gly Val Gly Ala Tyr Gln Ala Gly Lys Ile
                165                 170                 175

Asn Glu Asn Glu Leu Gln Glu Leu Glu Gln Phe Gly Cys Pro Thr Cys
            180                 185                 190

Gly Ser Cys Ser Gly Met Phe Thr Ala Asn Ser Met Asn Cys Leu Ser
        195                 200                 205

Glu Ala Leu Gly Leu Ala Leu Pro Gly Asn Gly Thr Ile Leu Ala Thr
    210                 215                 220

Ser Pro Glu Arg Lys Glu Phe Val Arg Lys Ser Ala Ala Gln Leu Met
225                 230                 235                 240

Glu Thr Ile Arg Lys Asp Ile Lys Pro Arg Asp Ile Val Thr Val Lys
                245                 250                 255

Ala Ile Asp Asn Ala Phe Ala Leu Asp Met Ala Leu Gly Gly Ser Thr
            260                 265                 270

Asn Thr Val Leu His Thr Leu Ala Leu Ala Asn Glu Ala Gly Val Glu
        275                 280                 285

Tyr Ser Leu Glu Arg Ile Asn Glu Val Ala Glu Arg Val Pro His Leu
    290                 295                 300

Ala Lys Leu Ala Pro Ala Ser Asp Val Phe Ile Glu Asp Leu His Glu
305                 310                 315                 320

Ala Gly Gly Val Ser Ala Ala Leu Asn Glu Leu Ser Lys Lys Glu Gly
                325                 330                 335

Ala Leu His Leu Asp Ala Leu Thr Val Thr Gly Lys Thr Leu Gly Glu
            340                 345                 350

Thr Ile Ala Gly His Glu Val Lys Asp Tyr Asp Val Ile His Pro Leu
        355                 360                 365

Asp Gln Pro Phe Thr Glu Lys Gly Gly Leu Ala Val Leu Phe Gly Asn
    370                 375                 380

Leu Ala Pro Asp Gly Ala Ile Ile Lys Thr Gly Gly Val Gln Asn Gly
385                 390                 395                 400

Ile Thr Arg His Glu Gly Pro Ala Val Val Phe Asp Ser Gln Asp Glu
                405                 410                 415

Ala Leu Asp Gly Ile Ile Asn Arg Lys Val Lys Glu Gly Asp Val Val
            420                 425                 430

Ile Ile Arg Tyr Glu Gly Pro Lys Gly Gly Pro Gly Met Pro Glu Met
        435                 440                 445

Leu Ala Pro Thr Ser Gln Ile Val Gly Met Gly Leu Gly Pro Lys Val
    450                 455                 460

Ala Leu Ile Thr Asp Gly Arg Phe Ser Gly Ala Ser Arg Gly Leu Ser
465                 470                 475                 480

Ile Gly His Val Ser Pro Glu Ala Ala Glu Gly Gly Pro Leu Ala Phe
                485                 490                 495

Val Glu Asn Gly Asp His Ile Ile Val Asp Ile Glu Lys Arg Ile Leu
            500                 505                 510

Asp Val Gln Val Pro Glu Glu Glu Trp Glu Lys Arg Lys Ala Asn Trp
        515                 520                 525

Lys Gly Phe Glu Pro Lys Val Lys Thr Gly Tyr Leu Ala Arg Tyr Ser
    530                 535                 540

Lys Leu Val Thr Ser Ala Asn Thr Gly Gly Ile Met Lys Ile
545                 550                 555

<210>  191
<211>  1647
<212>  DNA
<213>  Lactococcus lactis

<400>  191
atgtatacag taggagatta cctattagac cgattacacg agttaggaat tgaagaaatt      60

tttggagtcc ctggagacta taacttacaa tttttagatc aaattatttc ccacaaggat     120

atgaaatggg tcggaaatgc taatgaatta aatgcttcat atatggctga tggctatgct     180

cgtactaaaa aagctgccgc atttcttaca acctttggag taggtgaatt gagtgcagtt     240

aatggattag caggaagtta cgccgaaaat ttaccagtag tagaaatagt gggatcacct     300

acatcaaaag ttcaaaatga aggaaaattt gttcatcata cgctggctga cggtgatttt     360

aaacacttta tgaaaatgca cgaacctgtt acagcagctc gaactttact gacagcagaa     420

aatgcaaccg ttgaaattga ccgagtactt tctgcactat taaaagaaag aaaacctgtc     480

tatatcaact taccagttga tgttgctgct gcaaaagcag agaaaccctc actccctttg     540

aaaaaggaaa actcaacttc aaatacaagt gaccaagaaa ttttgaacaa aattcaagaa     600

agcttgaaaa atgccaaaaa accaatcgtg attacaggac atgaaataat tagttttggc     660

ttagaaaaaa cagtcactca atttatttca aagacaaaac tacctattac gacattaaac     720

tttggtaaaa gttcagttga tgaagccctc ccttcatttt taggaatcta taatggtaca     780

ctctcagagc ctaatcttaa agaattcgtg gaatcagccg acttcatctt gatgcttgga     840

gttaaactca cagactcttc aacaggagcc ttcactcatc atttaaatga aaataaaatg     900

atttcactga atatagatga aggaaaaata tttaacgaaa gaatccaaaa ttttgatttt     960

gaatccctca tctcctctct cttagaccta agcgaaatag aatacaaagg aaaatatatc    1020

gataaaaagc aagaagactt tgttccatca aatgcgcttt tatcacaaga ccgcctatgg    1080

caagcagttg aaaacctaac tcaaagcaat gaaacaatcg ttgctgaaca agggacatca    1140

ttctttggcg cttcatcaat ttttcttaaa tcaaagagtc attttattgg tcaaccctta    1200

tggggatcaa ttggatatac attcccagca gcattaggaa gccaaattgc agataaagaa 1260

agcagacacc ttttatttat tggtgatggt tcacttcaac ttacagtgca agaattagga 1320

ttagcaatca gagaaaaaat taatccaatt tgctttatta tcaataatga tggttataca 1380

gtcgaaagag aaattcatgg accaaatcaa agctacaatg atattccaat gtggaattac 1440

tcaaaattac cagaatcgtt tggagcaaca gaagatcgag tagtctcaaa aatcgttaga 1500

actgaaaatg aatttgtgtc tgtcatgaaa gaagctcaag cagatccaaa tagaatgtac 1560

tggattgagt taattttggc aaaagaaggt gcaccaaaag tactgaaaaa aatgggcaaa 1620

ctatttgctg aacaaaataa atcataa 1647

<210> 192
<211> 1644
<212> DNA
<213> Lactococcus lactis

<400> 192
atgtatacag taggagatta cctgttagac cgattacacg agttgggaat tgaagaaatt 60

tttggagttc ctggtgacta taacttacaa tttttagatc aaattatttc acgcgaagat 120

atgaaatgga ttggaaatgc taatgaatta aatgcttctt atatggctga tggttatgct 180

cgtactaaaa aagctgccgc atttctcacc acatttggag tcggcgaatt gagtgcgatc 240

aatggactgg caggaagtta tgccgaaaat ttaccagtag tagaaattgt tggttcacca 300

acttcaaaag tacaaaatga cggaaaattt gtccatcata cactagcaga tggtgatttt 360

aaacacttta tgaagatgca tgaacctgtt acagcagcgc ggactttact gacagcagaa 420

aatgccacat atgaaattga ccgagtactt tctcaattac taaaagaaag aaaaccagtc 480

tatattaact taccagtcga tgttgctgca gcaaaagcag agaagcctgc attatcttta 540

gaaaaagaaa gctctacaac aaatacaact gaacaagtga ttttgagtaa gattgaagaa 600

agtttgaaaa atgcccaaaa accagtagtg attgcaggac acgaagtaat tagttttggt 660

ttagaaaaaa cggtaactca gtttgtttca gaaacaaaac taccgattac gacactaaat 720

tttggtaaaa gtgctgttga tgaatctttg ccctcatttt taggaatata taacgggaaa 780

ctttcagaaa tcagtcttaa aaattttgtg gagtccgcag actttatcct aatgcttgga 840

gtgaagctta cggactcctc aacaggtgca ttcacacatc atttagatga aaataaaatg 900

atttcactaa acatagatga aggaataatt ttcaataaag tggtagaaga ttttgatttt 960

agagcagtgg tttcttcttt atcagaatta aaaggaatag aatatgaagg acaatatatt 1020

gataagcaat atgaagaatt tattccatca agtgctccct tatcacaaga ccgtctatgg 1080

caggcagttg aaagtttgac tcaaagcaat gaaacaatcg ttgctgaaca aggaacctca 1140

ttttttggag cttcaacaat tttcttaaaa tcaaatagtc gttttattgg acaaccttta 1200

tggggttcta ttggatatac ttttccagcg gctttaggaa gccaaattgc ggataaagag 1260

agcagacacc ttttatttat tggtgatggt tcacttcaac ttaccgtaca agaattagga 1320

ctatcaatca gagaaaaact caatccaatt tgttttatca taaataatga tggttataca 1380

gttgaaagag aaatccacgg acctactcaa agttataacg acattccaat gtggaattac 1440

tcgaaattac cagaaacatt tggagcaaca gaagatcgtg tagtatcaaa aattgttaga 1500

acagagaatg aatttgtgtc tgtcatgaaa gaagcccaag cagatgtcaa tagaatgtat 1560

tggatagaac tagttttgga aaaagaagat gcgccaaaat tactgaaaaa aatgggtaaa 1620

ttatttgctg agcaaaataa atag 1644

<210> 193
<211> 547
<212> PRT
<213> Lactococcus lactis

<400> 193

Met Tyr Thr Val Gly Asp Tyr Leu Leu Asp Arg Leu His Glu Leu Gly
1               5                   10                  15

Ile Glu Glu Ile Phe Gly Val Pro Gly Asp Tyr Asn Leu Gln Phe Leu
            20                  25                  30

Asp Gln Ile Ile Ser Arg Glu Asp Met Lys Trp Ile Gly Asn Ala Asn
        35                  40                  45

Glu Leu Asn Ala Ser Tyr Met Ala Asp Gly Tyr Ala Arg Thr Lys Lys
    50                  55                  60

Ala Ala Ala Phe Leu Thr Thr Phe Gly Val Gly Glu Leu Ser Ala Ile
65                  70                  75                  80

Asn Gly Leu Ala Gly Ser Tyr Ala Glu Asn Leu Pro Val Val Glu Ile
                85                  90                  95

Val Gly Ser Pro Thr Ser Lys Val Gln Asn Asp Gly Lys Phe Val His
            100                 105                 110

His Thr Leu Ala Asp Gly Asp Phe Lys His Phe Met Lys Met His Glu
        115                 120                 125

Pro Val Thr Ala Ala Arg Thr Leu Leu Thr Ala Glu Asn Ala Thr Tyr
    130                 135                 140

Glu Ile Asp Arg Val Leu Ser Gln Leu Leu Lys Glu Arg Lys Pro Val
145                 150                 155                 160

Tyr Ile Asn Leu Pro Val Asp Val Ala Ala Ala Lys Ala Glu Lys Pro
                165                 170                 175

Ala Leu Ser Leu Glu Lys Glu Ser Ser Thr Thr Asn Thr Thr Glu Gln
        180                 185                 190

Val Ile Leu Ser Lys Ile Glu Glu Ser Leu Lys Asn Ala Gln Lys Pro
    195                 200                 205

Val Val Ile Ala Gly His Glu Val Ile Ser Phe Gly Leu Glu Lys Thr
    210                 215                 220

Val Thr Gln Phe Val Ser Glu Thr Lys Leu Pro Ile Thr Thr Leu Asn
225                 230                 235                 240

Phe Gly Lys Ser Ala Val Asp Glu Ser Leu Pro Ser Phe Leu Gly Ile
                245                 250                 255

Tyr Asn Gly Lys Leu Ser Glu Ile Ser Leu Lys Asn Phe Val Glu Ser
        260                 265                 270

Ala Asp Phe Ile Leu Met Leu Gly Val Lys Leu Thr Asp Ser Ser Thr
        275                 280                 285

Gly Ala Phe Thr His His Leu Asp Glu Asn Lys Met Ile Ser Leu Asn
    290                 295                 300

Ile Asp Glu Gly Ile Ile Phe Asn Lys Val Val Glu Asp Phe Asp Phe
305                 310                 315                 320

Arg Ala Val Val Ser Ser Leu Ser Glu Leu Lys Gly Ile Glu Tyr Glu
                325                 330                 335

Gly Gln Tyr Ile Asp Lys Gln Tyr Glu Glu Phe Ile Pro Ser Ser Ala
        340                 345                 350

Pro Leu Ser Gln Asp Arg Leu Trp Gln Ala Val Glu Ser Leu Thr Gln
        355                 360                 365

Ser Asn Glu Thr Ile Val Ala Glu Gln Gly Thr Ser Phe Phe Gly Ala
    370                 375                 380

Ser Thr Ile Phe Leu Lys Ser Asn Ser Arg Phe Ile Gly Gln Pro Leu
385                 390                 395                 400

Trp Gly Ser Ile Gly Tyr Thr Phe Pro Ala Ala Leu Gly Ser Gln Ile
                405                 410                 415

Ala Asp Lys Glu Ser Arg His Leu Leu Phe Ile Gly Asp Gly Ser Leu
        420                 425                 430

Gln Leu Thr Val Gln Glu Leu Gly Leu Ser Ile Arg Glu Lys Leu Asn
      435                 440                 445

Pro Ile Cys Phe Ile Ile Asn Asn Asp Gly Tyr Thr Val Glu Arg Glu
    450                 455                 460

Ile His Gly Pro Thr Gln Ser Tyr Asn Asp Ile Pro Met Trp Asn Tyr
465                 470                 475                 480

Ser Lys Leu Pro Glu Thr Phe Gly Ala Thr Glu Asp Arg Val Val Ser
                485                 490                 495

Lys Ile Val Arg Thr Glu Asn Glu Phe Val Ser Val Met Lys Glu Ala
            500                 505                 510

Gln Ala Asp Val Asn Arg Met Tyr Trp Ile Glu Leu Val Leu Glu Lys
      515                 520                 525

Glu Asp Ala Pro Lys Leu Leu Lys Lys Met Gly Lys Leu Phe Ala Glu
    530                 535                 540

Gln Asn Lys
545

<210>  194
<211>  1653
<212>  DNA
<213>  Salmonella typhimurium

<400>  194
ttatcccccg ttgcgggctt ccagcgcccg ggtcacggta cgcagtaatt ccggcagatc      60

ggcttttggc aacatcactt caataaatga cagacgttgt gggcgcgcca accgttcgag     120

gacctctgcc agttggatag cctgcgtcac ccgccagcac tccgcctgtt gcgccgcgtt     180

tagcgccggt ggtatctgcg tccagttcca gctcgcgatg tcgttatacc gctgggccgc     240

gccgtgaatg gcgcgctcta cggtatagcc gtcattgttg agcagcagga tgaccggcgc     300

ctgcccgtcg cgtaacatcg agcccatctc ctgaatcgtg agctgcgccg cgccatcgcc     360

gataatcaga atcacccgcc gatcgggaca ggcggtttgc gcgccaaacg cggcgggcaa     420

ggaatagccg atagaccccc acagcggctg taacacaact tccgcgccgt caggaagcga     480

cagcgcggca gcgccaaaag ctgctgtccc ctggtcgaca aggataatat ctccgggttt     540

gagatactgc tgtaaggttt gccagaagct ttcctgggtc agttctcctt tatcaatccg     600

cactggctgt ccggcggaac gcgtcggcgg cggcgcaaaa gcgcattcca ggcacagttc     660

gcgcagcgta gacaccgcct gcgccatcgg gaggttgaac caggtttcgc cgatgcgcga     720

cgcgtaaggc tgaatctcca gcgtgcgttc cgccggtaat tgttgggtaa atccggccgt     780

aagggtatcg acaaaacggg tgccgacgca gataacccta tcggcgtcct ctatggcctg 840

acgcacttct ttgctgctgg cgccagcgct ataggtgcca acgaagttcg ggtgctgttc 900

atcaaaaagc cccttcccca tcagtagtgt cgcatgagcg atgggcgttt ccgccatcca 960

gcgctgcaac agtggtcgta aaccaaaacg cccggcaaga aagtcggcca atagcgcaat 1020

gcgccgactg ttcatcaggc actgacgggc gtgataacga aaggccgtct ccacgccgct 1080

ttgcgcttca tgcacgggca acgccagcgc ctgcgtaggt gggatggccg tttttttcgc 1140

cacatcggcg ggcaacatga tgtatcctgg cctgcgtgcg gcaagcattt cacccaacac 1200

gcggtcaatc tcgaaacagg cgttctgttc atctaatatt gcgctggcag cggatatcgc 1260

ctgactcatg cgataaaaat gacgaaaatc gccgtcaccg agggtatggt gcatcaattc 1320

gccacgctgc tgcgcagcgc tacagggcgc gccgacgata tgcaagaccg ggacatattc 1380

cgcgtaactg cccgcgatac cgttaatagc gctaagttct cccacgccaa aggtggtgag 1440

tagcgctcca gcgcccgaca tgcgcgcata gccgtccgcg gcataagcgg cgttcagctc 1500

attggcgcat cccacccaac gcagggtcgg gtggtcaatc acatggtcaa gaaactgcaa 1560

gttataatcg cccggtacgc caaaaagatg gccaatgccg catcctgcca gtctgtccag 1620

caaatagtcg gccacggtat aggggttttg cat 1653

<210> 195
<211> 550
<212> PRT
<213> Salmonella typhimurium

<400> 195

Met Gln Asn Pro Tyr Thr Val Ala Asp Tyr Leu Leu Asp Arg Leu Ala
1               5                   10                  15

Gly Cys Gly Ile Gly His Leu Phe Gly Val Pro Gly Asp Tyr Asn Leu
            20                  25                  30

Gln Phe Leu Asp His Val Ile Asp His Pro Thr Leu Arg Trp Val Gly
        35                  40                  45

Cys Ala Asn Glu Leu Asn Ala Ala Tyr Ala Ala Asp Gly Tyr Ala Arg
    50                  55                  60

Met Ser Gly Ala Gly Ala Leu Leu Thr Thr Phe Gly Val Gly Glu Leu
65                  70                  75                  80

Ser Ala Ile Asn Gly Ile Ala Gly Ser Tyr Ala Glu Tyr Val Pro Val
                85                  90                  95

Leu His Ile Val Gly Ala Pro Cys Ser Ala Ala Gln Gln Arg Gly Glu
            100                 105                 110

Leu Met His His Thr Leu Gly Asp Gly Asp Phe Arg His Phe Tyr Arg
        115                 120                 125

Met Ser Gln Ala Ile Ser Ala Ala Ser Ala Ile Leu Asp Glu Gln Asn
    130                 135                 140

Ala Cys Phe Glu Ile Asp Arg Val Leu Gly Glu Met Leu Ala Ala Arg
145                 150                 155                 160

Arg Pro Gly Tyr Ile Met Leu Pro Ala Asp Val Ala Lys Lys Thr Ala
                165                 170                 175

Ile Pro Pro Thr Gln Ala Leu Ala Leu Pro Val His Glu Ala Gln Ser
            180                 185                 190

Gly Val Glu Thr Ala Phe Arg Tyr His Ala Arg Gln Cys Leu Met Asn
        195                 200                 205

Ser Arg Arg Ile Ala Leu Leu Ala Asp Phe Leu Ala Gly Arg Phe Gly
    210                 215                 220

Leu Arg Pro Leu Leu Gln Arg Trp Met Ala Glu Thr Pro Ile Ala His
225                 230                 235                 240

Ala Thr Leu Leu Met Gly Lys Gly Leu Phe Asp Glu Gln His Pro Asn
                245                 250                 255

Phe Val Gly Thr Tyr Ser Ala Gly Ala Ser Ser Lys Glu Val Arg Gln
            260                 265                 270

Ala Ile Glu Asp Ala Asp Arg Val Ile Cys Val Gly Thr Arg Phe Val
        275                 280                 285

Asp Thr Leu Thr Ala Gly Phe Thr Gln Gln Leu Pro Ala Glu Arg Thr
    290                 295                 300

Leu Glu Ile Gln Pro Tyr Ala Ser Arg Ile Gly Glu Thr Trp Phe Asn
305                 310                 315                 320

Leu Pro Met Ala Gln Ala Val Ser Thr Leu Arg Glu Leu Cys Leu Glu
                325                 330                 335

Cys Ala Phe Ala Pro Pro Pro Thr Arg Ser Ala Gly Gln Pro Val Arg
            340                 345                 350

Ile Asp Lys Gly Glu Leu Thr Gln Glu Ser Phe Trp Gln Thr Leu Gln
        355                 360                 365

Gln Tyr Leu Lys Pro Gly Asp Ile Ile Leu Val Asp Gln Gly Thr Ala
    370                 375                 380

Ala Phe Gly Ala Ala Ala Leu Ser Leu Pro Asp Gly Ala Glu Val Val
385                 390                 395                 400

Leu Gln Pro Leu Trp Gly Ser Ile Gly Tyr Ser Leu Pro Ala Ala Phe
                405                 410                 415

Gly Ala Gln Thr Ala Cys Pro Asp Arg Arg Val Ile Leu Ile Ile Gly
            420                 425                 430

Asp Gly Ala Ala Gln Leu Thr Ile Gln Glu Met Gly Ser Met Leu Arg
        435                 440                 445

Asp Gly Gln Ala Pro Val Ile Leu Leu Leu Asn Asn Asp Gly Tyr Thr
    450                 455                 460

Val Glu Arg Ala Ile His Gly Ala Ala Gln Arg Tyr Asn Asp Ile Ala
465                 470                 475                 480

Ser Trp Asn Trp Thr Gln Ile Pro Pro Ala Leu Asn Ala Ala Gln Gln
                485                 490                 495

Ala Glu Cys Trp Arg Val Thr Gln Ala Ile Gln Leu Ala Glu Val Leu
            500                 505                 510

Glu Arg Leu Ala Arg Pro Gln Arg Leu Ser Phe Ile Glu Val Met Leu
        515                 520                 525

Pro Lys Ala Asp Leu Pro Glu Leu Leu Arg Thr Val Thr Arg Ala Leu
    530                 535                 540

Glu Ala Arg Asn Gly Gly
545                 550

<210>  196
<211>  1665
<212>  DNA
<213>  Clostridium acetobutylicum

<400>  196
ttgaagagtg aatacacaat tggaagatat ttgttagacc gtttatcaga gttgggtatt      60

cggcatatct ttggtgtacc tggagattac aatctatcct ttttagacta tataatggag     120

tacaaaggga tagattgggt tggaaattgc aatgaattga atgctgggta tgctgctgat     180

ggatatgcaa gaataaatgg aattggagcc atacttacaa catttggtgt tggagaatta     240

agtgccatta acgcaattgc tggggcatac gctgagcaag ttccagttgt taaaattaca     300

ggtatcccca cagcaaaagt tagggacaat ggattatatg tacaccacac attaggtgac     360

ggaaggtttg atcacttttt tgaaatgttt agagaagtaa cagttgctga ggcattacta 420

agcgaagaaa atgcagcaca agaaattgat cgtgttctta tttcatgctg gagacaaaaa 480

cgtcctgttc ttataaattt accgattgat gtatatgata aaccaattaa caaaccatta 540

aagccattac tcgattatac tatttcaagt aacaaagagg ctgcatgtga atttgttaca 600

gaaatagtac ctataataaa tagggcaaaa aagcctgtta ttcttgcaga ttatggagta 660

tatcgttacc aagttcaaca tgtgcttaaa aacttggccg aaaaaaccgg atttcctgtg 720

gctacactaa gtatgggaaa aggtgttttc aatgaagcac accctcaatt tattggtgtt 780

tataatggtg atgtaagttc tccttattta aggcagcgag ttgatgaagc agactgcatt 840

attagcgttg gtgtaaaatt gacggattca accacagggg gattttctca tggattttct 900

aaaaggaatg taattcacat tgatcctttt tcaataaagg caaaaggtaa aaaatatgca 960

cctattacga tgaaagatgc tttaacagaa ttaacaagta aaattgagca tagaaacttt 1020

gaggatttag atataaagcc ttacaaatca gataatcaaa agtattttgc aaaagagaag 1080

ccaattacac aaaaacgttt ttttgagcgt attgctcact ttataaaaga aaaagatgta 1140

ttattagcag aacagggtac atgctttttt ggtgcgtcaa ccatacaact acccaaagat 1200

gcaactttta ttggtcaacc tttatgggga tctattggat acacacttcc tgctttatta 1260

ggttcacaat tagctgatca aaaaaggcgt aatattcttt taattgggga tggtgcattt 1320

caaatgacag cacaagaaat ttcaacaatg cttcgtttac aaatcaaacc tattattttt 1380

ttaattaata acgatggtta tacaattgaa cgtgctattc atggtagaga acaagtatat 1440

aacaatattc aaatgtggcg atatcataat gttccaaagg ttttaggtcc taaagaatgc 1500

agcttaacct ttaaagtaca aagtgaaact gaacttgaaa aggctctttt agtggcagat 1560

aaggattgtg aacatttgat ttttatagaa gttgttatgg atcgttatga taaacccgag 1620

cctttagaac gtctttcgaa acgttttgca aatcaaaata attag 1665

<210> 197
<211> 554
<212> PRT
<213> Clostridium acetobutylicum

<400> 197

Met Lys Ser Glu Tyr Thr Ile Gly Arg Tyr Leu Leu Asp Arg Leu Ser
1               5                   10                  15

Glu Leu Gly Ile Arg His Ile Phe Gly Val Pro Gly Asp Tyr Asn Leu
            20                  25                  30

Ser Phe Leu Asp Tyr Ile Met Glu Tyr Lys Gly Ile Asp Trp Val Gly
        35                  40                  45

Asn Cys Asn Glu Leu Asn Ala Gly Tyr Ala Ala Asp Gly Tyr Ala Arg
    50                  55                  60

Ile Asn Gly Ile Gly Ala Ile Leu Thr Thr Phe Gly Val Gly Glu Leu
65                  70                  75                  80

Ser Ala Ile Asn Ala Ile Ala Gly Ala Tyr Ala Glu Gln Val Pro Val
                85                  90                  95

Val Lys Ile Thr Gly Ile Pro Thr Ala Lys Val Arg Asp Asn Gly Leu
            100                 105                 110

Tyr Val His His Thr Leu Gly Asp Gly Arg Phe Asp His Phe Phe Glu
        115                 120                 125

Met Phe Arg Glu Val Thr Val Ala Glu Ala Leu Leu Ser Glu Glu Asn
    130                 135                 140

Ala Ala Gln Glu Ile Asp Arg Val Leu Ile Ser Cys Trp Arg Gln Lys
145                 150                 155                 160

Arg Pro Val Leu Ile Asn Leu Pro Ile Asp Val Tyr Asp Lys Pro Ile
                165                 170                 175

Asn Lys Pro Leu Lys Pro Leu Leu Asp Tyr Thr Ile Ser Ser Asn Lys
            180                 185                 190

Glu Ala Ala Cys Glu Phe Val Thr Glu Ile Val Pro Ile Ile Asn Arg
        195                 200                 205

Ala Lys Lys Pro Val Ile Leu Ala Asp Tyr Gly Val Tyr Arg Tyr Gln
    210                 215                 220

Val Gln His Val Leu Lys Asn Leu Ala Glu Lys Thr Gly Phe Pro Val
225                 230                 235                 240

Ala Thr Leu Ser Met Gly Lys Gly Val Phe Asn Glu Ala His Pro Gln
                245                 250                 255

Phe Ile Gly Val Tyr Asn Gly Asp Val Ser Ser Pro Tyr Leu Arg Gln
            260                 265                 270

Arg Val Asp Glu Ala Asp Cys Ile Ile Ser Val Gly Val Lys Leu Thr
        275                 280                 285

Asp Ser Thr Thr Gly Gly Phe Ser His Gly Phe Ser Lys Arg Asn Val
    290                 295                 300

Ile His Ile Asp Pro Phe Ser Ile Lys Ala Lys Gly Lys Lys Tyr Ala

305                 310                 315                 320

Pro Ile Thr Met Lys Asp Ala Leu Thr Glu Leu Thr Ser Lys Ile Glu
                325                 330                 335

His Arg Asn Phe Glu Asp Leu Asp Ile Lys Pro Tyr Lys Ser Asp Asn
            340                 345                 350

Gln Lys Tyr Phe Ala Lys Glu Lys Pro Ile Thr Gln Lys Arg Phe Phe
        355                 360                 365

Glu Arg Ile Ala His Phe Ile Lys Glu Lys Asp Val Leu Leu Ala Glu
    370                 375                 380

Gln Gly Thr Cys Phe Phe Gly Ala Ser Thr Ile Gln Leu Pro Lys Asp
385                 390                 395                 400

Ala Thr Phe Ile Gly Gln Pro Leu Trp Gly Ser Ile Gly Tyr Thr Leu
                405                 410                 415

Pro Ala Leu Leu Gly Ser Gln Leu Ala Asp Gln Lys Arg Arg Asn Ile
            420                 425                 430

Leu Leu Ile Gly Asp Gly Ala Phe Gln Met Thr Ala Gln Glu Ile Ser
        435                 440                 445

Thr Met Leu Arg Leu Gln Ile Lys Pro Ile Ile Phe Leu Ile Asn Asn
    450                 455                 460

Asp Gly Tyr Thr Ile Glu Arg Ala Ile His Gly Arg Glu Gln Val Tyr
465                 470                 475                 480

Asn Asn Ile Gln Met Trp Arg Tyr His Asn Val Pro Lys Val Leu Gly
                485                 490                 495

Pro Lys Glu Cys Ser Leu Thr Phe Lys Val Gln Ser Glu Thr Glu Leu
            500                 505                 510

Glu Lys Ala Leu Leu Val Ala Asp Lys Asp Cys Glu His Leu Ile Phe
        515                 520                 525

Ile Glu Val Val Met Asp Arg Tyr Asp Lys Pro Glu Pro Leu Glu Arg
    530                 535                 540

Leu Ser Lys Arg Phe Ala Asn Gln Asn Asn
545                 550

<210>  198
<211>  939

<212>  DNA
<213>  Saccharomyces cerevisiae

<400>  198
atgcctgcta cgttaaagaa ttcttctgct acattaaaac taaatactgg tgcctccatt      60

ccagtgttgg gtttcggcac ttggcgttcc gttgacaata acggttacca ttctgtaatt     120

gcagctttga aagctggata cagacacatt gatgctgcgg ctatctattt gaatgaagaa     180

gaagttggca gggctattaa agattccgga gtccctcgtg aggaaatttt tattactact     240

aagctttggg gtacggaaca acgtgatccg gaagctgctc taaacaagtc tttgaaaaga     300

ctaggcttgg attatgttga cctatatctg atgcattggc cagtgccttt gaaaaccgac     360

agagttactg atggtaacgt tctgtgcatt ccaacattag aagatggcac tgttgacatc     420

gatactaagg aatggaattt tatcaagacg tgggagttga tgcaagagtt gccaaagacg     480

ggcaaaacta aagccgttgg tgtctctaat ttttctatta acaacattaa agaattatta     540

gaatctccaa ataacaaggt ggtaccagct actaatcaaa ttgaaattca tccattgcta     600

ccacaagacg aattgattgc cttttgtaag gaaaagggta ttgttgttga agcctactca     660

ccatttggga gtgctaatgc tcctttacta aaagagcaag caattattga tatggctaaa     720

aagcacggcg ttgagccagc acagcttatt atcagttgga gtattcaaag aggctacgtt     780

gttctggcca aatcggttaa tcctgaaaga attgtatcca attttaagat tttcactctg     840

cctgaggatg atttcaagac tattagtaac ctatccaaag tgcatggtac aaagagagtc     900

gttgatatga agtggggatc cttcccaatt ttccaatga                            939

<210>  199
<211>  312
<212>  PRT
<213>  Saccharomyces cerevisiae

<400>  199

Met Pro Ala Thr Leu Lys Asn Ser Ser Ala Thr Leu Lys Leu Asn Thr
1               5                   10                  15

Gly Ala Ser Ile Pro Val Leu Gly Phe Gly Thr Trp Arg Ser Val Asp
            20                  25                  30

Asn Asn Gly Tyr His Ser Val Ile Ala Ala Leu Lys Ala Gly Tyr Arg
        35                  40                  45

His Ile Asp Ala Ala Ala Ile Tyr Leu Asn Glu Glu Glu Val Gly Arg
    50                  55                  60

Ala Ile Lys Asp Ser Gly Val Pro Arg Glu Glu Ile Phe Ile Thr Thr
65                  70                  75                  80

Lys Leu Trp Gly Thr Glu Gln Arg Asp Pro Glu Ala Ala Leu Asn Lys

85                  90                  95

Ser Leu Lys Arg Leu Gly Leu Asp Tyr Val Asp Leu Tyr Leu Met His
            100                 105                 110

Trp Pro Val Pro Leu Lys Thr Asp Arg Val Thr Asp Gly Asn Val Leu
        115                 120                 125

Cys Ile Pro Thr Leu Glu Asp Gly Thr Val Asp Ile Asp Thr Lys Glu
    130                 135                 140

Trp Asn Phe Ile Lys Thr Trp Glu Leu Met Gln Glu Leu Pro Lys Thr
145                 150                 155                 160

Gly Lys Thr Lys Ala Val Gly Val Ser Asn Phe Ser Ile Asn Asn Ile
                165                 170                 175

Lys Glu Leu Leu Glu Ser Pro Asn Asn Lys Val Val Pro Ala Thr Asn
            180                 185                 190

Gln Ile Glu Ile His Pro Leu Leu Pro Gln Asp Glu Leu Ile Ala Phe
        195                 200                 205

Cys Lys Glu Lys Gly Ile Val Val Glu Ala Tyr Ser Pro Phe Gly Ser
    210                 215                 220

Ala Asn Ala Pro Leu Leu Lys Glu Gln Ala Ile Ile Asp Met Ala Lys
225                 230                 235                 240

Lys His Gly Val Glu Pro Ala Gln Leu Ile Ile Ser Trp Ser Ile Gln
                245                 250                 255

Arg Gly Tyr Val Val Leu Ala Lys Ser Val Asn Pro Glu Arg Ile Val
            260                 265                 270

Ser Asn Phe Lys Ile Phe Thr Leu Pro Glu Asp Asp Phe Lys Thr Ile
        275                 280                 285

Ser Asn Leu Ser Lys Val His Gly Thr Lys Arg Val Val Asp Met Lys
    290                 295                 300

Trp Gly Ser Phe Pro Ile Phe Gln
305                 310

<210>  200
<211>  1083
<212>  DNA
<213>  Saccharomyces cerevisiae

<400>  200

ctagtctgaa aattctttgt cgtagccgac taaggtaaat ctatatctaa cgtcaccctt 60

ttccatcctt tcgaaggctt catggacgcc ggcttcacca acaggtaatg tttccaccca 120

aattttgata tctttttcag agactaattt caagagttgg ttcaattctt tgatggaacc 180

taaagcactg taagaaatgg agacagcctt taagccatat ggctttagcg ataacatttc 240

gtgttgttct ggtatagaga ttgagacaat tctaccacca accttcatag cctttggcat 300

aatgttgaag tcaatgtcgg taagggagga agcacagact acaatcaggt cgaaggtgtc 360

aaagtacttt tcaccccaat caccttcttc taatgtagca atgtagtgat cggcgcccat 420

cttcattgca tcttctcttt ttctcgaaga acgagaaata acatacgtct ctgcccccat 480

ggctttggaa atcaatgtac ccatactgcc gataccacca agaccaacta taccaacttt 540

tttacctgga ccgcaaccgt tacgaaccaa tggagagtac acagtcaaac caccacataa 600

tagtggagca gccaaatgtg atggaatatt ctctgggata ggcaccacaa aatgttcatg 660

aactctgacg tagtttgcat agccaccctg cgacacatag ccgtcttcat aaggctgact 720

gtatgtggta acaaacttgg tgcagtatgg ttcattatca ttcttacaac ggtcacattc 780

caagcatgaa aagacttgag cacctacacc aacacgttga ccgactttca acccactgtt 840

tgacttgggc cctagcttga caactttacc aacgatttca tgaccaacga ctagcggcat 900

cttcatattg ccccaatgac cagctgcaca atgaatatca ctaccgcaga caccacatgc 960

ttcgatctta atgtcaatgt catgatcgta aaatggtttt gggtcatact ttgtcttctt 1020

tgggtttttc caatcttcgt gtgattgaat agcgatacct tcaaatttct caggataaga 1080

cat 1083

<210> 201
<211> 360
<212> PRT
<213> Saccharomyces cerevisiae

<400> 201

Met Ser Tyr Pro Glu Lys Phe Glu Gly Ile Ala Ile Gln Ser His Glu
1               5                   10                  15

Asp Trp Lys Asn Pro Lys Lys Thr Lys Tyr Asp Pro Lys Pro Phe Tyr
            20                  25                  30

Asp His Asp Ile Asp Ile Lys Ile Glu Ala Cys Gly Val Cys Gly Ser
        35                  40                  45

Asp Ile His Cys Ala Ala Gly His Trp Gly Asn Met Lys Met Pro Leu
    50                  55                  60

Val Val Gly His Glu Ile Val Gly Lys Val Val Lys Leu Gly Pro Lys
65                  70                  75                  80

Ser Asn Ser Gly Leu Lys Val Gly Gln Arg Val Gly Val Gly Ala Gln
                85                  90                  95

Val Phe Ser Cys Leu Glu Cys Asp Arg Cys Lys Asn Asp Asn Glu Pro
            100                 105                 110

Tyr Cys Thr Lys Phe Val Thr Thr Tyr Ser Gln Pro Tyr Glu Asp Gly
        115                 120                 125

Tyr Val Ser Gln Gly Gly Tyr Ala Asn Tyr Val Arg Val His Glu His
    130                 135                 140

Phe Val Val Pro Ile Pro Glu Asn Ile Pro Ser His Leu Ala Ala Pro
145                 150                 155                 160

Leu Leu Cys Gly Gly Leu Thr Val Tyr Ser Pro Leu Val Arg Asn Gly
                165                 170                 175

Cys Gly Pro Gly Lys Lys Val Gly Ile Val Gly Leu Gly Gly Ile Gly
            180                 185                 190

Ser Met Gly Thr Leu Ile Ser Lys Ala Met Gly Ala Glu Thr Tyr Val
        195                 200                 205

Ile Ser Arg Ser Ser Arg Lys Arg Glu Asp Ala Met Lys Met Gly Ala
    210                 215                 220

Asp His Tyr Ile Ala Thr Leu Glu Glu Gly Asp Trp Gly Glu Lys Tyr
225                 230                 235                 240

Phe Asp Thr Phe Asp Leu Ile Val Val Cys Ala Ser Ser Leu Thr Asp
                245                 250                 255

Ile Asp Phe Asn Ile Met Pro Lys Ala Met Lys Val Gly Gly Arg Ile
            260                 265                 270

Val Ser Ile Ser Ile Pro Glu Gln His Glu Met Leu Ser Leu Lys Pro
        275                 280                 285

Tyr Gly Leu Lys Ala Val Ser Ile Ser Tyr Ser Ala Leu Gly Ser Ile
    290                 295                 300

Lys Glu Leu Asn Gln Leu Leu Lys Leu Val Ser Glu Lys Asp Ile Lys
305                 310                 315                 320

Ile Trp Val Glu Thr Leu Pro Val Gly Glu Ala Gly Val His Glu Ala
                325                 330                 335

Phe Glu Arg Met Glu Lys Gly Asp Val Arg Tyr Arg Phe Thr Leu Val
            340                 345                 350

Gly Tyr Asp Lys Glu Phe Ser Asp
        355                 360

<210>  202
<211>  1170
<212>  DNA
<213>  Clostridium acetobutylicum

<400>  202
ttaataagat tttttaaata tctcaagaac atcctctgca tttattggtc ttaaacttcc      60

tattgttcct ccagaatttc taacagcttg ctttgccatt agttctagtt tatcttttcc     120

tattccaact tctctaagct ttgaaggaat acccaatgaa ttaaagtatt ctctcgtatt     180

tttaatagcc tctcgtgcta tttcatagtt atctttgttc ttgtctattc cccaaacatt     240

tattccataa gaaacaaatt tatgaagtgt atcgtcattt agaatatatt ccatccaatt     300

aggtgttaaa attgcaagtc ctacaccatg tgttatatca taatatgcac ttaactcgtg     360

ttccatagga tgacaactcc attttctatc cttaccaagt gataatagac catttatagc     420

taaacttgaa gcccacatca aattagctct agcctcgtaa tcatcagtct tctccattgc     480

tatttttcca tactttatac atgttcttaa gattgcttct gctataccgt cctgcacata     540

agcaccttca acaccactaa agtaagattc aaaggtgtga ctcataatgt cagctgttcc     600

cgctgctgtt tgatttttag gtactgtaaa agtatatgta ggatctaaca ctgaaaattt     660

aggtctcata tcatcatgtc ctactccaag cttttcatta gtctccatat ttgaaattac     720

tgcaatttga tccatttcag accctgttgc tgaaagagta agtatacttg caattggaag     780

aactttagtt attttagatg gatctttaac catgtcccat gtatcgccat cataataaac     840

tccagctgca attaccttag aacagtctat tgcacttcct ccccctattg ctaatactaa     900

atccacatta ttttctctac atatttctat gccttttttt actgttgtta tcctaggatt     960

tggctctact cctgaaagtt catagaaagc tatattgttt tcttttaata tagctgttgc    1020

tctatcatat ataccgttcc tttttatact tcctccgcca taaactataa gcactcttga    1080

gccatatttc ttaatttctt ctccaattac gtctattttt ccttttccaa aaaaaacttt    1140

agttggtatt gaataatcaa aacttagcat                                     1170

<210>  203
<211>  389
<212>  PRT
<213>  Clostridium acetobutylicum

<400>  203

Met Leu Ser Phe Asp Tyr Ser Ile Pro Thr Lys Val Phe Phe Gly Lys
1               5                   10                  15

Gly Lys Ile Asp Val Ile Gly Glu Glu Ile Lys Lys Tyr Gly Ser Arg
            20                  25                  30

Val Leu Ile Val Tyr Gly Gly Gly Ser Ile Lys Arg Asn Gly Ile Tyr
        35                  40                  45

Asp Arg Ala Thr Ala Ile Leu Lys Glu Asn Asn Ile Ala Phe Tyr Glu
    50                  55                  60

Leu Ser Gly Val Glu Pro Asn Pro Arg Ile Thr Thr Val Lys Lys Gly
65                  70                  75                  80

Ile Glu Ile Cys Arg Glu Asn Asn Val Asp Leu Val Leu Ala Ile Gly
                85                  90                  95

Gly Gly Ser Ala Ile Asp Cys Ser Lys Val Ile Ala Ala Gly Val Tyr
            100                 105                 110

Tyr Asp Gly Asp Thr Trp Asp Met Val Lys Asp Pro Ser Lys Ile Thr
        115                 120                 125

Lys Val Leu Pro Ile Ala Ser Ile Leu Thr Leu Ser Ala Thr Gly Ser
    130                 135                 140

Glu Met Asp Gln Ile Ala Val Ile Ser Asn Met Glu Thr Asn Glu Lys
145                 150                 155                 160

Leu Gly Val Gly His Asp Asp Met Arg Pro Lys Phe Ser Val Leu Asp
                165                 170                 175

Pro Thr Tyr Thr Phe Thr Val Pro Lys Asn Gln Thr Ala Ala Gly Thr
            180                 185                 190

Ala Asp Ile Met Ser His Thr Phe Glu Ser Tyr Phe Ser Gly Val Glu
        195                 200                 205

Gly Ala Tyr Val Gln Asp Gly Ile Ala Glu Ala Ile Leu Arg Thr Cys
    210                 215                 220

Ile Lys Tyr Gly Lys Ile Ala Met Glu Lys Thr Asp Asp Tyr Glu Ala
225                 230                 235                 240

Arg Ala Asn Leu Met Trp Ala Ser Ser Leu Ala Ile Asn Gly Leu Leu
                245                 250                 255

Ser Leu Gly Lys Asp Arg Lys Trp Ser Cys His Pro Met Glu His Glu
            260                 265                 270

Leu Ser Ala Tyr Tyr Asp Ile Thr His Gly Val Gly Leu Ala Ile Leu
        275                 280                 285

Thr Pro Asn Trp Met Glu Tyr Ile Leu Asn Asp Asp Thr Leu His Lys
    290                 295                 300

Phe Val Ser Tyr Gly Ile Asn Val Trp Gly Ile Asp Lys Asn Lys Asp
305                 310                 315                 320

Asn Tyr Glu Ile Ala Arg Glu Ala Ile Lys Asn Thr Arg Glu Tyr Phe
                325                 330                 335

Asn Ser Leu Gly Ile Pro Ser Lys Leu Arg Glu Val Gly Ile Gly Lys
            340                 345                 350

Asp Lys Leu Glu Leu Met Ala Lys Gln Ala Val Arg Asn Ser Gly Gly
        355                 360                 365

Thr Ile Gly Ser Leu Arg Pro Ile Asn Ala Glu Asp Val Leu Glu Ile
    370                 375                 380

Phe Lys Lys Ser Tyr
385

<210>  204
<211>  390
<212>  PRT
<213>  Clostridium acetobutylicum

<400>  204

Met Val Asp Phe Glu Tyr Ser Ile Pro Thr Arg Ile Phe Phe Gly Lys
1               5                   10                  15

Asp Lys Ile Asn Val Leu Gly Arg Glu Leu Lys Lys Tyr Gly Ser Lys
            20                  25                  30

Val Leu Ile Val Tyr Gly Gly Gly Ser Ile Lys Arg Asn Gly Ile Tyr
        35                  40                  45

Asp Lys Ala Val Ser Ile Leu Glu Lys Asn Ser Ile Lys Phe Tyr Glu
    50                  55                  60

Leu Ala Gly Val Glu Pro Asn Pro Arg Val Thr Thr Val Glu Lys Gly
65                  70                  75                  80

Val Lys Ile Cys Arg Glu Asn Gly Val Glu Val Val Leu Ala Ile Gly
                85                  90                  95

Gly Gly Ser Ala Ile Asp Cys Ala Lys Val Ile Ala Ala Ala Cys Glu
            100                 105                 110

Tyr Asp Gly Asn Pro Trp Asp Ile Val Leu Asp Gly Ser Lys Ile Lys
        115                 120                 125

Arg Val Leu Pro Ile Ala Ser Ile Leu Thr Ile Ala Ala Thr Gly Ser
    130                 135                 140

Glu Met Asp Thr Trp Ala Val Ile Asn Asn Met Asp Thr Asn Glu Lys
145                 150                 155                 160

Leu Ile Ala Ala His Pro Asp Met Ala Pro Lys Phe Ser Ile Leu Asp
                165                 170                 175

Pro Thr Tyr Thr Tyr Thr Val Pro Thr Asn Gln Thr Ala Ala Gly Thr
            180                 185                 190

Ala Asp Ile Met Ser His Ile Phe Glu Val Tyr Phe Ser Asn Thr Lys
        195                 200                 205

Thr Ala Tyr Leu Gln Asp Arg Met Ala Glu Ala Leu Leu Arg Thr Cys
    210                 215                 220

Ile Lys Tyr Gly Gly Ile Ala Leu Glu Lys Pro Asp Asp Tyr Glu Ala
225                 230                 235                 240

Arg Ala Asn Leu Met Trp Ala Ser Ser Leu Ala Ile Asn Gly Leu Leu
                245                 250                 255

Thr Tyr Gly Lys Asp Thr Asn Trp Ser Val His Leu Met Glu His Glu
            260                 265                 270

Leu Ser Ala Tyr Tyr Asp Ile Thr His Gly Val Gly Leu Ala Ile Leu
        275                 280                 285

Thr Pro Asn Trp Met Glu Tyr Ile Leu Asn Asn Asp Thr Val Tyr Lys
    290                 295                 300

Phe Val Glu Tyr Gly Val Asn Val Trp Gly Ile Asp Lys Glu Lys Asn
305                 310                 315                 320

His Tyr Asp Ile Ala His Gln Ala Ile Gln Lys Thr Arg Asp Tyr Phe
                325                 330                 335

Val Asn Val Leu Gly Leu Pro Ser Arg Leu Arg Asp Val Gly Ile Glu
            340                 345                 350

Glu Glu Lys Leu Asp Ile Met Ala Lys Glu Ser Val Lys Leu Thr Gly
        355                 360                 365

Gly Thr Ile Gly Asn Leu Arg Pro Val Asn Ala Ser Glu Val Leu Gln
    370                 375                 380

Ile Phe Lys Lys Ser Val
385                 390

<210>  205
<211>  993
<212>  DNA
<213>  Bacillus subtilis

<400>  205
atgagtacaa accgacatca agcactaggg ctgactgatc aggaagccgt tgatatgtat      60

agaaccatgc tgttagcaag aaaaatcgat gaaagaatgt ggctgttaaa ccgttctggc     120

aaaattccat ttgtaatctc ttgtcaagga caggaagcag cacaggtagg agcggctttc     180

gcacttgacc gtgaaatgga ttatgtattg ccgtactaca gagacatggg tgtcgtgctc     240

gcgtttggca tgacagcaaa ggacttaatg atgtccgggt ttgcaaaagc agcagatccg     300

aactcaggag gccgccagat gccgggacat ttcggacaaa agaaaaaccg cattgtgacg     360

ggatcatctc cggttacaac gcaagtgccg cacgcagtcg gtattgcgct tgcgggacgt     420

atggagaaaa aggatatcgc agcctttgtt acattcgggg aagggtcttc aaaccaaggc     480

gatttccatg aaggggcaaa ctttgccgct gtccataagc tgccggttat tttcatgtgt     540

gaaaacaaca aatacgcaat ctcagtgcct tacgataagc aagtcgcatg tgagaacatt     600

tccgaccgtg ccataggcta tgggatgcct ggcgtaactg tgaatggaaa tgatccgctg     660

gaagtttatc aagcggttaa agaagcacgc gaaagggcac gcagaggaga aggcccgaca     720

ttaattgaaa cgatttctta ccgccttaca ccacattcca gtgatgacga tgacagcagc     780

tacagaggcc gtgaagaagt agaggaagcg aaaaaaagtg atcccctgct tacttatcaa     840

gcttacttaa aggaaacagg cctgctgtcc gatgagatag aacaaaccat gctggatgaa     900

attatggcaa tcgtaaatga agcgacggat gaagcggaga acgccccata tgcagctcct     960

gagtcagcgc ttgattatgt ttatgcgaag tag                                  993

<210>  206
<211>  330
<212>  PRT
<213>  Bacillus subtilis

<400>  206

Met Ser Thr Asn Arg His Gln Ala Leu Gly Leu Thr Asp Gln Glu Ala
1               5                   10                  15

Val Asp Met Tyr Arg Thr Met Leu Leu Ala Arg Lys Ile Asp Glu Arg
            20                  25                  30

Met Trp Leu Leu Asn Arg Ser Gly Lys Ile Pro Phe Val Ile Ser Cys
        35                  40                  45

Gln Gly Gln Glu Ala Ala Gln Val Gly Ala Ala Phe Ala Leu Asp Arg
    50                  55                  60

Glu Met Asp Tyr Val Leu Pro Tyr Tyr Arg Asp Met Gly Val Val Leu
65                  70                  75                  80

Ala Phe Gly Met Thr Ala Lys Asp Leu Met Met Ser Gly Phe Ala Lys
                85                  90                  95

Ala Ala Asp Pro Asn Ser Gly Gly Arg Gln Met Pro Gly His Phe Gly
            100                 105                 110

Gln Lys Lys Asn Arg Ile Val Thr Gly Ser Ser Pro Val Thr Thr Gln
        115                 120                 125

Val Pro His Ala Val Gly Ile Ala Leu Ala Gly Arg Met Glu Lys Lys
    130                 135                 140

Asp Ile Ala Ala Phe Val Thr Phe Gly Glu Gly Ser Ser Asn Gln Gly
145                 150                 155                 160

Asp Phe His Glu Gly Ala Asn Phe Ala Ala Val His Lys Leu Pro Val
                165                 170                 175

Ile Phe Met Cys Glu Asn Asn Lys Tyr Ala Ile Ser Val Pro Tyr Asp
            180                 185                 190

Lys Gln Val Ala Cys Glu Asn Ile Ser Asp Arg Ala Ile Gly Tyr Gly
        195                 200                 205

Met Pro Gly Val Thr Val Asn Gly Asn Asp Pro Leu Glu Val Tyr Gln
    210                 215                 220

Ala Val Lys Glu Ala Arg Glu Arg Ala Arg Arg Gly Glu Gly Pro Thr
225                 230                 235                 240

Leu Ile Glu Thr Ile Ser Tyr Arg Leu Thr Pro His Ser Ser Asp Asp
                245                 250                 255

Asp Asp Ser Ser Tyr Arg Gly Arg Glu Glu Val Glu Glu Ala Lys Lys
            260                 265                 270

Ser Asp Pro Leu Leu Thr Tyr Gln Ala Tyr Leu Lys Glu Thr Gly Leu
        275                 280                 285

Leu Ser Asp Glu Ile Glu Gln Thr Met Leu Asp Glu Ile Met Ala Ile

290                 295                 300

Val Asn Glu Ala Thr Asp Glu Ala Glu Asn Ala Pro Tyr Ala Ala Pro
305                 310                 315                 320

Glu Ser Ala Leu Asp Tyr Val Tyr Ala Lys
                325                 330

<210>  207
<211>  984
<212>  DNA
<213>  Bacillus subtilis

<400>  207
atgtcagtaa tgtcatatat tgatgcaatc aatttggcga tgaaagaaga aatggaacga      60

gattctcgcg ttttcgtcct tggggaagat gtaggaagaa aaggcggtgt gtttaaagcg     120

acagcgggac tctatgaaca atttggggaa gagcgcgtta tggatacgcc gcttgctgaa     180

tctgcaatcg caggagtcgg tatcggagcg gcaatgtacg gaatgagacc gattgctgaa     240

atgcagtttg ctgatttcat tatgccggca gtcaaccaaa ttatttctga agcggctaaa     300

atccgctacc gcagcaacaa tgactggagc tgtccgattg tcgtcagagc gccatacggc     360

ggaggcgtgc acggagccct gtatcattct caatcagtcg aagcaatttt cgccaaccag     420

cccggactga aaattgtcat gccatcaaca ccatatgacg cgaaagggct cttaaaagcc     480

gcagttcgtg acgaagaccc cgtgctgttt tttgagcaca agcgggcata ccgtctgata     540

aagggcgagg ttccggctga tgattatgtc ctgccaatcg gcaaggcgga cgtaaaaagg     600

gaaggcgacg acatcacagt gatcacatac ggcctgtgtg tccacttcgc cttacaagct     660

gcagaacgtc tcgaaaaaga tggcatttca gcgcatgtgg tggatttaag aacagtttac     720

ccgcttgata aagaagccat catcgaagct gcgtccaaaa ctggaaaggt tcttttggtc     780

acagaagata caaaagaagg cagcatcatg agcgaagtag ccgcaattat atccgagcat     840

tgtctgttcg acttagacgc gccgatcaaa cggcttgcag gtcctgatat tccggctatg     900

ccttatgcgc cgacaatgga aaaatacttt atggtcaacc ctgataaagt ggaagcggcg     960

atgagagaat tagcggagtt ttaa                                            984

<210>  208
<211>  327
<212>  PRT
<213>  Bacillus subtilis

<400>  208

Met Ser Val Met Ser Tyr Ile Asp Ala Ile Asn Leu Ala Met Lys Glu
1               5                   10                  15

Glu Met Glu Arg Asp Ser Arg Val Phe Val Leu Gly Glu Asp Val Gly
            20                  25                  30

Arg Lys Gly Gly Val Phe Lys Ala Thr Ala Gly Leu Tyr Glu Gln Phe
        35                  40                  45

Gly Glu Glu Arg Val Met Asp Thr Pro Leu Ala Glu Ser Ala Ile Ala
    50                  55                  60

Gly Val Gly Ile Gly Ala Ala Met Tyr Gly Met Arg Pro Ile Ala Glu
65                  70                  75                  80

Met Gln Phe Ala Asp Phe Ile Met Pro Ala Val Asn Gln Ile Ile Ser
                85                  90                  95

Glu Ala Ala Lys Ile Arg Tyr Arg Ser Asn Asn Asp Trp Ser Cys Pro
            100                 105                 110

Ile Val Val Arg Ala Pro Tyr Gly Gly Gly Val His Gly Ala Leu Tyr
        115                 120                 125

His Ser Gln Ser Val Glu Ala Ile Phe Ala Asn Gln Pro Gly Leu Lys
    130                 135                 140

Ile Val Met Pro Ser Thr Pro Tyr Asp Ala Lys Gly Leu Leu Lys Ala
145                 150                 155                 160

Ala Val Arg Asp Glu Asp Pro Val Leu Phe Phe Glu His Lys Arg Ala
                165                 170                 175

Tyr Arg Leu Ile Lys Gly Glu Val Pro Ala Asp Asp Tyr Val Leu Pro
            180                 185                 190

Ile Gly Lys Ala Asp Val Lys Arg Glu Gly Asp Asp Ile Thr Val Ile
        195                 200                 205

Thr Tyr Gly Leu Cys Val His Phe Ala Leu Gln Ala Ala Glu Arg Leu
    210                 215                 220

Glu Lys Asp Gly Ile Ser Ala His Val Val Asp Leu Arg Thr Val Tyr
225                 230                 235                 240

Pro Leu Asp Lys Glu Ala Ile Ile Glu Ala Ala Ser Lys Thr Gly Lys
                245                 250                 255

Val Leu Leu Val Thr Glu Asp Thr Lys Glu Gly Ser Ile Met Ser Glu
            260                 265                 270

Val Ala Ala Ile Ile Ser Glu His Cys Leu Phe Asp Leu Asp Ala Pro
        275                 280                 285

Ile Lys Arg Leu Ala Gly Pro Asp Ile Pro Ala Met Pro Tyr Ala Pro
    290                 295                 300

Thr Met Glu Lys Tyr Phe Met Val Asn Pro Asp Lys Val Glu Ala Ala
305                 310                 315                 320

Met Arg Glu Leu Ala Glu Phe
                325

<210>  209
<211>  1275
<212>  DNA
<213>  Bacillus subtilis

<400>  209
   atggcaattg aacaaatgac gatgccgcag cttggagaaa gcgtaacaga ggggacgatc      60
   agcaaatggc ttgtcgcccc cggtgataaa gtgaacaaat acgatccgat cgcggaagtc     120
   atgacagata aggtaaatgc agaggttccg tcttctttta ctggtacgat aacagagctt     180
   gtgggagaag aaggccaaac cctgcaagtc ggagaaatga tttgcaaaat tgaaacagaa     240
   ggcgcgaatc cggctgaaca aaaacaagaa cagccagcag catcagaagc cgctgagaac     300
   cctgttgcaa aaagtgctgg agcagccgat cagcccaata aaaagcgcta ctcgccagct     360
   gttctccgtt tggccggaga gcacggcatt gacctcgatc aagtgacagg aactggtgcc     420
   ggcgggcgca tcacacgaaa agatattcag cgcttaattg aaacaggcgg cgtgcaagaa     480
   cagaatcctg aggagctgaa aacagcagct cctgcaccga agtctgcatc aaaacctgag     540
   ccaaaagaag agacgtcata tcctgcgtct gcagccggtg ataaagaaat ccctgtcaca     600
   ggtgtaagaa aagcaattgc ttccaatatg aagcgaagca aaacagaaat tccgcatgct     660
   tggacgatga tggaagtcga cgtcacaaat atggttgcat atcgcaacag tataaaagat     720
   tcttttaaga agacagaagg ctttaattta acgttcttcg ccttttttgt aaaagcggtc     780
   gctcaggcgt taaaagaatt cccgcaaatg aatagcatgt gggcggggga caaaattatt     840
   cagaaaaagg atatcaatat ttcaattgca gttgccacag aggattcttt atttgttccg     900
   gtgattaaaa acgctgatga aaaaacaatt aaaggcattg cgaaagacat taccggccta     960
   gctaaaaaag taagagacgg aaaactcact gcagatgaca tgcagggagg cacgtttacc    1020
   gtcaacaaca caggttcgtt cgggtctgtt cagtcgatgg gcattatcaa ctaccctcag    1080
   gctgcgattc ttcaagtaga atccatcgtc aaacgcccgg ttgtcatgga caatggcatg    1140
   attgctgtca gagacatggt taatctgtgc ctgtcattag atcacagagt gcttgacggt    1200
   ctcgtgtgcg gacgattcct cggacgagtg aaacaaattt tagaatcgat tgacgagaag    1260
   acatctgttt actaa                                                     1275

<210>  210

<211>  424
<212>  PRT
<213>  Bacillus subtilis

<400>  210

Met Ala Ile Glu Gln Met Thr Met Pro Gln Leu Gly Glu Ser Val Thr
1               5                   10                  15

Glu Gly Thr Ile Ser Lys Trp Leu Val Ala Pro Gly Asp Lys Val Asn
            20                  25                  30

Lys Tyr Asp Pro Ile Ala Glu Val Met Thr Asp Lys Val Asn Ala Glu
        35                  40                  45

Val Pro Ser Ser Phe Thr Gly Thr Ile Thr Glu Leu Val Gly Glu Glu
    50                  55                  60

Gly Gln Thr Leu Gln Val Gly Glu Met Ile Cys Lys Ile Glu Thr Glu
65                  70                  75                  80

Gly Ala Asn Pro Ala Glu Gln Lys Gln Glu Gln Pro Ala Ala Ser Glu
                85                  90                  95

Ala Ala Glu Asn Pro Val Ala Lys Ser Ala Gly Ala Ala Asp Gln Pro
            100                 105                 110

Asn Lys Lys Arg Tyr Ser Pro Ala Val Leu Arg Leu Ala Gly Glu His
        115                 120                 125

Gly Ile Asp Leu Asp Gln Val Thr Gly Thr Gly Ala Gly Gly Arg Ile
    130                 135                 140

Thr Arg Lys Asp Ile Gln Arg Leu Ile Glu Thr Gly Gly Val Gln Glu
145                 150                 155                 160

Gln Asn Pro Glu Glu Leu Lys Thr Ala Ala Pro Ala Pro Lys Ser Ala
                165                 170                 175

Ser Lys Pro Glu Pro Lys Glu Glu Thr Ser Tyr Pro Ala Ser Ala Ala
            180                 185                 190

Gly Asp Lys Glu Ile Pro Val Thr Gly Val Arg Lys Ala Ile Ala Ser
        195                 200                 205

Asn Met Lys Arg Ser Lys Thr Glu Ile Pro His Ala Trp Thr Met Met
    210                 215                 220

Glu Val Asp Val Thr Asn Met Val Ala Tyr Arg Asn Ser Ile Lys Asp
225                 230                 235                 240

Ser Phe Lys Lys Thr Glu Gly Phe Asn Leu Thr Phe Phe Ala Phe Phe
                245                 250                 255

Val Lys Ala Val Ala Gln Ala Leu Lys Glu Phe Pro Gln Met Asn Ser
            260                 265                 270

Met Trp Ala Gly Asp Lys Ile Ile Gln Lys Lys Asp Ile Asn Ile Ser
        275                 280                 285

Ile Ala Val Ala Thr Glu Asp Ser Leu Phe Val Pro Val Ile Lys Asn
    290                 295                 300

Ala Asp Glu Lys Thr Ile Lys Gly Ile Ala Lys Asp Ile Thr Gly Leu
305                 310                 315                 320

Ala Lys Lys Val Arg Asp Gly Lys Leu Thr Ala Asp Asp Met Gln Gly
                325                 330                 335

Gly Thr Phe Thr Val Asn Asn Thr Gly Ser Phe Gly Ser Val Gln Ser
            340                 345                 350

Met Gly Ile Ile Asn Tyr Pro Gln Ala Ala Ile Leu Gln Val Glu Ser
        355                 360                 365

Ile Val Lys Arg Pro Val Val Met Asp Asn Gly Met Ile Ala Val Arg
    370                 375                 380

Asp Met Val Asn Leu Cys Leu Ser Leu Asp His Arg Val Leu Asp Gly
385                 390                 395                 400

Leu Val Cys Gly Arg Phe Leu Gly Arg Val Lys Gln Ile Leu Glu Ser
                405                 410                 415

Ile Asp Glu Lys Thr Ser Val Tyr
            420

<210>  211
<211>  1374
<212>  DNA
<213>  Bacillus subtilis

<400>  211
atggcaactg agtatgacgt agtcattctg ggcggcggta ccggcggtta tgttgcggcc      60

atcagagccg ctcagctcgg cttaaaaaca gccgttgtgg aaaaggaaaa actcggggga     120

acatgtctgc ataaaggctg tatcccgagt aaagcgctgc ttagaagcgc agaggtatac     180

cggacagctc gtgaagccga tcaattcgga gtggaaacgg ctggcgtgtc cctcaacttt     240

gaaaaagtgc agcagcgtaa gcaagccgtt gttgataagc ttgcagcggg tgtaaatcat     300

ttaatgaaaa aaggaaaaat tgacgtgtac accggatatg gacgtatcct tggaccgtca 360

atcttctctc cgctgccggg aacaatttct gttgagcggg gaaatggcga agaaaatgac 420

atgctgatcc cgaaacaagt gatcattgca acaggatcaa gaccgagaat gcttccgggt 480

cttgaagtgg acggtaagtc tgtactgact tcagatgagg cgctccaaat ggaggagctg 540

ccacagtcaa tcatcattgt cggcggaggg gttatcggta tcgaatgggc gtctatgctt 600

catgattttg gcgttaaggt aacggttatt gaatacgcgg atcgcatatt gccgactgaa 660

gatctagaga tttcaaaaga aatggaaagt cttcttaaga aaaaaggcat ccagttcata 720

acaggggcaa aagtgctgcc tgacacaatg acaaaaacat cagacgatat cagcatacaa 780

gcggaaaaag acggagaaac cgttacctat tctgctgaga aaatgcttgt ttccatcggc 840

agacaggcaa atatcgaagg catcggccta gagaacaccg atattgttac tgaaaatggc 900

atgatttcag tcaatgaaag ctgccaaacg aaggaatctc atatttatgc aatcggagac 960

gtaatcggtg gcctgcagtt agctcacgtt gcttcacatg agggaattat tgctgttgag 1020

cattttgcag gtctcaatcc gcatccgctt gatccgacgc ttgtgccgaa gtgcatttac 1080

tcaagccctg aagctgccag tgtcggctta accgaagacg aagcaaaggc gaacgggcat 1140

aatgtcaaaa tcggcaagtt cccatttatg gcgattggaa aagcgcttgt atacggtgaa 1200

agcgacggtt ttgtcaaaat cgtggctgac cgagatacag atgatattct cggcgttcat 1260

atgattggcc cgcatgtcac cgacatgatt tctgaagcgg gtcttgccaa agtgctggac 1320

gcaacaccgt gggaggtcgg gcaaacgatt tcacccgcat ccaacgcttt ctga 1374

<210> 212
<211> 457
<212> PRT
<213> Bacillus subtilis

<400> 212

Met Ala Thr Glu Tyr Asp Val Val Ile Leu Gly Gly Gly Thr Gly Gly
1               5                   10                  15

Tyr Val Ala Ala Ile Arg Ala Ala Gln Leu Gly Leu Lys Thr Ala Val
            20                  25                  30

Val Glu Lys Glu Lys Leu Gly Gly Thr Cys Leu His Lys Gly Cys Ile
        35                  40                  45

Pro Ser Lys Ala Leu Leu Arg Ser Ala Glu Val Tyr Arg Thr Ala Arg
    50                  55                  60

Glu Ala Asp Gln Phe Gly Val Glu Thr Ala Gly Val Ser Leu Asn Phe
65                  70                  75                  80

Glu Lys Val Gln Gln Arg Lys Gln Ala Val Val Asp Lys Leu Ala Ala

                85                  90                  95

Gly Val Asn His Leu Met Lys Lys Gly Lys Ile Asp Val Tyr Thr Gly
            100                 105                 110

Tyr Gly Arg Ile Leu Gly Pro Ser Ile Phe Ser Pro Leu Pro Gly Thr
        115                 120                 125

Ile Ser Val Glu Arg Gly Asn Gly Glu Glu Asn Asp Met Leu Ile Pro
    130                 135                 140

Lys Gln Val Ile Ile Ala Thr Gly Ser Arg Pro Arg Met Leu Pro Gly
145                 150                 155                 160

Leu Glu Val Asp Gly Lys Ser Val Leu Thr Ser Asp Glu Ala Leu Gln
                165                 170                 175

Met Glu Glu Leu Pro Gln Ser Ile Ile Ile Val Gly Gly Gly Val Ile
            180                 185                 190

Gly Ile Glu Trp Ala Ser Met Leu His Asp Phe Gly Val Lys Val Thr
        195                 200                 205

Val Ile Glu Tyr Ala Asp Arg Ile Leu Pro Thr Glu Asp Leu Glu Ile
    210                 215                 220

Ser Lys Glu Met Glu Ser Leu Leu Lys Lys Lys Gly Ile Gln Phe Ile
225                 230                 235                 240

Thr Gly Ala Lys Val Leu Pro Asp Thr Met Thr Lys Thr Ser Asp Asp
                245                 250                 255

Ile Ser Ile Gln Ala Glu Lys Asp Gly Glu Thr Val Thr Tyr Ser Ala
            260                 265                 270

Glu Lys Met Leu Val Ser Ile Gly Arg Gln Ala Asn Ile Glu Gly Ile
        275                 280                 285

Gly Leu Glu Asn Thr Asp Ile Val Thr Glu Asn Gly Met Ile Ser Val
    290                 295                 300

Asn Glu Ser Cys Gln Thr Lys Glu Ser His Ile Tyr Ala Ile Gly Asp
305                 310                 315                 320

Val Ile Gly Gly Leu Gln Leu Ala His Val Ala Ser His Glu Gly Ile
                325                 330                 335

Ile Ala Val Glu His Phe Ala Gly Leu Asn Pro His Pro Leu Asp Pro
            340                 345                 350

Thr Leu Val Pro Lys Cys Ile Tyr Ser Ser Pro Glu Ala Ala Ser Val
        355                 360                 365

Gly Leu Thr Glu Asp Glu Ala Lys Ala Asn Gly His Asn Val Lys Ile
    370                 375                 380

Gly Lys Phe Pro Phe Met Ala Ile Gly Lys Ala Leu Val Tyr Gly Glu
385                 390                 395                 400

Ser Asp Gly Phe Val Lys Ile Val Ala Asp Arg Asp Thr Asp Asp Ile
                405                 410                 415

Leu Gly Val His Met Ile Gly Pro His Val Thr Asp Met Ile Ser Glu
            420                 425                 430

Ala Gly Leu Ala Lys Val Leu Asp Ala Thr Pro Trp Glu Val Gly Gln
        435                 440                 445

Thr Ile Ser Pro Ala Ser Asn Ala Phe
    450                 455

<210>  213
<211>  1233
<212>  DNA
<213>  Pseudomonas putida

<400>  213
atgaacgagt acgcccccct gcgtttgcat gtgcccgagc ccaccggccg gccaggctgc      60

cagaccgatt tttcctacct gcgcctgaac gatgcaggtc aagcccgtaa accccctgtc     120

gatgtcgacg ctgccgacac cgccgacctg tcctacagcc tggtccgcgt gctcgacgag     180

caaggcgacg cccaaggccc gtgggctgaa gacatcgacc cgcagatcct gcgccaaggc     240

atgcgcgcca tgctcaagac gcggatcttc gacagccgca tggtggttgc ccagcgccag     300

aagaagatgt ccttctacat gcagagcctg ggcgaagaag ccatcggcag cggccaggcg     360

ctggcgctta accgcaccga catgtgcttc cccacctacc gtcagcaaag catcctgatg     420

gcccgcgacg tgtcgctggt ggagatgatc tgccagttgc tgtccaacga acgcgacccc     480

ctcaagggcc gccagctgcc gatcatgtac tcggtacgcg aggccggctt cttcaccatc     540

agcggcaacc tggcgaccca gttcgtgcag gcggtcggct gggccatggc ctcggcgatc     600

aagggcgata ccaagattgc ctcggcctgg atcggcgacg gcgccactgc cgaatcggac     660

ttccacaccg ccctcacctt tgcccacgtt taccgcgccc cggtgatcct caacgtggtc     720

aacaaccagt gggccatctc aaccttccag gccatcgccg gtggcgagtc gaccaccttc     780

gccggccgtg gcgtgggctg cggcatcgct tcgctgcggg tggacggcaa cgacttcgtc     840

gccgtttacg ccgcttcgcg ctgggctgcc gaacgtgccc gccgtggttt gggcccgagc     900

ctgatcgagt gggtcaccta ccgtgccggc ccgcactcga cctcggacga cccgtccaag 960

taccgccctg ccgatgactg gagccacttc ccgctgggtg acccgatcgc ccgcctgaag 1020

cagcacctga tcaagatcgg ccactggtcc gaagaagaac accaggccac cacggccgag 1080

ttcgaagcgg ccgtgattgc tgcgcaaaaa gaagccgagc agtacggcac cctggccaac 1140

ggtcacatcc cgagcgccgc ctcgatgttc gaggacgtgt acaaggagat gcccgaccac 1200

ctgcgccgcc aacgccagga actgggggtt tga 1233

<210> 214
<211> 410
<212> PRT
<213> Pseudomonas putida

<400> 214

Met Asn Glu Tyr Ala Pro Leu Arg Leu His Val Pro Glu Pro Thr Gly
1               5                   10                  15

Arg Pro Gly Cys Gln Thr Asp Phe Ser Tyr Leu Arg Leu Asn Asp Ala
            20                  25                  30

Gly Gln Ala Arg Lys Pro Pro Val Asp Val Asp Ala Ala Asp Thr Ala
        35                  40                  45

Asp Leu Ser Tyr Ser Leu Val Arg Val Leu Asp Glu Gln Gly Asp Ala
    50                  55                  60

Gln Gly Pro Trp Ala Glu Asp Ile Asp Pro Gln Ile Leu Arg Gln Gly
65                  70                  75                  80

Met Arg Ala Met Leu Lys Thr Arg Ile Phe Asp Ser Arg Met Val Val
                85                  90                  95

Ala Gln Arg Gln Lys Lys Met Ser Phe Tyr Met Gln Ser Leu Gly Glu
            100                 105                 110

Glu Ala Ile Gly Ser Gly Gln Ala Leu Ala Leu Asn Arg Thr Asp Met
        115                 120                 125

Cys Phe Pro Thr Tyr Arg Gln Gln Ser Ile Leu Met Ala Arg Asp Val
    130                 135                 140

Ser Leu Val Glu Met Ile Cys Gln Leu Leu Ser Asn Glu Arg Asp Pro
145                 150                 155                 160

Leu Lys Gly Arg Gln Leu Pro Ile Met Tyr Ser Val Arg Glu Ala Gly
                165                 170                 175

Phe Phe Thr Ile Ser Gly Asn Leu Ala Thr Gln Phe Val Gln Ala Val
            180                 185                 190

Gly Trp Ala Met Ala Ser Ala Ile Lys Gly Asp Thr Lys Ile Ala Ser
        195                 200                 205

Ala Trp Ile Gly Asp Gly Ala Thr Ala Glu Ser Asp Phe His Thr Ala
    210                 215                 220

Leu Thr Phe Ala His Val Tyr Arg Ala Pro Val Ile Leu Asn Val Val
225                 230                 235                 240

Asn Asn Gln Trp Ala Ile Ser Thr Phe Gln Ala Ile Ala Gly Gly Glu
                245                 250                 255

Ser Thr Thr Phe Ala Gly Arg Gly Val Gly Cys Gly Ile Ala Ser Leu
            260                 265                 270

Arg Val Asp Gly Asn Asp Phe Val Ala Val Tyr Ala Ala Ser Arg Trp
        275                 280                 285

Ala Ala Glu Arg Ala Arg Arg Gly Leu Gly Pro Ser Leu Ile Glu Trp
    290                 295                 300

Val Thr Tyr Arg Ala Gly Pro His Ser Thr Ser Asp Asp Pro Ser Lys
305                 310                 315                 320

Tyr Arg Pro Ala Asp Asp Trp Ser His Phe Pro Leu Gly Asp Pro Ile
                325                 330                 335

Ala Arg Leu Lys Gln His Leu Ile Lys Ile Gly His Trp Ser Glu Glu
            340                 345                 350

Glu His Gln Ala Thr Thr Ala Glu Phe Glu Ala Ala Val Ile Ala Ala
        355                 360                 365

Gln Lys Glu Ala Glu Gln Tyr Gly Thr Leu Ala Asn Gly His Ile Pro
    370                 375                 380

Ser Ala Ala Ser Met Phe Glu Asp Val Tyr Lys Glu Met Pro Asp His
385                 390                 395                 400

Leu Arg Arg Gln Arg Gln Glu Leu Gly Val
                405                 410

<210>  215
<211>  1059
<212>  DNA
<213>  Pseudomonas putida

<400>  215
atgaacgacc acaacaacag catcaacccg gaaaccgcca tggccaccac taccatgacc      60

atgatccagg ccctgcgctc ggccatggat gtcatgcttg agcgcgacga caatgtggtg     120

gtgtacggcc aggacgtcgg ctacttcggc ggcgtgttcc gctgcaccga aggcctgcag     180

accaagtacg gcaagtcccg cgtgttcgac gcgcccatct ctgaaagcgg catcgtcggc     240

accgccgtgg gcatgggtgc ctacggcctg cgcccggtgg tggaaatcca gttcgctgac     300

tacttctacc cggcctccga ccagatcgtt tctgaaatgg cccgcctgcg ctaccgttcg     360

gccggcgagt tcatcgcccc gctgaccctg cgtatgccct gcggtggcgg tatctatggc     420

ggccagacac acagccagag cccggaagcg atgttcactc aggtgtgcgg cctgcgcacc     480

gtaatgccat ccaacccgta cgacgccaaa ggcctgctga ttgcctcgat cgaatgcgac     540

gacccggtga tcttcctgga gcccaagcgc ctgtacaacg gcccgttcga cggccaccat     600

gaccgcccgg ttacgccgtg gtcgaaacac ccgcacagcg ccgtgcccga tggctactac     660

accgtgccac tggacaaggc cgccatcacc cgccccggca atgacgtgag cgtgctcacc     720

tatggcacca ccgtgtacgt ggcccaggtg gccgccgaag aaagtggcgt ggatgccgaa     780

gtgatcgacc tgcgcagcct gtggccgcta gacctggaca ccatcgtcga gtcggtgaaa     840

aagaccggcc gttgcgtggt agtacacgag gccacccgta cttgtggctt tggcgcagaa     900

ctggtgtcgc tggtgcagga gcactgcttc caccacctgg aggcgccgat cgagcgcgtc     960

accggttggg acacccccta ccctcacgcg caggaatggg cttacttccc agggccttcg    1020

cgggtaggtg cggcattgaa aaaggtcatg gaggtctga                            1059

<210>  216
<211>  352
<212>  PRT
<213>  Pseudomonas putida

<400>  216

Met Asn Asp His Asn Asn Ser Ile Asn Pro Glu Thr Ala Met Ala Thr
1               5                   10                  15

Thr Thr Met Thr Met Ile Gln Ala Leu Arg Ser Ala Met Asp Val Met
            20                  25                  30

Leu Glu Arg Asp Asp Asn Val Val Val Tyr Gly Gln Asp Val Gly Tyr
        35                  40                  45

Phe Gly Gly Val Phe Arg Cys Thr Glu Gly Leu Gln Thr Lys Tyr Gly
    50                  55                  60

Lys Ser Arg Val Phe Asp Ala Pro Ile Ser Glu Ser Gly Ile Val Gly
65                  70                  75                  80

Thr Ala Val Gly Met Gly Ala Tyr Gly Leu Arg Pro Val Val Glu Ile
                85                  90                  95

Gln Phe Ala Asp Tyr Phe Tyr Pro Ala Ser Asp Gln Ile Val Ser Glu
            100                 105                 110

Met Ala Arg Leu Arg Tyr Arg Ser Ala Gly Glu Phe Ile Ala Pro Leu
        115                 120                 125

Thr Leu Arg Met Pro Cys Gly Gly Gly Ile Tyr Gly Gly Gln Thr His
    130                 135                 140

Ser Gln Ser Pro Glu Ala Met Phe Thr Gln Val Cys Gly Leu Arg Thr
145                 150                 155                 160

Val Met Pro Ser Asn Pro Tyr Asp Ala Lys Gly Leu Leu Ile Ala Ser
                165                 170                 175

Ile Glu Cys Asp Asp Pro Val Ile Phe Leu Glu Pro Lys Arg Leu Tyr
            180                 185                 190

Asn Gly Pro Phe Asp Gly His His Asp Arg Pro Val Thr Pro Trp Ser
        195                 200                 205

Lys His Pro His Ser Ala Val Pro Asp Gly Tyr Tyr Thr Val Pro Leu
    210                 215                 220

Asp Lys Ala Ala Ile Thr Arg Pro Gly Asn Asp Val Ser Val Leu Thr
225                 230                 235                 240

Tyr Gly Thr Thr Val Tyr Val Ala Gln Val Ala Ala Glu Glu Ser Gly
                245                 250                 255

Val Asp Ala Glu Val Ile Asp Leu Arg Ser Leu Trp Pro Leu Asp Leu
            260                 265                 270

Asp Thr Ile Val Glu Ser Val Lys Lys Thr Gly Arg Cys Val Val Val
        275                 280                 285

His Glu Ala Thr Arg Thr Cys Gly Phe Gly Ala Glu Leu Val Ser Leu
    290                 295                 300

Val Gln Glu His Cys Phe His His Leu Glu Ala Pro Ile Glu Arg Val
305                 310                 315                 320

Thr Gly Trp Asp Thr Pro Tyr Pro His Ala Gln Glu Trp Ala Tyr Phe
                325                 330                 335

Pro Gly Pro Ser Arg Val Gly Ala Ala Leu Lys Lys Val Met Glu Val

340                 345                 350

<210>  217
<211>  1272
<212>  DNA
<213>  Pseudomonas putida

<400>  217
atgggcacgc acgtcatcaa gatgccggac attggcgaag gcatcgcgca ggtcgaattg      60

gtggaatggt tcgtcaaggt gggcgacatc atcgccgagg accaagtggt agccgacgtc     120

atgaccgaca aggccaccgt ggaaatcccg tcgccggtca gcggcaaggt gctggccctg     180

ggtggccagc caggtgaagt gatggcggtc ggcagtgagc tgatccgcat cgaagtggaa     240

ggcagcggca accatgtgga tgtgccgcaa gccaagccgg ccgaagtgcc tgcggcaccg     300

gtagccgcta aacctgaacc acagaaagac gttaaaccgg cggcgtacca ggcgtcagcc     360

agccacgagg cagcgcccat cgtgccgcgc cagccgggcg acaagccgct ggcctcgccg     420

gcggtgcgca aacgcgccct cgatgccggc atcgaattgc gttatgtgca cggcagcggc     480

ccggccgggc gcatcctgca cgaagacctc gacgcgttca tgagcaaacc gcaaagcgct     540

gccgggcaaa cccccaatgg ctatgccagg cgcaccgaca gcgagcaggt gccggtgatc     600

ggcctgcgcc gcaagatcgc ccagcgcatg caggacgcca agcgccgggt cgcgcacttc     660

agctatgtgg aagaaatcga cgtcaccgcc ctggaagccc tgcgccagca gctcaacagc     720

aagcacggcg acagccgcgg caagctgaca ctgctgccgt tcctggtgcg cgccctggtc     780

gtggcactgc gtgacttccc gcagataaac gccacctacg atgacgaagc gcagatcatc     840

acccgccatg gcgcggtgca tgtgggcatc gccacccaag gtgacaacgg cctgatggta     900

cccgtgctgc gccacgccga agcgggcagc ctgtgggcca atgccggtga gatttcacgc     960

ctggccaacg ctgcgcgcaa caacaaggcc agccgcgaag agctgtccgg ttcgaccatt    1020

accctgacca gcctcggcgc cctgggcggc atcgtcagca cgccggtggt caacaccccg    1080

gaagtggcga tcgtcggtgt caaccgcatg gttgagcggc ccgtggtgat cgacggccag    1140

atcgtcgtgc gcaagatgat gaacctgtcc agctcgttcg accaccgcgt ggtcgatggc    1200

atggacgccg ccctgttcat ccaggccgtg cgtggcctgc tcgaacaacc cgcctgcctg    1260

ttcgtggagt ga                                                        1272

<210>  218
<211>  423
<212>  PRT
<213>  Pseudomonas putida

<400>  218

Met Gly Thr His Val Ile Lys Met Pro Asp Ile Gly Glu Gly Ile Ala
1               5                   10                  15

Gln Val Glu Leu Val Glu Trp Phe Val Lys Val Gly Asp Ile Ile Ala
            20                  25                  30

Glu Asp Gln Val Val Ala Asp Val Met Thr Asp Lys Ala Thr Val Glu
        35                  40                  45

Ile Pro Ser Pro Val Ser Gly Lys Val Leu Ala Leu Gly Gly Gln Pro
    50                  55                  60

Gly Glu Val Met Ala Val Gly Ser Glu Leu Ile Arg Ile Glu Val Glu
65                  70                  75                  80

Gly Ser Gly Asn His Val Asp Val Pro Gln Ala Lys Pro Ala Glu Val
                85                  90                  95

Pro Ala Ala Pro Val Ala Ala Lys Pro Glu Pro Gln Lys Asp Val Lys
            100                 105                 110

Pro Ala Ala Tyr Gln Ala Ser Ala Ser His Glu Ala Ala Pro Ile Val
        115                 120                 125

Pro Arg Gln Pro Gly Asp Lys Pro Leu Ala Ser Pro Ala Val Arg Lys
    130                 135                 140

Arg Ala Leu Asp Ala Gly Ile Glu Leu Arg Tyr Val His Gly Ser Gly
145                 150                 155                 160

Pro Ala Gly Arg Ile Leu His Glu Asp Leu Asp Ala Phe Met Ser Lys
                165                 170                 175

Pro Gln Ser Ala Ala Gly Gln Thr Pro Asn Gly Tyr Ala Arg Arg Thr
            180                 185                 190

Asp Ser Glu Gln Val Pro Val Ile Gly Leu Arg Arg Lys Ile Ala Gln
        195                 200                 205

Arg Met Gln Asp Ala Lys Arg Arg Val Ala His Phe Ser Tyr Val Glu
    210                 215                 220

Glu Ile Asp Val Thr Ala Leu Glu Ala Leu Arg Gln Gln Leu Asn Ser
225                 230                 235                 240

Lys His Gly Asp Ser Arg Gly Lys Leu Thr Leu Leu Pro Phe Leu Val
                245                 250                 255

Arg Ala Leu Val Val Ala Leu Arg Asp Phe Pro Gln Ile Asn Ala Thr
            260                 265                 270

Tyr Asp Asp Glu Ala Gln Ile Ile Thr Arg His Gly Ala Val His Val

275                 280                 285

Gly Ile Ala Thr Gln Gly Asp Asn Gly Leu Met Val Pro Val Leu Arg
    290                 295                 300

His Ala Glu Ala Gly Ser Leu Trp Ala Asn Ala Gly Glu Ile Ser Arg
305                 310                 315                 320

Leu Ala Asn Ala Ala Arg Asn Asn Lys Ala Ser Arg Glu Glu Leu Ser
                325                 330                 335

Gly Ser Thr Ile Thr Leu Thr Ser Leu Gly Ala Leu Gly Gly Ile Val
            340                 345                 350

Ser Thr Pro Val Val Asn Thr Pro Glu Val Ala Ile Val Gly Val Asn
        355                 360                 365

Arg Met Val Glu Arg Pro Val Val Ile Asp Gly Gln Ile Val Val Arg
    370                 375                 380

Lys Met Met Asn Leu Ser Ser Ser Phe Asp His Arg Val Val Asp Gly
385                 390                 395                 400

Met Asp Ala Ala Leu Phe Ile Gln Ala Val Arg Gly Leu Leu Glu Gln
                405                 410                 415

Pro Ala Cys Leu Phe Val Glu
            420

<210>  219
<211>  1380
<212>  DNA
<213>  Pseudomonas putida

<400>  219
atgcaacaga ctatccagac aaccctgttg atcatcggcg gcggccctgg cggctatgtg      60

gcggccatcc gcgccgggca actgggcatc cctaccgtgc tggtggaagg ccaggcgctg     120

ggcggtacct gcctgaacat cggctgcatt ccgtccaagg cgctgatcca tgtggccgag     180

cagttccacc aggcctcgcg ctttaccgaa ccctcgccgc tgggcatcag cgtggcttcg     240

ccacgcctgg acatcggcca gagcgtggcc tggaaagacg gcatcgtcga tcgcctgacc     300

actggtgtcg ccgccctgct gaaaaagcac ggggtgaagg tggtgcacgg ctgggccaag     360

gtgcttgatg gcaagcaggt cgaggtggat ggccagcgca tccagtgcga gcacctgttg     420

ctggccacgg gctccagcag tgtcgaactg ccgatgctgc cgttgggtgg gccggtgatt     480

tcctcgaccg aggccctggc accgaaagcc ctgccgcaac acctggtggt ggtgggcggt     540

ggctacatcg gcctggagct gggtatcgcc taccgcaagc tcggcgcgca ggtcagcgtg     600

gtggaagcgc gcgagcgcat cctgccgact tacgacagcg aactgaccgc cccggtggcc 660

gagtcgctga aaaagctggg tatcgccctg caccttggcc acagcgtcga aggttacgaa 720

aatggctgcc tgctggccaa cgatggcaag ggcggacaac tgcgcctgga agccgaccgg 780

gtgctggtgg ccgtgggccg ccgcccacgc accaagggct tcaacctgga atgcctggac 840

ctgaagatga atggtgccgc gattgccatc gacgagcgct gccagaccag catgcacaac 900

gtctgggcca tcggcgacgt ggccggcgaa ccgatgctgg cgcaccgggc catggcccag 960

ggcgagatgg tggccgagat catcgccggc aaggcacgcc gcttcgaacc cgctgcgata 1020

gccgccgtgt gcttcaccga cccggaagtg gtcgtggtcg gcaagacgcc ggaacaggcc 1080

agtcagcaag gcctggactg catcgtcgcg cagttcccgt tcgccgccaa cggccgggcc 1140

atgagcctgg agtcgaaaag cggtttcgtg cgcgtggtcg cgcggcgtga caaccacctg 1200

atcctgggct ggcaagcggt tggcgtggcg gtttccgagc tgtccacggc gtttgcccag 1260

tcgctggaga tgggtgcctg cctggaggat gtggccggta ccatccatgc ccacccgacc 1320

ctgggtgaag cggtacagga agcggcactg cgtgccctgg gccacgccct gcatatctga 1380

<210> 220
<211> 459
<212> PRT
<213> Pseudomonas putida

<400> 220

Met Gln Gln Thr Ile Gln Thr Thr Leu Leu Ile Ile Gly Gly Gly Pro
1               5                   10                  15

Gly Gly Tyr Val Ala Ala Ile Arg Ala Gly Gln Leu Gly Ile Pro Thr
            20                  25                  30

Val Leu Val Glu Gly Gln Ala Leu Gly Gly Thr Cys Leu Asn Ile Gly
        35                  40                  45

Cys Ile Pro Ser Lys Ala Leu Ile His Val Ala Glu Gln Phe His Gln
    50                  55                  60

Ala Ser Arg Phe Thr Glu Pro Ser Pro Leu Gly Ile Ser Val Ala Ser
65                  70                  75                  80

Pro Arg Leu Asp Ile Gly Gln Ser Val Ala Trp Lys Asp Gly Ile Val
                85                  90                  95

Asp Arg Leu Thr Thr Gly Val Ala Ala Leu Leu Lys Lys His Gly Val
            100                 105                 110

Lys Val Val His Gly Trp Ala Lys Val Leu Asp Gly Lys Gln Val Glu
        115                 120                 125

Val Asp Gly Gln Arg Ile Gln Cys Glu His Leu Leu Leu Ala Thr Gly
    130                 135                 140

Ser Ser Ser Val Glu Leu Pro Met Leu Pro Leu Gly Gly Pro Val Ile
145                 150                 155                 160

Ser Ser Thr Glu Ala Leu Ala Pro Lys Ala Leu Pro Gln His Leu Val
                165                 170                 175

Val Val Gly Gly Gly Tyr Ile Gly Leu Glu Leu Gly Ile Ala Tyr Arg
            180                 185                 190

Lys Leu Gly Ala Gln Val Ser Val Val Glu Ala Arg Glu Arg Ile Leu
        195                 200                 205

Pro Thr Tyr Asp Ser Glu Leu Thr Ala Pro Val Ala Glu Ser Leu Lys
    210                 215                 220

Lys Leu Gly Ile Ala Leu His Leu Gly His Ser Val Glu Gly Tyr Glu
225                 230                 235                 240

Asn Gly Cys Leu Leu Ala Asn Asp Gly Lys Gly Gly Gln Leu Arg Leu
                245                 250                 255

Glu Ala Asp Arg Val Leu Val Ala Val Gly Arg Arg Pro Arg Thr Lys
            260                 265                 270

Gly Phe Asn Leu Glu Cys Leu Asp Leu Lys Met Asn Gly Ala Ala Ile
        275                 280                 285

Ala Ile Asp Glu Arg Cys Gln Thr Ser Met His Asn Val Trp Ala Ile
    290                 295                 300

Gly Asp Val Ala Gly Glu Pro Met Leu Ala His Arg Ala Met Ala Gln
305                 310                 315                 320

Gly Glu Met Val Ala Glu Ile Ile Ala Gly Lys Ala Arg Arg Phe Glu
                325                 330                 335

Pro Ala Ala Ile Ala Ala Val Cys Phe Thr Asp Pro Glu Val Val Val
            340                 345                 350

Val Gly Lys Thr Pro Glu Gln Ala Ser Gln Gln Gly Leu Asp Cys Ile
        355                 360                 365

Val Ala Gln Phe Pro Phe Ala Ala Asn Gly Arg Ala Met Ser Leu Glu
    370                 375                 380

Ser Lys Ser Gly Phe Val Arg Val Val Ala Arg Arg Asp Asn His Leu
385                 390                 395                 400

Ile Leu Gly Trp Gln Ala Val Gly Val Ala Val Ser Glu Leu Ser Thr
                405                 410                 415

Ala Phe Ala Gln Ser Leu Glu Met Gly Ala Cys Leu Glu Asp Val Ala
            420                 425                 430

Gly Thr Ile His Ala His Pro Thr Leu Gly Glu Ala Val Gln Glu Ala
        435                 440                 445

Ala Leu Arg Ala Leu Gly His Ala Leu His Ile
    450                 455

<210>  221
<211>  1407
<212>  DNA
<213>  Clostridium beijerinckii

<400>  221
atgaataaag acacactaat acctacaact aaagatttaa aattaaaaac aaatgttgaa      60

aacattaatt taaagaacta caaggataat tcttcatgtt tcggagtatt cgaaaatgtt     120

gaaaatgcta taaacagcgc tgtacacgcg caaaagatat tatcccttca ttatacaaaa     180

gaacaaagag aaaaaatcat aactgagata agaaaggccg cattagaaaa taaagaggtt     240

ttagctacca tgattctgga agaaacacat atgggaaggt atgaagataa aatattaaag     300

catgaattag tagctaaata tactcctggt acagaagatt taactactac tgcttggtca     360

ggtgataatg gtcttacagt tgtagaaatg tctccatatg gcgttatagg tgcaataact     420

ccttctacga atccaactga aactgtaata tgtaatagca tcggcatgat agctgctgga     480

aatgctgtag tatttaacgg acacccaggc gctaaaaaat gtgttgcttt tgctattgaa     540

atgataaata aagcaattat ttcatgtggc ggtcctgaga atttagtaac aactataaaa     600

aatccaacta tggaatccct agatgcaatt attaagcatc ctttaataaa acttctttgc     660

ggaactggag gtccaggaat ggtaaaaacc ctcttaaatt ctggcaagaa agctataggt     720

gctggtgctg gaaatccacc agttattgta gatgataccg ctgatataga aaaggctggt     780

aagagtatca ttgaaggctg ttcttttgat aataatttac cttgtattgc agaaaaagaa     840

gtatttgttt ttgagaatgt tgcagatgat ttaatatcta acatgctaaa aaataatgct     900

gtaattataa atgaagatca agtatcaaaa ttaatagatt tagtattaca aaaaaataat     960

gaaactcaag aatactttat aaacaaaaaa tgggtaggaa aagatgcaaa attattctca    1020

gatgaaatag atgttgagtc tccttcaaat attaaatgca tagtctgcga agtaaatgca    1080

aatcatccat ttgtcatgac agaactcatg atgccaatat taccaattgt aagagttaaa    1140

gatatagatg aagctgttaa atatacaaag atagcagaac aaaatagaaa acatagtgcc    1200

tatatttatt ctaaaaatat agacaaccta aatagatttg aaagagaaat tgatactact 1260

atttttgtaa agaatgctaa atcttttgct ggtgttggtt atgaagctga aggatttaca 1320

actttcacta ttgctggatc tactggtgaa ggcataacct ctgcaagaaa ttttacaaga 1380

caaagaagat gtgtacttgc cggctaa 1407

<210> 222
<211> 468
<212> PRT
<213> Clostridium beijerinckii

<400> 222

Met Asn Lys Asp Thr Leu Ile Pro Thr Thr Lys Asp Leu Lys Leu Lys
1               5                   10                  15

Thr Asn Val Glu Asn Ile Asn Leu Lys Asn Tyr Lys Asp Asn Ser Ser
            20                  25                  30

Cys Phe Gly Val Phe Glu Asn Val Glu Asn Ala Ile Asn Ser Ala Val
        35                  40                  45

His Ala Gln Lys Ile Leu Ser Leu His Tyr Thr Lys Glu Gln Arg Glu
    50                  55                  60

Lys Ile Ile Thr Glu Ile Arg Lys Ala Ala Leu Glu Asn Lys Glu Val
65                  70                  75                  80

Leu Ala Thr Met Ile Leu Glu Glu Thr His Met Gly Arg Tyr Glu Asp
                85                  90                  95

Lys Ile Leu Lys His Glu Leu Val Ala Lys Tyr Thr Pro Gly Thr Glu
            100                 105                 110

Asp Leu Thr Thr Thr Ala Trp Ser Gly Asp Asn Gly Leu Thr Val Val
        115                 120                 125

Glu Met Ser Pro Tyr Gly Val Ile Gly Ala Ile Thr Pro Ser Thr Asn
    130                 135                 140

Pro Thr Glu Thr Val Ile Cys Asn Ser Ile Gly Met Ile Ala Ala Gly
145                 150                 155                 160

Asn Ala Val Val Phe Asn Gly His Pro Gly Ala Lys Lys Cys Val Ala
                165                 170                 175

Phe Ala Ile Glu Met Ile Asn Lys Ala Ile Ile Ser Cys Gly Gly Pro
            180                 185                 190

Glu Asn Leu Val Thr Thr Ile Lys Asn Pro Thr Met Glu Ser Leu Asp
        195                 200                 205

Ala Ile Ile Lys His Pro Leu Ile Lys Leu Leu Cys Gly Thr Gly Gly
    210                 215                 220

Pro Gly Met Val Lys Thr Leu Leu Asn Ser Gly Lys Lys Ala Ile Gly
225                 230                 235                 240

Ala Gly Ala Gly Asn Pro Pro Val Ile Val Asp Asp Thr Ala Asp Ile
                245                 250                 255

Glu Lys Ala Gly Lys Ser Ile Ile Glu Gly Cys Ser Phe Asp Asn Asn
            260                 265                 270

Leu Pro Cys Ile Ala Glu Lys Glu Val Phe Val Phe Glu Asn Val Ala
        275                 280                 285

Asp Asp Leu Ile Ser Asn Met Leu Lys Asn Asn Ala Val Ile Ile Asn
    290                 295                 300

Glu Asp Gln Val Ser Lys Leu Ile Asp Leu Val Leu Gln Lys Asn Asn
305                 310                 315                 320

Glu Thr Gln Glu Tyr Phe Ile Asn Lys Lys Trp Val Gly Lys Asp Ala
                325                 330                 335

Lys Leu Phe Ser Asp Glu Ile Asp Val Glu Ser Pro Ser Asn Ile Lys
            340                 345                 350

Cys Ile Val Cys Glu Val Asn Ala Asn His Pro Phe Val Met Thr Glu
        355                 360                 365

Leu Met Met Pro Ile Leu Pro Ile Val Arg Val Lys Asp Ile Asp Glu
    370                 375                 380

Ala Val Lys Tyr Thr Lys Ile Ala Glu Gln Asn Arg Lys His Ser Ala
385                 390                 395                 400

Tyr Ile Tyr Ser Lys Asn Ile Asp Asn Leu Asn Arg Phe Glu Arg Glu
                405                 410                 415

Ile Asp Thr Thr Ile Phe Val Lys Asn Ala Lys Ser Phe Ala Gly Val
            420                 425                 430

Gly Tyr Glu Ala Glu Gly Phe Thr Thr Phe Thr Ile Ala Gly Ser Thr
        435                 440                 445

Gly Glu Gly Ile Thr Ser Ala Arg Asn Phe Thr Arg Gln Arg Arg Cys

450                 455                 460

Val Leu Ala Gly
465

<210>  223
<211>  2589
<212>  DNA
<213>  Clostridium acetobutylicum

<400>  223
atgaaagtca caacagtaaa ggaattagat gaaaaactca aggtaattaa agaagctcaa      60

aaaaaattct cttgttactc gcaagaaatg gttgatgaaa tctttagaaa tgcagcaatg     120

gcagcaatcg acgcaaggat agagctagca aaagcagctg ttttggaaac cggtatgggc     180

ttagttgaag acaaggttat aaaaaatcat tttgcaggcg aatacatcta taacaaatat     240

aaggatgaaa aaacctgcgg tataattgaa cgaaatgaac cctacggaat tacaaaaata     300

gcagaaccta taggagttgt agctgctata atccctgtaa caaaccccac atcaacaaca     360

atatttaaat ccttaatatc ccttaaaact agaaatggaa ttttcttttc gcctcaccca     420

agggcaaaaa aatccacaat actagcagct aaaacaatac ttgatgcagc cgttaagagt     480

ggtgccccgg aaaatataat aggttggata gatgaacctt caattgaact aactcaatat     540

ttaatgcaaa aagcagatat aacccttgca actggtggtc cctcactagt taaatctgct     600

tattcttccg gaaaaccagc aataggtgtt ggtccgggta acaccccagt aataattgat     660

gaatctgctc atataaaaat ggcagtaagt tcaattatat tatccaaaac ctatgataat     720

ggtgttatat gtgcttctga acaatctgta atagtcttaa aatccatata taacaaggta     780

aaagatgagt tccaagaaag aggagcttat ataataaaga aaaacgaatt ggataaagtc     840

cgtgaagtga tttttaaaga tggatccgta aaccctaaaa tagtcggaca gtcagcttat     900

actatagcag ctatggctgg cataaaagta cctaaaacca caagaatatt aataggagaa     960

gttacctcct taggtgaaga agaacctttt gcccacgaaa aactatctcc tgttttggct    1020

atgtatgagg ctgacaattt tgatgatgct ttaaaaaaag cagtaactct aataaactta    1080

ggaggcctcg gccatacctc aggaatatat gcagatgaaa taaaagcacg agataaaata    1140

gatagattta gtagtgccat gaaaaccgta agaacctttg taaatatccc aacctcacaa    1200

ggtgcaagtg gagatctata taattttaga ataccacctt ctttcacgct tggctgcgga    1260

ttttggggag gaaattctgt ttccgagaat gttggtccaa aacatctttt gaatattaaa    1320

accgtagctg aaaggagaga aaacatgctt tggtttagag ttccacataa agtatatttt    1380

aagttcggtt gtcttcaatt tgctttaaaa gatttaaaag atctaaagaa aaaaagagcc    1440

tttatagtta ctgatagtga cccctataat ttaaactatg ttgattcaat aataaaaata    1500

cttgagcacc tagatattga ttttaaagta tttaataagg ttggaagaga agctgatctt    1560

aaaaccataa aaaaagcaac tgaagaaatg tcctccttta tgccagacac tataatagct 1620

ttaggtggta cccctgaaat gagctctgca aagctaatgt gggtactata tgaacatcca 1680

gaagtaaaat ttgaagatct tgcaataaaa tttatggaca taagaaagag aatatatact 1740

ttcccaaaac tcggtaaaaa ggctatgtta gttgcaatta caacttctgc tggttccggt 1800

tctgaggtta ctccttttgc tttagtaact gacaataaca ctggaaataa gtacatgtta 1860

gcagattatg aaatgacacc aaatatggca attgtagatg cagaacttat gatgaaaatg 1920

ccaaagggat taaccgctta ttcaggtata gatgcactag taaatagtat agaagcatac 1980

acatccgtat atgcttcaga atacacaaac ggactagcac tagaggcaat acgattaata 2040

tttaaatatt tgcctgaggc ttacaaaaac ggaagaacca atgaaaaagc aagagagaaa 2100

atggctcacg cttcaactat ggcaggtatg gcatccgcta atgcatttct aggtctatgt 2160

cattccatgg caataaaatt aagttcagaa cacaatattc ctagtggcat tgccaatgca 2220

ttactaatag aagaagtaat aaaatttaac gcagttgata atcctgtaaa acaagcccct 2280

tgcccacaat ataagtatcc aaacaccata tttagatatg ctcgaattgc agattatata 2340

aagcttggag gaaatactga tgaggaaaag gtagatctct taattaacaa aatacatgaa 2400

ctaaaaaaag ctttaaatat accaacttca ataaaggatg caggtgtttt ggaggaaaac 2460

ttctattcct cccttgatag aatatctgaa cttgcactag atgatcaatg cacaggcgct 2520

aatcctagat ttcctcttac aagtgagata aaagaaatgt atataaattg ttttaaaaaa 2580

caaccttaa 2589

<210> 224
<211> 862
<212> PRT
<213> Clostridium acetobutylicum

<400> 224

Met Lys Val Thr Thr Val Lys Glu Leu Asp Glu Lys Leu Lys Val Ile
1               5                   10                  15

Lys Glu Ala Gln Lys Lys Phe Ser Cys Tyr Ser Gln Glu Met Val Asp
            20                  25                  30

Glu Ile Phe Arg Asn Ala Ala Met Ala Ala Ile Asp Ala Arg Ile Glu
        35                  40                  45

Leu Ala Lys Ala Ala Val Leu Glu Thr Gly Met Gly Leu Val Glu Asp
    50                  55                  60

Lys Val Ile Lys Asn His Phe Ala Gly Glu Tyr Ile Tyr Asn Lys Tyr
65                  70                  75                  80

Lys Asp Glu Lys Thr Cys Gly Ile Ile Glu Arg Asn Glu Pro Tyr Gly

85                  90                  95

Ile Thr Lys Ile Ala Glu Pro Ile Gly Val Val Ala Ala Ile Ile Pro
            100                 105                 110

Val Thr Asn Pro Thr Ser Thr Thr Ile Phe Lys Ser Leu Ile Ser Leu
        115                 120                 125

Lys Thr Arg Asn Gly Ile Phe Phe Ser Pro His Pro Arg Ala Lys Lys
    130                 135                 140

Ser Thr Ile Leu Ala Ala Lys Thr Ile Leu Asp Ala Ala Val Lys Ser
145                 150                 155                 160

Gly Ala Pro Glu Asn Ile Ile Gly Trp Ile Asp Glu Pro Ser Ile Glu
                165                 170                 175

Leu Thr Gln Tyr Leu Met Gln Lys Ala Asp Ile Thr Leu Ala Thr Gly
            180                 185                 190

Gly Pro Ser Leu Val Lys Ser Ala Tyr Ser Ser Gly Lys Pro Ala Ile
        195                 200                 205

Gly Val Gly Pro Gly Asn Thr Pro Val Ile Ile Asp Glu Ser Ala His
    210                 215                 220

Ile Lys Met Ala Val Ser Ser Ile Ile Leu Ser Lys Thr Tyr Asp Asn
225                 230                 235                 240

Gly Val Ile Cys Ala Ser Glu Gln Ser Val Ile Val Leu Lys Ser Ile
                245                 250                 255

Tyr Asn Lys Val Lys Asp Glu Phe Gln Glu Arg Gly Ala Tyr Ile Ile
            260                 265                 270

Lys Lys Asn Glu Leu Asp Lys Val Arg Glu Val Ile Phe Lys Asp Gly
        275                 280                 285

Ser Val Asn Pro Lys Ile Val Gly Gln Ser Ala Tyr Thr Ile Ala Ala
    290                 295                 300

Met Ala Gly Ile Lys Val Pro Lys Thr Thr Arg Ile Leu Ile Gly Glu
305                 310                 315                 320

Val Thr Ser Leu Gly Glu Glu Glu Pro Phe Ala His Glu Lys Leu Ser
                325                 330                 335

Pro Val Leu Ala Met Tyr Glu Ala Asp Asn Phe Asp Asp Ala Leu Lys
            340                 345                 350

Lys Ala Val Thr Leu Ile Asn Leu Gly Gly Leu Gly His Thr Ser Gly
        355                 360                 365

Ile Tyr Ala Asp Glu Ile Lys Ala Arg Asp Lys Ile Asp Arg Phe Ser
    370                 375                 380

Ser Ala Met Lys Thr Val Arg Thr Phe Val Asn Ile Pro Thr Ser Gln
385                 390                 395                 400

Gly Ala Ser Gly Asp Leu Tyr Asn Phe Arg Ile Pro Pro Ser Phe Thr
                405                 410                 415

Leu Gly Cys Gly Phe Trp Gly Gly Asn Ser Val Ser Glu Asn Val Gly
            420                 425                 430

Pro Lys His Leu Leu Asn Ile Lys Thr Val Ala Glu Arg Arg Glu Asn
        435                 440                 445

Met Leu Trp Phe Arg Val Pro His Lys Val Tyr Phe Lys Phe Gly Cys
    450                 455                 460

Leu Gln Phe Ala Leu Lys Asp Leu Lys Asp Leu Lys Lys Lys Arg Ala
465                 470                 475                 480

Phe Ile Val Thr Asp Ser Asp Pro Tyr Asn Leu Asn Tyr Val Asp Ser
                485                 490                 495

Ile Ile Lys Ile Leu Glu His Leu Asp Ile Asp Phe Lys Val Phe Asn
            500                 505                 510

Lys Val Gly Arg Glu Ala Asp Leu Lys Thr Ile Lys Lys Ala Thr Glu
        515                 520                 525

Glu Met Ser Ser Phe Met Pro Asp Thr Ile Ile Ala Leu Gly Gly Thr
    530                 535                 540

Pro Glu Met Ser Ser Ala Lys Leu Met Trp Val Leu Tyr Glu His Pro
545                 550                 555                 560

Glu Val Lys Phe Glu Asp Leu Ala Ile Lys Phe Met Asp Ile Arg Lys
                565                 570                 575

Arg Ile Tyr Thr Phe Pro Lys Leu Gly Lys Lys Ala Met Leu Val Ala
            580                 585                 590

Ile Thr Thr Ser Ala Gly Ser Gly Ser Glu Val Thr Pro Phe Ala Leu
        595                 600                 605

Val Thr Asp Asn Asn Thr Gly Asn Lys Tyr Met Leu Ala Asp Tyr Glu
    610                 615                 620

Met Thr Pro Asn Met Ala Ile Val Asp Ala Glu Leu Met Met Lys Met
625                 630                 635                 640

Pro Lys Gly Leu Thr Ala Tyr Ser Gly Ile Asp Ala Leu Val Asn Ser
                645                 650                 655

Ile Glu Ala Tyr Thr Ser Val Tyr Ala Ser Glu Tyr Thr Asn Gly Leu
            660                 665                 670

Ala Leu Glu Ala Ile Arg Leu Ile Phe Lys Tyr Leu Pro Glu Ala Tyr
        675                 680                 685

Lys Asn Gly Arg Thr Asn Glu Lys Ala Arg Glu Lys Met Ala His Ala
    690                 695                 700

Ser Thr Met Ala Gly Met Ala Ser Ala Asn Ala Phe Leu Gly Leu Cys
705                 710                 715                 720

His Ser Met Ala Ile Lys Leu Ser Ser Glu His Asn Ile Pro Ser Gly
                725                 730                 735

Ile Ala Asn Ala Leu Leu Ile Glu Glu Val Ile Lys Phe Asn Ala Val
            740                 745                 750

Asp Asn Pro Val Lys Gln Ala Pro Cys Pro Gln Tyr Lys Tyr Pro Asn
        755                 760                 765

Thr Ile Phe Arg Tyr Ala Arg Ile Ala Asp Tyr Ile Lys Leu Gly Gly
    770                 775                 780

Asn Thr Asp Glu Glu Lys Val Asp Leu Leu Ile Asn Lys Ile His Glu
785                 790                 795                 800

Leu Lys Lys Ala Leu Asn Ile Pro Thr Ser Ile Lys Asp Ala Gly Val
                805                 810                 815

Leu Glu Glu Asn Phe Tyr Ser Ser Leu Asp Arg Ile Ser Glu Leu Ala
            820                 825                 830

Leu Asp Asp Gln Cys Thr Gly Ala Asn Pro Arg Phe Pro Leu Thr Ser
        835                 840                 845

Glu Ile Lys Glu Met Tyr Ile Asn Cys Phe Lys Lys Gln Pro
    850                 855                 860

<210>  225
<211>  2577
<212>  DNA
<213>  Clostridium acetobutylicum

<400>  225
atgaaagtta caaatcaaaa agaactaaaa caaaagctaa atgaattgag agaagcgcaa      60

aagaagtttg caacctatac tcaagagcaa gttgataaaa tttttaaaca atgtgccata     120

gccgcagcta aagaaagaat aaacttagct aaattagcag tagaagaaac aggaataggt     180

cttgtagaag ataaaattat aaaaaatcat tttgcagcag aatatatata caataaatat     240

aaaaatgaaa aaacttgtgg cataatagac catgacgatt ctttaggcat aacaaaggtt     300

gctgaaccaa ttggaattgt tgcagccata gttcctacta ctaatccaac ttccacagca     360

attttcaaat cattaatttc tttaaaaaca agaaacgcaa tattcttttc accacatcca     420

cgtgcaaaaa aatctacaat tgctgcagca aaattaattt tagatgcagc tgttaaagca     480

ggagcaccta aaaatataat aggctggata gatgagccat caatagaact ttctcaagat     540

ttgatgagtg aagctgatat aatattagca acaggaggtc cttcaatggt taaagcggcc     600

tattcatctg gaaaacctgc aattggtgtt ggagcaggaa atacaccagc aataatagat     660

gagagtgcag atatagatat ggcagtaagc tccataattt tatcaaagac ttatgacaat     720

ggagtaatat gcgcttctga acaatcaata ttagttatga attcaatata cgaaaaagtt     780

aaagaggaat ttgtaaaacg aggatcatat atactcaatc aaaatgaaat agctaaaata     840

aaagaaacta tgtttaaaaa tggagctatt aatgctgaca tagttggaaa atctgcttat     900

ataattgcta aaatggcagg aattgaagtt cctcaaacta caaagatact tataggcgaa     960

gtacaatctg ttgaaaaaag cgagctgttc tcacatgaaa aactatcacc agtacttgca    1020

atgtataaag ttaaggattt tgatgaagct ctaaaaaagg cacaaaggct aatagaatta    1080

ggtggaagtg gacacacgtc atctttatat atagattcac aaaacaataa ggataaagtt    1140

aaagaatttg gattagcaat gaaaacttca aggacattta ttaacatgcc ttcttcacag    1200

ggagcaagcg gagatttata caattttgcg atagcaccat catttactct tggatgcggc    1260

acttggggag gaaactctgt atcgcaaaat gtagagccta aacatttatt aaatattaaa    1320

agtgttgctg aaagaaggga aaatatgctt tggtttaaag tgccacaaaa aatatatttt    1380

aaatatggat gtcttagatt tgcattaaaa gaattaaaag atatgaataa gaaaagagcc    1440

tttatagtaa cagataaaga tctttttaaa cttggatatg ttaataaaat aacaaaggta    1500

ctagatgaga tagatattaa atacagtata tttacagata ttaaatctga tccaactatt    1560

gattcagtaa aaaaaggtgc taaagaaatg cttaactttg aacctgatac tataatctct    1620

attggtggtg gatcgccaat ggatgcagca aaggttatgc acttgttata tgaatatcca    1680

gaagcagaaa ttgaaaatct agctataaac tttatggata taagaaagag aatatgcaat    1740

ttccctaaat taggtacaaa ggcgatttca gtagctattc ctacaactgc tggtaccggt    1800

tcagaggcaa caccttttgc agttataact aatgatgaaa caggaatgaa atacccttta 1860

acttcttatg aattgacccc aaacatggca ataatagata ctgaattaat gttaaatatg 1920

cctagaaaat taacagcagc aactggaata gatgcattag ttcatgctat agaagcatat 1980

gtttcggtta tggctacgga ttatactgat gaattagcct taagagcaat aaaaatgata 2040

tttaaatatt tgcctagagc ctataaaaat gggactaacg acattgaagc aagagaaaaa 2100

atggcacatg cctctaatat tgcggggatg gcatttgcaa atgctttctt aggtgtatgc 2160

cattcaatgg ctcataaact tggggcaatg catcacgttc cacatggaat tgcttgtgct 2220

gtattaatag aagaagttat taaatataac gctacagact gtccaacaaa gcaaacagca 2280

ttccctcaat ataaatctcc taatgctaag agaaaatatg ctgaaattgc agagtatttg 2340

aatttaaagg gtactagcga taccgaaaag gtaacagcct taatagaagc tatttcaaag 2400

ttaaagatag atttgagtat tccacaaaat ataagtgccg ctggaataaa taaaaaagat 2460

ttttataata cgctagataa aatgtcagag cttgcttttg atgaccaatg tacaacagct 2520

aatcctaggt atccacttat aagtgaactt aaggatatct atataaaatc attttaa 2577

<210> 226
<211> 800
<212> PRT
<213> Clostridium acetobutylicum

<400> 226

Met Lys Val Thr Asn Gln Lys Glu Leu Lys Gln Lys Leu Asn Glu Leu
1               5                   10                  15

Arg Glu Ala Gln Lys Lys Phe Ala Thr Tyr Thr Gln Glu Gln Val Asp
            20                  25                  30

Lys Ile Phe Lys Gln Cys Ala Ile Ala Ala Ala Lys Glu Arg Ile Asn
        35                  40                  45

Leu Ala Lys Leu Ala Val Glu Glu Thr Gly Ile Gly Leu Val Glu Asp
    50                  55                  60

Lys Ile Ile Lys Asn His Phe Ala Ala Glu Tyr Ile Tyr Asn Lys Tyr
65                  70                  75                  80

Lys Asn Glu Lys Thr Cys Gly Ile Ile Asp His Asp Asp Ser Leu Gly
                85                  90                  95

Ile Thr Lys Val Ala Glu Pro Ile Gly Ile Val Ala Ala Ile Val Pro
            100                 105                 110

Thr Thr Asn Pro Thr Ser Thr Ala Ile Phe Lys Ser Leu Ile Ser Leu
        115                 120                 125

Lys Thr Arg Asn Ala Ile Phe Phe Ser Pro His Pro Arg Ala Lys Lys
    130                 135                 140

Ser Thr Ile Ala Ala Ala Lys Leu Ile Leu Asp Ala Ala Val Lys Ala
145                 150                 155                 160

Gly Ala Pro Lys Asn Ile Ile Gly Trp Ile Asp Glu Pro Ser Ile Glu
                165                 170                 175

Leu Ser Gln Asp Leu Met Ser Glu Ala Asp Ile Ile Leu Ala Thr Gly
            180                 185                 190

Gly Pro Ser Met Val Lys Ala Ala Tyr Ser Ser Gly Lys Pro Ala Ile
        195                 200                 205

Gly Val Gly Ala Gly Asn Thr Pro Ala Ile Ile Asp Glu Ser Ala Asp
    210                 215                 220

Ile Asp Met Ala Val Ser Ser Ile Ile Leu Ser Lys Thr Tyr Asp Asn
225                 230                 235                 240

Gly Val Ile Cys Ala Ser Glu Gln Ser Ile Leu Val Met Asn Ser Ile
                245                 250                 255

Tyr Glu Lys Val Lys Glu Glu Phe Val Lys Arg Gly Ser Tyr Ile Leu
            260                 265                 270

Asn Gln Asn Glu Ile Ala Lys Ile Lys Glu Thr Met Phe Lys Asn Gly
        275                 280                 285

Ala Ile Asn Ala Asp Ile Val Gly Lys Ser Ala Tyr Ile Ile Ala Lys
    290                 295                 300

Met Ala Gly Ile Glu Val Pro Gln Thr Thr Lys Ile Leu Ile Gly Glu
305                 310                 315                 320

Val Gln Ser Val Glu Lys Ser Glu Leu Phe Ser His Glu Lys Leu Ser
                325                 330                 335

Pro Val Leu Ala Met Tyr Lys Val Lys Asp Phe Asp Glu Ala Leu Lys
            340                 345                 350

Lys Ala Gln Arg Leu Ile Glu Leu Gly Gly Ser Gly His Thr Ser Ser
        355                 360                 365

Leu Tyr Ile Asp Ser Gln Asn Asn Lys Asp Lys Val Lys Glu Phe Gly
    370                 375                 380

Leu Ala Met Lys Thr Ser Arg Thr Phe Ile Asn Met Pro Ser Ser Gln
385                 390                 395                 400

Gly Ala Ser Gly Asp Leu Tyr Asn Phe Ala Ile Ala Pro Ser Phe Thr
                405                 410                 415

Leu Gly Cys Gly Thr Trp Gly Gly Asn Ser Val Ser Gln Asn Val Glu
            420                 425                 430

Pro Lys His Leu Leu Asn Ile Lys Ser Val Ala Glu Arg Arg Glu Asn
        435                 440                 445

Met Leu Trp Phe Lys Val Pro Gln Lys Ile Tyr Phe Lys Tyr Gly Cys
    450                 455                 460

Leu Arg Phe Ala Leu Lys Glu Leu Lys Asp Met Asn Lys Lys Arg Ala
465                 470                 475                 480

Phe Ile Val Thr Asp Lys Asp Leu Phe Lys Leu Gly Tyr Val Asn Lys
                485                 490                 495

Ile Thr Lys Val Leu Asp Glu Ile Asp Ile Lys Tyr Ser Ile Phe Thr
            500                 505                 510

Asp Ile Lys Ser Asp Pro Thr Ile Asp Ser Val Lys Lys Gly Ala Lys
        515                 520                 525

Glu Met Leu Asn Phe Glu Pro Asp Thr Ile Ile Ser Ile Gly Gly Gly
    530                 535                 540

Ser Pro Met Asp Ala Ala Lys Val Met His Leu Leu Tyr Glu Tyr Pro
545                 550                 555                 560

Glu Ala Glu Ile Glu Asn Leu Ala Ile Asn Phe Met Asp Ile Arg Lys
                565                 570                 575

Arg Ile Cys Asn Phe Pro Lys Leu Gly Thr Lys Ala Ile Ser Val Ala
            580                 585                 590

Ile Pro Thr Thr Ala Gly Thr Gly Ser Glu Ala Thr Pro Phe Ala Val
        595                 600                 605

Ile Thr Asn Asp Glu Thr Gly Met Lys Tyr Pro Leu Thr Ser Tyr Glu
    610                 615                 620

Leu Thr Pro Asn Met Ala Ile Ile Asp Thr Glu Leu Met Leu Asn Met
625                 630                 635                 640

Pro Arg Lys Leu Thr Ala Ala Thr Gly Ile Asp Ala Leu Val His Ala
                645                 650                 655

Ile Glu Ala Tyr Val Ser Val Met Ala Thr Asp Tyr Thr Asp Glu Leu
            660                 665                 670

Ala Leu Arg Ala Ile Lys Met Ile Phe Lys Tyr Leu Pro Arg Ala Tyr
        675                 680                 685

Lys Asn Gly Thr Asn Asp Ile Glu Ala Arg Glu Lys Met Ala His Ala
    690                 695                 700

Ser Asn Ile Ala Gly Met Ala Phe Ala Asn Ala Phe Leu Gly Val Cys
705                 710                 715                 720

His Ser Met Ala His Lys Leu Gly Ala Met His His Val Pro His Gly
                725                 730                 735

Ile Ala Cys Ala Val Leu Ile Glu Glu Val Ile Lys Tyr Asn Ala Thr
            740                 745                 750

Asp Cys Pro Thr Lys Gln Thr Ala Phe Pro Gln Tyr Lys Ser Pro Asn
        755                 760                 765

Ala Lys Arg Lys Tyr Ala Glu Ile Ala Glu Tyr Leu Asn Leu Lys Gly
    770                 775                 780

Thr Ser Asp Thr Glu Lys Val Thr Ala Leu Ile Glu Ala Ile Ser Lys
785                 790                 795                 800

<210>  227
<211>  924
<212>  DNA
<213>  Pseudomonas putida

<400>  227
atgagcaaga aactcaaggc ggccatcata ggccccggca atatcggtac cgatctggtg      60

atgaagatgc tccgttccga gtggattgag ccggtgtgga tggtcggcat cgaccccaac     120

tccgacggcc tcaaacgcgc ccgcgatttc ggcatgaaga ccacagccga aggcgtcgac     180

ggcctgctcc cgcacgtgct ggacgacgac atccgcatcg ccttcgacgc cacctcggcc     240

tatgtgcatg ccgagaatag ccgcaagctc aacgcgcttg gcgtgctgat ggtcgacctg     300

accccggcgg ccatcggccc ctactgcgtg ccgccggtca acctcaagca gcatgtcggc     360

cgcctggaaa tgaacgtcaa catggtcacc tgcggcggcc aggccaccat ccccatggtc     420

gccgcggtgt cccgcgtgca gccggtggcc tacgccgaga tcgtcgccac cgtctcctcg     480

cgctcggtcg gcccgggcac gcgcaagaac atcgacgagt tcacccgcac caccgccggc     540

gccatcgagc aggtcggcgg cgccagggaa ggcaaggcga tcatcgtcat caacccggcc     600

gagccgccgc tgatgatgcg cgacaccatc cactgcctga ccgacagcga gccggaccag     660

gctgcgatca ccgcttcggt tcacgcgatg atcgccgagg tgcagaaata cgtgcccggc     720

taccgcctga agaacggccc ggtgttcgac ggcaaccgcg tgtcgatctt catggaagtc     780

gaaggcctgg gcgactacct gcccaagtac gccggcaacc tcgacatcat gaccgccgcc     840

gcgctgcgta ccggcgagat gttcgccgag gaaatcgccg ccggcaccat tcaactgccg     900

cgtcgcgaca tcgcgctggc ttga                                            924

<210>  228
<211>  307
<212>  PRT
<213>  Pseudomonas putida

<400>  228

Met Ser Lys Lys Leu Lys Ala Ala Ile Ile Gly Pro Gly Asn Ile Gly
1               5                   10                  15

Thr Asp Leu Val Met Lys Met Leu Arg Ser Glu Trp Ile Glu Pro Val
            20                  25                  30

Trp Met Val Gly Ile Asp Pro Asn Ser Asp Gly Leu Lys Arg Ala Arg
        35                  40                  45

Asp Phe Gly Met Lys Thr Thr Ala Glu Gly Val Asp Gly Leu Leu Pro
    50                  55                  60

His Val Leu Asp Asp Asp Ile Arg Ile Ala Phe Asp Ala Thr Ser Ala
65                  70                  75                  80

Tyr Val His Ala Glu Asn Ser Arg Lys Leu Asn Ala Leu Gly Val Leu
                85                  90                  95

Met Val Asp Leu Thr Pro Ala Ala Ile Gly Pro Tyr Cys Val Pro Pro
            100                 105                 110

Val Asn Leu Lys Gln His Val Gly Arg Leu Glu Met Asn Val Asn Met
        115                 120                 125

Val Thr Cys Gly Gly Gln Ala Thr Ile Pro Met Val Ala Ala Val Ser
    130                 135                 140

Arg Val Gln Pro Val Ala Tyr Ala Glu Ile Val Ala Thr Val Ser Ser
145                 150                 155                 160

Arg Ser Val Gly Pro Gly Thr Arg Lys Asn Ile Asp Glu Phe Thr Arg
                165                 170                 175

Thr Thr Ala Gly Ala Ile Glu Gln Val Gly Gly Ala Arg Glu Gly Lys
        180                 185                 190

Ala Ile Ile Val Ile Asn Pro Ala Glu Pro Pro Leu Met Met Arg Asp
    195                 200                 205

Thr Ile His Cys Leu Thr Asp Ser Glu Pro Asp Gln Ala Ala Ile Thr
    210                 215                 220

Ala Ser Val His Ala Met Ile Ala Glu Val Gln Lys Tyr Val Pro Gly
225                 230                 235                 240

Tyr Arg Leu Lys Asn Gly Pro Val Phe Asp Gly Asn Arg Val Ser Ile
                245                 250                 255

Phe Met Glu Val Glu Gly Leu Gly Asp Tyr Leu Pro Lys Tyr Ala Gly
        260                 265                 270

Asn Leu Asp Ile Met Thr Ala Ala Ala Leu Arg Thr Gly Glu Met Phe
    275                 280                 285

Ala Glu Glu Ile Ala Ala Gly Thr Ile Gln Leu Pro Arg Arg Asp Ile
    290                 295                 300

Ala Leu Ala
305

<210>  229
<211>  924
<212>  DNA
<213>  Thermus thermophilus

<400>  229
atgtccgaaa gggttaaggt agccatcctg ggctccggca acatcgggac ggacctgatg      60

tacaagctcc tgaagaaccc gggccacatg gagcttgtgg cggtggtggg gatagacccc     120

aagtccgagg gcctggcccg ggcgcgggcc ttagggttag aggcgagcca cgaagggatc     180

gcctacatcc tggagaggcc ggagatcaag atcgtctttg acgccaccag cgccaaggcc     240

cacgtgcgcc acgccaagct cctgagggag gcggggaaga tcgccataga cctcacgccg     300

gcggcccggg gcccttacgt ggtgcccccg gtgaacctga aggaacacct ggacaaggac     360

aacgtgaacc tcatcacctg cggggggcag gccaccatcc ccctggtcta cgcggtgcac     420

cgggtggccc ccgtgctcta cgcggagatg gtctccacgg tggcctcccg ctccgcgggc     480

cccggcaccc ggcagaacat cgacgagttc accttcacca ccgcccgggg cctggaggcc     540

atcggggggg ccaagaaggg gaaggccatc atcatcctga acccggcgga accccccatc     600

ctcatgacca acaccgtgcg ctgcatcccc gaggacgagg gctttgaccg ggaggccgtg     660

gtggcgagcg tccgggccat ggagcgggag gtccaggcct acgtgcccgg ctaccgcctg     720

aaggcggacc cggtgtttga gaggcttccc accccctggg gggagcgcac cgtggtctcc 780

atgctcctgg aggtggaggg ggcgggggac tatttgccca aatacgccgg caacctggac 840

atcatgacgg cttctgcccg gagggtgggg gaggtcttcg cccagcacct cctggggaag 900

cccgtggagg aggtggtggc gtga 924

<210> 230
<211> 307
<212> PRT
<213> Thermus thermophilus

<400> 230

Met Ser Glu Arg Val Lys Val Ala Ile Leu Gly Ser Gly Asn Ile Gly
1               5                   10                  15

Thr Asp Leu Met Tyr Lys Leu Leu Lys Asn Pro Gly His Met Glu Leu
            20                  25                  30

Val Ala Val Val Gly Ile Asp Pro Lys Ser Glu Gly Leu Ala Arg Ala
        35                  40                  45

Arg Ala Leu Gly Leu Glu Ala Ser His Glu Gly Ile Ala Tyr Ile Leu
    50                  55                  60

Glu Arg Pro Glu Ile Lys Ile Val Phe Asp Ala Thr Ser Ala Lys Ala
65                  70                  75                  80

His Val Arg His Ala Lys Leu Leu Arg Glu Ala Gly Lys Ile Ala Ile
                85                  90                  95

Asp Leu Thr Pro Ala Ala Arg Gly Pro Tyr Val Val Pro Pro Val Asn
            100                 105                 110

Leu Lys Glu His Leu Asp Lys Asp Asn Val Asn Leu Ile Thr Cys Gly
        115                 120                 125

Gly Gln Ala Thr Ile Pro Leu Val Tyr Ala Val His Arg Val Ala Pro
    130                 135                 140

Val Leu Tyr Ala Glu Met Val Ser Thr Val Ala Ser Arg Ser Ala Gly
145                 150                 155                 160

Pro Gly Thr Arg Gln Asn Ile Asp Glu Phe Thr Phe Thr Thr Ala Arg
                165                 170                 175

Gly Leu Glu Ala Ile Gly Gly Ala Lys Lys Gly Lys Ala Ile Ile Ile
            180                 185                 190

Leu Asn Pro Ala Glu Pro Pro Ile Leu Met Thr Asn Thr Val Arg Cys
        195                 200                 205

Ile Pro Glu Asp Glu Gly Phe Asp Arg Glu Ala Val Val Ala Ser Val
    210                 215                 220

Arg Ala Met Glu Arg Glu Val Gln Ala Tyr Val Pro Gly Tyr Arg Leu
225                 230                 235                 240

Lys Ala Asp Pro Val Phe Glu Arg Leu Pro Thr Pro Trp Gly Glu Arg
                245                 250                 255

Thr Val Val Ser Met Leu Leu Glu Val Glu Gly Ala Gly Asp Tyr Leu
            260                 265                 270

Pro Lys Tyr Ala Gly Asn Leu Asp Ile Met Thr Ala Ser Ala Arg Arg
        275                 280                 285

Val Gly Glu Val Phe Ala Gln His Leu Leu Gly Lys Pro Val Glu Glu
    290                 295                 300

Val Val Ala
305

<210> 231
<211> 1254
<212> DNA
<213> Escherichia coli

<400> 231
   atgacattct ccctttttgg tgacaaattt acccgccact ccggcattac gctgttgatg 60
   gaagatctga acgacggttt acgcacgcct ggcgcgatta tgctcggcgg cggtaatccg 120
   gcgcagatcc cggaaatgca ggactacttc cagacgctac tgaccgacat gctggaaagt 180
   ggcaaagcga ctgatgcact gtgtaactac gacggtccac aggggaaaac ggagctactc 240
   acactgcttg ccggaatgct gcgcgagaag ttgggttggg atatcgaacc acagaatatt 300
   gcactaacaa acggcagcca gagcgcgttt ttctacttat ttaacctgtt tgccggacgc 360
   cgtgccgatg gtcgggtcaa aaaagtgctg ttcccgcttg caccggaata cattggctat 420
   gctgacgccg gactggaaga agatctgttt gtctctgcgc gtccgaatat tgaactgctg 480
   ccggaaggcc agtttaaata ccacgtcgat tttgagcatc tgcatattgg cgaagaaacc 540
   gggatgattt gcgtctcccg gccgacgaat ccaacaggca atgtgattac tgacgaagag 600
   ttgctgaagc ttgacgcgct ggcgaatcaa cacggcattc cgctggtgat tgataacgct 660
   tatggcgtcc cgttcccggg tatcatcttc agtgaagcgc gcccgctatg gaatccgaat 720
   atcgtgctgt gcatgagtct ttccaagctg ggtctacctg gctcccgctg cggcattatc 780
   atcgccaatg aaaaaatcat caccgccatc accaatatga acggcattat cagcctggca 840
   cctggcggta ttggtccggc gatgatgtgt gaaatgatta agcgtaacga tctgctgcgc     900
   ctgtctgaaa cagtcatcaa accgttttac taccagcgtg ttcaggaaac tatcgccatc     960
   attcgccgct atttaccgga aaatcgctgc ctgattcata aaccggaagg agccattttc    1020
   ctctggctat ggtttaagga tttgcccatt acgaccaagc agctctatca gcgcctgaaa    1080
   gcacgcggcg tgctgatggt gccggggcac aacttcttcc cagggctgga taaaccgtgg    1140
   ccgcatacgc atcaatgtat gcgcatgaac tacgtaccag agccggagaa aattgaggcg    1200
   ggggtgaaga ttctggcgga agagatagaa agagcctggg ctgaaagtca ctaa          1254

<210>  232
<211>  417
<212>  PRT
<213>  Escherichia coli

<400>  232

Met Thr Phe Ser Leu Phe Gly Asp Lys Phe Thr Arg His Ser Gly Ile
1               5                   10                  15

Thr Leu Leu Met Glu Asp Leu Asn Asp Gly Leu Arg Thr Pro Gly Ala
            20                  25                  30

Ile Met Leu Gly Gly Gly Asn Pro Ala Gln Ile Pro Glu Met Gln Asp
        35                  40                  45

Tyr Phe Gln Thr Leu Leu Thr Asp Met Leu Glu Ser Gly Lys Ala Thr
    50                  55                  60

Asp Ala Leu Cys Asn Tyr Asp Gly Pro Gln Gly Lys Thr Glu Leu Leu
65                  70                  75                  80

Thr Leu Leu Ala Gly Met Leu Arg Glu Lys Leu Gly Trp Asp Ile Glu
                85                  90                  95

Pro Gln Asn Ile Ala Leu Thr Asn Gly Ser Gln Ser Ala Phe Phe Tyr
            100                 105                 110

Leu Phe Asn Leu Phe Ala Gly Arg Arg Ala Asp Gly Arg Val Lys Lys
        115                 120                 125

Val Leu Phe Pro Leu Ala Pro Glu Tyr Ile Gly Tyr Ala Asp Ala Gly
    130                 135                 140

Leu Glu Glu Asp Leu Phe Val Ser Ala Arg Pro Asn Ile Glu Leu Leu
145                 150                 155                 160

Pro Glu Gly Gln Phe Lys Tyr His Val Asp Phe Glu His Leu His Ile
                165                 170                 175

Gly Glu Glu Thr Gly Met Ile Cys Val Ser Arg Pro Thr Asn Pro Thr
            180                 185                 190

Gly Asn Val Ile Thr Asp Glu Glu Leu Leu Lys Leu Asp Ala Leu Ala
        195                 200                 205

Asn Gln His Gly Ile Pro Leu Val Ile Asp Asn Ala Tyr Gly Val Pro
    210                 215                 220

Phe Pro Gly Ile Ile Phe Ser Glu Ala Arg Pro Leu Trp Asn Pro Asn
225                 230                 235                 240

Ile Val Leu Cys Met Ser Leu Ser Lys Leu Gly Leu Pro Gly Ser Arg
                245                 250                 255

Cys Gly Ile Ile Ile Ala Asn Glu Lys Ile Ile Thr Ala Ile Thr Asn
            260                 265                 270

Met Asn Gly Ile Ile Ser Leu Ala Pro Gly Gly Ile Gly Pro Ala Met
        275                 280                 285

Met Cys Glu Met Ile Lys Arg Asn Asp Leu Leu Arg Leu Ser Glu Thr
    290                 295                 300

Val Ile Lys Pro Phe Tyr Tyr Gln Arg Val Gln Glu Thr Ile Ala Ile
305                 310                 315                 320

Ile Arg Arg Tyr Leu Pro Glu Asn Arg Cys Leu Ile His Lys Pro Glu
                325                 330                 335

Gly Ala Ile Phe Leu Trp Leu Trp Phe Lys Asp Leu Pro Ile Thr Thr
            340                 345                 350

Lys Gln Leu Tyr Gln Arg Leu Lys Ala Arg Gly Val Leu Met Val Pro
        355                 360                 365

Gly His Asn Phe Phe Pro Gly Leu Asp Lys Pro Trp Pro His Thr His
    370                 375                 380

Gln Cys Met Arg Met Asn Tyr Val Pro Glu Pro Glu Lys Ile Glu Ala
385                 390                 395                 400

Gly Val Lys Ile Leu Ala Glu Glu Ile Glu Arg Ala Trp Ala Glu Ser
                405                 410                 415

His

<210>  233
<211>  1278
<212>  DNA
<213>  Bacillus licheniformis

<400>  233
ttataagtat tcaacctgtt tctcatatac acccttcgca attttagcta aaacatcgat      60

tccccttata atatcttcat ccgccgcggt taggctgatt cgtatacact ggtgtgaatg     120

cgccaggcgc cgggattgac ggtgaaagaa agatgatccg ggaacgataa tgactccatc     180

cgctttcata tactcataca gcgctgcatc ggtcaccggc aggtcttcaa accacagcca     240

tccgaaaagc gatccttccc cttgatgcag ataccatttg atgtcttcag gcatcttgca     300

taaaagcgtt tccttgagca gcatgaattt attgcggtaa tatggcctga cttcattcag     360

cgacacgtcg gcgaggcgcc cgtcattcaa tactgatgca gccatatact gccccagcct     420

tgaagaatgg atcgccgcat tcgactgaaa agcttccatt gcctgaatat accgggacgg     480

cccgatggcg attccgatcc tttcgccagg caggccggct tttgaaaggc tcatacagtg     540

aatgatctgc tcgttgaaaa tcggttccat gtcgataaag tgaatcgccg gaaaaggcgg     600

agcatatgcg gaatcaatga acagcggaac attcgcttct cggcatgcgt ctgaaatgaa     660

tgctacatct tctttaggca agatgtttcc gcaaggattg ttcgggcgcg atagcaagac     720

agcaccgatg cgcatcctct ctaaaaaccc cttacggtcg agctcatatc gaaacgtatg     780

atcatccaat ttcgatatga gcggagggat cccctcaatc atctcccgct ccagtgccgc     840

cccgctgtat cccgaatagt caggcagcat cgggatcaag gcttttttca tcacagatcc     900

gcttcccatt ccgcaaaacg aattgatcgc cagaaaaaac agctgctggc ttccggctgt     960

aatcaacacg ttctcttttc gaatgccggc gctataccgc tctgaaaaga agcggacaac    1020

acttgcaatc agttcatcgg ttccatagct cgatccgtat tggccgatca ccgaagaaaa    1080

cctgtcatcg tcaaggagat cggcaagagc cgacttccac atggctgaca cgccgggcaa    1140

aatcatcgga ttgcccgcac ttaaattaat gtatgaccgt tcaccgccgg ccaggacttc    1200

ctgaatatcg ctcatcacag ccctgacccc tgttttctca atcattttct ctccgatttt    1260

gcttaatggc ggcttcac                                                  1278

<210>  234
<211>  425
<212>  PRT
<213>  Bacillus licheniformis

<400>  234

Met Lys Pro Pro Leu Ser Lys Ile Gly Glu Lys Met Ile Glu Lys Thr
1               5                   10                  15

Gly Val Arg Ala Val Met Ser Asp Ile Gln Glu Val Leu Ala Gly Gly
            20                  25                  30

Glu Arg Ser Tyr Ile Asn Leu Ser Ala Gly Asn Pro Met Ile Leu Pro
        35                  40                  45

Gly Val Ser Ala Met Trp Lys Ser Ala Leu Ala Asp Leu Leu Asp Asp
    50                  55                  60

Asp Arg Phe Ser Ser Val Ile Gly Gln Tyr Gly Ser Ser Tyr Gly Thr
65                  70                  75                  80

Asp Glu Leu Ile Ala Ser Val Val Arg Phe Phe Ser Glu Arg Tyr Ser
                85                  90                  95

Ala Gly Ile Arg Lys Glu Asn Val Leu Ile Thr Ala Gly Ser Gln Gln
            100                 105                 110

Leu Phe Phe Leu Ala Ile Asn Ser Phe Cys Gly Met Gly Ser Gly Ser
        115                 120                 125

Val Met Lys Lys Ala Leu Ile Pro Met Leu Pro Asp Tyr Ser Gly Tyr
    130                 135                 140

Ser Gly Ala Ala Leu Glu Arg Glu Met Ile Glu Gly Ile Pro Pro Leu
145                 150                 155                 160

Ile Ser Lys Leu Asp Asp His Thr Phe Arg Tyr Glu Leu Asp Arg Lys
                165                 170                 175

Gly Phe Leu Glu Arg Met Arg Ile Gly Ala Val Leu Leu Ser Arg Pro
            180                 185                 190

Asn Asn Pro Cys Gly Asn Ile Leu Pro Lys Glu Asp Val Ala Phe Ile
        195                 200                 205

Ser Asp Ala Cys Arg Glu Ala Asn Val Pro Leu Phe Ile Asp Ser Ala
    210                 215                 220

Tyr Ala Pro Pro Phe Pro Ala Ile His Phe Ile Asp Met Glu Pro Ile
225                 230                 235                 240

Phe Asn Glu Gln Ile Ile His Cys Met Ser Leu Ser Lys Ala Gly Leu
                245                 250                 255

Pro Gly Glu Arg Ile Gly Ile Ala Ile Gly Pro Ser Arg Tyr Ile Gln
            260                 265                 270

Ala Met Glu Ala Phe Gln Ser Asn Ala Ala Ile His Ser Ser Arg Leu
        275                 280                 285

Gly Gln Tyr Met Ala Ala Ser Val Leu Asn Asp Gly Arg Leu Ala Asp
    290                 295                 300

Val Ser Leu Asn Glu Val Arg Pro Tyr Tyr Arg Asn Lys Phe Met Leu
305                 310                 315                 320

Leu Lys Glu Thr Leu Leu Cys Lys Met Pro Glu Asp Ile Lys Trp Tyr
                325                 330                 335

Leu His Gln Gly Glu Gly Ser Leu Phe Gly Trp Leu Trp Phe Glu Asp
            340                 345                 350

Leu Pro Val Thr Asp Ala Ala Leu Tyr Glu Tyr Met Lys Ala Asp Gly
        355                 360                 365

Val Ile Ile Val Pro Gly Ser Ser Phe Phe His Arg Gln Ser Arg Arg
    370                 375                 380

Leu Ala His Ser His Gln Cys Ile Arg Ile Ser Leu Thr Ala Ala Asp
385                 390                 395                 400

Glu Asp Ile Ile Arg Gly Ile Asp Val Leu Ala Lys Ile Ala Lys Gly
                405                 410                 415

Val Tyr Glu Lys Gln Val Glu Tyr Leu
            420                 425

<210>  235
<211>  930
<212>  DNA
<213>  Escherichia coli

<400>  235
atgaccacga agaaagctga ttacatttgg ttcaatgggg agatggttcg ctgggaagac      60

gcgaaggtgc atgtgatgtc gcacgcgctg cactatggca cttcggtttt tgaaggcatc     120

cgttgctacg actcgcacaa aggaccggtt gtattccgcc atcgtgagca tatgcagcgt     180

ctgcatgact ccgccaaaat ctatcgcttc ccggtttcgc agagcattga tgagctgatg     240

gaagcttgtc gtgacgtgat ccgcaaaaac aatctcacca gcgcctatat ccgtccgctg     300

atcttcgtcg gtgatgttgg catgggagta aacccgccag cgggatactc aaccgacgtg     360

attatcgctg ctttcccgtg gggagcgtat ctgggcgcag aagcgctgga gcaggggatc     420

gatgcgatgg tttcctcctg gaaccgcgca gcaccaaaca ccatcccgac ggcggcaaaa     480

gccggtggta actacctctc ttccctgctg gtgggtagcg aagcgcgccg ccacggttat     540

caggaaggta tcgcgctgga tgtgaacggt tatatctctg aaggcgcagg cgaaaacctg     600

tttgaagtga aagatggtgt gctgttcacc ccaccgttca cctcctccgc gctgccgggt     660

attacccgtg atgccatcat caaactggcg aaagagctgg gaattgaagt acgtgagcag 720

gtgctgtcgc gcgaatccct gtacctggcg gatgaagtgt ttatgtccgg tacggcggca 780

gaaatcacgc cagtgcgcag cgtagacggt attcaggttg gcgaaggccg ttgtggcccg 840

gttaccaaac gcattcagca agccttcttc ggcctcttca ctggcgaaac cgaagataaa 900

tggggctggt tagatcaagt taatcaataa 930

<210> 236
<211> 309
<212> PRT
<213> Escherichia coli

<400> 236

Met Thr Thr Lys Lys Ala Asp Tyr Ile Trp Phe Asn Gly Glu Met Val
1               5                   10                  15

Arg Trp Glu Asp Ala Lys Val His Val Met Ser His Ala Leu His Tyr
            20                  25                  30

Gly Thr Ser Val Phe Glu Gly Ile Arg Cys Tyr Asp Ser His Lys Gly
        35                  40                  45

Pro Val Val Phe Arg His Arg Glu His Met Gln Arg Leu His Asp Ser
    50                  55                  60

Ala Lys Ile Tyr Arg Phe Pro Val Ser Gln Ser Ile Asp Glu Leu Met
65                  70                  75                  80

Glu Ala Cys Arg Asp Val Ile Arg Lys Asn Asn Leu Thr Ser Ala Tyr
                85                  90                  95

Ile Arg Pro Leu Ile Phe Val Gly Asp Val Gly Met Gly Val Asn Pro
            100                 105                 110

Pro Ala Gly Tyr Ser Thr Asp Val Ile Ile Ala Ala Phe Pro Trp Gly
        115                 120                 125

Ala Tyr Leu Gly Ala Glu Ala Leu Glu Gln Gly Ile Asp Ala Met Val
    130                 135                 140

Ser Ser Trp Asn Arg Ala Ala Pro Asn Thr Ile Pro Thr Ala Ala Lys
145                 150                 155                 160

Ala Gly Gly Asn Tyr Leu Ser Ser Leu Leu Val Gly Ser Glu Ala Arg
                165                 170                 175

Arg His Gly Tyr Gln Glu Gly Ile Ala Leu Asp Val Asn Gly Tyr Ile
            180                 185                 190

Ser Glu Gly Ala Gly Glu Asn Leu Phe Glu Val Lys Asp Gly Val Leu
        195                 200                 205

Phe Thr Pro Pro Phe Thr Ser Ser Ala Leu Pro Gly Ile Thr Arg Asp
    210                 215                 220

Ala Ile Ile Lys Leu Ala Lys Glu Leu Gly Ile Glu Val Arg Glu Gln
225                 230                 235                 240

Val Leu Ser Arg Glu Ser Leu Tyr Leu Ala Asp Glu Val Phe Met Ser
                245                 250                 255

Gly Thr Ala Ala Glu Ile Thr Pro Val Arg Ser Val Asp Gly Ile Gln
            260                 265                 270

Val Gly Glu Gly Arg Cys Gly Pro Val Thr Lys Arg Ile Gln Gln Ala
        275                 280                 285

Phe Phe Gly Leu Phe Thr Gly Glu Thr Glu Asp Lys Trp Gly Trp Leu
    290                 295                 300

Asp Gln Val Asn Gln
305

<210>  237
<211>  1131
<212>  DNA
<213>  Saccharomyces cerevisiae

<400>  237
atgaccttgg cacccctaga cgcctccaaa gttaagataa ctaccacaca acatgcatct      60

aagccaaaac cgaacagtga gttagtgttt ggcaagagct tcacggacca catgttaact     120

gcggaatgga cagctgaaaa agggtggggt accccagaga ttaaacctta tcaaaatctg     180

tctttagacc cttccgcggt ggttttccat tatgcttttg agctattcga agggatgaag     240

gcttacagaa cggtggacaa caaaattaca atgtttcgtc cagatatgaa tatgaagcgc     300

atgaataagt ctgctcagag aatctgtttg ccaacgttcg acccagaaga gttgattacc     360

ctaattggga aactgatcca gcaagataag tgcttagttc ctgaaggaaa aggttactct     420

ttatatatca ggcctacatt aatcggcact acggccggtt taggggtttc cacgcctgat     480

agagccttgc tatatgtcat ttgctgccct gtgggtcctt attacaaaac tggatttaag     540

gcggtcagac tggaagccac tgattatgcc acaagagctt ggccaggagg ctgtggtgac     600

aagaaactag gtgcaaacta cgccccctgc gtcctgccac aattgcaagc tgcttcaagg     660

ggttaccaac aaaatttatg gctatttggt ccaaataaca acattactga agtcggcacc     720

atgaatgctt ttttcgtgtt taaagatagt aaaacgggca agaaggaact agttactgct     780

ccactagacg gtaccatttt ggaaggtgtt actagggatt ccattttaaa tcttgctaaa 840

gaaagactcg aaccaagtga atggaccatt agtgaacgct acttcactat aggcgaagtt 900

actgagagat ccaagaacgg tgaactactt gaagcctttg gttctggtac tgctgcgatt 960

gtttctccca ttaaggaaat cggctggaaa ggcgaacaaa ttaatattcc gttgttgccc 1020

ggcgaacaaa ccggtccatt ggccaaagaa gttgcacaat ggattaatgg aatccaatat 1080

ggcgagactg agcatggcaa ttggtcaagg gttgttactg atttgaactg a 1131

<210> 238
<211> 376
<212> PRT
<213> Saccharomyces cerevisiae

<400> 238

Met Thr Leu Ala Pro Leu Asp Ala Ser Lys Val Lys Ile Thr Thr Thr
1               5                   10                  15

Gln His Ala Ser Lys Pro Lys Pro Asn Ser Glu Leu Val Phe Gly Lys
            20                  25                  30

Ser Phe Thr Asp His Met Leu Thr Ala Glu Trp Thr Ala Glu Lys Gly
        35                  40                  45

Trp Gly Thr Pro Glu Ile Lys Pro Tyr Gln Asn Leu Ser Leu Asp Pro
    50                  55                  60

Ser Ala Val Val Phe His Tyr Ala Phe Glu Leu Phe Glu Gly Met Lys
65                  70                  75                  80

Ala Tyr Arg Thr Val Asp Asn Lys Ile Thr Met Phe Arg Pro Asp Met
                85                  90                  95

Asn Met Lys Arg Met Asn Lys Ser Ala Gln Arg Ile Cys Leu Pro Thr
            100                 105                 110

Phe Asp Pro Glu Glu Leu Ile Thr Leu Ile Gly Lys Leu Ile Gln Gln
        115                 120                 125

Asp Lys Cys Leu Val Pro Glu Gly Lys Gly Tyr Ser Leu Tyr Ile Arg
    130                 135                 140

Pro Thr Leu Ile Gly Thr Thr Ala Gly Leu Gly Val Ser Thr Pro Asp
145                 150                 155                 160

Arg Ala Leu Leu Tyr Val Ile Cys Cys Pro Val Gly Pro Tyr Tyr Lys
                165                 170                 175

Thr Gly Phe Lys Ala Val Arg Leu Glu Ala Thr Asp Tyr Ala Thr Arg

180                 185                 190

Ala Trp Pro Gly Gly Cys Gly Asp Lys Lys Leu Gly Ala Asn Tyr Ala
        195                 200                 205

Pro Cys Val Leu Pro Gln Leu Gln Ala Ala Ser Arg Gly Tyr Gln Gln
    210                 215                 220

Asn Leu Trp Leu Phe Gly Pro Asn Asn Asn Ile Thr Glu Val Gly Thr
225                 230                 235                 240

Met Asn Ala Phe Phe Val Phe Lys Asp Ser Lys Thr Gly Lys Lys Glu
                245                 250                 255

Leu Val Thr Ala Pro Leu Asp Gly Thr Ile Leu Glu Gly Val Thr Arg
            260                 265                 270

Asp Ser Ile Leu Asn Leu Ala Lys Glu Arg Leu Glu Pro Ser Glu Trp
        275                 280                 285

Thr Ile Ser Glu Arg Tyr Phe Thr Ile Gly Glu Val Thr Glu Arg Ser
    290                 295                 300

Lys Asn Gly Glu Leu Leu Glu Ala Phe Gly Ser Gly Thr Ala Ala Ile
305                 310                 315                 320

Val Ser Pro Ile Lys Glu Ile Gly Trp Lys Gly Glu Gln Ile Asn Ile
                325                 330                 335

Pro Leu Leu Pro Gly Glu Gln Thr Gly Pro Leu Ala Lys Glu Val Ala
            340                 345                 350

Gln Trp Ile Asn Gly Ile Gln Tyr Gly Glu Thr Glu His Gly Asn Trp
        355                 360                 365

Ser Arg Val Val Thr Asp Leu Asn
    370                 375

<210>  239
<211>  993
<212>  DNA
<213>  Methanobacterium thermoautotrophicum

<400>  239
   tcagatgtag gtgagccatc cgaagctgtc ctctgtctct gccctgatta tcctgaagaa      60
   ctcatcctgc agcagctttg taacggaccc ccttcgcccg gcacctatct ctataccatc     120
   aactgatctg atgggtgtta tctctgcggc tgtacctgtg aagaaggcct catctgcgat     180
   gtagagcatc tccctggtta tgggttcctc atgcacggta acaccctcgg tcctggctat     240
   ctttattacg gagtcccttg ttatccccct cagaagggat gatgaaacag ggggggtgta     300
   aatttcaccc tcactgacga ggaatatgtt ctccccgcta ccctcactta tgtagccatg     360
   gtagtccagc attatggcct catcatagcc gtgtctcaca gcctccatct tggcaagctg     420
   tgagttgagg tagttaccgc cggcctttgc catgttgggc attgtgtttg gtgccatcct     480
   ccgccaggtt gaaacaccag catcgacacc aacctcaagg gcctctgcac ccagataggc     540
   cccccattcc caggcagcca cagcgacgtc cactgggcag ttcaccgggt gaacacccat     600
   ctcaccgtat cccctgaata ccacgggtct tatatagcac tcctcaagtc cgttctccct     660
   gacggtctca actatggcat cacatatctg ctcctgggtg tagggtatgt ccatccggta     720
   tatctttgca gaatcaaaaa ggcgtttaac atgctcccgc aaacggaaga tggctgaccc     780
   cttactgttc ctgtagcacc ttattccctc aaagacagat gatccataat gcacaacatg     840
   tgagagtacg tggacggtgg cttcttccca ttcaaccatt tcaccgttta accatatctt     900
   tccactggct tcgcatgaca tgataataac ctcaggtgat ttactaggat aggttatggt     960
   tggaggccta tataatgctc tccataaccg caa                                  993

<210>  240
<211>  330
<212>  PRT
<213>  Methanobacterium thermoautotrophicum

<400>  240

Met Arg Leu Trp Arg Ala Leu Tyr Arg Pro Pro Thr Ile Thr Tyr Pro
1               5                   10                  15

Ser Lys Ser Pro Glu Val Ile Ile Met Ser Cys Glu Ala Ser Gly Lys
            20                  25                  30

Ile Trp Leu Asn Gly Glu Met Val Glu Trp Glu Glu Ala Thr Val His
        35                  40                  45

Val Leu Ser His Val Val His Tyr Gly Ser Ser Val Phe Glu Gly Ile
    50                  55                  60

Arg Cys Tyr Arg Asn Ser Lys Gly Ser Ala Ile Phe Arg Leu Arg Glu
65                  70                  75                  80

His Val Lys Arg Leu Phe Asp Ser Ala Lys Ile Tyr Arg Met Asp Ile
                85                  90                  95

Pro Tyr Thr Gln Glu Gln Ile Cys Asp Ala Ile Val Glu Thr Val Arg
            100                 105                 110

Glu Asn Gly Leu Glu Glu Cys Tyr Ile Arg Pro Val Val Phe Arg Gly
        115                 120                 125

Tyr Gly Glu Met Gly Val His Pro Val Asn Cys Pro Val Asp Val Ala
    130                 135                 140

Val Ala Ala Trp Glu Trp Gly Ala Tyr Leu Gly Ala Glu Ala Leu Glu
145                 150                 155                 160

Val Gly Val Asp Ala Gly Val Ser Thr Trp Arg Arg Met Ala Pro Asn
                165                 170                 175

Thr Met Pro Asn Met Ala Lys Ala Gly Gly Asn Tyr Leu Asn Ser Gln
            180                 185                 190

Leu Ala Lys Met Glu Ala Val Arg His Gly Tyr Asp Glu Ala Ile Met
        195                 200                 205

Leu Asp Tyr His Gly Tyr Ile Ser Glu Gly Ser Gly Glu Asn Ile Phe
    210                 215                 220

Leu Val Ser Glu Gly Glu Ile Tyr Thr Pro Pro Val Ser Ser Ser Leu
225                 230                 235                 240

Leu Arg Gly Ile Thr Arg Asp Ser Val Ile Lys Ile Ala Arg Thr Glu
                245                 250                 255

Gly Val Thr Val His Glu Glu Pro Ile Thr Arg Glu Met Leu Tyr Ile
            260                 265                 270

Ala Asp Glu Ala Phe Phe Thr Gly Thr Ala Ala Glu Ile Thr Pro Ile
        275                 280                 285

Arg Ser Val Asp Gly Ile Glu Ile Gly Ala Gly Arg Arg Gly Pro Val
    290                 295                 300

Thr Lys Leu Leu Gln Asp Glu Phe Phe Arg Ile Ile Arg Ala Glu Thr
305                 310                 315                 320

Glu Asp Ser Phe Gly Trp Leu Thr Tyr Ile
                325                 330

<210>  241
<211>  1095
<212>  DNA
<213>  Streptomyces coelicolor

<400>  241
tcacggccgg ggacgggcct ccgccatccg ctgctcggcg atccggtcgg ccgccgcggc      60

cggcggaata ccgtcctcct tcgcacgtgc gaatatggcc agcgtggtgt cgtagatctt     120

cgaggccttc gccttgcacc ggtcgaagtc gaacccgtgc agctcgtcgg cgacctggat     180

gacaccgccg gcgttcacca catagtccgg cgcgtagagg atcccgcggt cggcgaggtc 240

cttctcgacg cccgggtggg cgagctggtt gttggccgcg ccgcacacca ccttggcggt 300

cagcaccggc acggtgtcgt cgttcagcgc gccgccgagc gcgcagggcg cgtagatgtc 360

caggttctcc acccggatca gcgcgtcggt gtcggcgacg gcgaccaccg acgggtgccg 420

ctccgtgatc ccgcgcacca cgtccttgcg cacgtccgtg acgacgacgt gggcgccctc 480

ggcgagcagg tgctcgacca ggtggtggcc gaccttgccg acgcccgcga tgccgacggt 540

gcggtcgcgc agcgtcgggt cgccccacag gtgctgggcg gcggcccgca tgccctggta 600

gacgccgaag gaggtgagca cggaggagtc gcccgcgccg ccgttctccg ggaacgcccc 660

ggtcgtccag cggcactcgc gggccacgac gtccatgtcg gcgacgtagg tgccgacgtc 720

gcacgcggtg acgtagcggc cgcccagcga ggcgacgaac cggccgtagg cgaggagcag 780

ctcctcgctc ttgatctgct ccggatcgcc gatgatcacg gccttgccgc caccgtggtc 840

cagaccggcc atggcgttct tgtacgacat cccgcgggcg aggttcagcg cgtcggcgac 900

ggcctccgcc tcgctcgcgt acgggtagaa gcgggtaccg ccgagcgccg ggcccagggc 960

ggtggagtgg agggcgatca cggccttgag gccgctggca cggtcctggc agagcacgac 1020

ttgctcatgt cccccctgat ccgagtggaa cagggtgtgc agtacatcag caggtgcgcc 1080

gtttacgtcg gtcac 1095

<210> 242
<211> 364
<212> PRT
<213> Streptomyces coelicolor

<400> 242

Met Thr Asp Val Asn Gly Ala Pro Ala Asp Val Leu His Thr Leu Phe
1               5                   10                  15

His Ser Asp Gln Gly Gly His Glu Gln Val Val Leu Cys Gln Asp Arg
            20                  25                  30

Ala Ser Gly Leu Lys Ala Val Ile Ala Leu His Ser Thr Ala Leu Gly
        35                  40                  45

Pro Ala Leu Gly Gly Thr Arg Phe Tyr Pro Tyr Ala Ser Glu Ala Glu
    50                  55                  60

Ala Val Ala Asp Ala Leu Asn Leu Ala Arg Gly Met Ser Tyr Lys Asn
65                  70                  75                  80

Ala Met Ala Gly Leu Asp His Gly Gly Gly Lys Ala Val Ile Ile Gly
                85                  90                  95

Asp Pro Glu Gln Ile Lys Ser Glu Glu Leu Leu Leu Ala Tyr Gly Arg

            100                 105                 110

Phe Val Ala Ser Leu Gly Gly Arg Tyr Val Thr Ala Cys Asp Val Gly
        115                 120                 125

Thr Tyr Val Ala Asp Met Asp Val Val Ala Arg Glu Cys Arg Trp Thr
    130                 135                 140

Thr Gly Arg Ser Pro Glu Asn Gly Gly Ala Gly Asp Ser Ser Val Leu
145                 150                 155                 160

Thr Ser Phe Gly Val Tyr Gln Gly Met Arg Ala Ala Ala Gln His Leu
                165                 170                 175

Trp Gly Asp Pro Thr Leu Arg Asp Arg Thr Val Gly Ile Ala Gly Val
            180                 185                 190

Gly Lys Val Gly His His Leu Val Glu His Leu Leu Ala Glu Gly Ala
        195                 200                 205

His Val Val Val Thr Asp Val Arg Lys Asp Val Val Arg Gly Ile Thr
    210                 215                 220

Glu Arg His Pro Ser Val Val Ala Val Ala Asp Thr Asp Ala Leu Ile
225                 230                 235                 240

Arg Val Glu Asn Leu Asp Ile Tyr Ala Pro Cys Ala Leu Gly Gly Ala
                245                 250                 255

Leu Asn Asp Asp Thr Val Pro Val Leu Thr Ala Lys Val Val Cys Gly
            260                 265                 270

Ala Ala Asn Asn Gln Leu Ala His Pro Gly Val Glu Lys Asp Leu Ala
        275                 280                 285

Asp Arg Gly Ile Leu Tyr Ala Pro Asp Tyr Val Val Asn Ala Gly Gly
    290                 295                 300

Val Ile Gln Val Ala Asp Glu Leu His Gly Phe Asp Phe Asp Arg Cys
305                 310                 315                 320

Lys Ala Lys Ala Ser Lys Ile Tyr Asp Thr Thr Leu Ala Ile Phe Ala
                325                 330                 335

Arg Ala Lys Glu Asp Gly Ile Pro Pro Ala Ala Ala Ala Asp Arg Ile
            340                 345                 350

Ala Glu Gln Arg Met Ala Glu Ala Arg Pro Arg Pro
        355                 360

<210>  243
<211>  1095
<212>  DNA
<213>  Bacillus subtilis

<400>  243
atggaacttt ttaaatatat ggagaaatac gattacgaac aattggtatt ctgccaggat      60

gaacaatctg gattaaaagc gattatcgcc attcatgata caacgcttgg tccggcgctt     120

ggcggaacga gaatgtggac atatgaaaat gaagaagcgg caattgaaga tgcgctcaga     180

ttggcaagag gcatgaccta taagaacgcg gcggcaggct taaaccttgg cggcggaaaa     240

acagtcatta tcggcgatcc gcgcaaagac aaaaatgagg aaatgttccg cgcgtttggc     300

cgctatattc aaggactgaa tggcagatac atcacggctg aagatgtggg cacaacggtc     360

gaggatatgg atatcattca tgatgagaca gactatgtca cagggatttc tcctgctttc     420

ggctcttctg gaaatccgtc cccagtcaca gcgtacgggg tgtacagagg aatgaaggca     480

gcagctaaag ctgctttcgg aaccgattct cttgaaggaa aaaccattgc tgtacagggt     540

gttgggaacg tagcctataa cctttgccgc cacctgcatg aagaaggagc aaacttaatc     600

gttacggata tcaacaaaca atctgtacag cgtgcagttg aagattttgg cgcccgtgcg     660

gtagatcctg atgacattta ttcacaagac tgcgatattt atgcgccgtg tgcccttggt     720

gcgactatta acgacgacac cattaaacag ctgaaggcga aagtgatcgc aggtgcggct     780

aacaaccaat taaaagagac acgccatggt gatcaaattc acgaaatggg catcgtttat     840

gcaccggatt acgtgattaa cgcgggcggt gtcatcaacg tggcagatga gctttacggc     900

tataatgcag aacgtgcatt gaaaaaagtt gaaggcattt acggcaatat cgagcgtgta     960

cttgagattt ctcagcgtga cggcattcct gcatatttag cggctgaccg cttagcagag    1020

gaacggattg aacgcatgcg ccgctcaaga agccagtttt tgcaaaacgg ccacagtgta    1080

ttaagcagac gttaa                                                     1095

<210>  244
<211>  364
<212>  PRT
<213>  Bacillus subtilis

<400>  244

Met Glu Leu Phe Lys Tyr Met Glu Lys Tyr Asp Tyr Glu Gln Leu Val
1               5                   10                  15

Phe Cys Gln Asp Glu Gln Ser Gly Leu Lys Ala Ile Ile Ala Ile His
            20                  25                  30

Asp Thr Thr Leu Gly Pro Ala Leu Gly Gly Thr Arg Met Trp Thr Tyr
        35                  40                  45

Glu Asn Glu Glu Ala Ala Ile Glu Asp Ala Leu Arg Leu Ala Arg Gly
    50                  55                  60

Met Thr Tyr Lys Asn Ala Ala Ala Gly Leu Asn Leu Gly Gly Gly Lys
65                  70                  75                  80

Thr Val Ile Ile Gly Asp Pro Arg Lys Asp Lys Asn Glu Glu Met Phe
                85                  90                  95

Arg Ala Phe Gly Arg Tyr Ile Gln Gly Leu Asn Gly Arg Tyr Ile Thr
            100                 105                 110

Ala Glu Asp Val Gly Thr Thr Val Glu Asp Met Asp Ile Ile His Asp
        115                 120                 125

Glu Thr Asp Tyr Val Thr Gly Ile Ser Pro Ala Phe Gly Ser Ser Gly
    130                 135                 140

Asn Pro Ser Pro Val Thr Ala Tyr Gly Val Tyr Arg Gly Met Lys Ala
145                 150                 155                 160

Ala Ala Lys Ala Ala Phe Gly Thr Asp Ser Leu Glu Gly Lys Thr Ile
                165                 170                 175

Ala Val Gln Gly Val Gly Asn Val Ala Tyr Asn Leu Cys Arg His Leu
            180                 185                 190

His Glu Glu Gly Ala Asn Leu Ile Val Thr Asp Ile Asn Lys Gln Ser
        195                 200                 205

Val Gln Arg Ala Val Glu Asp Phe Gly Ala Arg Ala Val Asp Pro Asp
    210                 215                 220

Asp Ile Tyr Ser Gln Asp Cys Asp Ile Tyr Ala Pro Cys Ala Leu Gly
225                 230                 235                 240

Ala Thr Ile Asn Asp Asp Thr Ile Lys Gln Leu Lys Ala Lys Val Ile
                245                 250                 255

Ala Gly Ala Ala Asn Asn Gln Leu Lys Glu Thr Arg His Gly Asp Gln
            260                 265                 270

Ile His Glu Met Gly Ile Val Tyr Ala Pro Asp Tyr Val Ile Asn Ala
        275                 280                 285

Gly Gly Val Ile Asn Val Ala Asp Glu Leu Tyr Gly Tyr Asn Ala Glu
    290                 295                 300

Arg Ala Leu Lys Lys Val Glu Gly Ile Tyr Gly Asn Ile Glu Arg Val
305                 310                 315                 320

Leu Glu Ile Ser Gln Arg Asp Gly Ile Pro Ala Tyr Leu Ala Ala Asp
                325                 330                 335

Arg Leu Ala Glu Glu Arg Ile Glu Arg Met Arg Arg Ser Arg Ser Gln
            340                 345                 350

Phe Leu Gln Asn Gly His Ser Val Leu Ser Arg Arg
        355                 360

<210>  245
<211>  1785
<212>  DNA
<213>  Streptomyces viridifaciens

<400>  245
   gtgtcaactt cctccgcttc ttccgggccg gacctcccct tcgggcccga ggacacgcca      60
   tggcagaagg ccttcagcag gctgcgggcg gtggatggcg tgccgcgcgt caccgcgccg     120
   tccagtgatc cgcgtgaggt ctacatggac atcccggaga tccccttctc caaggtccag     180
   atcccccggg acggaatgga cgagcagcag tacgcagagg ccgagagcct cttccgccgc     240
   tacgtagacg cccagacccg caacttcgcg ggataccagg tcaccagcga cctcgactac     300
   cagcacctca gtcactatct caaccggcat ctgaacaacg tcggcgatcc ctatgagtcc     360
   agctcctaca cgctgaactc caaggtcctt gagcgagccg ttctcgacta cttcgcctcc     420
   ctgtggaacg ccaagtggcc ccatgacgca agcgatccgg aaacgtactg gggttacgtg     480
   ctgaccatgg gctccagcga aggcaacctg tacgggttgt ggaacgcacg ggactatctg     540
   tcgggcaagc tgctgcggcg ccagcaccgg gaggccggcg gcgacaaggc ctcggtcgtc     600
   tacacgcaag cgctgcgaca cgaagggcag agtccgcatg cctacgagcc ggtggcgttc     660
   ttctcgcagg acacgcacta ctcgctcacg aaggccgtgc gggttctggg catcgacacc     720
   ttccacagca tcggcagcag tcggtatccg gacgagaacc cgctgggccc cggcactccg     780
   tggccgaccg aagtgccctc ggttgacggt gccatcgatg tcgacaaact cgcctcgttg     840
   gtccgcttct tcgccagcaa gggctacccg atactggtca gcctcaacta cgggtcaacg     900
   ttcaagggcg cctacgacga cgtcccggcc gtggcacagg ccgtgcggga catctgcacg     960
   gaatacggtc tggatcggcg gcgggtatac cacgaccgca gtaaggacag tgacttcgac    1020
   gagcgcagcg gcttctggat ccacatcgat gccgccctgg gggcgggcta cgctccctac    1080
   ctgcagatgg cccgggatgc cggcatggtc gaggaggcgc cgcccgtttt cgacttccgg    1140
   ctcccggagg tgcactcgct gaccatgagc ggccacaagt ggatgggaac accgtgggca    1200
   tgcggtgtct acatgacacg gaccgggctg cagatgaccc cgccgaagtc gtccgagtac    1260
   atcggggcgg ccgacaccac cttcgcgggc tcccgcaacg gcttctcgtc actgctgctg    1320
   tgggactacc tgtcccggca ttcgtatgac gatctggtgc gcctggccgc cgactgcgac 1380
   cggctggccg gctacgccca cgaccggttg ctgaccttgc aggacaaact cggcatggat 1440
   ctgtgggtcg cccgcagccc gcagtccctc acggtgcgct tccgtcagcc atgtgcagac 1500
   atcgtccgca agtactcgct gtcgtgtgag acggtctacg aagacaacga gcaacggacc 1560
   tacgtacatc tctacgccgt tccccacctc actcgggaac tcgtggatga gctcgtgcgc 1620
   gatctgcgcc agcccggagc cttcaccaac gctggtgcac tggaggggga ggcctgggcc 1680
   ggggtgatcg atgccctcgg ccgcccggac cccgacggaa cctatgccgg cgccttgagc 1740
   gctccggctt ccggcccccg ctccgaggac ggcggcggga gctga 1785

<210> 246
<211> 594
<212> PRT
<213> Streptomyces viridifaciens

<400> 246

Met Ser Thr Ser Ser Ala Ser Ser Gly Pro Asp Leu Pro Phe Gly Pro
1               5                   10                  15

Glu Asp Thr Pro Trp Gln Lys Ala Phe Ser Arg Leu Arg Ala Val Asp
            20                  25                  30

Gly Val Pro Arg Val Thr Ala Pro Ser Ser Asp Pro Arg Glu Val Tyr
        35                  40                  45

Met Asp Ile Pro Glu Ile Pro Phe Ser Lys Val Gln Ile Pro Pro Asp
    50                  55                  60

Gly Met Asp Glu Gln Gln Tyr Ala Glu Ala Glu Ser Leu Phe Arg Arg
65                  70                  75                  80

Tyr Val Asp Ala Gln Thr Arg Asn Phe Ala Gly Tyr Gln Val Thr Ser
                85                  90                  95

Asp Leu Asp Tyr Gln His Leu Ser His Tyr Leu Asn Arg His Leu Asn
            100                 105                 110

Asn Val Gly Asp Pro Tyr Glu Ser Ser Ser Tyr Thr Leu Asn Ser Lys
        115                 120                 125

Val Leu Glu Arg Ala Val Leu Asp Tyr Phe Ala Ser Leu Trp Asn Ala
    130                 135                 140

Lys Trp Pro His Asp Ala Ser Asp Pro Glu Thr Tyr Trp Gly Tyr Val
145                 150                 155                 160

Leu Thr Met Gly Ser Ser Glu Gly Asn Leu Tyr Gly Leu Trp Asn Ala
                165                 170                 175

Arg Asp Tyr Leu Ser Gly Lys Leu Leu Arg Arg Gln His Arg Glu Ala
            180                 185                 190

Gly Gly Asp Lys Ala Ser Val Val Tyr Thr Gln Ala Leu Arg His Glu
        195                 200                 205

Gly Gln Ser Pro His Ala Tyr Glu Pro Val Ala Phe Phe Ser Gln Asp
    210                 215                 220

Thr His Tyr Ser Leu Thr Lys Ala Val Arg Val Leu Gly Ile Asp Thr
225                 230                 235                 240

Phe His Ser Ile Gly Ser Ser Arg Tyr Pro Asp Glu Asn Pro Leu Gly
                245                 250                 255

Pro Gly Thr Pro Trp Pro Thr Glu Val Pro Ser Val Asp Gly Ala Ile
            260                 265                 270

Asp Val Asp Lys Leu Ala Ser Leu Val Arg Phe Phe Ala Ser Lys Gly
        275                 280                 285

Tyr Pro Ile Leu Val Ser Leu Asn Tyr Gly Ser Thr Phe Lys Gly Ala
    290                 295                 300

Tyr Asp Asp Val Pro Ala Val Ala Gln Ala Val Arg Asp Ile Cys Thr
305                 310                 315                 320

Glu Tyr Gly Leu Asp Arg Arg Arg Val Tyr His Asp Arg Ser Lys Asp
                325                 330                 335

Ser Asp Phe Asp Glu Arg Ser Gly Phe Trp Ile His Ile Asp Ala Ala
            340                 345                 350

Leu Gly Ala Gly Tyr Ala Pro Tyr Leu Gln Met Ala Arg Asp Ala Gly
        355                 360                 365

Met Val Glu Glu Ala Pro Pro Val Phe Asp Phe Arg Leu Pro Glu Val
    370                 375                 380

His Ser Leu Thr Met Ser Gly His Lys Trp Met Gly Thr Pro Trp Ala
385                 390                 395                 400

Cys Gly Val Tyr Met Thr Arg Thr Gly Leu Gln Met Thr Pro Pro Lys
                405                 410                 415

Ser Ser Glu Tyr Ile Gly Ala Ala Asp Thr Thr Phe Ala Gly Ser Arg

420                 425                 430

Asn Gly Phe Ser Ser Leu Leu Leu Trp Asp Tyr Leu Ser Arg His Ser
        435                 440                 445

Tyr Asp Asp Leu Val Arg Leu Ala Ala Asp Cys Asp Arg Leu Ala Gly
    450                 455                 460

Tyr Ala His Asp Arg Leu Leu Thr Leu Gln Asp Lys Leu Gly Met Asp
465                 470                 475                 480

Leu Trp Val Ala Arg Ser Pro Gln Ser Leu Thr Val Arg Phe Arg Gln
                485                 490                 495

Pro Cys Ala Asp Ile Val Arg Lys Tyr Ser Leu Ser Cys Glu Thr Val
            500                 505                 510

Tyr Glu Asp Asn Glu Gln Arg Thr Tyr Val His Leu Tyr Ala Val Pro
        515                 520                 525

His Leu Thr Arg Glu Leu Val Asp Glu Leu Val Arg Asp Leu Arg Gln
    530                 535                 540

Pro Gly Ala Phe Thr Asn Ala Gly Ala Leu Glu Gly Glu Ala Trp Ala
545                 550                 555                 560

Gly Val Ile Asp Ala Leu Gly Arg Pro Asp Pro Asp Gly Thr Tyr Ala
                565                 570                 575

Gly Ala Leu Ser Ala Pro Ala Ser Gly Pro Arg Ser Glu Asp Gly Gly
            580                 585                 590

Gly Ser

<210>  247
<211>  1323
<212>  DNA
<213>  Alcaligenes denitrificans

<400>  247
atgagcgctg ccaaactgcc cgacctgtcc cacctctgga tgccctttac cgccaaccgg      60

cagttcaagg cgaacccccg cctgctggcc tcggccaagg gcatgtacta cacgtctttc     120

gacggccgcc agatcctgga cggcacggcc ggcctgtggt gcgtgaacgc cggccactgc     180

cgcgaagaaa tcgtctccgc catcgccagc caggccggcg tcatggacta cgcgccgggg     240

ttccagctcg gccacccgct ggccttcgag gccgccaccg ccgtggccgg cctgatgccg     300

cagggcctgg accgcgtgtt cttcaccaat tcgggctccg aatcggtgga caccgcgctg     360

aagatcgccc tggcctacca ccgcgcgcgc ggcgaggcgc agcgcacccg cctcatcggg 420

cgcgagcgcg gctaccacgg cgtgggcttc ggcggcattt ccgtgggcgg catctcgccc 480

aaccgcaaga ccttctccgg cgcgctgctg ccggccgtgg accacctgcc gcacacccac 540

agcctggaac acaacgcctt cacgcgcggc cagcccgagt ggggcgcgca cctggccgac 600

gagttggaac gcatcatcgc cctgcacgac gcctccacca tcgcggccgt gatcgtcgag 660

cccatggccg gctccaccgg cgtgctcgtc ccgcccaagg gctatctcga aaaactgcgc 720

gaaatcaccg cccgccacgg cattctgctg atcttcgacg aagtcatcac cgcgtacggc 780

cgcctgggcg aggccaccgc cgcggcctat ttcggcgtaa cgcccgacct catcaccatg 840

gccaagggcg tgagcaacgc cgccgttccg gccggcgccg tcgcggtgcg ccgcgaagtg 900

catgacgcca tcgtcaacgg accgcaaggc ggcatcgagt tcttccacgg ctacacctac 960

tcggcccacc cgctggccgc cgccgccgtg ctcgccacgc tggacatcta ccgccgcgaa 1020

gacctgttcg cccgcgcccg caagctgtcg gccgcgttcg aggaagccgc ccacagcctc 1080

aagggcgcgc cgcacgtcat cgacgtgcgc aacatcggcc tggtggccgg catcgagctg 1140

tcgccgcgcg aaggcgcccc gggcgcgcgc gccgccgaag ccttccagaa atgcttcgac 1200

accggcctca tggtgcgcta cacgggcgac atcctcgcgg tgtcgcctcc gctcatcgtc 1260

gacgaaaacc agatcggcca gatcttcgag ggcatcggca aggtgctcaa ggaagtggct 1320

tag 1323

<210> 248
<211> 440
<212> PRT
<213> Alcaligenes denitrificans

<400> 248

Met Ser Ala Ala Lys Leu Pro Asp Leu Ser His Leu Trp Met Pro Phe
1               5                   10                  15

Thr Ala Asn Arg Gln Phe Lys Ala Asn Pro Arg Leu Leu Ala Ser Ala
            20                  25                  30

Lys Gly Met Tyr Tyr Thr Ser Phe Asp Gly Arg Gln Ile Leu Asp Gly
        35                  40                  45

Thr Ala Gly Leu Trp Cys Val Asn Ala Gly His Cys Arg Glu Glu Ile
    50                  55                  60

Val Ser Ala Ile Ala Ser Gln Ala Gly Val Met Asp Tyr Ala Pro Gly
65                  70                  75                  80

Phe Gln Leu Gly His Pro Leu Ala Phe Glu Ala Ala Thr Ala Val Ala
                85                  90                  95

Gly Leu Met Pro Gln Gly Leu Asp Arg Val Phe Phe Thr Asn Ser Gly
            100                 105                 110

Ser Glu Ser Val Asp Thr Ala Leu Lys Ile Ala Leu Ala Tyr His Arg
        115                 120                 125

Ala Arg Gly Glu Ala Gln Arg Thr Arg Leu Ile Gly Arg Glu Arg Gly
    130                 135                 140

Tyr His Gly Val Gly Phe Gly Gly Ile Ser Val Gly Gly Ile Ser Pro
145                 150                 155                 160

Asn Arg Lys Thr Phe Ser Gly Ala Leu Leu Pro Ala Val Asp His Leu
                165                 170                 175

Pro His Thr His Ser Leu Glu His Asn Ala Phe Thr Arg Gly Gln Pro
            180                 185                 190

Glu Trp Gly Ala His Leu Ala Asp Glu Leu Glu Arg Ile Ile Ala Leu
        195                 200                 205

His Asp Ala Ser Thr Ile Ala Ala Val Ile Val Glu Pro Met Ala Gly
    210                 215                 220

Ser Thr Gly Val Leu Val Pro Pro Lys Gly Tyr Leu Glu Lys Leu Arg
225                 230                 235                 240

Glu Ile Thr Ala Arg His Gly Ile Leu Leu Ile Phe Asp Glu Val Ile
                245                 250                 255

Thr Ala Tyr Gly Arg Leu Gly Glu Ala Thr Ala Ala Ala Tyr Phe Gly
            260                 265                 270

Val Thr Pro Asp Leu Ile Thr Met Ala Lys Gly Val Ser Asn Ala Ala
        275                 280                 285

Val Pro Ala Gly Ala Val Ala Val Arg Arg Glu Val His Asp Ala Ile
    290                 295                 300

Val Asn Gly Pro Gln Gly Gly Ile Glu Phe Phe His Gly Tyr Thr Tyr
305                 310                 315                 320

Ser Ala His Pro Leu Ala Ala Ala Ala Val Leu Ala Thr Leu Asp Ile
                325                 330                 335

Tyr Arg Arg Glu Asp Leu Phe Ala Arg Ala Arg Lys Leu Ser Ala Ala
            340                 345                 350

Phe Glu Glu Ala Ala His Ser Leu Lys Gly Ala Pro His Val Ile Asp
        355                 360                 365

Val Arg Asn Ile Gly Leu Val Ala Gly Ile Glu Leu Ser Pro Arg Glu
    370                 375                 380

Gly Ala Pro Gly Ala Arg Ala Ala Glu Ala Phe Gln Lys Cys Phe Asp
385                 390                 395                 400

Thr Gly Leu Met Val Arg Tyr Thr Gly Asp Ile Leu Ala Val Ser Pro
                405                 410                 415

Pro Leu Ile Val Asp Glu Asn Gln Ile Gly Gln Ile Phe Glu Gly Ile
            420                 425                 430

Gly Lys Val Leu Lys Glu Val Ala
        435                 440

<210> 249
<211> 1332
<212> DNA
<213> Ralstonia eutropha

<400> 249
atggacgccg cgaagaccgt gattcccgat ctcgatgccc tgtggatgcc ctttaccgcg 60

aaccgccagt acaaggcggc gccgcgcctg ctggcctcgg ccagcggcat gtactacacc 120

acccacgacg gacgccagat cctcgacggt tgcgcgggcc tctggtgcgt agcggccggc 180

cactgccgca aggagattgc cgaggccgtg gcccgccagg ccgccacgct cgactacgcg 240

ccgccgttcc agatgggcca tccgctgtcg ttcgaagccg ccaccaaggt ggccgcgatc 300

atgccgcagg gactggaccg catcttcttc acgaattccg gttcggaatc ggtggacacc 360

gcgctgaaga ttgcgctggc ctaccaccgt gcgcgcggcg agggccagcg cacccgcttc 420

atcgggcgcg aacgcggtta ccacggcgtg ggctttggcg gcatggctgt cggtggcatc 480

gggccgaacc gcaaggcgtt ctcggccaac ctgatgccgg gcaccgacca tctgccggcg 540

acgctgaata tcgccgaagc ggcgttctcc aagggtcagc cgacatgggg cgcgcacctt 600

gccgacgaac tcgagcgcat cgtcgcgctg catgatccgt ccacgattgc cgccgtcatc 660

gtggaaccgc tggcgggctc cgccggggtg ctggtgccgc cggtcggcta cctcgacaag 720

ctgcgcgaga tcacgaccaa gcacggcatc ctgctgatct tcgacgaggt catcacggcc 780

tttggtcgcc tgggtaccgc caccgcggcg gaacgcttca aggtcacgcc ggacctgatc 840

accatggcca aggccatcaa caacgccgcc gtgccgatgg gtgccgtggc cgtgcgccgc 900

gaagtccatg acaccgtggt caactcggcc gcgccgggcg cgatcgaact cgcgcatggc 960

tacacctact cgggccaccc gctggccgcc gccgctgcca tcgccacgct ggacctgtat 1020

cagcgcgaga acctgttcgg ccgtgccgcg gagctgtcgc cggtgttcga agcggccgtt 1080

cacagcgtac gcagcgcgcc gcatgtgaag gacatccgca acctcggcat ggtggccggc 1140

atcgagctgg agccgcgtcc gggccagccc ggcgcacgcg cctacgaagc cttcctcaaa 1200

tgccttgagc gtggcgtgct ggtgcgctac accggcgata tcctcgcgtt ctcgccgccg 1260

ctgatcatca gcgaggcgca gattgccgag ctgttcgata cggtcaagca ggccttgcag 1320

gaagtgcagt aa 1332

<210> 250
<211> 443
<212> PRT
<213> Ralstonia eutropha

<400> 250

Met Asp Ala Ala Lys Thr Val Ile Pro Asp Leu Asp Ala Leu Trp Met
1               5                   10                  15

Pro Phe Thr Ala Asn Arg Gln Tyr Lys Ala Ala Pro Arg Leu Leu Ala
            20                  25                  30

Ser Ala Ser Gly Met Tyr Tyr Thr Thr His Asp Gly Arg Gln Ile Leu
        35                  40                  45

Asp Gly Cys Ala Gly Leu Trp Cys Val Ala Ala Gly His Cys Arg Lys
    50                  55                  60

Glu Ile Ala Glu Ala Val Ala Arg Gln Ala Ala Thr Leu Asp Tyr Ala
65                  70                  75                  80

Pro Pro Phe Gln Met Gly His Pro Leu Ser Phe Glu Ala Ala Thr Lys
                85                  90                  95

Val Ala Ala Ile Met Pro Gln Gly Leu Asp Arg Ile Phe Phe Thr Asn
            100                 105                 110

Ser Gly Ser Glu Ser Val Asp Thr Ala Leu Lys Ile Ala Leu Ala Tyr
        115                 120                 125

His Arg Ala Arg Gly Glu Gly Gln Arg Thr Arg Phe Ile Gly Arg Glu
    130                 135                 140

Arg Gly Tyr His Gly Val Gly Phe Gly Gly Met Ala Val Gly Gly Ile
145                 150                 155                 160

Gly Pro Asn Arg Lys Ala Phe Ser Ala Asn Leu Met Pro Gly Thr Asp
                165                 170                 175

His Leu Pro Ala Thr Leu Asn Ile Ala Glu Ala Ala Phe Ser Lys Gly
            180                 185                 190

Gln Pro Thr Trp Gly Ala His Leu Ala Asp Glu Leu Glu Arg Ile Val
        195                 200                 205

Ala Leu His Asp Pro Ser Thr Ile Ala Ala Val Ile Val Glu Pro Leu
    210                 215                 220

Ala Gly Ser Ala Gly Val Leu Val Pro Pro Val Gly Tyr Leu Asp Lys
225                 230                 235                 240

Leu Arg Glu Ile Thr Thr Lys His Gly Ile Leu Leu Ile Phe Asp Glu
                245                 250                 255

Val Ile Thr Ala Phe Gly Arg Leu Gly Thr Ala Thr Ala Ala Glu Arg
            260                 265                 270

Phe Lys Val Thr Pro Asp Leu Ile Thr Met Ala Lys Ala Ile Asn Asn
        275                 280                 285

Ala Ala Val Pro Met Gly Ala Val Ala Val Arg Arg Glu Val His Asp
    290                 295                 300

Thr Val Val Asn Ser Ala Ala Pro Gly Ala Ile Glu Leu Ala His Gly
305                 310                 315                 320

Tyr Thr Tyr Ser Gly His Pro Leu Ala Ala Ala Ala Ala Ile Ala Thr
                325                 330                 335

Leu Asp Leu Tyr Gln Arg Glu Asn Leu Phe Gly Arg Ala Ala Glu Leu
            340                 345                 350

Ser Pro Val Phe Glu Ala Ala Val His Ser Val Arg Ser Ala Pro His
        355                 360                 365

Val Lys Asp Ile Arg Asn Leu Gly Met Val Ala Gly Ile Glu Leu Glu
    370                 375                 380

Pro Arg Pro Gly Gln Pro Gly Ala Arg Ala Tyr Glu Ala Phe Leu Lys
385                 390                 395                 400

Cys Leu Glu Arg Gly Val Leu Val Arg Tyr Thr Gly Asp Ile Leu Ala
                405                 410                 415

Phe Ser Pro Pro Leu Ile Ile Ser Glu Ala Gln Ile Ala Glu Leu Phe
            420                 425                 430

Asp Thr Val Lys Gln Ala Leu Gln Glu Val Gln
        435                 440

<210>  251
<211>  1341
<212>  DNA
<213>  Shewanella oneidensis

<400>  251
atggccgact cacccaacaa cctcgctcac gaacatcctt cacttgaaca ctattggatg      60

ccttttaccg ccaatcgcca attcaaagcg agccctcgtt tactcgccca agctgaaggt     120

atgtattaca cagatatcaa tggcaacaag gtattagact ctacagcggg cttatggtgt     180

tgtaatgctg gccatggtcg ccgtgagatc agtgaagccg tcagcaaaca aattcggcag     240

atggattacg ctccctcctt ccaaatgggc catcccatcg cttttgaact ggccgaacgt     300

ttaaccgaac tcagcccaga aggactcaac aaagtattct ttaccaactc aggctctgag     360

tcggttgata ccgcgctaaa aatggctctt tgctaccata gagccaatgg ccaagcgtca     420

cgcacccgct ttattggccg tgaaatgggt taccatggcg taggatttgg tgggatctcg     480

gtgggtggtt taagcaataa ccgtaaagcc ttcagcggcc agctattgca aggcgtggat     540

cacctgcccc acaccttaga cattcaacat gccgccttta gtcgtggctt accgagcctc     600

ggtgctgaaa aagctgaggt attagaacaa ttagtcacac tccatggcgc cgaaaatatt     660

gccgccgtta ttgttgaacc catgtcaggt tctgcagggg taattttacc acctcaaggc     720

tacttaaaac gcttacgtga aatcactaaa aaacacggca tcttattgat tttcgatgaa     780

gtcattaccg catttggccg tgtaggtgca gcattcgcca gccaacgttg gggcgttatt     840

ccagacataa tcaccacggc taaagccatt aataatggcg ccatccccat gggcgcagtg     900

tttgtacagg attatatcca cgatacttgc atgcaagggc caaccgaact gattgaattt     960

ttccacggtt atacctattc gggccaccca gtcgccgcag cagcagcact cgccacgctc    1020

tccatctacc aaaacgagca actgtttgag cgcagttttg agcttgagcg gtatttcgaa    1080

gaagccgttc atagcctcaa agggttaccg aatgtgattg atattcgcaa caccggatta    1140

gtcgcgggtt tccagctagc accgaatagc caaggtgttg gtaaacgcgg atacagcgtg    1200

ttcgagcatt gtttccatca aggcacactc gtgcgggcaa cgggcgatat tatcgccatg    1260

tccccaccac tcattgttga gaaacatcag attgaccaaa tggtaaatag ccttagcgat    1320

gcaattcacg ccgttggatg a                                              1341

<210>  252
<211>  446
<212>  PRT
<213>  Shewanella oneidensis

<400>  252

Met Ala Asp Ser Pro Asn Asn Leu Ala His Glu His Pro Ser Leu Glu
1               5                   10                  15

His Tyr Trp Met Pro Phe Thr Ala Asn Arg Gln Phe Lys Ala Ser Pro
            20                  25                  30

Arg Leu Leu Ala Gln Ala Glu Gly Met Tyr Tyr Thr Asp Ile Asn Gly
        35                  40                  45

Asn Lys Val Leu Asp Ser Thr Ala Gly Leu Trp Cys Cys Asn Ala Gly
    50                  55                  60

His Gly Arg Arg Glu Ile Ser Glu Ala Val Ser Lys Gln Ile Arg Gln
65                  70                  75                  80

Met Asp Tyr Ala Pro Ser Phe Gln Met Gly His Pro Ile Ala Phe Glu
                85                  90                  95

Leu Ala Glu Arg Leu Thr Glu Leu Ser Pro Glu Gly Leu Asn Lys Val
            100                 105                 110

Phe Phe Thr Asn Ser Gly Ser Glu Ser Val Asp Thr Ala Leu Lys Met
        115                 120                 125

Ala Leu Cys Tyr His Arg Ala Asn Gly Gln Ala Ser Arg Thr Arg Phe
    130                 135                 140

Ile Gly Arg Glu Met Gly Tyr His Gly Val Gly Phe Gly Gly Ile Ser
145                 150                 155                 160

Val Gly Gly Leu Ser Asn Asn Arg Lys Ala Phe Ser Gly Gln Leu Leu
                165                 170                 175

Gln Gly Val Asp His Leu Pro His Thr Leu Asp Ile Gln His Ala Ala
            180                 185                 190

Phe Ser Arg Gly Leu Pro Ser Leu Gly Ala Glu Lys Ala Glu Val Leu
        195                 200                 205

Glu Gln Leu Val Thr Leu His Gly Ala Glu Asn Ile Ala Ala Val Ile
    210                 215                 220

Val Glu Pro Met Ser Gly Ser Ala Gly Val Ile Leu Pro Pro Gln Gly
225                 230                 235                 240

Tyr Leu Lys Arg Leu Arg Glu Ile Thr Lys Lys His Gly Ile Leu Leu
                245                 250                 255

Ile Phe Asp Glu Val Ile Thr Ala Phe Gly Arg Val Gly Ala Ala Phe
            260                 265                 270

Ala Ser Gln Arg Trp Gly Val Ile Pro Asp Ile Ile Thr Thr Ala Lys

275                 280                 285

Ala Ile Asn Asn Gly Ala Ile Pro Met Gly Ala Val Phe Val Gln Asp
    290                 295                 300

Tyr Ile His Asp Thr Cys Met Gln Gly Pro Thr Glu Leu Ile Glu Phe
305                 310                 315                 320

Phe His Gly Tyr Thr Tyr Ser Gly His Pro Val Ala Ala Ala Ala Ala
                325                 330                 335

Leu Ala Thr Leu Ser Ile Tyr Gln Asn Glu Gln Leu Phe Glu Arg Ser
            340                 345                 350

Phe Glu Leu Glu Arg Tyr Phe Glu Glu Ala Val His Ser Leu Lys Gly
        355                 360                 365

Leu Pro Asn Val Ile Asp Ile Arg Asn Thr Gly Leu Val Ala Gly Phe
    370                 375                 380

Gln Leu Ala Pro Asn Ser Gln Gly Val Gly Lys Arg Gly Tyr Ser Val
385                 390                 395                 400

Phe Glu His Cys Phe His Gln Gly Thr Leu Val Arg Ala Thr Gly Asp
                405                 410                 415

Ile Ile Ala Met Ser Pro Pro Leu Ile Val Glu Lys His Gln Ile Asp
            420                 425                 430

Gln Met Val Asn Ser Leu Ser Asp Ala Ile His Ala Val Gly
        435                 440                 445

<210>  253
<211>  1347
<212>  DNA
<213>  Pseudomonas putida

<400>  253
atgaacatgc ccgaaactgg tcctgccggt atcgccagcc agctcaagct ggacgcccac      60

tggatgccct acaccgccaa ccgcaacttc cagcgcgacc cacgcctgat cgtggcggcc     120

gaaggcaact acctggtcga tgaccacggg cgcaagatct tcgacgccct gtccggcctg     180

tggacctgcg gcgcagggca cactcgcaag gaaatcgctg acgcggtgac ccgtcaactg     240

agtacgctgg actactcccc agcgttccag ttcggccacc cgctgtcgtt ccagctggcg     300

gaaaagatcg ccgagctggt tccgggcaat ctgaatcacg tcttctatac caactccggt     360

tccgagtgcg ccgataccgc actgaagatg gtgcgtgcct actggcgcct gaaaggccag     420

gcaaccaaga ccaagatcat cggccgtgcc cgtggttacc atggcgtgaa catcgccggt     480

accagcctgg gtggcgtcaa cggtaaccgc aagatgtttg gccagctgct ggacgtcgac 540

cacctgcctc acactgtatt gccggtgaac gccttctcga aaggcttgcc ggaagagggc 600

ggtatcgcgc tggctgacga aatgctcaag ctgatcgagc tgcacgatgc ctccaacatc 660

gcagcagtca tcgtcgagcc gctggccggt tcggccggtg tgctgccgcc gccaaagggt 720

tacctgaagc gcctgcgtga aatctgcacc cagcacaaca ttctgctgat cttcgacgaa 780

gtgatcacag gcttcggccg catgggcgcg atgaccggct cggaagcctt cggcgttacc 840

ccggacctga tgtgcatcgc caagcaggtg accaacggcg ccatcccgat gggcgcagtg 900

attgccagca gcgagatcta ccagaccttc atgaaccagc cgaccccgga atacgccgtg 960

gaattcccac acggctacac ctattcggcg cacccggtag cctgtgccgc cggtctcgcc 1020

gcgctggacc tgctgcagaa ggaaaacctg gtgcagtccg cggctgaact ggcgccgcat 1080

ttcgagaagc tgctgcacgg cgtgaagggc accaagaata tcgtcgatat ccgcaactac 1140

ggcctggccg gcgccatcca gatcgccgcc cgtgacggtg atgccatcgt tcgcccttac 1200

gaagcggcca tgaagctgtg gaaagcgggc ttctatgtac gctttggtgg cgacaccctg 1260

cagttcggcc caaccttcaa taccaagccg caggaactgg accgcttgtt cgatgctgtt 1320

ggcgaaaccc tgaacctgat cgactga 1347

<210> 254
<211> 448
<212> PRT
<213> Pseudomonas putida

<400> 254

Met Asn Met Pro Glu Thr Gly Pro Ala Gly Ile Ala Ser Gln Leu Lys
1               5                   10                  15

Leu Asp Ala His Trp Met Pro Tyr Thr Ala Asn Arg Asn Phe Gln Arg
            20                  25                  30

Asp Pro Arg Leu Ile Val Ala Ala Glu Gly Asn Tyr Leu Val Asp Asp
        35                  40                  45

His Gly Arg Lys Ile Phe Asp Ala Leu Ser Gly Leu Trp Thr Cys Gly
    50                  55                  60

Ala Gly His Thr Arg Lys Glu Ile Ala Asp Ala Val Thr Arg Gln Leu
65                  70                  75                  80

Ser Thr Leu Asp Tyr Ser Pro Ala Phe Gln Phe Gly His Pro Leu Ser
                85                  90                  95

Phe Gln Leu Ala Glu Lys Ile Ala Glu Leu Val Pro Gly Asn Leu Asn
            100                 105                 110

His Val Phe Tyr Thr Asn Ser Gly Ser Glu Cys Ala Asp Thr Ala Leu
        115                 120                 125

Lys Met Val Arg Ala Tyr Trp Arg Leu Lys Gly Gln Ala Thr Lys Thr
    130                 135                 140

Lys Ile Ile Gly Arg Ala Arg Gly Tyr His Gly Val Asn Ile Ala Gly
145                 150                 155                 160

Thr Ser Leu Gly Gly Val Asn Gly Asn Arg Lys Met Phe Gly Gln Leu
                165                 170                 175

Leu Asp Val Asp His Leu Pro His Thr Val Leu Pro Val Asn Ala Phe
            180                 185                 190

Ser Lys Gly Leu Pro Glu Glu Gly Gly Ile Ala Leu Ala Asp Glu Met
        195                 200                 205

Leu Lys Leu Ile Glu Leu His Asp Ala Ser Asn Ile Ala Ala Val Ile
    210                 215                 220

Val Glu Pro Leu Ala Gly Ser Ala Gly Val Leu Pro Pro Pro Lys Gly
225                 230                 235                 240

Tyr Leu Lys Arg Leu Arg Glu Ile Cys Thr Gln His Asn Ile Leu Leu
                245                 250                 255

Ile Phe Asp Glu Val Ile Thr Gly Phe Gly Arg Met Gly Ala Met Thr
            260                 265                 270

Gly Ser Glu Ala Phe Gly Val Thr Pro Asp Leu Met Cys Ile Ala Lys
        275                 280                 285

Gln Val Thr Asn Gly Ala Ile Pro Met Gly Ala Val Ile Ala Ser Ser
    290                 295                 300

Glu Ile Tyr Gln Thr Phe Met Asn Gln Pro Thr Pro Glu Tyr Ala Val
305                 310                 315                 320

Glu Phe Pro His Gly Tyr Thr Tyr Ser Ala His Pro Val Ala Cys Ala
                325                 330                 335

Ala Gly Leu Ala Ala Leu Asp Leu Leu Gln Lys Glu Asn Leu Val Gln
            340                 345                 350

Ser Ala Ala Glu Leu Ala Pro His Phe Glu Lys Leu Leu His Gly Val
        355                 360                 365

Lys Gly Thr Lys Asn Ile Val Asp Ile Arg Asn Tyr Gly Leu Ala Gly
    370                 375                 380

Ala Ile Gln Ile Ala Ala Arg Asp Gly Asp Ala Ile Val Arg Pro Tyr
385                 390                 395                 400

Glu Ala Ala Met Lys Leu Trp Lys Ala Gly Phe Tyr Val Arg Phe Gly
                405                 410                 415

Gly Asp Thr Leu Gln Phe Gly Pro Thr Phe Asn Thr Lys Pro Gln Glu
            420                 425                 430

Leu Asp Arg Leu Phe Asp Ala Val Gly Glu Thr Leu Asn Leu Ile Asp
        435                 440                 445

<210>  255
<211>  1701
<212>  DNA
<213>  Streptomyces cinnamonensis

<400>  255
atggacgctg acgcgatcga ggaaggccgc cgacgctggc aggcccgtta cgacaaggcc      60

cgcaagcgcg acgcggactt caccacgctc tccggggacc ccgtcgaccc cgtctacggc     120

ccccggcccg gggacacgta cgacgggttc gagcggatcg gctggccggg ggagtacccc     180

ttcacccgcg ggctctacgc caccgggtac cgcggccgca cctggaccat ccgccagttc     240

gccggcttcg gcaacgccga gcagacgaac gagcgctaca agatgatcct ggccaacggc     300

ggcggcggcc tctccgtcgc cttcgacatg ccgaccctca tgggccgcga ctccgacgac     360

ccgcgctcgc tcggcgaggt cggccactgc ggtgtcgcca tcgactccgc cgccgacatg     420

gaggtcctct tcaaggacat cccgctcggc gacgtcacga cgtccatgac catcagcggg     480

cccgccgtgc ccgtcttctg catgtacctc gtcgcggccg agcgccaggg cgtcgacccg     540

gccgtcctca acggcacgct gcagaccgac atcttcaagg agtacatcgc ccagaaggag     600

tggctcttcc agcccgagcc gcacctgcgc ctcatcggcg acctgatgga gcactgcgcg     660

cgcgacatcc ccgcgtacaa gccgctctcg gtctccggct accacatccg cgaggccggg     720

gcgacggccg cgcaggagct cgcgtacacc ctcgcggacg gcttcgggta cgtggaactg     780

ggcctctcgc gcggcctgga cgtggacgtc ttcgcgcccg gcctctcctt cttcttcgac     840

gcgcacgtcg acttcttcga ggagatcgcg aagttccgcg ccgcacgccg catctgggcg     900

cgctggctcc gggacgagta cggagcgaag accgagaagg cacagtggct gcgcttccac     960

acgcagaccg cgggggtctc gctcacggcc cagcagccgt acaacaacgt ggtgcggacg    1020

gcggtggagg ccctcgccgc ggtgctcggc ggcacgaact ccctgcacac caacgctctc    1080

gacgagaccc ttgccctccc cagcgagcag gccgcggaga tcgcgctgcg cacccagcag    1140

gtgctgatgg aggagaccgg cgtcgccaac gtcgcggacc cgctgggcgg ctcctggtac    1200

atcgagcagc tcaccgaccg catcgaggcc gacgccgaga agatcttcga gcagatcagg 1260

gagcgggggc ggcgggcctg ccccgacggg cagcacccga tcgggccgat cacctccggc 1320

atcctgcgcg gcatcgagga cggctggttc accggcgaga tcgccgagtc cgccttccag 1380

taccagcggt ccctggagaa gggcgacaag cgggtcgtcg gcgtcaactg cctcgaaggc 1440

tccgtcaccg gcgacctgga gatcctgcgc gtcagccacg aggtcgagcg cgagcaggtg 1500

cgggagcttg cggggcgcaa ggggcggcgt gacgatgcgc gggtgcgggc ctcgctcgac 1560

gcgatgctcg ccgctgcgcg ggacgggtcg aacatgattg cccccatgct ggaggcggtg 1620

cgggccgagg cgaccctcgg ggagatctgc ggggtgcttc gcgatgagtg gggggtctac 1680

gtggagccgc ccgggttctg a 1701

<210> 256
<211> 566
<212> PRT
<213> Streptomyces cinnamonensis

<400> 256

Met Asp Ala Asp Ala Ile Glu Glu Gly Arg Arg Arg Trp Gln Ala Arg
1               5                   10                  15

Tyr Asp Lys Ala Arg Lys Arg Asp Ala Asp Phe Thr Thr Leu Ser Gly
            20                  25                  30

Asp Pro Val Asp Pro Val Tyr Gly Pro Arg Pro Gly Asp Thr Tyr Asp
        35                  40                  45

Gly Phe Glu Arg Ile Gly Trp Pro Gly Glu Tyr Pro Phe Thr Arg Gly
    50                  55                  60

Leu Tyr Ala Thr Gly Tyr Arg Gly Arg Thr Trp Thr Ile Arg Gln Phe
65                  70                  75                  80

Ala Gly Phe Gly Asn Ala Glu Gln Thr Asn Glu Arg Tyr Lys Met Ile
                85                  90                  95

Leu Ala Asn Gly Gly Gly Gly Leu Ser Val Ala Phe Asp Met Pro Thr
            100                 105                 110

Leu Met Gly Arg Asp Ser Asp Asp Pro Arg Ser Leu Gly Glu Val Gly
        115                 120                 125

His Cys Gly Val Ala Ile Asp Ser Ala Ala Asp Met Glu Val Leu Phe
    130                 135                 140

Lys Asp Ile Pro Leu Gly Asp Val Thr Thr Ser Met Thr Ile Ser Gly
145                 150                 155                 160

Pro Ala Val Pro Val Phe Cys Met Tyr Leu Val Ala Ala Glu Arg Gln
                165                 170                 175

Gly Val Asp Pro Ala Val Leu Asn Gly Thr Leu Gln Thr Asp Ile Phe
            180                 185                 190

Lys Glu Tyr Ile Ala Gln Lys Glu Trp Leu Phe Gln Pro Glu Pro His
        195                 200                 205

Leu Arg Leu Ile Gly Asp Leu Met Glu His Cys Ala Arg Asp Ile Pro
    210                 215                 220

Ala Tyr Lys Pro Leu Ser Val Ser Gly Tyr His Ile Arg Glu Ala Gly
225                 230                 235                 240

Ala Thr Ala Ala Gln Glu Leu Ala Tyr Thr Leu Ala Asp Gly Phe Gly
                245                 250                 255

Tyr Val Glu Leu Gly Leu Ser Arg Gly Leu Asp Val Asp Val Phe Ala
            260                 265                 270

Pro Gly Leu Ser Phe Phe Phe Asp Ala His Val Asp Phe Phe Glu Glu
        275                 280                 285

Ile Ala Lys Phe Arg Ala Ala Arg Arg Ile Trp Ala Arg Trp Leu Arg
    290                 295                 300

Asp Glu Tyr Gly Ala Lys Thr Glu Lys Ala Gln Trp Leu Arg Phe His
305                 310                 315                 320

Thr Gln Thr Ala Gly Val Ser Leu Thr Ala Gln Gln Pro Tyr Asn Asn
                325                 330                 335

Val Val Arg Thr Ala Val Glu Ala Leu Ala Ala Val Leu Gly Gly Thr
            340                 345                 350

Asn Ser Leu His Thr Asn Ala Leu Asp Glu Thr Leu Ala Leu Pro Ser
        355                 360                 365

Glu Gln Ala Ala Glu Ile Ala Leu Arg Thr Gln Gln Val Leu Met Glu
    370                 375                 380

Glu Thr Gly Val Ala Asn Val Ala Asp Pro Leu Gly Gly Ser Trp Tyr
385                 390                 395                 400

Ile Glu Gln Leu Thr Asp Arg Ile Glu Ala Asp Ala Glu Lys Ile Phe
                405                 410                 415

Glu Gln Ile Arg Glu Arg Gly Arg Arg Ala Cys Pro Asp Gly Gln His
            420                 425                 430

Pro Ile Gly Pro Ile Thr Ser Gly Ile Leu Arg Gly Ile Glu Asp Gly
        435                 440                 445

Trp Phe Thr Gly Glu Ile Ala Glu Ser Ala Phe Gln Tyr Gln Arg Ser
    450                 455                 460

Leu Glu Lys Gly Asp Lys Arg Val Val Gly Val Asn Cys Leu Glu Gly
465                 470                 475                 480

Ser Val Thr Gly Asp Leu Glu Ile Leu Arg Val Ser His Glu Val Glu
                485                 490                 495

Arg Glu Gln Val Arg Glu Leu Ala Gly Arg Lys Gly Arg Arg Asp Asp
            500                 505                 510

Ala Arg Val Arg Ala Ser Leu Asp Ala Met Leu Ala Ala Ala Arg Asp
        515                 520                 525

Gly Ser Asn Met Ile Ala Pro Met Leu Glu Ala Val Arg Ala Glu Ala
    530                 535                 540

Thr Leu Gly Glu Ile Cys Gly Val Leu Arg Asp Glu Trp Gly Val Tyr
545                 550                 555                 560

Val Glu Pro Pro Gly Phe
                565

<210>  257
<211>  411
<212>  DNA
<213>  Streptomyces cinnamonensis

<400>  257
atgggtgtgg cagccgggcc gatccgcgtg gtggtcgcca agccggggct cgacgggcac      60

gatcgcgggg ccaaggtgat cgcgcgggcg ttgcgtgacg cgggtatgga ggtcatctac     120

accgggctgc accagacgcc cgagcaggtg gtggacaccg cgatccagga ggacgccgac     180

gcgatcggcc tctccatcct ctccggagcg cacaacacgc tgttcgcgcg cgtgttggag     240

ctcttgaagg agcgggacgc ggaggacatc aaggtgtttg gtggcggcat catcccggag     300

gcggacatcg cgccgctgaa ggagaagggc gtcgcggaga tcttcacgcc cggggccacc     360

accacgtcga tcgtggagtg ggttcggggg aacgtgcgac aggccgtctg a              411

<210>  258
<211>  136
<212>  PRT

<213>  Streptomyces cinnamonensis

<400>  258

Met Gly Val Ala Ala Gly Pro Ile Arg Val Val Val Ala Lys Pro Gly
1               5                   10                  15

Leu Asp Gly His Asp Arg Gly Ala Lys Val Ile Ala Arg Ala Leu Arg
            20                  25                  30

Asp Ala Gly Met Glu Val Ile Tyr Thr Gly Leu His Gln Thr Pro Glu
        35                  40                  45

Gln Val Val Asp Thr Ala Ile Gln Glu Asp Ala Asp Ala Ile Gly Leu
    50                  55                  60

Ser Ile Leu Ser Gly Ala His Asn Thr Leu Phe Ala Arg Val Leu Glu
65                  70                  75                  80

Leu Leu Lys Glu Arg Asp Ala Glu Asp Ile Lys Val Phe Gly Gly Gly
                85                  90                  95

Ile Ile Pro Glu Ala Asp Ile Ala Pro Leu Lys Glu Lys Gly Val Ala
            100                 105                 110

Glu Ile Phe Thr Pro Gly Ala Thr Thr Thr Ser Ile Val Glu Trp Val
        115                 120                 125

Arg Gly Asn Val Arg Gln Ala Val
    130                 135

<210>  259
<211>  1701
<212>  DNA
<213>  Streptomyces coelicolor

<400>  259
atggacgctc atgccataga ggagggccgc cttcgctggc aggcccggta cgacgcggcg      60

cgcaagcgcg acgcggactt caccacgctc tccggagacc ccgtggagcc ggtgtacggg     120

ccccgccccg gggacgagta cgagggcttc gagcggatcg gctggccggg cgagtacccc     180

ttcacccgcg gcctgtatcc gaccgggtac cgggggcgta cgtggaccat ccggcagttc     240

gccgggttcg gcaacgccga gcagaccaac gagcgctaca agatgatcct ccgcaacggc     300

ggcggcgggc tctcggtcgc cttcgacatg ccgaccctga tgggccgcga ctccgacgac     360

ccgcgctcgc tgggcgaggt cgggcactgc ggggtggcca tcgactcggc cgccgacatg     420

gaagtgctgt tcaaggacat cccgctcggg gacgtgacga cctccatgac gatcagcggg     480

cccgccgtgc ccgtgttctg catgtacctc gtcgccgccg agcgccaggg cgtcgacgca     540

tccgtgctca acggcacgct gcagaccgac atcttcaagg agtacatcgc ccagaaggag     600

tggctcttcc agcccgagcc ccacctccgg ctcatcggcg acctcatgga gtactgcgcg 660

gccggcatcc ccgcctacaa gccgctctcc gtctccggct accacatccg cgaggcgggc 720

gcgacggccg cgcaggagct ggcgtacacg ctcgccgacg gcttcggata cgtggagctg 780

ggcctcagcc gcgggctcga cgtggacgtc ttcgcgcccg gcctctcctt cttcttcgac 840

gcgcacctcg acttcttcga ggagatcgcc aagttccgcg cggcccgcag gatctgggcc 900

cgctggatgc gcgacgtgta cggcgcgcgg accgacaagg cccagtggct gcggttccac 960

acccagaccg ccggagtctc gctcaccgcg cagcagccgt acaacaacgt cgtacgcacc 1020

gcggtggagg cgctggcggc cgtgctcggc ggcaccaact ccctgcacac caacgcgctc 1080

gacgagaccc tcgccctgcc cagcgagcag gccgccgaga tcgccctgcg cacccagcag 1140

gtgctgatgg aggagaccgg cgtcgccaac gtcgccgacc cgctgggcgg ttcctggttc 1200

atcgagcagc tgaccgaccg catcgaggcc gacgccgaga agatcttcga gcagatcaag 1260

gagcgggggc tgcgcgccca ccccgacggg cagcaccccg tcggaccgat cacctccggc 1320

ctgctgcgcg gcatcgagga cggctggttc accggcgaga tcgccgagtc cgccttccgc 1380

taccagcagt ccttggagaa ggacgacaag aaggtggtcg gcgtcaacgt ccacaccggc 1440

tccgtcaccg gcgacctgga gatcctgcgg gtcagccacg aggtcgagcg cgagcaggtg 1500

cgggtcctgg gcgagcgcaa ggacgcccgg gacgacgccg ccgtgcgcgg cgccctggac 1560

gccatgctgg ccgcggcccg ctccggcggc aacatgatcg ggccgatgct ggacgcggtg 1620

cgcgcggagg cgacgctggg cgagatctgc ggtgtgctgc gcgacgagtg gggggtgtac 1680

acggaaccgg cggggttctg a 1701

<210> 260
<211> 566
<212> PRT
<213> Streptomyces coelicolor

<400> 260

Met Asp Ala His Ala Ile Glu Glu Gly Arg Leu Arg Trp Gln Ala Arg
1               5                   10                  15

Tyr Asp Ala Ala Arg Lys Arg Asp Ala Asp Phe Thr Thr Leu Ser Gly
            20                  25                  30

Asp Pro Val Glu Pro Val Tyr Gly Pro Arg Pro Gly Asp Glu Tyr Glu
        35                  40                  45

Gly Phe Glu Arg Ile Gly Trp Pro Gly Glu Tyr Pro Phe Thr Arg Gly
    50                  55                  60

Leu Tyr Pro Thr Gly Tyr Arg Gly Arg Thr Trp Thr Ile Arg Gln Phe
65                  70                  75                  80

Ala Gly Phe Gly Asn Ala Glu Gln Thr Asn Glu Arg Tyr Lys Met Ile
                85                  90                  95

Leu Arg Asn Gly Gly Gly Gly Leu Ser Val Ala Phe Asp Met Pro Thr
            100                 105                 110

Leu Met Gly Arg Asp Ser Asp Asp Pro Arg Ser Leu Gly Glu Val Gly
        115                 120                 125

His Cys Gly Val Ala Ile Asp Ser Ala Ala Asp Met Glu Val Leu Phe
    130                 135                 140

Lys Asp Ile Pro Leu Gly Asp Val Thr Thr Ser Met Thr Ile Ser Gly
145                 150                 155                 160

Pro Ala Val Pro Val Phe Cys Met Tyr Leu Val Ala Ala Glu Arg Gln
                165                 170                 175

Gly Val Asp Ala Ser Val Leu Asn Gly Thr Leu Gln Thr Asp Ile Phe
            180                 185                 190

Lys Glu Tyr Ile Ala Gln Lys Glu Trp Leu Phe Gln Pro Glu Pro His
        195                 200                 205

Leu Arg Leu Ile Gly Asp Leu Met Glu Tyr Cys Ala Ala Gly Ile Pro
    210                 215                 220

Ala Tyr Lys Pro Leu Ser Val Ser Gly Tyr His Ile Arg Glu Ala Gly
225                 230                 235                 240

Ala Thr Ala Ala Gln Glu Leu Ala Tyr Thr Leu Ala Asp Gly Phe Gly
                245                 250                 255

Tyr Val Glu Leu Gly Leu Ser Arg Gly Leu Asp Val Asp Val Phe Ala
            260                 265                 270

Pro Gly Leu Ser Phe Phe Phe Asp Ala His Leu Asp Phe Phe Glu Glu
        275                 280                 285

Ile Ala Lys Phe Arg Ala Ala Arg Arg Ile Trp Ala Arg Trp Met Arg
    290                 295                 300

Asp Val Tyr Gly Ala Arg Thr Asp Lys Ala Gln Trp Leu Arg Phe His
305                 310                 315                 320

Thr Gln Thr Ala Gly Val Ser Leu Thr Ala Gln Gln Pro Tyr Asn Asn
                325                 330                 335

Val Val Arg Thr Ala Val Glu Ala Leu Ala Ala Val Leu Gly Gly Thr
            340                 345                 350

Asn Ser Leu His Thr Asn Ala Leu Asp Glu Thr Leu Ala Leu Pro Ser
        355                 360                 365

Glu Gln Ala Ala Glu Ile Ala Leu Arg Thr Gln Gln Val Leu Met Glu
    370                 375                 380

Glu Thr Gly Val Ala Asn Val Ala Asp Pro Leu Gly Gly Ser Trp Phe
385                 390                 395                 400

Ile Glu Gln Leu Thr Asp Arg Ile Glu Ala Asp Ala Glu Lys Ile Phe
                405                 410                 415

Glu Gln Ile Lys Glu Arg Gly Leu Arg Ala His Pro Asp Gly Gln His
            420                 425                 430

Pro Val Gly Pro Ile Thr Ser Gly Leu Leu Arg Gly Ile Glu Asp Gly
        435                 440                 445

Trp Phe Thr Gly Glu Ile Ala Glu Ser Ala Phe Arg Tyr Gln Gln Ser
    450                 455                 460

Leu Glu Lys Asp Asp Lys Lys Val Val Gly Val Asn Val His Thr Gly
465                 470                 475                 480

Ser Val Thr Gly Asp Leu Glu Ile Leu Arg Val Ser His Glu Val Glu
                485                 490                 495

Arg Glu Gln Val Arg Val Leu Gly Glu Arg Lys Asp Ala Arg Asp Asp
            500                 505                 510

Ala Ala Val Arg Gly Ala Leu Asp Ala Met Leu Ala Ala Ala Arg Ser
        515                 520                 525

Gly Gly Asn Met Ile Gly Pro Met Leu Asp Ala Val Arg Ala Glu Ala
    530                 535                 540

Thr Leu Gly Glu Ile Cys Gly Val Leu Arg Asp Glu Trp Gly Val Tyr
545                 550                 555                 560

Thr Glu Pro Ala Gly Phe
                565

<210>  261
<211>  417
<212>  DNA
<213>  Streptomyces coelicolor

<400>  261
atgggtgtgg cagccggtcc gatccgcgtg gtggtggcca agccggggct cgacggccac      60

gatcgcgggg ccaaggtgat cgcgagggcc ctgcgtgacg ccggtatgga ggtgatctac     120

accgggctcc accagacgcc cgagcagatc gtcgacaccg cgatccagga ggacgccgac     180

gcgatcgggc tgtccatcct ctccggtgcg cacaacacgc tcttcgccgc cgtgatcgag     240

ctgctccggg agcgggacgc cgcggacatc ctggtcttcg gcggcgggat catccccgag     300

gcggacatcg ccccgctgaa ggagaagggc gtcgcggaga tcttcacgcc cggcgccacc     360

acggcgtcca tcgtggactg ggtccgggcg aacgtgcggg agcccgcggg agcatag        417

<210>  262
<211>  138
<212>  PRT
<213>  Streptomyces coelicolor

<400>  262

Met Gly Val Ala Ala Gly Pro Ile Arg Val Val Val Ala Lys Pro Gly
1               5                   10                  15

Leu Asp Gly His Asp Arg Gly Ala Lys Val Ile Ala Arg Ala Leu Arg
            20                  25                  30

Asp Ala Gly Met Glu Val Ile Tyr Thr Gly Leu His Gln Thr Pro Glu
        35                  40                  45

Gln Ile Val Asp Thr Ala Ile Gln Glu Asp Ala Asp Ala Ile Gly Leu
    50                  55                  60

Ser Ile Leu Ser Gly Ala His Asn Thr Leu Phe Ala Ala Val Ile Glu
65                  70                  75                  80

Leu Leu Arg Glu Arg Asp Ala Ala Asp Ile Leu Val Phe Gly Gly Gly
                85                  90                  95

Ile Ile Pro Glu Ala Asp Ile Ala Pro Leu Lys Glu Lys Gly Val Ala
            100                 105                 110

Glu Ile Phe Thr Pro Gly Ala Thr Thr Ala Ser Ile Val Asp Trp Val
        115                 120                 125

Arg Ala Asn Val Arg Glu Pro Ala Gly Ala
    130                 135

<210>  263
<211>  1701
<212>  DNA
<213>  Streptomyces avermitilis

<400>  263
tcagaaaccg gcgggctccg tgtagacccc ccactcctcc cggaggacat cgcagatctc      60

gcccagcgtg gcctccgcgc ggaccgcgtc cagcatcggg gcgatcatgt tcgacccgtc     120

gcgcgcggcg gcgagcatcg cgtccagggc cgcggttacg gccgtgtcgt cgcgccccga     180

cttccgctcg cccagcaccc gcacctgctc gcgctccacc tcgtggctga cgcgcaggat     240

ctccaggtcg cccgtcacgg acccgtggtg gacgttgacg ccgacgaccc gcttgtcgcc     300

cttctccagc gcctgctggt actggaaggc cgactcggcg atctccccgg tgaaccagcc     360

gtcctcgatg ccgcgcagga tgccggaggt gatgggcccg atcgggtgcc gcccgtccgg     420

gtgggcccgc agcccgcgct ccctgatctg ttcgaagatc ttctcggcgt cggcctcgat     480

ccggtcggtc agctgctcca cgtaccagga accgcccagc ggatcggcca cgttggcgac     540

gcccgtctcc tccatcagca cctgctgggt gcgcagggcg atctcggccg cctgctcgga     600

cggcagggcg agggtctcgt cgagggcgtt ggtgtgcagc gagttcgtcc cgccgagcac     660

cgcggcgagg gcctccacgg ccgtccgtac gacgttgttg tacggctgct gcgcggtgag     720

cgagacgccc gcggtctggg tgtggaagcg cagccactgc gccttctccg acttcgcccc     780

gtacacgtcc cgcagccagc gcgcccagat gcgccgcgcc gcacggaact tggcgatctc     840

ctcgaagaag tcgacgtgcg cgtcgaagaa gaaggagagc ccgggcgcga acacgtccac     900

gtccaggccg cggctcagcc ccagctccac gtatccgaaa ccgtcggcga gggtgtacgc     960

cagctcctgg gcggccgtgg caccggcctc ccggatgtgg tacccggaga cggacagcgg    1020

cttgtacgcg gggatcttcg aggcgcagtg ctccatcagg tcgccgatga gccgcagatg    1080

gggctcgggc tggaagagcc actccttctg cgcgatgtac tccttgaaga tgtcggtctg    1140

gagggtgccg ttgaggacgg aggggtcgac gccctgccgc tcggccgcga ccaggtacat    1200

gcagaagacg ggcacggcgg gcccgctgat cgtcatcgac gtcgtcacgt cacccagcgg    1260

gatgtccttg aacaggacct ccatgtcggc cgccgagtcg atcgcgaccc cgcagtgccc    1320

gacctcgccg agcgcgcggc ggtcgtcgga gtcgcgcccc atgagcgtcg gcatgtcgaa    1380

ggccacggac agcccaccgc cgccgttggc gaggatcttc ttgtagcgct cgttggtctg    1440

ctcggcgttg ccgaacccgg cgaactgccg gatggtccag gtccggcccc ggtagccggt    1500

cggatacaga ccgcgcgtga aggggtactc acccggccag ccgatccgct cgaaaccctc    1560

gtacgcgtcc ccgggccggg gcccgtacgc cggctccacg ggatcgccgg agagcgtggt    1620

gaaatcggcc tcgcgcttgc gtgaggcgtc gtagcgggcc tgccagcgtc ggcggccttc    1680

ctcgatggcg tcagcgtcca t                                              1701

<210>  264
<211>  566
<212>  PRT
<213>  Streptomyces avermitilis

<400>  264

Met Asp Ala Asp Ala Ile Glu Glu Gly Arg Arg Arg Trp Gln Ala Arg
1               5                   10                  15

Tyr Asp Ala Ser Arg Lys Arg Glu Ala Asp Phe Thr Thr Leu Ser Gly
            20                  25                  30

Asp Pro Val Glu Pro Ala Tyr Gly Pro Arg Pro Gly Asp Ala Tyr Glu
        35                  40                  45

Gly Phe Glu Arg Ile Gly Trp Pro Gly Glu Tyr Pro Phe Thr Arg Gly
    50                  55                  60

Leu Tyr Pro Thr Gly Tyr Arg Gly Arg Thr Trp Thr Ile Arg Gln Phe
65                  70                  75                  80

Ala Gly Phe Gly Asn Ala Glu Gln Thr Asn Glu Arg Tyr Lys Lys Ile
                85                  90                  95

Leu Ala Asn Gly Gly Gly Gly Leu Ser Val Ala Phe Asp Met Pro Thr
            100                 105                 110

Leu Met Gly Arg Asp Ser Asp Asp Arg Arg Ala Leu Gly Glu Val Gly
        115                 120                 125

His Cys Gly Val Ala Ile Asp Ser Ala Ala Asp Met Glu Val Leu Phe
    130                 135                 140

Lys Asp Ile Pro Leu Gly Asp Val Thr Thr Ser Met Thr Ile Ser Gly
145                 150                 155                 160

Pro Ala Val Pro Val Phe Cys Met Tyr Leu Val Ala Ala Glu Arg Gln
                165                 170                 175

Gly Val Asp Pro Ser Val Leu Asn Gly Thr Leu Gln Thr Asp Ile Phe
            180                 185                 190

Lys Glu Tyr Ile Ala Gln Lys Glu Trp Leu Phe Gln Pro Glu Pro His
        195                 200                 205

Leu Arg Leu Ile Gly Asp Leu Met Glu His Cys Ala Ser Lys Ile Pro
    210                 215                 220

Ala Tyr Lys Pro Leu Ser Val Ser Gly Tyr His Ile Arg Glu Ala Gly
225                 230                 235                 240

Ala Thr Ala Ala Gln Glu Leu Ala Tyr Thr Leu Ala Asp Gly Phe Gly
                245                 250                 255

Tyr Val Glu Leu Gly Leu Ser Arg Gly Leu Asp Val Asp Val Phe Ala
            260                 265                 270

Pro Gly Leu Ser Phe Phe Phe Asp Ala His Val Asp Phe Phe Glu Glu
        275                 280                 285

Ile Ala Lys Phe Arg Ala Ala Arg Arg Ile Trp Ala Arg Trp Leu Arg
    290                 295                 300

Asp Val Tyr Gly Ala Lys Ser Glu Lys Ala Gln Trp Leu Arg Phe His
305                 310                 315                 320

Thr Gln Thr Ala Gly Val Ser Leu Thr Ala Gln Gln Pro Tyr Asn Asn
                325                 330                 335

Val Val Arg Thr Ala Val Glu Ala Leu Ala Ala Val Leu Gly Gly Thr
            340                 345                 350

Asn Ser Leu His Thr Asn Ala Leu Asp Glu Thr Leu Ala Leu Pro Ser
        355                 360                 365

Glu Gln Ala Ala Glu Ile Ala Leu Arg Thr Gln Gln Val Leu Met Glu
    370                 375                 380

Glu Thr Gly Val Ala Asn Val Ala Asp Pro Leu Gly Gly Ser Trp Tyr
385                 390                 395                 400

Val Glu Gln Leu Thr Asp Arg Ile Glu Ala Asp Ala Glu Lys Ile Phe
                405                 410                 415

Glu Gln Ile Arg Glu Arg Gly Leu Arg Ala His Pro Asp Gly Arg His
            420                 425                 430

Pro Ile Gly Pro Ile Thr Ser Gly Ile Leu Arg Gly Ile Glu Asp Gly
        435                 440                 445

Trp Phe Thr Gly Glu Ile Ala Glu Ser Ala Phe Gln Tyr Gln Gln Ala
    450                 455                 460

Leu Glu Lys Gly Asp Lys Arg Val Val Gly Val Asn Val His His Gly
465                 470                 475                 480

Ser Val Thr Gly Asp Leu Glu Ile Leu Arg Val Ser His Glu Val Glu
                485                 490                 495

Arg Glu Gln Val Arg Val Leu Gly Glu Arg Lys Ser Gly Arg Asp Asp
            500                 505                 510

Thr Ala Val Thr Ala Ala Leu Asp Ala Met Leu Ala Ala Ala Arg Asp
        515                 520                 525

Gly Ser Asn Met Ile Ala Pro Met Leu Asp Ala Val Arg Ala Glu Ala
    530                 535                 540

Thr Leu Gly Glu Ile Cys Asp Val Leu Arg Glu Glu Trp Gly Val Tyr
545                 550                 555                 560

Thr Glu Pro Ala Gly Phe
                565

<210>  265
<211>  417
<212>  DNA
<213>  Streptomyces avermitilis

<400>  265
ctacgccccg gcaggctgcc gcacgttcgc ccgcacccac tccacgatcg acgccgtggt      60

cgcccccgga gtgaagatct ccgcgacacc cttctccttc agcggcgcga tgtccgcctc     120

ggggatgatg ccgccaccga acaccttgat gtcctcggca tcgcgctcct tgagcagatc     180

gatgaccgcc gcgaacaacg tgttgtgcgc cccggacagg atcgacagcc cgatcgcgtc     240

ggcgtcctcc tggatggccg tgcccacgat ctgctccggc gtctggtgca gccccgtgta     300

aatgacctcc ataccggcat cgcgcagcgc ccgcgcgatc accttggccc cgcgatcgtg     360

gccatcgagc cccggcttgg ccaccaccac gcggatcgga ccggctgcca cacccat        417

<210>  266
<211>  138
<212>  PRT
<213>  Streptomyces avermitilis

<400>  266

Met Gly Val Ala Ala Gly Pro Ile Arg Val Val Val Ala Lys Pro Gly
1               5                   10                  15

Leu Asp Gly His Asp Arg Gly Ala Lys Val Ile Ala Arg Ala Leu Arg
            20                  25                  30

Asp Ala Gly Met Glu Val Ile Tyr Thr Gly Leu His Gln Thr Pro Glu
        35                  40                  45

Gln Ile Val Gly Thr Ala Ile Gln Glu Asp Ala Asp Ala Ile Gly Leu
    50                  55                  60

Ser Ile Leu Ser Gly Ala His Asn Thr Leu Phe Ala Ala Val Ile Asp
65                  70                  75                  80

Leu Leu Lys Glu Arg Asp Ala Glu Asp Ile Lys Val Phe Gly Gly Gly

85                  90                  95

Ile Ile Pro Glu Ala Asp Ile Ala Pro Leu Lys Glu Lys Gly Val Ala
            100                 105                 110

Glu Ile Phe Thr Pro Gly Ala Thr Thr Ala Ser Ile Val Glu Trp Val
        115                 120                 125

Arg Ala Asn Val Arg Gln Pro Ala Gly Ala
    130                 135

<210>  267
<211>  2910
<212>  DNA
<213>  Staphylococcus aureus

<400>  267
   gatcaatttc ttttaagtaa tctaaatccc cattttttaa tttcttttta gcctctttaa      60
   ataatcctga ataaactaat acctgtttac ctttaagtga tttataaaat gcatcaaaga     120
   ctttttgatt tatttattaa ataatcacta tctttaccag aatacttagc catttcatat     180
   aattctttat tattattttg tcttattttt tgaacttgaa cttgtgttat ttctgaaatg     240
   cccgttacat cacgccataa atctaaccat tcttgttggc taatataata tcttttatct     300
   gtgaaatacg atttatttac tgcaattaac acatgaaaat gaggattata atcatctctt     360
   tttttattat atgtaatctc taacttacga acatatccct ttataacact acctactttt     420
   tttctcttta taagttttct aaaagaatta ttataacgtt ttatttcatt ttctaattca     480
   tcactcatta cattaggtgt agtcaaagtt aaaaagataa actccttttt ctcttgctgc     540
   ttaatatatt gcatcatcaa agataaaccc aatgcatctt ttctagcttt tctccaagca     600
   cagacaggac aaaatcgatt tttacaagaa ttagctttat ataatttctg tttttctaaa     660
   gttttatcag ctacaaaaga cagaaatgta ttgcaatctt caactaaatc catttgattc     720
   tctccaatat gacgtttaat aaatttctga aatacttgat ttctttgttt tttctcagta     780
   tacttttcca tgttataaca cataaaaaca acttagtttt cacaaactat gacaataaaa     840
   aaagttgctt tttccccttt ctatgtatgt tttttactag tcatttaaaa cgatacatta     900
   ataggtacga aaaagcaact ttttttgcgc ttaaaaccag tcataccaat aacttaaggg     960
   taactagcct cgccggcaat agttaccctt attatcaaga taagaaagaa aaggattttt    1020
   cgctacgctc aaatccttta aaaaaacaca aaagaccaca ttttttaatg tggtctttat    1080
   tcttcaacta aagcacccat tagttcaaca aacgaaaatt ggataaagtg ggatattttt    1140
   aaaatatata tttatgttac agtaatattg acttttaaaa aaggattgat tctaatgaag    1200
   aaagcagaca agtaagcctc ctaaattcac tttagataaa aatttaggag gcatatcaaa    1260
   tgaactttaa taaaattgat ttagacaatt ggaagagaaa agagatattt aatcattatt    1320
   tgaaccaaca aacgactttt agtataacca cagaaattga tattagtgtt ttataccgaa 1380
   acataaaaca agaaggatat aaattttacc ctgcatttat tttcttagtg acaagggtga 1440
   taaactcaaa tacagctttt agaactggtt acaatagcga cggagagtta ggttattggg 1500
   ataagttaga gccactttat acaatttttg atggtgtatc taaaacattc tctggtattt 1560
   ggactcctgt aaagaatgac ttcaaagagt tttatgattt atacctttct gatgtagaga 1620
   aatataatgg ttcggggaaa ttgtttccca aaacacctat acctgaaaat gctttttctc 1680
   tttctattat tccatggact tcatttactg ggtttaactt aaatatcaat aataatagta 1740
   attaccttct acccattatt acagcaggaa aattcattaa taaaggtaat tcaatatatt 1800
   taccgctatc tttacaggta catcattctg tttgtgatgg ttatcatgca ggattgttta 1860
   tgaactctat tcaggaattg tcagataggc ctaatgactg gcttttataa tatgagataa 1920
   tgccgactgt actttttaca gtcggttttc taatgtcact aacctgcccc gttagttgaa 1980
   gaaggttttt atattacagc tccagatcca tatccttctt tttctgaacc gacttctcct 2040
   ttttcgcttc tttattccaa ttgctttatt gacgttgagc ctcggaaccc ttaacaatcc 2100
   caaaacttgt cgaatggtcg gcttaatagc tcacgctatg ccgacattcg tctgcaagtt 2160
   tagttaaggg ttcttctcaa cgcacaataa attttctcgg cataaatgcg tggtctaatt 2220
   tttattttta ataaccttga tagcaaaaaa tgccattcca atacaaaacc acatacctat 2280
   aatcgataac cacataacag tcataaaacc actccttttt aacaaacttt atcacaagaa 2340
   atatttaaat tttaaatgcc tttattttga attttaaggg gcattttaaa gatttagggg 2400
   taaatcatat agttttatgc ctaaaaacct acagaagctt ttaaaaagca aatatgagcc 2460
   aaataaatat attctaattc tacaaacaaa aatttgagca aattcagtgt cgatttttta 2520
   agacactgcc cagttacatg caaattaaaa ttttcatgat tttttatagt tcctaacagg 2580
   gttaaaattt gtataacgaa agtataatgt ttatataacg ttagtataat aaagcatttt 2640
   aacattatac ttttgataat cgtttatcgt cgtcatcaca ataactttta aaatactcgt 2700
   gcataattca cgctgacctc ccaataacta catggtgtta tcgggaggtc agctgttagc 2760
   acttatattt tgttattgtt cttcctcgat ttcgtctatc attttgtgat taatttctct 2820
   tttttcttgt tctgttaagt cataaagttc actagctaaa tactcttttt gtttccaaat 2880
   ataaaaaatt tgatagatat attacggttg 2910